# EUROPEAN PATENT APPLICATION

(11) **EP 4 711 368 A1**
(43) Date of publication of application: **18.03.2026**
(21) Application number: 24818801.3
(22) Date of filing: 07.06.2024
(51) Int. Cl.: C07D 487/04, C07D 401/04, C07D 401/12, C07D 403/12, C07D 243/10, A61K 31/5517, A61P 23/00, A61P 25/20, A61P 25/08

(54) **AZEPINE COMPOUND, AND COMPOSITION AND USE THEREOF**

(30) Priority: 09.06.2023 CN 202310682400
(71) Applicant: CHENGDU MFS PHARMA. CO., LTD., Chengdu, Sichuan 611135 (CN)
(72) Inventor: CHEN, Guangwu, Chengdu, Sichuan 611135 (CN); LIU, Jin, Chengdu, Sichuan 611135 (CN); ZHANG, Wensheng, Chengdu, Sichuan 611135 (CN); ZHANG, Hancheng, Chengdu, Sichuan 611135 (CN); WANG, Changhua, Chengdu, Sichuan 611135 (CN); GUO, Lina, Chengdu, Sichuan 611135 (CN)
(74) Representative: karo IP
(86) International application number: PCT/CN2024/098223
(87) International publication number: WO 2024/251275

(57) **Abstract**

A compound as represented by general formula I, and a composition and the use thereof, wherein the compound as represented by general formula I has the following structure; and a pharmaceutically acceptable salt, a stereoisomer, a prodrug, a solvate and a deuterated compound thereof.

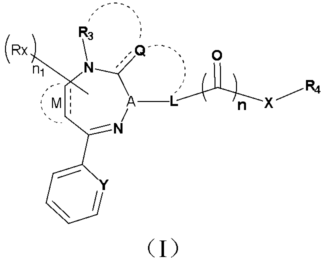

## Description

This application claims the benefit of priority from CNIPA Application No. 202310682400.4, filed June 9, 2023, and the invention title "Benzodiazepine Compounds, Compositions Thereof, and Uses Thereof," the entire contents of which are incorporated herein by reference.

### TECHNICAL FIELD

The present invention relates to the field of medicinal chemistry, and in particular relates to Benzodiazepine Compounds, Compositions Thereof, and Uses Thereof.

### BACKGROUND OF THE INVENTION

The benzodiazepine is the class of sedative-hypnotic drug developed in the late 1950s. Due to its lower toxicity and side effects compared to barbiturates, it has become the preferred clinical choice for sedation, hypnosis and anti-anxiety treatment.

The mechanism of action of benzodiazepine drugs is as follows: There are specific binding sites with high affinity for diazepam in the brain, known as benzodiazepine receptors, Their distribution is the densest in the cortex, followed by the limbic system and midbrain, and then the brainstem and spinal cord. This distribution is basically consistent with that of the GABAA receptors of γ-aminobutyric acid (GABA), the central inhibitory neurotransmitter. Benzodiazepine drugs can enhance the GABAergic neurotransmission function and synaptic inhibitory effect, and strengthen the binding of GABA to GABAA receptors, thus exerting anti-anxiety, sedative and hypnotic effects.

### SUMMARY OF THE INVENTION

The present invention provides the class of benzodiazepines compounds, as well as pharmaceutical compositions containing said benzodiazepines compounds, and uses of said azepine compounds.

In first aspect, the present invention provides a compound of general formula I, or a pharmaceutically acceptable salt thereof, a stereoisomer thereof, a prodrug thereof, a solvate thereof, or a deuterated derivative thereof: 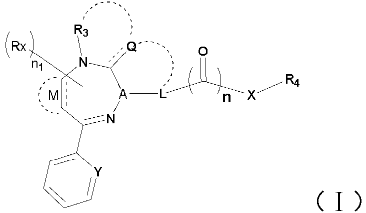
Wherein, Y is selected from N or -CF;
A is selected from N or CH;
M is selected from none, or M together with the adjacent carbon atom which they are attached to form fused ring, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl;
when M together with the adjacent carbon atom which they are attached to form fused ring, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
Q is selected from O, S-Rxx, N or 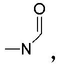 R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O, or S atom as ring atom;
When Q is N, then R₃ and Q together with N and C atoms which they are attached to form five-membered heteroaryl or six-membered heteroaryl, whcih containing at least two heteroatoms; in some embodiments, the five-membered aromatic heterocycle is imidazolyl, in some embodiments, the six-membered aromatic heterocycle is pyridinyl.

L is none, or L is selected from C₂₋₈ alkenyl, C₁₋₈ alkylene, wherein, the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
When Q is selected from N or 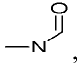 and A is N, then Q, A, L together with the adjacent atom which they are attached to form a five-membered or six-membered N-heterocycle.
n is 0 or 1, when n is 0, it means the -CO- is none;
X is none, or when n is 0 then X is O; when n is 0 then X is selected from O, substituted or unsubstituted N- alkyl, substituted or unsubstituted N-heterocycalkyl;
R₄ is selected from-H, halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, heterocyclic, aryl, N-heteroaryl, O-heteroaryl, S-heteroaryl, -SO₂(C₀₋₁₀ alkyl),-SO(C₀₋₁₀ alkyl),-SO₂O(C₀₋₁₀ alkyl),-SO₂N(C₀₋₁₀ alkyl)(C₀₋₁₀ alkyl), -SO₂(C₃₋₁₀ cycloalkyl),-SO₂-aryl,-CON(C₀₋₁₀ alkyl)(C₀₋₁₀ alkyl),-CO(C₀₋₁₀ alkyl),-CO(C₃₋₁₀ cycloalkyl),-CO(3-6-memenber heterocloalkyl),-(C₀₋₁₀ alkyl)COO(C₀₋₁₀ alkyl),-COO(C₃₋₁₀ cycloalkyl),-COO(3-6-memenber heterocloalkyl), alkenyl, alkyny, said heterocloalkyl contains at least one N, O or S ring atom, wherein the H on the aforementioned group is optionally substitured with one or more substituents selected from the group consisting of: halogen, -CN, -NO₂, -CF₃, straight-chain C₁₋₃ alkyl, -OC₀₋₁₀ alkyl, C₃₋₆ cycloalkyl, C₃₋₆ heterocycloalkyl, -N(C₀₋₁₀ alkyl)(C₀₋₁₀ alkyl), -COO(C₀₋₁₀ alkyl), -COO(C₃₋₁₀cycloalkyl), -COO(heterocycloalkyl), -CO(C₀₋₁₀ alkyl), -OCO(C₀₋₁₀ alkyl), -CON(C₀₋₁₀ alkyl)(C₀₋₁₀ alkyl), -OCOO(C₀₋₁₀ alkyl), phenyl, N-heteroaryl, O-heteroaryl, S-heteroaryl, alkenyl, alkyny.

Preferably, Y is N.

**In** Formula **I,** "- - - - - - " represents single bond or double bond.

**In** the present invention, the statement that "M forms fused ring with the adjacent carbon" means that M together with the adjacent carbon atom which they are attached to form the ring, which is fused with the seven-membered ring of the parent core structure to form a fused ring system. The ring formed by M and the adjacent carbon atom may be monocyclic ring or may itself form further fused ring system.

When Q is O, it is connected to the parent nucleus via double bond. When Q is S, it is connected to the parent nucleus via single bond. When Q is N or 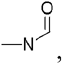 it is connected to the parent nucleus via single bond or double bond.

To clearly indicate the positions of M and the adjacent carbons, the atoms of the parent nucleus of the compound of Formula I are numbered as follows: 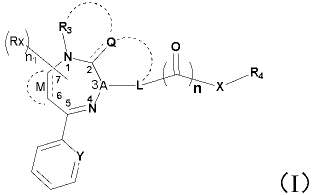 when M is selected from none, the compound of formula I has the following structure:

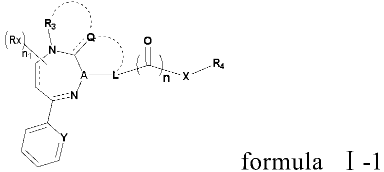

M and the adjacent carbon atoms form fused ring, which formed by M together with the carbon atoms at the 6-positions and 7-positions. The fused ring is selected from the group consisting of: saturated or unsaturated alicyclic ring, saturated or unsaturated heterocyclic ring, romatic ring and heteroaromatic ring.

Preferably, the compound, or a pharmaceutically acceptable salt thereof, a stereoisomer thereof, a prodrug thereof, a solvate thereof, or a deuterated derivative thereof, has the following structure: 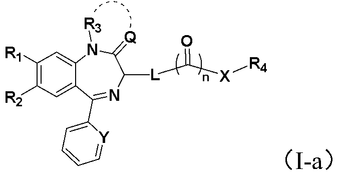
Wherein, Y is selected from N or -CF;
R₁ and R₂ are each independently selected from -H, halogen, -NO₂, -CN, -CF₃, C₂₋₈ alkenyl, C₂₋₈ alkyny, straight-chain or branched-chai C₁₋₁₀ alkyl, -N(C₀₋₁₀ alkyl)(C₀₋₁₀ alkyl), -OC₀₋₁₀ alkyl, C₃₋₁₀ cycloalky, N-heteroaryl, O-heteroaryl, S-heteroaryl, or R₁ and R₂ together with the carbon atom which they are attached to form five-membered aromatic heterocycle, six-membered aromatic heterocycle, aromatic ring; said five-membered aromatic heterocycle is selected from the group consisting of: furan, thiophene, pyrrole, pyrazole, imidazole, oxazole, thiazole; said six-membered aromatic heteroaryl is selected from the group consisting of: pyridine, pyridazine, pyrimidine, pyrazine; optionally, the hydrogen atoms on the said five-membered aromatic heterocycle, six-membered aromatic heterocycle or aromatic ring may be substituted with the following groups: halogen, -CN, -CF₃, straight-chain or branched C₁₋₁₀ alkyl, -N(C₀₋₁₀ alkyl)(C₀-₁₀ alkyl), -OC₀-₁₀ alkyl, C₃₋₁₀ cycloalkyl, N-heterocycloalkyl, O-heterocycloalkyl, S-heterocycloalkyl; Optionally, the said aromatic ring is selected from six-membered aromatic ring.
Q is selected from O, S or N, R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O, or S atom as ring atom;
When Q is N, R₃ and Q together with N and C atoms which they are attached to form five-membered heteroaryl or six-membered heteroaryl, which containing at least two heteroatoms; Optionally, the said five-membered heteroaryl is imidazolyl;
L is none, or L is selected from C₂₋₈ alkenyl or C₁₋₈ alkylene, wherein, H of the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
When Q is N and A is N, then Q, A, L together with the adjacent atoms which they are attached to form five-membered or six-membered N-heterocyclic;
n is 0 or 1, when n is 0, it means the -CO- is none;
X is none, or when n is 1 then X is O, when n is 0 then X is selected from O, substituted or unsubstituted N-alkyl, substituted or unsubstituted N-heterocycalkyl;
R₄ is selected from-H, halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, heterocyclic, aryl, N-heteroaryl, O-heteroaryl, S-heteroaryl, -SO₂(C₀₋₁₀ alkyl), -SO(C₀₋₁₀ alkyl), -SO₂O(C₀₋₁₀ alkyl), -SO₂N(C₀₋₁₀ alkyl)(C₀₋₁₀ alkyl), -SO₂(C₃₋₁₀ cycloalkyl), -SO₂-aryl, -CON(C₀₋₁₀ alkyl)(C₀₋₁₀ alkyl), -CO(C₀₋₁₀ alkyl), -CO(C₃₋₁₀ cycloalkyl), -CO(3-6-memenber heterocloalkyl), -(C₀₋₁₀ alkyl)COO(C₀₋₁₀ alkyl), -COO(C₃₋₁₀ cycloalkyl), -COO(3-6-memenber heterocloalkyl), alkenyl, alkynyl, said heterocloalkyl contains at least one N, O or S ring atom, wherein, said group is optionally substitured with one or more substituents selected from the group consisting of: halogen, -CN, -NO₂, -CF₃, straight-chain C₁₋₃ alkyl, -OC₀₋₁₀ alkyl, C₃₋₆ cycloalkyl, C₃₋₆ heterocycloalkyl, -N(C₀₋₁₀ alkyl)(C₀₋₁₀ alkyl), -COO(C₀₋₁₀ alkyl), -COO(C₃₋₁₀cycloalkyl), -COO(heterocycloalkyl), -CO(C₀₋₁₀ alkyl), -OCO(C₀₋₁₀ alkyl), -CON(C₀₋₁₀ alkyl)(C₀₋₁₀ alkyl), -OCOO(C₀₋₁₀ alkyl), phenyl, N-heteroaryl, O-heteroaryl, S-heteroaryl, alkenyl, alkynyl.

Preferably, the compound, or a pharmaceutically acceptable salt thereof, a stereoisomer thereof, a prodrug thereof, a solvate thereof, or a deuterated derivative thereof, has the following structure: 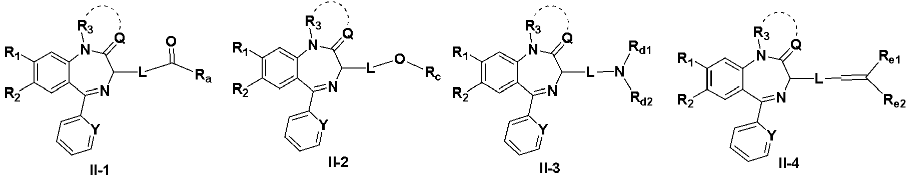 Wherein, Y is selected N or -CF;
R₁ and R₂ are each independently selected from -H, -F, -Cl, -Br, -NO₂, -CN, -CF₃, C₂₋₄ alkenyl, C₂₋₄ alkynyl, straight-chain or branched-chai C₁₋₁₀ alkyl, -N(C₀₋₁₀ alkyl)(C₀₋₁₀ alkyl), -OC₀₋₁₀ alkyl, C₃₋₁₀ cycloalky, N-heteroaryl, O-heteroaryl, S-heteroaryl, or R₁ and R₂ together with the carbon atom which they are attached to form five-membered aromatic heterocycle, six-membered aromatic heterocycle or benzene ring; said five-membered aromatic heterocycle is selected from the group consisting of: furan, thiophene, pyrrole, pyrazole, imidazole, oxazole, thiazole; said six-membered aromatic heteroaryl is selected from the group consisting of: pyridine, pyridazine, pyrimidine, pyrazine; optionally, any hydrogen atom on said five-membered aromatic heterocycle, six-membered aromatic heterocycle, or benzene may be substituted by substituent selected from the group consisting of: halogen, -CN, -CF₃, straight-chain or branched C₁₋₁₀ alkyl, -N(C₀₋₁₀ alkyl)(C₀-₁₀ alkyl), -OC₀-₁₀ alkyl, C₃₋₁₀ cycloalkyl, N-heterocycloalkyl, O-heterocycloalkyl, S-heterocycloalkyl;
Q is selected from O or N, R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, said the heterocyclyl contains at least one N, O, or S atom as ring atom;
When Q is N, then R₃ and Q together with N and C atom which they are attached to form five-membered aromatic heterocycle or six-membered aromatic heterocycle containing at least two heteroatoms;
L is none, or L is selected from C₂₋₈ alkenyl, C₁₋₈ alkylene, wherein, H of the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
Rₐ is selected from -H, straight-chain or branched-chain C₁₋₁₀ alkyl, -OR_{b}, R_{b} is selected from straight-chain or branched-chain C₁₋₁₀ alkyl, C₃₋₁₀ cycloalkyl, 3-8 membered heterocycloalkyl, aryl, heteroaryl, wherein, the aforementioned group is optionally substituents selected from the group consisting of: halogen, -CN, -CH₃, -C₂H₅, -OC₁₋₅, C₃₋₆ cycloalkyl, 3-6 membered heterocycloalkyl, -CO(C₀₋₁₀ alkyl), -OCO(C₀₋₁₀ alkyl), ethenyl, propadienyl, ethynyl; further, the aforementioned alkyl is optionally substituents selected from the group consisting of: -CN, -OCH₂F, -OCHF₂, -OCF₃, -OC₀₋₁₀ alkyl, C₃₋₄ alkyl;
R_{c} is selected from -H, straight-chain or branched-chain C₁₋₁₀ alkyl, C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocycloalkyl), -CH₂CON(C₀₋₁₀ alkyl)(C₀₋₁₀ alkyl), benzyl, aryl, the hydrogen atoms on these groups can be substituted with the following substituents: -F, -Cl, -CN, -NO₂, straight-chain or branched-chain C₁₋₁₀ alkyl, 3-6 membered heterocycloalkyl, -OC₁₋₅ alkyl, -N(C₁₋₃ alkyl)(C₁₋₃ alkyl), C₂₋₈ alkenyl, imidazolyl, oxazolyl, thiazolyl, pyridinyl, pyrimidinyl; or R_{c} is selected from 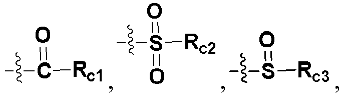 R_{c1}, R_{c2}, R_{c3} are independently selected from straight-chain or branched-chain C₁₋₁₀ alkyl, -OC₀₋₅ alkyl, -O(C₃₋₆ cycloalkyl), -O(C₃₋₆ heterocycloalkyl), C₃₋₁₀ cycloalkyl, 3-6 membered heterocycloalkyl, -N(C₀₋₁₀ alkyl)(C₀₋₁₀ alkyl), alkenyl, alkynyl, aryl, heteroaryl, the hydrogen atoms on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocycloalkyl, ethenyl; the aforementioned alkyl moieties are replaceable with a substituent selected from the group consisting of: -CN, -OCF₃, -OC₀₋₁₀ alkyl, C₃₋₄ cycloalkyl;
R_{d1} and R_{d2} are independently selected from H, straight-chain or branched-chain C₁₋₁₀ alkyl, -COO(C₀₋₁₀ alkyl), -CO(C₀₋₁₀ alkyl), 3-6 membered cycloalkyl, 3-6 membered heterocycloalkyl, aryl, or R_{d1} and R_{d2} together with the nitrogen atom which they are attached to form 3-6 membered heterocycloalkyl, the hydrogen atoms on these groups can be substituted with the following substituents: halogen, -CN, -NO₂, straight-chain or branched-chain C₁₋₃ alkyl, -OC₁₋₃ alkyl, C₃₋₆ cycloalkyl, -(C₀₋₁₀ alkyl)COO(C₀₋₁₀ alkyl), -CO(C₀₋₁₀ alkyl), aryl, -N(C₁₋₃ alkyl)(C₁₋₃ alkyl); the aforementioned alkyl groups are optionally substituted with a substituent selected from the group consisting of: -CN, -OCF₃, -OC₀₋₁₀ alkyl, -CO(C₀₋₁₀ alkyl), C₃₋₄ cycloalkyl, aryl;
Rₑ₁ and Rₑ₂ are independently selected from H, straight-chain or branched-chain C₁₋₁₀ alkyl.

In some embodiments, the said compound, as well as its pharmaceutically acceptable salt thereof, a stereoisomer thereof, a prodrug thereof, a solvate thereof, or a deuterated derivative thereof, has the following structural formula:

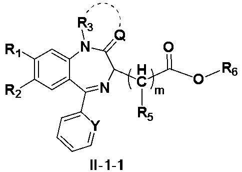

Wherein, the substituents Y, Q, R¹, R², and R³ are as specified above.
m is an integer from 0 to 4;
R₅ is selected from -H, halogen, -CN, -CF₃, -CO(C₀₋₁₀ alkyl), -COO(C₀₋₁₀ alkyl);
R₆ is selected from -H, straight-chain or branched-chain C₁₋₁₀ alkyl, C₃₋₁₀ cycloalkyl, membered heterocycloalkyl, aryl, wherein, the hydrogen atom on these groups can be substituted with the following substituents: halogen, -CN, -CH₃, -C₂H₅, -OCH₃, C₃₋₆ cycloalkyl, 3-6 membered heterocycloalkyl, -CO(C₀₋₁₀ alkyl), -OCO(C₀₋₁₀ alkyl), ethenyl, propadienyl, ethynyl, further, the aforementioned alkyl is optionally substituents selected from the group consisting of: -CN, -OCH₂F, -OCHF₂, -OCF₃, -OC₀₋₁₀ alkyl, C₃₋₄ alkyl;
Further, the compound, or a pharmaceutically acceptable salt thereof, a stereoisomer thereof, a prodrug thereof, a solvate thereof, or a deuterated derivative thereof, has the following structure: 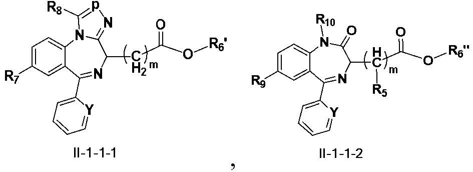
Wherein, Y is selected from N or -CF; P is N or CH;
m is an integer from 0 to 4;
R₇ is selected from H, -F, -Cl, -Br, **-I,** -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₈ is selected from -H, -CN, -CF₃, straight-chain or branched-chain C₁₋₄ alkyl, C₃₋₁₀ cycloalkyl;
R₉ is selected from -H, -F, -Cl, -Br, -I, -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₀ is selected from -H, straight-chain or branched-chain C₁₋₃ alkyl, hydrogen atoms on the alkyl are replaceable with substituent selected from the group consisting of: -N(C₁-C₃ alkyl)₂, 5-6 membered N-heterocycloalkyl;
R₅ is selected from -H, halogen, -CN, -CF₃, -CO(C₀₋₁₀ alkyl), -COO(C₀₋₁₀ alkyl);
R_{6'} and R_{6"} are independently selected from -H, straight-chain or branched-chain C₁₋₁₀ alkyl, C₃₋₁₀ cycloalkyl, 3-6 membered heterocycloalkyl, aryl, wherein, the hydrogen atoms on these groups can be substituted with the following substituents: halogen, -CN, -CH₃, -C₂H₅, -OCH₃, C₃₋₆ cycloalkyl, 3-6 membered heterocycloalkyl, -CO(C₀₋₁₀ alkyl), -OCO(C₀₋₁₀ alkyl), ethenyl, propadienyl, ethynyl, aryl, further, the aforementioned alkyl moieties are replaceable with substituent selected from the group consisting of: -CN, -OCH₂F, -OCHF₂, -OCF₃, -OC₀₋₁₀ alkyl, C₃₋₄ cycloalkyl.

Further, the compound, or a pharmaceutically acceptable salt thereof, a stereoisomer thereof, a prodrug thereof, a solvate thereof, or a deuterated derivative thereof, has the following structure: 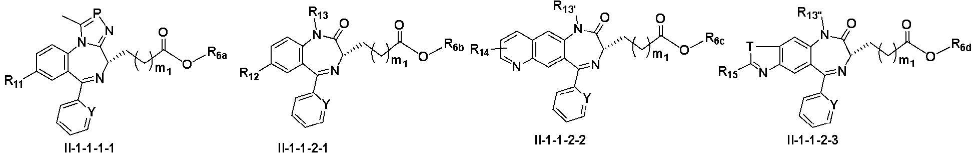 Wherein, Y is selected from N or -CF; P is N or CH;
m₁ is an integer from 0 to 2;
R₁₁ is selected from -F, -Cl, -Br, -I, -NO₂, ethyne, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₆ₐ is selected from 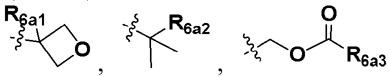 or C₁₋₄ alkyl, the alkyl is optionally substituted by one or more substituents selected from -F, -OCH₃ and C₃₋₆ cycloalkyl, R₆ₐ₁ is selected from -H, -CN, -CH₃, -C₂H₅, ethenyl, propadienyl, ethynyl; R₆ₐ₂ is selected from ethenyl, ethynyl, -COCH₃, -COC₂H₅, 3-4 membered epoxyalkyl; R₆ₐ₃ is selected from -CH₃, -C₂H₅, -OCH₃, -OC₂H₅;
R₁₂ is selected from -F, -Cl, -Br, -I, -NO₂, ethynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₃, R_{13'}, R_{13"} are independently selected from -H, straight-chain or branched-chain C₁₋₃ alkyl, the alkyl is optionally substituted by one or more substituents selected from -N(C₁₋₃ alkyl)(C₁₋₃ alkyl), 5-6 membered N-heterocycloalkyl;
R_{6b} is selected from -H, -CH₃, -C₂H₅, propyl, isopropyl, butyl, tert-Butyl, 3-4 membered saturated oxacycloalkyl, the hydrogen atoms on these groups can be substituted with the following substituents: -F, -Cl, -CN, -CH₃, -C₂H₅, -OCH₃, C₃₋₄ cycloalkyl, 3-6 membered heterocycloalkyl, -CO(C₁₋₃ alkyl), -OCO(C₁₋₃ alkyl), ethylene, ethynyl, further, the alkyl group is optionally substituted by -OCH₃,-OC₂H₅;
R₁₄ is selected from -H, halogen, -NO₂, -CN, -CF₃, straight-chain or branched-chain C₁₋₁₀ alkyl, N(C₀₋₁₀ alkyl)(C₀₋₁₀ alkyl), -OC₀₋₁₀ alkyl, C₃₋₁₀ cycloalkyl;
R_{6c} is selected from -H, traight-chain or branched-chain C₁₋₅ alkyl, -OC₀₋₅ alkyl, C₃₋₆ cycloalkyl, -O-heterocycloalkyl, the hydrogen atoms on these groups can be substituted with the following substituents: -F, -Cl, -CN, -CH₃, -C₂H₅, -OCH₃, C₃₋₄ cycloalkyl, ethylene, ethynyl;
T is selected from C, N, O or S;
R₁₅ is selected from -H, halogen, -NO₂, -CN, -CF₃, straight-chain or branched-chain C₁₋₁₀ alkyl, N(C₀₋₁₀ alkyl)(C₀₋₁₀ alkyl), -OC₀₋₁₀ alkyl, C₃₋₁₀ cycloalkyl;
R_{6d} is selected from -H, traight-chain or branched-chain C₁₋₅ alkyl, -OC₀₋₅ alkyl, C₃₋₆ cycloalkyl, -O heterocycloalkyl, the hydrogen atoms on these groups can be substituted with the following substituents: -F, -Cl, -CN, -CH₃, -C₂H₅, -OCH₃, C₃₋₄ cycloalkyl, ethylene, ethynyl.

In some embodiments, the said compound, as well as its pharmaceutically acceptable salt thereof, a stereoisomer thereof, a prodrug thereof, a solvate thereof, or a deuterated derivative thereof, has the following structural formula:

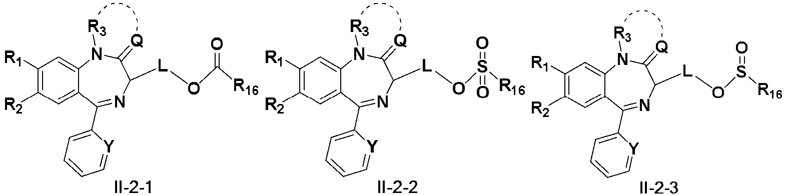

Wherein, the substituents Y, Q, R¹, R², and R³ are as specified above.

R₁₆ is selected from straight-chain or branched-chain C₁₋₁₀ alkyl, -OC₀₋₅ alkyl,-O(C₃₋₄ cycloalkyl),-O(C₃₋₄ heterocycloalkyl),C₃₋₁₀ cycloalkyl,3-6 membered heterocycloalkyl,-N(C₀₋₁₀ alkyl)(C₀₋₁₀ alkyl), ethenyl, ethynyl, aryl, pyridinyl, imidazolyl, the hydrogen atoms on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocycloalkyl, ethenyl, the aforementioned alkyl moieties are replaceable with a substituent selected from the group consisting of: -CN, -OCF₃, -OC₀₋₁₀ alkyl, C₃₋₄ cycloalkyl.

Further, the compound, or a pharmaceutically acceptable salt thereof, a stereoisomer thereof, a prodrug thereof, a solvate thereof, or a deuterated derivative thereof, has the following structure:

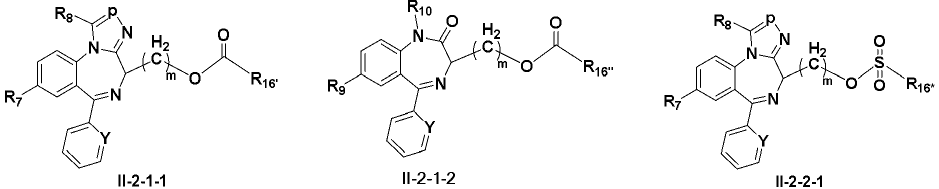

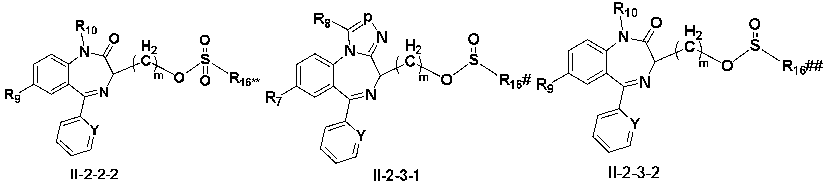

Wherein, Y is selected from N or -CF; P is N or CH;
m is an integer from 0 to 4;
R₇ is selected from H, -F, -Cl, -Br, -I, -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₈ is selected from -H, -CN, -CF₃, straight-chain or branched-chain C₁₋₄ alkyl, C₃₋₁₀ cycloalkyl;
R₉ is selected from -H, -F, -Cl, -Br, -I, -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₀ is selected from -H, straight-chain or branched-chain C₁₋₃ alkyl, hydrogen atoms on the alkyl are replaceable with a substituent selected from the group consisting of: -N(C₁-C₃ alkyl)₂, 5-6 membered N-heterocycloalkyl;
R_{16'}, R_{16"}, R_{16*}, R_{16**}, R_{16#} and R_{16##} are independently selected from straight-chain or branched-chain C₁₋₁₀ alkyl, -OC₀₋₅ alkyl, -O(C₃₋₄ cycloalkyl), -O(C₃₋₄ heterocycloalkyl), C₃₋₁₀ cycloalkyl, 3-6 membered heterocycloalkyl, -N(C₀₋₁₀ alkyl)(C₀₋₁₀ alkyl), ethenyl, ethynyl, aryl, pyridyl, imidazolyl, the hydrogen atoms on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocycloalkyl, ethenyl, the aforementioned alkyl moieties are replaceable with substituent selected from the group consisting of: -CN, -OCF₃, -OC₀₋₁₀ alkyl, C₃₋₄ cycloalkyl;

Further, the compound, or a pharmaceutically acceptable salt thereof, a stereoisomer thereof, a prodrug thereof, a solvate thereof, or a deuterated derivative thereof, has the following structure:

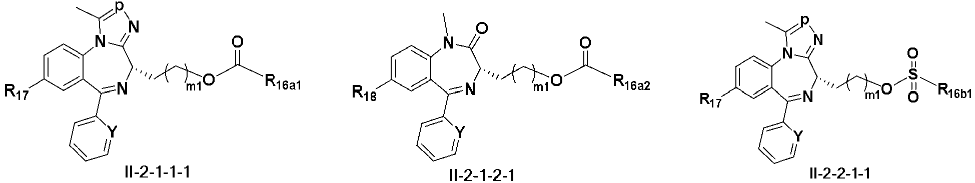

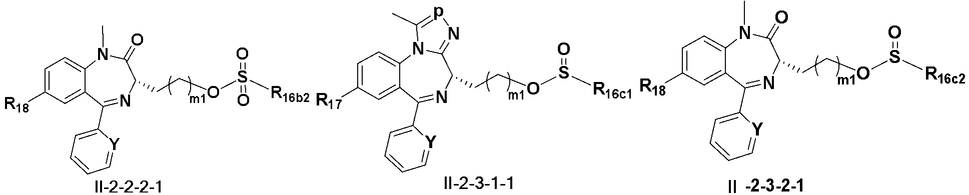

Wherein, Y is selected from N or -CF;
m₁ is an integer from 0 to 2;
R₁₇, R₁₈ are independently selected from -F, -Cl, -Br, -I, -NO₂, ethynyl, -CF₃;
R₁₆ₐ₁ and R₁₆ₐ₂ are independently selected from sraight-chain or branched-chain C₁₋₅ alkyl, -OC₁₋₃ alkyl, -O(C₃₋₄ cycloalkyl), -O(C₃₋₄ heterocycloalkyl), C₃₋₆ cycloalkyl, 3-6 membered N-heterocycloalkyl, 3-6 membered O-heterocycloalkyl, -N(C₀₋₁₀ alkyl)(C₀₋₁₀ alkyl), ethylene, ethynyl, aryl, pyridinyl, imidazolyl, the hydrogen atom on these groups can be substituted with the following substituents: halogen, -CN, -CH₃, -C₂H₅, -OCH₃, -N(C₁₋₃ alkyl)(C₁₋₃ alkyl), ethylene;
R_{16b1} and R_{16b} are independently selected from sraight-chain or branched-chain C₁₋₅ alkyl, -OC₁₋₃ alkyl, C₃₋₆ cycloalkyl, aryl, -N(C₁₋₃ alkyl)(C₁₋₃ alkyl), the hydrogen atom on these groups can be substituted with the following substituents: halogen, -CN, -CH₃, -C₂H₅, -OCH₃;
R_{16c1} and R_{16c2} are independently selected from sraight-chain or branched-chain C₁₋₅ alkyl, -OC₁₋₃ alkyl, C₃₋₆ cycloalkyl, aryl.

In some embodiments, the said compound, as well as its pharmaceutically acceptable salt thereof, a stereoisomer thereof, a prodrug thereof, a solvate thereof, or a deuterated derivative thereof, has the following structural:

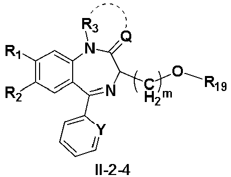

Wherein, the substituents Y, Q, R¹, R², and R³ are as specified above.
m is an integer from 0 to 4;
R₁₉ is selected from -H, straight-chain or branched-chain C₁₋₁₀ alkyl, -C₃₋₁₀ cycloalkyl), -CH₂CO(C₁₋₅ alkyl), -CH₂COO(C₁₋₅ alkyl), -CH₂COO(C₃₋₆ cycloalkyl), -CH₂COO(C₃₋₆ heterocycloalkyl), -CH₂CON(C₁₋₃ alkyl)(C₁₋₃ alkyl), benzyl, aryl, the hydrogen atom on these groups can be substituted with the following substituents: -F, -Cl, -CN, -NO₂, -CH₃, -C₂H₅, -C₃H₇, 3-6 membered heterocycloalkyl, -OC₁₋₃ alkyl, -N(C₁₋₃ alkyl)(C₁₋₃ alkyl), ethenyl, imidazolyl, oxazolyl, thiazolyl, pyridinyl;
Further, the compound, or a pharmaceutically acceptable salt thereof, a stereoisomer thereof, a prodrug thereof, a solvate thereof, or a deuterated derivative thereof, has the following structure: 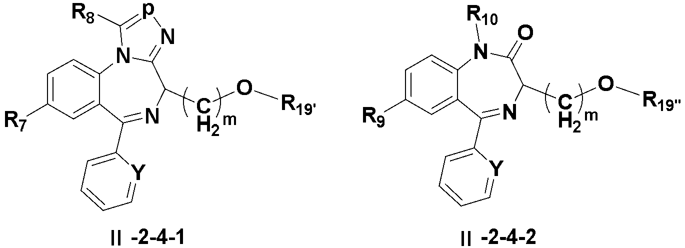
Wherein, Y is selected from N or -CF; P is N or CH;
m is an integer from 0 to 4;
R₇ is selected from H, -F, -Cl, -Br, -I, -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₈ is selected from -H, -CN, -CF₃, straight-chain or branched-chain C₁₋₄ alkyl, C₃₋₁₀ cycloalkyl;
R₉ is selected from -H, -F, -Cl, -Br, -I, -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₀ is selected from -H, straight-chain or branched-chain C₁₋₃ alkyl, hydrogen atoms on the alkyl are replaceable with substituent selected from the group consisting of: -N(C₁-C₃ alkyl)₂, 5-6 membered N-heterocycloalkyl;
R_{19'} and R_{19"} are independently selected from -H, straight-chain or branched-chain C₁₋₁₀ alkyl, C₃₋₁₀ cycloalkyl, -CH₂CO(C₁₋₅ alkyl), -CH₂COO(C₁₋₅ alkyl), -CH₂COO(C₃₋₆ cycloalkyl), -CH₂COO(C₃₋₆ heterocycloalkyl), -CH₂CON(C₁₋₃ alkyl)(C₁₋₃ alkyl), benzyl, aryl, the hydrogen atoms on these groups can be substituted with the following substituents: -F, -Cl, -CN, -NO₂, -CH₃, -C₂H₅, -C₃H₇, 3-6 membered heterocycloalkyl, -OC₁₋₃ alkyl, -N(C₁₋₃ alkyl)(C₁₋₃ alkyl), ethynyl, imizazole,oxazolyl, thiazolyl, pyridinyl;
Further, the compound, or a pharmaceutically acceptable salt thereof, a stereoisomer thereof, a prodrug thereof, a solvate thereof, or a deuterated derivative thereof, has the following structure: 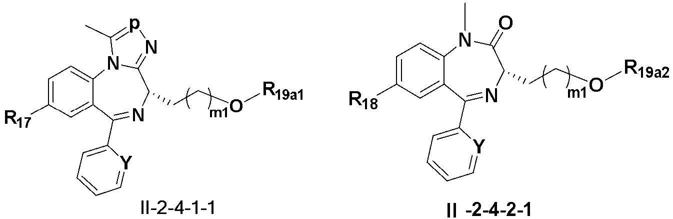
Wherein, Y is selected from N or -CF;
m₁ is an integer from 0 to 2;
R₁₇ and R₁₈ are independently selected from -F, -Cl, -Br, -I, -NO₂, ethyl, -CF₃, sraight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₉ₐ₁ and R₁₉ₐ₂ are independently selected from -H, sraight-chain or branched-chain C₁₋₃ alkyl, C₃₋₆ cycloalkyl, -CH₂CO(C₁₋₃ alkyl), -CH₂COO(C₁₋₃ alkyl), -CH₂COO(C₃₋₆ heterocycloalkyl), -CH₂CON(C₁₋₃ alkyl)(C₁₋₃ alkyl), benzyl, aryl, the hydrogen atom on these groups can be substituted with the following substituents: -F, -Cl, -CN, -NO₂, -CH₃, -C₂H₅, -C₃H₇, -OC₁₋₃ alkyl, -N(C₁₋₃ alkyl)(C₁₋₃ alkyl), ethylene, imidazolyl, thiophene, pyridinyl.

In some embodiments, the said compound, as well as its pharmaceutically acceptable salt thereof, a stereoisomer thereof, a prodrug thereof, a solvate thereof, or a deuterated derivative thereof, has the following structural:

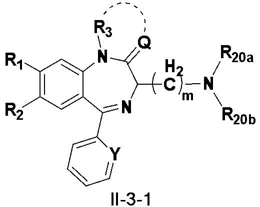

Wherein, Y is selected from N or -CF; P is N or CH;
m is an integer from 0 to 4;
R₂₀ₐ and R_{20b} are independently selected from -H, straight-chain or branched-chain C₁₋₁₀ alkyl, -COO(C₀₋₁₀ alkyl), -CO(C₀₋₁₀ alkyl), C₃₋₅ cycloalkyl, 3-6 membered heterocycloalkyl, ethenyl, or R_{d1} and R_{d2} together with the nitrogen atom which they are attached to form 3-6 membered N-heterocycloalkyl, the hydrogen atom on these groups can be substituted with the following substituents: -F, -Cl, -CN, -NO₂, -CH₃, -C₂H₅, -OC₁₋₃ alkyl, C₃₋₆ cycloalkyl, -(C₀₋₃ alkyl)COO(C₀₋₅ alkyl), -CO(C₀₋₅ alkyl), -N(C₁₋₃ alkyl)(C₁₋₃ alkyl), aryl.

Further, the compound, or a pharmaceutically acceptable salt thereof, a stereoisomer thereof, a prodrug thereof, a solvate thereof, or a deuterated derivative thereof, has the following structure:

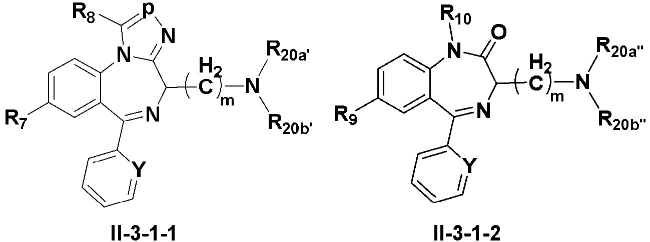

Wherein, Y is selected from N or -CF; P is N or CH;
m is an integer from 0 to 4;
R₇ is selected from H, -F, -Cl, -Br, -I, -NO₂, -CN, alkenyl, alkynyl, -CF₃;
R₈ is selected from -H, -CN, -CF₃, straight-chain or branched-chain C₁₋₄ alkyl, C₃₋₁₀ cycloalkyl;
R₉ is selected from -H, -F, -Cl, -Br, -I, -NO₂, -CN, alkenyl, alkynyl, -CF₃;
R₁₀ is selected from -H, straight-chain or branched-chain C₁₋₃ alkyl, hydrogen atoms on the alkyl are replaceable with substituent selected from the group consisting of: -N(C₁-C₃ alkyl)₂, 5-6 membered N-heterocycloalkyl;
R_{20a'}, R_{20a"}, R_{20b'} and R_{20b"} are independently selected from -H, straight-chain or branched-chain C₁₋₁₀ alkyl, -COO(C₀₋₁₀ alkyl), -CO(C₀₋₁₀ alkyl), C₃₋₅ cycloalkyl, 3-6 membered heterocycloalkyl, aryl, or R_{d1} and R_{d2} together with the nitrogen atom which they are attached to form 3-6 membered heterocycloalkyl, the hydrogen atoms on these groups can be substituted with the following substituents: -F, -Cl, -CN, -NO₂, -CH₃, -C₂H₅, -OC₁₋₃ alkyl, C₃₋₆ cycloalkyl, -(C₀₋₃ alkyl)COO(C₀₋₅ alkyl), -CO(C₀₋₅ alkyl), -N(C₁₋₃ alkyl)(C₁₋₃ alkyl), aryl, the aforementioned alkyl moieties are replaceable with substituent selected from the group consisting of: -CN, -OCF₃, -OC₀₋₃ alkyl, -CO(C₀₋₅ alkyl), C₃₋₄ cycloalkyl, aryl.

Further, the compound, or a pharmaceutically acceptable salt thereof, a stereoisomer thereof, a prodrug thereof, a solvate thereof, or a deuterated derivative thereof, has the following structure:

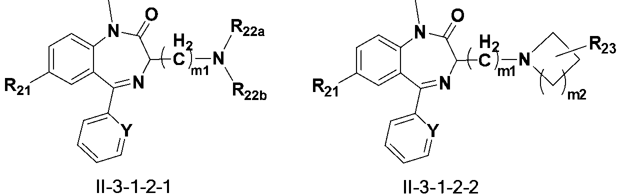

Wherein, Y is selected from N or -CF;
m₁ is an integer from 0 to 2;
R₂₁ is selected from -F, -Cl, -Br, -I, -NO₂, ethynyl, -CF₃,
R₂₂ₐ and R_{22b} are independently selected from -H, sraight-chain or branched-chain C₁₋₃ alkyl, -COO(C₁₋₃ alkyl), -CO(C₁₋₃ alkyl), C₃₋₅ cycloalkyl, 3-6 membered heterocycloalkyl, aryl, the hydrogen atoms on these groups can be substituted with the following substituents:-F, -Cl, -CN, -NO₂, -CH₃, -C₂H₅, -OCH₃, -(C₀₋₃ alkyl)COO(C₁₋₃ alkyl);
m₂ is an integer from 1 to 3;
R₂₃ is independently selected from -H, sraight-chain or branched-chain C₁₋₃ alkyl, -COO(C₁₋₃ alkyl), -CO(C₁₋₃ alkyl), C₃₋₅ cycloalkyl, 3-6 membered heterocycloalkyl, -N(C₀₋₃ alkyl)(C₀₋₃ alkyl), aryl, the hydrogen atoms on these groups can be substituted with the following substituents: -F, -Cl, -CN, -NO₂, -CH₃, -C₂H₅, -OC₁₋₃ alkyl, C₃₋₆ cycloalkyl, -(C₀₋₃ alkyl)COO(C₀₋₅ alkyl), -CO(C₀₋₅ alkyl), aryl.

Preferably, the compound, or a pharmaceutically acceptable salt thereof, a stereoisomer thereof, a prodrug thereof, a solvate thereof, or a deuterated derivative thereof, has the following structure:

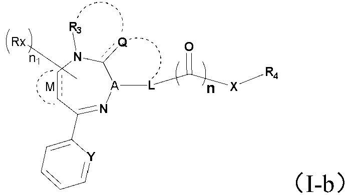

Wherein, Y is selected from N or -CF;
M is selected from none, or forms fused ring with the adjacent carbon, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl; preferably, Rx is selected from -H, halogen, -NO₂, -CN, -CF₃, C₂₋₃ alkenyl, C₂₋₃ alkynyl, straight-chain or branched-chain C₁₋₆ alkyl, -N(C₀₋₆ alkyl)(C₀₋₆ alkyl), -OC₀₋₆ alkyl, C₃₋₆ cycloalkyl, N-heterocyclyl, O-heterocyclyl, S-heterocyclyl; further preferably, Rx is selected from -H, halogen, -NO₂, -CN, -CF₃, C₂₋₃ alkenyl, C₂₋₃ alkynyl, straight-chain or branched-chain C₁₋₃ alkyl, -N(C₀₋₃ alkyl)(C₀₋₃ alkyl), -OC₀₋₃ alkyl, C₃₋₆ cycloalkyl, N-heterocyclyl, O-heterocyclyl, S-heterocyclyl; when M forms fused ring with adjacent carbon, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O heterocyclyl, S heterocyclyl, -NHCO(C₀₋₁₀ alkyl); preferably, the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₆ alkenyl, C₂₋₆ alkynyl, straight-chain or branched-chain C₁₋₆ alkyl, -N(C₀₋₆ alkyl)(C₀₋₆ alkyl), -OC₀₋₆ alkyl, C₃₋₆ cycloalkyl, N-heterocyclyl,
O-heterocyclyl, S-heterocyclyl, -NHCO(C₀₋₁₀ alkyl); further preferably, the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₃ alkenyl, C₂₋₃ alkynyl, straight-chain or branched-chain C₁₋₃ alkyl, -N(C₀₋₃ alkyl)(C₀₋₃ alkyl), -OC₀₋₃ alkyl, C₃₋₆ cycloalkyl, N-heterocyclyl,
O-heterocyclyl, S-heterocyclyl, -NHCO(C₀₋₁₀ alkyl);
Q is selected from O, S-Rxx, N or 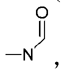 R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O, or S atom as ring atom;
Rxx is selected from H, straight-chain or branched-chain C₁₋₅ alkyl, said the H of alkyl which are optionally substituents selected from the group consisting of: halogen, nitro, cyano, -OC₀₋₁₀ alkyl;
When Q is N, R₃ and Q are linked by N and C atoms to form five-membered aromatic heterocycle or six-membered aromatic heterocycle containing at least two heteroatoms; preferably, said five-membered aromatic heterocycle is imidazole; preferably, the hydrogen atom on imidazole can be substituted with the following substituents: halogen, cyano, -C₀₋₁₀ alkyl, -N(C₀₋₁₀ alkyl)(C₀₋₁₀ alkyl), -OC₀₋₁₀ alkyl; preferably, said five-membered aromatic heterocycle is pyridine; the hydrogen atom on pyridine can be substituted with the following substituents: halogen, cyano -C₁₋₆ alkyl, -N(C₁₋₆ alkyl)(C₁₋₆ alkyl), -OC₁₋₆ alkyl;
L is none, or L is selected from C₂₋₈ alkenyl, C₁₋₈ alkylene, wherein, the aforementioned groupis optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
When Q is selected from N or 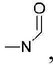 and A is N, then Q, A, L together with adjacent atom which they are attached to form five-membered or six-membered N-heterocycle;
n is 0 or 1, when n is 0, it means the -CO- is none;
X is none, or when n is 1, X is O, when n is 0, X is selected from O, substituted or unsubstituted N-alkyl, substituted or unsubstituted N-heterocycalkyl;
R₄ is selected from-H, halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, heterocyclic, aryl, N heteroaryl, O heteroaryl, S heteroaryl, -SO₂(C₀₋₁₀ alkyl), -SO(C₀₋₁₀ alkyl), -SO₂O(C₀₋₁₀ alkyl), -SO₂N(C₀₋₁₀ alkyl)(C₀₋₁₀ alkyl), -SO₂(C₃₋₁₀ cycloalkyl), -SO₂-aryl, -CON(C₀₋₁₀ alkyl)(C₀₋₁₀ alkyl), -CO(C₀₋₁₀ alkyl),-CO(C₃₋₁₀ cycloalkyl), -CO(3-6-memenber heterocloalkyl), -(C₀₋₁₀ alkyl)COO(C₀₋₁₀ alkyl), -COO(C₃₋₁₀ cycloalkyl), -COO(3-6-memenber heterocloalkyl), alkenyl, alkynyl, said heterocloalkyl contains at least one N, O or S ring atom, wherein said group is optionally substitured with one or more substituents selected from the group consisting of: halogen, -CN, -NO₂, -CF₃, straight-chain C₁₋₃ alkyl, -OC₀₋₁₀ alkyl, C₃₋₆ cycloalkyl, C₃₋₆ heterocycloalkyl, -N(C₀₋₁₀ alkyl)(C₀₋₁₀ alkyl), -COO(C₀₋₁₀ alkyl), -COO(C₃₋₁₀ cycloalkyl), -COO(heterocycloalkyl), -CO(C₀₋₁₀ alkyl), -OCO(C₀₋₁₀ alkyl), -CON(C₀₋₁₀ alkyl)(C₀₋₁₀ alkyl), -OCOO(C₀₋₁₀ alkyl), phenyl, N-heteroaryl, O-heteroaryl, S-heteroaryl, alkenyl, alkynyl.

Preferably, the compound, or a pharmaceutically acceptable salt thereof, a stereoisomer thereof, a prodrug thereof, a solvate thereof, or a deuterated derivative thereof, has the following structure:

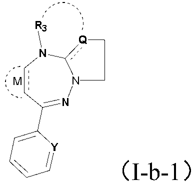

Wherein, Y is selected from N or -CF;
M is selected from none, or forms a fused ring with the adjacent carbon, together forming saturated or unsaturated cycloalkyl, saturated or unsaturated heterocyclyl, aryl or heteroaryl;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl,
N-heterocyclyl, O-heterocyclyl, S-heterocyclyl, -NHCO(C₀₋₁₀ alkyl); particularly, the H of above in saturated or unsaturated heterocyclyl, aryl or heteroaryl, which are optionally substituents selected from the group consisting of: halogen, -NO₂, -CN, -NHCO(C₀₋₁₀ alkyl), CF₃, straight-chain or branched-chain C₁₋₁₀ alkyl, -OC₀₋₁₀ alkyl;
Q is selected from N or 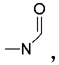 R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O, or S atom as ring atom;
When Q is N, R₃ and Q together with N and C atoms which they are attached to form five-membered heteroaryl or six-membered heteroaryl, which containing at least two heteroatoms, optionally, the five-membered aromatic heterocycle is imidazolyl; optionally, the six-membered aromatic heterocycle is pyridyl;
Particularly, Q is N, and R₃ together with the adjacent N atom of the triazine and Q which they are attached to form imidazole;
Particularly, when R₃ and Q do not form a ring, and Q is N or -NCO(C0-10 alkyl); When Q is N, it is connected to the parent nucleus via a double bond; when Q is -NCO(C0-10 alkyl), it is connected to the parent nucleus via a single bond;
Preferably, the compound, or a pharmaceutically acceptable salt thereof, a stereoisomer thereof, a prodrug thereof, a solvate thereof, or a deuterated derivative thereof, has the following structure: 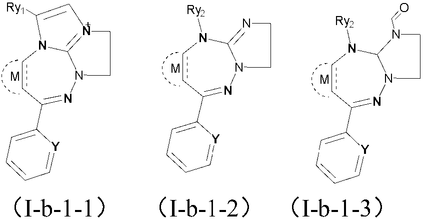
Wherein, Y is selected from N or -CF;
Wherein, Y is selected from N or -CF;
M is selected from none, or forms fused ring with the adjacent carbon, together forming a saturated or unsaturated cycloalkyl, saturated or unsaturated heterocyclyl, aryl or heteroaryl;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl); preferably, the H of above in saturated or unsaturated cycloalkyl, saturated or unsaturated heterocyclyl, aryl or heteroaryl, which are optionally substituents selected from the group consisting of: halogen, -NO₂, -CN, -CF₃, -NHCO(C₀₋₁₀ alkyl), straight-chain C₁₋₃ alkyl, -OC₀₋₁₀ alkyl;
R_{y1} is selected from -H, -CN, -CF₃, straight-chain or branched-chain C₁₋₄ alkyl, C₃₋₁₀ cycloalkyl; preferably, R_{y1} is selected from methyl;
R_{y2} is selected from -H, straight-chain or branched-chain C₁₋₃ alkyl, the hydrogen atom on said alkyl may be replaced by a substituent selected from the group consisting of: -N(C₁₋₃ alkyl)(C₁₋₃ alkyl), -COO(C₀₋₅ alkyl), 5-6 membered N-heterocycloalkyl;
Preferably, the compound, or a pharmaceutically acceptable salt thereof, a stereoisomer thereof, a prodrug thereof, a solvate thereof, or a deuterated derivative thereof, has the following structure: 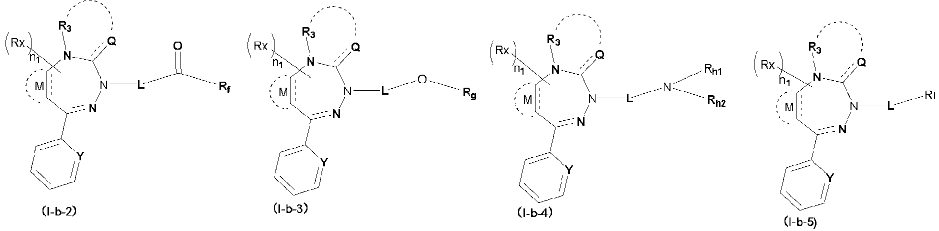 Wherein, Y is selected from N or -CF;
M is selected from none, or forms fused ring with the adjacent carbon, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl;
when M forms fused ring with the adjacent carbon, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
Q is selected from O, S-Rxx, N or 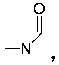 R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O, or S atom as ring atom;
When Q is N, R₃ and Q together with N and C atoms which they are attached to form five-membered heteroaryl or six-membered heteroaryl,which containing at least two heteroatoms;
Rxx is selected from H, straight-chain or branched-chain C₁₋₅ alkyl, said the H of alkyl which are optionally substituents selected from the group consisting of: halogen, nitro, cyano, -OC₀₋₁₀ alkyl;
L is none, or L is selected from C₂₋₈ alkenyl, C₁₋₈ alkylene, wherein, the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
R_{f} is selected from H, straight-chain or branched-chain C₁₋₁₀ alkyl, ORⱼ, Rⱼ is selected from straight-chain or branched-chain C₃₋₁₀ alkyl, C₃₋₁₀ cycloalkyl, 3-8 membered heterocyclyl, aryl, heteroaryl, wherein, the hydrogen atom on these groups can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, ethynyl, furthermore, said alkyl may be substituted by a substituent selected from the group consisting of: -CN, -OCH₂F, -OCHF₂, -OCF₃, -OC₀₋₁₀ alkyl, and C₃₋₄ cycloalkyl;
R_{g} is selected from H, straight-chain or branched-chain C₁₋₁₀ alkyl, -C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocycloalkyl), -CH₂CON(C₀₋₁₀ alkyl)(C₀₋₁₀ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OC₁₋₅ alkyl, -N(C₁₋₃ alkyl)(C₁₋₃ alkyl), C₂₋₈ alkenyl, imidazolyl, oxazolyl, thiazolyl, pyridinyl, pyrimidinyl, pheny; or R_{g} is selected from 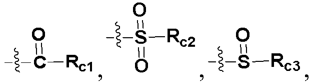 R_{c1}, R_{c2}, R_{c3} are independently selected from C₁₋₁₀ alkyl, -OC₀₋₅ alkyl, -O(C₃₋₆ cycloalkyl), -O(C₃₋₆ heterocycloalkyl), C₃₋₁₀ cycloalkyl, 3-6 membered heterocyclyl, -N(C₀₋₁₀ alkyl)(C₀₋₁₀ alkyl), alkenyl, alkynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl, ethenyl, furthermore, said alkyl is substituted with a substituent selected from the group consisting of: -CN, -OCF₃, -OC₀₋₁₀ alkyl, and C₃₋₄ cycloalkyl;
Rₕ₁ and Rₕ₂ are independently selected from H, straight-chain or branched-chain C₁₋₁₀ alkyl, -COO(C₀₋₁₀ alkyl), -CO(C₀₋₁₀ alkyl), 3-6 membered cycloalkyl, 3-6 membered heterocyclyl, aryl, or Rh¹ and Rh² together with the nitrogen atom which they are attached to form 3-6-membered heterocycloalkyl, the hydrogen atom on these groups can be substituted with the following substituents: halogen, -CN, -NO₂, straight-chain or branched-chain C₁₋₃ alkyl, -OC₁₋₃ alkyl, C₃₋₆ cycloalkyl, -(C₀₋₁₀ alkyl)COO(C₀₋₁₀ alkyl), -CO(C₀₋₁₀ alkyl), aryl, -N(C₁₋₃ alkyl)(C₁₋₃ alkyl), further, said alkyl is substituted with substituent selected from the group consisting of: -CN, -OCF₃, -OC₀₋₁₀, -CO(C₀₋₁₀ alkyl), C₃₋₄ cycloalkyl, aryl;
Rⱼ is selected from halogen, cyano, aryl, or heteroaryl, 3-6 membered cycloalkyl, 3-6 membered heterocyclyl; the hydrogen atom on these groups can be substituted with the following substituents: halogen, -CN, -NO₂, straight-chain or branched-chain C₁₋₁₀ alkyl, -OC₁₋₁₀ alkyl;
Preferably, the compound, or a pharmaceutically acceptable salt thereof, a stereoisomer thereof, a prodrug thereof, a solvate thereof, or a deuterated derivative thereof, has the following structure: 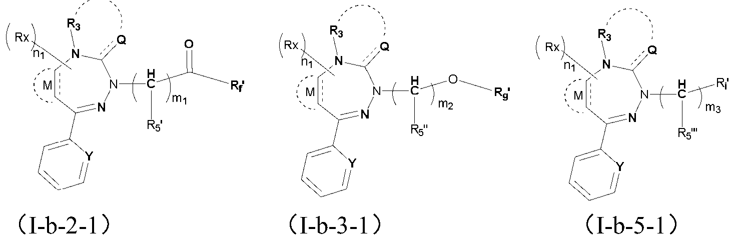
Wherein, Y is selected from N or -CF;
M is selected from none, or forms a fused ring with an adjacent carbon, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl; when M forms fused ring with the adjacent carbon, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
Q is selected from O, S-Rxx, N, R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8 membered heterocyclyl, -COO(C₀₋₁₀ alkyl), said the heterocyclyl contains at least one N, O, or S atom as ring atom;
When Q is N, R₃ and Q together with N and C atoms which they are attached to form five-membered heteroaryl or six-membered heteroaryl, which containing at least two heteroatoms;
Rxx is selected from H, straight-chain or branched-chain C₁₋₅ alkyl, said the H of alkyl which are optionally substituents selected from the group consisting of: halogen, nitro, cyano, -OC₀₋₁₀ alkyl;
m₁, m₂, m₃ are independently selected from integer from 0 to 5;
R₅', R₅", R₅‴ are independently selected from H, halogen, -CN, -CF₃, -C₁₋₁₀ alkyl, -CO(C₀₋₁₀ alkyl), -COO(C₀₋₁₀ alkyl);
R_{f}' is selected from -H, straight-chain or branched-chain C₁₋₁₀ alkyl, -N(C₀₋₁₀ alkyl)(C₀₋₁₀ alkyl), ORⱼ', Rⱼ' is selected from straight-chain or branched-chain C₁₋₁₀ alkyl, C₃₋₁₀ cycloalkyl, 3-8 membered heterocyclyl, aryl, heteroaryl, wherein, the hydrogen atom on these groups can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, and ethynyl, futher, said alkyl may be substituted by substituent selected from the group consisting of: -CN, -OCH₂F, -OCHF₂, -OCF₃, -OC₀₋₁₀ alkyl, C₃₋₄ cycloalkyl;
Preferably, R_{f}' is selected from -H, straight-chain or branched-chain C₁₋₆ alkyl, -N(C₁₋₆ alkyl)(C₁₋₆ alkyl), ORⱼ', Rⱼ' is selected from straight-chain or branched-chain C₁₋₆ alkyl, C₃₋₅ cycloalkyl, 3-5 membered heterocyclyl containing N, O and/or S atoms, 3-5-membered heteroaryl group containing N, O and/or S atoms, wherein, the hydrogen atom on these groups can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, and ethynyl, futher, said alkyl may be substituted by substituent selected from the group consisting of: -CN, -OCH₂F, -OCHF₂, -OCF₃, -OC₀₋₁₀ alkyl, C₃₋₄ cycloalkyl;
R_{g}' is selected from -H, straight-chain or branched-chain C₁₋₁₀ alkyl, -C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocyclyl), -CH₂CON(C₀₋₁₀ alkyl)(C₀₋₁₀ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OC₁₋₅ alkyl, -N(C₁₋₃ alkyl)(C₁₋₃ alkyl), C₂₋₈ alkenyl, imidazolyl, oxazolyl, thiophene, pyridinyl, pyrimidinyl, phenyl; or R_{g}' is selected from 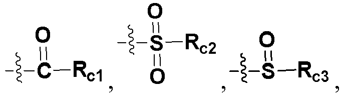 R_{c1}, R_{c2}, R_{c3} are independently selected from straight-chain or branched-chain C₁₋₁₀ alkyl, -OC₀₋₅ alkyl, -O(C₃₋₆ cycloalkyl), -O(C₃₋₆ heterocyclyl), C₃₋₁₀ cycloalkyl, 3-6 membered heterocyclyl, -N(C₀₋₁₀ alkyl)(C₀₋₁₀ alkyl), ethenyl, ethynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl, ethenyl, aid alkyl may be substituted by substituent selected from the group consisting of: -CN, -OCF₃, -OC₀₋₁₀ alkyl, C₃₋₄ cycloalkyl;
Preferably, R_{g}' is selected from -H, straight-chain or branched-chain C₁₋₆ alkyl, -C₃₋₆ cycloalkyl, the hydrogen atom on these groups can be substituted with the following substituents: -F, -Cl, -CN, -NO₂, straight-chain or branched-chain C₁₋₆ alkyl, 3-6 membered heterocyclyl, -OC₁₋₅ alkyl, -N(C₁₋₃ alkyl)(C₁₋₃ alkyl), C₂₋₈ alkenyl, imidazolyl, oxazolyl, thiophene, pyridinyl, pyrimidinyl, phenyl; or R_{g}' is selected from 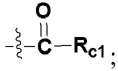 R_{c1} is selected from straight-chain or branched-chain C₁₋₆ alkyl, -OC₁₋₅ alkyl, -O(C₃₋₆ cycloalkyl), -O(C₃₋₆ heterocyclyl), C₃₋₆ cycloalkyl, 3-6 membered heterocyclyl, -N(C₁₋₆ alkyl)(C₁₋₆ alkyl), ethenyl, ethynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl,ethenyl, said alkyl may be substituted by substituent selected from the group consisting of: -CN, -OCF₃, -OC₀₋₁₀ alkyl, C₃₋₄ cycloalkyl;
Rᵢ' is selected from halogen, cyano, phenyl, 5-6 membered heteroaryl containing one or more heteroatoms selected from N, O and/or S, 3-6 membered cycloalkyl or 3-6 membered heterocyclyl; the hydrogen atom on these groups can be substituted with the following substituents: halogen, -CN, -NO₂, straight-chain or branched-chain C₁₋₆ alkyl, -OC₁₋₆ alkyl; preferably, Rᵢ' is selected from halogen, cyano, phenyl, pyrrolyl, thienyl, furyl, imidazolyl, thiazolyl, triazolyl, isoxazolyl, oxazolyl, pyridyl, pyrimidinyl, pyrazinyl, triazinyl, cyclopropyl, cyclobutyl, cyclopentyl, cyclohexyl, or the following groups: 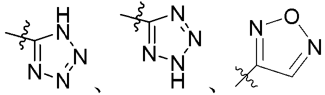
the hydrogen atom on Rᵢ' can be substituted with the following substituents: halogen, -CN, -NO₂, straight-chain or branched-chain C₁₋₆ alkyl, -OC₁₋₆ alkyl;
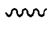 means substitution position.

Preferably, the compound, or a pharmaceutically acceptable salt thereof, a stereoisomer thereof, a prodrug thereof, a solvate thereof, or a deuterated derivative thereof, has the following structure:

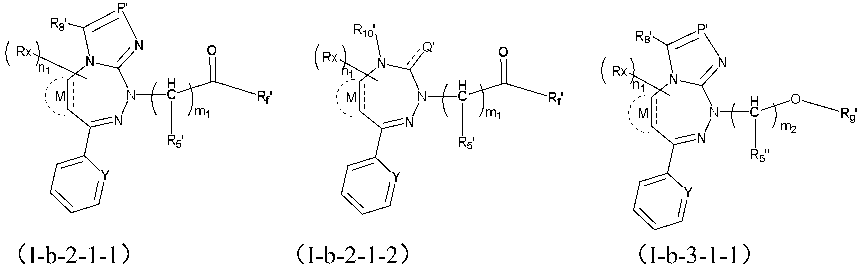

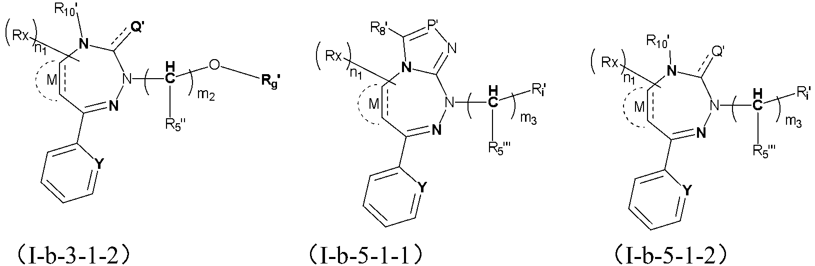

Wherein, Y is selected from N or -CF; P' is selected from N or CH;
M is selected from none, or form fused ring with the adjacent carbon, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl; when M form fused ring with the adjacent carbon, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
R₈' is selected from -H, halogen, -CN, -NO₂, -CF₃, straight-chain or branched-chain C₁₋₄ alkyl, C₃₋₁₀ cycloalkyl;
R₁₀' is selected from -H, straight-chain or branched-chain C₁₋₃ alkyl, the hydrogen atom on these groups can be substituted with the following substituents: -N(C₁₋₃ alkyl)(C₁₋₃ alkyl), -CO(C₀₋₁₀ alkyl), -COO(C₀₋₁₀ alkyl), 5-6 membered heterocyclyl;
Q' is selected from =O or S-Rxx; Rxx is selected from H, straight-chain or branched-chain C₁₋₃ alkyl, said the H of alkyl which are optionally substituents selected from the group consisting of: halogen, nitro, cyano, -OC₀₋₁₀ alkyl;
m₁, m₂, m₃ are independently selected from integer from 0 to 4;
R₅', R₅", R₅‴ are independently selected from H, halogen, -CN, -CF₃, -C₁₋₁₀ alkyl, -CO(C₀₋₁₀ alkyl), -COO(C₀₋₁₀ alkyl);
R_{f}' is selected from -H, straight-chain or branched-chain C₁₋₁₀ alkyl, -N(C₀₋₁₀ alkyl)(C₀₋₁₀ alkyl), ORⱼ', Rⱼ' is selected from straight-chain or branched-chain C₁₋₁₀ alkyl, C₃₋₁₀ cycloalkyl, 3-8 membered heterocyclyl, aryl, heteroaryl, wherein, the hydrogen atom on these groups can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, ethynyl, further, the aforementioned alkyl moieties are replaceable with substituent selected from the group consisting of: -CN, -OCH₂F, -OCHF₂, -OCF₃, -OC₀₋₁₀ alky, C₃₋₄ cycloalkyl;
R_{g}' is selected from -H, straight-chain or branched-chain C₁₋₁₀ alkyl, -C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocyclyl), -CH₂CON(C₀₋₁₀ alkyl)(C₀₋₁₀ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OC₁₋₅ alkyl, -N(C₁₋₃ alkyl)(C₁₋₃ alkyl), C₂₋₈ alkeny, imidazolyl, oxazolyl, thiazolyl, pyridinyl, pyrimidinyl, phenyl; or, R_{g}' is selected from 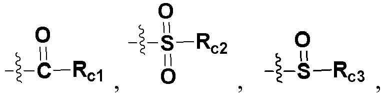 R_{c1}, R_{c2}, R_{c3} are independently selected from straight-chain or branched-chain C₁₋₁₀ alkyl, -OC₀₋₅ alkyl, -O(C₃₋₆ cycloalkyl), -O(C₃₋₆ heterocyclyl), C₃₋₁₀ cycloalkyl, 3-6 membered heterocyclyl, -N(C₀₋₁₀ alkyl)(C₀₋₁₀ alkyl), alkenyl, alkynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl, ethenyl, the aforementioned alkyl moieties are replaceable with a substituent selected from the group consisting of: -CN, -OCF₃, -OC₀₋₁₀ alkyl, C₃₋₄ cycloalkyl;
Rᵢ' is selected from halogen, cyano, phenyl, 5-6 membered heteroaryl containing N, O and/or S, 3-6 membered cycloalkyl or 3-6 membered heterocyclyl; the hydrogen atom on these groups can be substituted with the following substituents: halogen, -CN, -NO₂, straight-chain or branched-chain C₁₋₆ alkyl, -OC₁₋₆ alkyl.

Said Y is selected from N or -CF; preferably, Y is selected from N.

Said P' is selected from N or CH; preferably, P' is selected fromCH.

Preferably, said R₈' is selected from -H, halogen, -CN, -NO₂, -CF₃, straight-chain or branched-chain C₁₋₃ alkyl, C₃₋₆ cycloalkyl; further preferably, said R₈' is selected from -H, methyl.

Said R₁₀' is selected from -H, straight-chain or branched-chain C₁₋₃ alkyl, the hydrogen atom on alkyl can be substituted with the following substituents: -N(C₁₋₃ alkyl)(C₁₋₃ alkyl), -CO(C₁₋₃ alkyl), -COO(C₁₋₃ alkyl), piperazinyl, N-methylpiperazinyl or N-ethylpiperazinyl;
Q' is selected from =O or -S-Rxx; Rxx is selected from H or methyl;
Said m₁, m₂, m₃ are independently selected from integer from 0 to 4; preferably, m₁, m₂, m₃ are independently selected from integer from 0 to 2, in some specific embodiments of the present invention, m1, m2, and m3 are independently selected from 0, 1, 2, 3, or 4.

R₅', R₅", R₅‴ are independently selected from H, halogen, -CN, -CF₃, -C₁₋₆ alkyl, -CO(C₀₋₁₀ alkyl), -COO(C₀₋₁₀ alkyl); preferably, R₅', R₅", R₅‴ are independently selected from H, halogen, -CN, -CF₃, -C₁₋₃ alkyl, -CO(C₁₋₃ alkyl), -COO(C₁₋₃ alkyl).

Preferably, R_{f}' is selected from -H, straight-chain or branched-chain C₁₋₆ alkyl, -N(C₀₋₆ alkyl)(C₀₋₆ alkyl), ORⱼ', Rⱼ' is selected from straight-chain or branched-chain C₁₋₆ alkyl, cyclopropyl, cyclobutyl, cyclopentyl, cyclohexyl, phenyl, thiophenyl, furanyl, pyrrolyl, pyridinyl or the following group(s): 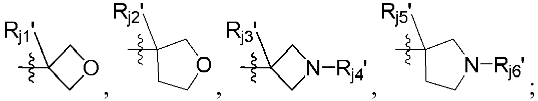
wherein, Rⱼ₁', Rⱼ₂', Rⱼ₃', Rⱼ₄', Rⱼ₅', Rⱼ₆' are independently selected from H, halogen, -CN, straight-chain or branched-chain C₁₋₆ alkyl, straight-chain or branched-chain C₂₋₆ alkenyl, straight-chain or branched-chain -OC₁₋₆ alkyl, C₃₋₆ cycloalkyl, 3-6 membered heterocyclyl, -CO(C₀₋₁₀ alkyl), -OCO(C₀₋₁₀ alkyl), ethynyl;
wherein, the hydrogen atom on R_{f}' can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, ethynyl, further, the aforementioned alkyl moieties are replaceable with a substituent selected from the group consisting of: -CN, -OCH₂F, -OCHF₂, -OCF₃, -OC₀₋₁₀ alky, C₃₋₄ cycloalkyl;
R_{g}' is selected from -H, straight-chain or branched-chain C₁₋₆ alkyl, -C₃₋₆ cycloalkyl, -CH₂CO(C₁₋₆ alkyl), -CH₂COO(C₀₋₆ alkyl), -CH₂COO(C₃₋₆ cycloalkyl), -CH₂COO(C₃₋₆ heterocyclyl), -CH₂CON(C₀₋₆ alkyl)(C₀₋₆ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OH, -OC₁₋₅ alkyl, -N(C₁₋₃ alkyl)(C₁₋₃ alkyl), C₂₋₈ alkenyl, imidazolyl, oxazolyl, thiazolyl, pyridinyl, pyrimidinyl, phenyl; the hydrogen atom on these groups can be substituted with the following substituents: -F, -Cl, -CN, -NO₂, straight-chain or branched-chain C₁₋₁₀ alkyl, 3-6 membered heterocyclyl, -OH, -OC₁₋₅ alkyl, -N(C₁₋₃ alkyl)(C₁₋₃ alkyl), C₂₋₈ alkenyl, imidazolyl, oxazolyl, thiazolyl, pyridinyl, pyrimidinyl, phenyl; or R_{g}' is selected from 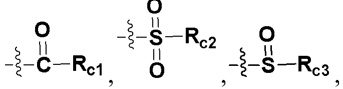 R_{c1}, R_{c2}, R_{c3} are independently selected from are independently selected from C₁₋₁₀ alkyl, -OC₀₋₅ alkyl, -O(C₃₋₆ cycloalkyl), -O(C₃₋₆ heterocyclyl), C₃₋₁₀ cycloalkyl, 3-6 membered heterocyclyl, -N(C₀₋₁₀ alkyl)(C₀₋₁₀ alkyl), alkenyl, alkynyl, aryl, heteroaryl; preferably, R_{c1}, R_{c2}, R_{c3} are independently selected from are independently selected from C₁₋₆ alkyl, -OC₁₋₆ alkyl, -O(C₃₋₆ cycloalkyl), -O(C₃₋₆ heterocyclyl), C₃₋₆ cycloalkyl, 3-6 membered heterocyclyl, -N(C₀₋₆ alkyl)(C₀₋₆ alkyl), alkenyl, alkynyl, aryl, heteroaryl; further, R_{c1}, R_{c2}, R_{c3} are independently selected from are independently selected from C₁₋₃ alkyl, -OC₁₋₃ alkyl, -O(C₃₋₆ cycloalkyl), -O(C₃₋₆ heterocyclyl), cyclopropyl, cyclobutyl, cyclopentyl, cyclohexyl, 3-membered N/O heterocycloalkyl, 4-membered N/O heterocycloalkyl, 5-membered N/O heterocycloalkyl, 6-membered N/O heterocycloalkyl, -N(C1-3 alkyl)2, ethylene, ethynyl, phenyl, pyridyl, thienyl, furyl, pyrrolyl, imidazolyl, thiazolyl, oxazolyl.

Preferably, the hydrogen atom on these groups can be substituted with the following substituents: halogen, -CN, -CH3, -C2H5, -OCH3, -N(C1-3 alkyl)(C1-3 alkyl), -C₃₋₁₀ cycloalkyl, 3-6 membered heterocyclyl, ethenyl, the aforementioned alkyl moieties are replaceable with substituent selected from the group consisting of: -CN, -OCF₃, -OC₀₋₁₀ alkyl, C₃₋₄ cycloalkyl;

Preferably, Rᵢ' is selected from halogen, cyano, phenyl, pyrrolyl, thiophenyl, furanyl, imidazolyl, oxazolyl, triazolyl, isoxazolyl, oxazolyl, pyridinyl, pyrimidinyl, pyrazinyl, triazinyl, cyclopropyl, cyclobutyl, cyclopentyl, cyclohexyl or the following group: 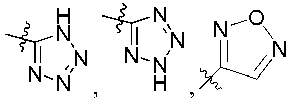
the aforementioned Rᵢ' are replaceable with substituent selected from the group consisting of: halogen, -CN, -NO₂, straight-chain or branched-chain C₁₋₆ alkyl, -OC₁₋₆ alkyl;
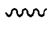 means substitution position.

Preferably, M together the adjacent carbon atom which they are attached to form the moiety selected from: 3-8 membered saturated or unsaturated carbocycle, 3-8 membered saturated or unsaturated heterocycle, benzene, 5-6 membered monocyclic heteroaromatic ring, heteroaromatic ring formed by benzene fused with 1 or 2 five-membered or six-membered monocyclic heteroaryl groups, or heteroaromatic ring formed by 2 or 3 fused five-membered and/or six-membered monocyclic heteroaryl groups; wherein hydrogen atom on the aforementioned groups may be substituted by substituent selected from: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀-₁₀ alkyl), -OC₀₋₁₀ alkyl, C₃₋₁₀ cycloalkyl, N-heterocycloalkyl, O-heterocycloalkyl, S-heterocycloalkyl, -NHC(O)(C₀-₁₀ alkyl).

Preferably, hydrogen atom on the aforementioned groups may be substituted by substituent selected from: halogen, -CN, -NO₂, -NHC(O)(C₀-₁₀ alkyl), -CF₃, straight-chain or branched-chain C₁₋₃ alkyl, -OC₀-₁₀ alkyl.

Further preferably, the hydrogen atom on the aforementioned groups may be substituted by substituent selected from: halogen, -CN, -NO₂, -NHC(O)(C₁-₆ alkyl), -CF₃, straight-chain or branched-chain C₁₋₃ alkyl, -OC₁-₆ alkyl.

Preferably, the heteroatom in said 3-8 membered saturated or unsaturated heterocycle is selected from one or more of N, O, and S.

Preferably, M together the adjacent carbon atom which they are attached to form the moiety selected from: 3-6 membered saturated or unsaturated carbocycle, 3-6 membered saturated or unsaturated heterocycle, benzene, pyrrole, thiophene, furan, pyridine, pyrimidine, pyrazine, triazine, imidazole, oxazole, thiazole, pyrazole, benzofuran, benzoxazole, benzimidazole, benzothiophene, benzothiazole, indole, or imidazopyridine; wherein hydrogen atom on the aforementioned groups may be substituted by a substituent selected from the group consisting of: halogen, -NO₂, -CN, -CF₃, C₂-₈ alkenyl, C₂-₈ alkynyl, straight-chain or branched-chain C₁-₁₀ alkyl, -N(C₀-₁₀ alkyl)(C₀-₁₀ alkyl), -OC₀-₁₀ alkyl, C₃-₁₀ cycloalkyl, N-heterocycloalkyl, O-heterocycloalkyl, S-heterocycloalkyl, -NHC(O)(C₀-₁₀ alkyl).

Preferably, hydrogen atom on the aforementioned group may be substituted selected from the group consisting of: halogen, -CN, -NO₂, -NHC(O)(C₀-₁₀ alkyl), -CF₃, straight-chain or branched-chain C₁-₃ alkyl, -OC₀-₁₀ alkyl.

Preferably, said 3-6 membered saturated or unsaturated carbocycle is selected from saturated or unsaturated cyclopropyl, cyclobutyl, cyclopentyl, or cyclohexyl group.

Preferably, said 3-6 membered saturated or unsaturated heterocycle is saturated or unsaturated 3 membered, 4 membered, 5 membered or 6 membered heterocycle containing one or more heteroatoms selected from N, O, and S; Preferably, said heterocycle is monocyclic heterocycle.

When M forms the heteroaromatic ring with the adjacent carbon atom, it can be fused to the parent nucleus through either the phenyl side or the heterocyclic group side of the heteroaromatic ring.

Preferably, M forms any one of the following structures with the adjacent carbon: 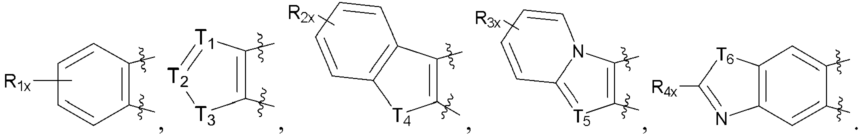
R₁ₓ, R₂ₓ, R₃ₓ, R₄ₓ are independently selected from: H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl); further preferably, R₁ₓ, R₂ₓ, R₃ₓ, R₄ₓ are independently selected from: H, halogen, -NO₂, -CN, -NHCO(C₀₋₁₀ alkyl), CF₃, straight-chain or branched-chain C₁₋₃ alkyl, -OC₀₋₁₀ alkyl; further preferably, R₁ₓ, R₂ₓ, R₃ₓ, R₄ₓ are independently selected from: H, halogen, -NO₂, -CN, -NHCO(C₀₋₁₀ alkyl), CF₃, straight-chain C₁₋₃ alkyl, -OC₀₋₁₀ alkyl;
T₁, T₂, T₃, T₄, T₅ are independently selected from: C-R₂₄, N, O or S;
T₆ is selected from C, N, O or S;
R₂₄ is selected from H, halogen, -CN, -NO₂, -NHCO(C₀₋₁₀ alkyl), CF₃, straight-chain or branched-chain C₁₋₃ alkyl, -OC₀₋₁₀ alkyl; preferably, R₂₄ is selected from H, halogen, -CN, -NO₂, -NHCO(C₀₋₆ alkyl), CF₃, straight-chain or branched-chain C₁₋₃ alkyl, -OC₀₋₆ alkyl.
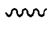 means substitution position.

Further preferably, said M and the adjacent carbon atom together form structure selected from: 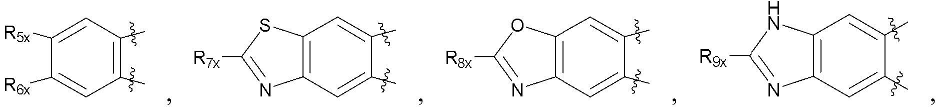 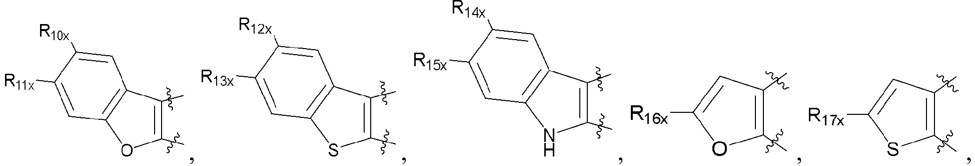 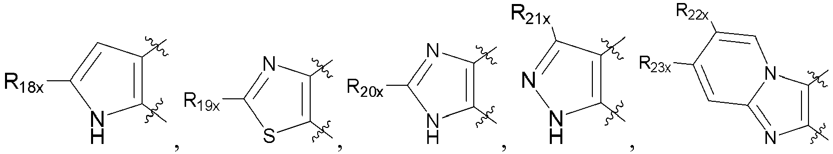
R₅ₓ-R₂₃ₓ are independently selected from:H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N heterocyclyl, O heterocyclyl, S heterocyclyl, -NHCO(C₀₋₁₀ alkyl); preferably, R₅ₓ-R₂₃ₓ are independently selected from:H, halogen, straight-chain or branched-chain C₁₋₃ alkyl, straight-chain or branched-chain -OC₀₋₃, -CF₃, -CN, -NO₂;
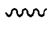 means substitution position.

Further, the structure of said compound is selected from:

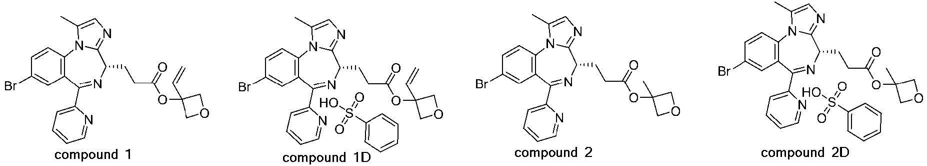

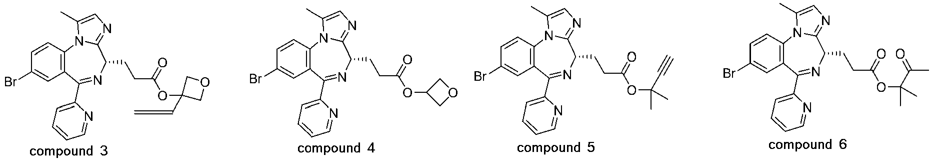

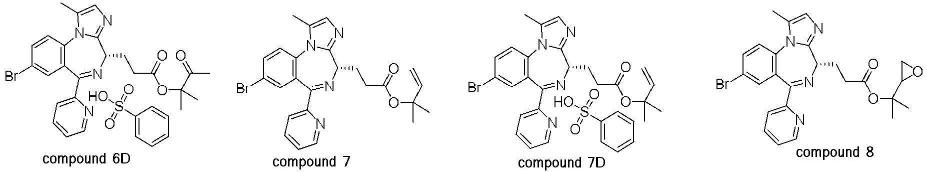

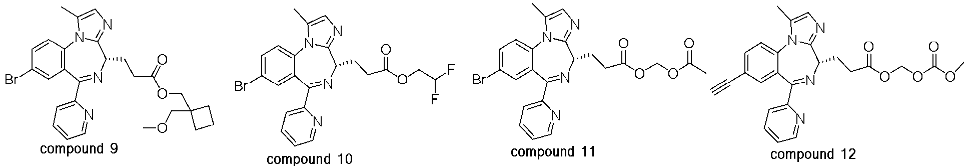

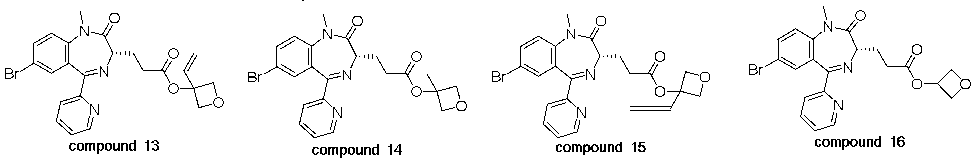

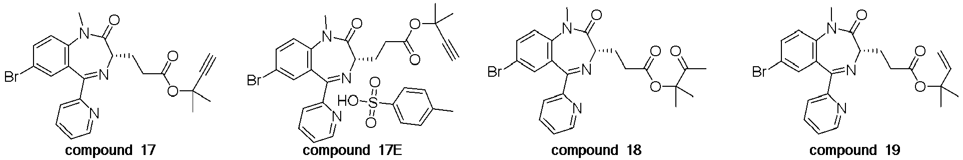

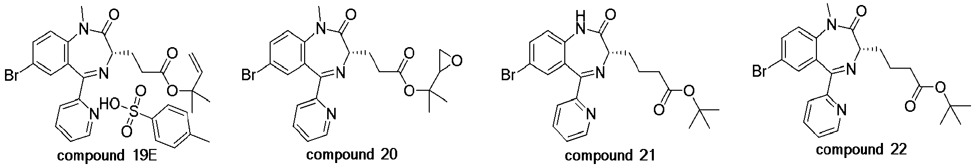

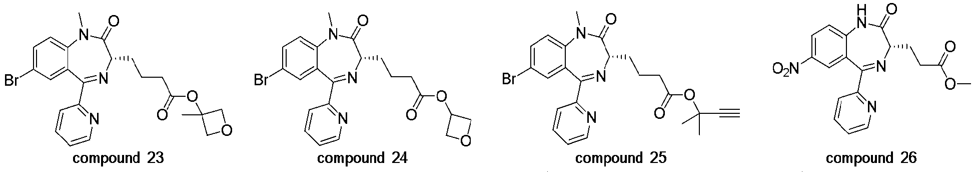

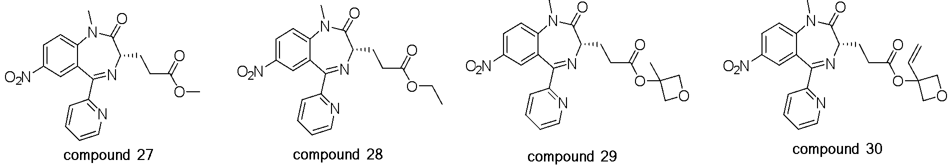

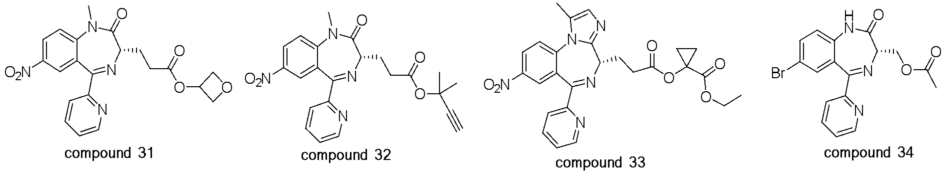

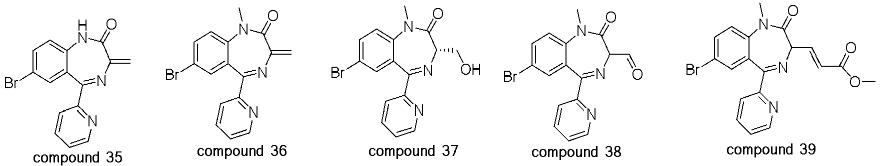

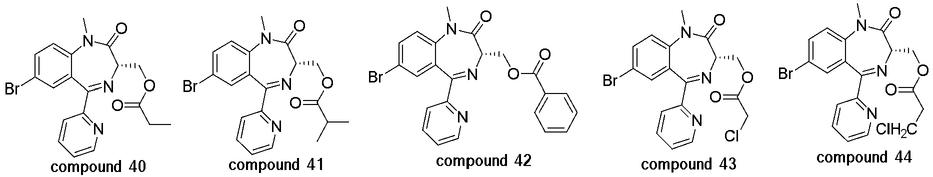

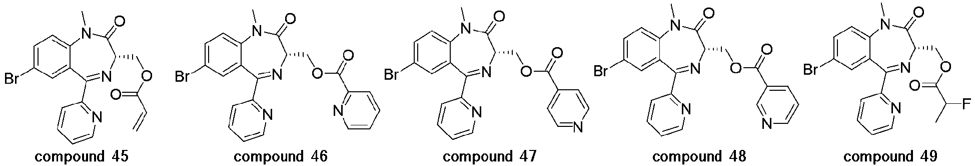

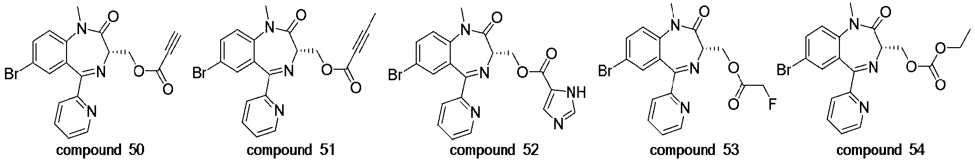

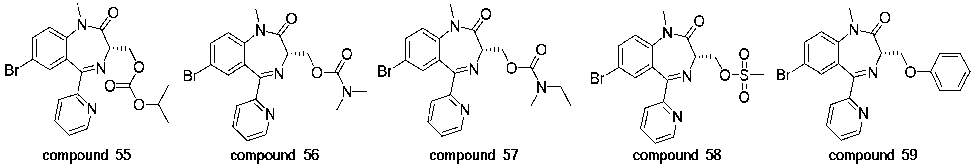

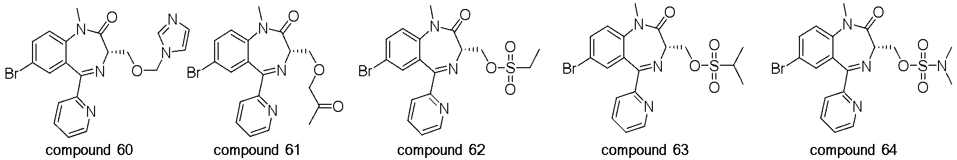

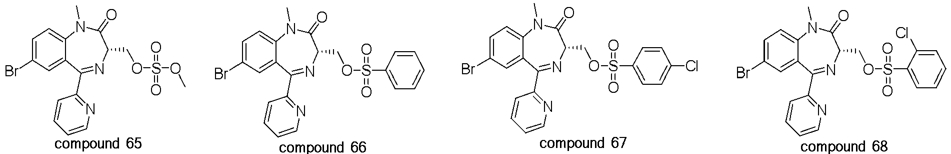

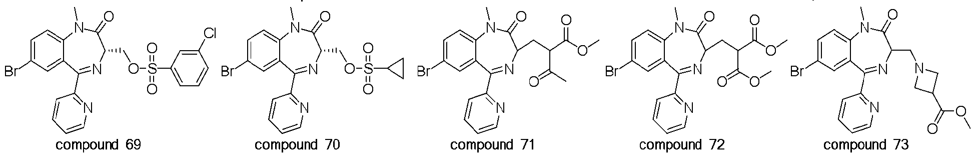

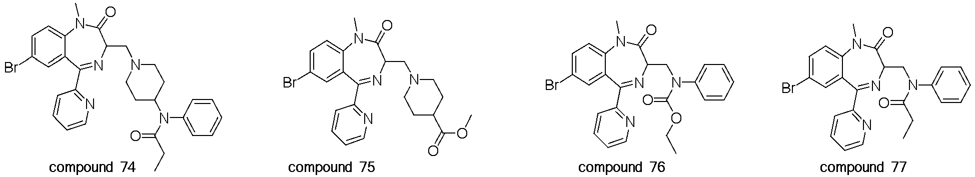

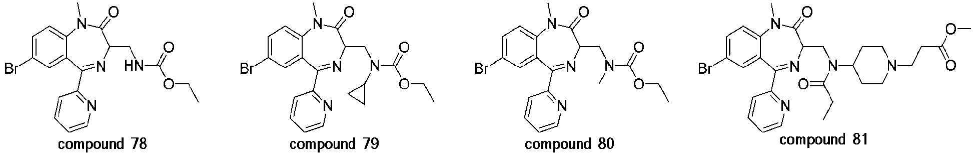

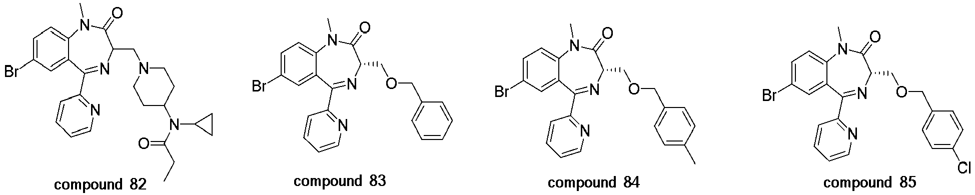

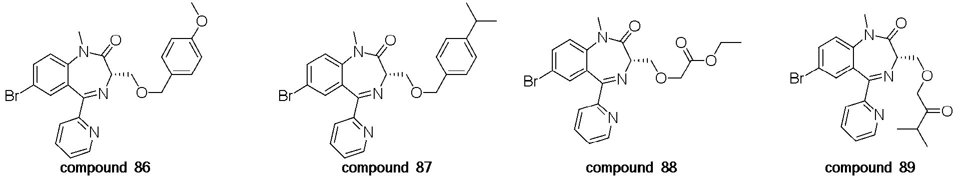

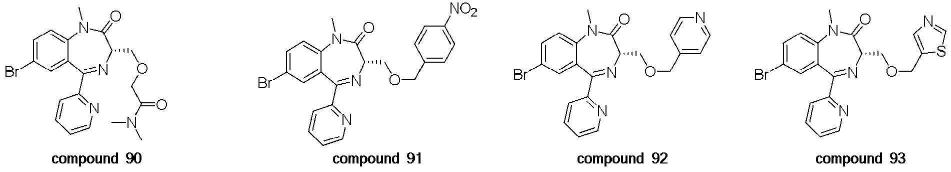

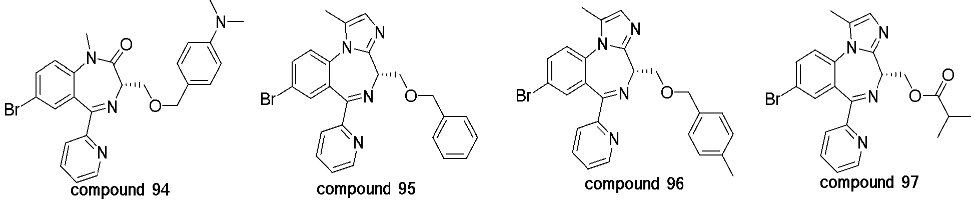

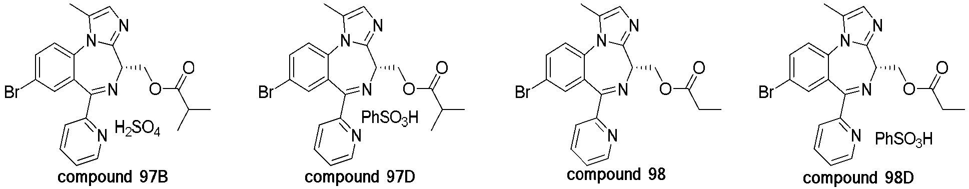

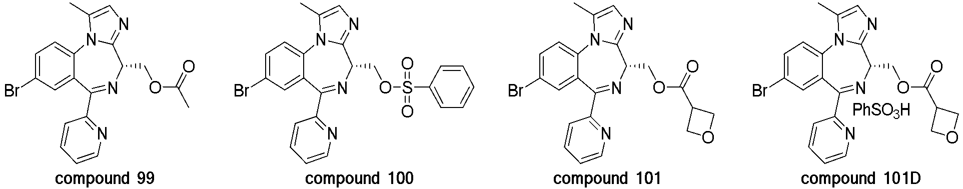

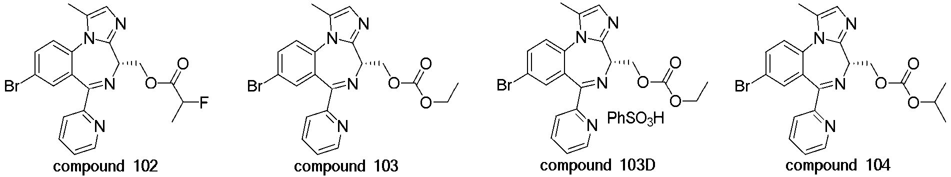

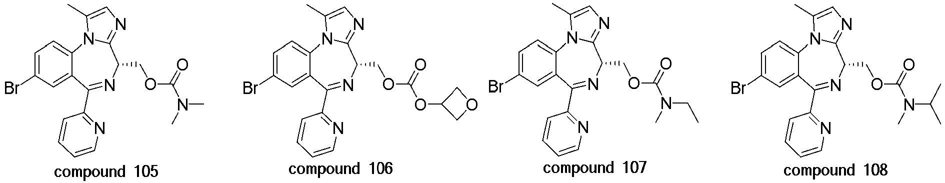

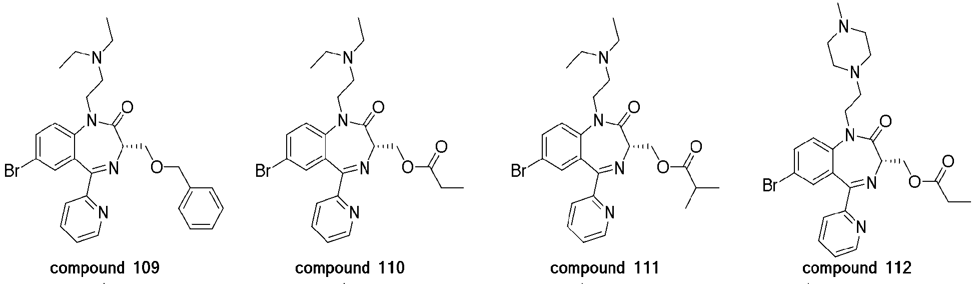

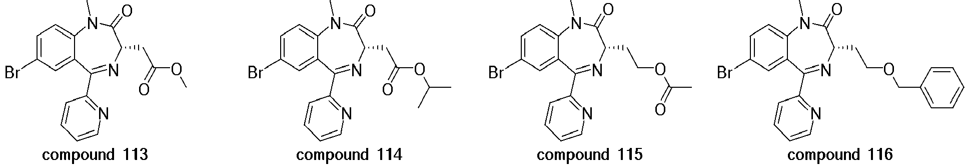

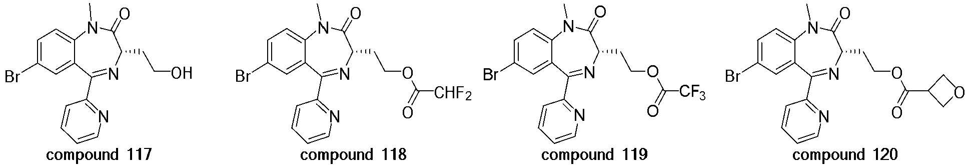

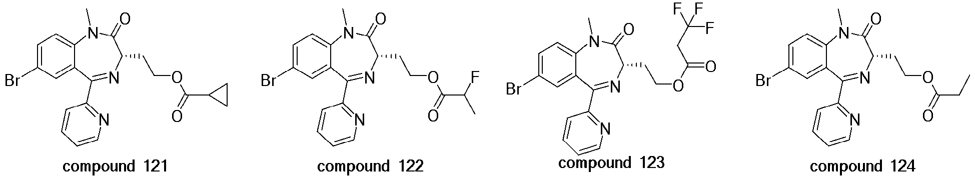

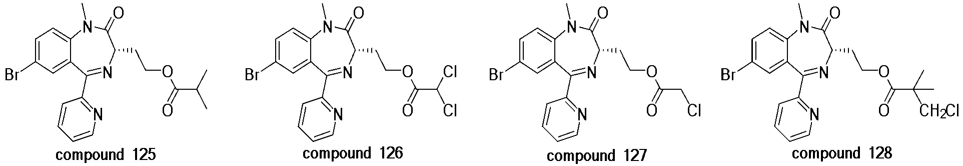

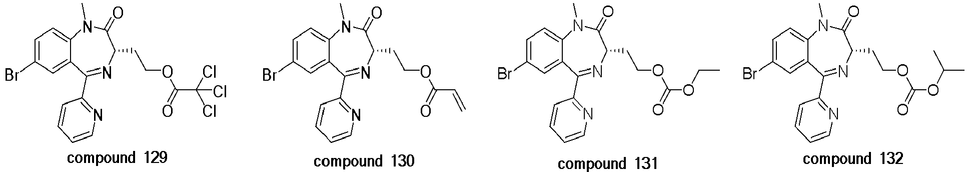

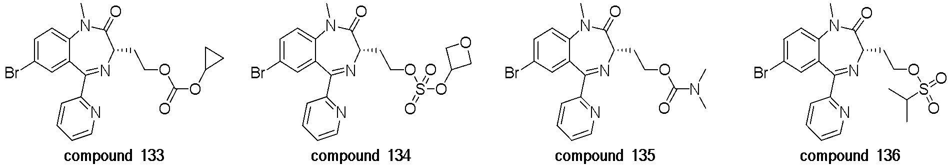

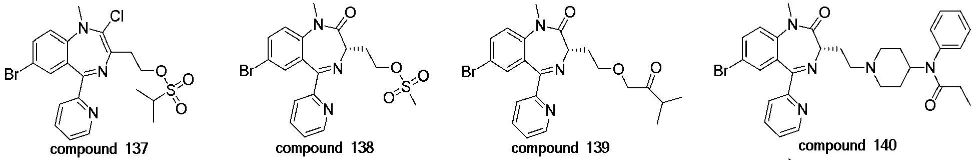

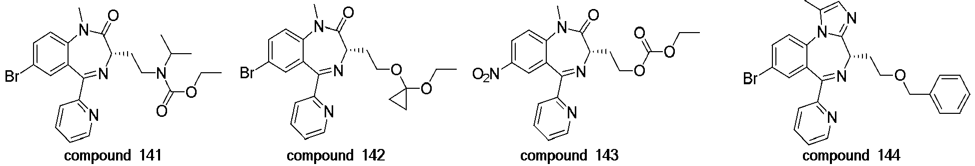

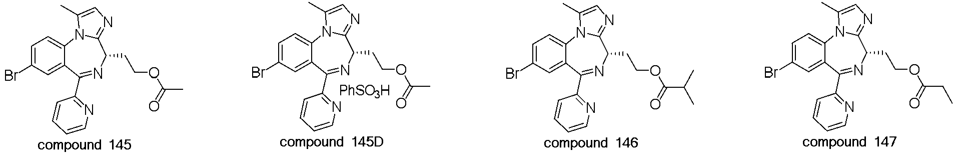

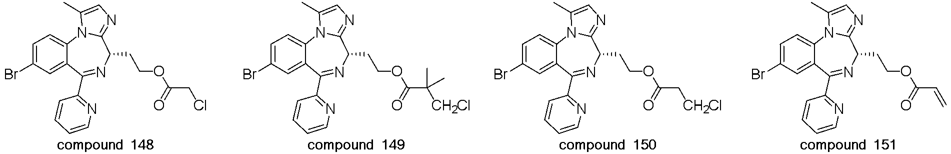

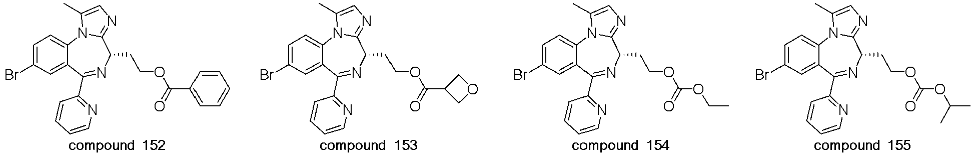

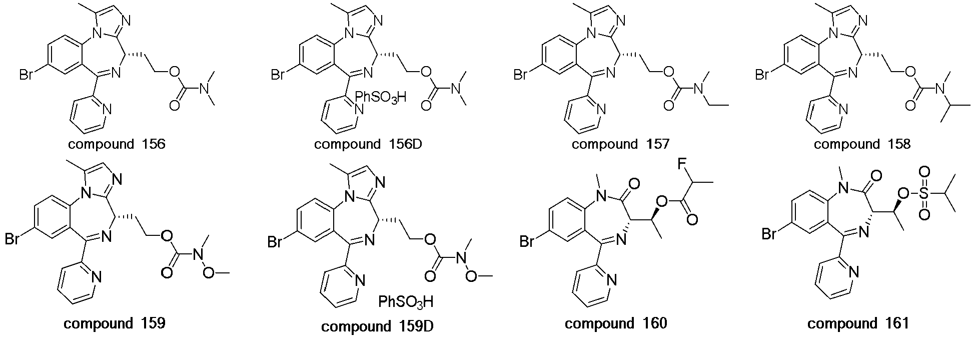

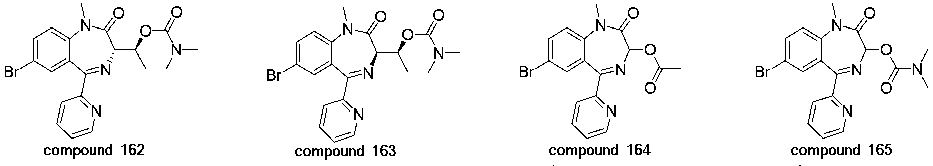

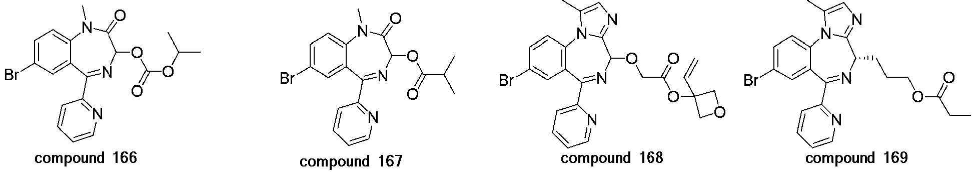

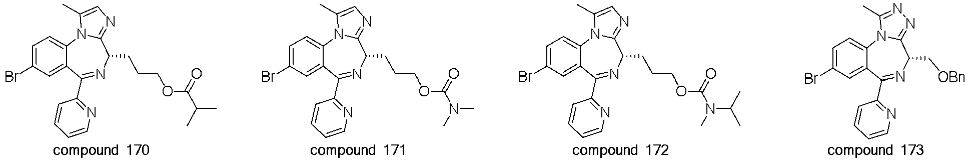

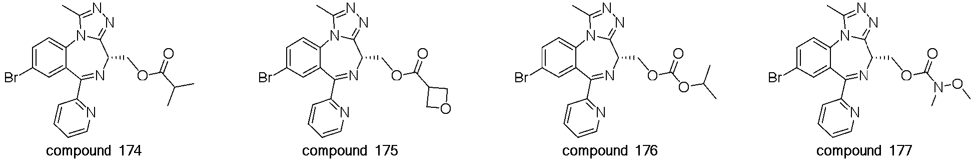

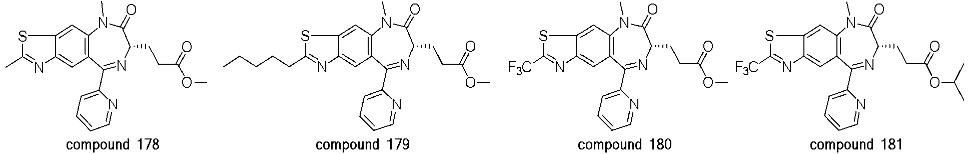

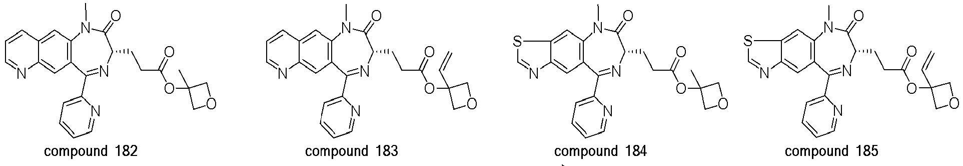

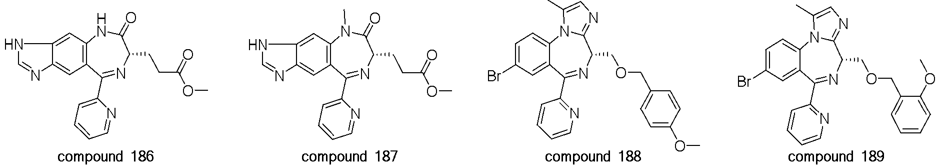

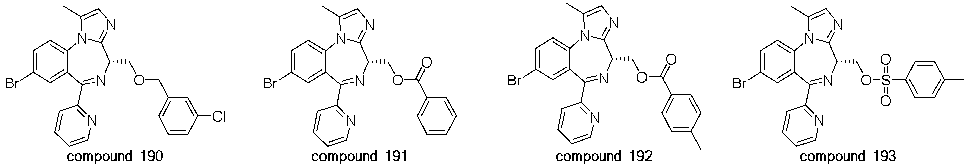

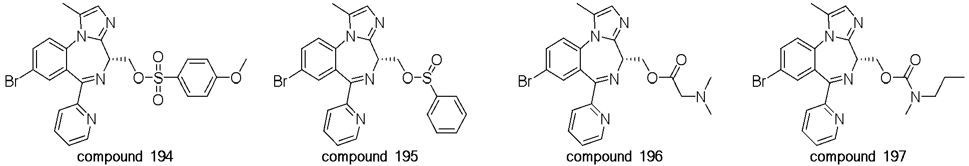

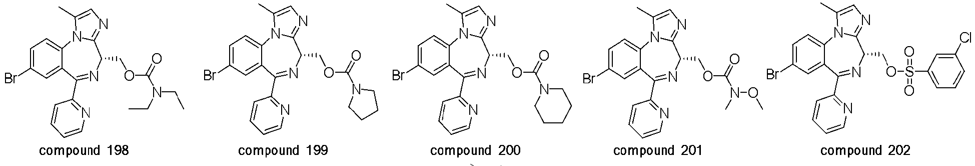

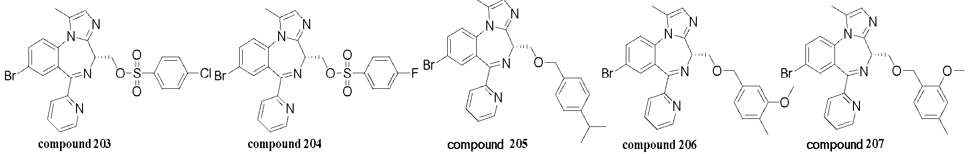

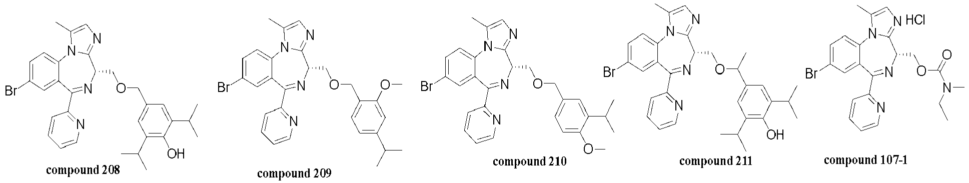

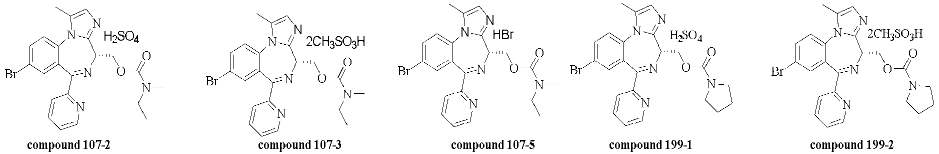

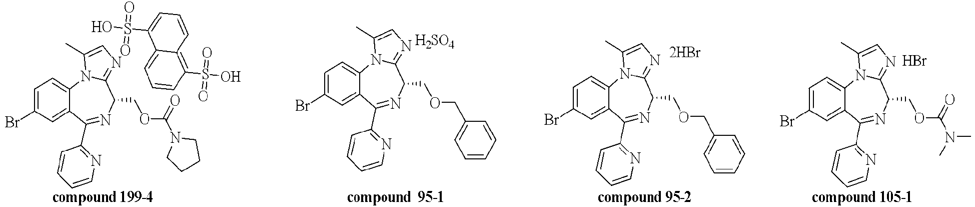

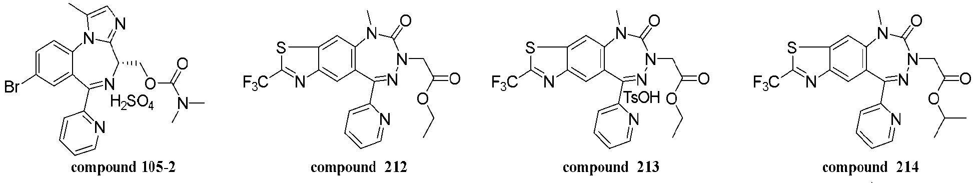

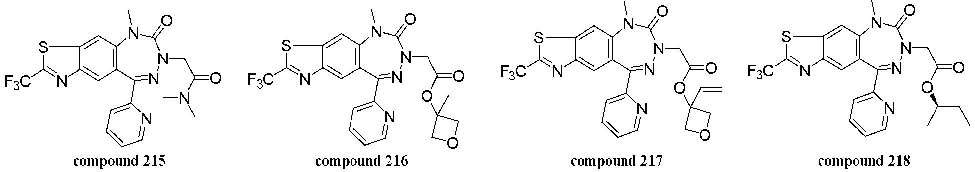

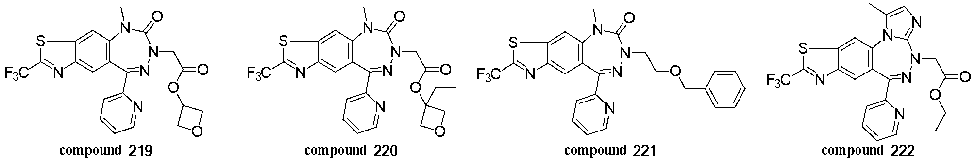

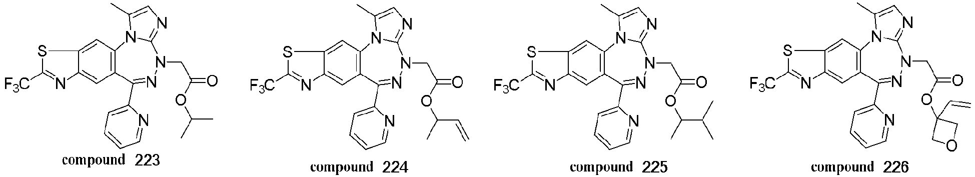

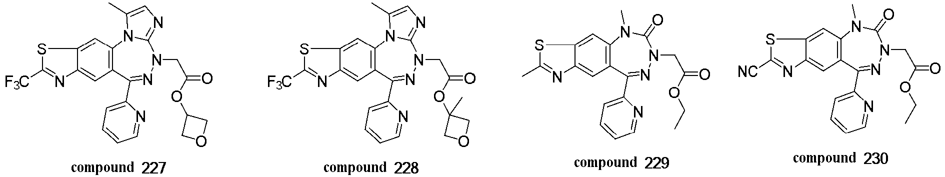

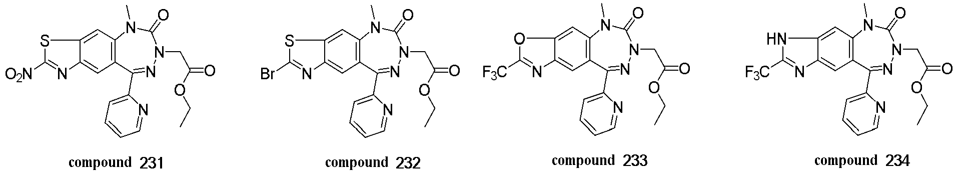

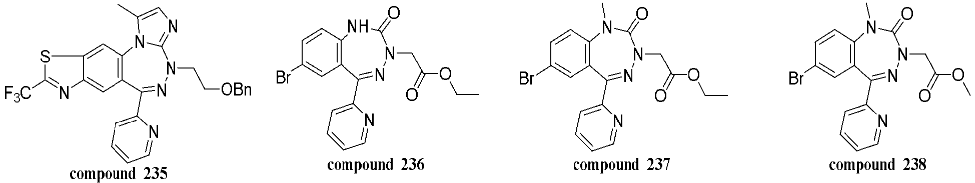

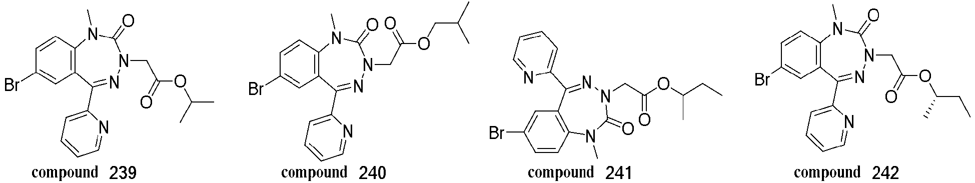

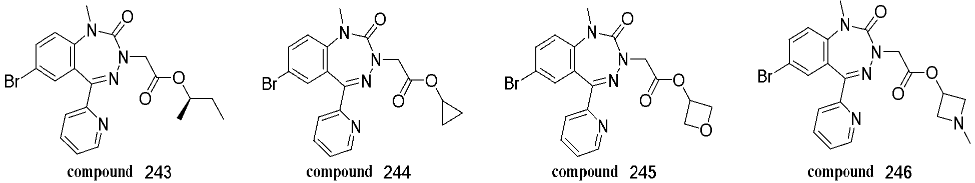

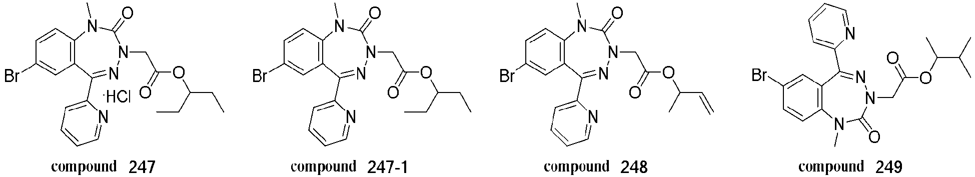

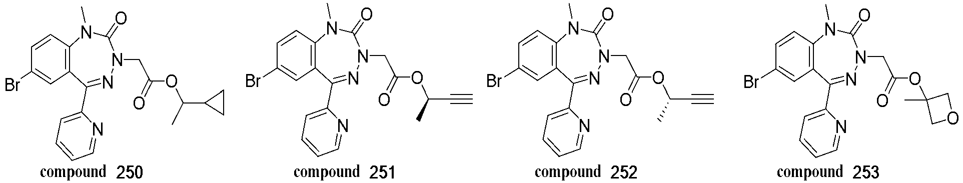

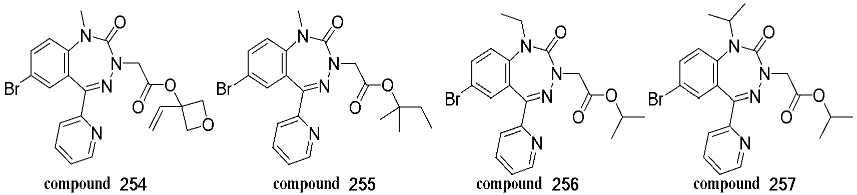

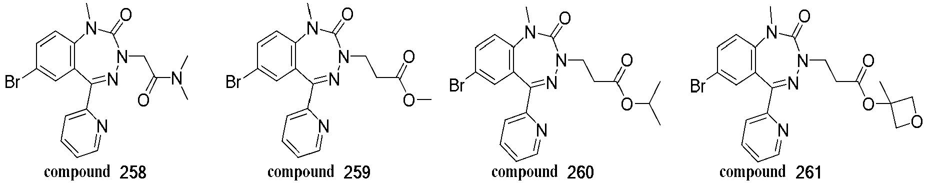

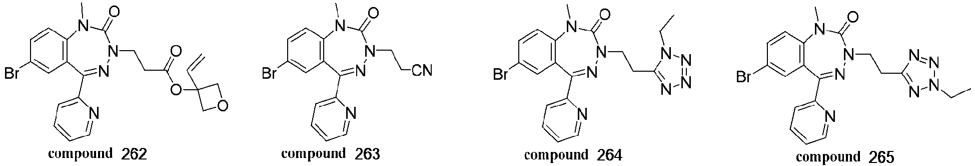

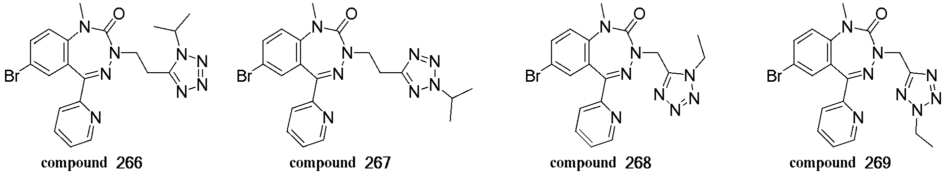

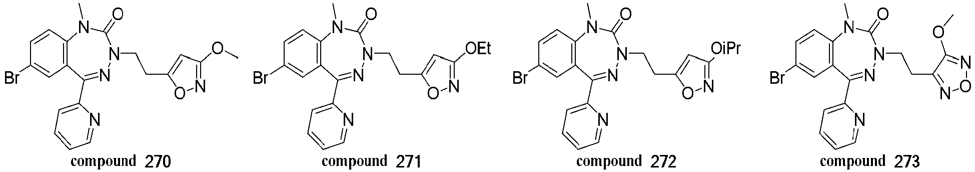

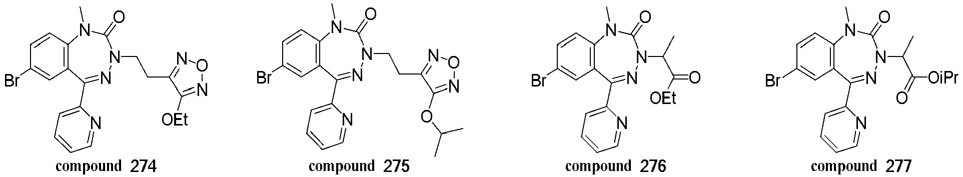

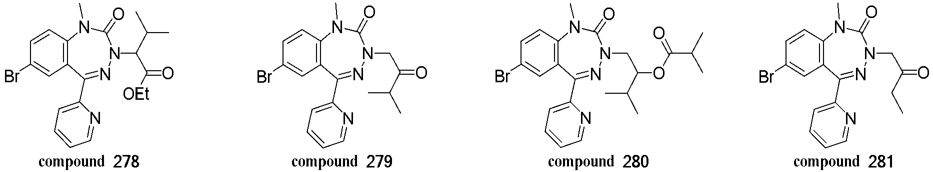

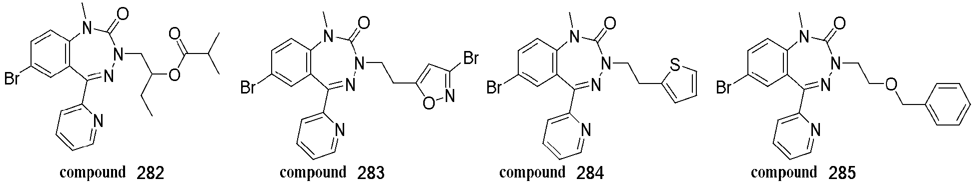

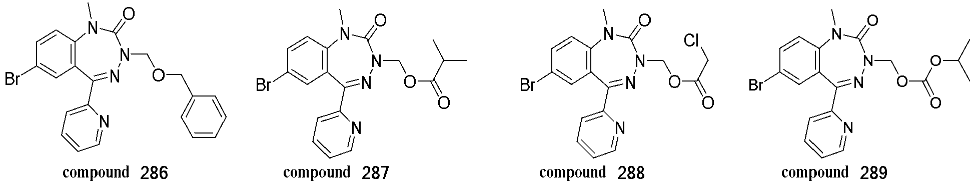

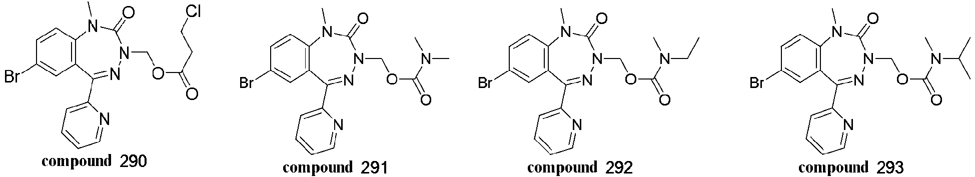

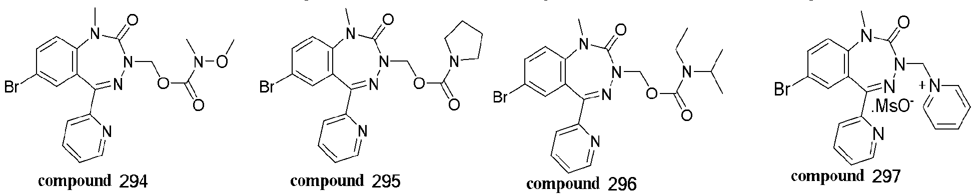

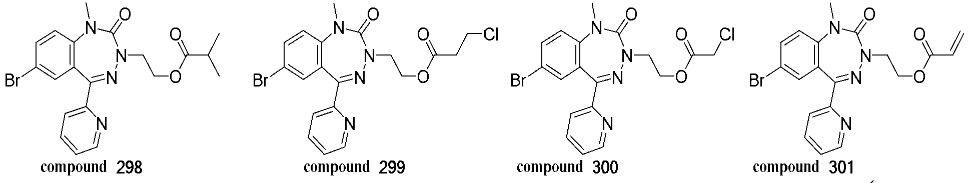

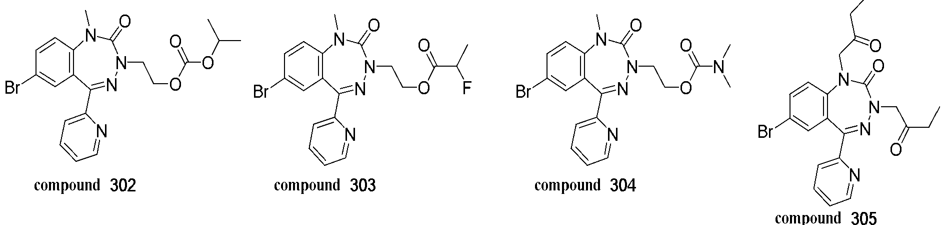

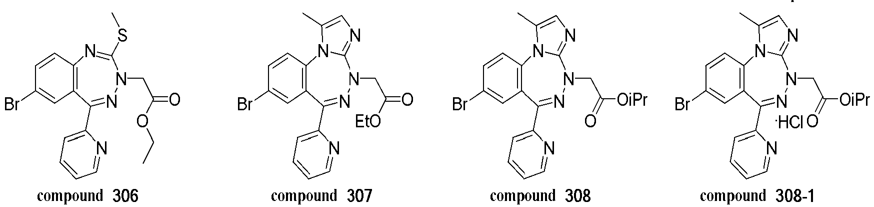

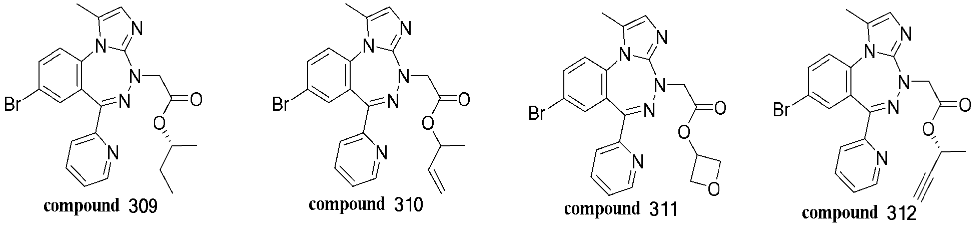

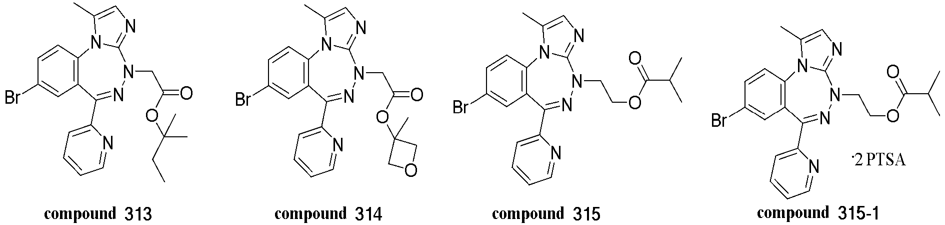

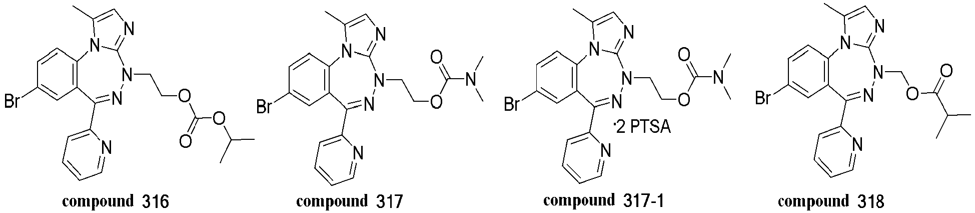

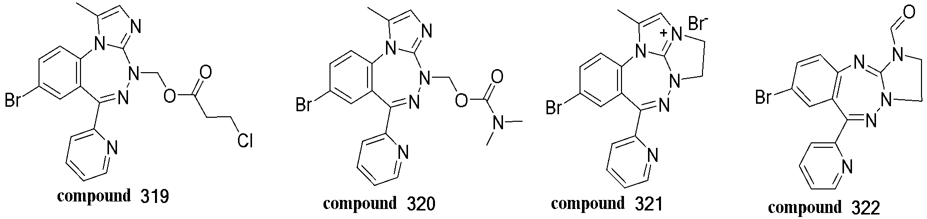

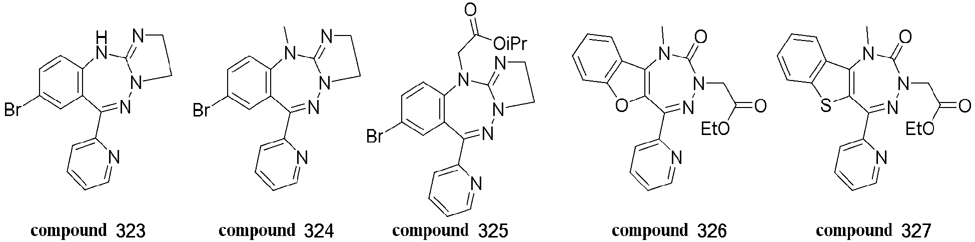

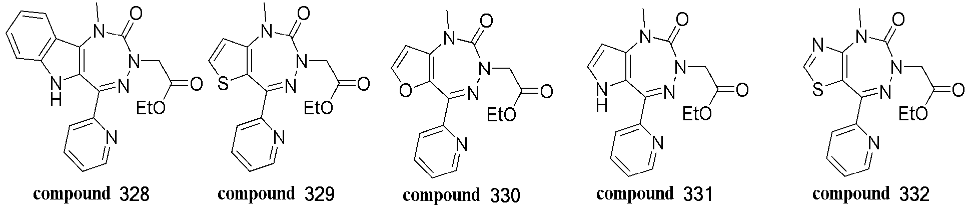

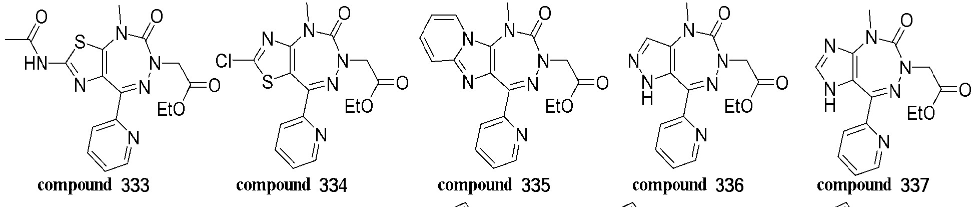

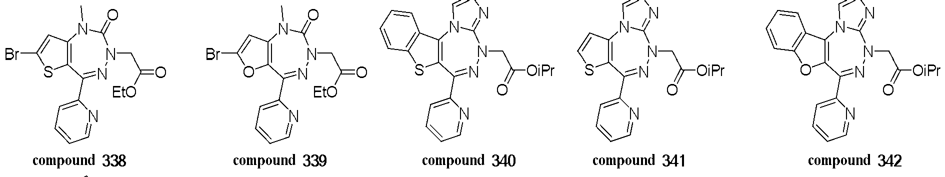

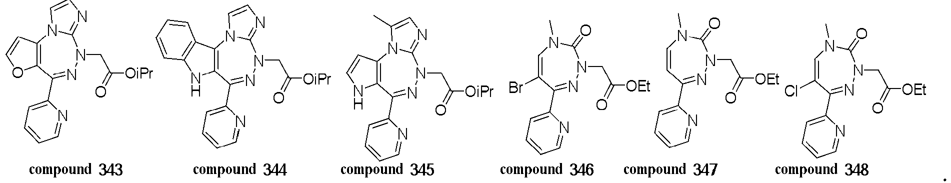

In a second aspect, a pharmaceutical composition comprising the compound provided by the present invention, as well as its pharmaceutically acceptable salts, stereoisomers, prodrugs, solvates and deuterated compounds.

Preferably, the pharmaceutical composition further comprises pharmaceutically acceptable excipients, including but not limited to carriers, diluents, binders, lubricants and wetting agents.

In embodiments, the pharmaceutical composition can be used alone or in combination with other active ingredients having anesthetic and/or analgesic effects.

The pharmaceutical composition can be administered to humans and/or animals.

The pharmaceutical composition is suitable for enteral or parenteral administration, such as via intravenous, intramuscular, intradermal and subcutaneous routes. Therefore, the pharmaceutical composition further includes antioxidants, buffers, bacteriostats, solutes that render the preparation isotonic with the recipient's blood, suspending agents, solubilizers, thickeners, stabilizers and preservatives.

The pharmaceutical composition of the present invention can be formulated into the following forms of pharmaceutical preparations: syrups, elixirs, suspensions, powders, granules, tablets, capsules, lozenges, aqueous solutions, creams, ointments, lotions, gels, emulsions, etc. The pharmaceutical preparation is preferably a unit dosage form, containing a therapeutically effective amount of the compound of Formula I, as well as its pharmaceutically acceptable salts, stereoisomers, prodrugs, solvates and deuterated compounds. The unit dosage form can be a capsule, a tablet or any dosage form; further, it can also be a packaged preparation, such as tablets, capsules and powders packaged in vials or ampoules. The amount of the active component in the unit dosage form can vary or be adjusted from 0.001 mg to 1000 mg, depending on the specific application and potency of the active component. If necessary, it may also contain other suitable active ingredients.

In the third aspect, the use of the compound provided by the present invention, its pharmaceutically acceptable salts, stereoisomers, prodrugs, solvates, deuterated compounds, or the aforementioned pharmaceutical composition in the preparation of drugs having analgesic effects, and/or anesthetic, sedative, hypnotic effects, and/or capable of controlling status epilepticus.

The pharmaceutically acceptable salts according to the present invention are acetate, adipate, aspartate, benzoate, besylate, bicarbonate, carbonate, bisulfate, sulfate, borate, camphorsulfonate, citrate, cyclamate, edisylate, esylate, formate, fumarate, glucoheptonate, gluconate, glucuronate, hexafluorophosphate, hydrochloride, hydrobromide, hydroiodide, isethionate, lactate, malate, maleate, malonate, mesylate, methyl sulfate, naphthoate, napsylate, nicotinate, nitrate, orotate, oxalate, palmitate, pamoate, phosphate, hydrogen phosphate, dihydrogen phosphate, pyroglutamate, saccharate, stearate, succinate, tannate, tartrate, tosylate, trifluoroacetate, xinafoate, methanesulfonate, or p-toluenesulfonate.

Preferably, the pharmaceutically acceptable salt according to the present invention is selected from one of besylate, tosylate, isethionate, sulfate, hydrochloride, mesylate, hydrobromide, and napsylate.

As used herein, the term "C₀₋₁₀ alkyl" means that C₀ alkyl refers to H. Therefore, C₀₋₁₀ alkyl includes H, C₁ alkyl, C₂ alkyl, C₃ alkyl, C₄ alkyl, C₅ alkyl, C₆ alkyl, C₇ alkyl, C₈ alkyl, C₉ alkyl, and C₁₀ alkyl.

As used herein, the term "C₁₋₁₀ straight-chain/branched-chain alkyl" includes methyl, ethyl, straight-chain/branched-chain C₃ alkyl, straight-chain/branched-chain C₄ alkyl, straight-chain/branched-chain C₅ alkyl, straight-chain/branched-chain C₆ alkyl, straight-chain/branched-chain C₇ alkyl, straight-chain/branched-chain C₈ alkyl, straight-chain/branched-chain C₉ alkyl, and straight-chain/branched-chain C₁₀ alkyl.

As used herein, the term "C₃₋₁₀ cycloalkyl" includes C₃ cycloalkyl, C₄ cycloalkyl, C₅ cycloalkyl, C₆ cycloalkyl, C₇ cycloalkyl, C₈ cycloalkyl, C₉ cycloalkyl and C₁₀ cycloalkyl.

As used herein, the term "halogen" includes fluorine, chlorine, bromine, and iodine.

As used herein, the term "heterocycloalkyl" refers to a cycloalkyl containing heteroatoms, where the heteroatoms include N, O, and S. The heterocycloalkyl contains at least one N, O, or S as a ring atom, including those with one N, O, or S as a ring atom, two or more N, O, or S as ring atoms, or a combination of N and O, N and S, O and S, or N, O, and S as ring atoms.

As used herein, the term "aryl" refers to phenyl, benzyl, and other aromatic compounds with aromaticity, including but not limited to aromatic compounds formed by the fusion of 2 to 4 phenyl groups.

As used herein, the term "heteroaryl" refers to aromatic heterocyclic compounds containing one or more heteroatoms selected from N, O, and S, including but not limited to monocyclic aromatic heterocyclic compounds, aromatic heterocyclic compounds formed by the fusion of multiple monocyclic aromatic heterocyclic compounds, and aromatic heterocyclic compounds formed by the fusion of one or more phenyl groups with one or more monocyclic aromatic heterocyclic compounds.

For the structure " 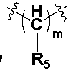 " described in the present invention, when m ≥ 2, each R₅ substituent on the alkylene group is independently substituted, and the R₅ substituents may be the same or different.

### EXEMPLIFICATION

The technical solutions in the embodiments of the present invention will be clearly and completely described below. Obviously, the described embodiments are only part of the embodiments of the present invention, not all of them. Based on the embodiments in the present invention, all other embodiments obtained by those of ordinary skill in the art without creative work shall fall within the protection scope of the present invention.

The raw materials and equipment used in the specific embodiments of the present invention are all known products, which can be obtained by purchasing commercial products.

The structures of the compounds are determined by nuclear magnetic resonance (NMR) and/or mass spectrometry (MS). NMR chemical shifts (δ) are given in units of 10⁻⁶ (ppm). NMR measurements are performed using a Bruker Avance III 400 NMR spectrometer, with deuterated dimethyl sulfoxide (d₆-DMSO) or deuterated methanol (CD₃OD) as the solvent and tetramethylsilane (TMS) as the internal standard.

LCMS measurements are carried out using an Agilent LCMS 1260-6110 (ESI) system, with a column of Waters X-Bridge C18 (50 mm × 4.6 mm × 3.5 µm). Column temperature: 40 °C; flow rate: 2.0 mL/min; mobile phase: gradient elution from 95% [water + 0.05% TFA] and 5% [CH₃CN + 0.05% TFA] to 0% [water + 0.05% TFA] and 100% [CH₃CN + 0.05% TFA] within 3 minutes, maintaining this condition for 1 minute, then gradient eluting back to 95% [water + 0.05% TFA] and 5% [CH₃CN + 0.05% TFA] within 0.05 minutes, and maintaining this condition for another 0.7 minutes.

### 1) Medicinal materials and reagents

Thin-layer chromatography (TLC) silica gel plates used are HSGF254 silica gel plates from Yantai Xinnuo Chemical Co., Ltd., with a thickness of 1 mm.Products from Yantai Jiangyou Silica Gel Development Co., Ltd. are used for thin-layer chromatography (TLC), with a specification of 0.2±0.03 mm.Column chromatography generally uses 100-200 mesh or 200-300 mesh silica gel as the carrier, which is purchased from Rushan Sun Desiccant Co., Ltd. (Weihai, Shandong).

### 2) Main instruments Sartorius

BSA124S electronic balance (Sartorius Scientific Instruments Beijing Co., Ltd.), 98-2 magnetic stirrer (Shanghai Sile Instrument Co., Ltd.), MS-H-PRO+ digital-controlled heating magnetic stirrer (Dragon Laboratory Instruments Beijing Co., Ltd.), TDGC2-1 contact voltage regulator (Zhejiang Tengen Electric Co., Ltd.), WMNK-01 temperature controller (Shanghai Lulin Electric Co., Ltd.), ZF-I three-purpose ultraviolet instrument (Shanghai Anting Electronic Instrument Factory), R-201 rotary evaporator (Shanghai Shenshun Biotechnology Co., Ltd.), W201D constant temperature water bath (Shanghai Shenshun Biotechnology Co., Ltd.), SHB-III circulating water vacuum pump (Zhengzhou Huicheng Technology & Trade Co., Ltd.), SHB-B95 mobile water pump (Zhengzhou Huicheng Technology & Trade Co., Ltd.), DLSB-5/20 °C low-temperature cooling circulating pump (Gongyi Yuhua Instrument Co., Ltd.), 2XZ-2 rotary vane vacuum pump (Linhai Yonghao Vacuum Equipment Co., Ltd.).

### Example 1 Preparation of Compound 1 and compound 1D

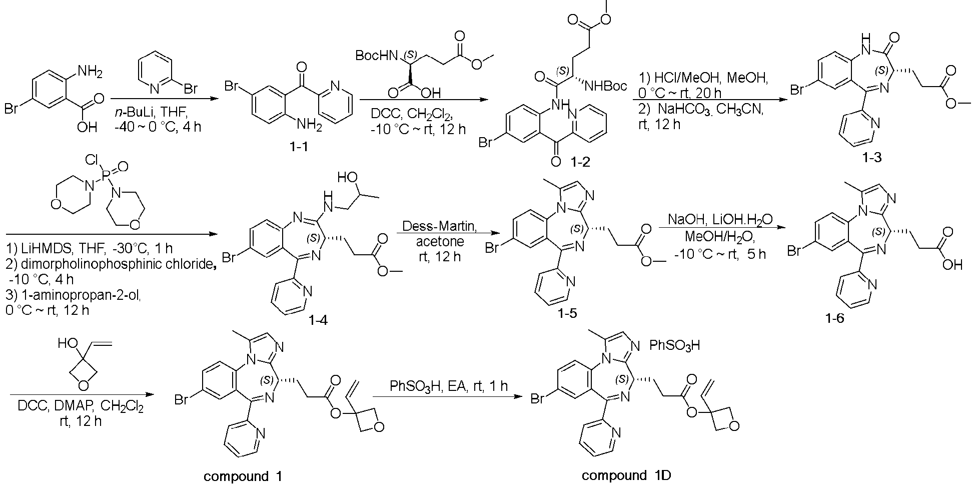

### 1. Preparation of Compound 1-1

At -40°C, n-Butyllithium (305 mL, 2.5 mol/L in hexane, 762.5 mmol) was added to tetrahydrofuran (280 mL), and 2-bromopyridine (132.6 g, 839.2 mmol) was slowly added dropwise into the mixture, then the mixture was stirred for 1 hour. A solution of 2-amino-5-bromobenzoic acid (41.2 g, 190.7 mmol) in THF (280 mL) was added dropwise to the reaction system. After the dropwise addition was completed, the temperature was allowed to rise naturally to 0 °C, and the mixture was stirred at 0°C for 3 hours. After the reaction was monitored by TLC until completion, saturated aqueous ammonium chloride solution (67 mL) and water (318 mL) were added to the reaction system. The mixture was extracted with EtOAc (3 × 200 mL), the combined organic layers were washed with saturated brine and dried over anhydrous Na₂SO₄, filtered by suction, and concentrated under reduced pressure to obtain a crude product, the crude product was purified by silica gel column chromatography (ethyl acetate / petroleum ether (v/v) = 1/100 - 1/5), with TLC (ethyl acetate / petroleum ether (v/v) = 1/3) monitoring, and collecting the fraction with Rf = 0.5 - 0.6, to give **compound 1-1** (34.7 g, yield 65.7%) as yellow solid. ESI[M + H]+ = 277.1.

### 2. Preparation of Compound 1-2

Compound 1-1 (20.0 g, 72.2 mmol) and Boc-L-glutamic acid 5-methyl ester (20.8 g, 79.6 mmol) were dissolved in dichloromethane (100 mL). A solution of DCC (16.4 g, 79.5 mmol) in dichloromethane (40 mL) was added dropwise to the mixture at -10 °C. After the dropwise addition was completed, the mixture was stirred for 12 hours at room temperature. After the reaction was monitored by TLC until completion, methyl tert-butyl ether (140 mL) was added to the reaction system, and the mixture was stirred for 5 minutes. the mixture was filtered and concentrated under reduced pressure to give a crude product. the crude product was purified by silica gel column chromatography (ethyl acetate / petroleum ether (v/v) = 1/20 - 1/2), with TLC (ethyl acetate / petroleum ether (v/v) = 1/3) monitoring, and collecting the fraction with Rf = 0.2 ~ 0.3, to give compound **1-2** (35.5 g, yield 94.5%) as white solid. ESI[M + H]⁺ = 520.1.

### 3. Preparation of Compound 1-3

At 0°C, a solution of hydrochloric acid in methanol (170.7 mL, 4 mol/L, 682.8 mmol) was added dropwise to a solution of **1-2** (35.5 g, 68.2 mmol) in methanol (300 mL). After the addition was complete, the mixture was allowed to warm up to room temperature and stirred for 20 hours. After the reaction was monitored by TLC until completion, the reaction solution was added dropwise to a solution of sodium bicarbonate (172.1 g, 2.05 mol) in acetonitrile (300 mL), and the mixture was stirred at room temperature for 12 hours. After the reaction was monitored by TLC until completion. The mixture was filtered and concentrated under reduced pressure to obtain a crude product. The crude product was purified by flash silica gel chromatography (ethyl acetate/petroleum ether (v/v) = 1/10 - 1/1). TLC (ethyl acetate/petroleum ether (v/v) = 1/1) monitoring was performed, and the fractions with Rf =0.2-0.3 were collected to yield compound **1-3** as white solid (24.1 g, yield 87.8%). ESI[M + H]⁺ = 402.1.

### 4. Preparation of Compound 1-4

Under nitrogen protection, phosphorus oxychloride (40 g, 260.9 mmol) was dissolved in toluene (200 mL), cooled to 5 °C, and morpholine (89.7 g, 1.025 mol) was slowly added dropwise into the mixture, while maintaining the reaction temperature below 20 °C. the mixture was stirred at room temperature for 3 hours. The mixture was filtered, and was washed with toluene three times (3 × 35 mL). The combined filtrates were concentrated under reduced pressure to give the residue, the residue was dissolved in toluene (64 mL) with heating to give a homogeneous solution. Petroleum ether (29 mL) was added with stirring, followed by the addition of another portion of petroleum ether (116 mL). The mixture was cooled to room temperature and filtered. The filter cake was washed with petroleum ether and dried under reduced pressure to obtain bis(morpholino)phosphinic chloride as a white solid (40 g, 60.2% yield).

At -30°C, the solution of lithium bis(trimethylsilyl)amide (72 mL, 1 mol/L, 72 mmol) was added dropwise to a solution of **1-3** (24.1 g, 59.9 mmol) in tetrahydrofuran (280 mL). The mixture was stirred for 1 hour. Bis(morpholino)phosphinic chloride (36.1 g, 141.8 mmol) was added portionwise. After the addition was complete, the mixture was stirred at -10°C for 4 hours. Isopropanolamine (20.3 g, 270.3 mmol) was added dropwise to the mixture. The mixture was then allowed to warm to room temperature and stirred for 12 hours. After confirming reaction completion by TLC, ice-water (100 mL) was added to the reaction system, the organic phase was extracted with ethyl acetate (3 × 100 mL), washed with saturated brine, dried over anhydrous Na₂SO₄, filtered and concentrated under reduced pressure to obtain a crude product. The crude product was purified by flash column chromatography (ethyl acetate/petroleum ether (v/v) = 1/10 - 1/1). TLC (ethyl acetate/petroleum ether (v/v) = 1/1) momnitoring was performed, and the fractions with Rf = 0.2-0.3 were collected to yield compound **1-4** as white solid (20.0 g, yield 72.7%). ESI[M + H]⁺ = 459.1.

### 5. Preparation of Compound 1-5

Dess-Martin periodinane (45.8 g, 108.0 mmol) was added to a solution of 1-4 (20 g, 43.5 mmol) in acetone (200 mL), the mixture was stirred at room temperature for 12 hours. After confirming reaction completion by TLC, The mixture was filtered, and concentrated under reduced pressure to obtain the residue. The residue was dissolved in ethyl acetate (300 mL), was washed with a saturated sodium bicarbonate solution, dried over anhydrous Na₂SO₄, filtered and concentrated under reduced pressure to obtain a crude product. The crude product was purified by flash column chromatography (ethyl acetate/petroleum ether (v/v) = 1/10 - 1/1). TLC (ethyl acetate/petroleum ether (v/v) = 1/1) momnitoring was performed, and the fractions with Rf = 0.2-0.3 were collected to yield compound **1-5** as white solid (12.7 g, yield 66.4%). ESI[M+H]⁺= 439.1.

### 6. Preparation of Compound 1-6

At -10°C, LiOH·H₂O (619 mg, 14.8 mmol) and NaOH (536 mg, 13.4 mmol) were successively added to a solution of **1-5** (5.9 g, 13.4 mmol) in MeOH/H₂O (50 mL, v/v = 1/1). The mixture was allowed to warm to room temperature and stirred for 5 hours. After confirming reaction completion by TLC, the pH of the mixture was adjusted to 6-7 with 1 mol/L hydrochloric acid in an ice-water bath. The mixture was then extracted with ethyl acetate (3 × 70 mL). The organic layer was washed with brine, ried over anhydrous Na₂SO₄, filtered and concentrated under reduced pressure to obtain a crude product. The crude product was purified by flash column chromatography (MeOH/CH₂Cl₂ (v/v) = 1/100 - 1/20). TLC (MeOH/CH₂Cl₂ (v/v) = 1/10) momnitoring was performed, and the fractions with Rf = 0.3-0.4 were collected to yield compound **1-6** as white solid (4.7 g, yield 82.3%). ESI[M + H]⁺ = 425.1.

### 7. Preparation of Compound 1

At 0°C, a solution of ethynyllmagnesium bromide in THF (13.9 mL, 1.3 mol/L, 18.1 mmol) was added dropwise to a solution of 3-oxetanone (1 g, 13.9 mmol) in anhydrous tetrahydrofuran (20 mL). The mixture was stirred at 0°C for 30 minutes. After confirming reaction completion by TLC, saturated aqueous ammonium chloride solution (15 mL) was added to the mixture. The mixture was extracted with ethyl acetate (3 × 20 mL). The combined organic layers were washed with brine, dried over anhydrous Na₂SO₄, filtered, and concentrated under reduced pressure to give the crude product. The crude product was purified by flash column chromatography (ethyl acetate/petroleum ether, v/v = 1/10 to 1/1), TLC (ethyl acetate/petroleum ether (v/v) = 1/1) momnitoring was performed, and the fractions with Rf = 0.2-0.3 were collected to yield compound 3-ethynylloxetan-3-ol (1.36 g, yield 97.9%).

A mixture of **1-6** (634 mg, 1.49 mmol), DCC (463 mg, 2.24 mmol), and DMAP (274 mg, 2.24 mmol) in dichloromethane (10 mL) were stirred at room temperature for 15 minutes. Then 3-ethynylloxetan-3-ol (227.6 mg, 2.27 mmol) was added to the mixture, and stirring was continued at room temperature for 12 hours. After confirming reaction completion by TLC, methyl tert-butyl ether (10 mL) was added, and the mixture was stirred for 5 minutes. The mixture was filtered, and the filtrate was concentrated under reduced pressure to give the crude product. The crude product was purified by silica gel column chromatography (ethyl acetate/petroleum ether, v/v = 1/10 - 1/1) , TLC (ethyl acetate/petroleum ether (v/v) = 1/1) momnitoring was performed, and the fractions with Rf = 0.2-0.3 were collected to yield compound **1** as gray solid (290 mg, yield 38.3%). ESI[M + H]⁺ = 507.1_{∘}

¹H NMR (400 MHz, DMSO-*d6*) δ 8.55 (d, *J =* 4.0 Hz, 1H), 8.11 (d, *J =* 7.9 Hz, 1H), 7.95 (td, *J =* 7.8, 1.7 Hz, 1H), 7.89 (dd, *J =* 8.7, 2.3 Hz, 1H), 7.66 (d, *J =* 8.7 Hz, 1H), 7.60 (d, *J =* 2.3 Hz, 1H), 7.54 - 7.46 (m, 1H), 6.82 (d, *J =* 1.0 Hz, 1H), 6.23 (dd, *J =* 17.4, 10.9 Hz, 1H), 5.26 (dd, *J =* 25.2, 14.2 Hz, 2H), 4.70 (d, *J =* 6.0 Hz, 2H), 4.60 (d, *J=* 7.5 Hz, 2H), 4.07 - 4.02 (m, 1H), 2.87 - 2.68 (m, 2H), 2.63 - 2.50 (m, 2H), 2.30 (s, 3H).

### 8. Preparation of Compound 1D

**Compound 1** (192.7 mg, 0.38 mmol) was dissolved in ethyl acetate (15 mL). A solution of benzenesulfonic acid (60.1 mg, 0.38 mmol) in ethanol (0.6 mL) was added dropwise to the mixture. The mixture was stirred at room temperature for 1 hour. The mixture was filtered, and the filter cake was dried under reduced pressure to give **Compound 1D** as white solid (196.8 mg, yield 77.9%). ESI[M + H]⁺ = 507.1.

¹H NMR (400 MHz, DMSO-*d6*) δ 8.61 - 8.56 (m, 1H), 8.15 (d, *J =* 7.9 Hz, 1H), 8.05 - 7.94 (m, 2H), 7.84 (d, *J =* 8.8 Hz, 1H), 7.74 (d, *J =* 2.3 Hz, 1H), 7.62 - 7.58 (m, 2H), 7.57 - 7.53 (m, 1H), 7.44 (s, 1H), 7.36 - 7.25 (m, 3H), 6.25 (dd, *J =* 17.4, 10.9 Hz, 1H), 5.35 - 5.25 (m, 2H), 4.71 (dd, *J =* 7.4, 2.4 Hz, 2H), 4.62 (dd, *J =* 7.3, 3.1 Hz, 2H), 4.33 (dd, *J =* 8.3, 5.2 Hz, 1H), 2.90 - 2.79 (m, 1H), 2.77 - 2.64 (m, 2H), 2.54 - 2.50 (m, 1H), 2.39 (d, *J =* 0.8 Hz, 3H).

### Example 2 Preparation of Compound 2 and compound 2D

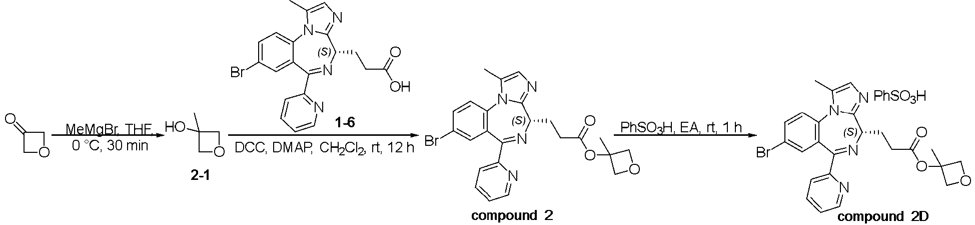

### 1. Preparation of Compound 2-1

At 0°C, a solution of methylmagnesium bromide in THF (23 mL, 3 mol/L, 69 mmol) was added dropwise to the solution of 3-oxetanone (4 g, 55.5 mmol) in anhydrous tetrahydrofuran (40 mL). The mixture was stirred at 0°C for 30 minutes. After confirming reaction completion by TLC, the saturated aqueous ammonium chloride solution (20 mL) was added to the mixture. The mixture was extracted with ethyl acetate (3 × 30 mL). The combined organic layers were washed with brine, dried over anhydrous Na₂SO₄, filtered, the filtrate was concentrated under reduced pressure to give the crude product. The crude product was purified by silica gel column chromatography (ethyl acetate/petroleum ether, v/v=1/10-1/1), TLC (ethyl acetate/petroleum ether (v/v)=1/1) momnitoring was performed, and the fractions with Rf = 0.2-0.3 were collected to yield compound **3-Methyloxetan-3-ol** (2.91 g, yield 59.5%).

### 2. Preparation of Compound 2

A mixture of **1-6** (350 mg, 0.823 mmol), DCC (255 mg, 1.24 mmol), and DMAP (151 mg, 1.24 mmol) in dichloromethane (10 mL) was stirred at room temperature for 15 minutes. Then, 3-methyloxetan-3-ol (148 mg, 1.68 mmol) was added to the mixture, and stirred at room temperature for 12 hours. After confirming reaction completion by TLC, methyl tert-butyl ether (10 mL) was added, and the mixture was stirred for 5 minutes, filtered, the filtrate was concentrated under reduced pressure to give the crude product. The crude product was purified by silica gel column chromatography (ethyl acetate/petroleum ether, v/v = 1/10 - 1/1), TLC (ethyl acetate/petroleum ether (v/v) = 1/1) momnitoring was performed, and the fractions with Rf = 0.2-0.3 were collected to give **Compound 2** as white solid (148 mg, yield 36.3%). ESI[M + H]⁺ =495.1.

¹H NMR (400 MHz, CDCl₃) δ 8.58 (d, *J =* 3.9 Hz, 1H), 8.21 (d, *J =* 7.9 Hz, 1H), 7.88 - 7.77 (m, 2H), 7.73 (d, *J =* 1.9 Hz, 1H), 7.44 - 7.34 (m, 2H), 7.08 (s, 1H), 4.71 (dd, *J* = 7.1, 3.9 Hz, 2H), 4.46 (d, *J* = 7.6 Hz, 2H), 4.20 (brs, 1H), 3.00 (brs, 1H), 2.85 - 2.81 (m, 3H), 2.39 (s, 3H), 1.66 (s, 3H).

### 3. Preparation of Compound 2D

**Compound 2** (148 mg, 0.30 mmol) was dissolved in ethyl acetate (35 mL). A solution of benzenesulfonic acid (47.3 mg, 0.30 mmol) in ethanol (0.5 mL) was added dropwise to the mixture. The mixture was stirred at room temperature for 1 hour. The mixture was filtered, and the filter cake was dried under reduced pressure to give **Compound 2D** as white solid (162.1 mg, yield 83%). ESI[M + H]⁺ = 495.1.

¹H NMR (400 MHz, DMSO-d6) δ 8.60 - 8.56 (m, 1H), 8.14 (d, *J =* 7.9 Hz, 1H), 8.04 - 7.95 (m, 2H), 7.84 (d, *J =* 8.7 Hz, 1H), 7.74 (d, *J =* 2.3 Hz, 1H), 7.62 - 7.57 (m, 2H), 7.55 (ddd, *J =* 7.6, 4.8, 1.1 Hz, 1H), 7.43 (s, 1H), 7.35 - 7.26 (m, 3H), 4.62 (d, *J =* 6.1 Hz, 2H), 4.43 (d, *J =* 7.7 Hz, 2H), 4.34 (dd, *J =* 8.6, 5.1 Hz, 1H), 2.84 - 2.71 (m, 1H), 2.71 - 2.58 (m, 2H), 2.52 - 2.50 (m, 1H), 2.39 (d, *J =* 0.8 Hz, 3H), 1.62 (s, 3H).

### Example 3 Preparation of Compound 3

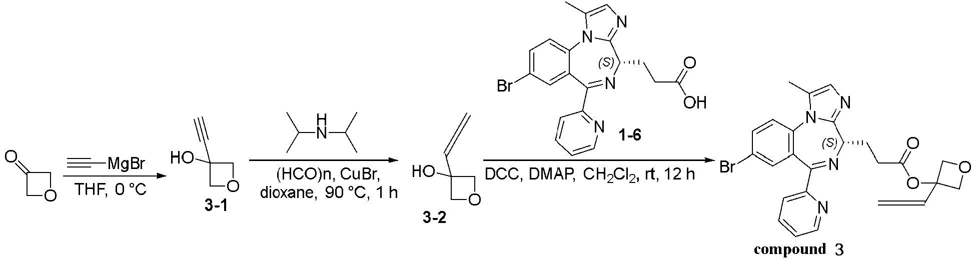

### 1. Preparation of Compound 3-1

At 0°C, a solution of ethynylmagnesium bromide in THF (72.2 mL, 0.5 mol/L, 36.1 mmol) was added dropwise to a solution of 3-oxetanone (2 g, 27.8 mmol) in anhydrous tetrahydrofuran (30 mL). The mixture was stirred at 0°C for 30 minutes. After confirming reaction completion by TLC, A saturated aqueous ammonium chloride solution (20 mL) was added to the mixture, the mixture was extracted with ethyl acetate (3 × 30 mL). The combined organic layers were washed with brine, dried over anhydrous Na₂SO₄, filtered, the filtrate was concentrated under reduced pressure to give the crude product. The crude product was purified by silica gel column chromatography (ethyl acetate/petroleum ether, v/v = 1/10 - 1/1), TLC (ethyl acetate/petroleum ether (v/v) = 1/1) momnitoring was performed, and the fractions with Rf = 0.2-0.3 were collected to yield Compound **3-1** as colorless oil (1.32 g, yield 48.5%). ESI[M + H]⁺ = 99.1.

### 2. Preparation of Compound 3-2

A mixture of **3-1** (1.07 g, 10.9 mmol), paraformaldehyde (811 mg, 27.0 mmol), diisopropylamine (1.965 g, 19.4 mmol), and copper(I) bromide (770 mg, 5.37 mmol) in 1,4-dioxane (30 mL) was stirred at 90°C for 1 hour. After confirming reaction completion by TLC, the mixture was filtered, the filtrate was concentrated under reduced pressure to give the crude product. The crude product was purified by silica gel column chromatography (ethyl acetate/petroleum ether, v/v = 1/20-1/2), TLC (ethyl acetate/petroleum ether (v/v) = 1/1) momnitoring was performed, and the fractions with Rf = 0.3-0.4 were collected to yield Compound **3-2** as colorless oil (72 mg, yield 5.9%). ESI[M + H]⁺ = 113.1.

### 3. Preparation of Compound 3

A mixture of **1-6** (100 mg, 0.235 mmol), DCC (73 mg, 0.354 mmol), and DMAP (43.2 mg, 0.354 mmol) in dichloromethane (2 mL) was stirred at room temperature for 15 minutes. Then, **3-2** (26.6 mg, 0.237 mmol) was added to the mixture, and stirred at room temperature for 12 hours. After confirming reaction completion by TLC, methyl tert-butyl ether (2 mL) was added, and the mixture was stirred for 5 minutes, filtered, the filtrate was concentrated under reduced pressure to give the crude product. The crude product was purified by silica gel column chromatography (ethyl acetate/petroleum ether, v/v = 1/10 - 1/1), TLC (ethyl acetate/petroleum ether (v/v) = 1/1) momnitoring was performed, and the fractions with Rf = 0.2-0.3 were collected to give **Compound 3** as white solid (57.4 mg, yield 47%). ESI[M + H]⁺ =519.1.

¹H NMR (400 MHz, CDCl₃) δ 8.58 (d, *J =* 4.6 Hz, 1H), 8.20 (d, *J =* 7.9 Hz, 1H), 7.88 - 7.76 (m, 2H), 7.73 (s, 1H), 7.42 - 7.31 (m, 2H), 7.06 (s, 1H), 5.57 (t, *J =* 6.6 Hz, 1H), 4.99 - 4.94 (m, 2H), 4.78 - 4.75 (m, 2H), 4.72 - 4.68 (m, 2H), 4.20 - 4.18 (m, 1H), 3.02 - 3.00 (m, 1H), 2.88 - 2.86 (m, 3H), 2.39 (s, 3H).

### Example 4 Preparation of Compound 4

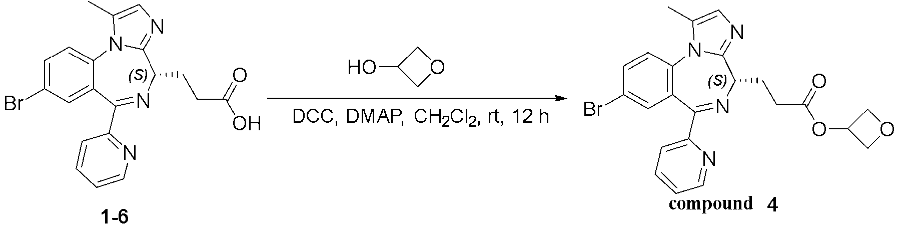

A mixture of **1-6** (80 mg, 0.188 mmol), DCC (58.3 mg, 0.283 mmol), and DMAP (34.5 mg, 0.282 mmol) in dichloromethane (2 mL) was stirred at room temperature for 15 minutes. Then, 3-methyloxetan-3-ol (28 mg, 0.378 mmol) was added to the mixture, and stirred at room temperature for 12 hours. After confirming reaction completion by TLC, methyl tert-butyl ether (10 mL) was added, and the mixture was stirred for 5 minutes, filtered, the filtrate was concentrated under reduced pressure to give the crude product. The crude product was purified by silica gel column chromatography (ethyl acetate/petroleum ether, v/v = 1/10 - 1/1), TLC (ethyl acetate/petroleum ether (v/v) = 1/1) momnitoring was performed, and the fractions with Rf = 0.2-0.3 were collected to give **Compound 4** as white solid (42.5 mg, yield 46.9%). ESI[M + H]⁺ =481.0.

¹H NMR (400 MHz, CDCl₃) δ 8.58 (d, *J =* 4.0 Hz, 1H), 8.18 (d, *J =* 8.0 Hz, 1H), 7.82 (td, *J* = 7.8, 1.7 Hz, 1H), 7.76 (dd, *J =* 8.6, 2.1 Hz, 1H), 7.70 (d, *J =* 2.1 Hz, 1H), 7.40 - 7.35 (m, 1H), 7.34 (d, *J =* 8.6 Hz, 1H), 6.99 (s, 1H), 5.48 - 5.38 (m, 1H), 4.85 (dd, *J =* 11.6, 6.8 Hz, 2H), 4.67 - 4.55 (m, 2H), 4.13 (s, 1H), 2.97 - 2.95 (m, 1H), 2.91 - 2.76 (m, 3H), 2.37 (s, 3H).

### Example 5 Preparation of Compound 5, 6, 6D, 7 and 7D

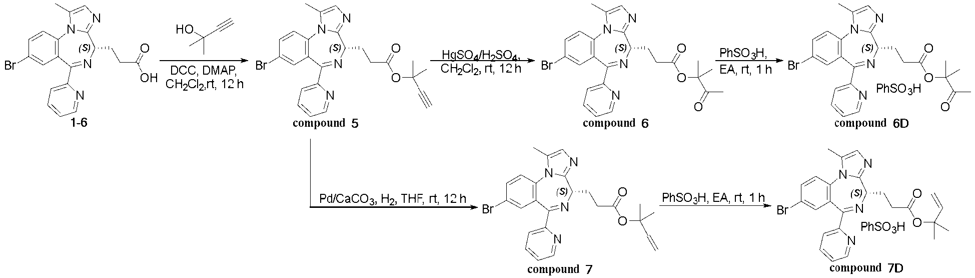

### 1. Preparation of Compound 5

A mixture of 1-6 (3 g, 7.05 mmol), DCC (2.89 g, 14.0 mmol), and DMAP (1.71 g, 14.0 mmol) in dichloromethane (30 mL) was stirred at room temperature for 15 minutes. Then, 3-methyloxetan-3-ol (1.18 g, 14.0 mmol) was added to the mixture, and stirred at room temperature for 12 hours. After confirming reaction completion by TLC, methyl tert-butyl ether (30 mL) was added, and the mixture was stirred for 5 minutes, filtered, the filtrate was concentrated under reduced pressure to give the crude product. The crude product was purified by silica gel column chromatography (ethyl acetate/petroleum ether, v/v = 1/10 - 1/1), TLC (ethyl acetate/petroleum ether (v/v) = 1/1) momnitoring was performed, and the fractions with Rf = 0.3-0.4 were collected to give **Compound 5** as white solid (1.8 g, yield 51.9%). ESI[M + H]⁺ =491.0.

¹H NMR (400 MHz, DMSO-d6) δ 8.54 (d, *J=* 4.0 Hz, 1H), 8.11 (d, *J* = 7.9 Hz, 1H), 7.94 (td, *J =* 7.8, 1.7 Hz, 1H), 7.88 (dd, *J =* 8.7, 2.3 Hz, 1H), 7.66 (d, *J =* 8.7 Hz, 1H), 7.60 (d, *J =* 2.3 Hz, 1H), 7.54 - 7.47 (m, 1H), 6.81 (d, *J =* 1.1 Hz, 1H), 4.05 (t, *J =* 6.6 Hz, 1H), 3.47 (s, 1H), 2.73 - 2.52 (m, 4H), 2.30 (s, 3H), 1.58 (s, 6H).

### 2. Preparation of Compound 6

**Compound 5** (600 mg, 1.22 mmol) was dissolved in dichloromethane (10 mL). mercury(II) sulfate/sulfuric acid/silica gel (600 mg) was added, and the mixture was stirred at room temperature for 12 hours. After confirming reaction completion by TLC, the mixture was neutralized with a saturated aqueous sodium bicarbonate solution. The mixture was extracted with dichloromethane (3 × 10 mL). The combined organic layers were washed with brine, dried over anhydrous Na₂SO₄, filtered, the filtrate was concentrated under reduced pressure to give the crude product. The crude product was purified by TCL (MeOH/CH₂Cl₂, v/v = 1/30), and the fractions with Rf = 0.4-0.5 were collected to yield Compound 6 as white solid (267.1 mg, yield 42.9%). ESI[M + H]⁺ = 509.2.

¹H NMR (400 MHz, DMSO-d6) δ 8.54 (d, *J =* 4.0 Hz, 1H), 8.11 (d, *J =* 7.9 Hz, 1H), 7.95 (td, *J =* 7.8, 1.7 Hz, 1H), 7.88 (dd, *J =* 8.7, 2.3 Hz, 1H), 7.66 (d, *J =* 8.7 Hz, 1H), 7.60 (d, *J =* 2.3 Hz, 1H), 7.54 - 7.46 (m, 1H), 6.83 (s, 1H), 4.11 - 4.02 (m, 1H), 2.86 - 2.51 (m, 4H), 2.30 (s, 3H), 2.01 (s, 3H), 1.37 (s, 3H), 1.36 (s, 3H).

### 3. Preparation of Compound 6D

**Compound 6** (199 mg, 0.391 mmol) was dissolved in ethyl acetate (10 mL). A solution of benzenesulfonic acid (61.9 mg, 0.391 mmol) in ethanol (0.6 mL) was added dropwise to the mixture. The mixture was stirred at room temperature for 1 hour. The mixture was filtered, and the filter cake was dried under reduced pressure to give **Compound 6D** as white solid (236.4 mg, yield 90.6%). ESI[M + H]⁺ = 509.1.

¹H NMR (400 MHz, DMSO-*d6*) δ 8.57 (d, *J =* 4.1 Hz, 1H), 8.14 (d, *J =* 7.9 Hz, 1H), 8.02 - 7.94 (m, 2H), 7.82 (d, *J =* 8.8 Hz, 1H), 7.72 (d, *J =* 2.3 Hz, 1H), 7.62 - 7.57 (m, 2H), 7.57 - 7.51 (m, 1H), 7.37 (s, 1H), 7.34 - 7.25 (m, 3H), 4.29 - 4.28 (m, 1H), 2.86 - 2.75 (m, 1H), 2.71 - 2.59 (m, 2H), 2.51 - 2.50 (m, 1H), 2.38 (s, 3H), 2.04 (s, 3H), 1.40 (s, 3H), 1.38 (s, 3H).

### 4. Preparation of Compound 7

**Compound** 5 (600 mg, 1.22 mmol) was dissolved in tetrahydrofuran (10 mL) at room temperature, Lindlar catalyst (600 mg, 5% Pd) was added. The mixture was stirred under hydrogen atmosphere at room temperature for 12 hours. After confirming reaction completion by TLC, the mixture was filtered, the filtrate was concentrated under reduced pressure to give the crude product. The residue was purified by preparative TLC (MeOH/CH₂Cl₂, v/v = 1/30), and the fractions with Rf = 0.3-0.4 were collected to yield Compound 7 as white solid (369.8 mg, yield 61.4%). ESI[M + H]⁺ = 493.2_{∘}

¹H NMR (400 MHz, CDCl₃) δ 8.57 (d, *J =* 3.7 Hz, 1H), 8.21 (d, *J =* 7.9 Hz, 1H), 7.84 - 7.80 (m, 1H), 7.78 - 7.76 (m, 1H), 7.69 (s, 1H), 7.37 (dd, *J =* 7.1, 4.8 Hz, 2H), 7.01 (s, 1H), 6.01 (dd, *J* = 17.5, 10.8 Hz, 1H), 5.09 (d, *J =* 17.5 Hz, 1H), 4.99 (d, *J =* 10.8 Hz, 1H), 4.22 - 4.19 (m, 1H), 2.95 - 2.94 (m, 1H), 2.77 - 2.75 (m, 3H), 2.38 (s, 3H), 1.46 (s, 6H).

### 5. Preparation of Compound 7D

**Compound 7** (220 mg, 0.446 mmol) was dissolved in ethyl acetate (2.2 mL). A solution of benzenesulfonic acid (70.7 mg, 0.446 mmol) in ethanol (0.7 mL) was added dropwise to the mixture. The mixture was stirred at room temperature for 1 hour. The mixture was filtered, and the filter cake was dried under reduced pressure to give **Compound 7D** as white solid (251.5 mg, yield 86.6%). ESI[M + H]⁺ = 493.1.

¹H NMR (400 MHz, DMSO-*d6*) δ 8.57 (d, *J =* 4.1 Hz, 1H), 8.13 (d, *J =* 7.9 Hz, 1H), 8.03 - 7.94 (m, 2H), 7.81 (d, *J* = 8.9 Hz, 1H), 7.71 (s, 1H), 7.62 - 7.57 (m, 2H), 7.56 - 7.51 (m, 1H), 7.38 - 7.26 (m, 4H), 6.04 (dd, *J =* 17.5, 10.9 Hz, 1H), 5.13 (d, *J =* 17.5 Hz, 1H), 5.01 (dd, *J =* 10.9, 0.9 Hz, 1H), 4.26 (s, 1H), 2.62 - 2.50 (m, 4H), 2.37 (s, 3H), 1.45 (s, 6H).

### Example 6 Preparation of Compound 9

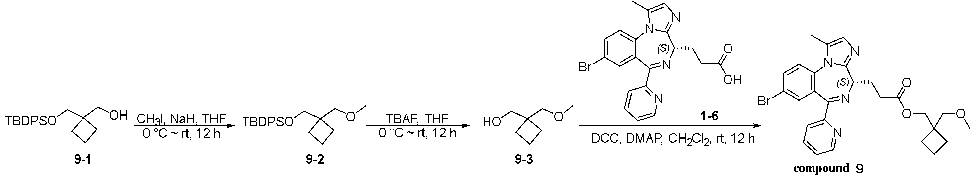

### 1. Preparation of Compound 9-2

At 0 °C, sodium hydride (26.7 mg, 60%, 0.667 mmol) was added portionwise to a solution of 9-1 (197 mg, 0.556 mmol) in anhydrous tetrahydrofuran (5 mL). The mixture was stirred at this temperature for 30 minutes. Iodomethane (118.3 mg, 0.833 mmol) was then added to the mixture. The mixture was allowed to warm to room temperature naturally and stirred for 12 hours. After confirming reaction completion by TLC, ice-water (10 mL) was added to the reaction system. The organic phase was extracted with ethyl acetate (3 × 10 mL), dried over anhydrous Na₂SO₄, filtered and concentrated under reduced pressure to obtain a crude product. The crude product was purified by silica gel column chromatography (ethyl acetate/petroleum ether (v/v) = 1/100-1/10), TLC (ethyl acetate/petroleum ether (v/v) = 1/10) momnitoring was performed, and the fractions with Rf = 0.4-0.5 were collected to give **Compound 9-2** as white solid (124 mg, yield 60.5%). ESI[M + H]⁺ = 369.2.

### 2. Preparation of Compound 9-3

At 0°C, TBAF (176 mg, 0.673 mmol) was added to a solution of 9-2 (124 mg, 0.336 mmol) in tetrahydrofuran (5 mL). The mixture was allowed to warm to room temperature naturally and stirred for 12 hours. After confirming reaction completion by TLC, ice-water (10 mL) was added to the reaction system. The organic phase was extracted with ethyl acetate (3 × 10 mL), dried over anhydrous Na₂SO₄, filtered and concentrated under reduced pressure to obtain a crude product. The crude product was purified by silica gel column chromatography (ethyl acetate/petroleum ether (v/v) = 1/10-1/2), TLC (ethyl acetate/petroleum ether (v/v) = 1/2) momnitoring was performed, and the fractions with Rf = 0.3-0.4 were collected to give **Compound 9-3** as colorless oil (38 mg, yield 86.8%). ESI[M + H]⁺ = 131.2.

### 3. Preparation of Compound 9

A mixture of **1-6** (100 mg, 0.235 mmol), DCC (73.0 mg, 0.354 mmol), and DMAP (43.2 mg, 0.353 mmol) in dichloromethane (2 mL) was stirred at room temperature for 15 minutes. Then, **9-3** (30.6 mg, 0.235 mmol) was added to the mixture, and stirred at room temperature for 12 hours. After confirming reaction completion by TLC, methyl tert-butyl ether (2 mL) was added, and the mixture was stirred for 5 minutes, filtered, the filtrate was concentrated under reduced pressure to give the crude product. The crude product was purified by silica gel column chromatography (ethyl acetate/petroleum ether, v/v = 1/10 - 2/1), TLC (ethyl acetate/petroleum ether (v/v) = 3/1) momnitoring was performed, and the fractions with Rf = 0.3-0.4 were collected to give **Compound 9** as white solid (81.7 mg, yield 64.6%). ESI[M + H]⁺ =537.2.

¹H NMR (400 MHz, CDCl₃) δ 8.57 (d, *J =* 4.0 Hz, 1H), 8.22 - 8.20 (m, 1H), 7.85 - 7.83 (m, 2H), 7.76 - 7.65 (m, 1H), 7.54 - 7.29 (m, 2H), 7.26 - 7.13 (m, 1H), 4.47 - 4.44 (m, 1H), 4.17 - 3.91 (m, 2H), 3.30 (s, 5H), 3.17 - 2.63 (m, 4H), 2.39 (s, 3H), 1.85 - 1.71 (m, 6H).

### Example 7 Preparation of Compound 10

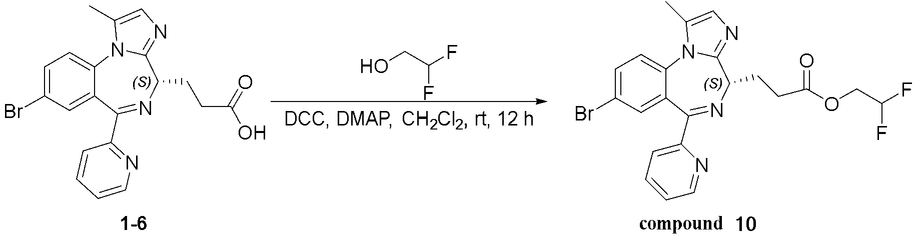

A mixture of **1-6** (100 mg, 0.235 mmol), DCC (73.0 mg, 0.354 mmol), and DMAP (43.2 mg, 0.353 mmol) in dichloromethane (2 mL) was stirred at room temperature for 15 minutes. Then, **2,2-Difluoroethanol** (21.3 mg, 0.260 mmol) was added to the mixture, and stirred at room temperature for 12 hours. After confirming reaction completion by TLC, methyl tert-butyl ether (2 mL) was added, and the mixture was stirred for 5 minutes, filtered, the filtrate was concentrated under reduced pressure to give the crude product. The crude product was purified by silica gel column chromatography (MeOH/CH₂Cl₂, v/v = 1/30), TLC (ethyl acetate/petroleum ether (v/v) = 3/1) momnitoring was performed, and the fractions with Rf = 0.4-0.5 were collected to give **Compound 10** as Colorless oil (90.1 mg, yield 78.3%). ESI[M + H]⁺ =489.0.

¹H NMR (400 MHz, CDCl₃) δ 8.58 (d, *J* = 4.0 Hz, 1H), 8.22 - 8.19 (m, 1H), 7.86 (t, *J =* 7.8 Hz, 2H), 7.80 (s, 1H), 7.42 (dd, *J =* 7.3, 5.0 Hz, 2H), 7.28 - 7.26 (m, 1H), 6.10 - 5.70 (m, 1H), 4.40 - 4.37 (m, 1H), 4.32 - 4.16 (m, 2H), 3.22 - 3.17 (m, 1H), 3.09 - 2.80 (m, 3H), 2.44 (s, 3H).

### Example 8 Preparation of Compound 11

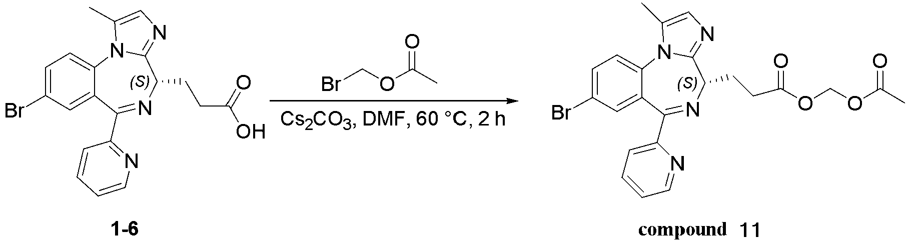

To solution of **1-6** (81 mg, 0.190 mmol), methyl bromoacetate (35.1 mg, 0.229 mmol), and cesium carbonate (93 mg, 0.285 mmol) in DMF (2 mL), the mixture was stirred at 60 °C for 2 hours. After confirming reaction completion by TLC, ice-water was added to the reaction system. The organic phase was extracted with ethyl acetate (3 × 15 mL), dried over anhydrous Na₂SO₄, filtered and concentrated under reduced pressure to obtain a crude product. The crude product was purified by preparative TLC (MeOH/CH₂Cl₂, v/v = 1/30), and the fractions with Rf = 0.3-0.4 were collected to yield **Compound 11** as white solid (39.1 mg, yield 41.3%). ESI[M + H]⁺ = 497.2.

¹H NMR (400 MHz, CDCl₃) δ 8.58 (d, *J =* 4.7 Hz, 1H), 8.19 (d, *J =* 7.9 Hz, 1H), 7.87 - 7.77 (m, 2H), 7.75 (s, 1H), 7.43 - 7.33 (m, 2H), 7.12 (s, 1H), 5.75 - 5.66 (m, 2H), 4.28 - 4.26 (m, 1H), 3.06 - 3.04 (m, 1H), 2.92 - 2.88 (m, 3H), 2.40 (s, 3H), 2.06 (s, 3H).

### Example 9 Preparation of Compound 12

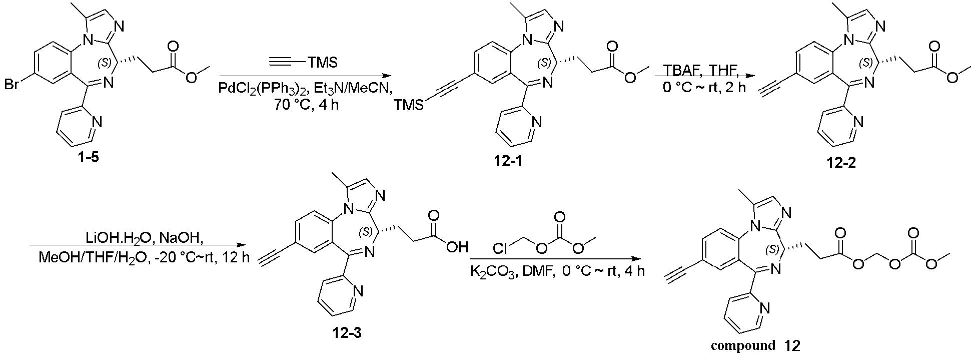

### 1. Preparation of Compound 12-1

A mixture of **1-5** (632 mg, 1.44 mmol), trimethylsilylacetylene (2.82 g, 28.7 mmol), and PdCl₂(PPh₃)₂ (101.1 mg, 0.144 mmol) was dissolved in a mixture of triethylamine (20 mL) and acetonitrile (30 mL). The mixture was stirred at 70°C for 4 hours. After confirming reaction completion by TLC, the filtrate was concentrated under reduced pressure to give the crude product, the mixture was concentrated under reduced pressure. The residue was dissolved in ethyl acetate (50 mL) and washed with brine, dried over anhydrous Na₂SO₄, and filtration. The filtrate was concentrated under reduced pressure to give the crude product, the crude product was purified by silica gel column chromatography (ethyl acetate/petroleum ether, v/v = 1/20-1/1), TLC (ethyl acetate/petroleum ether (v/v) = 1/1) momnitoring was performed, and the fractions with Rf = 0.2-0.3 were collected to give **Compound 12-1** as white solid (627 mg, yield 95.4%).ESI[M + H]⁺ = 457.2.

### 2. Preparation of Compound 12-2

At 0°C, TBAF (538.5 mg, 2.06 mmol) was added to a solution of **12-1** (627 mg, 1.37 mmol) in tetrahydrofuran (5 mL). The mixture was allowed to warm to room temperature and stirred for 2 hours. After confirming reaction completion by TLC, ice-water (10 mL) was added to the reaction system. The organic phase was extracted with ethyl acetate (3 × 15 mL), washed with brine, dried over anhydrous Na₂SO₄, filtered and concentrated under reduced pressure to obtain crude product. The crude product was purified by preparative TLC (MeOH/CH₂Cl₂, v/v = 1/10), and the fractions with Rf = 0.3-0.4 were collected to yield **Compound 12-2** as yellow solid (287.5 mg, yield 54.5%). ESI[M + H]⁺ = 385.2.

### 3. Preparation of Compound 12-3

At -20 °C, LiOH·H₂O (34.5 mg, 0.822 mmol) and NaOH (29.9 mg, 0.747 mmol) were added successively to a solution of **12-2** (287.5 mg, 0.748 mmol) in a mixture of MeOH/THF/H₂O (10 mL, v/v/v = 1/1/1). The mixture was then allowed to warm to room temperature and stirred for 12 hours. After confirming reaction completion by TLC, the mixture was adjusted to pH 6-7 with 1 mol/L hydrochloric acid in an ice bath, and then extracted with ethyl acetate (3 × 50 mL), washed with brine, dried over anhydrous Na₂SO₄, filtered and concentrated under reduced pressure to obtain crude product. The crude product was purified by preparative TLC (MeOH/CH₂Cl₂, v/v = 1/8), and the fractions with Rf = 0.3-0.4 were collected to yield **Compound 12-3** as white solid (267.9 mg, yield 96.7%). ESI[M + H]⁺ = 371.1.

### 4. Preparation of Compound 12

At 0°C, a mixture of **12-3** (146.5 mg, 0.396 mmol), dimethyl chloromethyl carbonate (148.4 mg, 1.19 mmol), and potassium carbonate (129.5 mg, 0.937 mmol) in DMF (3 mL) was stirred and allowed to warm to room temperature naturally over 4 hours. After confirming reaction completion by TLC, water (10 mL) was added to the reaction system, the organic phase was extracted with ethyl acetate (3 × 10 mL), washed with brine, dried over anhydrous Na₂SO₄, filtered and concentrated under reduced pressure to obtain crude product. The crude product was purified by preparative TLC (ethyl acetate/petroleum ether, v/v = 1/1), and the fractions with Rf = 0.2-0.3 were collected to yield **Compound 12** as white solid (49.4 mg, yield 27.2%). ESI[M + H]⁺ = 459.1.

¹H NMR (400 MHz, DMSO-d6) δ 8.53 (d, *J =* 4.0 Hz, 1H), 8.09 (d, *J =* 7.9 Hz, 1H), 7.98 - 7.90 (m, 1H), 7.77 (dd, *J =* 8.4, 1.9 Hz, 1H), 7.70 (d, *J =* 8.4 Hz, 1H), 7.51 - 7.48 (m, 2H), 6.82 (d, *J =* 1.0 Hz, 1H), 5.71 (s, 2H), 4.32 (s, 1H), 4.06 - 4.02 (m, 1H), 3.73 (s, 3H), 2.85 - 2.73 (m, 2H), 2.66 -2.50 (m, 2H), 2.31 (s, 3H).

### Example 10 Preparation of Compound 13

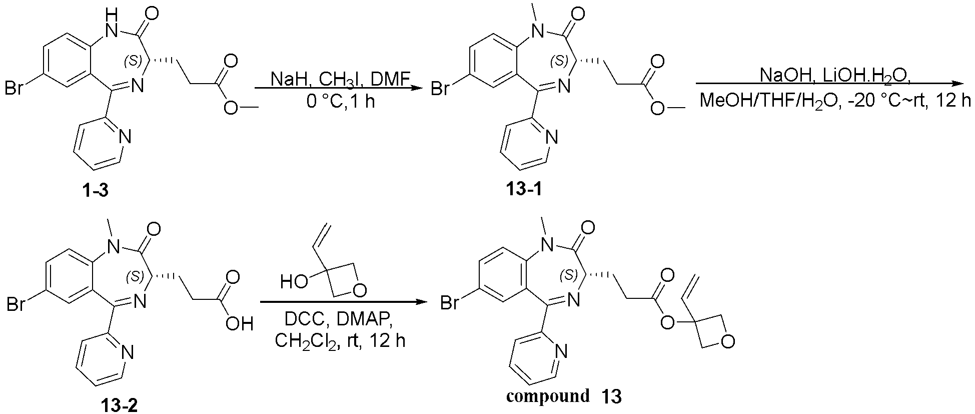

### 1. Preparation of Compound 13-1

At 0°C, sodium hydride (175 mg, 60% dispersion in mineral oil, 4.37 mmol) was added cautiously to a solution of **1-3** (1.6 g, 3.98 mmol) in dry DMF (15 mL). The mixture was maintained at 0 °C and stirred for 30 minutes. Subsequently, methyl iodide (623 mg, 4.39 mmol) was added to the mixture, and stirring was continued for 1 hour. After confirming reaction completion by TLC, ice-water (30 mL) was added to the reaction system, the organic phase was extracted with ethyl acetate (3 × 20 mL), washed with brine, dried over anhydrous Na₂SO₄, filtered and concentrated under reduced pressure to obtain crude product. The crude product was purified by silica gel column chromatography (ethyl acetate/petroleum ether, v/v = 1/10-1/1), and the fractions with Rf = 0.3-0.4 were collected to yield **Compound 13-1** as white solid (1.603 g, yield 96.8%). ESI[M + H]⁺ = 416.1.

### 2. Preparation of Compound 13-2

At -20°C, LiOH·H₂O (177.7 mg, 4.23 mmol) and NaOH (154 mg, 3.85 mmol) were added successively to a solution of **13-1** (1.603 g, 3.85 mmol) in a mixture of MeOH/THF/H₂O (20 mL, v/v/v = 1/1/1). The mixture was allowed to warm to room temperature and stirred for 12 hours. After confirming reaction completion by TLC, the mixture was adjusted to pH 6-7 with 1 mol/L HCl in ice-bath and then extracted with ethyl acetate (3 × 50 mL). The organic phase was washed with brine, dried over anhydrous Na₂SO₄, filtered and concentrated under reduced pressure to obtain crude product. The crude product was purified by silica gel column chromatography (MeOH/CH₂Cl₂, v/v = 1/10), and the fractions with Rf = 0.2-0.3 were collected to yield **Compound 13-2** as white solid (1.54 g, yield 99.4%). ESI[M + H]⁺ = 402.1.

### 3. Preparation of Compound 13

**13-2** (100 mg, 0.249 mmol), DCC (77 mg, 0.373 mmol), and DMAP (46 mg, 0.377 mmol) were dissolved in dichloromethane (2 mL), and the mixture was stirred at room temperature for 15 minutes. To this mixture was then added 3-ethynylloxetan-3-ol (50 mg, 0.499 mmol), and was stirred at room temperature for 12 hours. After confirming reaction completion by TLC, the mixture was added methyl tert-butyl ether (2 mL). After stirring for 5 minutes, the mixture was filtered and the filtrate was concentrated under reduced pressure to obtain the crude product. The crude product was purified by preparative TLC (ethyl acetate/petroleum ether, v/v = 3/1), and the fractions with Rf = 0.3-0.4 were collected to yield **Compound 13** as white solid (39.0 mg, yield 32.4%). ESI[M + H]⁺ = 484.1.

¹H NMR (400 MHz, CDCl₃) δ 8.74 (s, 1H), 8.19 (d, *J =* 7.9 Hz, 1H), 8.04 - 8.02 (m, 1H), 7.69 (d, *J =* 8.7 Hz, 1H), 7.59 - 7.57 (m, 1H), 7.44 (s, 1H), 7.28 - 7.26 (m, 1H), 6.17 (dd, *J =* 17.4, 10.9 Hz, 1H), 5.29 (d, *J =* 17.4 Hz, 1H), 5.23 (d, *J =* 11.0 Hz, 1H), 4.78 (d, *J =* 7.1 Hz, 2H), 4.66 (dd, *J =* 7.4, 3.2 Hz, 2H), 3.76 (t, *J =* 6.8 Hz, 1H), 3.43 (s, 3H), 2.74 (t, *J =* 6.9 Hz, 2H), 2.69 - 2.59 (m, 1H), 2.56 - 2.47 (m, 1H).

### Example 11 Preparation of Compound 14-16

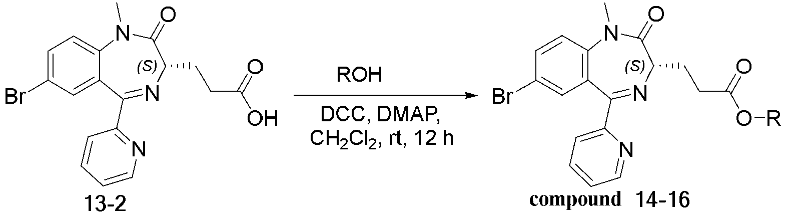

The preparation method of compound **14-16** are similar to compound **13** in Example 10, using **13-2** and the respective alcohol as starting materials to furnish the corresponding esters.

Compound **14:** 80 mg, ESI[M+H]⁺ =472.1_{∘}

¹H NMR (400 MHz, CDCl₃) δ 8.69 (d, *J =* 4.1 Hz, 1H), 8.18 (d, *J =* 7.8 Hz, 1H), 7.94 (t, *J =* 7.2 Hz, 1H), 7.68 (dd, *J =* 8.8, 2.1 Hz, 1H), 7.49 (d, *J =* 2.0 Hz, 2H), 7.24 (s, 1H), 4.71 (d, *J* = 7.1 Hz, 2H), 4.46 (d, *J =* 7.6 Hz, 2H), 3.74 - 3.72 (m, 1H), 3.42 (s, 3H), 2.71 - 2.43 (m, 4H), 1.65 (s, 3H).

Compound **15:** 102.7 mg, ESI[M+H]⁺ =496.0_{∘}

¹H NMR (400 MHz, CDCl₃) δ 8.65 (d, *J =* 4.2 Hz, 1H), 8.17 (d, *J =* 7.9 Hz, 1H), 7.86 (t, *J =* 7.3 Hz, 1H), 7.66 (dd, *J =* 8.8, 2.2 Hz, 1H), 7.52 (d, *J =* 2.2 Hz, 1H), 7.46 - 7.38 (m, 1H), 7.24 (d, *J* = 8.8 Hz, 1H), 5.58 (t, *J =* 6.6 Hz, 1H), 4.97 (d, *J =* 6.6 Hz, 2H), 4.76 (t, *J =* 7.0 Hz, 2H), 4.71 (t, *J =* 6.6 Hz, 2H), 3.73 - 3.68 (m, 1H), 3.40 (s, 3H), 2.72 - 2.65 (m, 2H), 2.63 - 2.55 (m, 1H), 2.53 - 2.45 (m, 1H).

Compound **16:** 65.6 mg, ESI[M+H]⁺ =458.1_{∘}

¹H NMR (400 MHz, CDCl₃) δ 8.69 (d, *J =* 4.4 Hz, 1H), 8.16 (d, *J =* 7.8 Hz, 1H), 7.93 (t, *J =* 7.5 Hz, 1H), 7.67 (dd, *J =* 8.8, 2.0 Hz, 1H), 7.49 - 7.47 (m, 2H), 7.26 - 7.25 (m, 1H), 5.52 - 5.36 (m, 1H), 4.85 (dd, *J* = 11.9, 6.7 Hz, 2H), 4.60 (t, *J* = 6.4 Hz, 2H), 3.75 - 3.72 (m, 1H), 3.41 (s, 3H), 2.71 (t, *J* = 7.1 Hz, 2H), 2.66 - 2.55 (m, 1H), 2.54 - 2.44 (m, 1H).

### Example 12 Preparation of Compound 17, 17E, 18, 19 and 19E

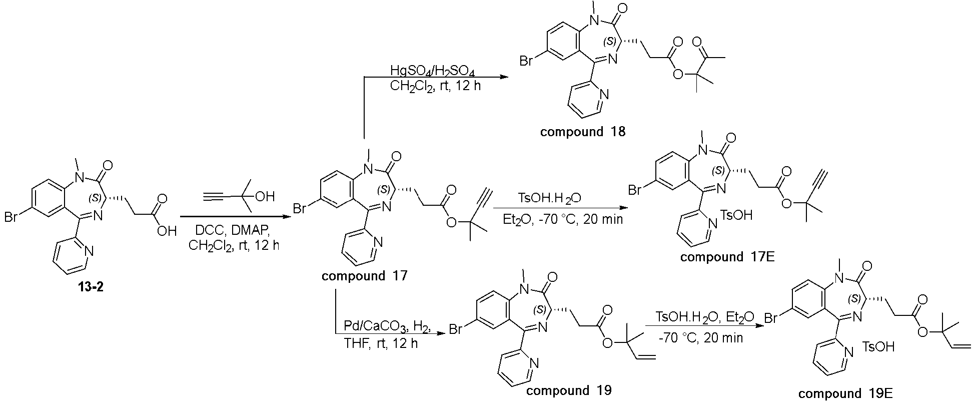

### 1. Preparation of Compound 17

**13-2** (1.0 g, 2.49 mmol), DCC (1.03 g, 4.99 mmol), and DMAP (610 mg, 4.99 mmol) were dissolved in dichloromethane (10 mL), and the mixture was stirred at room temperature for 15 minutes. The mixture was added 3-methylbut-3-yn-1-ol (420 mg, 4.99 mmol), and stirring was continued at room temperature for 12 hours. After confirming reaction completion by TLC, Methyl *tert*-butyl ether (10 mL) was added to the mixture, then, stirring for 5 minutes, the mixture was filtered and the filtrate was concentrated under reduced pressure to obtain the crude product. The crude product was purified by silica gel column chromatography (ethyl acetate/petroleum ether, v/v = 1/20-1/1), and the fractions with Rf = 0.3-0.4 were collected to yield **Compound 17** as white solid (621.0 mg, yield 53.3%). ESI[M + H]⁺ = 468.0.

¹H NMR (400 MHz, CDCl₃) δ 8.66 (d, *J =* 3.9 Hz, 1H), 8.20 (d, *J =* 7.7 Hz, 1H), 7.91 (t, *J =* 7.1 Hz, 1H), 7.65 (dd, *J =* 8.8, 2.1 Hz, 1H), 7.48 - 7.42 (m, 2H), 7.24 (d, *J =* 8.8 Hz, 1H), 3.75 (t, *J =* 6.5 Hz, 1H), 3.41 (s, 3H), 2.66 - 2.57 (m, 3H), 2.53 - 2.44 (m, 1H), 2.40 (s, 1H), 1.61 (s, 3H), 1.61 (s, 3H).

### 2. Preparation of Compound 17E

At -70°C, **Compound 17** (218 mg, 0.465 mmol) was dissolved in diethyl ether (10 mL), the solution of p-toluenesulfonic acid monohydrate (88.3 mg, 0.464 mmol) in acetone (0.5 mL) was added dropwise, then the mixture was stirred at -70°C for 20 minutes. The mixture was filtered, and the filter cake was dried under reduced pressure to give **Compound 17E** as yellow solid (258.3 mg, yield 86.6%). ESI[M + H]⁺ = 468.2.

¹H NMR (400 MHz, DMSO-d6) δ 8.62 (d, *J* = 4.1 Hz, 1H), 8.12 (d, *J =* 7.9 Hz, 1H), 8.03 (td, *J =* 7.7, 1.6 Hz, 1H), 7.82 (dd, *J =* 8.8, 2.4 Hz, 1H), 7.62 - 7.56 (m, 1H), 7.53 (t, *J =* 5.8 Hz, 2H), 7.47 (d, *J =* 8.1 Hz, 2H), 7.11 (d, *J* = 7.9 Hz, 2H), 3.71 (dd, *J =* 8.0, 5.5 Hz, 1H), 3.45 (s, 1H), 3.31 (s, 3H), 2.55 - 2.50 (m, 1H), 2.47 - 2.30 (m, 2H), 2.29 (s, 3H), 2.26 - 2.16 (m, 1H), 1.55 (s, 3H), 1.55 (s, 3H).

### 3. Preparation of Compound 18

**Compound 17** (310 mg, 0.662 mmol) was dissolved in dichloromethane (10 mL), mercury(II) sulfate/sulfuric acid/silica gel (310 mg) were added into the mixture. The mixture was stirred at room temperature for 12 hours. After confirming reaction completion by TLC, the mixture was neutralized with saturated aqueous sodium bicarbonate solution and extracted with dichloromethane (3 × 10 mL, washed with brine, dried over anhydrous Na₂SO₄, filtered and concentrated under reduced pressure to obtain crude product. The crude product was purified by preparative TLC (MeOH/CH₂Cl₂, v/v = 1/30), and the fractions with Rf = 0.3-0.4 were collected to yield **Compound 18** as white solid (284 mg, yield 88.2%). ESI[M + H]⁺ = 486.2.

¹H NMR (400 MHz, DMSO-*d6*) δ 8.61 - 8.56 (m, 1H), 8.12 (d, *J =* 7.9 Hz, 1H), 7.97 (td, *J =* 7.8, 1.7 Hz, 1H), 7.81 (dd, *J =* 8.9, 2.4 Hz, 1H), 7.57 - 7.50 (m, 2H), 7.48 (d, *J =* 2.4 Hz, 1H), 3.71 (dd, *J =* 8.3, 5.5 Hz, 1H), 3.31 (s, 3H), 2.66 - 2.51 (m, 2H), 2.39 - 2.30 (m, 1H), 2.28 - 2.14 (m, 1H), 1.96 (s, 3H), 1.34 (s, 3H), 1.32 (s, 3H).

### 4. Preparation of Compound 19

**Compound 17** (300 mg, 0.641 mmol) was dissolved in tetrahydrofuran (10 mL) at room temperature, Lindlar catalyst (300 mg, 5% Pd) was added into the mixture. The mixture was stirred under hydrogen atmosphere for 12 hours. After confirming reaction completion by TLC, the mixture was filtered and the filtrate was concentrated under reduced pressure to obtain the crude product. The crude product was purified by silica gel column chromatography (e MeOH/CH₂Cl₂, v/v = 1/30), and the fractions with Rf = 0.3-0.4 were collected to yield **Compound 19** as white solid (296 mg, yield 98.2%). ESI[M + H]⁺ = 470.2.

¹H NMR (400 MHz, DMSO-*d6*) δ 8.58 (d, *J =* 4.0 Hz, 1H), 8.11 (d, *J* = 7.9 Hz, 1H), 7.96 (td, *J =* 7.8, 1.8 Hz, 1H), 7.81 (dd, *J =* 8.8, 2.4 Hz, 1H), 7.54 - 7.51 (m, 2H), 7.48 (d, *J =* 2.4 Hz, 1H), 5.97 (dd, *J =* 17.5, 10.9 Hz, 1H), 5.08 (d, *J =* 17.5 Hz, 1H), 4.97 (dd, *J* = 10.9, 0.9 Hz, 1H), 3.67 (dd, *J* = 8.2, 5.6 Hz, 1H), 3.30 (s, 3H), 2.44 - 2.14 (m, 4H), 1.39 (s, 6H).

### 5. Preparation of Compound 19E

The preparation method of compound 19E is similar to Compound **17E,** it was prepared from 19 and p-toluenesulfonic acid monohydrate.

Compound **19E:** 265.1 mg, ESI[M+H]+=470.2.

¹H NMR (400 MHz, DMSO-*d6*) δ 8.62 (d, *J =* 4.3 Hz, 1H), 8.11 (d, *J =* 7.9 Hz, 1H), 8.05 - 8.02 (m, 1H), 7.83 (dd, *J* = 8.9, 2.4 Hz, 1H), 7.63 - 7.57 (m, 1H), 7.55 - 7.51 (m, 2H), 7.47 (d, *J =* 8.1 Hz, 2H), 7.11 (d, *J =* 7.8 Hz, 2H), 5.97 (dd, *J =* 17.5, 10.9 Hz, 1H), 5.12 - 5.05 (m, 1H), 4.97 (dd, *J =* 10.9, 0.9 Hz, 1H), 3.69 (dd, *J =* 7.9, 5.6 Hz, 1H), 3.30 (s, 3H), 2.48 - 2.30 (m, 3H), 2.29 (s, 3H), 2.24 - 2.19 (m, 1H), 1.39 (s, 6H).

### Example 13 Preparation of Compound 20

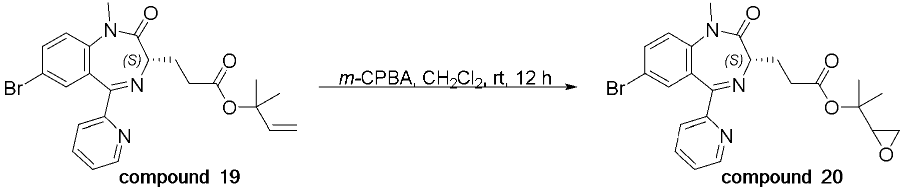

**Compound 19** (100 mg, 0.213 mmol) was dissolved in dichloromethane (5 mL). To the solution, m-CPBA (56 mg, 0.325 mmol) was added portionwise at room temperature, and the resulting mixture was stirred for 12 hours. After confirming reaction completion by TLC, the mixture was extracted with dichloromethane (3 × 15 mL). The organic phase was washed with brine, dried over anhydrous Na₂SO₄, filtered and concentrated under reduced pressure to obtain crude product. The crude product was purified by preparative TLC (ethyl acetate/petroleum ether, v/v = 1/1) to yield **Compound 20** as white solid (84.3 mg, yield 81.5%). ESI[M + H]⁺ = 486.2.

¹H NMR (400 MHz, DMSO-*d6*) δ 8.19 (d, *J =* 5.8 Hz, 1H), 7.79 - 7.73 (m, 2H), 7.63 - 7.49 (m, 3H), 7.22 (d, *J* = 2.3 Hz, 1H), 6.02 - 5.93 (m, 1H), 5.11 - 5.04 (m, 1H), 4.96 (dd, *J =* 10.9, 0.9 Hz, 1H), 3.66 (dd, *J =* 8.3, 5.5 Hz, 1H), 3.32 (s, 3H), 2.43 - 2.35 (m, 2H), 2.32 - 2.25 (m, 1H), 2.20 - 2.15 (m, 1H), 1.39 (s, 6H).

### Example 14 Preparation of Compound 21-23

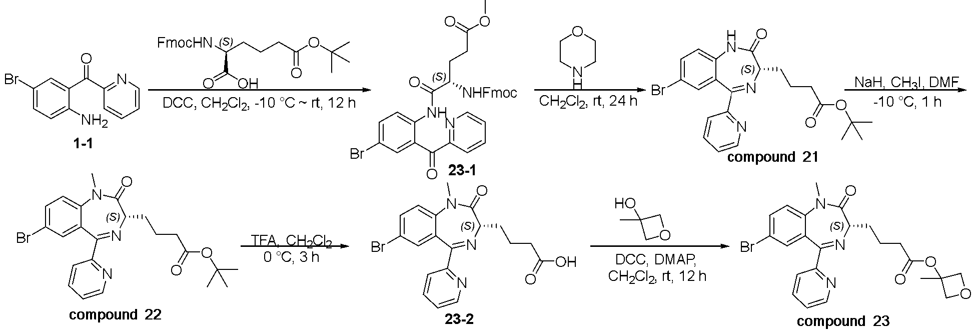

### 1. Preparation of Compound 23-1

1-1 (630 mg, 2.27 mmol) and **(S)-2-(((9H-fluoren-9-yl)methoxy)carbonyl)amino)hexanedioic acid 6-tert-butyl ester** (1 g, 2.28 mmol) were dissolved in dichloromethane (10 mL). At -10°C, a solution of DCC (705 mg, 3.42 mmol) in dichloromethane (2 mL) was added dropwise. The mixture was allowed to warm to room temperature and stirred for 12 hours. After confirming reaction completion by TLC, methyl tert-butyl ether (12 mL) was added to the mixture, and stirred for 5 minutes, then the mixture was filtered and concentrated under reduced pressure to obtain crude product. ESI[M + H]⁺ = 642.1.

### 2. Preparation of Compound 21

The crude product from the previous step was dissolved in a mixture of morpholine and dichloromethane (10 mL, v/v = 1:1). The mixture was stirred at room temperature for 24 hours. After confirming reaction completion by TLC, the mixture was concentrated under reduced pressure. The crude product was purified by silica gel column chromatography (MeOH/CH₂Cl₂, v/v = /100-1/10), TLC (MeOH/CH₂Cl₂, v/v =1/10) momnitoring was performed, and the fractions with Rf = 0.2-0.3 were collected to give **Compound 21** as white solid (294 mg, yield 28.2%). ESI[M + H]⁺ = 458.2.

¹H NMR (400 MHz, CDCl₃) δ 8.69 (s, 1H), 8.13 (brs, 1H), 8.11 (d, *J =* 7.7 Hz, 1H), 7.91 (t, *J* = 7.3 Hz, 1H), 7.62 (d, *J =* 8.5 Hz, 1H), 7.52 - 7.50 (m, 1H), 7.48 - 7.46 (m, 1H), 7.05 (d, *J =* 7.7 Hz, 1H), 3.63 (s, 1H), 2.33 - 2.17 (m, 2H), 1.93 - 1.80 (m, 2H), 1.76 - 1.58 (m, 2H), 1.44 (s, 9H).

### 3. Preparation of Compound 22

At -10°C, sodium hydride (25.2 mg, 60% dispersion in mineral oil, 0.63 mmol) was added cautiously to a solution of **21** (264 mg, 0.576 mmol) in dry DMF (3 mL). The mixture was stirred at this temperature for 30 minutes. Subsequently, methyl iodide (89.6 mg, 0.631 mmol) was added to the mixture, and stirring was continued for 1 hour. After confirming reaction completion by TLC, ice-water (10 mL) was added into the mixture and extracted with ethyl acetate (3 × 10 mL). The organic phase was washed with brine, dried over anhydrous Na₂SO₄, filtered and concentrated under reduced pressure to obtain crude product. The crude product was purified by preparative TLC (MeOH/CH₂Cl₂, v/v = 1/10), and the fractions with Rf = 0.3-0.4 were collected to yield **Compound 22** as white solid (152 mg, yield 55.9%). ESI[M + H]+ = 472.2.

¹H NMR (400 MHz, CDCl₃) δ 8.66 (s, 1H), 8.23 - 8.17 (m, 1H), 7.95 - 7.85 (m, 1H), 7.66 (d, *J* = 8.8 Hz, 1H), 7.52 - 7.40 (m, 2H), 7.24 (d, *J =* 9.0 Hz, 1H), 3.62 - 3.58 (m, 1H), 3.41 (s, 3H), 2.40 - 2.27 (m, 3H), 2.25 - 2.15 (m, 1H), 1.84 - 1.80 (m, 2H), 1.44 (s, 9H).

### 4. Preparation of Compound 23-2

At 0°C, trifluoroacetic acid (0.55 mL) was added to a solution of **22** (130 mg, 0.275 mmol) in dichloromethane (3.25 mL). The mixture was stirred at this temperature for 3 hours. After confirming reaction completion by TLC, the mixture was concentrated under reduced pressure. The residue was dissolved in water, and the pH of the solution was adjusted to 8-9 wit saturated aqueous sodium bicarbonate solution, and extracted with ethyl acetate (3 × 10 mL). The organic phase was washed with brine, dried over anhydrous Na₂SO₄, filtered and concentrated under reduced pressure to obtain crude product. The crude product was purified by preparative TLC (MeOH/CH₂Cl₂, v/v = 1/8), and the fractions with Rf = 0.3-0.4 were collected to yield **Compound 23-2** as white solid (95 mg, yield 82.9%). ESI[M + H]⁺ = 416.1.

### 5. Preparation of Compound 23

**23-2** (52 mg, 0.125 mmol), DCC (50.5 mg, 0.245 mmol), and DMAP (29.9 mg, 0.245 mmol) were dissolved in dichloromethane (2 mL), and the resulting mixture was stirred at room temperature for 15 minutes. To this mixture was then added 3-methyloxetan-3-ol (22.0 mg, 0.250 mmol), and stirring was continued at room temperature for 12 hours. After confirming reaction completion by TLC, methyl tert-butyl ether (2 mL) was added to the mixture, the mixture was stirred for 5 minutes, the mixture was filtered and the filtrate was concentrated under reduced pressure to obtain the crude product. The crude product was purified by preparative TLC (MeOH/CH₂Cl, v/v = 1/10), and the fractions with Rf = 0.3-0.4 were collected to yield **Compound 23** as white solid (26.7 mg, yield 43.9%). ESI[M + H]+ = 486.2.

¹H NMR (400 MHz, DMSO-d6) δ 8.58 (d, *J =* 4.5 Hz, 1H), 8.09 (d, *J =* 7.9 Hz, 1H), 7.96 (td, *J=* 7.8, 1.7 Hz, 1H), 7.80 (dd, *J =* 8.8, 2.4 Hz, 1H), 7.53 - 7.50 (m, 3H), 4.60 (d, *J =* 6.9 Hz, 2H), 4.40 (d, *J =* 7.5 Hz, 2H), 3.63 (dd, *J =* 8.2, 5.3 Hz, 1H), 3.30 (s, 3H), 2.39 (t, *J =* 7.4 Hz, 2H), 2.17 - 1.98 (m, 2H), 1.74 - 1.70 (m, 1H), 1.62 - 1.56 (m, 4H).

### Example 15 Preparation of Compound 24

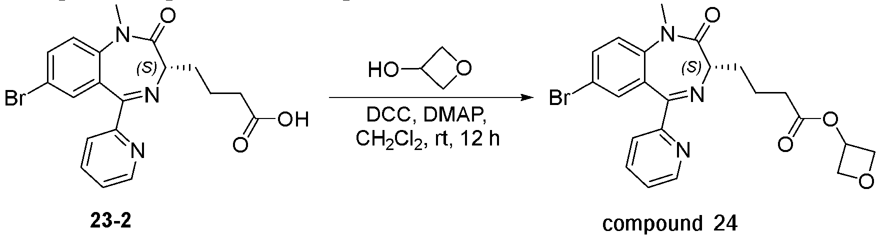

The preparation method of compound **24** is similar to compound 23 in example 14, it was prepared from **23-2** and oxetan-3-ol.

Compound**24:** 14.5 mg, ESI[M+H]⁺ =472.2.

¹H NMR (400 MHz, DMSO-*d6*) δ 8.58 (d, *J =* 4.0 Hz, 1H), 8.09 (d, *J =* 7.9 Hz, 1H), 7.96 (td, *J* = 7.7, 1.7 Hz, 1H), 7.80 (dd, *J =* 8.8, 2.4 Hz, 1H), 7.55 - 7.49 (m, 3H), 5.37 - 5.30 (m, 1H), 4.77 (t, *J* = 7.3 Hz, 2H), 4.51 - 4.41 (m, 2H), 3.63 (dd, *J=* 8.3, 5.5 Hz, 1H), 3.30 (s, 3H), 2.44 (t, *J=* 7.5 Hz, 2H), 2.12 - 2.00 (m, 2H), 1.80 - 1.70 (m, 1H), 1.65 - 1.55 (m, 1H).

### Example 16 Preparation of Compound 26-28

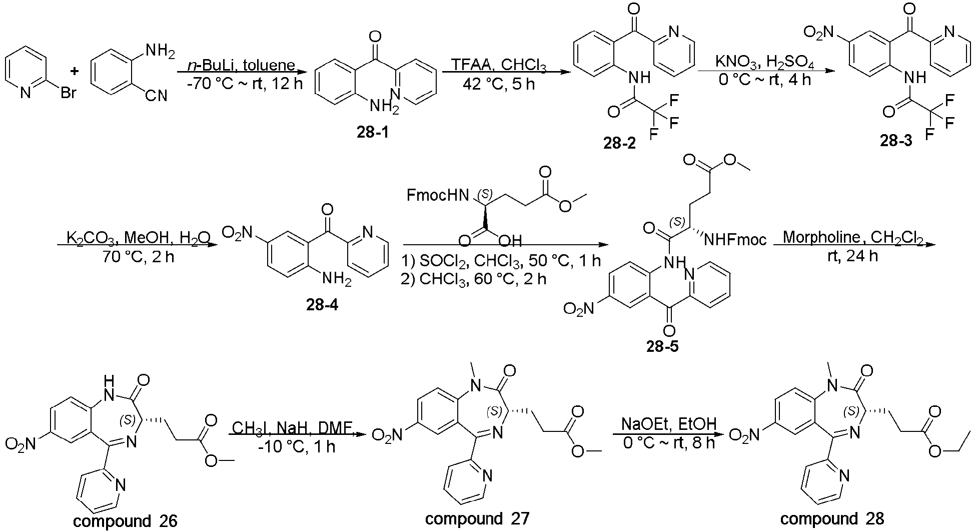

### 1. Preparation of Compound 28-1

At -70 °C, n-butyllithium (179 mL, 2.5 mol/L in hexane, 447.5 mmol) was added dropwise to solution of 2-bromopyridine (71.95 g, 455.4 mmol) in toluene (492 mL).The mixture was stirred at this temperature for 30 minutes. A solution of 2-aminobenzonitrile (23.5 g, 198.9 mmol) in toluene (163 mL) was then added dropwise to the mixture. The resulting mixture was allowed to warm to room temperature and stirred for 12 hours. After confirming reaction completion by TLC, ice-water was added to the mixture and extracted with ethyl acetate (3 × 200 mL). The organic phase was washed with brine, dried over anhydrous Na₂SO₄, filtered and concentrated under reduced pressure to obtain crude product. The crude product was purified by by silica gel column chromatography (ethyl acetate/petroleum ether, v/v = 1/50-1/5), TLC (ethyl acetate/petroleum ether, v/v = 1/3) momnitoring was performed, and the fractions with Rf = 0.4-0.6 were collected to give **Compound 28-1** as white solid (12.4 g, yield 31.4%). ESI[M + H]⁺ = 199.1.

### 2. Preparation of Compound 28-2

Trifluoroacetic anhydride (16.1 g, 76.7 mmol) was added to a solution of **28-1** (12.73 g, 64.2 mmol) in chloroform (408 mL). The mixture was stirred at 42°C for 5 hours. After confirming reaction completion by TLC, the mixture was concentrated under reduced pressure. The resulting residue was dissolved in water, and the aqueous solution was adjusted to pH 8-9 with saturated aqueous sodium bicarbonate solution, followed by extraction with ethyl acetate (3 × 100 mL). The organic phase was washed with brine, dried over anhydrous Na₂SO₄, filtered and concentrated under reduced pressure to obtain crude product. The crude product was purified by by silica gel column chromatography (ethyl acetate/petroleum ether, v/v = 1/50-1/5), TLC (ethyl acetate/petroleum ether, v/v = 1/3) momnitoring was performed, and the fractions with Rf = 0.4-0.6 were collected to give **Compound 28-2** as white solid (10.8 g, yield 57.2%). ESI[M + H]⁺ = 295.1.

### 3. Preparation of Compound 28-3

Potassium nitrate (5.565 g, 55.0 mmol) was dissolved in concentrated sulfuric acid (50 mL). This solution was added dropwise to a solution of **28-2** (10.6 g, 36.0 mmol) in concentrated sulfuric acid (50 mL) at 0 °C, while maintaining the temperature below 10°C. The mixture was allowed to warm to room temperature and stirred for 4 hours. After confirming reaction completion by TLC, ice-water was added into the mixture. The resulting aqueous mixture was adjusted to pH 8-9 with a 25% aqueous sodium hydroxide solution and then extracted with ethyl acetate (3 × 60 mL). The organic phase was washed with brine, dried over anhydrous Na₂SO₄, filtered and concentrated under reduced pressure to obtain crude product. The crude product was purified by by silica gel column chromatography (ethyl acetate/petroleum ether, v/v = 1/50-1/2), TLC (ethyl acetate/petroleum ether, v/v = 1/2) momnitoring was performed, and the fractions with Rf = 0.4-0.6 were collected to give **Compound 28-3** as yellow solid (9.5 g, yield 77.7%). ESI[M + H]⁺ =340.1.

### 4. Preparation of Compound 28-4

**28-3** (9.5 g, 28.0 mmol) and potassium carbonate (7.71 g, 55.8 mmol) were added to mixture of methanol and water (100 mL, v/v = 1:1). The mixture was stirred at 70°C for 2 hours. After confirming reaction completion by TLC, the mixture was concentrated under reduced pressure. Ice-water was added to the residue, and the solid was collected by suction filtration. The filter cake was washed with water (3 × [volume, if known, e.g., 10 mL]) and dried under vacuum to obtain **Compound 28-4** as a yellow solid (6.8 g, yield 99.8%). ESI [M + H]⁺ = 244.1.

### 5. Preparation of Compound 28-5

Thionyl chloride (30 mL) was added to a solution of Fmoc-L-glutamic acid 1-methyl ester (13.3 g, 34.7 mmol) in chloroform (100 mL). The mixture was stirred at 50°C for 1 hour. The mixture was concentrated under reduced pressure, and the resulting residue was dissolved in chloroform (50 mL). This solution was added dropwise to a mixture of **28-4** (4.21 g, 17.3 mmol) and pyridine (1.35 g, 17.1 mmol) in chloroform (40 mL). The resulting mixture was stirred at 60°C for 2 hours. After confirming reaction completion by TLC, the mixture was concentrated under reduced pressure to obtain crude product. The crude product was purified by by silica gel column chromatography (ethyl acetate/petroleum ether, v/v = 1/50-1/5), TLC (ethyl acetate/petroleum ether, v/v = 1/5) momnitoring was performed, and the fractions with Rf = 0.4-0.5 were collected to give **Compound 28-5** as white solid (7.7 g, yield 73.1%). ESI[M + H]⁺ =609.2.

### 6. Preparation of Compound 26

**Compound 28-5** (7.67 g, 12.6 mmol) was dissolved in a mixture of morpholine (13 mL) and dichloromethane (67 mL). The mixture was stirred at room temperature for 24 hours. After confirming reaction completion by TLC, ice-water (30 mL) was added to the mixture, and then extracted with CH₂Cl₂ (3 × 30 mL). The organic phase was washed with brine, dried over anhydrous Na₂SO₄, filtered and concentrated under reduced pressure to obtain crude product. The crude product was purified by by silica gel column chromatography (ethyl acetate/petroleum ether, v/v = 1/10-1/1), TLC (ethyl acetate/petroleum ether, v/v = 1/2) momnitoring was performed, and the fractions with Rf = 0.3-0.1 were collected to give **Compound 26** as white solid (2.2 g, yield 47.4%). ESI[M + H]⁺ =369.2.

¹H NMR (400 MHz, CDCl₃) δ 9.05 - 8.90 (m, 1H), 8.60 - 8.57 (m, 1H), 8.39 - 8.27 (m, 2H), 8.20 (d, *J* = 7.9 Hz, 1H), 7.90 - 7.86 (m, 1H), 7.46 - 7.41 (m, 1H), 7.25 - 7.14 (m, 1H), 3.83 - 3.78 (m, 1H), 3.67 - 3.66 (m, 3H), 2.75 - 2.65 (m, 2H), 2.65 - 2.44 (m, 2H).

### 7. Preparation of Compound 27

At -10 °C, sodium hydride (215 mg, 60% dispersion in mineral oil, 5.37 mmol) was added cautiously to a solution of **26** (1.8 g, 4.89 mmol) in dry DMF (20 mL). The mixture was stirred at this temperature for 30 minutes. Subsequently, methyl iodide (764 mg, 5.38 mmol) was added to the mixture, and stirring was continued for 1 hour. After confirming reaction completion by TLC, ice-water (40 mL) was added to the mixture, and then extracted with ethyl acetate (3 × 30 mL). The organic phase was washed with brine, dried over anhydrous Na₂SO₄, filtered and concentrated under reduced pressure to obtain crude product. The crude product was purified by by silica gel column chromatography (MeOH/CH₂Cl₂, v/v = 1/100-1/20), TLC (MeOH/CH₂Cl₂, v/v = 1/10) momnitoring was performed, and the fractions with Rf = 0.4-0.5 were collected to give **Compound 27** as white solid (1.505 g, yield 80.5%). ESI[M + H]⁺ = 383.2.

¹H NMR (400 MHz, CDCl₃) δ 8.62 (s, 1H), 8.41 (d, *J* = 9.0 Hz, 1H), 8.34 - 8.21 (m, 2H), 7.96 - 7.94 (m, 1H), 7.50 (d, *J* = 9.0 Hz, 2H), 3.79 - 3.77 (m, 1H), 3.65 (s, 3H), 3.49 (s, 3H), 2.71 - 2.58 (m, 3H), 2.57 - 2.46 (m, 1H).

### 8. Preparation of Compound 28

At 0°C, sodium ethoxide (219.5 mg, 3.23 mmol) was added to a solution of **27** (857 mg, 2.24 mmol) in a mixture of EtOH/THF (18 mL, v/v = 1:1). The mixture was allowed to warm to room temperature naturally and stirred for 8 hours. After confirming reaction completion by TLC, ice-water (20 mL) was added to the mixture, and then extracted with ethyl acetate (3 × 30 mL). The organic phase was washed with brine, dried over anhydrous Na₂SO₄, filtered and concentrated under reduced pressure to obtain crude product. The crude product was purified by by preparative TLC (ethyl acetate/petroleum ether, v/v = 1/1), and the fractions with Rf = 0.2-0.3 were collected to give **Compound 28** as white solid (242.9 mg, yield 27.3%). ESI[M + H]⁺ = 397.2.

¹H NMR (400 MHz, DMSO-d6) δ 8.57 (d, *J =* 4.7 Hz, 1H), 8.45 (dd, *J =* 9.2, 2.8 Hz, 1H), 8.22 (d, *J* = 2.7 Hz, 1H), 8.19 (d, *J =* 7.9 Hz, 1H), 8.00 (td, *J* = 7.8, 1.7 Hz, 1H), 7.79 (d, *J =* 9.2 Hz, 1H), 7.57 - 7.54 (m, 1H), 4.04 (q, *J =* 7.1 Hz, 2H), 3.80 - 3.74 (m, 1H), 3.38 (s, 3H), 2.59 - 2.53 (m, 1H), 2.45 - 2.35 (m, 2H), 2.31 - 2.21 (m, 1H), 1.14 (t, *J =* 7.1 Hz, 3H).

### Example 17 Preparation of Compound 29

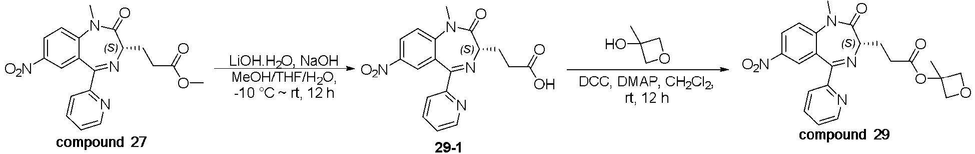

### 1. Preparation of Compound 29-1

At -10°C, LiOH·H₂O (64.3 mg, 1.53 mmol) and NaOH (55.7 mg, 1.39 mmol) were added successively to a solution of **27** (532 mg, 1.39 mmol) in a mixture of MeOH/THF/H₂O (7 mL, v/v/v = 1:1:1). The mixture was allowed to warm to room temperature and stirred for 12 hours. After confirming reaction completion by TLC, the mixture was adjusted to pH 6-7 with 1 mol/L dilute hydrochloric acid and extracted with ethyl acetate (3 × 30 mL). The organic phase was washed with brine, dried over anhydrous Na₂SO₄, filtered and concentrated under reduced pressure to obtain crude product. The crude product was purified by silica gel column chromatography ((MeOH/CH₂Cl₂, v/v = 1/100-1/10), TLC (MeOH/CH₂Cl₂, v/v = 1/10) momnitoring was performed, and the fractions with Rf = 0.3-0.4 were collected to give **Compound 29-1** as white solid (254 mg, yield 49.6%). ESI[M + H]⁺ = 369.1.

### 2. Preparation of Compound 29

**29-1** (50 mg, 0.136 mmol), DCC (39.6 mg, 0.192 mmol), and DMAP (23.5 mg, 0.192 mmol) were dissolved in dichloromethane (2 mL), and the resulting mixture was stirred at room temperature for 15 minutes. Then 3-methyloxetan-3-ol (12.5 mg, 0.142 mmol) was added and stirred at room temperature for 12 hours. After confirming reaction completion by TLC, methyl tert-butyl ether (2 mL) was added to the mixture. After stirring for 5 minutes, the mixture was filtered and the filtrate was concentrated under reduced pressure to afford the crude product. The crude product was purified by by preparative TLC (ethyl acetate/petroleum ether, v/v = 1/1), and the fractions with Rf = 0.2-0.3 were collected to give **Compound 29** as white solid (15.4 mg, yield 25.9%). ESI[M + H]⁺ = 439.2.

¹H NMR (400 MHz, CDCl₃) δ 8.67 (s, 1H), 8.44 (d, *J* = 8.9 Hz, 1H), 8.34 - 8.28 (m, 1H), 8.27 - 8.23 (m, 1H), 8.10 - 8.06 (m, 1H), 7.64 - 7.58 (m, 1H), 7.54 - 7.52 (m, 1H), 4.71 (d, *J =* 6.7 Hz, 2H), 4.47 (d, *J =* 7.5 Hz, 2H), 3.80 - 3.73 (m, 1H), 3.52 (s, 3H), 2.70 - 2.58 (m, 3H), 2.55 - 2.46 (m, 1H), 1.66 (s, 3H).

### Example 18 Preparation of Compound 30-32

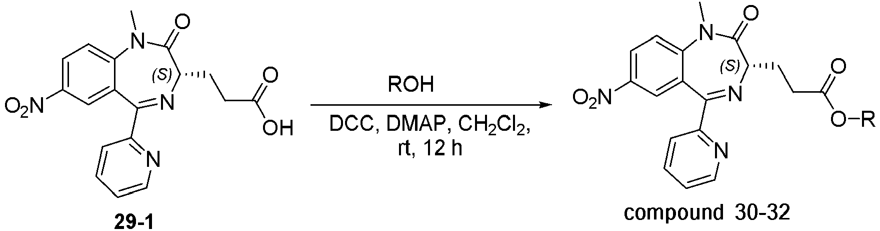

The preparation method of compound **30-32** are similar to compound **29 in example 17,** using **29-1** and the corresponding alcohols as starting materials to furnish the respective esters.

Compound**30**: 18.8 mg, ESI[M+H]⁺=451.2.

¹H NMR (400 MHz, CDCl₃) δ 8.64 (s, 1H), 8.42 (d, *J* = 8.7 Hz, 1H), 8.33 - 8.27 (m, 2H), 8.01 - 7.95 (m, 1H), 7.55 - 7.50 (m, 2H), 6.18 (dd, *J =* 17.3, 10.8 Hz, 1H), 5.31 - 5.22 (m, 2H), 4.79 (dd, *J =* 7.2, 3.0 Hz, 2H), 4.70 - 4.64 (m, 2H), 3.78 - 3.74 (m, 1H), 3.51 (s, 3H), 2.73 - 2.60 (m, 3H), 2.55 - 2.45 (m, 1H).

Compound**31**: 23.1 mg, ESI[M+H]⁺ = 425.2_{∘}

¹H NMR (400 MHz, CDCl₃) δ 8.62 (s, 1H), 8.41 (d, *J =* 9.0 Hz, 1H), 8.34 (s, 1H), 8.27 (d, *J* = 7.5 Hz, 1H), 7.96 - 7.90 (m, 1H), 7.54 - 7.42 (m, 2H), 5.46 - 5.38 (m, 1H), 4.86 (dd, *J =* 11.2, 6.6 Hz, 2H), 4.62 - 4.59 (m, 2H), 3.78 - 3.74 (m, 1H), 3.49 (s, 3H), 2.76 - 2.57 (m, 3H), 2.55 - 2.45 (m, 1H).

Compound**32**: 41.4 mg, ESI[M+H]⁺ = 435.1_{∘}

¹H NMR (400 MHz, DMSO-*d6*) δ 8.57 (ddd, *J* = 4.8, 1.7, 0.9 Hz, 1H), 8.44 (dd, *J =* 9.2, 2.8 Hz, 1H), 8.22 - 8.20 (m, 2H), 8.00 (td, *J* = 7.7, 1.8 Hz, 1H), 7.80 (d, *J =* 9.2 Hz, 1H), 7.55 (ddd, *J* = 7.5, 4.8, 1.1 Hz, 1H), 3.76 (dd, *J* = 7.8, 5.5 Hz, 1H), 3.44 (s, 1H), 3.39 (s, 3H), 2.49 - 2.29 (m, 3H), 2.27 - 2.21 (m, 1H), 1.56 (s, 3H), 1.55 (s, 3H).

### Example 19 Preparation of Compound 34-37

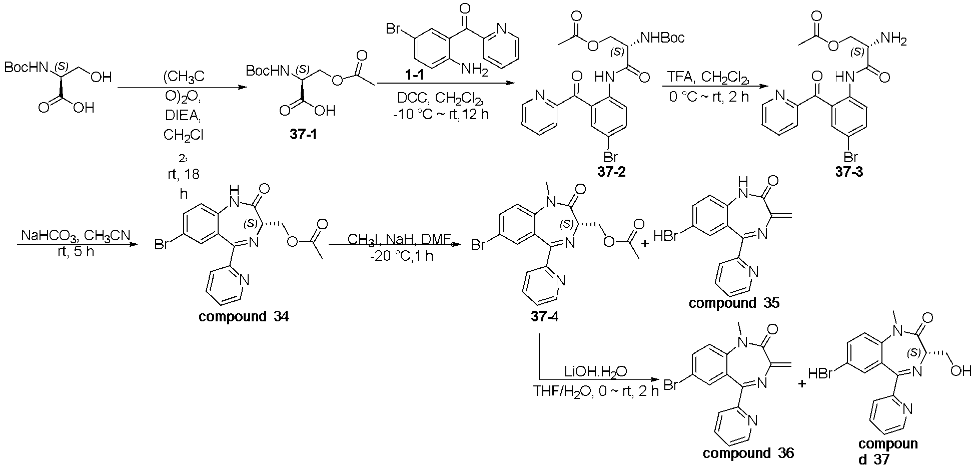

### 1. Preparation of Compound 37-1

BOC-L-Serine (18.0 g, 87.7 mmol) was dissolved in dichloromethane (180 mL), followed by the addition of DIEA (13.6 g, 105.2 mmol) and acetic anhydride (9.78 g, 95.8 mmol). The mixture was stirred at room temperature for 18 hours. After confirming reaction completion by TLC, ice-water (50 mL) was added to the mixture, and then extracted with CH₂Cl₂ (3 × 100 mL). The organic phase was washed with 1% dilute HCl, brine, and dried over anhydrous Na₂SO₄, filtered and concentrated under reduced pressure to obtain **Compound 37-1** as colorless oil (16.7 mg, yield 77.0%). ESI[M + H]⁺ = 248.1.

### 2. Preparation of Compound 37-2

To a solution of **1-1** (12.4 g, 44.7 mmol) and **37-1** (16.7 g, 67.5 mmol) in dichloromethane (100 mL) was added a solution of DCC (18.5 g, 89.7 mmol) in dichloromethane (40 mL) dropwise at -10 °C. The mixture was then allowed to warm to room temperature and stirred for 12 hours. After confirming reaction completion by TLC, ethyl tert-butyl ether (MTBE, 140 mL) was added, and the mixture was stirred for an additional 5 minutes. The mixture was filtered and concentrated under reduced pressure to obtain crude product. The crude product was purified by silica gel column chromatography (ethyl acetate/petroleum ether, v/v=1/20-1/2), TLC(ethyl acetate/petroleum ether, v/v =1/3) momnitoring was performed, and the fractions with Rf = 0.3-0.4 were collected to yield **Compound 37-2** as white solid (10.5 g, yield 46.3%). ESI[M+H]⁺= 506.1.

### 3. Preparation of Compound 37-3

Trifluoroacetic acid (10 mL) was added to a solution of **37-2** (10.5 g, 20.7 mmol) in dichloromethane (20 mL) at 0°C. The mixture was then allowed to warm to room temperature and stirred for 2 hours. After confirming reaction completion by TLC, The mixture was concentrated under reduced pressure to obtain crude product, and the crude product was carried forward to the next step without purification.

### 4. Preparation of Compound 34

The crude product from the previous step was dissolved in acetonitrile (100 mL). Sodium bicarbonate (52.0 g, 619 mmol) was added, and the mixture was stirred at room temperature for 5 hours. After confirming reaction completion by TLC, the mixture was filtered and concentrated under reduced pressure to obtain crude product. The crude product was purified by silica gel column chromatography (ethyl acetate/petroleum ether, v/v = 1/10-1/2), TLC (ethyl acetate/petroleum ether, v/v =1/1) momnitoring was performed, and the fractions with Rf = 0.2-0.3 were collected to yield **Compound 34** as white solid (10.5 g, two steps yield 78.3%). ESI[M + H]⁺ = 388.2.

¹H NMR (400 MHz, DMSO-d6) δ 10.85 (s, 1H), 8.57 (dd, *J =* 4.0, 0.8 Hz, 1H), 8.04 (d, *J =* 7.9 Hz, 1H), 7.97 (td, *J =* 7.7, 1.7 Hz, 1H), 7.75 (dd, *J =* 8.7, 2.3 Hz, 1H), 7.55 - 7.49 (m, 2H), 7.19 (d, *J =* 8.8 Hz, 1H), 4.72 - 4.58 (m, 2H), 3.93 (t, *J =* 6.4 Hz, 1H), 2.03 (s, 3H).

### 5. Preparation of Compound 37-4 and Compound 35

At -20 °C, sodium hydride (716 mg, 60% dispersion in mineral oil, 17.9 mmol) was slowly added to a solution of 34 (6.3 g, 16.2 mmol) in dry DMF (60 mL). The mixture was stirred at this temperature for 30 minutes. After which, methyl iodide (2.54 g, 17.9 mmol) was added, and stirring was continued at -20 °C for 1 hour. After confirming reaction completion by TLC, ice-water (120 mL) was added to the mixture, and then extracted with ethyl acetate (3 × 50 mL). The organic phase was washed with brine, and dried over anhydrous Na₂SO₄, filtered and concentrated under reduced pressure to obtain crude product, the crude product was purified by silica gel column chromatography (ethyl acetate/petroleum ether, v/v = 1/10-1/1), TLC (ethyl acetate/petroleum ether, v/v =1/1) momnitoring was performed, and the fractions with Rf = 0.2-0.3 were collected to yield **Compound 37-4** as white solid (2.86 g, yield 43.8%), **Compound 35** as yellow solid (62.8 g, yield 1.2%).

Compound **37-4:** ESI[M + H]⁺ = 402.1.

Compound **35:** ESI[M + H]⁺ = 328.2.

¹H NMR (400 MHz, DMSO-*d6*) δ 10.77 (s, 1H), 8.69 - 8.58 (m, 1H), 8.13 (d, *J =* 7.9 Hz, 1H), 8.01 (td, *J* = 7.8, 1.7 Hz, 1H), 7.71 (dd, *J =* 8.7, 2.3 Hz, 1H), 7.56 (ddd, *J =* 7.5, 4.8, 1.1 Hz, 1H), 7.42 (d, *J =* 2.3 Hz, 1H), 7.19 (d, *J =* 8.7 Hz, 1H), 5.19 (s, 1H), 4.99 (s, 1H).

### 6. Preparation of Compound 36 and Compound 37

At 0°C, lithium hydroxide monohydrate (LiOH·H₂O, 450 mg, 10.7 mmol) was added to a solution of 37-4 (2.86 g, 7.11 mmol) in THF/water (25 mL, v/v = 1:1). The mixture was then allowed to warm to room temperature and stirred for 2 hours. After confirming reaction completion by TLC, ice-water (10 mL) was added to the mixture, and then extracted with ethyl acetate (3 × 30 mL). The organic phase was washed with brine, and dried over anhydrous Na₂SO₄, filtered and concentrated under reduced pressure to obtain crude product, the crude product was purified by silica gel column chromatography (ethyl acetate/petroleum ether, v/v = 1/20-1/1), TLC (ethyl acetate/petroleum ether, v/v =1/1) momnitoring was performed, and the fractions with Rf = 0.2-0.3 were collected to yield **Compound 36** as yellow solid (1.2 g, yield 49.3%), **Compound 37** as colorless oil (1.01 g, yield 39.4%).

Compound **36:** ESI[M + H]⁺ = 342.2.

¹H NMR (400 MHz, DMSO-*d6*) δ 8.63 (d, *J =* 4.7 Hz, 1H), 8.19 (d, *J =* 7.9 Hz, 1H), 8.01 (td, *J=* 7.8, 1.7 Hz, 1H), 7.78 (dd, *J =* 8.8, 2.4 Hz, 1H), 7.61 - 7.55 (m, 1H), 7.50 (d, *J =* 2.4 Hz, 1H), 7.48 (d, *J* = 8.9 Hz, 1H), 5.10 (s, 1H), 4.92 (s, 1H), 3.32 (s, 3H).

Compound **37:** ESI[M + H]⁺ = 360.2.

¹H NMR (400 MHz, DMSO-d6) δ 8.58 (d, *J* = 4.0 Hz, 1H), 8.15 (d, *J =* 7.9 Hz, 1H), 7.96 (td, *J =* 7.8, 1.7 Hz, 1H), 7.82 (dd, *J =* 8.8, 2.4 Hz, 1H), 7.55 (d, *J =* 8.9 Hz, 1H), 7.53 - 7.50 (m, 2H), 4.71 (t, *J* = 5.8 Hz, 1H), 4.21 - 4.15 (m, 1H), 4.03 - 3.94 (m, 1H), 3.65 (t, *J =* 6.5 Hz, 1H), 3.30 (s, 3H).

### Example 20 Preparation of Compound 40

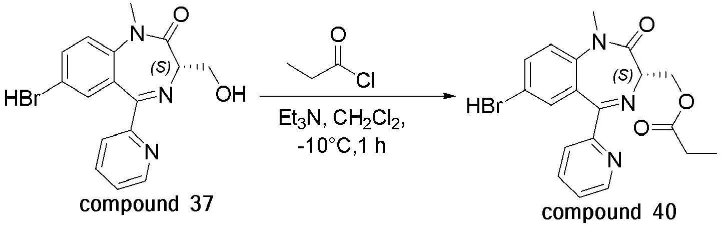

At 0°C, lithium hydroxide monohydrate (LiOH·H₂O, 450 mg, 10.7 mmol) was added to a solution of **37-4** (2.86 g, 7.11 mmol) in THF/water (25 mL, v/v = 1:1). The mixture was then allowed to warm to room temperature and stirred for 2 hours. After confirming reaction completion by TLC, ice-water (10 mL) was added to the mixture, and then extracted with CH₂Cl₂ (3 × 10 mL). The organic phase was washed with brine, and dried over anhydrous Na₂SO₄, filtered and concentrated under reduced pressure to obtain crude product, the crude product was purified by silica gel column chromatography (MeOH/CH₂Cl₂, v/v = 1/100-1/20), TLC (MeOH/CH₂Cl₂, v/v =1/10) momnitoring was performed, and the fractions with Rf = 0.4-0.5 were collected to yield **Compound 40** as white solid (73.5 mg, yield 63.3%). ESI[M + H]⁺ = 416.0.

¹H NMR (400 MHz, DMSO-d6) δ 8.59 (ddd, *J =* 4.8, 1.7, 0.9 Hz, 1H), 8.08 (d, *J =* 7.9 Hz, 1H), 7.97 (td, *J =* 7.7, 1.8 Hz, 1H), 7.83 (dd, *J =* 8.8, 2.4 Hz, 1H), 7.58 - 7.49 (m, 3H), 4.72 - 4.59 (m, 2H), 3.97 (t, *J =* 6.5 Hz, 1H), 3.32 (s, 3H), 2.31 (q, *J =* 7.5 Hz, 2H), 1.02 (t, *J =* 7.5 Hz, 3H).

### Example 21 Preparation of Compound 41-43

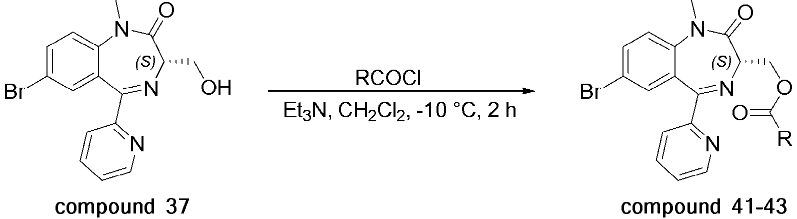

The preparation method of compound **41-43** are similar to compound **40** in **example 20,** using **37** and the corresponding acid chlorides as starting materials, the respective esters were prepared.

Compound **41:** 72.4 mg, ESI[M+H]⁺=430.0.

¹H NMR (400 MHz, DMSO-*d6*) δ 8.59 (d, *J =* 4.1 Hz, 1H), 8.06 (d, *J =* 7.9 Hz, 1H), 7.97 (td, *J* = 7.7, 1.7 Hz, 1H), 7.83 (dd, *J =* 8.9, 2.4 Hz, 1H), 7.59 - 7.50 (m, 3H), 4.71 - 4.59 (m, 2H), 3.99 (t, *J* = 6.5 Hz, 1H), 3.32 (s, 3H), 2.57 - 2.51 (m, 1H), 1.09 - 1.04 (m, 6H).

Compound **42:** 19.7 mg, ESI[M+H]⁺ = 464.0.

¹H NMR (400 MHz, DMSO-*d6*) δ 8.59 (d, *J =* 4.0 Hz, 1H), 8.07 (d, *J =* 7.9 Hz, 1H), 7.99 - 7.91 (m, 3H), 7.84 (dd, *J* = 8.9, 2.4 Hz, 1H), 7.65 (t, *J =* 7.4 Hz, 1H), 7.58 - 7.50 (m, 5H), 4.97 - 4.87 (m, 2H), 4.19 (t, *J =* 6.4 Hz, 1H), 3.34 (s, 3H).

Compound **43:** 54.1 mg, ESI[M+H]⁺ = 436.0.

¹H NMR (400 MHz, DMSO-*d6*) δ 8.59 (d, *J =* 4.0 Hz, 1H), 8.09 (d, *J =* 7.9 Hz, 1H), 7.97 (td, *J =* 7.7, 1.7 Hz, 1H), 7.84 (dd, *J =* 8.9, 2.4 Hz, 1H), 7.60 - 7.49 (m, 3H), 4.78 (d, *J =* 6.5 Hz, 2H), 4.40 (s, 2H), 4.05 - 4.00 (m, 1H), 3.32 (s, 3H).

### Example 22 Preparation of Compound 44 and 45

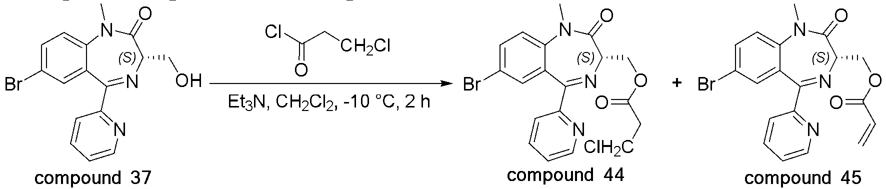

At -10°C, 3-chloropropionyl chloride (53.1 mg, 0.418 mmol) was added dropwise to a solution of **37** (100 mg, 0.278 mmol) and triethylamine (56.3 mg, 0.556 mmol) in dichloromethane (2 mL). The mixture was stirred at this temperature for 2 hours. After confirming reaction completion by TLC, ice-water (10 mL) was added to the mixture, and then extracted with CH₂Cl₂ (3 × 10 mL). The organic phase was washed with brine, and dried over anhydrous Na₂SO₄, filtered and concentrated under reduced pressure to obtain crude product, the crude product was purified by silica gel column chromatography (MeOH/CH₂Cl₂, v/v = 1/100-1/20), TLC (MeOH/CH₂Cl₂, v/v =1/10) momnitoring was performed to yield **Compound 45** as white solid (19.5 mg, yield 17.0%). Compound**44**: ESI[M + H]⁺ = 450.0.

¹H NMR (400 MHz, DMSO-*d6*) δ 8.62 - 8.57 (m, 1H), 8.10 (d, *J =* 7.9 Hz, 1H), 7.97 (td, *J =* 7.7, 1.8 Hz, 1H), 7.83 (dd, *J =* 8.9, 2.4 Hz, 1H), 7.59 - 7.49 (m, 3H), 4.77 - 4.70 (m, 2H), 3.99 (t, *J =* 6.4 Hz, 1H), 3.79 (t, *J =* 6.2 Hz, 2H), 3.32 (s, 3H), 2.83 (dd, *J =* 6.5, 5.8 Hz, 2H).

Compound**45:** ESI[M + H]⁺ = 414.0.

¹H NMR (400 MHz, DMSO-d6) δ 8.59 (ddd, *J =* 4.8, 1.6, 0.9 Hz, 1H), 8.07 (d, *J =* 7.9 Hz, 1H), 7.97 (td, *J* = 7.7, 1.8 Hz, 1H), 7.83 (dd, *J =* 8.8, 2.4 Hz, 1H), 7.60 - 7.49 (m, 3H), 6.35 (dd, *J* = 17.3, 1.6 Hz, 1H), 6.19 (dd, *J =* 17.3, 10.3 Hz, 1H), 5.95 (dd, *J =* 10.3, 1.6 Hz, 1H), 4.80 - 4.71 (m, 2H), 4.04 (t, *J =* 6.5 Hz, 1H), 3.32 (s, 3H).

### Example 23 Preparation of Compound 46

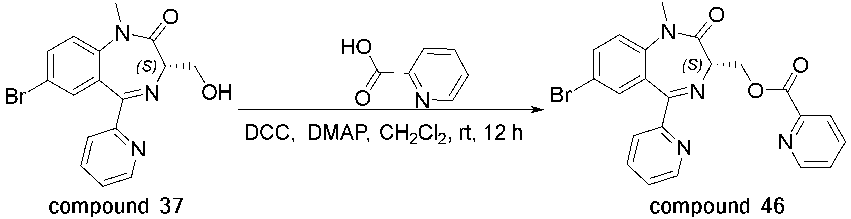

A mixture of picolinic acid (2-pyridinecarboxylic acid, 51.4 mg, 0.418 mmol), DCC (86.1 mg, 0.417 mmol), and DMAP (68 mg, 0.557 mmol) in dichloromethane (2 mL) was stirred at room temperature for 15 minutes. To this mixture was then added **37** (100 mg, 0.278 mmol), and stirring was continued at room temperature for 12 hours. After confirming reaction completion by TLC, methyl tert-butyl ether (MTBE, 2 mL) was added to the mixture. The mixture was stirred for 5 minutes and then filtered. The filtrate was concentrated under reduced pressure to afford the crude product, the crude product was purified by silica gel column chromatography (ethyl acetate/petroleum ether, v/v = 1/100-1/20), TLC (MeOH/CH₂Cl₂, v/v =1/10) momnitoring and the fractions with Rf = 0.4-0.5 were collected to yield **Compound 46** as white solid (39.1 mg, yield 30.3%). ESI[M + H]⁺ = 465.0.

¹H NMR (400 MHz, DMSO-d6) δ 8.72 - 8.70 (m, 1H), 8.63 - 8.58 (m, 1H), 8.10 - 8.03 (m, 2H), 7.99 - 7.93 (m, 2H), 7.85 (dd, *J =* 8.9, 2.4 Hz, 1H), 7.64 (ddd, *J =* 7.5, 4.7, 1.3 Hz, 1H), 7.59 - 7.56 (m, 2H), 7.53 (ddd, *J* = 7.5, 4.8, 1.2 Hz, 1H), 5.02 - 4.91 (m, 2H), 4.19 (t, *J =* 6.4 Hz, 1H), 3.33 (s, 3H).

### Example 24 Preparation of Compound 47-49 and Compound 51

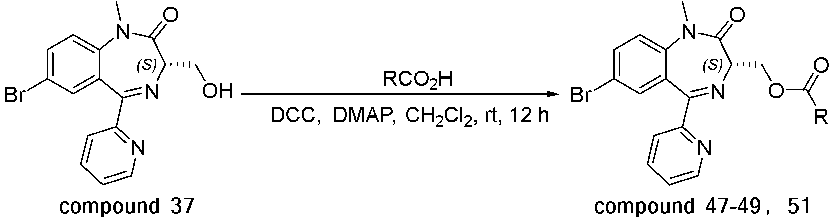

The preparation method of compound **47-49, 51** are similar to compound **46** in **example 23,** using compound 37 and the corresponding carboxylic acids as starting materials, the respective esters were prepared.

Compound **47:** 48.3 mg, ESI[M+H]⁺= 465.1.

¹H NMR (400 MHz, DMSO-d6) δ 8.80 - 8.79 (m, 2H), 8.62 - 8.57 (m, 1H), 8.07 (d, *J* = 7.9 Hz, 1H), 7.95 (td, *J* = 7.7, 1.8 Hz, 1H), 7.86 - 7.82 (m, 3H), 7.59 - 7.49 (m, 3H), 5.01 - 4.92 (m, 2H), 4.22 (t, *J =* 6.3 Hz, 1H), 3.34 (m, 3H).

Compound **48:** 72.3 mg, ESI[M+H]⁺= 465.1.

¹H NMR (400 MHz, DMSO-*d6*) δ 9.03 (s, 1H), 8.75 (d, *J =* 4.7 Hz, 1H), 8.53 (d, *J =* 4.1 Hz, 1H), 8.23 (d, *J =* 8.0 Hz, 1H), 8.01 (d, *J =* 8.0 Hz, 1H), 7.89 (t, *J =* 7.7 Hz, 1H), 7.81 - 7.75 (m, 1H), 7.52 - 7.45 (m, 4H), 4.96 - 4.83 (m, 2H), 4.16 (t, *J =* 6.3 Hz, 1H), 3.28 (s, 3H).

Compound **49:** 32.3 mg, ESI[M+H]⁺= 434.0.

¹H NMR (400 MHz, DMSO-*d6*) δ 8.61 - 8.57 (m, 1H), 8.09 - 8.02 (m, 1H), 8.02 - 7.94 (m, 1H), 7.84 (dd, *J =* 8.9, 2.4 Hz, 1H), 7.57 - 7.52 (m, 3H), 5.31 - 5.25 (m, 1H), 4.88 - 4.68 (m, 2H), 4.06 (dd, *J* = 9.6, 3.2 Hz, 1H), 3.32 (s, 3H), 1.51 - 1.42 (m, 3H).

Compound **51:** 22.7 mg, ESI[M+H]⁺= 426.1.

¹H NMR (400 MHz, DMSO-*d6*) δ 8.62 (d, *J =* 4.2 Hz, 1H), 8.09 (d, *J =* 7.9 Hz, 1H), 8.00 (td, *J* = 7.7, 1.7 Hz, 1H), 7.86 (dd, *J =* 8.9, 2.3 Hz, 1H), 7.63 - 7.50 (m, 3H), 4.83 - 4.71 (m, 2H), 4.06 (t, *J* = 6.3 Hz, 1H), 3.34 (s, 3H), 2.04 (s, 3H).

### Example 25 Preparation of Compound 53

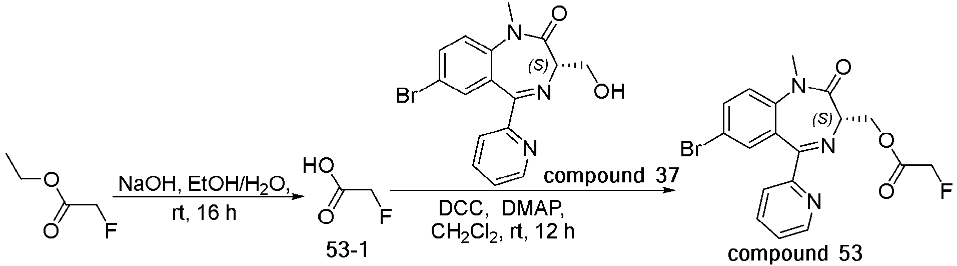

### 1. Preparation of Compound 53-1

Sodium hydroxide (232 mg, 5.8 mmol) was added to a solution of ethyl fluoroacetate (530 mg, 5.0 mmol) in EtOH/water (7 mL, v/v = 1:1). The mixture was stirred at room temperature for 16 hours. After confirming reaction completion by TLC, the mixture was adjusted to pH 6-7 with 1 M aqueous HCl. The mixture was then extracted with ethyl ether (3 × 30 mL). The organic phase was washed with brine, and dried over anhydrous Na2SO4, filtered and concentrated under reduced pressure to yield Compound 53-1 as colorless oil (150 mg, yield 38.5%).

### 2. Preparation of Compound 53

A mixture of **53-1** (116.1 mg, 1.49 mmol), DCC (592.4 mg, 2.87 mmol), and DMAP (526.0 mg, 4.31 mmol) in dichloromethane (2 mL) was stirred at room temperature for 15 minutes. To this mixture was then added **37** (50 mg, 0.139 mmol), and stirring was continued at room temperature for 12 hours. After confirming reaction completion by TLC, methyl tert-butyl ether (MTBE, 2 mL) was added to the mixture. After stirring for 5 minutes, the mixture was filtered, and the filtrate was concentrated under reduced pressure to afford the crude product. The filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 53** as white solid (5.6 mg, yield 9.6%). ESI[M + H]⁺ = 420.0.

¹H NMR (400 MHz, DMSO-*d6*) δ 8.62 (d, *J =* 4.0 Hz, 1H), 8.11 (d, *J* = 7.9 Hz, 1H), 8.00 (td, *J* = 7.7, 1.7 Hz, 1H), 7.86 (dd, *J* = 8.9, 2.4 Hz, 1H), 7.59 - 7.56 (m, 3H), 5.06 (d, *J* = 46.2 Hz, 2H), 4.84 - 4.81 (m, 2H), 4.06 (t, *J* = 6.4 Hz, 1H), 3.34 (s, 3H).

### Example 26 Preparation of Compound 54

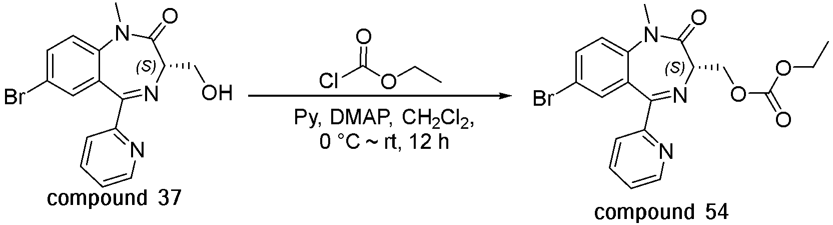

At 0°C, ethyl chloroformate (272.1 mg, 2.51 mmol) was added dropwise to a solution of **37** (300 mg, 0.833 mmol), pyridine (198.3 mg, 2.51 mmol), and DMAP (101.9 mg, 0.834 mmol) in dichloromethane (5 mL). The mixture was then allowed to warm to room temperature and stirred for 12 hours. After confirming reaction completion by TLC, ice-water (10 mL) was added to the mixture. The mixture was extracted with CH₂Cl₂ (3 × 10 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 54** as white solid (170.3 mg, yield 47.3%). ESI[M + H]⁺ = 432.2.

¹H NMR (400 MHz, DMSO-*d6*) δ 8.59 (d, *J* = 4.0 Hz, 1H), 8.07 (d, *J* = 7.9 Hz, 1H), 8.00 - 7.93 (m, 1H), 7.84 (dd, *J* = 8.9, 2.4 Hz, 1H), 7.58 - 7.51 (m, 3H), 4.77 - 4.73 (m, 1H), 4.70 - 4.65 (m, 1H), 4.13 (q, *J =* 7.1 Hz, 2H), 4.02 (t, *J=* 6.4 Hz, 1H), 3.31 (s, 3H), 1.20 (t, *J* = 7.1 Hz, 3H).

### Example 27 Preparation of Compound 55

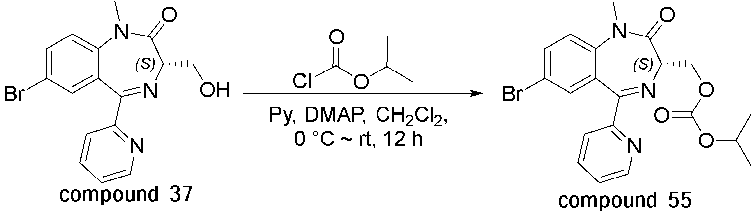

The preparation method of **compound 55** are similar to **compound 54** in **example 26,** using **37** and the corresponding Isopropyl chloroformate as starting materials, the respective esters were prepared.

**Compound 55:** 279.7 mg, ESI[M+H]⁺ = 446.0.

¹H NMR (400 MHz, DMSO-*d6*) δ 8.60 - 8.58 (m, 1H), 8.09 - 8.03 (m, 1H), 7.97 (td, *J* = 7.7, 1.8 Hz, 1H), 7.84 (dd, *J* = 8.9, 2.4 Hz, 1H), 7.58 - 7.50 (m, 3H), 4.81 - 4.63 (m, 3H), 4.01 (t, *J =* 6.4 Hz, 1H), 3.31 (s, 3H), 1.28 - 1.20 (m, 6H).

### Example 28 Preparation of Compound 56 and Compound 57

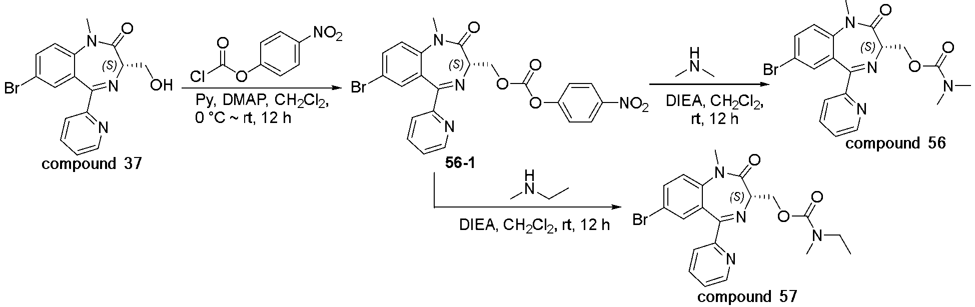

### 1. Preparation of Compound 56-1

At 0°C, p-nitrophenyl chloroformate (505.3 mg, 2.51 mmol) was added to a solution of **37** (300 mg, 0.833 mmol), pyridine (193.1 mg, 2.44 mmol), and DMAP (102 mg, 0.835 mmol) in dichloromethane (5 mL). The mixture was then allowed to warm to room temperature and stirred for 12 hours. After confirming reaction completion by TLC, ice-water (10 mL) was added to the mixture. The mixture was extracted with CH₂Cl₂ (3 × 10 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.3-0.4 were collected to yield **Compound 56-1** as white solid (272.8 mg, yield 62.4%). ESI[M + H]⁺ = 525.1.

### 2. Preparation of Compound 56

Dimethylamine (0.095 mL, 2 mol/L in THF, 0.19 mmol) was added to a solution of **56-1** (100 mg, 0.19 mmol) and DIEA (24.6 mg, 0.19 mmol) in dichloromethane (2 mL). The mixture was stirred at room temperature for 12 hours. After confirming reaction completion by TLC, ice-water (10 mL) was added to the mixture. The mixture was extracted with CH₂Cl₂ (3 × 10 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.3-0.4 were collected to yield **Compound 56** as white solid (34.8 mg, yield 42.4%). ESI[M + H]⁺ = 431.1.

¹H NMR (400 MHz, DMSO-*d6*) δ 8.61 (d, *J=* 4.1 Hz, 1H), 8.09 (d, *J =* 7.9 Hz, 1H), 7.99 (td, *J =* 7.7, 1.7 Hz, 1H), 7.85 (dd, *J* = 8.8, 2.4 Hz, 1H), 7.58 (d, *J* = 8.9 Hz, 1H), 7.56 - 7.54 (m, 2H), 4.69 (dd, *J =* 10.9, 5.9 Hz, 1H), 4.60 (dd, *J =* 10.8, 7.1 Hz, 1H), 3.97 (t, *J* = 6.5 Hz, 1H), 3.34 (s, 3H), 2.82 (s, 6H).

### 3. Preparation of Compound 57

The preparation method of **compound 57** are similar to **compound 56** in **example 28,** using **Compound 56-1 and N-ethylmethylamine** as starting materials.

Compound **57:** 62.1 mg, ESI[M+H]⁺ = 445.1.

¹H NMR (400 MHz, DMSO-*d6*) δ 8.61 - 8.56 (m, 1H), 8.07 (d, *J =* 7.9 Hz, 1H), 8.00 - 7.93 (m, 1H), 7.83 (dd, *J* = 8.8, 2.4 Hz, 1H), 7.56 (d, *J =* 8.9 Hz, 1H), 7.53 - 7.52 (m, 2H), 4.67 - 4.60 (m, 2H), 3.95 (t, *J* = 6.5 Hz, 1H), 3.32 (s, 3H), 3.25 - 3.15 (m, 2H), 2.78 (s, 3H), 1.00 (t, *J* = 7.0 Hz, 3H).

### Example 29 Preparation of Compound 58 and Compound 61

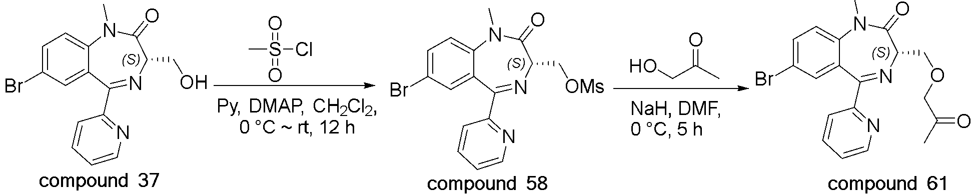

### 1. Preparation of Compound 58

At 0°C, methanesulfonyl chloride (572.5 mg, 5.0 mmol) was added dropwise to a solution of 37 (359 mg, 0.997 mmol), pyridine (395 mg, 4.99 mmol), and DMAP (122 mg, 0.999 mmol) in dichloromethane (10 mL). The mixture was then allowed to warm to room temperature and stirred for 12 hours. After confirming reaction completion by TLC, ice-water (10 mL) was added to the mixture. The mixture was extracted with CH₂Cl₂ (3 × 10 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 58** as white solid (190 mg, yield 43.5%). ESI[M + H]⁺ = 438.0.

¹H NMR (400 MHz, CDCl₃) δ 8.65 (d, *J =* 3.8 Hz, 1H), 8.16 (d, *J* = 7.9 Hz, 1H), 7.88 - 7.84 (m, 1H), 7.69 (dd, *J* = 8.8, 2.2 Hz, 1H), 7.54 (d, *J* = 2.3 Hz, 1H), 7.42 (dd, *J =* 6.9, 4.9 Hz, 1H), 7.28 - 7.25 (m, 1H), 5.08 (dd, *J* = 10.1, 7.0 Hz, 1H), 4.88 (dd, *J* = 10.1, 6.1 Hz, 1H), 4.05 (t, *J* = 6.5 Hz, 1H), 3.42 (s, 3H), 3.16 (s, 3H).

### 2. Preparation of Compound 61

At 0°C, sodium hydride (47.2 mg, 60% dispersion in mineral oil, 1.18 mmol) was slowly added to a solution of hydroxyacetone (88.6 mg, 1.20 mmol) in dry DMF (3 mL). The mixture was stirred at this temperature for 30 minutes. **Compound 58** (172 mg, 0.392 mmol) was then added to the mixture, and stirring was continued at 0 °C for 5 hours. After confirming reaction completion by TLC, ice-water (10 mL) was added to the mixture. The mixture was extracted with ethyl acetate (3 × 10 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 61** as white solid (8.2 mg, yield 5.0%). ESI[M + H]⁺ = 416.0.

¹H NMR (400 MHz, DMSO-*d6*) δ 8.60 - 8.56 (m, 1H), 7.98 - 7.97 (m, 2H), 7.82 (dd, *J* = 8.8, 2.4 Hz, 1H), 7.56 - 7.50 (m, 2H), 7.46 (d, *J* = 2.4 Hz, 1H), 5.26 (d, *J* = 5.3 Hz, 1H), 4.17 (dd, *J =* 11.5, 5.7 Hz, 1H), 3.86 (t, *J* = 6.9 Hz, 1H), 3.31 (s, 3H), 2.55 - 2.50 (m, 1H), 2.43 - 2.38 (m, 1H), 2.19 (s, 3H).

### Example 30 Preparation of Compound 62, 63 66-69

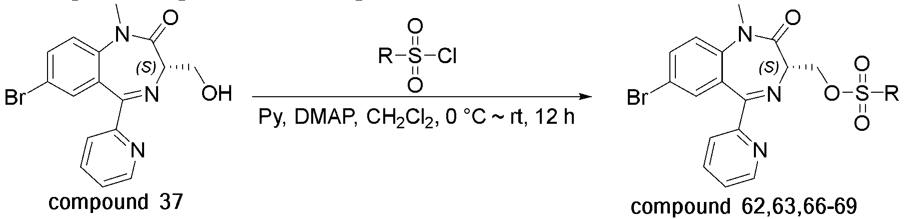

The preparation method of **compound 58** are similar to **compound 62, 63, 66-69** in example **29,** using **Compound 37** and **Sulfonyl chloride** as starting materials.

Compound **62:** 18.5 mg, ESI[M+H]⁺ = 452.0.

¹H NMR (400 MHz, DMSO-*d6*) δ 8.62 - 8.58 (m, 1H), 8.10 (d, *J* = 7.9 Hz, 1H), 7.99 (td, *J* = 7.7, 1.7 Hz, 1H), 7.84 (dd, *J* = 8.8, 2.4 Hz, 1H), 7.59 - 7.50 (m, 3H), 4.82 - 4.73 (m, 2H), 4.11 (dd, *J* = 7.0, 5.7 Hz, 1H), 3.47 - 3.41 (m, 2H), 3.32 (s, 3H), 1.27 (t, *J* = 7.3 Hz, 3H).

Compound **63:** 15.8 mg, ESI[M+H]⁺ = 466.0.

¹H NMR (400 MHz, DMSO-*d6*) δ 8.58 (d, *J =* 4.0 Hz, 1H), 8.07 (d, *J =* 7.9 Hz, 1H), 7.97 (td, *J* = 7.7, 1.7 Hz, 1H), 7.82 (dd, *J* = 8.9, 2.4 Hz, 1H), 7.59 - 7.47 (m, 3H), 4.83 - 4.69 (m, 2H), 4.09 (t, *J =* 6.3 Hz, 1H), 3.78 - 3.65 (m, 1H), 3.30 (s, 3H), 1.32 - 1.28 (m, 6H).

Compound **66:** 17.1 mg, ESI[M+H]⁺ = 500.0.

¹H NMR (400 MHz, DMSO-*d6*) δ 8.60 (d, *J* = 4.1 Hz, 1H), 8.02 - 7.96 (m, 3H), 7.92 (d, *J* = 7.9 Hz, 1H), 7.88 - 7.79 (m, 2H), 7.71 (t, *J* = 7.7 Hz, 2H), 7.58 - 7.50 (m, 3H), 4.73 - 4.60 (m, 2H), 4.02 (dd, *J =* 7.2, 5.4 Hz, 1H), 3.29 (s, 3H).

Compound **67:** 26.6 mg, ESI[M+H]⁺ = 534.0.

¹H NMR (400 MHz, DMSO-*d6*) δ 8.60 (d, *J* = 4.2 Hz, 1H), 7.98 - 7.96 (m, 3H), 7.90 (d, *J* = 7.9 Hz, 1H), 7.85 (dd, *J* = 8.9, 2.4 Hz, 1H), 7.79 - 7.77 (m, 2H), 7.60 - 7.51 (m, 3H), 4.73 - 4.63 (m, 2H), 4.08 - 3.98 (m, 1H), 3.29 (s, 3H).

Compound **68:** 80.3 mg, ESI[M+H]⁺ = 534.1.

¹H NMR (400 MHz, DMSO-*d6*) δ 8.60 (d, *J* = 4.6 Hz, 1H), 8.14 (d, *J* = 8.0 Hz, 1H), 8.04 - 7.97 (m, 2H), 7.85 (dd, *J* = 8.9, 2.4 Hz, 1H), 7.79 - 7.78 (m, 2H), 7.66 - 7.59 (m, 1H), 7.58 - 7.53 (m, 2H), 7.51 (d, *J* = 2.3 Hz, 1H), 4.79 - 4.69 (m, 2H), 4.04 (dd, *J* = 7.7, 4.9 Hz, 1H), 3.28 (s, 3H).

Compound **69:** 106.0 mg, ESI[M+H]⁺ = 534.2.

¹H NMR (400 MHz, DMSO-*d6*) δ 8.60 (d, *J =* 4.3 Hz, 1H), 8.03 - 8.00 (m, 1H), 7.98 - 7.96 (m, 2H), 7.94 - 7.88 (m, 2H), 7.85 (dd, *J* = 8.9, 2.4 Hz, 1H), 7.73 (t, *J* = 8.0 Hz, 1H), 7.57 - 7.52 (m, 2H), 7.51 (d, *J* = 2.4 Hz, 1H), 4.77 - 4.66 (m, 2H), 4.04 (dd, *J* = 7.3, 5.2 Hz, 1H), 3.29 (s, 3H).

### Example 31 Preparation of Compound 71

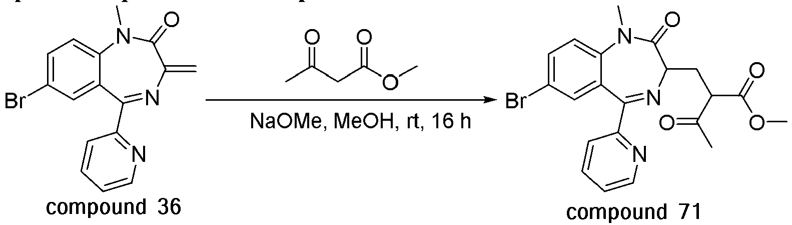

Methyl acetoacetate (68 mg, 0.586 mmol) was added to a solution of sodium methoxide (32 mg, 0.592 mmol) in methanol (3 mL). The mixture was stirred at room temperature for 30 minutes. Compound **36** (200 mg, 0.584 mmol) was then added, and stirring was continued at room temperature for 16 hours. After confirming reaction completion by TLC, the mixture was adjusted to pH 6-7 with 1 M aqueous HCl in an ice bath. The resulting mixture was then extracted with ethyl acetate (3 × 50 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.2-0.3 were collected to yield **Compound 71** as white solid (113.0 mg, yield 42.2%). ESI[M + H]⁺ = 458.2.

¹H NMR (400 MHz, DMSO-*d6*) δ 8.58 (d, *J* = 4.6 Hz, 1H), 8.05 - 7.96 (m, 2H), 7.81 (dd, *J* = 8.6, 2.2 Hz, 1H), 7.56 - 7.50 (m, 2H), 7.48 (dd, *J* = 9.5, 2.4 Hz, 1H), 3.95 - 3.91 (m, 1H), 3.76 - 3.71 (m, 1H), 3.67 (s, 1.5H), 3.61 (s, 1.5H), 3.30 (s, 3H), 2.68 - 2.57 (m, 1H), 2.45 - 2.40 (m, 1H), 2.27 (s, 1.5H), 2.20 (s, 1.5H).

### Example 32 Preparation of Compound 72

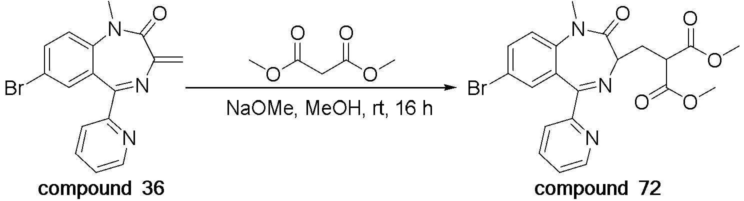

The preparation method of **compound 72** are similar to **compound 71** in **example 31,** using **Compound 36** and **Dimethyl malonate** as starting materials.

Compound**72**: 165.4 mg, ESI[M+H]⁺ = 474.2.

¹H NMR (400 MHz, DMSO-*d6*) δ 8.58 (d, *J* = 4.4 Hz, 1H), 8.04 (d, *J* = 7.8 Hz, 1H), 8.00 - 7.97 (m, 1H), 7.82 (dd, *J* = 8.8, 2.4 Hz, 1H), 7.56 - 7.50 (m, 2H), 7.49 (d, *J=* 2.4 Hz, 1H), 3.85 - 3.73 (m, 2H), 3.67 (s, 3H), 3.60 (s, 3H), 3.30 (s, 3H), 2.71 - 2.62 (m, 1H), 2.50 - 2.45 (m, 1H).

### Example 33 Preparation of Compound 73

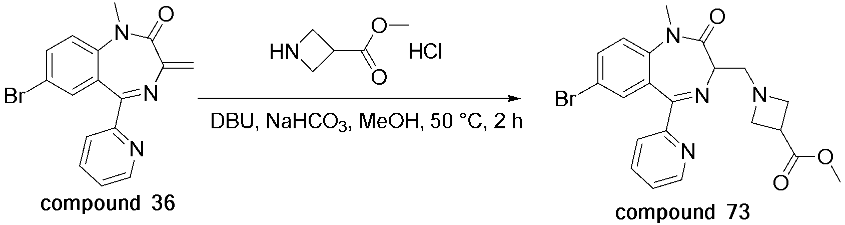

A mixture of **Compound 36** (95 mg, 0.278 mmol), methyl azetidine-3-carboxylate hydrochloride (63.3 mg, 0.418 mmol), DBU (46.5 mg, 0.305 mmol), and sodium bicarbonate (44.3 mg, 0.527 mmol) in methanol (2 mL) was stirred at 50 °C for 2 hours. After confirming reaction completion by TLC, ice-water (10 mL) was added to the mixture. The resulting mixture was then extracted with ethyl acetate (3 × 50 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by preparative TLC (MeOH/CH₂Cl₂, v/v = 1/30), and the fractions with Rf = 0.3-0.4 were collected to yield **Compound 73** as white solid (71.5 mg, yield 56.3%). ESI[M + H]⁺ = 457.1.

¹H NMR (400 MHz, DMSO-*d6*) δ 8.58 (s, 1H), 8.04 - 7.98 (m, 2H), 7.81 (d, *J =* 8.3 Hz, 1H), 7.53 - 7.49 (m, 3H), 3.62 (s, 3H), 3.60 - 3.37 (m, 4H), 3.31 - 3.29 (m, 1H), 3.28 (s, 3H), 3.22 - 3.05 (m, 3H).

### Example 34 Preparation of Compound 74 and Compound 75

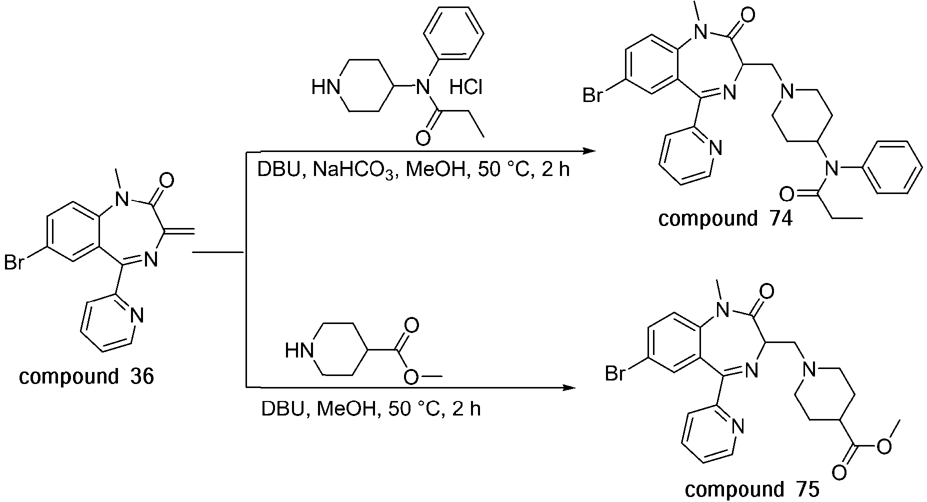

The preparation method of **compound 74 and compound 75** are similar to **compound 73 in example 33,** using **Compound 36** and **the respective amines (or their salts)** as starting materials.

Compound **74:** 121.2 mg, ESI[M+H]⁺ = 574.2.

¹H NMR (400 MHz, DMSO-*d6*) δ 8.80 - 8.20 (m, 1H), 8.01 - 7.71 (m, 3H), 7.65 - 7.29 (m, 6H), 7.21 - 7.10 (m, 2H), 4.57 - 4.37 (m, 1H), 3.32 - 3.30 (m, 1H), 3.28 (s, 3H), 3.20 - 2.80 (m, 4H), 2.48 - 2.37 (m, 1H), 2.30 - 1.97 (m, 1H), 1.81 - 1.79 (m, 2H), 1.69 - 1.51 (m, 2H), 1.50 - 1.28 (m, 1H), 1.20 - 1.04 (m, 1H), 0.86 (t, *J* = 7.4 Hz, 3H).

Compound **75:** 70.3 mg, ESI[M+H]⁺ = 485.1.

¹H NMR (400 MHz, DMSO-*d6*) δ 8.58 (d, *J* = 4.6 Hz, 1H), 8.05 (d, *J =* 7.9 Hz, 1H), 7.99 - 7.95 (m, 1H), 7.85 - 7.78 (m, 1H), 7.59 - 7.49 (m, 3H), 3.78 - 3.75 (m, 1H), 3.58 (s, 3H), 3.30 (s, 3H), 3.10 - 2.95 (m, 1H), 2.92 - 2.75 (m, 2H), 2.42 - 2.23 (m, 2H), 2.22 - 1.95 (m, 2H), 1.82 - 1.62 (m, 2H), 1.57 - 1.38 (m, 2H).

### Example 35 Preparation of Compound 76 and Compound 77

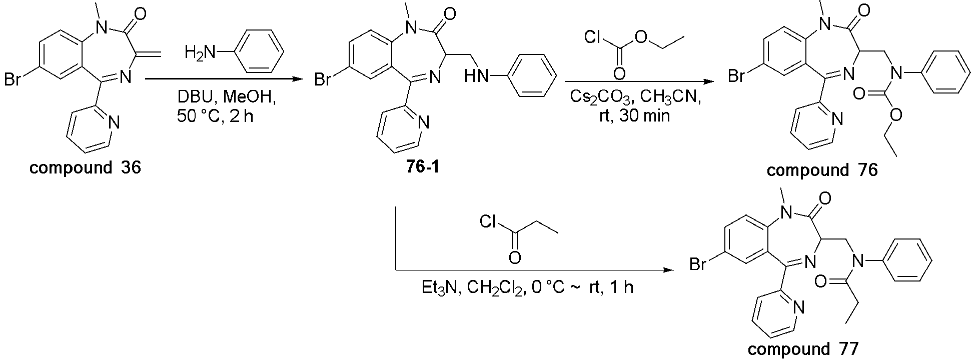

### 1. Preparation of Compound 76-1

A mixture of **Compound 36** (170 mg, 0.497 mmol), aniline (69.75 mg, 0.749 mmol), and DBU (83.6 mg, 0.549 mmol) in methanol (2 mL) was stirred at 50 °C for 2 hours. After confirming reaction completion by TLC, ice-water (10 mL) was added to the mixture. The resulting mixture was then extracted with ethyl acetate (3 × 50 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by preparative TLC (MeOH/CH₂Cl₂, v/v = 1/30), and the fractions with Rf = 0.3-0.4 were collected to yield **Compound 76-1** as white solid (198.6 mg, yield 91.8%). ESI[M + H]⁺ = 435.1.

### 2. Preparation of Compound 76

Ethyl chloroformate (45 mg, 0.415 mmol) was added to a solution of 76-1 (90.1 mg, 0.207 mmol) and cesium carbonate (135 mg, 0.414 mmol) in acetonitrile (2 mL). The mixture was stirred at room temperature for 30 minutes. After confirming reaction completion by TLC, ice-water (10 mL) was added to the mixture. The resulting mixture was then extracted with ethyl acetate (3 × 50 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by preparative TLC (MeOH/CH₂Cl₂, v/v = 1/30), and the fractions with Rf = 0.3-0.4 were collected to yield **Compound 76** as white solid (35.66 mg, yield 34.0%). ESI[M + H]⁺ = 507.1.

¹H NMR (400 MHz, DMSO-*d6*) δ 8.59 - 8.53 (m, 1H), 7.90 (td, *J* = 7.8, 1.8 Hz, 1H), 7.80 (dd, *J* = 8.8, 2.4 Hz, 1H), 7.71 (d, *J* = 7.9 Hz, 1H), 7.53 - 7.49 (m, 2H), 7.48 (d, *J* = 2.3 Hz, 1H), 7.38 - 7.32 (m, 2H), 7.31 - 7.29 (m, 2H), 7.25 - 7.21 (m, 1H), 4.48 - 4.34 (m, 1H), 4.29 (dd, *J* = 14.2, 3.8 Hz, 1H), 4.07 - 3.95 (m, 2H), 3.90 (dd, *J =* 9.3, 3.8 Hz, 1H), 3.28 (s, 3H), 1.04 (t, *J* = 7.1 Hz, 3H).

### 3. Preparation of Compound 77

At 0°C, propionyl chloride (28.2 mg, 0.305 mmol) was added dropwise to a solution of 76-1 (88.5 mg, 0.203 mmol) and triethylamine (41.1 mg, 0.406 mmol) in dichloromethane (2 mL). The mixture was then allowed to warm to room temperature and stirred for 1 hour. After confirming reaction completion by TLC, ice-water (10 mL) was added to the mixture. The resulting mixture was then extracted with CH₂Cl₂ (3 × 10 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by preparative TLC (MeOH/CH₂Cl₂, v/v = 1/50), and the fractions with Rf = 0.3-0.4 were collected to yield **Compound 77** as white solid (34.48 mg, yield 34.5%). ESI[M + H]⁺ =491.1.

¹H NMR (400 MHz, DMSO-*d6*) δ 8.56 (d, *J=* 4.7 Hz, 1H), 7.89 (t, *J =* 7.8 Hz, 1H), 7.81 (dd, *J* = 8.8, 2.1 Hz, 1H), 7.69 (d, *J* = 7.7 Hz, 1H), 7.55 - 7.47 (m, 3H), 7.46 - 7.42 (m, 2H), 7.38 - 7.34 (m, 3H), 4.46 (d, *J* = 11.2 Hz, 1H), 4.27 - 4.20 (m, 1H), 3.96 (d, *J* = 6.8 Hz, 1H), 3.27 (s, 3H), 1.99 - 1.85 (m, 2H), 0.85 (t, *J =* 7.1 Hz, 3H).

### Example 36 Preparation of Compound 79 and Compound 80

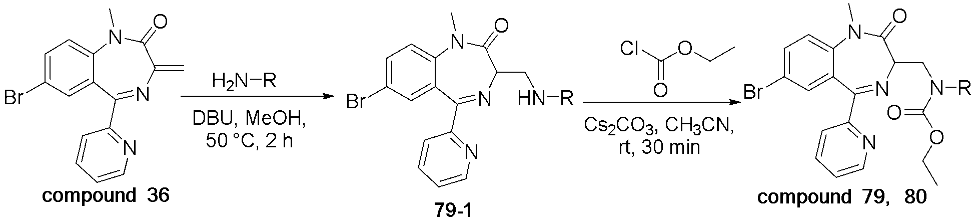

The preparation method of **compound 79 and compound 80** are similar to **compound 76** in **example 35,** using **Compound 36** and **corresponding amine to give intermediate 79-1,** followed by subsequent reaction with ethyl chloroformate to yield the compound.

Compound **79:** 68.0 mg, ESI[M+H]⁺ = 471.1.

¹H NMR (400 MHz, DMSO-*d6*) δ 8.58 (d, *J* = 4.7 Hz, 1H), 8.05 - 7.94 (m, 2H), 7.82 (dd, *J* = 8.9, 2.4 Hz, 1H), 7.55 - 7.51 (m, 3H), 4.15 - 3.90 (m, 4H), 3.80 - 3.77 (m, 1H), 3.31 (s, 3H), 2.59 - 2.55 (m, 1H), 1.10 - 1.06 (m, 3H), 0.81- 0.78 (m, 2H), 0.68 - 0.64 (m, 2H).

Compound **80:** 68.0 mg, ESI[M+H]⁺ = 445.0.

¹H NMR (400 MHz, DMSO-*d6*) δ 8.59 (d, *J =* 4.1 Hz, 1H), 8.08 - 8.05 (m, 1H), 7.97 (td, *J* = 7.7, 1.7 Hz, 1H), 7.83 (dd, *J* = 8.9, 2.4 Hz, 1H), 7.57 - 7.50 (m, 3H), 4.06 - 3.81 (m, 5H), 3.31 (s, 3H), 3.03 - 2.93 (m, 3H), 1.16 - 0.99 (m, 3H).

### Example 37 Preparation of Compound 81

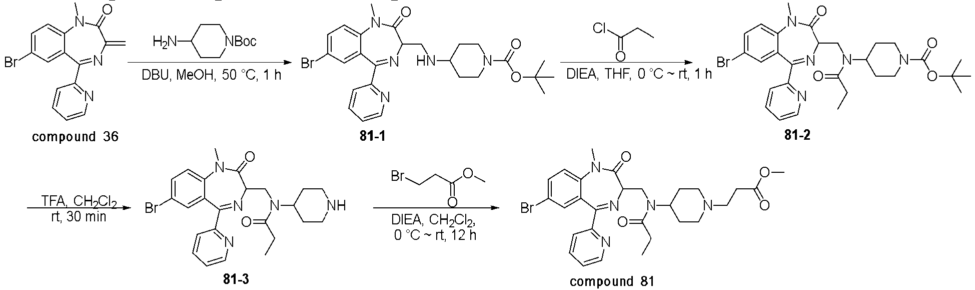

### 1. Preparation of Compound 81-1

A mixture of **Compound 36** (376.2 mg, 1.10 mmol), 1-Boc-4-aminopiperidine (440 mg, 2.20 mmol), and DBU (183.7 mg, 1.21 mmol) in methanol (4 mL) was stirred at 50 °C for 1 hour. After confirming reaction completion by TLC, ice-water (15 mL) was added to the mixture. The resulting mixture was then extracted with ethyl acetate (3 × 15 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by preparative TLC (MeOH/CH₂Cl₂, v/v = 1/10), and the fractions with Rf = 0.3-0.4 were collected to yield **Compound 81-1** as white solid (408 mg, yield 68.4%). ESI[M + H]⁺ = 542.2.

### 2. Preparation of Compound 81-2

At 0°C, propionyl chloride (83.25 mg, 0.90 mmol) was added dropwise to a solution of **81-1** (408 mg, 0.752 mmol) and DIEA (193.9 mg, 1.50 mmol) in THF (4 mL). The mixture was then allowed to warm to room temperature and stirred for 1 hour. After confirming reaction completion by TLC, ice-water (10 mL) was added to the mixture. The resulting mixture was then extracted with ethyl acetate (3 × 10 mL). The organic phase was washed with brine, and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (ethyl acetate/petroleum ether, v/v = 1/100-1/10), TLC (ethyl acetate/petroleum ether, v/v =1/1) momnitoring and the fractions with Rf = 0.3-0.4 were collected to yield **Compound 81-2** as white solid (438 mg, yield 97.3%). ESI[M + H]⁺ = 598.3.

### 3. Preparation of Compound 81-3

Trifluoroacetic acid (5 mL) was added to a solution of **81-2** (438 mg, 0.732 mmol) in dichloromethane (10 mL), and the mixture was stirred at room temperature for 30 minutes. After confirming reaction completion by TLC, the mixture was concentrated under reduced pressure. The residue was dissolved in water, and the pH of the aqueous solution was adjusted to 8-9 with a saturated aqueous sodium bicarbonate solution. The resulting mixture was then extracted with CH₂Cl₂ (3 × 15 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by preparative TLC (MeOH/CH₂Cl₂, v/v = 1/10), and the fractions with Rf = 0.3-0.4 were collected to yield **Compound 81-3** as white solid (348 mg, yield 95.4%). ESI[M + H]⁺ = 498.2.

### 4. Preparation of Compound 81

At 0°C, methyl 3-bromopropanoate (100.8 mg, 0.604 mmol) was added to a solution of **81-3** (200 mg, 0.401 mmol) and DIEA (156 mg, 1.21 mmol) in dichloromethane (3 mL). The mixture was then allowed to warm to room temperature and stirred for 12 hours. After confirming reaction completion by TLC, ice-water (10 mL) was added to the mixture, the resulting mixture was then extracted with CH₂Cl₂ (3 × 15 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by preparative TLC (MeOH/CH₂Cl₂, v/v = 1/10), and the fractions with Rf = 0.3-0.4 were collected to yield **Compound 81** as white solid (46.8 mg, yield 20.0%). ESI[M + H]⁺ = 584.1.

¹H NMR (400 MHz, DMSO-*d6*) δ 8.58 (d, *J* = 4.6 Hz, 1H), 8.08 (d, *J* = 7.7 Hz, 0.5H), 7.98 (t, *J =* 7.9 Hz, 1H), 7.91 (d, *J* = 8.0 Hz, 0.5H), 7.82 (td, *J* = 8.6, 2.3 Hz, 1H), 7.57 - 7.50 (m, 2H), 7.49 (d, *J* = 2.1 Hz, 1H), 4.38 - 4.28 (m, 0.5H), 4.05 - 3.95 (m, 1H), 3.93 - 3.80 (m, 1H), 3.79 - 3.70 (m, 1H), 3.63 - 3.60 (m, 0.5H), 3.60 (s, 1.5H), 3.54 (s, 1.5H), 3.31 (s, 3H), 2.97 - 2.90 (m, 1H), 2.85 - 2.66 (m, 2H), 2.62 - 2.55 (m, 1H), 2.41 - 2.30 (m, 3H), 2.09 - 2.00 (m, 1H), 1.98 - 1.69 (m, 3H), 1.65 - 1.50 (m, 1H), 1.45 - 1.31 (m, 2H), 0.99 (t, *J =* 8.0 Hz, 1.5H), 0.88 (t, *J =* 8.0 Hz, 1.5H).

### Example 38 Preparation of Compound 82

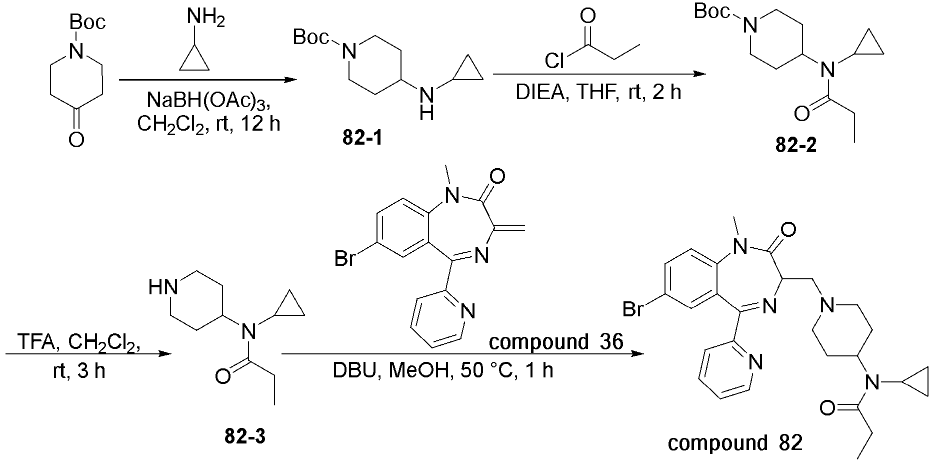

### 1. Preparation of Compound 82-1

A mixture of N-Boc-4-piperidone (10.0 g, 50.2 mmol) and cyclopropylamine (5.73 g, 100.4 mmol) in dichloromethane (150 mL) was stirred at room temperature for 2 hours. Sodium triacetoxyborohydride (NaBH(OAc)₃, 31.9 g, 150.5 mmol) was then added to the mixture, and stirring was continued at room temperature for 10 hours. After confirming reaction completion by TLC, saturated aqueous sodium bicarbonate solution (100 mL) was added, the mixture was extracted with ethyl acetate (3 × 100 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (ethyl acetate/petroleum ether, v/v = 1/100-1/2), TLC (ethyl acetate/petroleum ether, v/v =1/2) momnitoring and and the fractions with Rf = 0.3-0.4 were collected to yield **Compound 82-1** as white solid (9.9 g, yield 82.1%). ESI[M + H]⁺ = 241.1.

### 2. Preparation of Compound 82-2

Propionyl chloride (920 mg, 9.94 mmol) was added to a solution of **82-1** (1.20 g, 5.0 mmol) and DIEA (1.94 g, 15.0 mmol) in dry tetrahydrofuran (25 mL). The mixture was stirred at room temperature for 2 hours. After confirming reaction completion by TLC, ice-water (50 mL) was added, the mixture was extracted with ethyl acetate (3 × 50 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (ethyl acetate/petroleum ether, v/v = 1/100-1/3), TLC (ethyl acetate/petroleum ether, v/v =1/3) momnitoring and and the fractions with Rf = 0.3-0.4 were collected to yield **Compound 82-2** as white solid (11.33 g, yield 89.9%). ESI[M + H]⁺ = 297.2.

### 3. Preparation of Compound 82-3

Trifluoroacetic acid (2.57 g, 22.5 mmol) was added to a solution of **82-2** (1.33 g, 4.49 mmol) in dichloromethane (10 mL). The mixture was stirred at room temperature for 3 hours. After confirming reaction completion by TLC, the mixture was concentrated under reduced pressure. The residue was dissolved in water, and the pH of the resulting aqueous solution was adjusted to 8-9 with saturated aqueous sodium bicarbonate solution. The mixture was then extracted with ethyl acetate (3 × 50 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and and the fractions with Rf = 0.3-0.4 were collected to yield **Compound 82-3** as oil colorless (684 mg, yield 77.7%). ESI[M + H]⁺ = 197.1.

### 4. Preparation of Compound 82

A mixture of **Compound 36** (100 mg, 0.292 mmol), **82-3** (86.0 mg, 0.438 mmol), and DBU (49.0 mg, 0.322 mmol) in methanol (2 mL) was stirred at 50 °C for 1 hour. After confirming reaction completion by TLC, ice-water (50 mL) was added, the mixture was then extracted with ethyl acetate (3 × 50 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by preparative TLC (ethyl acetate/petroleum ether, v/v = 2/1), and and the fractions with Rf = 0.2-0.3 were collected to yield **Compound 82** as white solid (77.7 mg, yield 49.4%). ESI[M + H]⁺ = 538.2.

¹H NMR (400 MHz, DMSO-*d6*) δ 8.97 - 8.52 (m, 1H), 8.33 - 7.79 (m, 4H), 7.71 - 7.46 (m, 2H), 3.90 - 3.71 (m, 1H), 3.60- 3.46 (m, 1H), 3.31 (s, 3H), 3.30 - 3.28 (m, 1H), 3.25 - 2.75 (m, 3H), 2.60 - 2.52 (m, 1H), 2.49 - 2.33 (m, 3H), 2.35 - 1.75 (m, 3H), 1.52 - 1.25 (m, 2H), 1.02 - 0.90 (m, 3H), 0.81 - 0.71 ( m, 4H).

### Example 39 Preparation of Compound 83 and Compound 88

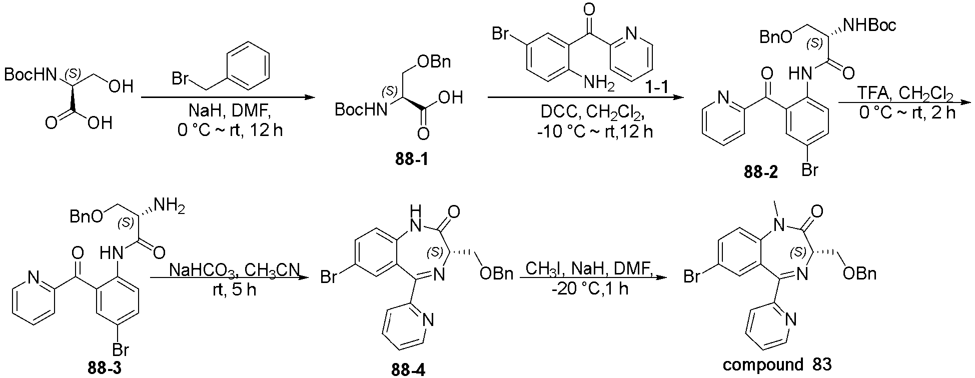

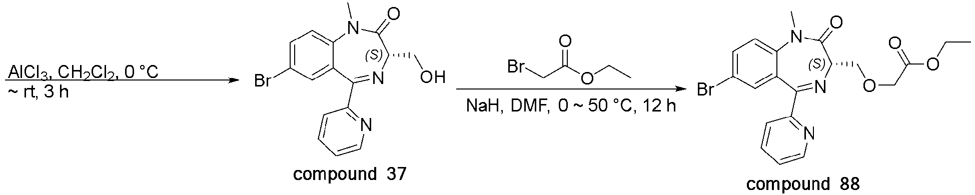

### 1. Preparation of Compound 88-1

At 0 °C, sodium hydride (2.146 g, 60% dispersion in mineral oil, 53.6 mmol) was slowly added to a solution of BOC-L-serine (5.0 g, 24.4 mmol) in dry DMF (60 mL). The mixture was stirred at this temperature for 30 minutes. Benzyl bromide (4.67 g, 27.3 mmol) was then added. The mixture was allowed to warm to room temperature and stirring was continued for 12 hours. After confirming reaction completion by TLC, the ice-water (100 mL) was added to the mixture, the pH of the resulting mixture was adjusted to 6-7 with 1 M aqueous HCl, then extracted with ethyl acetate (3 × 50 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by preparative TLC (ethyl acetate/petroleum ether, v/v = 2/1), and and the fractions with Rf = 0.2-0.3 were collected to yield **Compound 88-1** as colorless oil (6.8 g, yield94.5%). ESI[M + H]⁺ = 296.2.

### 2. Preparation of Compound 88-2

A mixture of 1-1 (2.76 g, 9.96 mmol) and **88-1** (4.42 g, 15.0 mmol) in dichloromethane (30 mL) was cooled to -10°C. A solution of DCC (3.09 g, 15.0 mmol) in dichloromethane (10 mL) was added dropwise at this temperature. After the addition was complete, the mixture was allowed to warm to room temperature and stirred for 12 hours. After confirming reaction completion by TLC, then methyl tert-butyl ether (MTBE, 40 mL) was added to the mixture and stirring was continued for 5 minutes. The mixture was filtered, and the filtrate was concentrated under reduced pressure to afford the crude product, and the crude product was purified by silica gel column chromatography (ethyl acetate/petroleum ether, v/v = 1/20-1/2), TLC (ethyl acetate/petroleum ether, v/v =1/2) momnitoring and and the fractions with Rf = 0.3-0.4 were collected to yield **Compound 88-2** as white solid (4.857 g, yield 88.0%). ESI[M + H]⁺ = 554.1.

### 3. Preparation of Compound 88-3

At 0°C, trifluoroacetic acid (20 mL) was added to a solution of **88-2** (4.857 g, 8.76 mmol) in dichloromethane (40 mL). The mixture was allowed to warm to room temperature and stirred for 2 hours. After confirming reaction completion by TLC, the mixture was concentrated under reduced pressure. The crude product was used directly in the next step without further purification.

### 4. Preparation of Compound 88-4

The crude product from the previous step was dissolved in acetonitrile (50 mL). Sodium bicarbonate (22.1 g, 263.1 mmol) was added, and the mixture was stirred at room temperature for 5 hours. After confirming reaction completion by TLC, the mixture was filtered, and the filtrate was concentrated under reduced pressure to afford the crude product, and the crude product was purified by silica gel column chromatography (ethyl acetate/petroleum ether, v/v = 1/10-1/2), TLC (ethyl acetate/petroleum ether, v/v =1/1) momnitoring and the fractions with Rf = 0.2-0.3 were collected to yield **Compound 88-4 as** white solid (4.857 g, two steps yield 65.2%). ESI[M + H]⁺ = 436.1.

### 5. Preparation of Compound 83

At -20°C, sodium hydride (275 mg, 60% dispersion in mineral oil, 6.87 mmol) was added to a solution of **88-4** (2.49 g, 5.71 mmol) in dry DMF (30 mL). The mixture was stirred at this temperature for 30 minutes. Methyl iodide (975 mg, 6.87 mmol) was then added, and stirring was continued at -20 °C for 1 hour. After confirming reaction completion by TLC, the ice-water (50 mL) was added to the mixture, then extracted with ethyl acetate (3 × 50 mL). The organic phase was washed with brine and dried over anhydrous Na₂SO₄, filtered and the filtrate was concentrated under reduced pressure to afford the crude product. The filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 83** as white solid (2.47 g, yield 96.1%). ESI[M + H]⁺ = 450.4.

¹H NMR (400 MHz, DMSO-*d6*) δ 8.61 (d, *J =* 4.6 Hz, 1H), 8.10 (d, *J* = 7.9 Hz, 1H), 7.99 (td, *J* = 7.8, 1.7 Hz, 1H), 7.84 (dd, *J =* 8.8, 2.4 Hz, 1H), 7.56 - 7.53 (m, 3H), 7.37 - 7.34 (m, 4H), 7.32 - 7.26 (m, 1H), 4.62 (s, 2H), 4.25 (dd, *J* = 9.6, 6.0 Hz, 1H), 4.08 (dd, *J =* 9.6, 6.9 Hz, 1H), 3.87 (t, *J* = 6.3 Hz, 1H), 3.32 (s, 3H).

### 6. Preparation of Compound 37

At 0°C, aluminum chloride (7.19 g, 53.9 mmol) was slowly added to a solution of **83** (2.43 g, 5.40 mmol) in dichloromethane (50 mL). The mixture was allowed to warm to room temperature and stirred for 3 hours. After confirming reaction completion by TLC, ice-water (50 mL) was poured into the mixture and the mixture was filtered. The filtrate was extracted with dichloromethane (3 × 60 mL). The combined organic layers were washed with 5% aqueous sodium bicarbonate solution, followed by brine. the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield **Compound 37** as colorless oil (1.15 g, yield 59.2%). ESI[M + H]⁺ = 360.2.

### 7. Preparation of Compound 88

At 0°C, sodium hydride (67 mg, 60% dispersion in mineral oil, 1.67 mmol) was slowly added to a solution of **37** (200 mg, 0.555 mmol) in dry DMF (3 mL). The mixture was stirred at this temperature for 30 minutes. Ethyl bromoacetate (186 mg, 1.11 mmol) was then added, and the mixture was warmed to 50°C and stirred for 12 hours. After confirming reaction completion by TLC, ice-water (10 mL) was poured into the mixture and extracted with ethyl acetate (3 × 10 mL). The organic phase was washed with brine and dried over anhydrous Na₂SO₄, filtered and the filtrate was concentrated under reduced pressure to afford the crude product, the crude product was purified by preparative TLC (ethyl acetate/petroleum ether, v/v = 1/1), and the fractions with Rf = 0.2-0.3 were collected to yield **Compound 88** as white solid (11.5 mg, yield 4.6%). ESI[M + H]⁺ = 446.2.

¹H NMR (400 MHz, DMSO-*d6*) δ 8.59 (d, *J =* 4.2 Hz, 1H), 8.08 (d, *J =* 7.6 Hz, 1H), 7.99 - 7.95 (m, 1H), 7.83 (dd, *J* = 8.8, 2.5 Hz, 1H), 7.56 - 7.51 (m, 3H), 4.27 (dd, *J* = 10.3, 6.0 Hz, 1H), 4.23 (s, 2H), 4.15 - 4.14 (m, 1H), 4.10 (q, *J* = 7.3 Hz, 2H), 3.88 (t, *J* = 6.4 Hz, 1H), 3.30 (s, 3H), 1.18 (t, *J* = 7.1 Hz, 3H).

### Example 40 Preparation of Compound 84-87

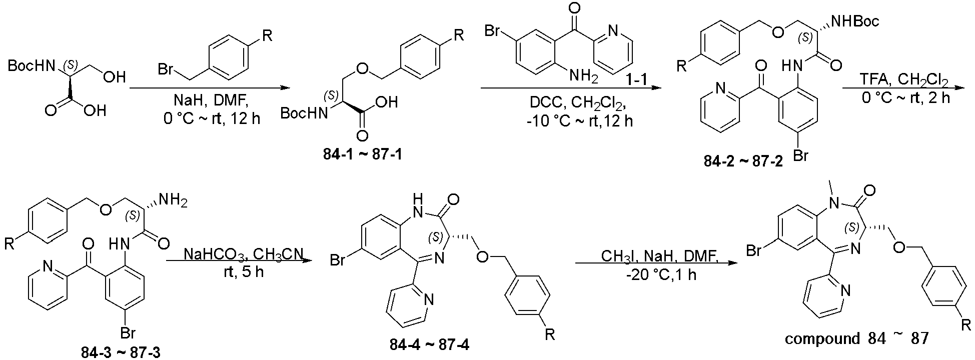

The preparation method of **compound 84-87** are similar to **compound 83 in example 39,** using BOC-L-serine and variously substituted benzyl bromides to furnish the corresponding carboxylic acids followed by condensation with **Compound 1-1,** Boc deprotection, cyclization, and finally N-methylation to yield the target compound.

Compound **84:** 18.9 mg, ESI[M+H]⁺ = 464.1.

¹H NMR (400 MHz, DMSO-*d6*) δ 8.60 (d, *J =* 4.2 Hz, 1H), 8.09 (d, *J* = 7.9 Hz, 1H), 7.99 (td, *J* = 7.7, 1.7 Hz, 1H), 7.83 (dd, *J* = 8.8, 2.4 Hz, 1H), 7.57 - 7.50 (m, 3H), 7.24 (d, *J* = 7.9 Hz, 2H), 7.16 (d, *J* = 7.8 Hz, 2H), 4.56 (s, 2H), 4.22 (dd, *J* = 9.7, 6.0 Hz, 1H), 4.05 (dd, *J* = 9.7, 6.8 Hz, 1H), 3.83 (t, *J* = 6.4 Hz, 1H), 3.31 (s, 3H), 2.30 (s, 3H).

Compound **85:** 13.5 mg, ESI[M+H]⁺ = 484.0.

¹H NMR (400 MHz, DMSO-*d6*) δ 8.60 (d, *J =* 4.1 Hz, 1H), 8.09 (d, *J* = 7.9 Hz, 1H), 7.99 (td, *J* = 7.7, 1.7 Hz, 1H), 7.83 (dd, *J =* 8.8, 2.4 Hz, 1H), 7.56 - 7.53 (m, 3H), 7.49 - 7.34 (m, 4H), 4.61 (s, 2H), 4.25 (dd, *J* = 9.7, 6.0 Hz, 1H), 4.07 (dd, *J* = 9.6, 6.8 Hz, 1H), 3.87 (t, *J* = 6.4 Hz, 1H), 3.32 (s, 3H).

Compound **86:** 24.2 mg, ESI[M+H]⁺ = 480.1.

¹H NMR (400 MHz, DMSO-*d6*) δ 8.60 (d, *J =* 4.2 Hz, 1H), 8.09 (d, *J* = 7.9 Hz, 1H), 7.99 (td, *J* = 7.8, 1.7 Hz, 1H), 7.83 (dd, *J* = 8.8, 2.4 Hz, 1H), 7.55 - 7.52 (m, 3H), 7.28 (d, *J* = 8.6 Hz, 2H), 6.91 (d, *J* = 8.6 Hz, 2H), 4.53 (s, 2H), 4.21 (dd, *J* = 9.6, 6.1 Hz, 1H), 4.03 (dd, *J* = 9.6, 6.8 Hz, 1H), 3.82 (t, *J =* 6.4 Hz, 1H), 3.75 (s, 3H), 3.31 (s, 3H).

Compound **87:** 51.2 mg, ESI[M+H]⁺ = 492.2.

¹H NMR (400 MHz, DMSO-*d6*) δ 8.60 (d, *J =* 4.3 Hz, 1H), 8.09 (d, *J =* 7.9 Hz, 1H), 7.98 (td, *J =* 7.7, 1.7 Hz, 1H), 7.83 (dd, *J* = 8.8, 2.4 Hz, 1H), 7.57 - 7.50 (m, 3H), 7.27 (d, *J=* 8.1 Hz, 2H), 7.22 (d, *J* = 8.1 Hz, 2H), 4.56 (s, 2H), 4.21 (dd, *J* = 9.6, 6.0 Hz, 1H), 4.05 (dd, *J* = 9.6, 6.9 Hz, 1H), 3.85 (t, *J* = 6.4 Hz, 1H), 3.31 (s, 3H), 2.94 - 2.83 (m, 1H), 1.21 (s, 3H), 1.19 (s, 3H).

### Example 41 Preparation of Compound 91

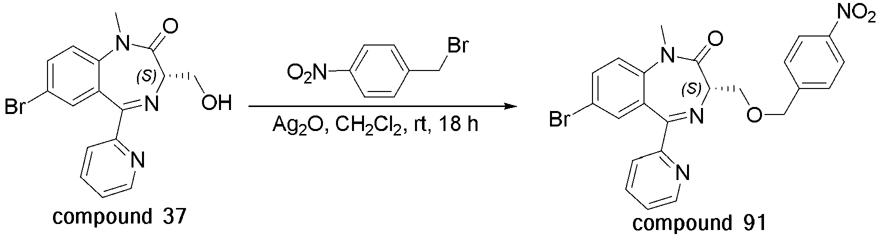

A mixture of **37** (300 mg, 0.833 mmol), p-nitrobenzyl bromide (198.6 mg, 0.919 mmol), and silver(I) oxide (232.4 mg, 1.00 mmol) in dichloromethane (5 mL) was stirred at room temperature for 18 hours. After confirming reaction completion by TLC, the mixture was filtered, and the filtrate was concentrated under reduced pressure to afford the crude product. the crude product was purified by preparative TLC (ethyl acetate/petroleum ether, v/v = 1/1), and the fractions with Rf = 0.2-0.3 were collected to yield **Compound 91** as white solid (21.7 mg, yield 5.3%). ESI[M + H]⁺ = 495.1.

¹H NMR (400 MHz, DMSO-*d6*) δ 8.61 (d, *J =* 4.2 Hz, 1H), 8.23 (d, *J =* 8.7 Hz, 2H), 8.11 (d, *J* = 7.9 Hz, 1H), 8.01 - 7.98 (m, 1H), 7.84 (dd, *J =* 8.9, 2.3 Hz, 1H), 7.65 (d, *J* = 8.7 Hz, 2H), 7.57 - 7.54 (m, 3H), 4.79 (s, 2H), 4.30 (dd, *J =* 9.7, 5.9 Hz, 1H), 4.14 (dd, *J =* 9.7, 6.9 Hz, 1H), 3.93 (t, *J* = 6.4 Hz, 1H), 3.33 (s, 3H).

### Example 42 Preparation of Compound 95

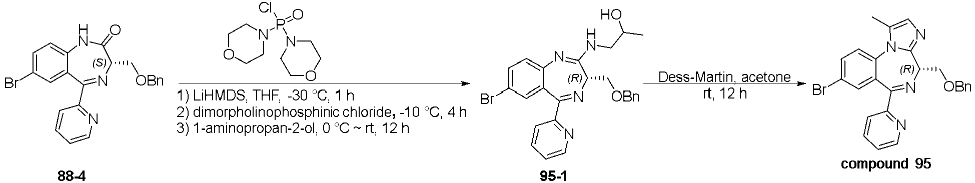

### 1. Preparation of Compound 95-1

At -30°C, a solution of lithium bis(trimethylsilyl)amide (5.98 mL of a 1 M solution, 5.98 mmol) was added dropwise to a solution of 88-4 (2.24 g, 5.13 mmol) in tetrahydrofuran (20 mL). The mixture was stirred at this temperature for 1 hour. Morphorphosphorodichloridate (2.99 g, 11.7 mmol) was added portionwise. After the addition was complete, the reaction was stirred at -10 °C for 4 hours. Isopropanolamine (1.68 g, 22.4 mmol) was then added dropwise to the resulting mixture. The reaction was allowed to warm to room temperature and stirred for 12 hours. After confirming reaction completion by TLC, ice-water (20 mL) was poured into the mixture and extracted with ethyl acetate (3 × 20 mL). The organic phase was washed with brine and dried over anhydrous Na₂SO₄, filtered and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 95-1** as white solid (1.6 g, yield 63.2%). ESI[M + H]⁺ = 493.1.

### 2. Preparation of Compound 95

Dess-Martin periodinane (3.5 g, 8.25 mmol) was added to a solution of **95-1** (1.6 g, 3.24 mmol) in acetone (20 mL). The mixture was stirred at room temperature for 12 hours. After confirming reaction completion by TLC, the mixture was filtered and the filtrate was concentrated under reduced pressure. The residue was dissolved in ethyl acetate (300 mL), and the resulting solution was washed with saturated aqueous sodium bicarbonate solution and dried over anhydrous Na₂SO₄, filtered and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 95** as white solid (1.164 g, yield 75.8%). ESI[M + H]⁺ = 473.1.

¹H NMR (400 MHz, DMSO-*d6*) δ 8.56 - 8.52 (m, 1H), 8.08 (d, *J* = 7.9 Hz, 1H), 7.95 (td, *J* = 7.7, 1.8 Hz, 1H), 7.88 (dd, *J* = 8.7, 2.3 Hz, 1H), 7.66 - 7.63 (m, 2H), 7.51 - 7.48 (m, 1H), 7.43 - 7.32 (m, 4H), 7.30 - 7.26 (m, 1H), 6.80 (d, *J* = 0.9 Hz, 1H), 4.68 (s, 2H), 4.40 - 4.32 (m, 2H), 4.23 (dd, *J =* 7.5, 5.3 Hz, 1H), 2.30 (d, *J =* 0.8 Hz, 3H).

### Example 43 Preparation of Compound 96

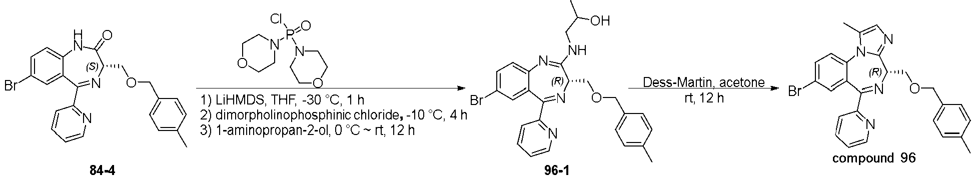

### 1. Preparation of Compound 96-1

At -30°C, lithium bis (trimethylsilyl) amide (2.7 mL, 1 mol/L, 2.7 mmol) was slowly added dropwise to tetrahydrofuran (10 mL) solution of **84-4** (1.0 g, 2.22 mmol), followed by stirring for 1 hour. Morpholinophosphorodiamidic chloride (1.34 g, 5.26 mmol) was added in batches, the mixture was stirred at -10°C for 4 hours. Isopropanolamine (752 mg, 10.0 mmol) was added dropwise to the above reaction system, which was then allowed to warm to room temperature naturally and stirred for 12 hours. After confirming reaction completion by TLC, ice-water (20 mL) was poured into the mixture and extracted with ethyl acetate (3 × 20 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 96-1** as white solid (811 mg, yield 72.0%). ESI[M + H]⁺ = 507.1.

### 2. Preparation of Compound 96

Dess-Martin periodinane (1.698 g, 4.0 mmol) was added to a solution of **96-1** (811 mg, 1.60 mmol) in acetone (20 mL). The mixture was stirred at room temperature for 12 hours. After confirming reaction completion by TLC, the mixture was filtered and the filtrate was concentrated under reduced pressure. The residue was dissolved in ethyl acetate (30 mL), and the resulting solution was washed with saturated aqueous sodium bicarbonate solution and dried over anhydrous Na₂SO₄, filtered and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 96** as white solid (228.7 mg, yield 29.4%). ESI[M + H]⁺ = 487.2.

¹H NMR (400 MHz, DMSO-*d6*) δ 8.56 (d, *J* = 4.2 Hz, 1H), 8.09 (d, *J =* 7.9 Hz, 1H), 7.97 (td, *J =* 7.8, 1.7 Hz, 1H), 7.90 (dd, *J* = 8.7, 2.3 Hz, 1H), 7.67 (d, *J =* 8.7 Hz, 1H), 7.64 (d, *J =* 2.3 Hz, 1H), 7.51 (dd, *J* = 6.5, 4.9 Hz, 1H), 7.28 (d, *J* = 7.9 Hz, 2H), 7.17 (d, *J* = 7.8 Hz, 2H), 6.81 (s, 1H), 4.64 (s, 2H), 4.44 - 4.31 (m, 2H), 4.22 (dd, *J* = 7.3, 5.4 Hz, 1H), 2.31 (s, 3H), 2.30 (s, 3H).

### Example 44 Preparation of Compound 97, 97B, 97D

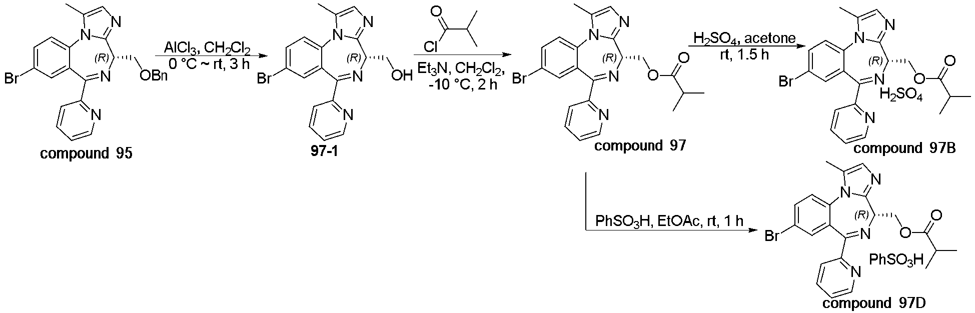

### 1. Preparation of Compound 97-1

At 0°C, aluminum trichloride (2.44 g, 18.3 mmol) was slowly added to the solution of **95** (866 mg, 1.83 mmol) in dichloromethane (50 mL). The mixture was allowed to warm to room temperature and stirred for 3 hours. After confirming reaction completion by TLC, ice-water (50 mL) was poured into the mixture, filtered and the filtrate was extracted with CH₂Cl₂ (3 × 60 mL). The organic phase was washed with5% aqueous sodium bicarbonate solution, brine and dried over anhydrous Na₂SO₄, filtered and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 97-1** as white solid ((620 mg, yield 88.4%). ESI[M + H]⁺ = 383.1.

### 2. Preparation of Compound 97

At -10°C, isobutyryl chloride (125 mg, 1.17 mmol) was added dropwise to a solution of **97-1** (300 mg, 0.783 mmol) and triethylamine (159 mg, 1.57 mmol) in dichloromethane (3 mL). The mixture was stirred for 2 hours. After confirming reaction completion by TLC, ice-water (10 mL) was poured into the mixture, filtered and the filtrate was extracted with CH₂Cl₂ (3 × 10 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.4-0.5 were collected to yield **Compound 97** as white solid ((208 mg, yield 58.6%). ESI[M + H]⁺ = 453.1.

¹H NMR (400 MHz, DMSO-*d6*) δ 8.57 - 8.53 (m, 1H), 8.04 (d, *J =* 7.9 Hz, 1H), 7.95 (td, *J =* 7.7, 1.7 Hz, 1H), 7.90 (dd, *J* = 8.7, 2.3 Hz, 1H), 7.68 (d, *J =* 8.7 Hz, 1H), 7.64 (d, *J =* 2.3 Hz, 1H), 7.50 (ddd, *J =* 7.5, 4.8, 1.2 Hz, 1H), 6.83 (d, *J=* 1.1 Hz, 1H), 4.93 (dd, *J* = 10.9, 8.0 Hz, 1H), 4.84 (dd, *J =* 11.0, 5.4 Hz, 1H), 4.36 (dd, *J* = 7.9, 5.4 Hz, 1H), 2.60 - 2.53 (m, 1H), 2.31 (d, *J* = 0.7 Hz, 3H), 1.11 (d, *J* = 7.0 Hz, 3H), 1.09 (d, *J* = 7.0 Hz, 3H).

### 3. Preparation of Compound 97B

Compound 97 (109.8 mg, 0.242 mmol) was dissolved in acetone (10 mL), the solution of sulfuric acid (23.7 mg, 0.242 mmol) in acetone (1 mL) was added dropwise into the mixture. The mixture was stirred at room temperature for 1.5 hours. The mixture was then filtered, and the filter cake was dried under reduced pressure to yield **Compound 97B** as white solid (85.5 mg, yield 64.0%). ESI[M + H]⁺ = 453.1.

¹H NMR (400 MHz, DMSO-*d6*) δ 8.59 (d, *J* = 4.7 Hz, 1H), 8.08 (d, *J* = 7.9 Hz, 1H), 8.04 - 7.96 (m, 2H), 7.80 (d, *J =* 8.7 Hz, 1H), 7.75 (d, *J* = 2.2 Hz, 1H), 7.55 (dd, *J* = 6.3, 4.9 Hz, 1H), 7.20 (s, 1H), 4.95 (dd, *J* = 11.0, 7.3 Hz, 1H), 4.87 (dd, *J* = 11.1, 6.0 Hz, 1H), 4.58 (t, *J* = 6.7 Hz, 1H), 2.60 - 2.52 (m, 1H), 2.38 (s, 3H), 1.14 (d, *J* = 5.2 Hz, 3H), 1.12 (d, *J* = 5.2 Hz, 3H).

### 4. Preparation of Compound 97D

A solution of Compound **97** (27.2 mg, 0.06 mmol) in ethyl acetate (1 mL) was treated with a solution of benzenesulfonic acid (9.47 mg, 0.06 mmol) in ethanol (0.1 mL) added dropwise. After stirring at room temperature for 1 hour, the mixture was filtered. The collected solid was dried under reduced pressure to yield **Compound 97D** as a white solid (10.3 mg, yield 28.1%). ESI [M + H]⁺ = 453.1.

¹H NMR (400 MHz, DMSO-*d6*) δ 8.57 (d, *J* = 4.6 Hz, 1H), 8.06 (d, *J* = 7.8 Hz, 1H), 8.01 - 7.93 (m, 2H), 7.77 (d, *J* = 8.7 Hz, 1H), 7.72 (d, *J* = 2.2 Hz, 1H), 7.60 - 7.58 (m, 2H), 7.56 - 7.51 (m, 1H), 7.35 - 7.28 (m, 3H), 7.14 (s, 1H), 4.93 (dd, *J =* 11.1, 7.4 Hz, 1H), 4.85 (dd, *J =* 11.1, 6.0 Hz, 1H), 4.54 (t, *J* = 6.6 Hz, 1H), 2.61 - 2.54 (m, 1H), 2.35 (s, 3H), 1.11 (d, *J =* 5.6 Hz, 3H), 1.10 (d, *J* = 5.6 Hz, 3H).

### Example 45 Preparation of Compound 98, 98D

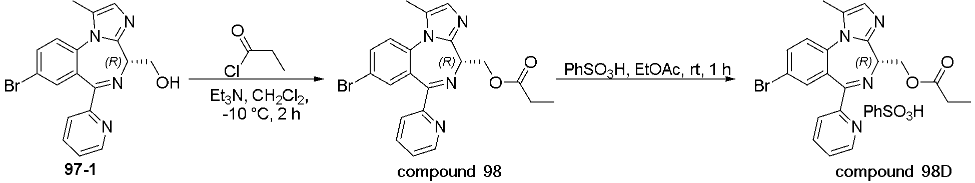

The preparation method of **compound 98** and **compound 98D** are similar to **compound 97 and 97D** in **example 44,** using **97-1** and propionyl chloride as starting materials to yield the Compound **98,** then treated with benzenesulfonic acid to yield Compound **98D.**

**Compound98:** 73.1 mg, ESI[M+H]⁺ = 439.0.

¹H NMR (400 MHz, DMSO-*d6*) δ 8.55 (ddd, *J* = 4.8, 1.7, 0.9 Hz, 1H), 8.06 (d, *J* = 7.9 Hz, 1H), 7.95 (td, *J* = 7.7*,* 1.8 Hz, 1H), 7.90 (dd, *J* = 8.7, 2.3 Hz, 1H), 7.68 (d, *J* = 8.7 Hz, 1H), 7.66 (d, *J* = 2.3 Hz, 1H), 7.50 (ddd, *J* = 7.5, 4.8, 1.2 Hz, 1H), 6.83 (d, *J* = 1.1 Hz, 1H), 4.98 - 4.82 (m, 2H), 4.34 (dd, *J* = 7.5, 5.7 Hz, 1H), 2.38 - 2.32 (m, 2H), 2.31 (d, *J* = 0.8 Hz, 3H), 1.05 (t, *J* = 7.5 Hz, 3H).

Compound**98D**: 9.3 mg, ESI[M+H]⁺ = 439.1.

¹H NMR (400 MHz, DMSO-*d6*) δ 8.57 (d, *J* = 4.0 Hz, 1H), 8.08 (d, *J* = 8.0 Hz, 1H), 8.01 - 7.93 (m, 2H), 7.77 (d, *J* = 8.7 Hz, 1H), 7.73 (d, *J =* 2.3 Hz, 1H), 7.62 - 7.57 (m, 2H), 7.56 - 7.50 (m, 1H), 7.34 - 7.26 (m, 3H), 7.16 (s, 1H), 4.94 - 4.85 (m, 2H), 4.53 (t, *J* = 6.3 Hz, 1H), 2.39 - 2.32 (m, 5H), 1.05 (t, *J* = 7.5 Hz, 3H).

### Example 46 Preparation of Compound 99

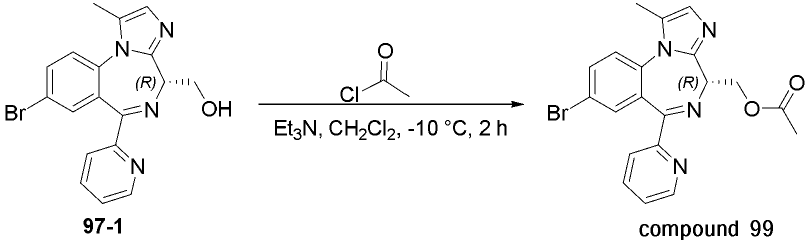

The preparation method of **compound 99** is similar to **compound 97** in **example 44,** using **97-1** and acetyl chloride as starting materials.

Compound **99:** 42.8 mg, ESI[M+H]⁺ = 425.0.

¹H NMR (400 MHz, DMSO-*d6*) δ 8.55 (ddd, *J* = 4.8, 1.7, 0.9 Hz, 1H), 8.08 - 8.06 (m, 1H), 7.95 (td, *J* = 7.7, 1.8 Hz, 1H), 7.90 (dd, *J* = 8.7, 2.3 Hz, 1H), 7.68 (d, *J* = 8.7 Hz, 1H), 7.66 (d, *J =* 2.3 Hz, 1H), 7.50 (ddd, *J* = 7.5, 4.8, 1.2 Hz, 1H), 6.83 (d, *J* = 1.1 Hz, 1H), 4.95 - 4.84 (m, 2H), 4.34 (t, *J* = 6.6 Hz, 1H), 2.31 (d, *J* = 0.8 Hz, 3H), 2.05 (s, 3H).

### Example 47 Preparation of Compound 100

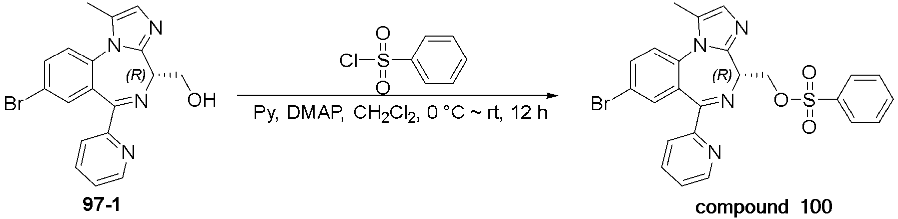

At 0°C, benzenesulfonyl chloride (691.1 mg, 3.91 mmol) was added to solution of **97-1** (300 mg, 0.783 mmol), pyridine (302.3 mg, 3.82 mmol), and DMAP (287.4 mg, 2.35 mmol) in dichloromethane (5 mL). The mixture was allowed to warm to room temperature and stirred for 12 hours. After confirming reaction completion by TLC, ice-water (10 mL) was poured into the mixture, filtered and the filtrate was extracted with CH₂Cl₂ (3 × 10 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 100** as off-yellow solid (126.4 mg, yield 30.9%). ESI[M + H]⁺ = 523.1.

¹H NMR (400 MHz, DMSO-*d6*) δ 8.55 (d, *J* = 4.2 Hz, 1H), 8.03 (d, *J* = 7.5 Hz, 2H), 7.97 (t, *J* = 7.0 Hz, 1H), 7.92 - 7.88 (m, 2H), 7.83 (t, *J=* 7.4 Hz, 1H), 7.73 (t, *J* = 7.7 Hz, 2H), 7.66 (d, *J* = 8.7 Hz, 1H), 7.63 (d, *J* = 2.2 Hz, 1H), 7.55 - 7.49 (m, 1H), 6.81 (s, 1H), 4.94 - 4.79 (m, 2H), 4.42 (dd, *J =* 7.8, 5.0 Hz, 1H), 2.30 (s, 3H).

### Example 48 Preparation of Compound 101 and Compound 100D

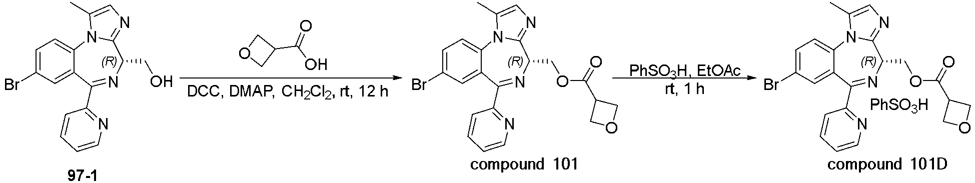

### 1. Preparation of Compound 101

A mixture of 3-oxetanecarboxylic acid (80 mg, 0.784 mmol), DCC (162 mg, 0.785 mmol), and DMAP (192 mg, 1.57 mmol) in dichloromethane (2 mL) was stirred at room temperature for 15 minutes. **97-1** (200 mg, 0.522 mmol) was then added to the mixture, and was stirred at room temperature for 12 hours. After confirming reaction completion by TLC, methyl tert-butyl ether (MTBE) (2 mL) was added, and the mixture was stirred for an additional 5 minutes. The mixture was filtered, and the filtrate was concentrated under reduced pressure to afford the crude product. The filtrate was concentrated in vacuo to yield the crude product. The crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 101** as white solid (81.5 mg, yield 33.4%). ESI[M + H]⁺ = 467.1.

¹H NMR (400 MHz, DMSO-*d6*) δ 8.55 (d, *J =* 4.0 Hz, 1H), 8.05 (d, *J* = 7.9 Hz, 1H), 7.95 (td, *J =* 7.7, 1.8 Hz, 1H), 7.90 (dd, *J* = 8.7, 2.3 Hz, 1H), 7.68 (d, *J =* 8.7 Hz, 1H), 7.65 (d, *J =* 2.3 Hz, 1H), 7.50 (ddd, *J =* 7.5, 4.8, 1.2 Hz, 1H), 6.84 (d, *J =* 1.1 Hz, 1H), 5.04 - 4.91 (m, 2H), 4.74 - 4.69 (m, 2H), 4.67 - 4.62 (m, 2H), 4.40 (dd, *J* = 7.4, 5.7 Hz, 1H), 3.96 - 3.90 (m, 1H), 2.31 (d, *J* = 0.8 Hz, 3H).

### 2. Preparation of Compound 101D

Compound **101** (81.5 mg, 0.174 mmol) was dissolved in ethyl acetate (1 mL), the solution of benzenesulfonic acid (27.8 mg, 0.176 mmol) in ethanol (0.1 mL) was added dropwise into the mixture, then was stirred at room temperature for 1 hour. The mixture was filtered, and the filter cake was dried under reduced pressure to afford **Compound 101D** as a white solid (60.8 mg, 55.7% yield). ESI[M + H]⁺ = 467.0.

¹H NMR (400 MHz, DMSO-*d6*) δ 8.56 (d, *J* = 4.0 Hz, 1H), 8.06 (d, *J* = 7.9 Hz, 1H), 8.00 - 7.91 (m, 2H), 7.75 (d, *J* = 8.7 Hz, 1H), 7.70 (d, *J* = 2.3 Hz, 1H), 7.62 - 7.56 (m, 2H), 7.56 - 7.47 (m, 1H), 7.35 - 7.25 (m, 3H), 7.08 (s, 1H), 5.04 - 4.91 (m, 2H), 4.74 - 4.69 (m, 2H), 4.68 - 4.63 (m, 2H), 4.53 (t, *J* = 6.5 Hz, 1H), 3.96 - 3.90 (m, 1H), 2.34 (s, 3H).

### Example 49 Preparation of Compound 102

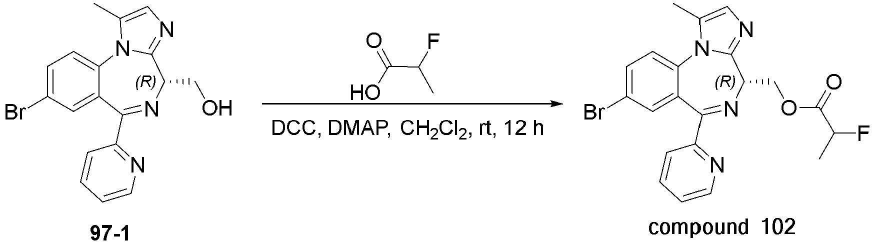

The preparation method of compound 102 is similar to compound 101 in example 48, using 97-1 and 2-Fluoropropanoic acid as starting materials.

Compound**102**: 61.6 mg, ESI[M+H]⁺ = 457.3.

¹H NMR (400 MHz, DMSO-*d6*) δ 8.57 (d, *J* = 4.1 Hz, 1H), 8.07 (dd, *J* = 7.9, 3.9 Hz, 1H), 8.00 - 7.96 (m, 1H), 7.93 (dd, *J* = 8.7, 2.3 Hz, 1H), 7.71 (d, *J =* 8.7 Hz, 1H), 7.67 (d, *J =* 2.2 Hz, 1H), 7.56 - 7.48 (m, 1H), 6.86 (d, *J =* 1.0 Hz, 1H), 5.38 - 5.16 (m, 1H), 5.12 - 4.94 (m, 2H), 4.45 (dd, *J =* 7.3, 5.7 Hz, 1H), 2.34 (s, 3H), 1.57 - 1.47 (m, 3H).

### Example 50 Preparation of Compound 103 and 103D

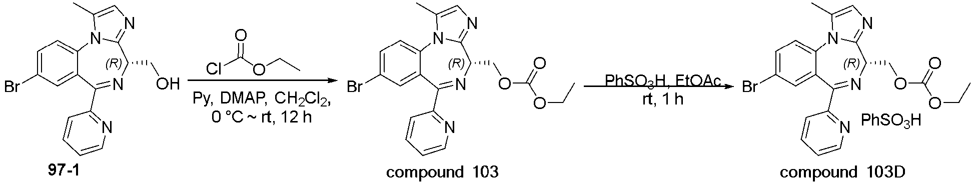

### 1. Preparation of Compound 101D

At 0°C, ethyl chloroformate (128 mg, 1.18 mmol) was added dropwise to a solution of **97-1** (150 mg, 0.391 mmol), pyridine (90.7 mg, 1.15 mmol), and DMAP (48 mg, 0.393 mmol) in dichloromethane (2 mL). The mixture was stirred at room temperature for 12 hours. After confirming reaction completion by TLC, ice-water (10 mL) was poured into the mixture, filtered and the filtrate was extracted with CH₂Cl₂ (3 × 10 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 103** as white solid (138 mg, yield 77.4%). ESI[M + H]⁺ = 455.0.

¹H NMR (400 MHz, DMSO-*d6*) δ 8.55 (dd, *J* = 3.9, 0.8 Hz, 1H), 8.05 (d, *J* = 7.9 Hz, 1H), 7.95 (td, *J* = 7.7, 1.7 Hz, 1H), 7.90 (dd, *J* = 8.7, 2.3 Hz, 1H), 7.69 - 7.66 (m, 2H), 7.51 (ddd, *J* = 7.5, 4.8, 1.2 Hz, 1H), 6.83 (d, *J =* 1.1 Hz, 1H), 4.97 - 4.92 (m, 2H), 4.39 (t, *J =* 6.6 Hz, 1H), 4.15 (q, *J =* 7.0 Hz, 2H), 2.31 (d, *J* = 0.8 Hz, 3H), 1.23 (t, *J* = 7.1 Hz, 3H).

### 2. Preparation of Compound 103D

To a solution of Compound **103** (125 mg, 0.275 mmol) in ethyl acetate (4 mL) was added solution of benzenesulfonic acid (43.6 mg, 0.276 mmol) in ethyl acetate (0.5 mL) dropwise. The mixture was stirred at room temperature for 1 hour and then filtered. The filter cake was dried under reduced pressure to yield Compound **103D** as white solid (120 mg, yield 71.2% ). ESI [M + H]⁺ = 455.0.

¹H NMR (400 MHz, DMSO-*d6*) δ 8.61 - 8.56 (m, 1H), 8.08 (d, *J =* 7.9 Hz, 1H), 8.03 - 7.94 (m, 2H), 7.80 (d, *J* = 8.7 Hz, 1H), 7.76 (d, *J* = 2.3 Hz, 1H), 7.62 - 7.49 (m, 3H), 7.36 - 7.26 (m, 4H), 4.95 (d, *J =* 6.7 Hz, 2H), 4.64 (t, *J* = 6.5 Hz, 1H), 4.17 (q, *J =* 7.1 Hz, 2H), 2.37 (d, *J =* 0.7 Hz, 3H), 1.23 (t, *J* = 7.1 Hz, 3H).

### Example 51 Preparation of Compound 104

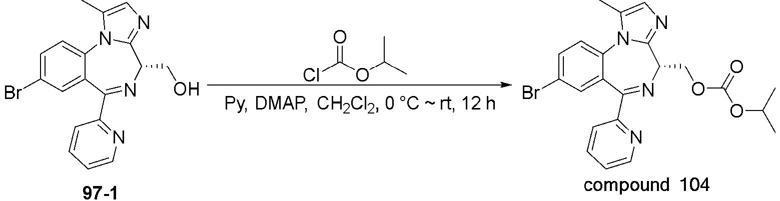

The preparation method of **compound 104** is similar to **compound 103** in **example 50,** using **97-1** and **Isopropyl chloroformate** as starting materials.

Compound**104:** 68.8 mg, ESI[M+H]⁺ = 469.0.

¹H NMR (400 MHz, DMSO-d6) δ 8.55 (dd, *J* = 3.9, 0.8 Hz, 1H), 8.05 (d, *J* = 7.9 Hz, 1H), 7.95 (td, *J* = 7.7, 1.7 Hz, 1H), 7.90 (dd, *J* = 8.7, 2.3 Hz, 1H), 7.69 - 7.66 (m, 2H), 7.51 (ddd, *J* = 7.5, 4.8, 1.2 Hz, 1H), 6.83 (d, *J* = 1.1 Hz, 1H), 5.00 - 4.88 (m, 2H), 4.86 - 4.75 (m, 1H), 4.39 (dd, *J =* 7.4, 5.7 Hz, 1H), 2.31 (d, *J* = 0.7 Hz, 3H), 1.25 (d, *J =* 6.2 Hz, 3H), 1.23 (d, *J =* 6.2 Hz, 3H).

### Example 52 Preparation of Compound 105

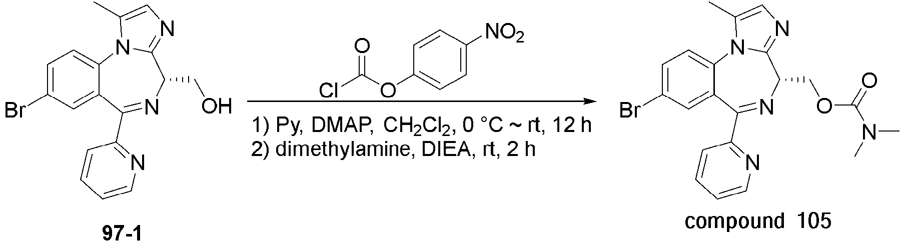

A solution of compound **97-1** (100 mg, 0.261 mmol), pyridine (61.7 mg, 0.780 mmol), and DMAP (63.6 mg, 0.521 mmol) in dichloromethane (2 mL) was treated with *p*-nitrophenyl chloroformate (157 mg, 0.779 mmol) at 0°C. The mixture was stirred at room temperature for 12 hours. After confirming reaction completion by TLC, dimethylamine (0.4 mL, 2 mol/L in THF, 0.8 mmol) and DIEA (33.6 mg, 0.26 mmol) were added. The resulting mixture was stirred at room temperature for an additional 2 hours.After confirming reaction completion by TLC, ice-water (10 mL) was poured into the mixture, the mixture was extracted with CH₂Cl₂ (3 × 10 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.3-0.4 were collected to yield **Compound 105** as white solid (47.2 mg, yield 39.8%). ESI[M + H]⁺ = 454.3.

¹H NMR (400 MHz, DMSO-*d6*) δ 8.57 (d, *J* = 4.0 Hz, 1H), 8.08 (d, *J* = 7.8 Hz, 1H), 7.99 - 7.96 (m, 1H), 7.92 (dd, *J* = 8.7, 2.3 Hz, 1H), 7.70 (d, *J =* 8.7 Hz, 1H), 7.67 (d, *J =* 2.3 Hz, 1H), 7.56 - 7.48 (m, 1H), 6.86 (s, 1H), 4.91 - 4.83 (m, 2H), 4.34 (t, *J* = 6.5 Hz, 1H), 2.85 (s, 6H), 2.33 (s, 3H).

### Example 53 Preparation of Compound 109 and Compound 110

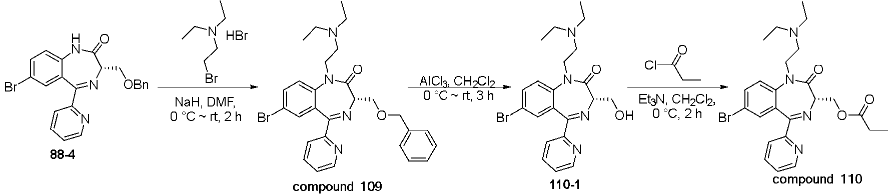

### 1. Preparation of Compound 109

A solution of **88-4** (300 mg, 0.688 mmol) in anhydrous DMF (5 mL) was treated with sodium hydride (60.6 mg, 60% dispersion in mineral oil, 1.51 mmol) at 0°C. The mixture was stirred at 0°C for 30 minutes. Then 2-(diethylamino)ethyl bromide hydrobromide (216 mg, 0.828 mmol) was added to the mixture. The mixture was stirred for 2 hours at room temperature. After confirming reaction completion by TLC, ice-water (10 mL) was poured into the mixture, the mixture was extracted with ethyl acetate (3 × 10 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield **Compound 109** as white solid (161 mg, yield 43.7%). ESI[M + H]⁺ = 535.4.

¹H NMR (400 MHz, DMSO-*d6*) δ 8.62 (d, *J* = 4.3 Hz, 1H), 8.10 (d, *J* = 8.0 Hz, 1H), 7.97 (t, *J =* 7.8 Hz, 1H), 7.81 (dd, *J* = 8.8, 2.2 Hz, 1H), 7.71 (d, *J =* 9.0 Hz, 1H), 7.60 (d, *J =* 2.2 Hz, 1H), 7.55 - 7.50 (m, 1H), 7.36 - 7.35 (m, 4H), 7.31 - 7.28 (m, 1H), 4.61 (s, 2H), 4.32 - 4.14 (m, 2H), 4.05 (dd, *J =* 9.5, 6.6 Hz, 1H), 3.84 (t, *J =* 6.5 Hz, 1H), 3.74 - 3.71 (m, 1H), 2.37 - 2.35 (m, 2H), 2.25 - 2.22 (m, 4H), 0.67 (t, *J* = 6.9 Hz, 6H).

### 2. Preparation of Compound 110-1

At 0°C, solution of **109** (161 mg, 0.301 mmol) in dichloromethane (10 mL) was treated with aluminum chloride (401 mg, 3.01 mmol) added portion-wise. The mixture was then allowed to warm to room temperature and stirred for 3 hours. After confirming reaction completion by TLC, ice-water (10 mL) was poured into the mixture, filtered and the filtrate was extracted with CH₂Cl₂ (3 × 20 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield **Compound 110-1** as white solid (84 mg, yield 62.7%). ESI[M + H]⁺ = 445.1.

### 3. Preparation of Compound 110

A solution of **110-1** (42 mg, 0.094 mmol) and triethylamine (19.1 mg, 0.189 mmol) in dichloromethane (2 mL) was treated with propionyl chloride (13.0 mg, 0.141 mmol) added dropwise at 0°C. The mixture was stirred at 0°C for 2 hours. After confirming reaction completion by TLC, ice-water (10 mL) was poured into the mixture, was extracted with CH₂Cl₂ (3 × 10 mL). The organic phase was washed with brine and dried over anhydrous Na₂SO₄, filtered and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.3-0.4 were collected to yield **Compound 110** as syrupy solid (10.6 mg, yield 22.4%). ESI[M + H]⁺ = 501.1.

¹H NMR (400 MHz, DMSO-d6) δ 8.63 (d, *J* = 4.7 Hz, 1H), 8.10 (d, *J* = 7.9 Hz, 1H), 7.97 (t, *J =* 7.0 Hz, 1H), 7.83 (dd, *J =* 8.8, 2.3 Hz, 1H), 7.76 (d, *J =* 8.9 Hz, 1H), 7.63 (d, *J =* 2.2 Hz, 1H), 7.57 - 7.51 (m, 1H), 4.73 (dd, *J =* 10.7, 6.4 Hz, 1H), 4.65 (dd, *J =* 10.8, 6.7 Hz, 1H), 4.20 (dd, *J =* 14.0, 6.9 Hz, 1H), 3.96 (t, *J* = 6.6 Hz, 1H), 3.80 - 3.70 (m, 1H), 2.38 (t, *J* = 6.0 Hz, 2H), 2.35 - 2.17 (m, 6H), 1.03 (t, *J* = 7.5 Hz, 3H), 0.66 (t, *J* = 7.1 Hz, 6H).

### Example 54 Preparation of Compound 111

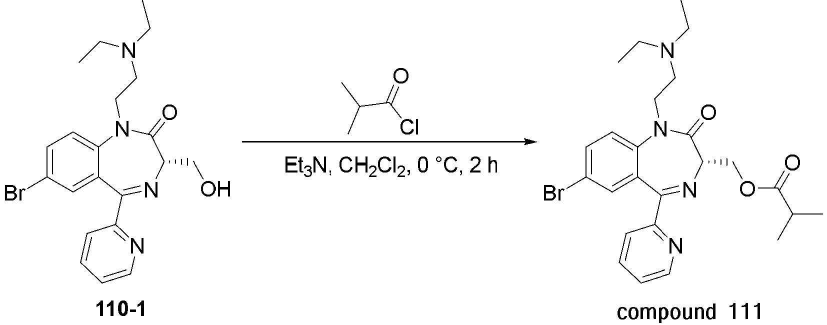

The preparation method of **compound 111** is similar to **compound 110** in **example 53,** using **110-1** and isobutyryl chloride as starting materials.

Compound **111:** 26.2 mg, ESI[M+H]⁺ = 515.2.

¹H NMR (400 MHz, DMSO-*d6*) δ 8.63 (d, *J =* 4.7 Hz, 1H), 8.10 (d, *J =* 7.8 Hz, 1H), 7.98 (t, *J =* 7.2 Hz, 1H), 7.83 (dd, *J* = 8.9, 2.2 Hz, 1H), 7.77 (d, *J =* 8.9 Hz, 1H), 7.62 (d, *J=* 2.1 Hz, 1H), 7.57 - 7.48 (m, 1H), 4.73 - 4.64 (m, 2H), 4.23 - 4.16 (m, 1H), 3.99 (t, *J* = 6.6 Hz, 1H), 3.84 - 3.64 (m, 1H), 2.57 - 2.52 (m, 1H), 2.42 - 2.38 (m, 2H), 2.32 - 2.13 (m, 4H), 1.09 (d, *J* = 2.4 Hz, 3H), 1.08 (d, *J* = 2.4 Hz, 3H), 0.68 (t, *J* = 7.0 Hz, 6H).

### Example 55 Preparation of Compound 113 and Compound 114

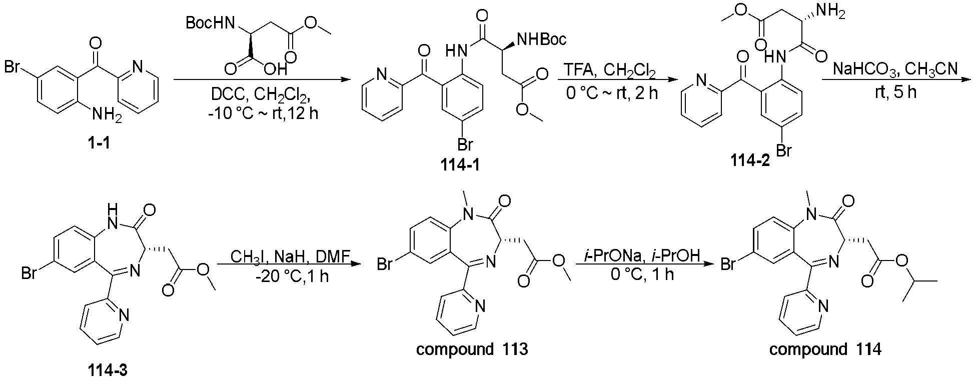

### 1. Preparation of Compound 114-1

At -10°C, solution of **1-1** (2.76 g, 9.96 mmol) and Boc-L-aspartic acid 4-methyl ester (3.70 g, 15.0 mmol) in dichloromethane (30 mL) was treated with solution of DCC (3.09 g, 15.0 mmol) in dichloromethane (10 mL). The mixture was allowed to warm to room temperature and stirred for 12 hours. After confirming reaction completion by TLC, methyl tert-butyl ether (MTBE, 40 mL) was added and the mixture was stirred for 5 minutes. The mixture was filtered and the filtrate was concentrated under reduced pressure to afford the crude product. The crude product was purified by pre-TLC (ethyl acetate/petroleum ether, v/v = 1/20-1/2), and the fractions with Rf = 0.3-0.4 were collected to yield **Compound 114-1** as white solid (3.356 g, yield 66.5%). ESI[M + H]⁺ = 506.1.

### 2. Preparation of Compound 114-2

At 0°C, trifluoroacetic acid (15 mL) was added to solution of **114-1** (3.356 g, 6.63 mmol) in dichloromethane (30 mL). The mixture was allowed to warm to room temperature and stirred for 2 hours. After confirming reaction completion by TLC, the mixture was concentrated under reduced pressure. The crude product was used directly in the next step without further purification.

### 3. Preparation of Compound 114-3

The crude product from the previous step was dissolved in acetonitrile (40 mL). To this solution was added sodium bicarbonate (31.8 g, 378.5 mmol), and the mixture was stirred at room temperature for 5 hours. After confirming reaction completion by TLC, the mixture was filtered and the filtrate was concentrated under reduced pressure to afford the crude product. The crude product was purified by silica gel column chromatography (ethyl acetate/petroleum ether, v/v = 1/10-1/2), TLC (ethyl acetate/petroleum ether, v/v =1/1) momnitoring and the fractions with Rf = 0.2-0.3 were collected to yield **Compound 114-3** as white solid (2.48 g, two steps yield 96.4%). ESI[M + H]⁺ = 388.1.

### 4. Preparation of Compound 113

A solution of **114-3** (2.48 g, 6.39 mmol) in anhydrous DMF (30 mL) was cooled to -20°C Sodium hydride (320 mg, 60% dispersion in mineral oil, 8.0 mmol) was added portion-wise at this temperature, and the mixture was stirred for 30 minutes. Iodomethane (1.136 g, 8.0 mmol) was then added, and stirred at -20°C for 1 hour. After confirming reaction completion by TLC, ice-water (60 mL) was poured into the mixture, the mixture was extracted with ethyl acetate (3 × 50 mL). The organic phase was washed with brine and dried over anhydrous Na₂SO₄, filtered and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring to yield **Compound 113** as white solid (2.459 g, yield 95.7%). ESI[M + H]⁺ = 402.0.

¹H NMR (400 MHz, DMSO-d6) δ 8.62 - 8.58 (m, 1H), 8.03 (d, *J* = 7.9 Hz, 1H), 7.96 (td, *J* = 7.7, 1.7 Hz, 1H), 7.85 (dd, *J =* 8.9, 2.4 Hz, 1H), 7.58 (d, *J =* 8.9 Hz, 1H), 7.55 - 7.49 (m, 2H), 4.09 (t, *J* = 7.1 Hz, 1H), 3.61 (s, 3H), 3.32 (s, 3H), 3.25 (dd, *J* = 16.6, 7.5 Hz, 1H), 3.09 (dd, *J* = 16.6, 6.6 Hz, 1H).

### 5. Preparation of Compound 114

A solution of **113** (200 mg, 0.497 mmol) in isopropanol (2 mL) was cooled to 0°C. Sodium isopropoxide (307 mg, 20% in THF, 0.748 mmol) was added dropwise at 0°C. The mixture was then allowed to warm to room temperature and stirred for 1 hour. After confirming reaction completion by TLC, ice-water (10 mL) was poured into the mixture, the mixture was extracted with ethyl acetate (3 × 10 mL). The organic phase was washed with brine and dried over anhydrous Na₂SO₄, filtered and the filtrate was concentrated under reduced pressure to afford the crude product, the crude product was purified by silica gel column chromatography (ethyl acetate/petroleum ether, v/v = 1/1), and the fractions with Rf = 0.2-0.3 were collected to yield **Compound 114** as white solid (71.0 mg, yield 33.2%). ESI[M + H]⁺ = 430.0.

¹H NMR (400 MHz, DMSO-*d6*) δ 8.59 (ddd, *J* = 4.8, 1.7, 0.9 Hz, 1H), 8.05 - 8.00 (m, 1H), 7.99 - 7.93 (m, 1H), 7.84 (dd, *J* = 8.9, 2.4 Hz, 1H), 7.58 (d, *J* = 8.9 Hz, 1H), 7.55 - 7.49 (m, 2H), 4.92 - 4.86 (m, 1H), 4.08 (t, *J* = 7.1 Hz, 1H), 3.31 (s, 3H), 3.18 (dd, *J* = 16.5, 7.5 Hz, 1H), 3.04 (dd, *J =* 16.5, 6.8 Hz, 1H), 1.24 - 1.14 (m, 6H).

### Example 56 Preparation of Compound 115

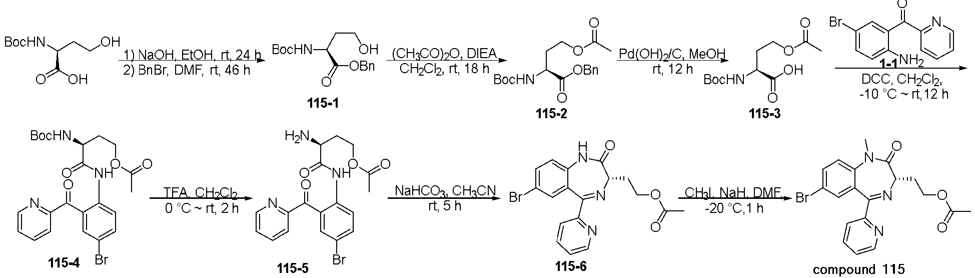

### 1. Preparation of Compound 115-1

N-Boc-L-homoserine (9.0 g, 41.1 mmol) was dissolved in ethanol (70 mL). Then the mixture was added the solution of sodium hydroxide (1.769 g, 44.2 mmol) in water (21 mL). The mixture was stirred at room temperature for 24 hours and then concentrated under reduced pressure. The resulting white solid was dissolved in DMF (42 mL). Benzyl bromide (14 g, 81.9 mmol) was added to the resulting solution, and stirred at room temperature for 46 hours. After confirming reaction completion by TLC, ice-water (50 mL) was poured into the mixture, the mixture was extracted with ethyl acetate (3 × 30 mL). The organic phase was washed with brine and dried over anhydrous Na₂SO₄, filtered and the filtrate was concentrated under reduced pressure to afford the crude product, the crude product was purified by silica gel column chromatography (ethyl acetate/petroleum ether, v/v = 1/20-1/2), and the fractions with Rf = 0.3-0.4 were collected to yield **Compound 115-1** as white solid (2.34 g, yield 18.4%). ESI[M + H]⁺ = 310.1.

### 2. Preparation of Compound 115-2

A solution of **115-1** (2.34 g, 7.56 mmol) in dichloromethane (31 mL) was treated with DIEA (2.14 g, 16.6 mmol) and acetic anhydride (1.53 g, 15.0 mmol). The mixture was stirred at room temperature for 18 hours. After confirming reaction completion by TLC, ice-water (50 mL) was poured into the mixture, the mixture was extracted with dichloromethane (3 × 30 mL). The organic phase was washed with 1% aqueous HCl solution and brine, dried over anhydrous Na₂SO₄, filtered and the filtrate was concentrated under reduced pressure to yield **Compound 115-2** as white solid (1.2 g, yield 45.1%). ESI[M + H]⁺ = 352.2.

### 3. Preparation of Compound 115-3

A solution of **115-2** (1.2 g, 3.41 mmol) in methanol (10 mL) was treated with palladium hydroxide on carbon (200 mg, 10% Pd) under a hydrogen atmosphere. The reaction mixture was stirred at room temperature for 12 hours. After confirming reaction completion by TLC, the mixture was filtered and the filtrate was concentrated under reduced pressure to afford the crude product, the crude product was purified by silica gel column chromatography (ethyl acetate/petroleum ether, v/v = 1/20-1/1), and the fractions with Rf = 0.2-0.3 were collected to yield **Compound 115-3** as white solid (769 mg, yield 86.2%). ESI[M + H]⁺ = 262.1.

### 4. Preparation of Compound 115-4

**1-1** (1.2 g, 4.33 mmol) and **115-3** (769 mg, 2.94 mmol) were dissolved in dichloromethane (12 mL). A solution of DCC (910 mg, 4.41 mmol) in dichloromethane (2 mL) was added dropwise at -10 °C. The reaction mixture was allowed to warm to room temperature and stirred for 12 hours. After confirming reaction completion by TLC, methyl tert-butyl ether (14 mL) was added and the mixture was stirred for 5 min.The mixture was filtered, and the filtrate was concentrated under reduced pressure to afford the crude product, the crude product was purified by silica gel column chromatography (ethyl acetate/petroleum ether, v/v = 1/20-1/2), TLC (ethyl acetate/petroleum ether, v/v =1/1) momnitoring and the fractions with Rf = 0.3-0.4 were collected to yield **Compound 115-4** as white solid (1.42 g, yield 92.7%). ESI[M + H]⁺ = 520.1.

### 5. Preparation of Compound 115-5

At 0°C, trifluoroacetic acid (7.3 mL) was added to a solution of **115-4** (1.42 g, 2.73 mmol) in dichloromethane (14.6 mL). The reaction mixture was allowed to warm to room temperature naturally and was stirred for 2 hours. After confirming reaction completion by TLC, the mixture was concentrated under reduced pressure. The crude product was used directly in the next step without further purification.

### 6. Preparation of Compound 115-6

The crude product from the previous step was dissolved in acetonitrile (30 mL). Sodium bicarbonate (8.62 g, 102.6 mmol) was added, and the mixture was stirred at room temperature for 5 hours. After confirming reaction completion by TLC, the mixture was filtered, and the filtrate was concentrated under reduced pressure to afford the crude product, the crude product was purified by silica gel column chromatography (ethyl acetate/petroleum ether, v/v = 1/10-1/2), TLC (ethyl acetate/petroleum ether, v/v =1/1) momnitoring and the fractions with Rf = 0.2-0.3 were collected to yield **Compound 115-6** as white solid (525 mg, two steps yield 47.8%). ESI[M + H]⁺ = 402.1.

### 7. Preparation of Compound 115

At -20°C, sodium hydride (78.5 mg, 60% dispersion in mineral oil, 1.96 mmol) was added slowly to solution of **115-6** (525 mg, 1.31 mmol) in dry DMF (10 mL). The mixture was stirred at -20°C for 30 minutes. Then, methyl iodide (279 mg, 1.97 mmol) was added to the reaction mixture at -20 °C, and stirred for 1 hour at the -20°C. After confirming reaction completion by TLC, ice-water (20 mL) was poured into the mixture, the mixture was extracted with ethyl acetate (3 × 20 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.3-0.4 were collected to yield **Compound 115** as white solid (395 mg, yield 72.7%). ESI[M + H]⁺ =416.0.

¹H NMR (400 MHz, DMSO-*d6*) δ 8.58 (d, *J =* 4.0 Hz, 1H), 8.09 (d, *J =* 7.9 Hz, 1H), 7.97 (td, *J =* 7.7, 1.8 Hz, 1H), 7.82 (dd, *J* = 8.9, 2.4 Hz, 1H), 7.59 - 7.47 (m, 3H), 4.26 - 4.16 (m, 2H), 3.76 (dd, *J =* 7.9, 5.7 Hz, 1H), 3.31 (s, 3H), 2.41 - 2.27 (m, 2H), 1.94 (s, 3H).

### Example 57 Preparation of Compound 116

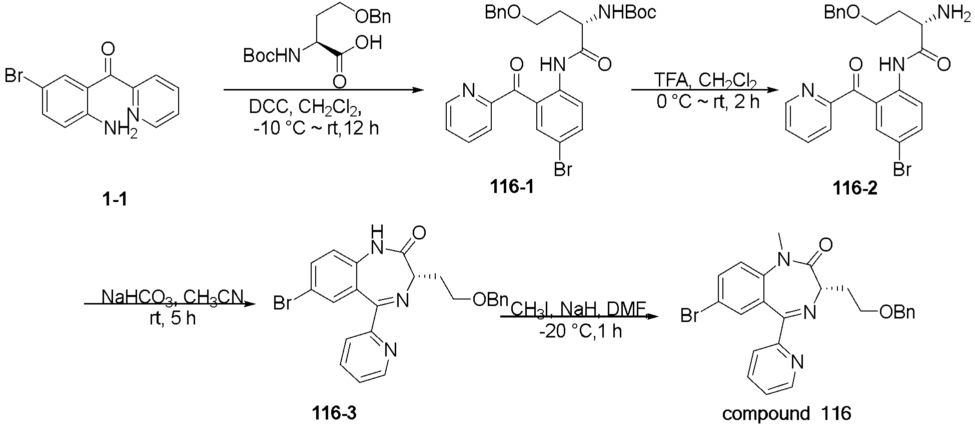

### 1. Preparation of Compound 116-1

**1-1** (4.46 g, 16.1 mmol) and Boc-O-benzyl-L-homoserine (5.0 g, 16.2 mmol) were dissolved in dichloromethane (45 mL). A solution of DCC (5.0 g, 24.2 mmol) in dichloromethane (15 mL) was added dropwise at -10 °C. The reaction mixture was allowed to warm to room temperature and stirred for 12 hours. After confirming reaction completion by TLC, methyl tert-butyl ether (60 mL) was added, and the mixture was stirred for 5 minutes. The reaction mixture was filtered, and the filtrate was concentrated under reduced pressure to afford the crude product. The crude product was purified by silica gel column chromatography (ethyl acetate/petroleum ether, v/v = 1/20-1/2), TLC (ethyl acetate/petroleum ether, v/v =1/2) momnitoring and the fractions with Rf = 0.3-0.4 were collected to yield **Compound 116-1** as white solid (4.037g, yield44.1%). ESI[M+H]⁺ = 568.1.

### 2. Preparation of Compound 116-2

At 0 °C, trifluoroacetic acid (10.1 mL) was added to a solution of **116-1** (4.037 g, 7.10 mmol) in dichloromethane (30.3 mL). The reaction mixture was allowed to warm to room temperature naturally and was stirred for 2 hours. After confirming reaction completion by TLC, the mixture was concentrated under reduced pressure. The crude product was used directly in the next step without further purification.

### 3. Preparation of Compound 116-3

The crude product from the previous step was dissolved in acetonitrile (50 mL). Sodium bicarbonate (22.0 g, 261.9 mmol) was added, and the mixture was stirred at room temperature for 5 hours. After confirming reaction completion by TLC, the mixture was filtered. The filtrate was concentrated under reduced pressure to afford a crude product, which was purified by column chromatography (ethyl acetate/petroleum ether (v/v) = 1/10 to 1/1). The fractions with Rf = 0.2 - 0.3 (TLC, ethyl acetate/petroleum ether (v/v) = 1/1) were collected and concentrated to give the title compound **116-3** as a white solid (3.029 g, two steps yield 94.7%). ESI[M + H]⁺ = 450.1.

### 4. Preparation of Compound 116

At -20°C, sodium hydride (385.7 mg, 60% dispersion in mineral oil, 9.64 mmol) was added to a solution of **116-3** (3.029 g, 6.73 mmol) in dry DMF (30 mL). The mixture was stirred at this temperature for 30 minutes. Methyl iodide (1.053 g, 7.42 mmol) was then added to the mixture at -20 °C, and stirred for 1 hour at the same temperature. After confirming reaction completion by TLC, ice-water (50 mL) was poured into the mixture, the mixture was extracted with ethyl acetate (3 × 50 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 116** as white solid (2.903 g, yield 92.9%). ESI[M + H]⁺ = 464.3.

¹H NMR (400 MHz, DMSO-*d6*) δ 8.60 (d, *J* = 4.7 Hz, 1H), 8.00 - 7.93 (m, 2H), 7.84 (dd, *J* = 8.9, 2.2 Hz, 1H), 7.56 - 7.52 (m, 2H), 7.49 (d, *J* = 2.2 Hz, 1H), 7.29 - 7.22 (m, 3H), 7.17 (d, *J* = 7.4 Hz, 2H), 4.46 (d, *J* = 12.3 Hz, 1H), 4.38 (d, *J* = 12.4 Hz, 1H), 3.85 - 3.77 (m, 1H), 3.65 - 3.64 (m, 2H), 3.31 (s, 3H), 2.40 - 2.28 (m, 2H).

### Example 58 Preparation of Compound 117

### Method A:

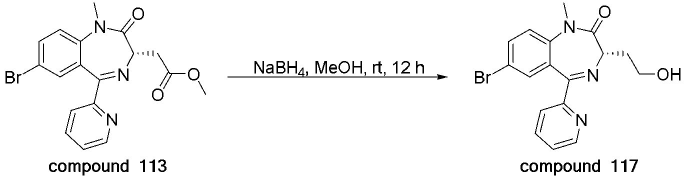

### Method B:

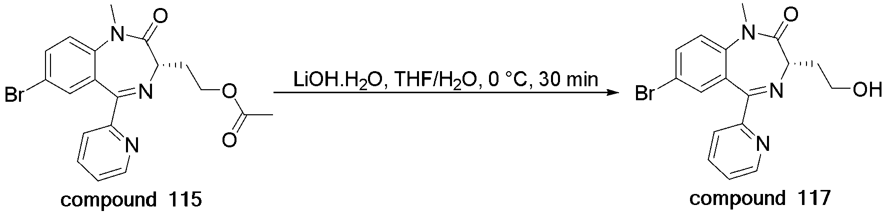

### Method C:

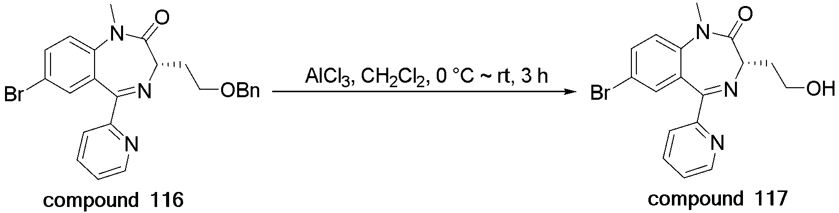

### Method A:

Sodium borohydride (581 mg, 15.4 mmol) was added portionwise to a solution of **113** (2.045 g, 5.08 mmol) in methanol (30 mL). The reaction mixture was stirred at room temperature for 12 hours. After confirming reaction completion by TLC, ice-water (50 mL) was poured into the mixture, the mixture was extracted with ethyl acetate (3 × 40 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.2-0.3 were collected to yield **Compound 117** as white solid (439 mg, yield 23.1%).

### Method B:

At 0°C, LiOH·H₂O (49 mg, 1.17 mmol) was added to a solution of **115** (323 mg, 0.776 mmol) in THF/H₂O (5 mL, v/v = 1:1). The mixture was stirred at this temperature for 30 minutes. After confirming reaction completion by TLC, ice-water (50 mL) was poured into the mixture, the mixture was extracted with ethyl acetate (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.2-0.3 were collected to yield **Compound 117** as white solid (249 mg, yield 85.7%).

### Method C:

At 0°C, aluminum chloride (5.83 g, 43.7 mmol) was added slowly to a solution of **116** (2.903 g, 6.25 mmol) in dichloromethane (70 mL). The reaction mixture was then allowed to warm to room temperature and stirred for 3 hours. After confirming reaction completion by TLC, ice-water (70 mL) was poured into the mixture, the mixture was extracted with CH₂Cl₂ (3 × 70 mL). The organic phase was washed with 5% aqueous sodium bicarbonate solution and brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.2-0.3 were collected to yield **Compound 117** as white solid (2.08 g, yield 88.9%). ESI[M + H]⁺ = 374.0.

¹H NMR (400 MHz, DMSO-*d6*) δ 8.58 (ddd, *J* = 4.8, 1.7, 0.9 Hz, 1H), 8.09 (d, *J* = 7.9 Hz, 1H), 7.96 (td, *J* = 7.7, 1.8 Hz, 1H), 7.81 (dd, *J =* 8.8, 2.4 Hz, 1H), 7.57 - 7.48 (m, 3H), 4.40 (t, *J* = 5.2 Hz, 1H), 3.79 (t, *J =* 6.9 Hz, 1H), 3.59 (q, *J* = 6.0 Hz, 2H), 3.30 (s, 3H), 2.21 (q, *J* = 6.7 Hz, 2H).

### Example 59 Preparation of Compound 118

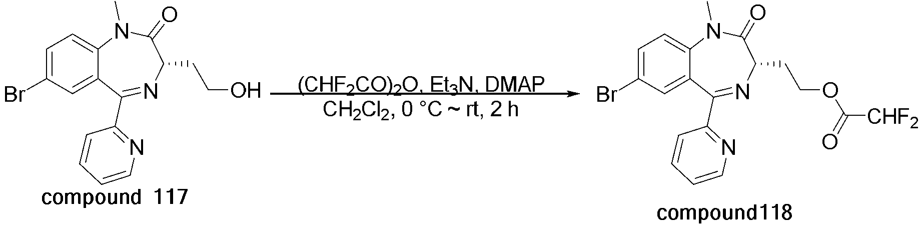

At 0°C, difluoroacetic anhydride (112 mg, 0.643 mmol) was added dropwise to a solution of **117** (120 mg, 0.321 mmol), triethylamine (65.0 mg, 0.642 mmol), and DMAP (78.5 mg, 0.643 mmol) in dichloromethane (2 mL). The reaction mixture was allowed to warm to room temperature and stirred for 2 hours. After confirming reaction completion by TLC, ice-water (5 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 118** as white solid (24.0 mg, yield 16.5%). ESI[M + H]⁺ = 452.0.

¹H NMR (400 MHz, DMSO-*d6*) δ 8.58 (ddd, *J =* 4.8, 1.7, 0.9 Hz, 1H), 8.11 (d, *J* = 7.9 Hz, 1H), 7.97 (td, *J =* 7.7, 1.8 Hz, 1H), 7.82 (dd, *J* = 8.9, 2.4 Hz, 1H), 7.56 - 7.50 (m, 2H), 7.49 (d, *J* = 2.4 Hz, 1H), 6.37 (t, *J* = 52.7 Hz, 1H), 4.51 - 4.46 (m, 2H), 3.82 (dd, *J* = 8.0, 5.6 Hz, 1H), 3.31 (s, 3H), 2.48 - 2.37 (m, 2H).

### Example 60 Preparation of Compound 120

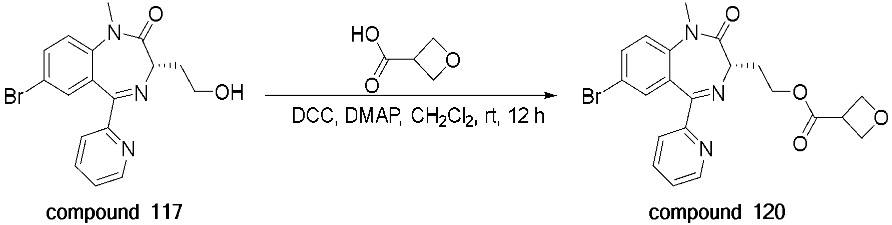

3-Oxetane carboxylic acid (41 mg, 0.402 mmol), DCC (82.8 mg, 0.401 mmol), and DMAP (49.1 mg, 0.402 mmol) were dissolved in dichloromethane (2 mL), and the mixture was stirred at room temperature for 15 minutes. **117** (100 mg, 0.267 mmol) was then added to the mixture, and was stirred at room temperature for 12 hours. After confirming reaction completion by TLC, methyl tert-butyl ether (2 mL) was added. The mixture was stirred for 5 minutes, filtered, and the filtrate was concentrated under reduced pressure to afford the crude product. the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.2-0.3 were collected to yield **Compound 120** as white solid (46.1 mg, yield 37.6%). ESI[M + H]⁺ = 458.0.

¹H NMR (400 MHz, DMSO-*d6*) δ 8.58 (ddd, *J* = 4.8, 1.7, 0.9 Hz, 1H), 8.10 - 8.08 (m, 1H), 7.97 (td, *J* = 7.7, 1.8 Hz, 1H), 7.81 (dd, *J =* 8.9, 2.4 Hz, 1H), 7.56 - 7.48 (m, 3H), 4.65 - 4.61 (m, 2H), 4.53 - 4.48 (m, 2H), 4.39 - 4.25 (m, 2H), 3.87 - 3.81 (m, 1H), 3.80 - 3.71 (m, 1H), 3.31 (s, 3H), 2.45 - 2.32 (m, 2H).

### Example 61 Preparation of Compound 121-123

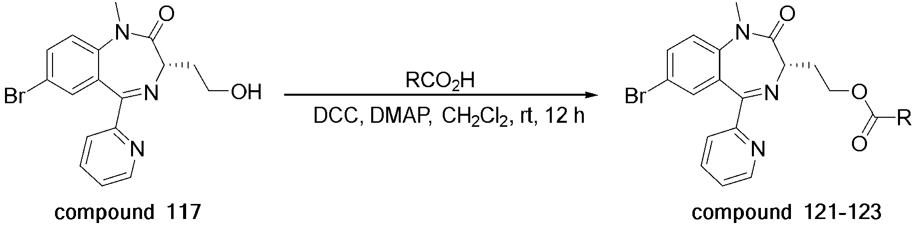

The preparation method of **compound 121-123 are** similar to **compound 120 in example 60,** using **117** and **the appropriate carboxylic acid** as starting materials to yield the corresponding ester.

Compound**121:** 80.5 mg, ESI[M+H]⁺ = 442.0.

¹H NMR (400 MHz, DMSO-*d6*) δ 8.61 - 8.56 (m, 1H), 8.10 (d, *J* = 7.9 Hz, 1H), 7.96 (td, *J* = 7.7, 1.8 Hz, 1H), 7.82 (dd, *J* = 8.8, 2.4 Hz, 1H), 7.56 - 7.47 (m, 3H), 4.24 (t, *J* = 6.8 Hz, 2H), 3.73 (dd, *J =* 8.1, 5.5 Hz, 1H), 3.31 (s, 3H), 2.45 - 2.28 (m, 2H), 1.58 - 1.51 (m, 1H), 0.85 - 0.76 (m, 2H), 0.75 - 0.65 (m, 2H).

Compound**122:** 22.7 mg, ESI[M+H]⁺ = 448.0.

¹H NMR (400 MHz, DMSO-*d6*) δ 8.62 - 8.56 (m, 1H), 8.13 - 8.08 (m, 1H), 8.01 - 7.92 (m, 1H), 7.82 (dd, *J* = 8.9, 2.4 Hz, 1H), 7.54 - 7.51 (m, 2H), 7.49 (t, *J* = 2.4 Hz, 1H), 5.23 - 5.03 (m, 1H), 4.40 - 4.33 (m, 2H), 3.82 - 3.72 (m, 1H), 3.31 (s, 3H), 2.47 - 2.34 (m, 2H), 1.40 - 1.30 (m, 3H).

Compound**123:** 18.0 mg, ESI[M+H]⁺ = 484.0.

¹H NMR (400 MHz, DMSO-d6) δ 8.62 - 8.55 (m, 1H), 8.10 (d, *J* = 7.9 Hz, 1H), 7.97 (td, *J =* 7.7, 1.8 Hz, 1H), 7.82 (dd, *J =* 8.9, 2.4 Hz, 1H), 7.55 - 7.50 (m, 2H), 7.48 (d, *J =* 2.4 Hz, 1H), 4.37 - 4.33 (m, 2H), 3.78 (dd, *J* = 8.3, 5.4 Hz, 1H), 3.62 (q, *J =* 11.0 Hz, 2H), 3.31 (s, 3H), 2.45 - 2.33 (m, 2H).

### Example 62 Preparation of Compound 124

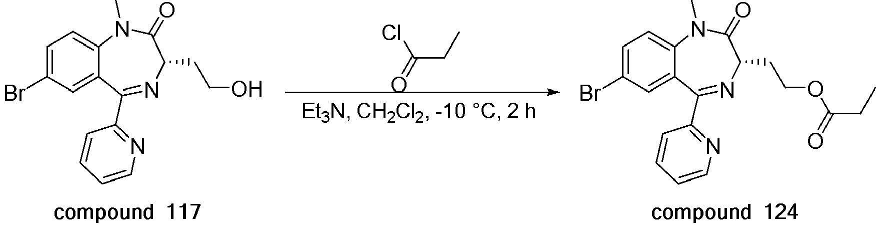

At -10°C, propionyl chloride (21 mg, 0.227 mmol) was added dropwise to a solution of **117** (56.7 mg, 0.152 mmol) and triethylamine (30.7 mg, 0.303 mmol) in dichloromethane (2 mL). The mixture was stirred at -10°C for 2 hours. After confirming reaction completion by TLC, ice-water (50 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 124** as white solid (37.3 mg, yield 57.2%). ESI[M + H]⁺ = 430.0.

¹H NMR (400 MHz, DMSO-*d6*) δ 8.61 - 8.56 (m, 1H), 8.09 (d, *J =* 7.9 Hz, 1H), 7.97 (td, *J* = 7.7, 1.8 Hz, 1H), 7.81 (dd, *J* = 8.9, 2.4 Hz, 1H), 7.55 - 7.45 (m, 3H), 4.30 - 4.16 (m, 2H), 3.75 (dd, *J =* 8.0, 5.7 Hz, 1H), 3.31 (s, 3H), 2.42 - 2.29 (m, 2H), 2.23 (q, *J =* 7.5 Hz, 2H), 0.93 (t, *J* = 7.5 Hz, 3H).

### Example 63 Preparation of Compound 125-129

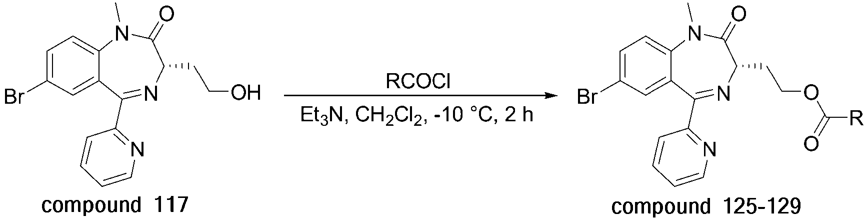

The preparation method of **compound 125-129 are** similar to **compound 124** in **example 62,** using **117** and **corresponding acid chloride** as starting materials to yield the corresponding ester.

Compound **125:** 49.1 mg, ESI[M+H]⁺ = 444.1.

¹H NMR (400 MHz, DMSO-*d6*) δ 8.58 (ddd, *J* = 4.8, 1.7, 0.9 Hz, 1H), 8.10 (d, *J* = 7.9 Hz, 1H), 7.97 (td, *J =* 7.7, 1.8 Hz, 1H), 7.81 (dd, *J* = 8.9, 2.4 Hz, 1H), 7.56 - 7.50 (m, 2H), 7.48 (d, *J* = 2.4 Hz, 1H), 4.34 - 4.16 (m, 2H), 3.73 (dd, *J* = 8.2, 5.6 Hz, 1H), 3.31 (s, 3H), 2.45 - 2.31 (m, 3H), 0.95 (d, *J* = 7.1 Hz, 3H), 0.93 (d, *J* = 7.1 Hz, 3H).

Compound **126:** 17.6 mg, ESI[M+H]⁺ = 484.0.

¹H NMR (400 MHz, DMSO-d6) δ 8.59 - 8.58 (m, 1H), 8.12 (d, *J =* 7.9 Hz, 1H), 7.97 (td, *J=* 7.7, 1.8 Hz, 1H), 7.81 (dd, *J* = 8.9, 2.4 Hz, 1H), 7.54 - 7.52 (m, 2H), 7.46 (d, *J =* 2.4 Hz, 1H), 6.81 (s, 1H), 4.58 - 4.41 (m, 2H), 3.81 (dd, *J* = 8.4, 5.4 Hz, 1H), 3.31 (s, 3H), 2.48 - 2.32 (m, 2H).

Compound **127:** 33.1 mg, ESI[M+H]⁺ = 450.0.

¹H NMR (400 MHz, DMSO-*d6*) δ 8.59 (ddd, *J* = 4.8, 1.7, 0.9 Hz, 1H), 8.10 (d, *J* = 7.9 Hz, 1H), 7.97 (td, *J* = 7.7*,* 1.8 Hz, 1H), 7.82 (dd, *J* = 8.9, 2.4 Hz, 1H), 7.55 - 7.51 (m, 2H), 7.49 (d, *J* = 2.4 Hz, 1H), 4.41 - 4.33 (m, 2H), 4.32 (s, 2H), 3.81 (dd, *J* = 8.0, 5.6 Hz, 1H), 3.31 (s, 3H), 2.46 - 2.35 (m, 2H).

Compound **128:** 21.4 mg, ESI[M+H]⁺ = 492.0.

¹H NMR (400 MHz, DMSO-*d6*) δ 8.61 - 8.56 (m, 1H), 8.10 (d, *J =* 7.9 Hz, 1H), 7.97 (td, *J* = 7.7, 1.8 Hz, 1H), 7.80 (dd, *J* = 8.9, 2.4 Hz, 1H), 7.57 - 7.49 (m, 2H), 7.44 (d, *J* = 2.4 Hz, 1H), 4.39 - 4.33 (m, 1H), 4.28 - 4.22 (m, 1H), 3.79 (dd, *J* = 8.3, 5.5 Hz, 1H), 3.54 (s, 2H), 3.31 (s, 3H), 2.46 - 2.29 (m, 2H), 1.07 (s, 3H), 1.06 (s, 3H).

Compound **129:** 24.5 mg, ESI[M+H]⁺ = 517.9.

¹H NMR (400 MHz, DMSO-*d6*) δ 8.61 - 8.57 (m, 1H), 8.14 (d, *J =* 7.9 Hz, 1H), 7.98 (td, *J* = 7.7, 1.8 Hz, 1H), 7.81 (dd, *J =* 8.9, 2.4 Hz, 1H), 7.54 - 7.52 (m, 2H), 7.46 (d, *J* = 2.4 Hz, 1H), 4.74 - 4.57 (m, 2H), 3.84 (dd, *J* = 8.2, 5.4 Hz, 1H), 3.31 (s, 3H), 2.55 - 2.44 (m, 2H).

### Example 64 Preparation of Compound 130

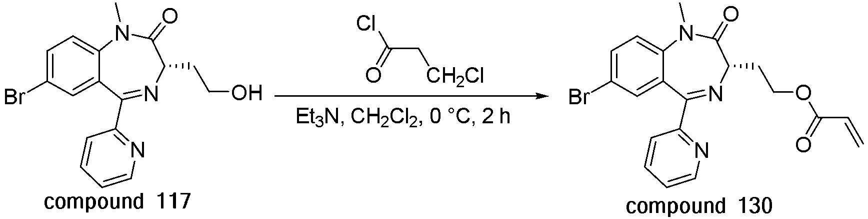

At 0°C, 3-chloropropionyl chloride (26.0 mg, 0.205 mmol) was added dropwise to a solution of **117** (50 mg, 0.134 mmol) and triethylamine (27.0 mg, 0.267 mmol) in dichloromethane (2 mL). The mixture was stirred at this temperature for 2 hours. After confirming reaction completion by TLC, ice-water (5 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 130** as white solid (22.1 mg, yield 38.6%). ESI[M + H]⁺ = 428.0.

¹H NMR (400 MHz, DMSO-*d6*) δ 8.61 - 8.57 (m, 1H), 8.10 (d, *J* = 7.9 Hz, 1H), 7.97 (td, *J* = 7.7, 1.8 Hz, 1H), 7.81 (dd, *J* = 8.9, 2.4 Hz, 1H), 7.55 - 7.50 (m, 2H), 7.49 (d, *J* = 2.4 Hz, 1H), 6.26 (dd, *J =* 17.3, 1.7 Hz, 1H), 6.12 (dd, *J* = 17.3, 10.2 Hz, 1H), 5.89 (dd, *J* = 10.2, 1.7 Hz, 1H), 4.39 - 4.23 (m, 2H), 3.79 (dd, *J* = 8.1, 5.5 Hz, 1H), 3.31 (s, 3H), 2.48 - 2.36 (m, 2H).

### Example 65 Preparation of Compound 131

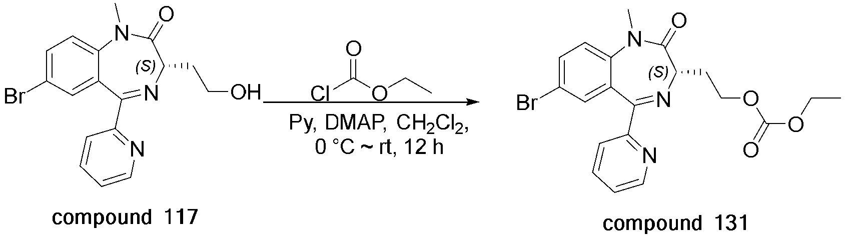

At 0°C, ethyl chloroformate (87.3 mg, 0.804 mmol) was added dropwise to a solution of **117** (100 mg, 0.267 mmol), pyridine (61.9 mg, 0.783 mmol), and DMAP (32.7 mg, 0.268 mmol) in dichloromethane (2 mL). The reaction mixture was allowed to warm to room temperature and stirred for 12 hours. After confirming reaction completion by TLC, ice-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 131** as white solid (90.0 mg, yield 75.5%). ESI[M + H]⁺ = 446.0.

¹H NMR (400 MHz, DMSO-*d6*) δ 8.58 (d, *J* = 4.7 Hz, 1H), 8.11 (d, *J =* 7.8 Hz, 1H), 7.97 (t, *J =* 7.8 Hz, 1H), 7.82 (dd, *J* = 8.9, 2.2 Hz, 1H), 7.57 - 7.46 (m, 3H), 4.31 (t, *J* = 6.5 Hz, 2H), 4.05 (q, *J* = 7.0 Hz, 2H), 3.75 (dd, *J* = 8.2, 5.4 Hz, 1H), 3.31 (s, 3H), 2.44 - 2.27 (m, 2H), 1.14 (t, *J* = 7.1 Hz, 3H).

### Example 66 Preparation of Compound 132

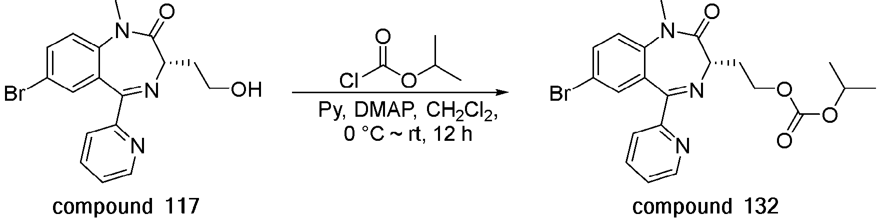

The preparation method of **compound 132 is** similar to **compound 131** in **example 65,** using **117** and **isopropyl chloroformate as** starting materials.

Compound**132**: 84.5 mg, ESI[M+H]⁺ = 460.1.

¹H NMR (400 MHz, DMSO-*d6*) δ 8.61 - 8.55 (m, 1H), 8.11 (d, *J* = 7.9 Hz, 1H), 7.97 (td, *J* = 7.7, 1.8 Hz, 1H), 7.82 (dd, *J* = 8.9, 2.4 Hz, 1H), 7.55 - 7.50 (m, 2H), 7.48 (d, *J* = 2.4 Hz, 1H), 4.76 - 4.61 (m, 1H), 4.32 - 4.29 (m, 2H), 3.73 (dd, *J* = 8.3, 5.4 Hz, 1H), 3.31 (s, 3H), 2.45 - 2.24 (m, 2H), 1.15 (d, *J* = 5.0 Hz, 3H), 1.14 (d, *J* = 5.0 Hz, 3H).

### Example 67 Preparation of Compound 133-135

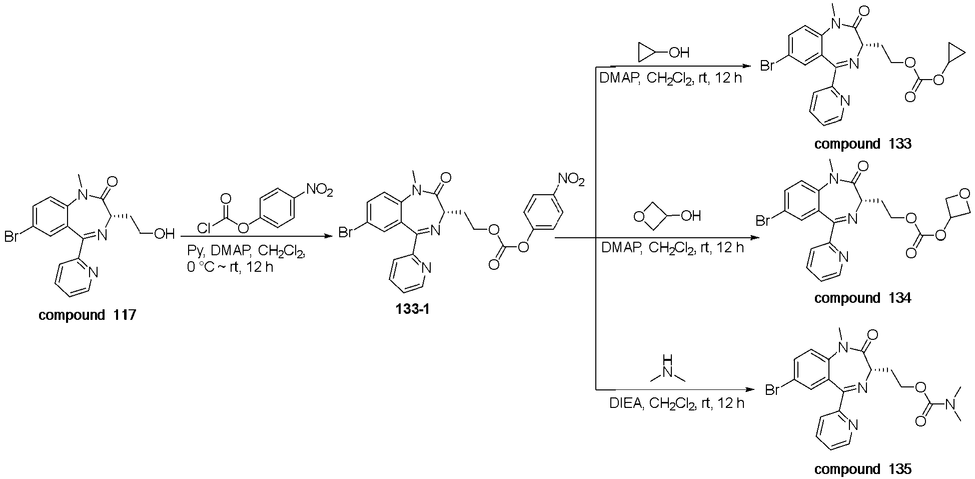

### 1. Preparation of Compound 133-1

At 0°C, *p*-nitrophenyl chloroformate (810 mg, 4.02 mmol) was added to a solution of **117** (500 mg, 1.34 mmol), pyridine (310 mg, 3.92 mmol), and DMAP (163.5 mg, 1.34 mmol) in dichloromethane (5 mL). The reaction mixture was allowed to warm to room temperature and stirred for 12 hours. After confirming reaction completion by TLC, ice-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.3-0.4 were collected to yield **Compound 133-1** as white solid (369.9 mg, yield 51.3%). ESI[M + H]⁺ = 539.1.

### 2. Preparation of Compound 133

Cyclopropanol (10.8 mg, 0.186 mmol) was added to a solution of **133-1** (100 mg, 0.185 mmol) and DMAP (22.6 mg, 0.185 mmol) in dichloromethane (2 mL). The mixture was stirred at room temperature for 12 hours. After confirming reaction completion by TLC, ice-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.2-0.3 were collected to yield **Compound 133** as white solid (56.1 mg, yield 66.0%). ESI[M + H]⁺ = 458.0.

¹H NMR (400 MHz, DMSO-d6) δ 8.58 (ddd, *J =* 4.8, 1.7, 0.9 Hz, 1H), 8.11 (d, *J* = 7.9 Hz, 1H), 7.97 (td, *J =* 7.7, 1.8 Hz, 1H), 7.82 (dd, *J* = 8.9, 2.4 Hz, 1H), 7.56 - 7.50 (m, 2H), 7.49 (d, *J* = 2.4 Hz, 1H), 4.32 (t, *J =* 6.6 Hz, 2H), 4.04 - 3.94 (m, 1H), 3.74 (dd, *J =* 8.2, 5.4 Hz, 1H), 3.31 (s, 3H), 2.46 - 2.32 (m, 2H), 0.67 - 0.54 (m, 4H).

### 3. Preparation of Compound 134

The preparation method of compound **134** is similar to compound **133** in example 67, using **133-1** and **Oxetan-3-ol** as starting materials.

**Compound134:** 56.7 mg, ESI[M+H]⁺ = 474.0.

¹H NMR (400 MHz, DMSO-d6) δ 8.58 (ddd, *J* = 4.8, 1.7, 0.9 Hz, 1H), 8.14 - 8.09 (m, 1H), 7.97 (td, *J* = 7.7*,* 1.8 Hz, 1H), 7.82 (dd, *J* = 8.9, 2.4 Hz, 1H), 7.56 - 7.48 (m, 3H), 5.34 - 5.24 (m, 1H), 4.76 - 4.67 (m, 2H), 4.50 - 4.39 (m, 2H), 4.36 - 4.32 (m, 2H), 3.77 (dd, *J* = 8.2, 5.4 Hz, 1H), 3.31 (s, 3H), 2.44 - 2.34 (m, 2H).

### 4. Preparation of Compound 135

**133-1** (100 mg, 0.185 mmol) and DIEA (24.0 mg, 0.186 mmol) were dissolved in dichloromethane (2 mL). Dimethylamine (0.1 mL, 2 mol/L in THF, 0.2 mmol) was then added, and the resulting mixture was stirred at room temperature for 12 hours. After confirming reaction completion by TLC, ice-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.2-0.3 were collected to yield **Compound 135** as white solid (54.5 mg, yield 66.0%). ESI[M + H]⁺ = 445.0.

¹H NMR (400 MHz, DMSO-*d6*) δ 8.59 - 8.57 (m, 1H), 8.09 (dd, *J* = 7.9, 1.0 Hz, 1H), 7.97 (td, *J* = 7.7*,* 1.8 Hz, 1H), 7.81 (dd, *J* = 8.8, 2.4 Hz, 1H), 7.55 - 7.48 (m, 3H), 4.21 - 4.12 (m, 2H), 3.76 (dd, *J =* 7.8, 5.8 Hz, 1H), 3.30 (s, 3H), 2.74 (s, 3H), 2.70 (s, 3H), 2.40 - 2.32 (m, 2H).

### Example 68 Preparation of Compound 136-137

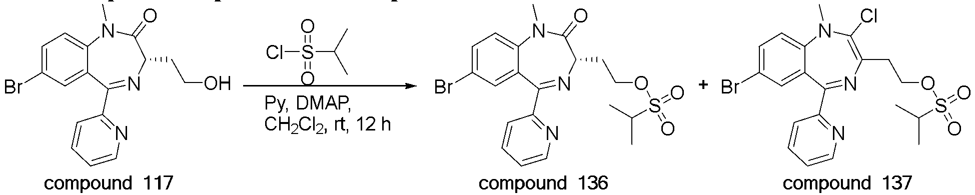

Isopropylsulfonyl chloride (268 mg, 1.88 mmol) was added to a solution of **117** (70.0 mg, 0.187 mmol), pyridine (72.3 mg, 0.914 mmol), and DMAP (137.4 mg, 1.12 mmol) in dichloromethane (2 mL). The mixture was stirred at room temperature for 12 hours. After confirming reaction completion by TLC, water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring to yield **Compound 137** as white solid (10.7 mg, yield 11.5%).

Compound **136:** ESI[M + H]⁺ = 480.1.

¹H NMR (400 MHz, DMSO-*d6*) δ 8.61 (d, *J* = 4.2 Hz, 1H), 8.16 (d, *J =* 7.9 Hz, 1H), 8.00 (td, *J =* 7.8, 1.7 Hz, 1H), 7.84 (dd, *J* = 8.9, 2.4 Hz, 1H), 7.59 - 7.52 (m, 2H), 7.48 (d, *J* = 2.4 Hz, 1H), 4.59 - 4.39 (m, 2H), 3.82 (dd, *J* = 8.6, 5.1 Hz, 1H), 3.55 - 3.49 (m, 1H), 3.34 (s, 3H), 2.51 - 2.36 (m, 2H), 1.22 (d, *J* = 6.8 Hz, 3H), 1.19 (d, *J =* 6.8 Hz, 3H).

Compound **137:** ESI[M + H]⁺ = 498.0.

¹H NMR (400 MHz, DMSO-*d6*) δ 8.60 (d, *J* = 4.6 Hz, 1H), 8.15 (d, *J* = 7.9 Hz, 1H), 7.99 (t, *J* = 7.7 Hz, 1H), 7.82 (d, *J =* 8.9 Hz, 1H), 7.56 - 7.53 (m, 2H), 7.47 (dd, *J =* 6.1, 2.2 Hz, 1H), 4.48 - 4.25 (m, 2H), 3.86 - 3.83 (m, 1H), 3.33 (s, 3H), 2.50 - 2.34 (m, 2H), 1.58 - 1.51 (m, 6H).

### Example 69 Preparation of Compound 138 and Compound 140

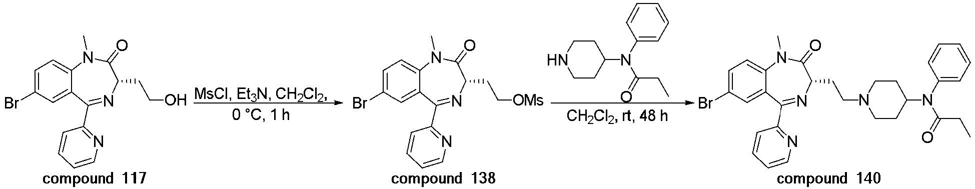

### 1. Preparation of Compound 138

At 0°C, methanesulfonyl chloride (62.9 mg, 0.549 mmol) was added dropwise to a solution of **117** (165 mg, 0.441 mmol) and triethylamine (106.8 mg, 1.06 mmol) in dichloromethane (2 mL). The mixture was stirred at 0°C for 1 hour. After confirming reaction completion by TLC, the mixture was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.2-0.3 were collected to yield **Compound 138** as white solid (50.7 mg, yield 28.6%). ESI[M + H]⁺ = 452.2.

¹H NMR (400 MHz, DMSO-*d6*) δ 8.61 (d, *J* = 4.7 Hz, 1H), 8.16 (d, *J =* 7.9 Hz, 1H), 8.02 - 7.96 (m, 1H), 7.86 - 7.81 (m, 1H), 7.58 - 7.50 (m, 3H), 4.55 - 4.44 (m, 2H), 3.85 - 3.80 (m, 1H), 3.34 (s, 3H), 3.16 (s, 3H), 2.60 - 2.38 (m, 2H).

### 2. Preparation of Compound 140

N-Phenyl-N-(piperidin-4-yl)propanamide (43.5 mg, 0.187 mmol) was added to a mixture of **138** (57 mg, 0.126 mmol) and DIEA (32.6 mg, 0.253 mmol) in dichloromethane (2 mL). The resulting mixture was stirred at room temperature for 48 hours. After confirming reaction completion by TLC, icr-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.2-0.3 were collected to yield **Compound 140** as white solid (13.2 mg, yield 17.8%). ESI[M + H]⁺ = 588.2.

¹H NMR (400 MHz, DMSO-*d6*) δ 8.56 (d, *J* = 4.1 Hz, 1H), 8.05 (d, *J =* 7.9 Hz, 1H), 7.96 - 7.93 (m, 1H), 7.78 (dd, *J* = 8.9, 2.4 Hz, 1H), 7.54 - 7.49 (m, 1H), 7.46 - 7.43 (m, 5H), 7.20 - 7.16 (m, 2H), 4.49 - 4.47 (m, 1H), 3.64 - 3.60 (m, 1H), 3.25 (s, 3H), 3.12 - 2.73 (m, 2H), 2.44 - 2.34 (m, 2H), 2.30 - 1.90 (m, 4H), 1.82 - 1.80 (m, 2H), 1.75 - 1.60 (m, 2H), 1.23 - 1.01 (m, 2H), 0.87 (t, *J =* 7.4 Hz, 3H).

### Example 70 Preparation of Compound 144, Compound 145, Compound 145D

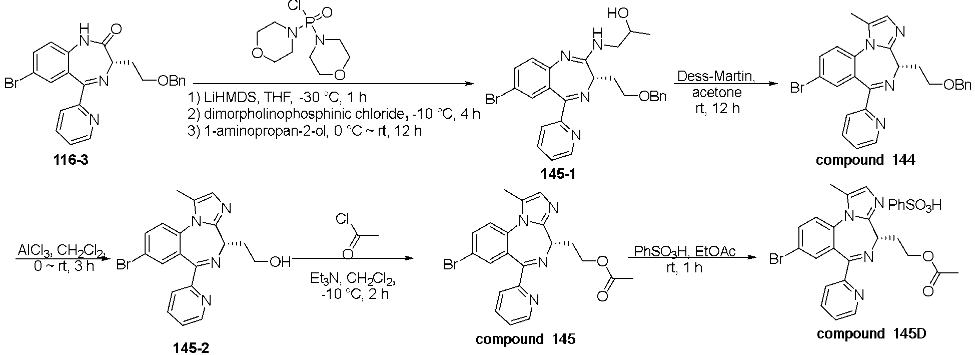

### 1. Preparation of Compound 145-1

At -30°C, solution of lithium bis(trimethylsilyl)amide (9.08 mL, 1 mol/L in THF, 9.08 mmol) was added slowly to a solution of **116-3** (3.4 g, 7.55 mmol) in THF (40 mL). The mixture was stirred at this temperature for 1 hour. Diphosphoramidous chloride (4.54 g, 17.8 mmol) was then added portionwise. After the addition was complete, the reaction was stirred at -10°C for 4 hours. Isopropanolamine (2.56 g, 34.1 mmol) was then added dropwise to the mixture. The reaction was allowed to warm to room temperature and stirred for 12 hours. After confirming reaction completion by TLC, icr-water (40 mL) was poured into the mixture, the mixture was extracted with ethyl acetate (3 × 40 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 145-1** as white solid (3.254 g, yield 84.9%). ESI[M + H]⁺ = 507.1.

### 2. Preparation of Compound 144

Dess-Martin periodinane (6.817 g, 16.1 mmol) was added to a solution of **145-1** (3.254 g, 6.41 mmol) in acetone (40 mL). The mixture was stirred at room temperature for 12 hours. After confirming reaction completion by TLC, the mixture was filtered, and the filtrate was concentrated under reduced pressure to afford the residue, the residue was dissolved in ethyl acetate (50 mL). The resulting solution was washed with saturated aqueous sodium bicarbonate, dried over anhydrous Na₂SO₄, filtered, and concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield **Compound 144** as white solid (2.312 g, yield 74.0%). ESI[M + H]⁺ = 487.1.

¹H NMR (400 MHz, DMSO-*d6*) δ 8.54 - 8.53 (m, 1H), 7.96 (d, *J =* 7.7 Hz, 1H), 7.94 - 7.86 (m, 2H), 7.66 (d, *J* = 8.7 Hz, 1H), 7.58 (d, *J* = 2.3 Hz, 1H), 7.52 - 7.42 (m, 1H), 7.29 - 7.19 (m, 5H), 6.80 (d, *J* = 1.1 Hz, 1H), 4.48 (d, *J=* 12.3 Hz, 1H), 4.41 (d, *J* = 12.3 Hz, 1H), 4.10 (t, *J* = 7.1 Hz, 1H), 3.82 - 3.69 (m, 2H), 2.59 - 2.54 (m, 2H), 2.29 (d, *J* = 0.8 Hz, 3H).

### 3. Preparation of Compound 145-2

At 0°C, aluminum chloride (6.326 g, 47.4 mmol) was added slowly to a solution of **144** (2.312 g, 4.74 mmol) in dichloromethane (50 mL). The reaction mixture was allowed to warm to room temperature and stirred for 3 hours. After confirming reaction completion by TLC, icr-water (50 mL) was poured into the mixture, the mixture was extracted with CH₂Cl₂ (3 × 60 mL). The organic phase was washed with 5% aqueous sodium bicarbonate solution and brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield **Compound 145-2** as white solid (1.7 g, yield 90.2%). ESI[M + H]⁺ = 397.1.

### 4. Preparation of Compound 145

At -10°C, acetyl chloride (55.1 mg, 0.702 mmol) was added dropwise to a solution of **145-2** (300 mg, 0.755 mmol) and triethylamine (153 mg, 1.512 mmol) in dichloromethane (3 mL). The mixture was stirred at this temperature for 2 hours. After confirming reaction completion by TLC, icr-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.4-0.5 were collected to yield **Compound 145** as white solid (248 mg, yield 74.8%). ESI[M + H]⁺ = 439.1.

¹H NMR (400 MHz, DMSO-*d6*) δ 8.54 (ddd, *J* = 4.8, 1.7, 0.9 Hz, 1H), 8.08 (d, *J* = 7.9 Hz, 1H), 7.94 (td, *J* = 7.7, 1.8 Hz, 1H), 7.89 (dd, *J* = 8.7, 2.3 Hz, 1H), 7.66 (d, *J* = 8.7 Hz, 1H), 7.62 (d, *J* = 2.3 Hz, 1H), 7.49 (ddd, *J* = 7.5, 4.8, 1.2 Hz, 1H), 6.82 (d, *J =* 1.1 Hz, 1H), 4.35 - 4.31 (m, 2H), 4.12 (t, *J =* 7.0 Hz, 1H), 2.65 - 2.59 (m, 2H), 2.30 (d, *J =* 0.8 Hz, 3H), 1.97 (s, 3H).

### 5. Preparation of Compound 145D

Compound **145** (248 mg, 0.565 mmol) was dissolved in ethyl acetate (20 mL). A solution of benzenesulfonic acid (89.6 mg, 0.566 mmol) in ethyl acetate (1 mL) was added dropwise to the resulting solution. The mixture was stirred at room temperature for 1 hour. The reaction mixture was then filtered, and the filter cake was dried under reduced pressure to afford **Compound 145D** as a white solid (281.2 mg, yield 83.4%). ESI[M + H]⁺ = 439.1.

¹H NMR (400 MHz, CD₃OD) δ 9.42 (d, *J* = 4.6 Hz, 1H), 8.95 (d, *J* = 7.9 Hz, 1H), 8.84 (ddd, *J =* 9.3, 8.3, 1.8 Hz, 2H), 8.70 (d, *J* = 8.7 Hz, 1H), 8.61 (d, *J* = 2.2 Hz, 1H), 8.47 - 8.33 (m, 4H), 8.18 - 8.10 (m, 3H), 5.28 - 5.21 (m, 2H), 5.17 - 5.10 (m, 1H), 3.63 - 3.39 (m, 2H), 3.23 (s, 3H), 2.81 (s, 3H).

### Example 71 Preparation of Compound 146-149

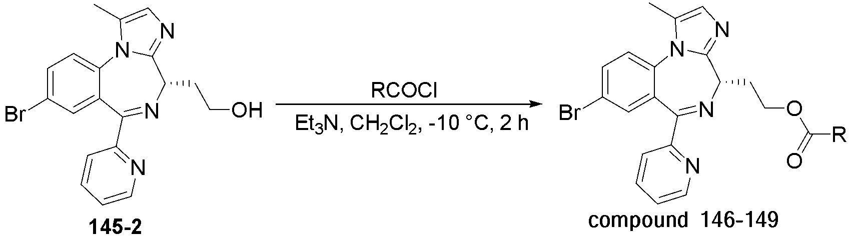

The preparation method of **compound 146 and compound 149 are** similar to **compound 145** in **example 70,** using **compound 145-2** and **corresponding acid chloride** as starting materials.

Compound **146:** 38.8 mg, ESI[M+H]⁺ = 467.1.

¹H NMR (400 MHz, DMSO-*d6*) δ 8.54 (ddd, *J* = 4.8, 1.7, 0.9 Hz, 1H), 8.08 (d, *J* = 7.9 Hz, 1H), 7.95 (td, *J* = 7.7*,* 1.8 Hz, 1H), 7.89 (dd, *J* = 8.7, 2.3 Hz, 1H), 7.66 (d, *J* = 8.7 Hz, 1H), 7.60 (d, *J* = 2.3 Hz, 1H), 7.49 (ddd, *J* = 7.5, 4.8, 1.2 Hz, 1H), 6.82 (d, *J* = 1.1 Hz, 1H), 4.44 - 4.33 (m, 2H), 4.09 (dd, *J =* 8.2, 5.9 Hz, 1H), 2.72 - 2.52 (m, 2H), 2.49 - 2.43 (m, 1H), 2.30 (d, *J* = 0.8 Hz, 3H), 1.00 (d, *J* = 4.4 Hz, 3H), 0.98 (d, *J* = 4.4 Hz, 3H).

Compound **147:** 51.7 mg, ESI[M+H]⁺ = 453.1.

¹H NMR (400 MHz, DMSO-*d6*) δ 8.56 - 8.51 (m, 1H), 8.08 (d, *J =* 7.9 Hz, 1H), 7.94 (td, *J =* 7.7, 1.8 Hz, 1H), 7.89 (dd, *J* = 8.7, 2.3 Hz, 1H), 7.66 (d, *J =* 8.7 Hz, 1H), 7.61 (d, *J =* 2.3 Hz, 1H), 7.49 (ddd, *J* = 7.5, 4.8, 1.1 Hz, 1H), 6.82 (d, *J* = 1.1 Hz, 1H), 4.47 - 4.28 (m, 2H), 4.11 (t, *J* = 7.0 Hz, 1H), 2.65 - 2.59 (m, 2H), 2.30 (d, *J* = 0.8 Hz, 3H), 2.29 - 2.24 (m, 2H), 0.96 (t, *J* = 7.5 Hz, 3H).

Compound **148:** 90.1 mg, ESI[M+H]⁺ = 473.0.

¹H NMR (400 MHz, DMSO-*d6*) δ 8.58 - 8.50 (m, 1H), 8.09 (d, *J* = 7.9 Hz, 1H), 7.95 (td, *J* = 7.8, 1.7 Hz, 1H), 7.89 (dd, *J* = 8.7, 2.3 Hz, 1H), 7.66 (d, *J =* 8.7 Hz, 1H), 7.61 (d, *J =* 2.3 Hz, 1H), 7.50 (ddd, *J =* 7.5, 4.8, 1.1 Hz, 1H), 6.82 (d, *J* = 1.0 Hz, 1H), 4.56 - 4.44 (m, 2H), 4.35 (s, 2H), 4.16 (t, *J =* 7.0 Hz, 1H), 2.69 - 2.62 (m, 2H), 2.30 (d, *J* = 0.7 Hz, 3H).

Compound **149:** 101.7 mg, ESI[M+H]⁺ = 515.4.

¹H NMR (400 MHz, DMSO-*d6*) δ 8.56 (d, *J =* 4.2 Hz, 1H), 8.10 (d, *J* = 7.9 Hz, 1H), 7.97 (td, *J = 7.8,* 1.6 Hz, 1H), 7.89 (dd, *J* = 8.7, 2.3 Hz, 1H), 7.67 (d, *J* = 8.7 Hz, 1H), 7.57 (d, *J* = 2.3 Hz, 1H), 7.51 (dd, *J* = 6.9, 5.3 Hz, 1H), 6.84 (s, 1H), 4.55 - 4.48 (m, 1H), 4.47 - 4.33 (m, 1H), 4.17 (dd, *J =* 8.5, 5.7 Hz, 1H), 3.60 (s, 2H), 2.73 - 2.60 (m, 2H), 2.32 (s, 3H), 1.13 (s, 6H).

### Example 72 Preparation of Compound 150 and Compound 151

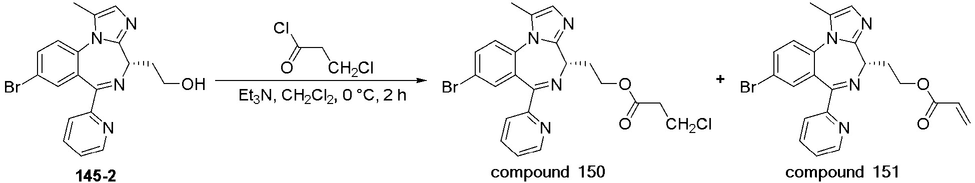

At 0°C, 3-chloropropionyl chloride (58.7 mg, 0.462 mmol) was added dropwise to a solution of **145-2** (100 mg, 0.252 mmol) and triethylamine (51 mg, 0.504 mmol) in dichloromethane (2 mL). The mixture was stirred at this temperature for 2 hours. After confirming reaction completion by TLC, icr-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.4-0.5 were collected to yield **Compound 150** as white solid (21.1 mg, yield 17.2%) and **Compound 151** as white solid (19.0 mg, yield 16.7%).

Compound **150:** ESI[M + H]⁺ = 487.0.

¹H NMR (400 MHz, DMSO-*d6*) δ 8.58 - 8.51 (m, 1H), 8.08 (d, *J =* 7.9 Hz, 1H), 7.95 (td, *J =* 7.7, 1.8 Hz, 1H), 7.88 (dd, *J* = 8.7, 2.3 Hz, 1H), 7.65 (d, *J =* 8.7 Hz, 1H), 7.58 (d, *J =* 2.3 Hz, 1H), 7.50 (ddd, *J =* 7.5, 4.8, 1.1 Hz, 1H), 6.82 (d, *J* = 1.1 Hz, 1H), 4.52 - 4.38 (m, 2H), 4.19 - 4.11 (m, 1H), 3.71 (t, *J* = 6.1 Hz, 2H), 2.77 (t, *J* = 6.1 Hz, 2H), 2.71 - 2.59 (m, 2H), 2.30 (d, *J =* 0.7 Hz, 3H).

Compound **151:** ESI[M + H]⁺ = 451.1.

¹H NMR (400 MHz, DMSO-*d6*) δ 8.54 (d, *J =* 3.9 Hz, 1H), 8.09 (d, *J =* 7.9 Hz, 1H), 7.94 (td, *J =* 7.7, 1.7 Hz, 1H), 7.89 (dd, *J* = 8.7, 2.3 Hz, 1H), 7.66 (d, *J =* 8.7 Hz, 1H), 7.61 (d, *J =* 2.3 Hz, 1H), 7.49 (dd, *J =* 6.4, 4.9 Hz, 1H), 6.82 (d, *J =* 1.0 Hz, 1H), 6.29 (dd, *J* = 17.3, 1.6 Hz, 1H), 6.15 (dd, *J* = 17.3, 10.2 Hz, 1H), 5.90 (dd, *J* = 10.2, 1.7 Hz, 1H), 4.49 - 4.44 (m, 2H), 4.21 - 4.10 (m, 1H), 2.73 - 2.60 (m, 2H), 2.30 (s, 3H).

### Example 73 Preparation of Compound 152

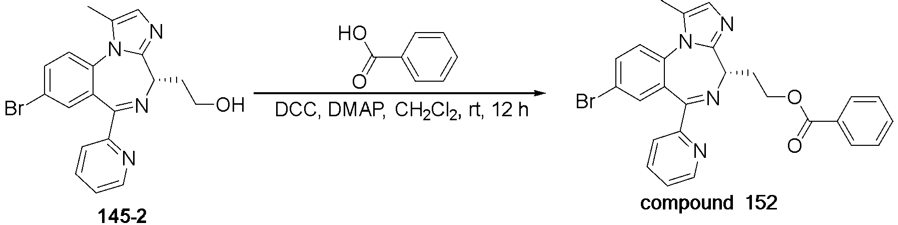

Benzoic acid (46.2 mg, 0.378 mmol), DCC (78 mg, 0.378 mmol), and DMAP (61.6 mg, 0.504 mmol) were dissolved in dichloromethane (2 mL), and the mixture was stirred at room temperature for 15 minutes. **145-2** (100 mg, 0.252 mmol) was then added to the reaction mixture, and stirring was continued at room temperature for 12 hours. After confirming reaction completion by TLC, methyl tert-butyl ether (2 mL) was added. The mixture was stirred for 5 minutes, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 152** as white solid (58.8 mg, yield 46.6%) and **Compound 152** as white solid (58.8 mg, yield 46.6%). ESI[M + H]⁺ = 501.1.

¹H NMR (400 MHz, DMSO-*d6*) δ 8.56 - 8.51 (m, 1H), 8.07 (d, *J =* 7.9 Hz, 1H), 7.92 - 7.90 (m, 3H), 7.89 - 7.86 (m, 1H), 7.68 - 7.60 (m, 2H), 7.58 (d, *J* = 2.3 Hz, 1H), 7.51 - 7.45 (m, 3H), 6.83 (d, *J* = 1.1 Hz, 1H), 4.64 (t, *J* = 6.8 Hz, 2H), 4.24 (dd, *J* = 8.2, 5.7 Hz, 1H), 2.85 - 2.73 (m, 2H), 2.31 (d, *J =* 0.7 Hz, 3H).

### Example 74 Preparation of Compound 153

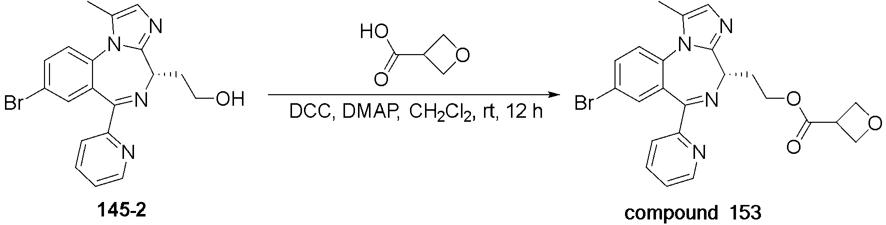

The preparation method of **compound 153 is** similar to **compound 152** in **example73,** using **compound 145-2** and **Oxetane-3-carboxylic acid** as starting materials.

Compound **153:** 17.6 mg, ESI[M+H]⁺ = 481.1.

¹H NMR (400 MHz, DMSO-*d6*) δ 8.58 - 8.53 (m, 1H), 8.08 (d, *J* = 7.9 Hz, 1H), 7.95 (td, *J* = 7.7, 1.8 Hz, 1H), 7.89 (dd, *J* = 8.7, 2.3 Hz, 1H), 7.66 (d, *J =* 8.7 Hz, 1H), 7.61 (d, *J* = 2.3 Hz, 1H), 7.50 (ddd, *J* = 7.5, 4.8, 1.2 Hz, 1H), 6.82 (d, *J =* 1.1 Hz, 1H), 4.65 (dd, *J =* 8.7, 5.9 Hz, 2H), 4.54 (dd, *J=* 12.1, 5.8 Hz, 2H), 4.50 - 4.40 (m, 2H), 4.13 (t, *J=* 7.0 Hz, 1H), 3.90 - 3.82 (m, 1H), 2.68 - 2.62 (m, 2H), 2.30 (d, *J* = 0.8 Hz, 3H).

### Example 75 Preparation of Compound 154

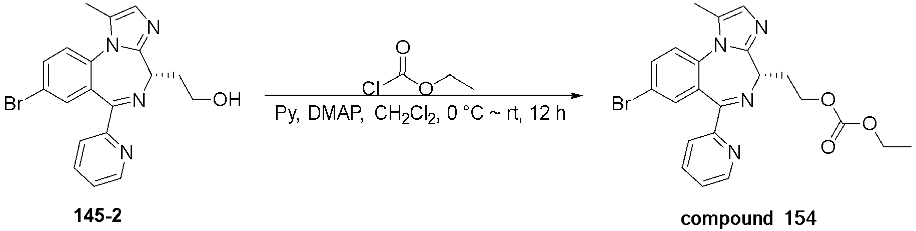

At 0°C, ethyl chloroformate (66 mg, 0.608 mmol) was added dropwise to a solution of **145-2** (80 mg, 0.201 mmol), pyridine (47 mg, 0.594 mmol) and DMAP (25 mg, 0.205 mmol) in dichloromethane (2 mL). The reaction mixture was allowed to warm to room temperatur and stirred for 12 hours. After confirming reaction completion by TLC, icr-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield **Compound 154** as white solid (42.3 mg, yield 44.8%). ESI[M + H]⁺ = 469.1.

¹H NMR (400 MHz, DMSO-d6) δ 8.54 (ddd, *J =* 4.8, 1.6, 0.8 Hz, 1H), 8.09 (d, *J* = 7.9 Hz, 1H), 7.94 (td, *J* = 7.7*,* 1.8 Hz, 1H), 7.91 - 7.87 (m, 1H), 7.68 - 7.64 (m, 1H), 7.61 (d, *J* = 2.3 Hz, 1H), 7.49 (ddd, *J* = 7.5, 4.8, 1.2 Hz, 1H), 6.82 (d, *J =* 1.1 Hz, 1H), 4.44 (t, *J* = 6.6 Hz, 2H), 4.15 - 4.00 (m, 3H), 2.72 - 2.57 (m, 2H), 2.30 (d, *J =* 0.8 Hz, 3H), 1.16 (t, *J* = 7.1 Hz, 3H).

### Example 76 Preparation of Compound 155

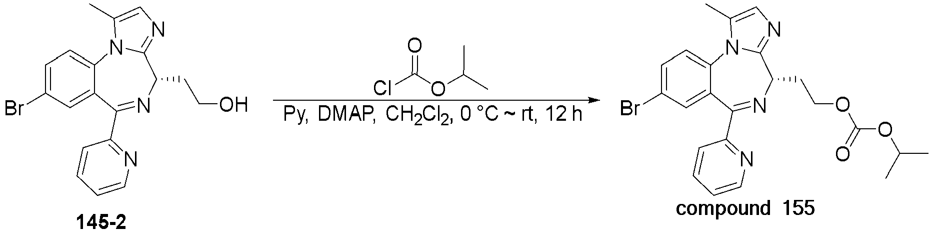

The preparation method of **compound 155 is** similar to **compound 154** in **example75,** using **compound 145-2** and Isopropyl chloroformate as starting materials.

Compound **155:** 45.7 mg, ESI[M+H]⁺ = 483.1

¹H NMR (400 MHz, DMSO-*d6*) δ 8.57 - 8.51 (m, 1H), 8.10 (d, *J* = 7.9 Hz, 1H), 7.94 (td, *J* = 7.7, 1.8 Hz, 1H), 7.89 (dd, *J* = 8.7, 2.3 Hz, 1H), 7.66 (d, *J* = 8.7 Hz, 1H), 7.60 (d, *J* = 2.3 Hz, 1H), 7.50 (ddd, *J =* 7.5, 4.8, 1.1 Hz, 1H), 6.82 (d, *J =* 1.1 Hz, 1H), 4.80 - 4.66 (m, 1H), 4.43 (t, *J* = 6.6 Hz, 2H), 4.09 (dd, *J* = 8.1, 6.0 Hz, 1H), 2.69 - 2.59 (m, 2H), 2.30 (d, *J* = 0.8 Hz, 3H), 1.18 (d, *J* = 2.7 Hz, 3H), 1.16 (d, *J =* 2.7 Hz, 3H).

### Example 77 Preparation of Compound 156, 156D, 157, 158, 159 and 159D

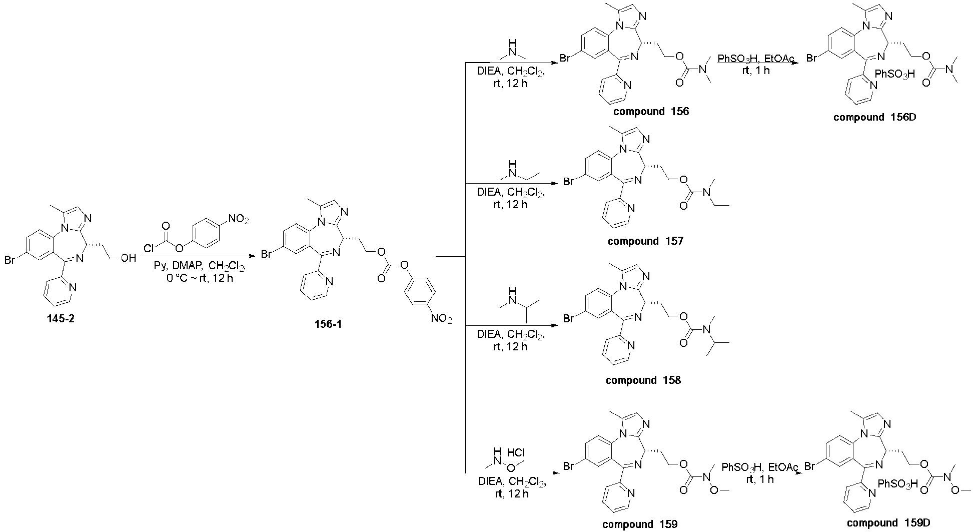

### 1. Preparation of Compound 156-1

At 0°C, *p*-nitrophenyl chloroformate (487 mg, 2.42 mmol) was added to a solution of **145-2** (320 mg, 0.805 mmol), pyridine (186.2 mg, 2.35 mmol) and DMAP (98 mg, 0.802 mmol) in dichloromethane (5 mL). The reaction mixture was stirred at room temperature for 12 hours. After confirming reaction completion by TLC, icr-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.3-0.4 were collected to yield Compound **156-1** as white solid (404 mg, yield 89.2%). ESI[M + H]+ = 562.1.

### 2. Preparation of Compound 156

**156-1** (200 mg, 0.356 mmol) and DIEA (46.0 mg, 0.356 mmol) were dissolved in dichloromethane (2 mL), then dimethylamine (0.356 mL, 2 mol/L in THF, 0.712 mmol) was added, and the mixture was stirred at room temperature for 12 hours. After confirming reaction completion by TLC, icr-water (10 mL) was poured into the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield Compound **156** as white solid (144.7 mg, yield 86.9%). ESI[M + H]+ = 468.1.

¹H NMR (400 MHz, DMSO-*d6*) δ 8.54 (ddd, *J* = 4.8, 1.7, 0.9 Hz, 1H), 8.08 (d, *J* = 7.9 Hz, 1H), 7.94 (td, *J* = 7.7, 1.8 Hz, 1H), 7.88 (dd, *J* = 8.7, 2.3 Hz, 1H), 7.66 (d, *J* = 8.7 Hz, 1H), 7.62 (d, *J* = 2.3 Hz, 1H), 7.49 (ddd, *J* = 7.5, 4.8, 1.2 Hz, 1H), 6.81 (d, *J* = 1.1 Hz, 1H), 4.39 - 4.25 (m, 2H), 4.11 (dd, *J =* 7.9, 6.1 Hz, 1H), 2.76 (s, 3H), 2.75 (s, 3H), 2.69 - 2.56 (m, 2H), 2.30 (d, *J =* 0.8 Hz, 3H).

### 3. Preparation of Compound 156D

Compound **156** (144.7 mg, 0.309 mmol) was dissolved in ethyl acetate (10 mL), the solution of benzenesulfonic acid (49.0 mg, 0.31 mmol) in ethyl acetate (0.5 mL) was added dropwise to the resulting solution, the mixture was stirred at room temperature for 1 hour. The mixture was filtered, the filter cake was dried under reduced pressure to yield **Compound 156D** as white solid (117.4 mg, yield 60.7%). ESI[M + H]⁺ = 468.1.

¹H NMR (400 MHz, DMSO-*d6*) δ 8.60 (d, *J* = 4.1 Hz, 1H), 8.13 (d, *J =* 7.9 Hz, 1H), 8.06 - 7.95 (m, 2H), 7.86 (d, *J =* 8.7 Hz, 1H), 7.78 (d, *J =* 1.3 Hz, 1H), 7.63 - 7.59 (m, 2H), 7.57 (dd, *J* = 6.6, 5.0 Hz, 1H), 7.46 (s, 1H), 7.36 - 7.24 (m, 3H), 4.45 - 4.34 (m, 2H), 4.32 - 4.23 (m, 1H), 2.79 (s, 3H), 2.77 (s, 3H), 2.74 - 2.59 (m, 2H), 2.41 (s, 3H).

### 4. Preparation of Compound 157

The preparation method of **compound 157 is** similar to **compound 156** in **example 77,** using **compound 156-1** and **N-Methylethanamine** as starting materials.

Compound **157:** 45.5 mg, ESI[M+H]⁺ = 482.1.

¹H NMR (400 MHz, DMSO-d6) δ 8.57 - 8.51 (m, 1H), 8.09 (d, *J* = 7.9 Hz, 1H), 7.94 (td, *J* = 7.7, 1.8 Hz, 1H), 7.88 (dd, *J* = 8.7, 2.3 Hz, 1H), 7.66 (d, *J =* 8.7 Hz, 1H), 7.61 (d, *J* = 2.3 Hz, 1H), 7.49 (ddd, *J* = 7.5, 4.8, 1.2 Hz, 1H), 6.81 (d, *J* = 1.1 Hz, 1H), 4.32 (t, *J* = 6.4 Hz, 2H), 4.10 (dd, *J* = 8.0, 6.0 Hz, 1H), 3.15 - 3.10 (m, 2H), 2.73 (s, 3H), 2.68 - 2.54 (m, 2H), 2.30 (d, *J* = 0.8 Hz, 3H), 0.95 - 0.82 (m, 3H).

### 5. Preparation of Compound 158

The preparation method of **compound 158** is similar to **compound 156** in example 77, using compound **156-1** and **N-Methylpropan-2-amine** as starting materials.

Compound **158:** 44.6 mg, ESI[M+H]⁺ = 496.1.

¹H NMR (400 MHz, DMSO-*d6*) δ 8.57 - 8.53 (m, 1H), 8.09 (d, *J =* 7.9 Hz, 1H), 7.94 (td, *J =* 7.7, 1.8 Hz, 1H), 7.88 (dd, *J* = 8.7, 2.3 Hz, 1H), 7.66 (d, *J* = 8.7 Hz, 1H), 7.60 (d, *J* = 2.3 Hz, 1H), 7.49 (ddd, *J* = 7.5, 4.8, 1.2 Hz, 1H), 6.81 (d, *J =* 1.1 Hz, 1H), 4.33 (t, *J* = 6.6 Hz, 2H), 4.27 - 3.94 (m, 2H), 2.68 - 2.60 (m, 2H), 2.59 (s, 3H), 2.30 (d, *J =* 0.8 Hz, 3H), 0.96 - 0.91 (m, 6H).

### 6. Preparation of Compound 159 and Compound 159D

The preparation method of **compound 159 and compound 159D** are similar to **compound 156 and compound 156D** in example 77, using compound **156-1** and **N,O-Dimethylhydroxylamine hydrochloride** as starting materials to yield **compound 159,** then **compound 159** and benzenesulfonic acid as starting materials to yield **compound 159D.**

Compound **159:** 144.7 mg, ESI[M+H]⁺ = 484.1.

¹H NMR (400 MHz, DMSO-*d6*) δ 8.59 - 8.51 (m, 1H), 8.10 (d, *J =* 7.9 Hz, 1H), 7.95 (td, *J* = 7.7, 1.8 Hz, 1H), 7.88 (dd, *J* = 8.7, 2.3 Hz, 1H), 7.66 (d, *J* = 8.7 Hz, 1H), 7.62 (d, *J* = 2.3 Hz, 1H), 7.49 (ddd, *J* = 7.5, 4.8, 1.1 Hz, 1H), 6.82 (d, *J =* 1.1 Hz, 1H), 4.49 - 4.34 (m, 2H), 4.13 (dd, *J* = 8.1, 5.9 Hz, 1H), 3.47 (s, 3H), 3.01 (s, 3H), 2.71 - 2.60 (m, 2H), 2.30 (d, *J* = 0.7 Hz, 3H).

Compound **159D:** 63.3 mg, ESI[M+H]⁺ = 484.1.

¹H NMR (400 MHz, DMSO-*d6*) δ 8.59 - 8.58 (m, 1H), 8.15 (d, *J =* 7.6 Hz, 1H), 8.01 - 7.98 (m, 2H), 7.79 - 7.74 (m, 2H), 7.64 - 7.59 (m, 2H), 7.57 - 7.52 (m, 1H), 7.37 - 7.23 (m, 4H), 4.46 - 4.41 (m, 3H), 3.50 (s, 3H), 3.03 (s, 3H), 2.79 - 2.62 (m, 2H), 2.38 - 2.35 (m, 3H).

### Example 78 Preparation of Compound 160

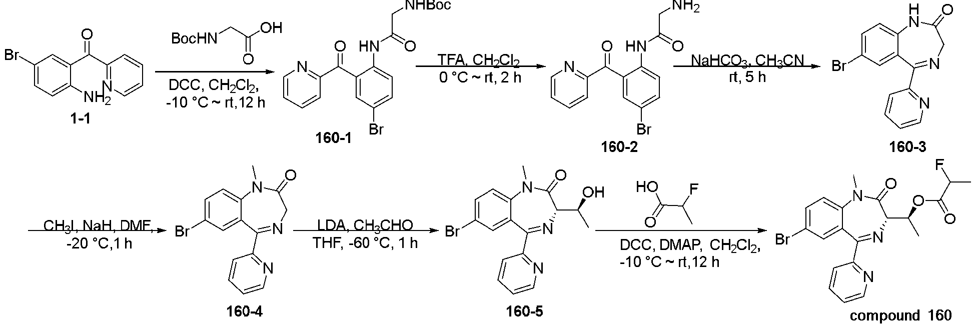

### 1. Preparation of Compound 160-1

**1-1** (2.341 g, 8.45 mmol) and Boc-glycine (2.23 g, 12.7 mmol) were dissolved in dichloromethane (30 mL), the solution of DCC (2.62 g, 12.7 mmol) in dichloromethane (10 mL) was added dropwise at -10°C, the mixture was stirred at room temperature for 12 hours. After confirming reaction completion by TLC, methyl tert-butyl ether (40 mL) was added, and the mixture was stirred for 5 minutes. The reaction mixture was then filtered, and the filtrate was concentrated under reduced pressure to afford the crude product. The crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.3-0.4 were collected to yield Compound **160-1** as white solid (2.5 g, yield 68.1%). ESI[M + H]+ = 434.1.

### 2. Preparation of Compound 160-2

At 0°C, trifluoroacetic acid (10 mL) was added to the solution of **160-1** (2.5 g, 5.76 mmol) in dichloromethane (30 mL), the reaction mixture was stirred at room temperature for 2 hours. After confirming reaction completion by TLC, the mixture was concentrated under reduced pressure. The crude product was used directly in the next step without further purification.

### 3. Preparation of Compound 160-3

The crude product from the previous step was dissolved in acetonitrile (30 mL). Sodium bicarbonate (18.9 g, 225 mmol) was added, and the mixture was stirred at room temperature for 5 hours. After confirming reaction completion by TLC, the mixture was filtered and the filtrate was concentrated under reduced pressure to afford the crude product. The crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.2-0.3 were collected to yield Compound **160-3** as white solid (672 mg, yield 36.9%). ESI[M + H]⁺ = 316.0.

### 4. Preparation of Compound 160-4

At -20°C, sodium hydride (110.6 mg, 60% dispersion in mineral oil, 2.76 mmol) was added slowly to a solution of **160-3** (672 mg, 2.13 mmol) in dry DMF (6 mL). The mixture was stirred at this temperature for 30 minutes. Methyl iodide (392.6 mg, 2.77 mmol) was then added to the mixture at -20°C, and was stirred for 1 hour at the same temperature. After confirming reaction completion by TLC, icr-water (20 mL) was poured into the mixture, the mixture was extracted with ethyl acetate (3 × 20 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.3-0.4 were collected to yield Compound **160-4** as white solid (450 mg, yield 64.1%). ESI[M + H]+ = 330.1.

### 5. Preparation of Compound 160-5

At -60°C, solution of LDA (0.926 mL, 2 mol/L in THF, 1.85 mmol) was added dropwise slowly to a solution of **160-4** (450 mg, 1.36 mmol) in dry tetrahydrofuran (8 mL), the mixture was stirred at this temperature for 15 minutes. Acetaldehyde (0.85 mL, 5 mol/L in THF, 4.25 mmol) was then added to the mixture at -60°C, and the mixture was stirred at -60°C for 1 hour. After confirming reaction completion by TLC, saturated aqueous ammonium chloride solution (10 mL) was poured into the mixture, the mixture was extracted with ethyl acetate (3 × 10 mL). The organic phase was washed with brine, dried over anhydrous Na₂SO₄, filtered, and the filtrate was concentrated under reduced pressure to afford the crude product, the crude product was purified by silica gel column chromatography (CH₂Cl₂/MTBE/MeOH/Et₃N, v/v = 50/50/2.5/0.5) to yield Compound **160-5** as white solid (180 mg, yield 35.3%). ESI[M + H]+ = 374.1.

### 6. Preparation of Compound 160

2-Fluoropropanoic acid (18.5 mg, 0.201 mmol), DCC (41.4 mg, 0.201 mmol) and DMAP (32.7 mg, 0.268 mmol) were dissolved in dichloromethane (2 mL), and the mixture was stirred at room temperature for 15 minutes. **160-5** (50 mg, 0.134 mmol) was then added to the mixture, and stirred at room temperature for 12 hours. After confirming reaction completion by TLC, Methyl tert-butyl ether (2 mL) was added to the mixture,then the mixture was stirred 5 minutes, filtered, and the filtrate was concentrated under reduced pressure to afford the crude product, the crude product was purified by silica gel column chromatography (ethyl acetate/petroleum ether, v/v = 1/10/-1/1) to yield Compound **160** as white solid (9.9 mg, yield 16.5%). ESI[M + H]⁺ = 448.1.

¹H NMR (400 MHz, DMSO-*d6*) δ 8.62 (d, *J =* 4.6 Hz, 1H), 8.15 (d, *J* = 7.7 Hz, 1H), 8.01 (t, *J* = 7.7 Hz, 1H), 7.86 (dd, *J* = 8.8, 2.2 Hz, 1H), 7.61 (d, *J* = 8.9 Hz, 1H), 7.58 - 7.55 (m, 2H), 5.80 - 5.64 (m, 1H), 5.22 - 5.08 (m, 1H), 3.83 (t, *J =* 8.8 Hz, 1H), 3.33 (s, 3H), 1.48 - 1.28 (m, 6H).

### Example 79 Preparation of Compound 162

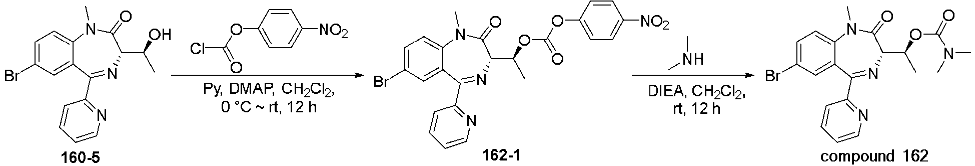

### 1. Preparation of Compound 162-1

At 0°C, *p*-nitrophenyl chloroformate (80.8 mg, 0.401 mmol) was added to the solution of **160-5** (50 mg, 0.134 mmol), pyridine (31 mg, 0.392 mmol) and DMAP (32.7 mg, 0.268 mmol) in dichloromethane (2 mL). The mixture was stirred at room temperature for 12 hours. After confirming reaction completion by TLC, ice-water (10 mL) was added to the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography ethyl acetate/petroleum ether, v/v = 1/20-1/2), TLC (ethyl acetate/petroleum ether, v/v =1/2) momnitoring and the fractions with Rf = 0.3-0.4 were collected to yield Compound **162-1** as white solide (60 mg, yield 83.3%). ESI[M + H]⁺ = 539.1.

### 2. Preparation of Compound 162

**162-1** (60 mg, 0.111 mmol) and DIEA (14.4 mg, 0.111 mmol) were dissolved in dichloromethane (2 mL), then dimethylamine (0.17 mL, 2 mol/L in THF, 0.34 mmol) was added, and the resulting mixture was stirred at room temperature for 12 hours. After confirming reaction completion by TLC, ice-water (10 mL) was added to the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield Compound **162** as white solide (16.9 mg, yield 34.1%). ESI[M + H]⁺ = 445.1.

¹H NMR (400 MHz, DMSO-*d6*) δ 8.61 (d, *J =* 4.4 Hz, 1H), 8.14 (d, *J =* 7.9 Hz, 1H), 8.00 (td, *J =* 7.7, 1.5 Hz, 1H), 7.85 (dd, *J* = 8.8, 2.3 Hz, 1H), 7.59 (d, *J* = 8.9 Hz, 1H), 7.58 - 7.53 (m, 2H), 5.49 - 5.43 (m, 1H), 3.76 (d, *J* = 7.6 Hz, 1H), 3.33 (s, 3H), 2.81 (s, 3H), 2.76 (s, 3H), 1.44 (d, *J* = 6.3 Hz, 3H).

### Example 80 Preparation of Compound 164 and Compound 165

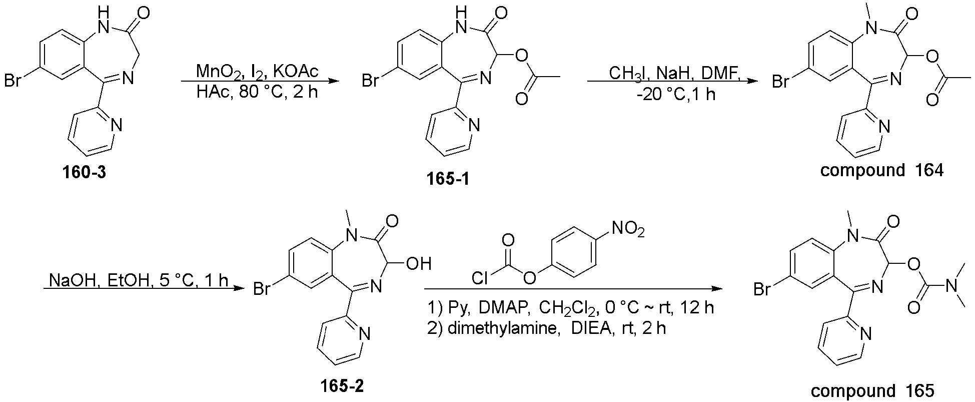

### 1. Preparation of Compound 165-1

**160-3** (1.616 g, 5.11 mmol) was dissolved in glacial acetic acid (20 mL), then potassium acetate (1.004 g, 10.23 mmol), iodine (1.303 g, 5.13 mmol) and manganese dioxide (1.454 g, 16.7 mmol) were added. The reaction mixture was stirred at 80°C for 2 hours. After confirming reaction completion by TLC, the mixture was filtered and the filtrate was concentrated under reduced pressure, the residue was dissolved in ethyl acetate (30 mL). The organic phase was washed with 10% aqueous sodium thiosulfate solution, followed by brine, dried over anhydrous sodium sulfate, filtered, and concentrated under reduced pressure to afford the crude product. The crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.3-0.4 were collected to yield Compound **165-1** as white solide (1.03 g, yield 53.9%). ESI[M + H]⁺= 374.1.

### 2. Preparation of Compound 164

At -20°C, sodium hydride (141 mg, 60% dispersion in mineral oil, 3.52 mmol) was added slowly to solution of **165-1** (1.013 g, 2.71 mmol) in dry DMF (10 mL), the mixture was stirred at -20°C for 30 minutes. Methyl iodide (385 mg, 2.71 mmol) was then added to the mixture at -20°C, and the mixture was stirred at -20°C for 1 hour. After confirming reaction completion by TLC, ice-water (30 mL) was added to the mixture, the mixture was extracted with ethyl acetate (3 × 30 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/100-1/1), TLC (ethyl acetate/petroleum ether, v/v =1/1) momnitoring and the fractions with Rf = 0.3-0.4 were collected to yield Compound **164** as off-white solide (550 mg, yield 52.3%). ESI[M + H]⁺ = 388.0.

¹H NMR (400 MHz, DMSO-*d6*) δ 8.64 (d, *J =* 4.0 Hz, 1H), 8.13 (d, *J =* 7.9 Hz, 1H), 8.02 (td, *J =* 7.8, 1.7 Hz, 1H), 7.90 (dd, *J* = 8.9, 2.4 Hz, 1H), 7.65 (d, *J* = 8.9 Hz, 1H), 7.61 - 7.55 (m, 2H), 5.93 (s, 1H), 3.37 (s, 3H), 2.23 (s, 3H).

### 3. Preparation of Compound 165-2

At 5°C, solution of sodium hydroxide (102.3 mg, 2.56 mmol) in water (5 mL) was added to the solution of **164** (450 mg, 1.16 mmol) in ethanol (10 mL), the mixture was stirred at 5°C for 1 hour. After confirming reaction completion by TLC, the mixture was concentrated under reduced pressure. The residue was dissolved in ethyl acetate (20 mL). The resulting organic phase was washed with brine, dried over anhydrous sodium sulfate, filtered, and concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield Compound **165-2** as white solide (350 mg, yield 87.2%). ESI[M + H]⁺ = 346.1.

### 4. Preparation of Compound 165

At 0°C, *p*-nitrophenyl chloroformate (175 mg, 0.868 mmol) was added to the solution of **165-2** (100 mg, 0.289 mmol), pyridine (67 mg, 0.847 mmol) and DMAP (35.5 mg, 0.291 mmol) in dichloromethane (3 mL), the mixture was stirred at room temperature for 12 hours. After confirming reaction completion by TLC, dimethylamine (0.87 mL, 2 mol/L in THF, 1.74 mmol) and DIEA (37 mg, 0.286 mmol) were added to the mixture. The mixture was stirred at room temperature for 2 hours. After confirming reaction completion by TLC, water (10 mL) was added to the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.3-0.4 were collected to yield Compound **165** as off-white solide (2.5 mg, yield 2.1%). ESI[M + H]⁺ = 417.1.

¹H NMR (400 MHz, DMSO-d6) δ 8.63 (d, *J =* 4.6 Hz, 1H), 8.11 (d, *J* = 7.9 Hz, 1H), 8.00 (td, *J =* 7.7, 1.6 Hz, 1H), 7.89 (dd, *J =* 8.9, 2.4 Hz, 1H), 7.62 (d, *J =* 8.9 Hz, 1H), 7.59 - 7.56 (m, 2H), 5.84 (s, 1H), 3.35 (s, 3H), 3.09 (s, 3H), 2.86 (s, 3H).

### Example 81 Preparation of Compound 166

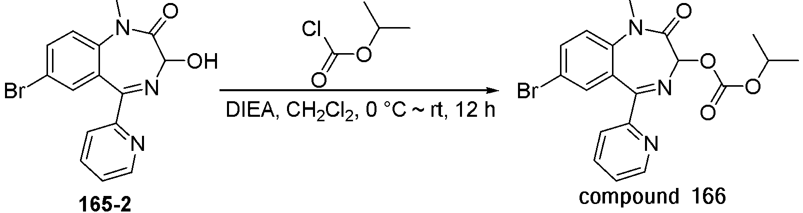

At 0°C, isopropyl chloroformate (52.3 mg, 0.427 mmol) was added dropwise to solution of **165-2** (80 mg, 0.231 mmol) and DIEA (94.1 mg, 0.728 mmol) in dichloromethane (2 mL), the mixture was stirred at room temperatur and stirred for 12 hours. After confirming reaction completion by TLC, ice-water (10 mL) was added to the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.3-0.4 were collected to yield Compound **166** as yellow solid (21.4 mg, yield 21.4%). ESI[M + H]+ = 432.1.

¹H NMR (400 MHz, DMSO-d6) δ 8.51 (d, *J =* 4.7 Hz, 1H), 8.05 (d, *J =* 2.1 Hz, 1H), 7.84 - 7.79 (m, 2H), 7.54 (d, *J =* 8.6 Hz, 1H), 7.41 - 7.30 (m, 1H), 7.17 (d, *J =* 8.0 Hz, 1H), 6.89 (s, 1H), 5.11 - 5.05 (m, 1H), 2.80 (s, 3H), 1.36 (d, *J =* 6.2 Hz, 3H), 1.32 (d, *J =* 6.2 Hz, 3H).

### Example 82 Preparation of Compound 167

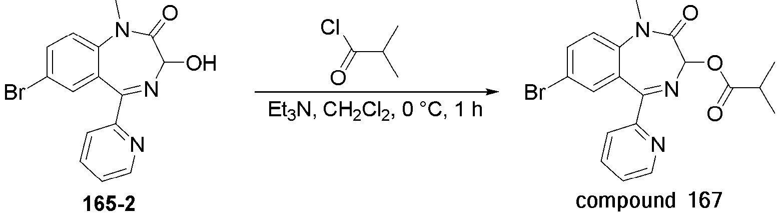

At 0°C, isobutyryl chloride (32.3 mg, 0.303 mmol) was added dropwise to solution of **165-2** (70 mg, 0.202 mmol) and triethylamine (41 mg, 0.405 mmol) in dichloromethane (2 mL), the mixture was stirred at 0°C for 1 hour. After confirming reaction completion by TLC, ice-water (10 mL) was added to the mixture, the mixture was extracted with CH₂Cl₂ (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.3-0.4 were collected to yield Compound **167** as white solid (42.7 mg, yield 50.7%). ESI[M + H]⁺ = 416.1.

¹H NMR (400 MHz, DMSO-d6) δ 8.49 (d, *J =* 4.3 Hz, 1H), 8.01 (d, *J =* 2.2 Hz, 1H), 7.85 - 7.76 (m, 2H), 7.55 (d, *J =* 8.6 Hz, 1H), 7.38 - 7.31 (m, 1H), 7.16 - 7.08 (m, 2H), 3.58 - 3.51 (m, 1H), 2.83 (s, 3H), 1.26 (d, *J =* 6.8 Hz, 3H), 1.16 (d, *J =* 6.7 Hz, 3H).

### Example 83 Preparation of Compound 169-172

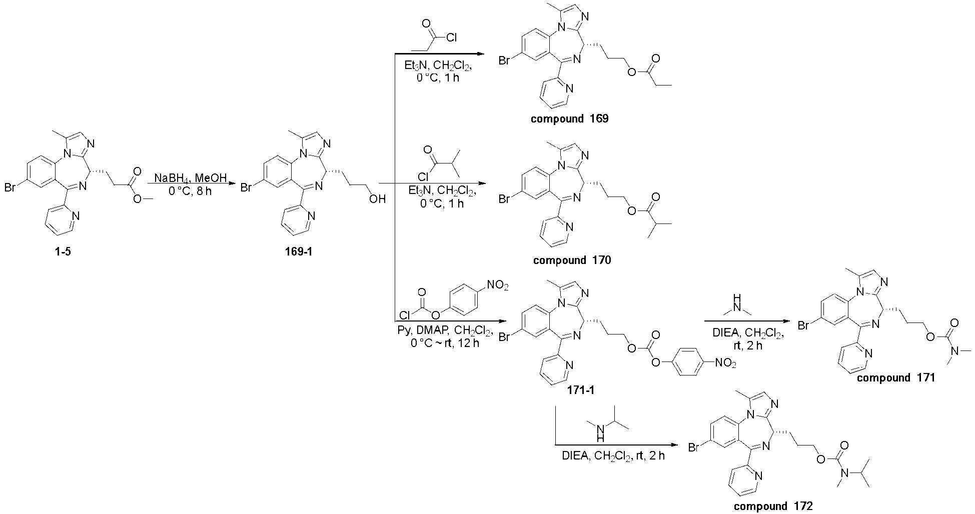

### 1. Preparation of Compound 169-1

At 0°C, sodium borohydride (1.3 g, 34.4 mmol) was added portionwise to solution of **1-5** (1.0 g, 2.28 mmol) in methanol (15 mL), the mixture was stirred at 0°C for 8 hours. After confirming reaction completion by TLC, ice-water (10 mL) was added to the mixture, the mixture was extracted with ethyl acetate (3 × 30 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield Compound **169-1** as white solid (266 mg, yield 28.4%). ESI[M + H]⁺ = 411.1.

### 2. Preparation of Compound 169

At 0°C, propionyl chloride (20.2 mg, 0.218 mmol) was added dropwise to solution of **169-1** (60 mg, 0.146 mmol) and triethylamine (30 mg, 0.296 mmol) in dichloromethane (2 mL). The mixture was stirred at 0°C for 1 hour. After confirming reaction completion by TLC, ice-water (10 mL) was added to the mixture, the mixture was extracted with dichloromethane (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.4-0.5 were collected to yield Compound **169** as white solid (29.6 mg, yield 43.4%). ESI[M + H]⁺ = 467.1.

¹H NMR (400 MHz, DMSO-d6) δ 8.54 (ddd, *J =* 4.8, 1.7, 0.9 Hz, 1H), 8.08 (d, *J =* 7.9 Hz, 1H), 7.94 (td, *J* = 7.7*,* 1.8 Hz, 1H), 7.88 (dd, *J =* 8.7, 2.3 Hz, 1H), 7.65 (d, *J =* 8.7 Hz, 1H), 7.61 (d, *J =* 2.3 Hz, 1H), 7.49 (ddd, *J* = 7.5, 4.8, 1.2 Hz, 1H), 6.80 (d, *J =* 1.1 Hz, 1H), 4.19 - 4.09 (m, 2H), 4.02 (dd, *J =* 8.2, 6.0 Hz, 1H), 2.42 - 2.32 (m, 2H), 2.31 - 2.24 (m, 5H), 2.03 - 1.90 (m, 1H), 1.88 - 1.80 (m, 1H), 1.00 (t, *J* = 7.5 Hz, 3H).

### 3. Preparation of Compound 170

The preparation method of **compound 170 is** similar to **compound 169** in **example 83,** using **compound 169-1** and **Isopropyl chloroformate** as starting materials.

Compound **170:** 37.3 mg, ESI[M+H]⁺ = 481.1_{∘}

¹H NMR (400 MHz, DMSO-d6) δ 8.57 - 8.51 (m, 1H), 8.08 (d, *J =* 7.9 Hz, 1H), 7.94 (td, *J =* 7.7, 1.8 Hz, 1H), 7.87 (dd, *J =* 8.7, 2.3 Hz, 1H), 7.65 (d, *J =* 8.7 Hz, 1H), 7.61 (d, *J = 2.3* Hz, 1H), 7.49 (ddd, *J =* 7.5, 4.8, 1.2 Hz, 1H), 6.80 (d, *J =* 1.1 Hz, 1H), 4.13 (t, *J =* 6.5 Hz, 2H), 4.02 (dd, *J* = 8.0, 6.1 Hz, 1H), 2.48 - 2.43 (m, 1H), 2.42 - 2.31 (m, 2H), 2.29 (d, *J =* 0.8 Hz, 3H), 1.94 - 1.91 (m, 1H), 1.89 - 1.80 (m, 1H), 1.06 (d, *J =* 1.2 Hz, 3H), 1.04 (d, *J =* 1.2 Hz, 3H).

### 4. Preparation of Compound 171-1

At 0 °C, *p*-nitrophenyl chloroformate (215 mg, 1.07 mmol) was added to solution of **169-1** (146 mg, 0.355 mmol), pyridine (55 mg, 0.695 mmol) and DMAP (87 mg, 0.712 mmol) in dichloromethane (5 mL). The mixture was a stirred at room temperature for 12 hours. After confirming reaction completion by TLC, ice-water (10 mL) was added to the mixture, the mixture was extracted with dichloromethane (3 × 10 mL). The organic phase was washed with brine, dried over anhydrous Na₂SO₄, filtered, and the filtrate was concentrated under reduced pressure to afford the crude product, the crude product was purified by silica gel column chromatography (ethyl acetate/petroleum ether, v/v = 1/20-1/1), TLC (ethyl acetate/petroleum ether, v/v =1/1) momnitoring and the fractions with Rf = 0.3-0.4 were collected to yield Compound **171-1** as white solid (120 mg, yield 58.6%). ESI[M + H]⁺ = 576.1.

### 5. Preparation of Compound 171

**171-1** (60 mg, 0.104 mmol) and DIEA (13 mg, 0.101 mmol) were dissolved in dichloromethane (2 mL), then dimethylamine (0.1 mL, 2 mol/L in THF, 0.2 mmol) was added, and the mixture was stirred at room temperature for 2 hours. After confirming reaction completion by TLC, ice-water (10 mL) was added to the mixture, the mixture was extracted with dichloromethane (3 × 10 mL). The organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield Compound **171** as white solid (13.7 mg, yield 27.3%). ESI[M + H]⁺ = 482.1.

¹H NMR (400 MHz, DMSO-d6) δ 8.54 (d, *J =* 4.0 Hz, 1H), 8.08 (d, *J =* 8.0 Hz, 1H), 7.94 (td, *J =* 7.7, 1.7 Hz, 1H), 7.87 (dd, *J =* 8.7, 2.3 Hz, 1H), 7.65 (d, *J=* 8.7 Hz, 1H), 7.60 (d, *J =* 2.3 Hz, 1H), 7.49 (dd, *J =* 6.4, 4.8 Hz, 1H), 6.80 (d, *J =* 1.0 Hz, 1H), 4.10 - 4.07 (m, 2H), 4.04 - 3.98 (m, 1H), 2.78 (s, 6H), 2.41 - 2.32 (m, 2H), 2.30 (s, 3H), 1.98 - 1.79 (m, 2H).

### 6. Preparation of Compound 172

The preparation method of **compound 172** is similar to **compound 171** in **example 83,** using **compound 171-1** and **N-Isopropylmethylamine** as starting materials.

Compound **172:** 22.9 mg, ESI[M+H]⁺ = 510.2.

¹H NMR (400 MHz, DMSO-*d6*) δ 8.54 (d, *J =* 4.0 Hz, 1H), 8.08 (d, *J* = 7.9 Hz, 1H), 7.94 (td, *J =* 7.7, 1.7 Hz, 1H), 7.88 (dd, *J =* 8.7, 2.3 Hz, 1H), 7.65 (d, *J =* 8.7 Hz, 1H), 7.61 (d, *J =* 2.3 Hz, 1H), 7.49 (ddd, *J =* 7.5, 4.8, 1.1 Hz, 1H), 6.81 (s, 1H), 4.22 (s, 1H), 4.10 - 4.08 (m, 2H), 4.05 - 3.98 (m, 1H), 2.64 (s, 3H), 2.40 - 2.33 (m, 2H), 2.30 (s, 3H), 1.99 - 1.89 (m, 1H), 1.85 - 1.81 (m, 1H), 1.03 (d, *J =* 6.7 Hz, 6H).

### Example 84 Preparation of Compound 173 and 174

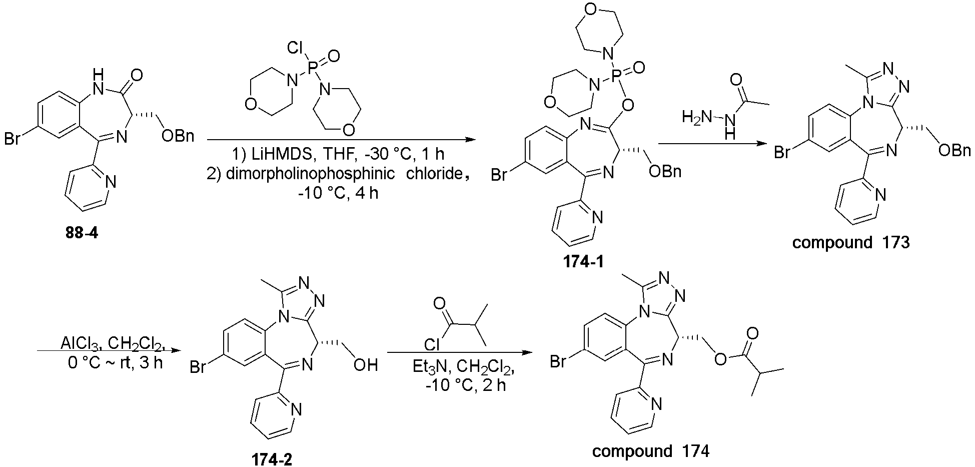

### 1. Preparation of Compound 174-1

At -30°C, a solution of lithium bis(trimethylsilyl)amide (8.3 mL, 1 mol/L in THF, 8.3 mmol) was added dropwise slowly to a solution of **88-4** (3.0 g, 6.88 mmol) in tetrahydrofuran (30 mL). The mixture was stirred at -30°C for 1 hour, then diphosphoramidous chloride (4.14 g, 16.3 mmol) was added portionwise, the mixture was stirred at -10°C for 4 hours. After confirming reaction completion by TLC, ice-water (40 mL) was added to the mixture, the mixture was extracted with ethyl acetate (3 × 40 mL). The organic phase was washed with brine, dried over anhydrous Na₂SO₄, filtered, and the filtrate was concentrated under reduced pressure to afford the crude product, the crude product was used directly in the next step without further purification.

### 2. Preparation of Compound 173

The crude product from the previous step was dissolved in 1,4-dioxane (30 mL), acetyl hydrazide (2.7 g, 36.4 mmol) was then added, and the resulting mixture was stirred at room temperature for 2 hours. The temperature was then raised to 100°C, and stirring was continued for an additional 5 hours. Ice-water (40 mL) was added to the mixture, the mixture was extracted with ethyl acetate (3 × 40 mL). The organic phase was washed with brine, dried over anhydrous Na₂SO₄, filtered, and the filtrate was concentrated under reduced pressure to afford the crude product, the organic phase was washed with brine, dried over anhydrous Na₂SO₄, filtered, and the filtrate was concentrated under reduced pressure to afford the crude product, the crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield Compound **173** as off-yellow solid (1.395 g, two steps yield 42.8%). ESI[M + H]⁺ = 474.1.

¹H NMR (400 MHz, DMSO-d6) δ 8.56 (d, *J =* 4.3 Hz, 1H), 8.10 (d, *J =* 7.8 Hz, 1H), 8.00 - 7.96 (m, 2H), 7.79 (d, *J =* 8.8 Hz, 1H), 7.68 (d, *J =* 2.1 Hz, 1H), 7.57 - 7.48 (m, 1H), 7.45 - 7.35 (m, 4H), 7.32 - 7.29 (m, 1H), 4.72 (s, 2H), 4.52 - 4.43 (m, 2H), 4.41 - 4.36 (m, 1H), 2.55 (s, 3H).

### 3. Preparation of Compound 174-2

At 0°C, aluminum chloride (3.79 g, 28.4 mmol) was added slowly to the solution of 173 (1.345 g, 2.84 mmol) in dichloromethane (30 mL), the mixture was stirred at room temperature for 3 hours. After confirming reaction completion by TLC, ice-water (50 mL) was added to the mixture and filtered, the filtrate was extracted with dichloromethane (3 × 60 mL). The combined organic phases were washed with 5% aqueous sodium bicarbonate solution, followed by brine, dried over anhydrous sodium sulfate, filtered, and concentrated under reduced pressure to afford the crude product. The crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield Compound **174-2** as white solid (650 mg, yield 59.7%). ESI[M + H]⁺ = 384.1.

### 4. Preparation of Compound 174

At -10°C, isobutyryl chloride (22.1 mg, 0.207 mmol) was added dropwise to the solution of **174-2** (80 mg, 0.208 mmol) and triethylamine (52.7 mg, 0.521 mmol) in dichloromethane (3 mL), the mixture was stirred at -10°C for 2 hours. After confirming reaction completion by TLC, ice-water (50 mL) was added to the mixture and filtered. The filtrate was extracted with dichloromethane (3 × 10 mL). The combined organic phases were washed with brine, dried over anhydrous sodium sulfate, filtered, and concentrated under reduced pressure to afford the crude product. The crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.4-0.5 were collected to yield Compound **174** as white solid (46.8 mg, yield 49.5%). ESI[M + H]⁺ = 454.1.

¹H NMR (400 MHz, DMSO-d6) δ 8.57 (d, *J =* 4.5 Hz, 1H), 8.07 (d, *J =* 7.9 Hz, 1H), 8.02 - 7.94 (m, 2H), 7.82 (d, *J* = 8.7 Hz, 1H), 7.71 (d, *J =* 2.2 Hz, 1H), 7.54 (dd, *J =* 6.5, 4.9 Hz, 1H), 4.98 (dd, *J =* 11.0, 7.7 Hz, 1H), 4.90 (dd, *J =* 11.0, 5.6 Hz, 1H), 4.63 (dd, *J =* 7.5, 5.7 Hz, 1H), 2.63 - 2.58 (m, 1H), 2.57 (s, 3H), 1.15 - 1.12 (m, 6H).

### Example 85 Preparation of Compound 175

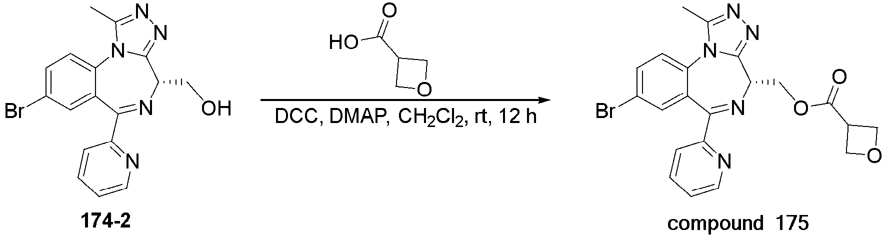

At room temperature, 3-Oxetanecarboxylic acid (39.9 mg, 0.391 mmol), DCC (80.7 mg, 0.391 mmol) and DMAP (95.6 mg, 0.783 mmol) were dissolved in dichloromethane (2 mL), and the mixture was stirred for 15 minutes. Compound **174-2** (100 mg, 0.260 mmol) was then added to the mixture, and stirred at room temperature for 12 hours. After confirming reaction completion by TLC, methyl tert-butyl ether (2 mL) was added to the mixture and stirred for 5 minutes. The mixture was filtered, and the filtrate was concentrated under reduced pressure to afford the crude product. The crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield Compound **175** as white solid (36.5 mg, yield 29.9%). ESI[M + H]⁺ = 468.1.

¹H NMR (400 MHz, DMSO-d6) δ 8.57 (d, *J =* 4.1 Hz, 1H), 8.08 (d, *J =* 7.9 Hz, 1H), 8.02 - 7.94 (m, 2H), 7.82 (d, *J =* 8.7 Hz, 1H), 7.71 (d, *J =* 2.3 Hz, 1H), 7.57 - 7.50 (m, 1H), 5.09 - 4.99 (m, 2H), 4.76 - 4.72 (m, 2H), 4.71 - 4.66 (m, 3H), 4.01 - 3.94 (m, 1H), 2.57 (s, 3H).

### Example 86 Preparation of Compound 176

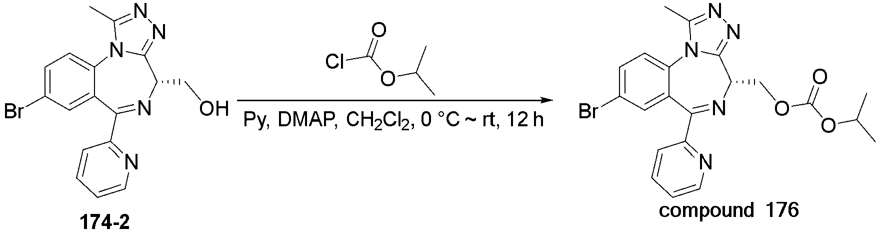

At 0°C, isopropyl chloroformate (95.6 mg, 0.780 mmol) was added dropwise to the solution of **174-2** (100 mg, 0.260 mmol), pyridine (60.3 mg, 0.762 mmol) and DMAP (31.9 mg, 0.261 mmol) in dichloromethane (2 mL). The mixture was stirred room temperature for 12 hours. After confirming reaction completion by TLC, ice-water (10 mL) was added to the mixture and filtered. The filtrate was extracted with dichloromethane (3 × 10 mL). The combined organic phases were washed with brine, dried over anhydrous sodium sulfate, filtered, and concentrated under reduced pressure to afford the crude product. The crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.3-0.4 were collected to yield Compound 176 as white solid (45.4 mg, yield 37.1%). ESI[M + H]⁺ = 470.1.

¹H NMR (400 MHz, DMSO-d6) δ 8.58 (d, *J =* 4.8 Hz, 1H), 8.08 (d, *J =* 7.9 Hz, 1H), 8.02 - 7.95 (m, 2H), 7.82 (d, *J =* 8.7 Hz, 1H), 7.72 (d, *J =* 2.3 Hz, 1H), 7.58 - 7.50 (m, 1H), 5.05 - 4.95 (m, 2H), 4.89 - 4.79 (m, 1H), 4.67 (t, *J=* 6.5 Hz, 1H), 2.57 (s, 3H), 1.29 - 1.25 (m, 6H).

### Example 87 Preparation of Compound 177

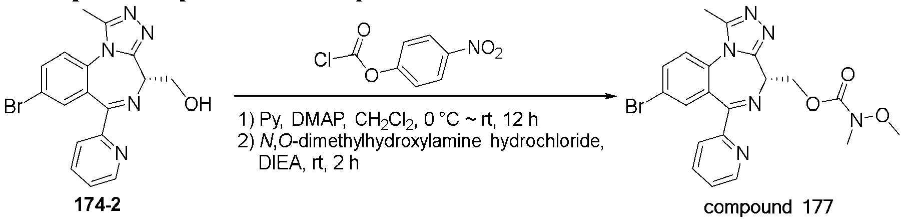

At 0°C, *p*-nitrophenyl chloroformate (158 mg, 0.784 mmol) was added to solution of **174-2** (100 mg, 0.260 mmol), pyridine (60.3 mg, 0.762 mmol) and DMAP (63.7 mg, 0.521 mmol) in dichloromethane (5 mL). The mixture was stirred at room temperature for 12 hours. After confirming reaction completion by TLC, dimethylhydroxylamine hydrochloride (153 mg, 1.57 mmol) and DIEA (202 mg, 1.56 mmol) were added to the mixture and stirred at room temperature for 2 hours. After confirming reaction completion by TLC, ice-water (10 mL) was added to the mixture and filtered, the filtrate was extracted with dichloromethane (3 × 10 mL). The combined organic phases were washed with brine, dried over anhydrous sodium sulfate, filtered, and concentrated under reduced pressure to afford the crude product. The crude product was purified by silica gel column chromatography (MeOH/CH₂Cl₂, v/v = 1/100-1/20), TLC (MeOH/CH₂Cl₂, v/v =1/10) momnitoring and the fractions with Rf = 0.3-0.4 were collected to yield Compound **177** as white solid (66.3 mg, yield 54.1%). ESI[M + H]⁺ = 471.2.

¹H NMR (400 MHz, DMSO-d6) δ 8.58 (d, *J =* 4.2 Hz, 1H), 8.08 (d, *J =* 7.9 Hz, 1H), 8.02 - 7.96 (m, 2H), 7.83 (d, *J =* 8.7 Hz, 1H), 7.71 (d, *J =* 2.2 Hz, 1H), 7.54 (dd, *J =* 6.4, 4.9 Hz, 1H), 5.04 - 4.93 (m, 2H), 4.66 (t, *J =* 6.6 Hz, 1H), 3.63 (s, 3H), 3.12 (s, 3H), 2.57 (s, 3H).

### Example 88 Preparation of Compound 178

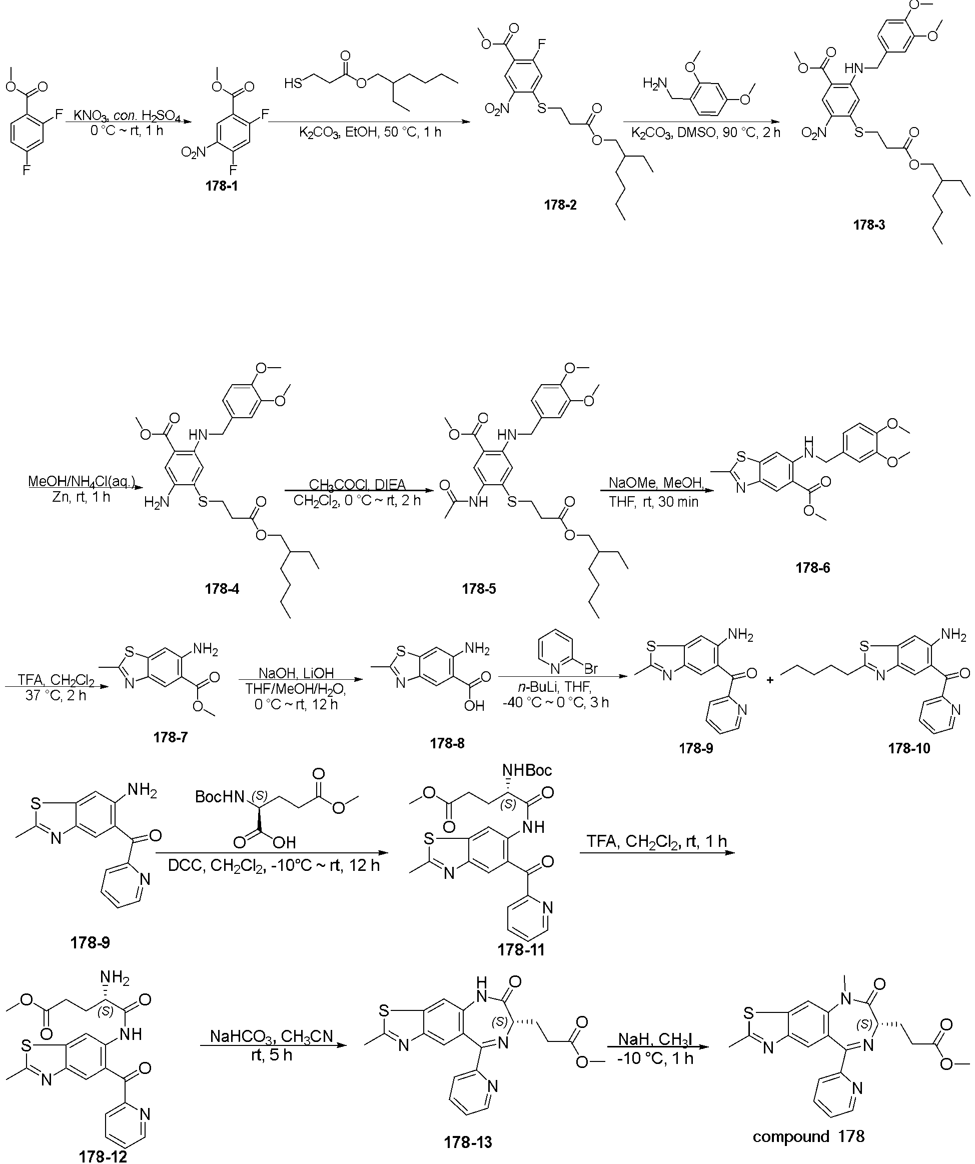

### 1. Preparation of Compound 178-1

Potassium nitrate (15.3 g, 151.3 mmol) was dissolved in concentrated sulfuric acid (50 mL). The resulting solution was added dropwise to the solution of methyl 2,4-difluorobenzoate (20.0 g, 116.2 mmol) in concentrated sulfuric acid (200 mL) at 0°C, while maintaining the temperature below 10°C. Then the mixture was stirred at room temperature for 1 hour. After confirming reaction completion by TLC, ice-water was added to the mixture and filtered. The filter cake was washed three times with water and dried under vacuum to yield Compound **178-1** (24.5 g, yield 97.1%). ESI[M + H]⁺ = 218.1.

### 2. Preparation of Compound 178-2

Compound **178-1** (24.5 g, 112.8 mmol) was dissolved in ethanol (250 mL), potassium carbonate (43.6 g, 315.5 mmol) and 2-ethylhexyl 3-mercaptopropanoate (24.6 g, 112.8 mmol) were added to the mixture. The mixture was stirred at 50°C for 1 hour. After confirming reaction completion by TLC, the mixture was concentrated under reduced pressure. The residue was purified by silica gel column chromatography (ethyl acetate/petroleum ether (v/v) = 1/50-1/10). TLC (ethyl acetate/petroleum ether, v/v =1/5) momnitoring and the fractions with Rf = 0.3-0.4 were collected to yield Compound **178-2** as white solid (41.9 g, yield 89.4%). ESI[M + H]⁺ = 416.1.

### 3. Preparation of Compound 178-3

Compound **178-2** (41.0 g, 98.7 mmol) was dissolved in DMSO (400 mL), potassium carbonate (20.5 g, 148.3 mmol) and 2,4-dimethoxybenzylamine (16.5 g, 98.7 mmol) were added to the mixture, the mixture was stirred at 90 °C for 2 hours. After confirming reaction completion by TLC, ice-water was added to the mixture and filtered, the filtrate was extracted with ethyl acetate (3 × 200 mL). The combined organic phases were washed with brine, dried over anhydrous sodium sulfate, filtered, and concentrated under reduced pressure to afford the crude product. The crude product was purified by silica gel column chromatography (ethyl acetate/petroleum ether, v/v = 1/50-1/10), TLC (ethyl acetate/petroleum ether, v/v =1/5) momnitoring and the fractions with Rf = 0.3-0.4 were collected to yield Compound **178-3** as white solid (44.6 mg, yield 80.3%). ESI[M + H]⁺ = 563.3.

### 4. Preparation of Compound 178-4

At room temperature, Zinc powder (13.0 g, 198.8 mmol) was added portionwise to the solution of Compound **178-3** (37.6 g, 66.8 mmol) in a mixed solvent of methanol and saturated ammonium chloride aqueous solution (500 mL, v/v = 4:1). The reaction mixture was stirred at room temperature for 1 hour. After confirming reaction completion by TLC, the mixture was filtered, the filtrate was concentrated under reduced pressure. The residue was dissolved in ethyl acetate (300 mL), washed with brine, dried over anhydrous Na₂SO₄, filtered, and concentrated under reduced pressure to afford the crude product. The crude product was purified by silica gel column chromatography (ethyl acetate/petroleum ether, v/v = 1/100-1/10), TLC (ethyl acetate/petroleum ether, v/v =1/3) momnitoring and the fractions with Rf = 0.2-0.3 were collected to yield Compound **178-4** (26.3 g, yield 73.9%). ESI[M + H]⁺ = 533.2.

### 5. Preparation of Compound 178-5

At 0°C, acetyl chloride (1.7 g, 21.7 mmol) was added dropwise to solution of Compound **178-4** (14.4 g, 27.0 mmol) and DIEA (6.98 g, 54.0 mmol) in dichloromethane (150 mL). The mixture was stirred at room temperature for 2 hours. After confirming reaction completion by TLC, ice-water (100 mL) was added to the mixture and filtered. The filtrate was extracted with dichloromethane (3 × 100 mL). The combined organic phases were washed with brine, dried over anhydrous sodium sulfate, filtered, and concentrated under reduced pressure to afford the crude product. The crude product was purified by silica gel column chromatography (ethyl acetate/petroleum ether, v/v = 1/10-1/1), TLC (ethyl acetate/petroleum ether, v/v =1/1) momnitoring and the fractions with Rf = 0.3-0.4 were collected to yield Compound **178-5** (4.2 g, yield 27.0%). ESI[M + H]⁺ = 575.3.

### 6. Preparation of Compound 178-6

At room temperature, Compound **178-5** (4.2 g, 7.31 mmol) was dissolved in tetrahydrofuran (42 mL), the solution of sodium methoxide (1.18 g, 21.8 mmol) in methanol (20 mL) was added dropwise to the mixture, then the mixture was stirred for 30 minutes. After confirming reaction completion by TLC, the reaction mixture was concentrated under reduced pressure. The crude product was used directly in the next step without further purification. ESI[M + H]⁺ = 373.1.

### 7. Preparation of Compound 178-7

The crude product from the previous step was dissolved in dichloromethane (30 mL), and trifluoroacetic acid (10 mL) was added. The mixture was stirred at 37°C for 2 hours. After confirming reaction completion by TLC, the mixture was concentrated under reduced pressure. The residue was dissolved in water, and the pH of the solution was adjusted to 8-9 with saturated aqueous sodium bicarbonate solution. The mixture was then extracted with ethyl acetate (3 × 30 mL). The combined organic phases were washed with brine, dried over anhydrous Na₂SO₄, filtered, and concentrated under reduced pressure to afford the crude product. The crude product was purified by silica gel column chromatography (MeOH/CH₂Cl₂, v/v = 1/100-1/30), TLC (MeOH/CH₂Cl₂, v/v =1/30) momnitoring and the fractions with Rf = 0.4-0.5 were collected to yield Compound **178-7** (1.6 g, two steps yield 98.5%). ESI[M + H]⁺ = 223.1.

### 8. Preparation of Compound 178-8

At 0°C, LiOH·H₂O (360 mg, 8.58 mmol) and NaOH (378 mg, 9.45 mmol) were successively added to a solution of Compound **178-7** (1.6 g, 7.20 mmol) in a mixed solvent of THF/MeOH/H₂O (20 mL, v/v/v = 1:1:1). The mixture was stirred at room temperature for 12 hours. After confirming reaction completion by TLC, the pH was adjusted to 6-7 with 1 mol/L hydrochloric acid in ice-bath, the mixture was then extracted with ethyl acetate (3 × 30 mL). The combined organic phases were washed with brine, dried over anhydrous Na₂SO₄, filtered, and concentrated under reduced pressure to afford the crude product. The crude product was purified by silica gel column chromatography (MeOH/CH₂Cl₂, v/v = 1/100-1/10), TLC (MeOH/CH₂Cl₂, v/v =1/10) momnitoring and the fractions with Rf = 0.3-0.4 were collected to yield Compound **178-8** (1.3 g, yield 86.7%). ESI[M + H]⁺ = 209.1.

### 9. Preparation of Compound 178-9 and Compound 178-10

At -40°C, 2-bromopyridine (347.6 mg, 2.2 mmol) was added dropwise to the solution of n-butyllithium (0.8 mL, 2.5 mol/L in hexane, 2.0 mmol) in tetrahydrofuran (3 mL), then the mixture was stirred for 30 minutes. A solution of Compound **178-8** (111 mg, 0.533 mmol) in tetrahydrofuran (2 mL) was then added dropwise to th mixture. The mixture was stirred at 0°C and stirred for 3 hours. After confirming reaction completion by TLC, ice-water was added to the mixture and filtered. The filtrate was extracted with ethyl acetate (3 × 20 mL). The combined organic phases were washed with brine, dried over anhydrous sodium sulfate, filtered, and concentrated under reduced pressure to afford the crude product. The crude product was purified by silica gel column chromatography (ethyl acetate/petroleum ether, v/v = 1/50-1/1), TLC (ethyl acetate/petroleum ether, v/v =1/1) momnitoring and the fractions with Rf = 0.3-0.5 were collected to yield Compound **178-9** (10 mg, yield 6.97%) and Compound **178-10** (31.7 mg, yield 18.3%)_{∘}

**178-9:** ESI[M + H]⁺ = 270.1. **178-10:** ESI[M + H]⁺ = 326.1.

### 10. Preparation of Compound 178-11

At room temperature, Compound **178-9** (10 mg, 0.037 mmol) and Boc-L-glutamic acid 5-methyl ester (12.6 mg, 0.048 mmol) were dissolved in dichloromethane (2 mL), then the solution of DCC (17.6 mg, 0.085 mmol) in dichloromethane (1 mL) was added dropwise to the mixture at -10°C, the mixture was stirred at room temperature for 12 hours. After confirming reaction completion by TLC, methyl tert-butyl ether (3 mL) was added, and the mixture was stirred for 5 minutes. The mixture was filtered, and the filtrate was concentrated under reduced pressure to afford the crude product. The crude product was purified by pre-TLC (ethyl acetate/petroleum ether, v/v = 1/1), and the fractions with Rf = 0.2-0.3 were collected to yield Compound **178-11** (16.2 mg, yield 85.1%)_{∘} ESI[M + H]⁺ = 513.2.

### 11. Preparation of Compound 178-12

At room temperature, Trifluoroacetic acid (1 mL) was added to solution of Compound **178-11** (16.2 mg, 0.032 mmol) in dichloromethane (2 mL), and the mixture was stirred at room temperature for 1 hour. After confirming reaction completion by TLC, the mixture was concentrated under reduced pressure. The crude product was used directly in the next step without further purification. ESI [M + H]⁺ = 413.1.

### 12. Preparation of Compound 178-13

The crude product from the previous step was dissolved in acetonitrile (2 mL), and sodium bicarbonate (80 mg, 0.952 mmol) was added. The mixture was stirred at room temperature for 5 hours. After confirming reaction completion by TLC, the mixture was filtered, the filtrate was concentrated under reduced pressure to afford the crude product, and the crude product was purified by pre-TLC (ethyl acetate/petroleum ether (v/v) = 4:1). The fraction with Rf = 0.2-0.3 was collected to yield Compound **178-13** (12.0 mg, two steps yield 96.3%). ESI[M + H]⁺ = 395.1.

### 13. Preparation of Compound 178

At -10°C, sodium hydride (1.34 mg, 60% dispersion in mineral oil, 0.033 mmol) was added to solution of Compound **178-13** (12.0 mg, 0.03 mmol) in anhydrous DMF (2 mL). The mixture was stirred for 30 minutes. Iodomethane (4.76 mg, 0.033 mmol) was then added to the reaction mixture, and stirring was continued at -10°C for 1 hour. After confirming reaction completion by TLC, ice-water (5 mL) was added to the mixture and filtered. The filtrate was extracted with ethyl acetate (3 × 10 mL). The combined organic phases were washed with brine, dried over anhydrous sodium sulfate, filtered, and concentrated under reduced pressure to afford the crude product. The crude product was purified by pre-TLC (MeOH/CH₂Cl₂, v/v = 1/10), TLC (MeOH/CH₂Cl₂, v/v =1/10) momnitoring and the fractions with Rf = 0.4-0.5 were collected to yield Compound 178 (7.2 mg, yield 57.9%). ESI[M + H]⁺ = 409.1.

¹H NMR (400 MHz, DMSO-d6) δ 8.55 (d, *J =* 4.0 Hz, 1H), 8.29 (s, 1H), 8.14 (d, *J =* 7.9 Hz, 1H), 8.01 - 7.95 (m, 1H), 7.78 (s, 1H), 7.52 (ddd, *J =* 7.5, 4.8, 1.1 Hz, 1H), 3.75 (dd, *J =* 7.8, 5.6 Hz, 1H), 3.57 (s, 3H), 3.37 (s, 3H), 2.81 (s, 3H), 2.52 - 2.42 (m, 2H), 2.41 - 2.30 (m, 1H), 2.29 - 2.22 (m, 1H).

### Example 89 Preparation of Compound 179

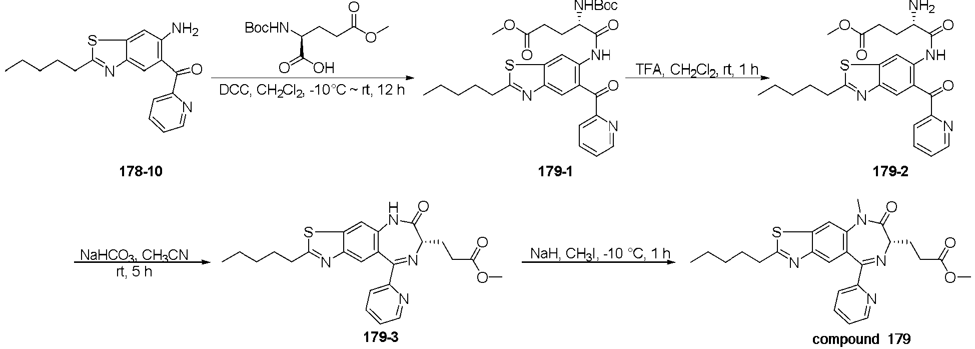

### 1. Preparation of Compound 179-1

Compound **178-10** (31.7 mg, 0.097 mmol) and Boc-L-glutamic acid 5-methyl ester (33.0 mg, 0.126 mmol) were dissolved in dichloromethane (2 mL). At -10 °C, the solution of DCC (26.0 mg, 0.126 mmol) in dichloromethane (1 mL) was added dropwise to the mixture. Then the mixture was stirred at room temperature for 12 hours. After confirming reaction completion by TLC, methyl tert-butyl ether (3 mL) was added, and the mixture was stirred for 5 minutes. The mixture was then filtered, and the filtrate was concentrated under reduced pressure to afford the crude product. The crude product was purified by pre-TLC (MeOH/CH₂Cl₂, v/v = 1/10), TLC (ethyl acetate/petroleum ether, v/v =1/1) momnitoring and the fractions with Rf = 0.2-0.3 were collected to yield Compound **179-1** (51.1 mg, yield 92.2%). ESI[M + H]⁺ = 569.2.

### 2. Preparation of Compound 179-2

Trifluoroacetic acid (1 mL) was added to the solution of Compound **179-1** (51.1 mg, 0.09 mmol) in dichloromethane (2 mL). The reaction mixture was stirred at room temperature for 1 hour. After the reaction was confirmed to be complete by TLC, the mixture was concentrated under reduced pressure. The crude product was used directly in the next step without further purification. ESI[M + H]⁺ = 469.2.

### 3. Preparation of Compound 179-3

The crude product from the previous step was dissolved in acetonitrile (2 mL), and sodium bicarbonate (226 mg, 2.69 mmol) was added. The mixture was stirred at room temperature for 5 hours. After confirming reaction completion by TLC, the mixture was filtered. the filtrate was concentrated under reduced pressure to afford the crude product. The crude product was purified by pre-TLC (ethyl acetate/petroleum ether, v/v = 5/1), and the fractions with Rf = 0.2-0.3 were collected to yield Compound **179-3** (34.9 mg,two steps yield 86.2%). ESI[M + H]⁺ = 451.2.

### 4. Preparation of Compound 179

At -10°C, sodium hydride (3.7 mg, 60% dispersion in mineral oil, 0.092 mmol) was added to the solution of Compound **179-3** (34.9 mg, 0.077 mmol) in anhydrous DMF (2 mL). The mixture was stirred for 30 minutes. Then Iodomethane (13.2 mg, 0.093 mmol) was added to the mixture, and stirred at -10°C for 1 hour. After confirming reaction completion by TLC, ice-water (5 mL) was added to the mixture and filtered, the filtrate was extracted with ethyl acetate (3 × 10 mL). The combined organic phases were washed with brine, dried over anhydrous sodium sulfate, filtered, and concentrated under reduced pressure to afford the crude product. The crude product was purified by pre-TLC (MeOH/CH₂Cl₂, v/v = 1/10), and the fractions with Rf = 0.4-0.5 were collected to yield Compound **179** (16.4 mg, yield 45.6%). ESI[M + H]⁺ = 465.2.

¹H NMR (400 MHz, DMSO-*d6*) δ 8.55 (d, *J =* 4.2 Hz, 1H), 8.30 (s, 1H), 8.14 (d, *J =* 7.9 Hz, 1H), 8.02 - 7.94 (m, 1H), 7.80 (s, 1H), 7.56 - 7.49 (m, 1H), 3.80 - 3.73 (m, 1H), 3.57 (s, 3H), 3.38 (s, 3H), 3.11 (t, *J =* 7.5 Hz, 2H), 2.52 - 2.41 (m, 2H), 2.36 - 2.34 (m, 1H), 2.30 - 2.19 (m, 1H), 1.87 - 1.75 (m, 2H), 1.38 - 1.32 (m, 4H), 0.87 (t, *J =* 7.0 Hz, 3H).

### Example 90 Preparation of Compound 180 and Compound 181

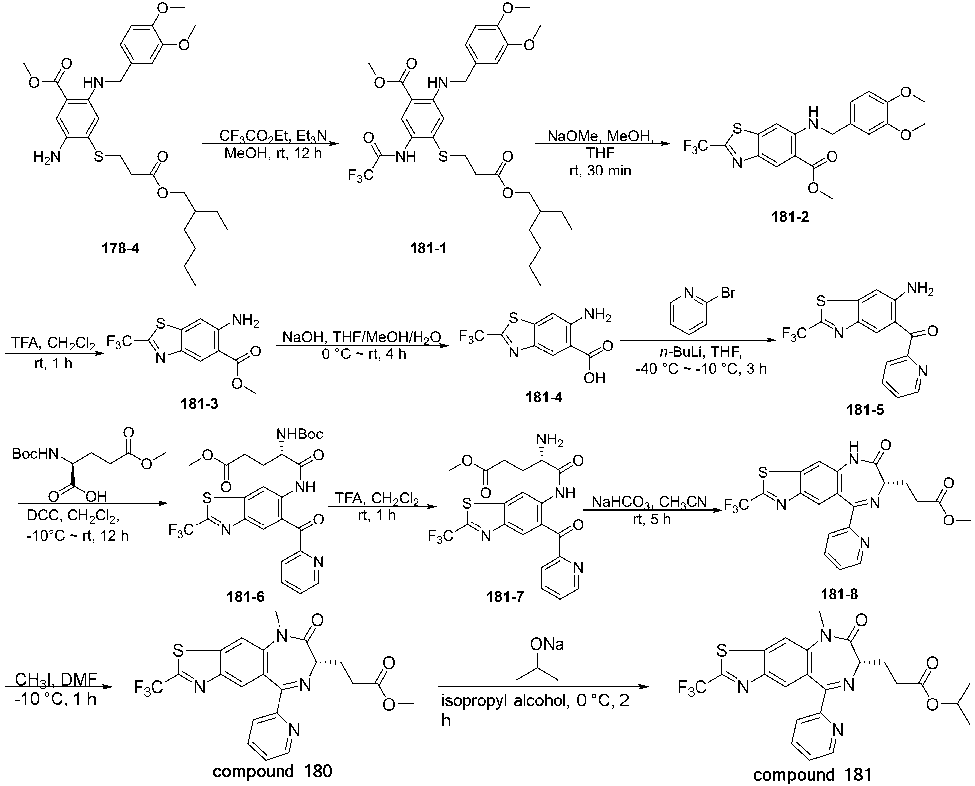

### 1. Preparation of Compound 181-1

Compound **178-4** (11.9 g, 22.3 mmol) was dissolved in methanol (120 mL), ethyl trifluoroacetate (9.53 g, 67.1 mmol) and triethylamine (2.26 g, 22.3 mmol) were added to the solution. The mixture was stirred at room temperature for 12 hours. After confirming reaction completion by TLC, the mixture was concentrated under reduced pressure. The crude product was purified by silica gel column chromatography (ethyl acetate/petroleum ether, v/v = 1/100-1/3), TLC (ethyl acetate/petroleum ether, v/v =1/3) momnitoring and the fractions with Rf = 0.4-0.5 were collected to yield Compound **181-1** (6.2 g, yield 44.1%) .ESI[M + H]⁺ = 629.2.

### 2. Preparation of Compound 181-2

At room temperature, Compound **181-1** (6.2 g, 9.86 mmol) was dissolved in tetrahydrofuran (62 mL), the solution of sodium methoxide (1.75 g, 32.4 mmol) in methanol (20 mL) was added dropwise to the mixture, then the mixture was stirred for 30 minutes. After confirming reaction completion by TLC, the mixture was concentrated under reduced pressure. The crude product was used directly in the next step without further purification. ESI[M + H]⁺ = 427.1.

### 3. Preparation of Compound 181-3

The crude product from the previous step was dissolved in dichloromethane (40 mL), and trifluoroacetic acid (20 mL) was added. The mixture was stirred at room temperature for 1 hour. After confirming reaction completion by TLC, the mixture was concentrated under reduced pressure. The residue was dissolved in water, and the pH was adjusted to 8-9 with saturated aqueous sodium bicarbonate solution. The mixture was extracted with ethyl acetate (3 × 50 mL). The combined organic phases were washed with brine, dried over anhydrous Na₂SO₄, filtered, and concentrated under reduced pressure to afford the crude product. Purification by silica gel column chromatography (ethyl acetate/petroleum ether (v/v) = 1/50-1/2). TLC (ethyl acetate/petroleum ether, v/v =1/2) momnitoring and the fractions with Rf = 0.2-0.3 were collected to yield Compound **181-3** (1.224 g, two steps yield 44.9%) .ESI[M + H]⁺ = 277.0.

### 4. Preparation of Compound 181-4

At 0°C, sodium hydroxide (348 mg, 8.69 mmol) was added to the solution of Compound **181-3** (1.2 g, 4.34 mmol) in a mixed solvent of THF/MeOH/H₂O (12 mL, v/v/v = 1:1:1). The mixture was stirred at room temperature for 4 hours. After confirming reaction completion by TLC, the pH was adjusted to 6-7 with 1 mol/L hydrochloric acid in ice bath. The mixture was then extracted with ethyl acetate (3 × 30 mL), the combined organic phases were washed with brine, dried over anhydrous Na₂SO₄, filtered, and concentrated under reduced pressure to afford the crude product. Purification by silica gel column chromatography (MeOH/CH₂Cl₂ (v/v) = 1/100-1/10). TLC (MeOH/CH₂Cl₂, v/v =1/10) momnitoring and the fractions with Rf = 0.3-0.4 were collected to yield Compound **181-4** (1.05 g, yield 92.2%) .ESI[M + H]⁺ = 263.0.

### 5. Preparation of Compound 181-5

At -40°C, 2-bromopyridine (2.222 g, 14.1 mmol) was added dropwise to the solution of n-butyllithium (5.11 mL, 2.5 mol/L in hexane, 12.8 mmol) in tetrahydrofuran (10 mL), then the mixture was stirred for 30 minutes. The solution of Compound **181-4** (908 mg, 3.46 mmol) in tetrahydrofuran (2 mL) was then added dropwise to the mixture. The mixture was stirred at -10°C for 3 hours. After confirming reaction completion by TLC, ice-water was added to the mixture and filtered, the filtrate was extracted with ethyl acetate (3 × 20 mL). The combined organic phases were washed with brine, dried over anhydrous sodium sulfate, filtered, and concentrated under reduced pressure to afford the crude product. The crude product was purified by pre-TLC (MeOH/CH₂Cl₂, v/v = 1/100-1/10), TLC (MeOH/CH₂Cl₂, v/v =1/10) momnitoring and the fractions with Rf = 0.2-0.4 were collected to yield Compound **181-5** (506 mg, yield 45.2%). ESI[M + H]⁺ = 324.0.

### 6. Preparation of Compound 181-6

Compound **181-5** (506 mg, 1.57 mmol) and Boc-L-glutamic acid 5-methyl ester (613 mg, 2.35 mmol) were dissolved in dichloromethane (5 mL). At -10°C, the solution of DCC (484 mg, 2.35 mmol) in dichloromethane (2 mL) was added dropwise to the mixture. Then the mixture was stirred at room temperature for 12 hours. After confirming reaction completion by TLC, methyl tert-butyl ether (7 mL) was added, and the mixture was stirred for 5 minutes. The mixture was then filtered, and the filtrate was concentrated under reduced pressure to afford the crude product. Purification by silica gel column chromatography (ethyl acetate/petroleum ether (v/v) = 1/50-1/2). TLC (ethyl acetate/petroleum ether, v/v =1/2) momnitoring and the fractions with Rf = 0.2-0.4 were collected to yield Compound **181-6** (840 mg, yield 94.7%) .ESI[M + H]⁺ = 567.1.

### 7. Preparation of Compound 181-7

Trifluoroacetic acid (6 mL) was added to solution of Compound **181-6** (840 mg, 1.48 mmol) in dichloromethane (11 mL). The mixture was stirred at room temperature for 1 hour. After confirming reaction completion by TLC, the mixture was concentrated under reduced pressure. The crude product was used directly in the next step without further purification. ESI[M + H]⁺ = 467.1.

### 8. Preparation of Compound 181-8

The crude product from the previous step was dissolved in acetonitrile (10 mL), and sodium bicarbonate (4.716 g, 56.1 mmol) was added. The mixture was stirred at room temperature for 5 hours. After confirming reaction completion by TLC, filtered. The filtrate was concentrated under reduced pressure to afford the crude product. The crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield Compound **181-8** (638 mg, yield 96.0%). ESI[M + H]⁺ = 449.1.

### 9. Preparation of Compound 180

At -10°C, sodium hydride (85 mg, 60% dispersion in mineral oil, 2.12 mmol) was added to the solution of Compound **181-8** (638 mg, 1.42 mmol) in anhydrous DMF (10 mL), the mixture was stirred for 30 minutes. Iodomethane (242.6 mg, 1.71 mmol) was then added to the mixture, and stirred at -10°C for 1 hour. After confirming reaction completion by TLC, ice-water (5 mL) was added to the mixture, and filtered, the filtrate was extracted with ethyl acetate (3 × 10 mL). The combined organic phases were washed with brine, dried over anhydrous Na₂SO₄, filtered, and concentrated under reduced pressure to afford the crude product. The crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.4-0.5 were collected to yield Compound **180** (604 mg, yield 91.5%). ESI[M + H]⁺ = 463.1.

¹H NMR (400 MHz, DMSO-*d6*) δ 8.58 (s, 1H), 8.55 (d, *J =* 4.0 Hz, 1H), 8.23 (s, 1H), 8.19 (d, *J =* 7.9 Hz, 1H), 7.99 (td, *J* = 7.6, 1.7 Hz, 1H), 7.54 (dd, *J =* 6.4, 4.8 Hz, 1H), 3.81 (dd, *J =* 7.7, 5.5 Hz, 1H), 3.57 (s, 3H), 3.41 (s, 3H), 2.55 - 2.50 (m, 1H), 2.49 - 2.21 (m, 3H).

### 10. Preparation of Compound 181

At 0°C, the solution of sodium isopropoxide (196.8 mg, 20% in THF, 0.48 mmol) was added dropwise to solution of Compound **180** (184.8 mg, 0.40 mmol) in isopropanol (2 mL). Then the mixture was stirred for 2 hours. After confirming reaction completion by TLC, ice-water (10 mL) was added to the mixture and filtered. The filtrate was extracted with ethyl acetate (3 × 10 mL). The combined organic phases were washed with brine, dried over anhydrous Na₂SO₄, filtered, and concentrated under reduced pressure to afford the crude product. The crude product was purified by pre-TLC (ethyl acetate/petroleum ether, v/v = 1/1), and the fractions with Rf = 0.2-0.3 were collected to yield Compound **181** (43.2 mg, yield 22.0%). ESI[M + H]+ = 491.1.

¹H NMR (400 MHz, DMSO-d6) δ 8.59 (s, 1H), 8.56 (ddd, *J =* 4.8, 1.8, 0.9 Hz, 1H), 8.23 (s, 1H), 8.22 - 8.18 (m, 1H), 8.00 (td, *J =* 7.8, 1.8 Hz, 1H), 7.54 (ddd, *J =* 7.6, 4.8, 1.2 Hz, 1H), 4.90 - 4.81 (m, 1H), 3.81 (dd, *J =* 7.7, 5.5 Hz, 1H), 3.42 (s, 3H), 2.49 - 2.21 (m, 4H), 1.11 (t, *J =* 6.3 Hz, 6H).

### Example 91 Preparation of Compound 188

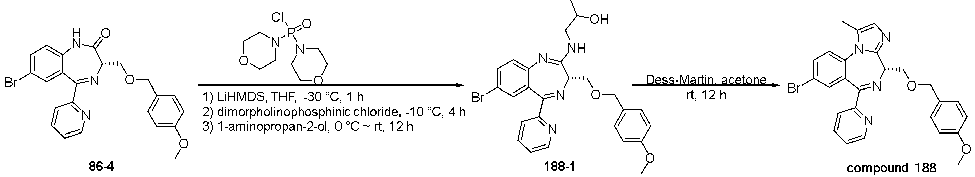

The preparation method of compound **188** is similar to compound **95** in example 42, using compound **86-4** as starting materials.

Compound **188:** 61.7 mg, ESI[M+H]⁺ = 502.8_{∘}

¹H NMR (400 MHz, DMSO-*d6*) δ 8.56 (d, *J=* 4.1 Hz, 1H), 8.09 (d, *J* = 7.9 Hz, 1H), 7.97 (td, *J =* 7.8, 1.7 Hz, 1H), 7.90 (dd, *J =* 8.7, 2.3 Hz, 1H), 7.67 - 7.64 (m, 2H), 7.54 - 7.48 (m, 1H), 7.32 (d, *J =* 8.6 Hz, 2H), 6.92 (d, *J =* 8.6 Hz, 2H), 6.81 (d, *J =* 0.9 Hz, 1H), 4.62 (s, 2H), 4.43 - 4.28 (m, 2H), 4.20 (dd, *J =* 7.4, 5.3 Hz, 1H), 3.76 (s, 3H), 2.32 (s, 3H).

### Example 92 Preparation of Compound 189

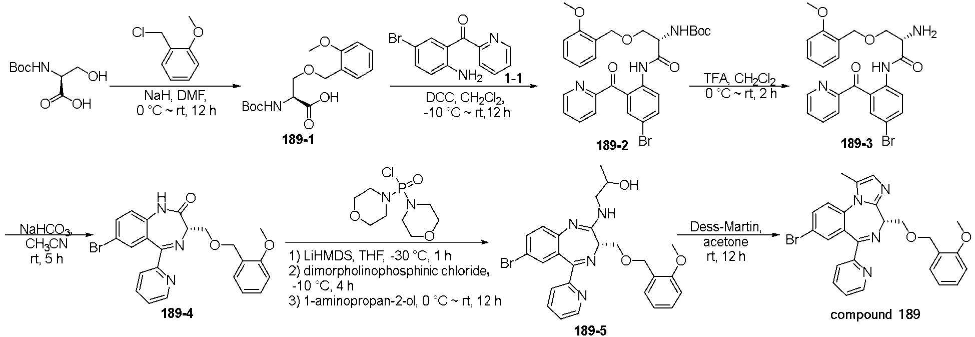

The preparation method of compound **189** is similar to compound **95** in example 42, using compound **189-4** as starting materials. Wherein, the preparation method of compound **189-4** is similar to compound **88-4** in example 39, using Boc-L-serine and 2-methoxybenzyl chloride to give the corresponding carboxylic acid. The carboxylic acid was then condensed with Compound **1-1,** followed by Boc deprotection and cyclization to afford the final product.

Compound **189:** 223.3 mg, ESI[M+H]⁺ = 502.8.

¹H NMR (400 MHz, DMSO-d6) δ 8.56 (d, *J =* 4.1 Hz, 1H), 8.09 (d, *J =* 7.9 Hz, 1H), 7.97 (td, *J =* 7.7, 1.7 Hz, 1H), 7.90 (dd, *J =* 8.7, 2.3 Hz, 1H), 7.67 (d, *J =* 8.7 Hz, 1H), 7.65 (d, *J =* 2.3 Hz, 1H), 7.51 (ddd, *J =* 7.5, 4.8, 1.0 Hz, 1H), 7.45 - 7.41 (m, 1H), 7.32 - 7.24 (m, 1H), 7.00 (d, *J =* 8.1 Hz, 1H), 6.95 (t, *J =* 7.4 Hz, 1H), 6.81 (d, *J* = 1.0 Hz, 1H), 4.76 - 4.64 (m, 2H), 4.46 (dd, *J* = 9.9, 5.2 Hz, 1H), 4.39 (dd, *J* = 9.9, 7.6 Hz, 1H), 4.25 (dd, *J =* 7.5, 5.2 Hz, 1H), 3.79 (s, 3H), 2.32 (s, 3H).

### Example 93 Preparation of Compound 190

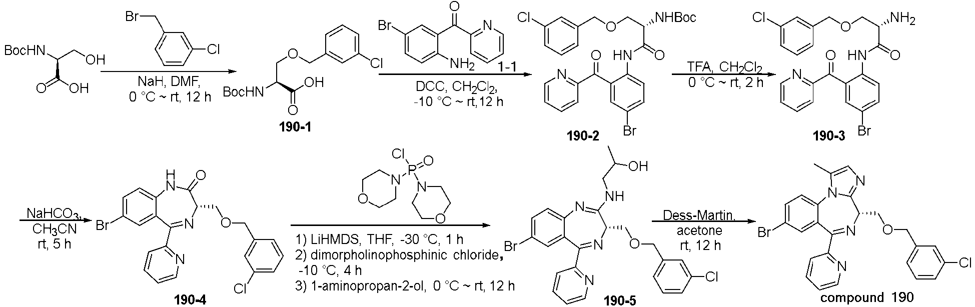

The preparation method of compound **190** is similar to compound **95** in example 42, using compound 190-**4** as starting materials. Wherein, the preparation method of compound **190-4** is similar to compound **88-4** in example 39, using Boc-L-serine and3-Chlorobenzyl bromide to give the corresponding carboxylic acid. The carboxylic acid was then condensed with Compound **1-1,** followed by Boc deprotection and cyclization to afford the final product.

Compound **190:** 466.8 mg, ESI[M+H]⁺ = 506.8.

¹H NMR (400 MHz, DMSO-d6) δ 8.57 (d, *J =* 4.1 Hz, 1H), 8.12 (d, *J =* 7.9 Hz, 1H), 7.97 (td, *J* = 7.7, 1.7 Hz, 1H), 7.91 (dd, *J =* 8.7, 2.3 Hz, 1H), 7.68 - 7.65 (m, 2H), 7.55 - 7.49 (m, 2H), 7.43 - 7.33 (m, 3H), 6.82 (d, *J =* 1.0 Hz, 1H), 4.73 (s, 2H), 4.48 - 4.35 (m, 2H), 4.27 (dd, *J =* 7.5, 5.2 Hz, 1H), 2.32 (s, 3H).

### Example 94 Preparation of Compound 191, Compound 192, Compound 196

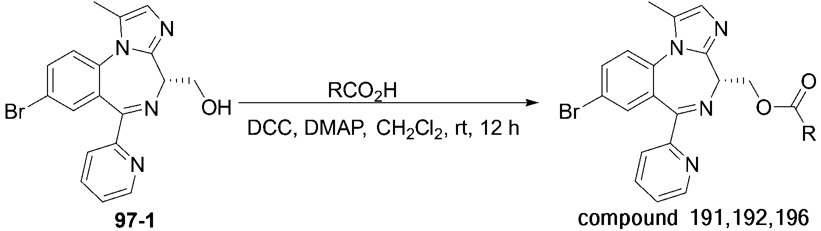

The preparation method of **Compound 191, Compound 192 and Compound 196** are similar to compound **101** in example 48, using compound **97-1** and the corresponding carboxylic acid as starting materials.

Compound **191:** 53.4 mg, ESI[M+H]⁺ = 487.1.

¹H NMR (400 MHz, DMSO-d6) δ 8.57 (d, *J =* 4.1 Hz, 1H), 8.07 (d, *J =* 7.9 Hz, 1H), 8.04 - 7.99 (m, 2H), 7.97 - 7.92 (m, 2H), 7.74 - 7.64 (m, 3H), 7.58 - 7.49 (m, 3H), 6.88 (d, *J =* 1.0 Hz, 1H), 5.18 (d, *J =* 6.6 Hz, 2H), 4.58 (t, *J =* 6.6 Hz, 1H), 2.35 (s, 3H).

Compound **192:** 70.3 mg, ESI[M+H]⁺ = 501.1.

¹H NMR (400 MHz, DMSO-*d6*) δ 8.57 (d, *J =* 4.2 Hz, 1H), 8.06 (d, *J =* 7.9 Hz, 1H), 7.98 - 7.87 (m, 4H), 7.71 (d, *J =* 8.7 Hz, 1H), 7.68 (d, *J =* 2.3 Hz, 1H), 7.53 - 7.50 (m, 1H), 7.35 (d, *J =* 8.1 Hz, 2H), 6.87 (d, *J =* 0.9 Hz, 1H), 5.16 (d, *J =* 7.1 Hz, 2H), 4.56 (t, *J =* 6.5 Hz, 1H), 2.40 (s, 3H), 2.35 (s, 3H).

Compound **196:** 90.9 mg, ESI[M+H]⁺ = 467.8.

¹H NMR (400 MHz, DMSO-d6) δ 8.57 (d, *J =* 4.1 Hz, 1H), 8.08 (d, *J* = 7.9 Hz, 1H), 7.96 (td, *J =* 7.8, 1.7 Hz, 1H), 7.92 (dd, *J =* 8.7, 2.3 Hz, 1H), 7.69 (d, *J =* 8.7 Hz, 1H), 7.67 (d, *J =* 2.3 Hz, 1H), 7.54 - 7.48 (m, 1H), 6.85 (d, *J =* 0.9 Hz, 1H), 5.01 - 4.90 (m, 2H), 4.38 (dd, *J* = 7.6, 5.5 Hz, 1H), 3.22 (d, *J =* 1.0 Hz, 2H), 2.33 (s, 3H), 2.27 (s, 6H).

### Example 95 Preparation of Compound 193, Compound 194, Compound 202-204

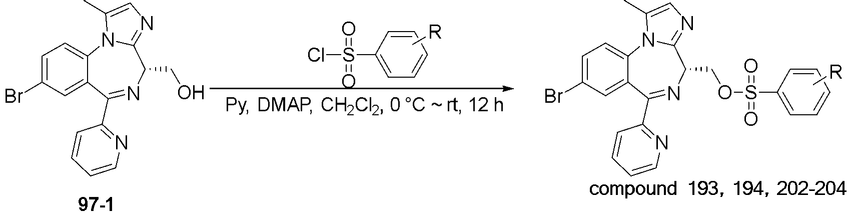

The preparation method of **Compound 193, Compound 194, Compound 202-204** are similar to compound **100** in example 47, using compound **97-1** and the corresponding carboxylic acid as starting materials.

Compound **193:** 16.4 mg, ESI[M+H]⁺ = 537.2.

¹H NMR (400 MHz, DMSO-d6) δ 8.56 (d, *J* = 4.5 Hz, 1H), 7.99 - 7.89 (m, 5H), 7.67 - 7.63 (m, 2H), 7.54 - 7.51 (m, 3H), 6.81 (s, 1H), 4.89 - 4.85 (m, 2H), 4.42 - 4.34 (m, 1H), 2.44 (s, 3H), 2.31 (s, 3H).

Compound **194:** 37.0 mg, ESI[M+H]⁺ = 553.2.

¹H NMR (400 MHz, DMSO-d6) δ 8.55 (d, *J =* 4.6 Hz, 1H), 7.99 - 7.87 (m, 5H), 7.66 - 7.63 (m, 2H), 7.55 - 7.48 (m, 1H), 7.21 (d, *J =* 9.0 Hz, 2H), 6.81 (s, 1H), 4.87 - 4.83 (m, 2H), 4.37 (dd, *J =* 7.5, 5.5 Hz, 1H), 3.88 (s, 3H), 2.31 (s, 3H).

Compound **202:** 69.6 mg, ESI[M+H]⁺ = 556.7.

¹H NMR (400 MHz, DMSO-d6) δ 8.56 (d, *J =* 4.8 Hz, 1H), 8.07 (t, *J =* 1.8 Hz, 1H), 8.01 (d, *J =* 7.8 Hz, 1H), 7.96 (td, *J =* 7.7, 1.7 Hz, 1H), 7.93 - 7.89 (m, 3H), 7.75 (t, *J =* 8.0 Hz, 1H), 7.66 (d, *J =* 8.7 Hz, 1H), 7.62 (d, *J =* 2.3 Hz, 1H), 7.55 - 7.49 (m, 1H), 6.82 (d, *J =* 1.1 Hz, 1H), 4.95 (d, *J =* 6.4 Hz, 2H), 4.45 (t, *J =* 6.4 Hz, 1H), 2.31 (s, 3H).

Compound **203:** 37.5 mg, ESI[M+H]⁺ = 556.7.

¹H NMR (400 MHz, DMSO-d6) δ 8.56 (d, *J =* 4.1 Hz, 1H), 8.08 - 8.02 (m, 2H), 7.97 (td, *J =* 7.7, 1.7 Hz, 1H), 7.91 (dd, *J =* 8.7, 2.3 Hz, 1H), 7.88 (d, *J =* 7.9 Hz, 1H), 7.82 - 7.76 (m, 2H), 7.66 (d, *J =* 8.7 Hz, 1H), 7.63 (d, *J =* 2.3 Hz, 1H), 7.53 (ddd, *J =* 7.4, 4.8, 1.2 Hz, 1H), 6.81 (d, *J* = 1.0 Hz, 1H), 4.93 - 4.90 (m, 2H), 4.43 (dd, *J =* 7.4, 5.4 Hz, 1H), 2.31 (s, 3H).

Compound **204:** 52.2 mg, ESI[M+H]⁺ = 540.7.

¹H NMR (400 MHz, DMSO-d6) δ 8.56 (d, *J =* 4.1 Hz, 1H), 8.16 - 8.09 (m, 2H), 8.01 - 7.95 (m, 1H), 7.91 (dd, *J =* 8.7, 2.2 Hz, 2H), 7.66 (d, *J=* 8.7 Hz, 1H), 7.64 (d, *J =* 2.3 Hz, 1H), 7.59 - 7.50 (m, 3H), 6.81 (d, *J =* 1.1 Hz, 1H), 4.91 - 4.88 (m, 2H), 4.42 (dd, *J =* 7.4, 5.5 Hz, 1H), 2.31 (s, 3H).

### Example 96 Preparation of Compound 195

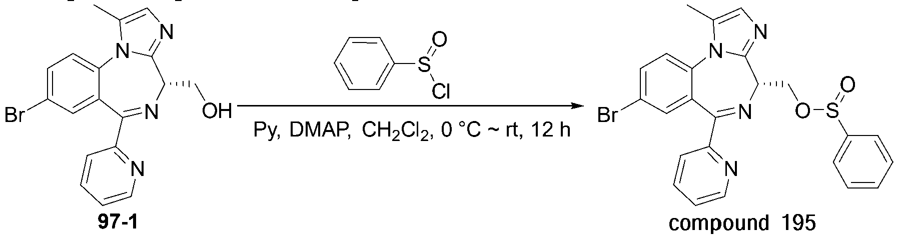

At 0°C, benzenesulfinyl chloride (250 mg, 1.56 mmol) was added to a solution of Compound **97-1** (100 mg, 0.26 mmol), pyridine (123 mg, 1.55 mmol), and DMAP (95.3 mg, 0.78 mmol) in dichloromethane (2 mL). The reaction mixture was allowed to warm to room temperature and stirred for 12 hours. After confirming reaction completion by TLC, ice-water (10 mL) was added to the mixture and filtered. The filtrate was extracted with CH₂Cl₂ (3 × 10 mL). The combined organic phases were washed with brine, dried over anhydrous Na₂SO₄, filtered, and concentrated under reduced pressure to afford the crude product. The crude product was purified by silica gel column chromatography (MeOH/CH₂Cl₂, v/v = 1/100-1/20), TLC (MeOH/CH₂Cl₂, v/v =1/20) momnitoring and the fractions with Rf = 0.3-0.4 were collected to yield Compound **195** (17.2 mg, yield 13.0%). ESI[M + H]⁺ = 506.7.

¹H NMR (400 MHz, DMSO-*d6*) δ 8.57 - 8.56 (m, 1H), 8.15 - 8.08 (m, 1H), 8.03 - 7.95 (m, 1H), 7.93 - 7.90 (m, 1H), 7.83 - 7.79 (m, 2H), 7.71 - 7.62 (m, 5H), 7.57 - 7.49 (m, 1H), 6.82 (d, *J* = 1.1 Hz, 1H), 4.95 - 4.86 (m, 1H), 4.69 - 4.55 (m, 1H), 4.36 - 4.28 (m, 1H), 2.32 (s, 3H).

### Example 97 Preparation of Compound 107, 108, 197-198, 201

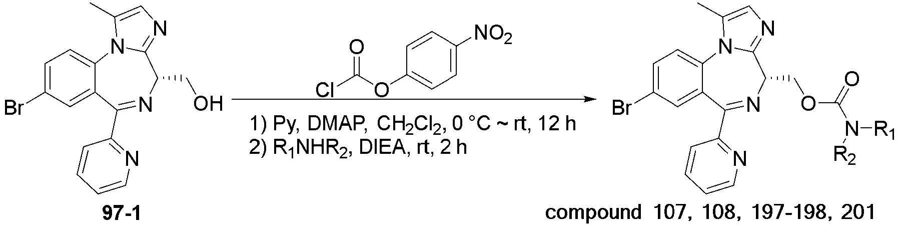

The preparation method of **Compound 107, Compound 108, Compound 197-198, Compound 201** are similar to compound **105** in example 52, using compound **97-1** and the p-Nitrophenyl chloroformate as starting materials.

Compound **107:** 29.4 mg, ESI[M+H]⁺ = 468.2.

¹H NMR (400 MHz, DMSO-d6) δ 8.57 (d, *J =* 4.0 Hz, 1H), 8.07 (d, *J =* 7.9 Hz, 1H), 7.99 - 7.96 (m, 1H), 7.92 (dd, *J =* 8.7, 2.3 Hz, 1H), 7.71 (d, *J =* 8.7 Hz, 1H), 7.66 (d, *J =* 2.1 Hz, 1H), 7.57 - 7.49 (m, 1H), 6.86 (d, *J =* 1.0 Hz, 1H), 4.88 (d, *J =* 6.7 Hz, 2H), 4.34 (t, *J =* 6.6 Hz, 1H), 3.26 - 3.24 (m, 2H), 2.84 (s, 3H), 2.33 (s, 3H), 1.05 (t, *J =* 7.1 Hz, 3H).

Compound **108:** 34.3 mg, ESI[M+H]⁺ = 482.2.

¹H NMR (400 MHz, DMSO-d6) δ 8.57 (d, *J =* 4.1 Hz, 1H), 8.07 (d, *J =* 7.9 Hz, 1H), 7.99 - 7.97 (m, 1H), 7.92 (dd, *J =* 8.7, 2.3 Hz, 1H), 7.71 (d, *J =* 8.7 Hz, 1H), 7.66 (d, *J =* 2.2 Hz, 1H), 7.54 - 7.51 (m, 1H), 6.87 (s, 1H), 4.92 - 4.87 (m, 2H), 4.37 - 4.35 (m, 1H), 4.32 - 4.14 (m, 1H), 2.71 (s, 3H), 2.34 (s, 3H), 1.08 (d, *J =* 6.7 Hz, 6H).

Compound **197:** 37.7 mg, ESI[M+H]⁺ = 482.2.

¹H NMR (400 MHz, DMSO-d6) δ 8.57 (d, *J =* 4.2 Hz, 1H), 8.07 (d, *J =* 7.9 Hz, 1H), 7.97 (t, *J =* 7.1 Hz, 1H), 7.92 (dd, *J* = 8.7, 2.3 Hz, 1H), 7.70 (d, *J =* 8.7 Hz, 1H), 7.65 (s, 1H), 7.54 - 7.51 (m, 1H), 6.85 (d, *J =* 1.0 Hz, 1H), 4.88 (d, *J =* 6.7 Hz, 2H), 4.33 - 4.31 (m, 1H), 3.20 - 3.17 (m, 2H), 2.84 (s, 3H), 2.33 (s, 3H), 1.54 - 1.45 (m, 2H), 0.93 - 0.69 (m, 3H).

Compound **198:** 55.5 mg, ESI[M+H]⁺ = 481.8.

¹H NMR (400 MHz, DMSO *d6*) δ 8.57 (d, *J =* 4.1 Hz, 1H), 8.06 (d, *J* = 7.9 Hz, 1H), 7.97 (td, *J =* 7.7, 1.7 Hz, 1H), 7.92 (dd, *J =* 8.7, 2.3 Hz, 1H), 7.70 (d, *J =* 8.7 Hz, 1H), 7.65 (d, *J =* 2.3 Hz, 1H), 7.55 - 7.49 (m, 1H), 6.86 (s, 1H), 4.93 - 4.83 (m, 2H), 4.42 - 4.20 (m, 1H), 3.24 - 3.23 (m, 4H), 2.33 (s, 3H), 1.06 (t, *J =* 7.0 Hz, 6H).

Compound **201:** 11.9 mg, ESI[M+H]⁺ = 469.8.

¹H NMR (400 MHz, DMSO-d6) δ 8.57 (d, *J =* 4.7 Hz, 1H), 8.07 (d, *J* = 7.9 Hz, 1H), 7.98 (td, *J =* 7.7, 1.7 Hz, 1H), 7.93 (dd, *J =* 8.8, 2.3 Hz, 1H), 7.70 (d, *J =* 8.7 Hz, 1H), 7.67 (d, *J* = 2.3 Hz, 1H), 7.53 (dd, *J =* 6.9, 5.4 Hz, 1H), 6.86 (d, *J =* 1.0 Hz, 1H), 5.03 - 4.86 (m, 2H), 4.40 (dd, *J =* 7.6, 5.6 Hz, 1H), 3.63 (s, 3H), 3.11 (s, 3H), 2.34 (s, 3H).

### Example 98 Preparation of Compound 199

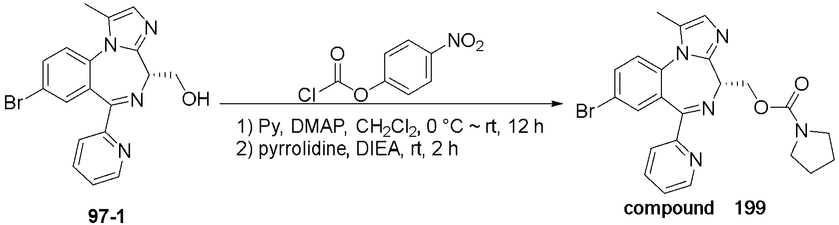

The preparation method of **Compound 199** is similar to compound **105** in example 52, using compound **97-1** and *p*-nitrophenyl chloroformate, which was subsequently reacted with pyrrolidine to afford the target compound.

Compound **199:** 54.6 mg, ESI[M+H]⁺ = 479.8.

¹H NMR (400 MHz, DMSO-*d6*) δ 8.56 (d, *J =* 4.0 Hz, 1H), 8.07 (d, *J =* 7.9 Hz, 1H), 8.00 - 7.94 (m, 1H), 7.91 (dd, *J =* 8.7, 2.3 Hz, 1H), 7.69 (d, *J =* 8.7 Hz, 1H), 7.66 (d, *J =* 2.3 Hz, 1H), 7.51 (ddd, *J* = 7.5, 4.8, 1.1 Hz, 1H), 6.84 (d, *J =* 1.0 Hz, 1H), 4.93 - 4.83 (m, 2H), 4.32 (dd, *J* = 7.4, 5.8 Hz, 1H), 3.30 - 3.28 (m, 4H), 2.33 (s, 3H), 1.85 - 1.74 (m, 4H).

### Example 99 Preparation of Compound 200

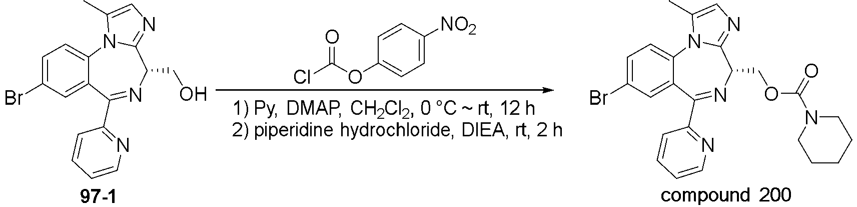

The preparation method of **Compound 200** is similar to compound **105** in example 52, using compound **97-1** and *p*-nitrophenyl chloroformate, which was subsequently reacted with piperidine hydrochloride to afford the target compound.

Compound **200:** 44.8 mg, ESI[M+H]⁺ = 493.8.

¹H NMR (400 MHz, DMSO-*d6*) δ 8.57 (d, *J =* 4.5 Hz, 1H), 8.07 (d, *J* = 7.9 Hz, 1H), 7.97 (td, *J =* 7.8, 1.6 Hz, 1H), 7.92 (dd, *J =* 8.7, 2.3 Hz, 1H), 7.70 (d, *J =* 8.7 Hz, 1H), 7.67 (d, *J =* 2.2 Hz, 1H), 7.56 - 7.47 (m, 1H), 6.86 (s, 1H), 4.88 (d, *J =* 6.6 Hz, 2H), 4.35 (t, *J =* 6.6 Hz, 1H), 3.35 - 3.27 (m, 4H), 2.34 (s, 3H), 1.62 - 1.38 (m, 6H).

### Example 100 Preparation of Compound 205

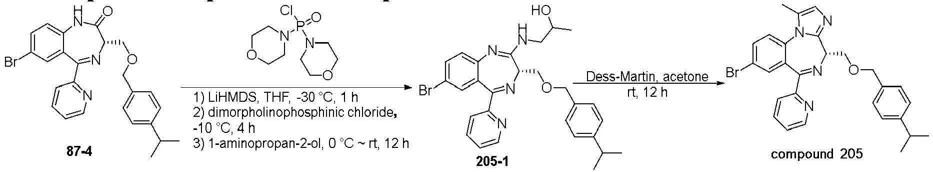

The preparation method of **Compound 205 is** similar to compound **95** in example 42, using compound 8**7-4** as starting materials.

Compound **205:** 266.0 mg, ESI[M+H]⁺ = 514.5.

¹H NMR (400 MHz, DMSO-d6) δ 8.56 (d, *J =* 4.1 Hz, 1H), 8.09 (d, *J =* 7.9 Hz, 1H), 7.97 (td, *J =* 7.8, 1.7 Hz, 1H), 7.90 (dd, *J =* 8.7, 2.3 Hz, 1H), 7.65 (dd, *J =* 6.7, 5.6 Hz, 2H), 7.51 (ddd, *J* = 7.5, 4.8, 1.1 Hz, 1H), 7.32 (d, *J =* 8.1 Hz, 2H), 7.23 (d, *J =* 8.1 Hz, 2H), 6.81 (d, *J =* 1.0 Hz, 1H), 4.65 (s, 2H), 4.42 - 4.33 (m, 2H), 4.23 (dd, *J =* 7.4, 5.4 Hz, 1H), 2.88 (dd, *J =* 13.8, 6.9 Hz, 1H), 2.31 (s, 3H), 1.20 (d, *J =* 6.9 Hz, 6H).

### Example 101 Preparation of Compound 206

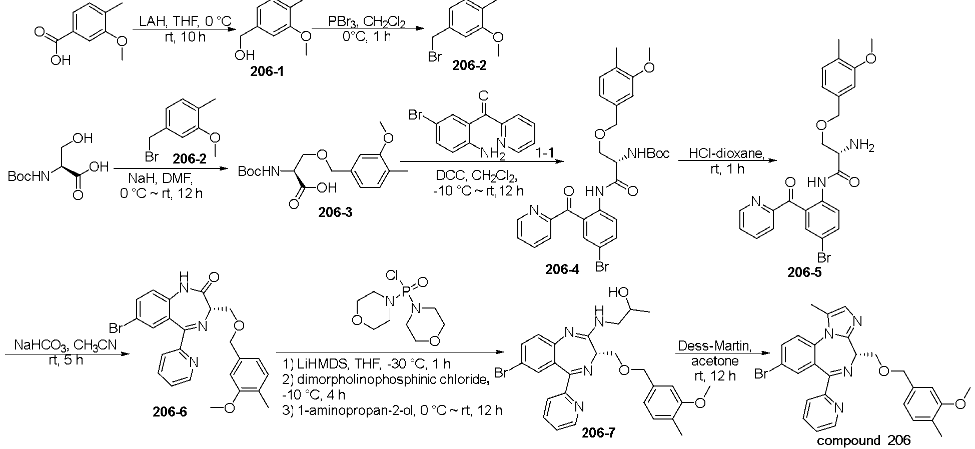

### 1. Preparation of Compound 206-2

At 0°C, lithium aluminum hydride (4.57 g, 120.4 mmol) was added portionwise to the solution of 3-methoxy-4-methylbenzoic acid (10.0 g, 60.2 mmol) in tetrahydrofuran (100 mL). The mixture was then stirred at room temperature for 10 hours. After confirming reaction completion by TLC, Na₂SO₄.10H₂O (20 mL) was added to the mixture and filtered. The filtrate was concentrated under reduced pressure to afford the crude product. The crude product was purified by silica gel column chromatography (ethyl acetate/petroleum ether, v/v = 1/10-1/2), and the fractions with Rf = 0.5-0.6 were collected to yield Compound **206-1** as colorless oil (3.952 g, yield 43.2%). ESI[M + H]+ = 153.1.

At 0°C, phosphorus tribromide (10.546 g, 38.96 mmol) was added dropwise to solution of Compound **206-1** (3.952 g, 25.97 mmol) in dichloromethane (117 mL). The mixture was stirred for 1 hour. After confirming reaction completion by TLC, ice-water (50 mL) was added to the mixture and filtered. The filtrate was extracted with CH₂Cl₂ (3 × 50 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v = 1/100-1/10), TLC (ethyl acetate/petroleum ether, v/v =1/10) momnitoring and the fractions with Rf = 0.4-0.6 were collected to yield Compound **206-2** (4.6 g, yield 30.9%). ESI[M + H]⁺ = 215.1.

### 2. Preparation of Compound 206

The preparation method of **Compound 206** is similar to compound **190** in example 93, using compound **206-2** and **Boc-L-serine** as starting materials to the corresponding carboxylic acid, then the corresponding carboxylic acid was condensed with Compound **1-1,** followed by Boc deprotection using 1M HCl in dioxane and subsequent cyclization to afford Compound **206-6.** The target compound **Compound 206** was finally prepared from Compound **206-6.**

Compound **206:** 252.5 mg, ESI[M+H]⁺ = 516.5.

¹H NMR (400 MHz, DMSO-d6) δ 8.57 (d, *J =* 4.7 Hz, 1H), 8.09 (d, *J =* 7.9 Hz, 1H), 7.96 (td, *J =* 7.8, 1.7 Hz, 1H), 7.90 (dd, *J =* 8.7, 2.3 Hz, 1H), 7.66 (d, *J =* 8.7 Hz, 1H), 7.64 (d, *J =* 2.3 Hz, 1H), 7.51 (dd, *J* = 6.9*,* 5.4 Hz, 1H), 7.10 (d, *J =* 7.5 Hz, 1H), 6.98 (s, 1H), 6.87 (d, *J* = 7.6 Hz, 1H), 6.81 (s, 1H), 4.72 - 4.61 (m, 2H), 4.42 (dd, *J =* 9.9, 5.4 Hz, 1H), 4.36 (t, *J =* 8.7 Hz, 1H), 4.23 (dd, *J =* 7.4, 5.4 Hz, 1H), 3.78 (s, 3H), 2.32 (s, 3H), 2.14 (s, 3H).

### Example 102 Preparation of Compound 207

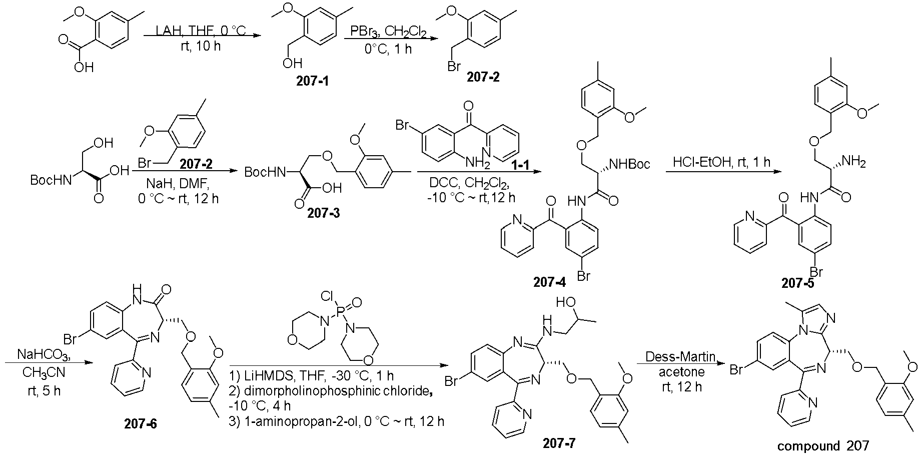

The preparation method of **Compound 207 is** similar to compound **206** in example 101, using **4-Methyl-2-methoxybenzoic acid** as starting materials

Compound **207:** 38.3 mg, ESI[M+H]⁺ = 516.5.

¹H NMR (400 MHz, DMSO-d6) δ 8.56 (d, *J =* 4.0 Hz, 1H), 8.09 (d, *J* = 7.9 Hz, 1H), 7.97 (td, *J =* 7.8, 1.7 Hz, 1H), 7.91 (dd, *J =* 8.7, 2.3 Hz, 1H), 7.67 (d, *J =* 8.7 Hz, 1H), 7.64 (d, *J =* 2.3 Hz, 1H), 7.56 - 7.48 (m, 1H), 7.28 (d, *J =* 7.5 Hz, 1H), 6.82 (s, 2H), 6.76 (d, *J =* 7.7 Hz, 1H), 4.73 - 4.55 (m, 2H), 4.42 (dd, *J =* 9.9, 5.2 Hz, 1H), 4.36 (dd, *J =* 9.9, 7.6 Hz, 1H), 4.22 (dd, *J =* 7.5, 5.2 Hz, 1H), 3.77 (s, 3H), 2.32 (s, 3H), 2.32 (s, 3H).

### Example 103 Preparation of Compound 208

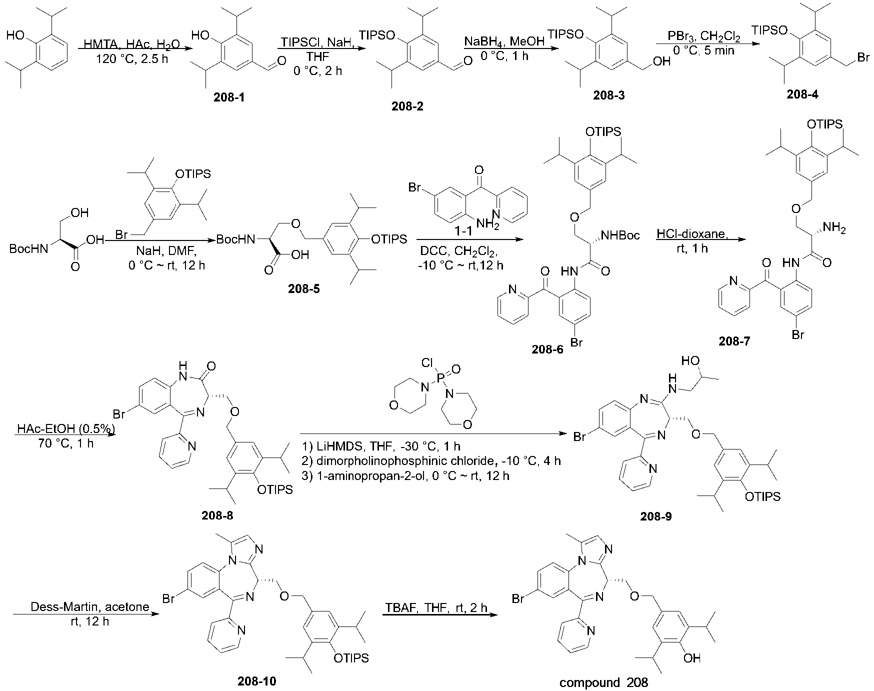

### 1. Preparation of Compound 206

Hexamethylenetetramine (14.0 g, 99.9 mmol) was added to a solution of propofol (9.2 g, 51.6 mmol) in AcOH/H₂O (41.5 mL/8.5 mL). The mixture was stirred at 120°C for 2.5 hours. After confirming reaction completion by TLC, the mixture was cooled to room temperature. Water (10 mL) was then added, and the mixture was further cooled to 0°C and maintained at this temperature for 1 hour. The precipitate was collected by filtration, washed with cold water, and dried to yeild Compound **208-1** as off-yellow solid (5.293 g, yield 49.7%)_{∘} ESI[M + H]⁺ = 207.1.

At 0°C, sodium hydride (1.13 g, 60% dispersion in mineral oil, 28.2 mmol) was added portionwise to solution of Compound **208-1** (5.293 g, 25.7 mmol) in anhydrous tetrahydrofuran (55 mL). After stirring for 30 minutes, triisopropylsilyl chloride (5.917 g, 30.7 mmol) was added to the mixture. The resulting mixture was stirred at this temperature for 2 hours. After confirming reaction completion by TLC, ice-water (30 mL) was added to the mixture and filtered. The filtrate was extracted with ethyl acetate (3 × 50 mL). The combined organic phases were washed with brine, dried over anhydrous Na₂SO₄, filtered, and concentrated under reduced pressure to afford the crude product. The crude product was purified by silica gel column chromatography (petroleum ether), TLC (ethyl acetate/petroleum ether, v/v =1/50) momnitoring and the fractions with Rf = 0.5-0.6 were collected to yield Compound **208-2** as white solid (7.28 g, yield 78.2%). ESI[M + H]⁺ = 363.3.

At 0°C, sodium borohydride (755.8 mg, 20.0 mmol) was added portionwise to a solution of Compound **208-2** (3.6 g, 9.93 mmol) in methanol (104 mL). The reaction mixture was stirred at this temperature for 1 hour. After confirming reaction completion by TLC, ice-water (200 mL) was added to the mixture and filtered. The filtrate was extracted with ethyl acetate (3 × 10 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/100-1/10), TLC (ethyl acetate/petroleum ether, v/v =1/10) momnitoring and the fractions with Rf = 0.3-0.5 were collected to yield Compound **208-3** as colorless oil (3.49 g, yield 96.4%). ESI[M + H]⁺ = 365.3.

At 0°C, phosphorus tribromide (4.596 g, 17.0 mmol) was added dropwise to a solution of Compound **208-3** (3.09 g, 8.47 mmol) in dichloromethane (90 mL). The mixture was stirred for 5 minutes. After confirming reaction completion by TLC, ice-water (100 mL) was added to the mixture and filtered. The filtrate was extracted with CH₂Cl₂ (3 × 50 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/100-1/10), TLC (ethyl acetate/petroleum ether, v/v =1/10) momnitoring and the fractions with Rf = 0.3-0.5 were collected to yield Compound **208-4** as colorless oil (3.3 g, yield 91.1%). ESI[M + H]⁺ = 427.1.

### 2. Preparation of Compound 208-10

The preparation method of **Compound 208-10** is similar to compound **190** in example 93, using compound **208-4** and *Boc-L-serine* to yield the corresponding carboxylic acid, which was subsequently reacted with piperidine hydrochloride to afford the target compound. This carboxylic acid was then condensed with Compound **1-1,** followed by Boc deprotection using 1M HCl in dioxane and subsequent cyclization to afford Compound **208-8.** The compound **208-10** was prepared from Compound **208-8.**

### 3. Preparation of Compound 208

At 0°C, tetrabutylammonium fluoride (0.42 mL, 1 M in THF, 0.42 mmol) was added to the solution of Compound **208-8** (307 mg, 0.42 mmol) in tetrahydrofuran (3.5 mL). The mixture was stirred at room temperature for 2 hours. After confirming reaction completion by TLC, ice-water (10 mL) was added to the mixture and filtered. The filtrate was extracted with ethyl acetate (3 × 10 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/10-1/1), TLC (ethyl acetate/petroleum ether, v/v =1/1) momnitoring and the fractions with Rf = 0.3-0.4 were collected to yield Compound **208** as colorless oil (102.2 mg, yield 42.3%). ESI[M + H]⁺ = 572.5.

¹H NMR (400 MHz, DMSO-d6) δ 8.57 (d, *J =* 4.1 Hz, 1H), 8.10 (d, *J =* 7.9 Hz, 1H), 7.99 (s, 1H), 7.94 (td, *J =* 7.8, 1.8 Hz, 1H), 7.90 (dd, *J =* 8.7, 2.3 Hz, 1H), 7.65 (d, *J =* 8.7 Hz, 1H), 7.63 (d, *J =* 2.3 Hz, 1H), 7.51 (ddd, *J =* 7.5, 4.8, 1.1 Hz, 1H), 7.00 (s, 2H), 6.81 (d, *J =* 1.0 Hz, 1H), 4.57 (s, 2H), 4.42 - 4.32 (m, 2H), 4.21 (dd, *J =* 7.4, 5.4 Hz, 1H), 3.31 - 3.24 (m, 2H), 2.32 (s, 3H), 1.16 (d, *J =* 3.0 Hz, 6H), 1.14 (d, *J =* 3.0 Hz, 6H).

### Example 104 Preparation of Compound 209

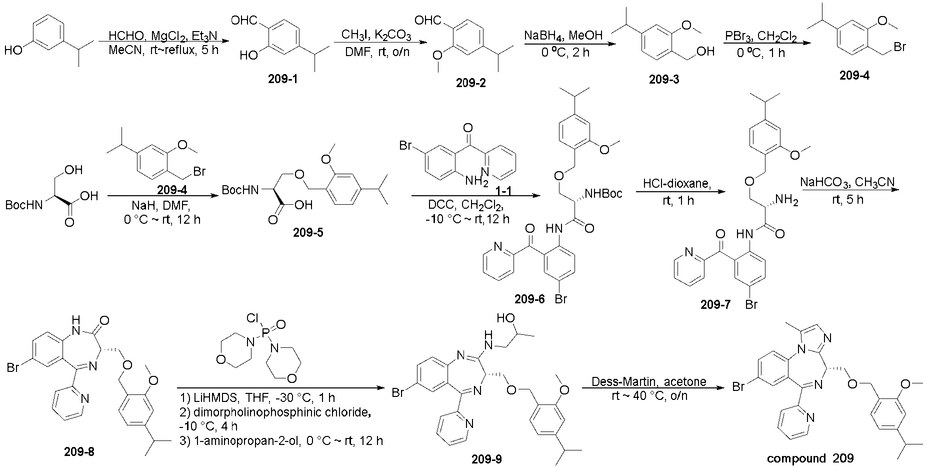

### 1. Preparation of Compound 209-4

At room temperature, paraformaldehyde (5.95 g, 198.3 mmol), magnesium chloride (12.6 g, 132.2 mmol) and triethylamine (13.4 g, 132.2 mmol) were added to the solution of 3-isopropylphenol (9.0 g, 66.1 mmol) in acetonitrile (130 mL). The mixture was refluxed for 5 hours. After confirming reaction completion by TLC, brine (100 mL) was added to the mixture and filtered. the filtrate was extracted with ethyl acetate (3 × 100 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/10-1/5), TLC (ethyl acetate/petroleum ether, v/v =1/5) momnitoring and the fractions with Rf = 0.5-0.6 were collected to yield Compound **209-1** as colorless oil (4.926 g, yield 45.4%). ESI[M + H]⁺ = 165.1.

At room temperature, potassium carbonate (12.4 g, 89.6 mmol) and iodomethane (8.5 g, 59.6 mmol) were added to solution of Compound **209-1** (4.926 g, 30.0 mmol) in DMF (50 mL). The reaction mixture was stirred at room temperature overnight. After confirming reaction completion by TLC, brine (50 mL) was added to the mixture and filtered, the filtrate was extracted with ethyl acetate (3 × 50 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/20-1/10), TLC (ethyl acetate/petroleum ether, v/v =1/10) momnitoring and the fractions with Rf = 0.5-0.6 were collected to yield Compound **209-2** as colorless oil (5.0 g, yield 93.6%). ESI[M + H]⁺ = 179.1.

At 0°C, sodium borohydride (1.6 g, 42.1 mmol) was added to the solution of Compound **209-2** (5.0 g, 28.1 mmol) in methanol (40 mL). The mixture was stirred at 0°C for 2 hours. After confirming reaction completion by TLC, brine (50 mL) was added to the mixture and filtered. The filtrate was extracted with ethyl acetate (3 × 30 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/10-1/5), TLC (ethyl acetate/petroleum ether, v/v =1/5) momnitoring and the fractions with Rf = 0.5-0.6 were collected to yield Compound **209-3** as colorless oil (4.6 g, yield90.9%). ESI[M + H]⁺ = 181.1.

At 0°C, phosphorus tribromide (10.37 g, 38.3 mmol) was added dropwise to solution of Compound **209-3** (4.6 g, 25.5 mmol) in dichloromethane (100 mL). The mixture was stirred for 1 hour. After confirming reaction completion by TLC, ice-water (50 mL) was added to the mixture and filtered, the filtrate was extracted with CH₂Cl₂ (3 × 50 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/100-1/10), TLC (ethyl acetate/petroleum ether, v/v =1/10) momnitoring and the fractions with Rf = 0.4-0.6 were collected to yield Compound **209-4** as colorless oil (3.7 g, yield 59.8%). ESI[M + H]⁺ = 243.0.

### 2. Preparation of Compound 209

The preparation method of Compound **209** is similar to compound **190** in example 93, using Boc-L-serine and Compound **209-4** as starting materials to the corresponding carboxylic acid. The carboxylic acid was then condensed with Compound **1-1,** followed by Boc deprotection using 1M HCl in dioxane and subsequent cyclization to afford Compound **209-8.** The compound **209** was prepared from Compound **209-8.**

Compound **209:** 51 mg, ESI[M+H]⁺ = 545.1.

¹H NMR (400 MHz, MeOD) δ 8.53 (d, J = 4.8 Hz, 1H), 8.15 (d, J = 7.9 Hz, 1H), 8.03 - 7.96 (m, 1H), 7.89 (dd, J = 8.7, 2.3 Hz, 1H), 7.61 (t, J = 9.2 Hz, 1H), 7.57 - 7.50 (m, 2H), 7.25 (d, J = 2.0 Hz, 1H), 7.21 (d, J = 8.3 Hz, 1H), 6.86 (t, J = 11.3 Hz, 2H), 4.68 (s, 2H), 4.50 (d, J = 6.7 Hz, 2H), 4.22 (t, *J =* 6.7 Hz, 1H), 3.83 (s, 3H), 3.34 - 3.27 (m, 1H), 2.42 (s, 3H), 1.24 - 1.10 (m, 6H).

### Example 105 Preparation of Compound 210

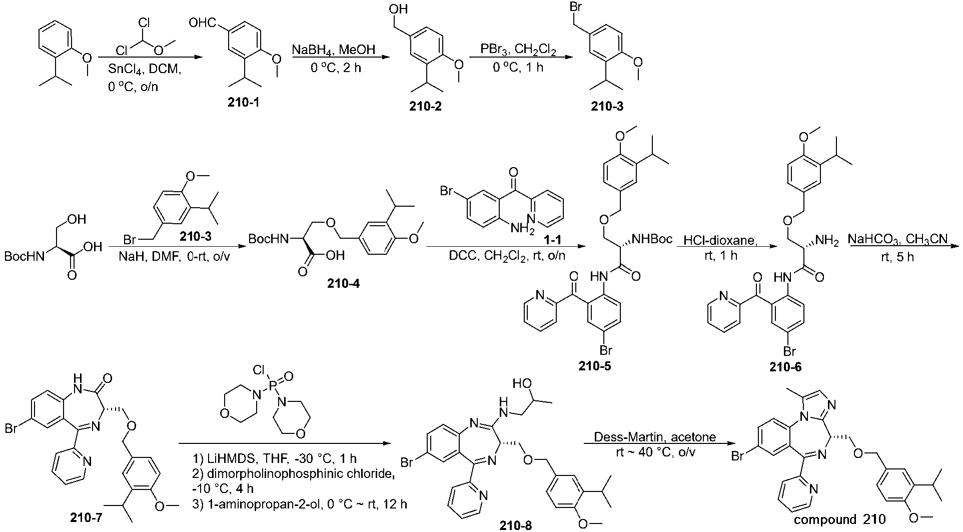

### 1. Preparation of Compound 210-3

At 0°C, 1,1-dichlorodimethyl ether (5.95 g, 198.3 mmol) and tin(IV) chloride (12.6 g, 132.2 mmol) were added to the solution of 2-isopropylanisole (9.0 g, 60.0 mmol) in dichloromethane (100 mL). The mixture was stirred at room temperature overnight. After confirming reaction completion by TLC, brine (100 mL) was added to the mixture and filtered. The filtrate was extracted with ethyl acetate (3 × 50 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/20-1/10), TLC (ethyl acetate/petroleum ether, v/v =1/10) momnitoring and the fractions with Rf = 0.5-0.6 were collected to yield Compound **210-1** as colorless oil (10.61 g, yield 99.3%). ESI[M + H]⁺ = 179.1.

At 0°C, sodium borohydride (3.4 g, 89.4 mmol) was added to the solution of Compound **210-1** (10.61 g, 59.61 mmol) in methanol (60 mL). The mixture was stirred at 0°C for 2 hours. After confirming reaction completion by TLC, brine (50 mL) was added to the mixture and filtered. The filtrate was extracted with ethyl acetate (3 × 30 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/10-1/5), TLC (ethyl acetate/petroleum ether, v/v =1/5) momnitoring and the fractions with Rf = 0.5-0.6 were collected to yield Compound **210-2** as colorless oil (10.0 g, yield 93.2%). ESI[M + H]⁺ = 181.1.

At 0°C, phosphorus tribromide (23.0 g, 83.4 mmol) was added dropwise to the solution of Compound **210-2** (10.0 g, 55.6 mmol) in dichloromethane (100 mL). The mixture was stirred for 1 hour. After confirming reaction completion by TLC, brine (100 mL) was added to the mixture and filtered. The filtrate was extracted with CH₂Cl₂ (3 × 50 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether, v/v =1/100-1/10), TLC (ethyl acetate/petroleum ether, v/v =1/10) momnitoring and the fractions with Rf = 0.4-0.6 were collected to yield Compound **210-3** as colorless oil (5.7 g, yield42.3%). ESI[M + H]⁺ = 243.0.

### 2. Preparation of Compound 210

The preparation method of Compound **210** is similar to compound **190** in example 93, using Boc-L-serine and Compound **210-3** as starting materials to the corresponding carboxylic acid. This carboxylic acid was then condensed with Compound **1-1,** followed by Boc deprotection using 1M HCl in dioxane and subsequent cyclization to afford Compound **210-7.** The compound **210** was prepared from Compound **210-7.**

Compound **210:** 367 mg, ESI[M+H]⁺ = 545.2.

¹H NMR (400 MHz, MeOD) δ 8.53 (d, *J =* 4.2 Hz, 1H), 8.15 (d, *J =* 7.9 Hz, 1H), 7.99 (td, *J* = 7.8, 1.7 Hz, 1H), 7.89 (dd, *J =* 8.7, 2.3 Hz, 1H), 7.60 (d, *J =* 8.7 Hz, 1H), 7.56 - 7.45 (m, 2H), 7.25 (d, *J =* 2.0 Hz, 1H), 7.20 (dd, *J =* 8.3, 2.1 Hz, 1H), 6.87 (dd, *J =* 4.6, 3.6 Hz, 2H), 4.68 (s, 2H), 4.50 (d, *J =* 6.7 Hz, 2H), 4.22 (t, *J=* 6.7 Hz, 1H), 3.83 (s, 3H), 3.32 - 3.25 (m, 1H), 2.41 (d, *J* = 0.7 Hz, 3H), 1.19 (dd, *J =* 6.8, 6.0 Hz, 6H).

### Example 106 Preparation of Compound 107-1 - 107-6

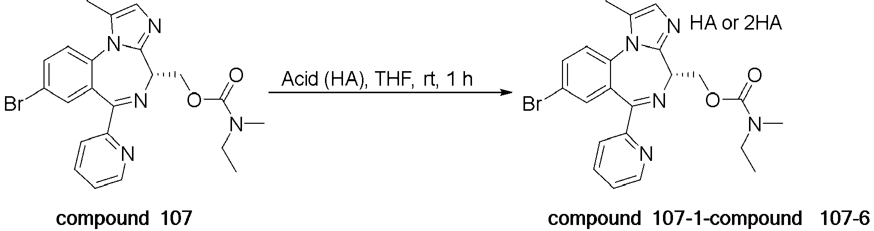

### General Procedure:

The corresponding acid HA was dissolved in tetrahydrofuran and added dropwise to the solution of Compound **107** (50 mg, 0.107 mmol) in tetrahydrofuran (2.5 mL) at room temperature. After stirring for 1 hour, the mixture was filtered. The filter cake was dried under reduced pressure to yield the corresponding **compounds 107-1 to 107-6.**

Compound **107-1:** 36.2 mg, ESI[M+H]⁺ = 467.5.

¹H NMR (400 MHz, DMSO *d6*) δ 8.60 (d, *J =* 4.1 Hz, 1H), 8.10 (d, *J =* 7.9 Hz, 1H), 8.04 - 7.97 (m, 2H), 7.84 (d, *J =* 8.7 Hz, 1H), 7.77 (d, *J =* 2.1 Hz, 1H), 7.56 (dd, *J =* 6.3, 4.8 Hz, 1H), 7.35 (s, 1H), 4.95 - 4.84 (m, 2H), 4.64 - 4.60 (m, 1H), 3.27 - 3.25 (m, 2H), 2.85 - 2.83 (m, 3H), 2.40 (s, 3H), 1.05 - 1.03 (m, 3H).

Compound **107-2:** 29.3 mg, ESI[M+H]⁺ = 467.5.

¹H NMR (400 MHz, DMSO *d6*) δ 8.60 (d, *J =* 4.7 Hz, 1H), 8.10 (d, *J =* 7.8 Hz, 1H), 8.02 - 7.97 (m, 2H), 7.82 (d, *J =* 8.6 Hz, 1H), 7.75 (s, 1H), 7.60 - 7.53 (m, 1H), 7.25 (s, 1H), 4.87 (dd, *J* = 13.0, 9.0 Hz, 2H), 4.59 - 4.55 (m, 1H), 3.29 - 3.25 (m, 2H), 2.85 - 2.83 (m, 3H), 2.39 (s, 3H), 1.07 - 1.01 (m, 3H).

Compound **107-3:** 37.6 mg, ESI[M+H]⁺ = 467.5.

¹H NMR (400 MHz, DMSO *d6)* δ 8.60 (d, *J =* 4.1 Hz, 1H), 8.10 (d, *J =* 7.9 Hz, 1H), 8.05 - 7.98 (m, 2H), 7.86 (d, *J =* 8.7 Hz, 1H), 7.79 (d, *J =* 2.1 Hz, 1H), 7.61 - 7.54 (m, 1H), 7.41 (s, 1H), 4.90 (dd, *J =* 11.2, 7.0 Hz, 2H), 4.68 - 4.65 (m, 1H), 3.28 - 3.26 (m, 2H), 2.86 - 2.83 (m, 3H), 2.41 (s, 3H), 2.36 (s, 6H), 1.06 - 1.02 (m, 3H).

Compound **107-4:** 25.1 mg, ESI[M+H]⁺ = 467.5.

¹H NMR (400 MHz, DMSO *d6*) δ 8.59 (d, *J =* 4.2 Hz, 1H), 8.09 (d, *J =* 7.9 Hz, 1H), 8.03 - 7.95 (m, 2H), 7.83 (d, *J* = 8.7 Hz, 1H), 7.76 (d, *J =* 2.1 Hz, 1H), 7.64 - 7.59 (m, 2H), 7.58 - 7.52 (m, 1H), 7.35 - 7.29 (m, 4H), 4.92 - 4.84 (m, 2H), 4.63 (d, *J =* 6.6 Hz, 1H), 3.27 - 3.23 (m, 2H), 2.85 - 2.81 (m, 3H), 2.39 (s, 3H), 1.06 - 1.02 (m, 3H).

Compound **107-5:** 50.7 mg, ESI[M+H]⁺ = 467.5.

¹H NMR (400 MHz, DMSO *d6*) δ 8.63 (d, *J =* 4.2 Hz, 1H), 8.12 (d, *J =* 7.9 Hz, 1H), 8.04 (ddd, *J* = 9.5, 8.3, 2.0 Hz, 2H), 7.91 (d, *J =* 8.8 Hz, 1H), 7.82 (d, *J =* 2.2 Hz, 1H), 7.63 - 7.56 (m, 1H), 7.52 (s, 1H), 4.94 - 4.85 (m, 2H), 4.73 (t, *J =* 6.6 Hz, 1H), 3.30 - 3.28 (m, 2H), 2.85 (d, *J =* 7.0 Hz, 3H), 2.44 (s, 3H), 1.08 - 1.04 (m, 3H).

Compound **107-6:** 59.2 mg, ESI[M+H]⁺ = 467.5.

¹H NMR (400 MHz, DMSO-*d6*) δ 8.88 (d, *J =* 8.9 Hz, 2H), 8.61 (d, *J =* 4.1 Hz, 1H), 8.10 (d, *J =* 7.9 Hz, 1H), 8.07 - 7.98 (m, 2H), 7.95 (dd, *J =* 7.0, 1.0 Hz, 2H), 7.87 (d, *J* = 8.8 Hz, 1H), 7.80 (d, *J* = 2.2 Hz, 1H), 7.58 (dd, *J =* 6.3, 4.8 Hz, 1H), 7.44 - 7.40 (m, 3H), 4.91 - 4.87 (m, 2H), 4.71 - 4.67 (m, 1H), 3.29 - 3.25 (m, 2H), 2.87 - 2.83 (m, 3H), 2.41 (s, 3H), 1.07 - 1.02 (m, 3H).

### Example 107 Preparation of Compound 199-1 - 199-4

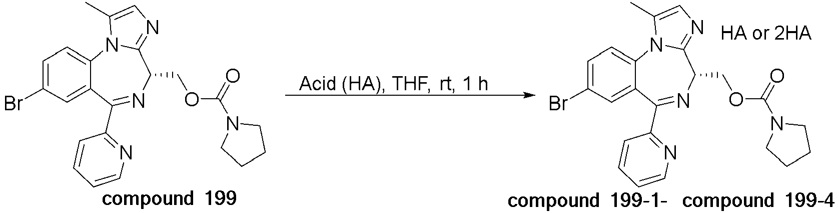

The preparation method of **Compound 199-1** - **199-4** are similar to Compound**107-1 -107-6** in example 101, using Compound **199** as starting materials

Compound **199-1:** 23.2 mg, ESI[M+H]⁺ = 479.5.

¹H NMR (400 MHz, DMSO-d6) δ 8.60 (d, *J =* 4.6 Hz, 1H), 8.10 (d, *J =* 7.9 Hz, 1H), 8.03 - 7.99 (m, 2H), 7.85 (d, *J =* 8.8 Hz, 1H), 7.79 (d, *J =* 1.8 Hz, 1H), 7.60 - 7.53 (m, 1H), 7.39 (s, 1H), 4.89 - 4.84 (m, 2H), 4.66 - 4.63 (m, 1H), 3.36 - 3.21 (m, 4H), 2.40 (s, 3H), 1.82 - 1.78 (m, 4H).

Compound **199-2:** 33.2 mg, ESI[M+H]⁺ = 479.5.

¹H NMR (400 MHz, DMSO-*d6*) δ 8.61 (d, *J =* 4.1 Hz, 1H), 8.11 (d, *J =* 7.9 Hz, 1H), 8.05 - 7.99 (m, 2H), 7.88 (d, *J =* 8.7 Hz, 1H), 7.81 (d, *J =* 2.3 Hz, 1H), 7.59 - 7.56 (m, 1H), 7.47 (s, 1H), 4.93 - 4.84 (m, 2H), 4.69 (t, *J =* 6.6 Hz, 1H), 3.30 - 3.26 (m, 4H), 2.42 (s, 3H), 2.36 (s, 6H), 1.82 - 1.79 (m, 4H).

Compound **199-3:** 61.8 mg, ESI[M+H]⁺ = 479.5.

¹H NMR (400 MHz, DMSO-*d6*) δ 8.62 (d, *J =* 4.1 Hz, 1H), 8.12 (d, *J =* 7.9 Hz, 1H), 8.04 (ddd, *J* = 9.5, 8.3, 2.0 Hz, 2H), 7.91 (d, *J =* 8.8 Hz, 1H), 7.83 (d, *J =* 2.3 Hz, 1H), 7.63 - 7.57 (m, 1H), 7.55 (s, 1H), 4.94 - 4.85 (m, 2H), 4.74 (t, *J =* 6.6 Hz, 1H), 3.37 - 3.23 (m, 4H), 2.43 (s, 3H), 1.87 - 1.75 (m, 4H).

Compound **199-4:** 227.7 mg, ESI[M+H]⁺ = 479.5.

¹H NMR (400 MHz, DMSO-*d6*) δ 8.88 (d, *J =* 8.7 Hz, 2H), 8.61 (d, *J* = 4.8 Hz, 1H), 8.11 (d, *J =* 7.8 Hz, 1H), 8.05 - 8.00 (m, 2H), 7.95 (d, *J =* 7.0 Hz, 2H), 7.87 (d, *J =* 8.5 Hz, 1H), 7.81 (s, 1H), 7.61 - 7.54 (m, 1H), 7.46 - 7.40 (m, 3H), 4.96 - 4.81 (m, 2H), 4.69 (s, 1H), 3.37 - 3.15 (m, 4H), 2.41 (s, 3H), 1.82 - 1.79 (m, 4H).

### Example 108 Preparation of Compound 222 - 223

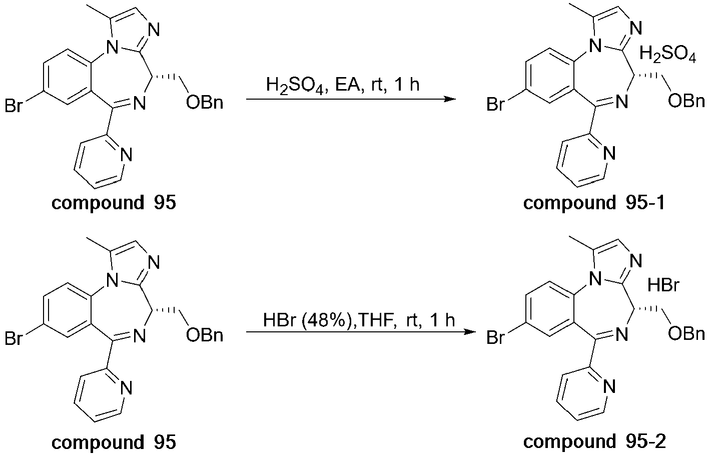

The preparation method of **Compound 95-1** - **95-2** are similar to Compound **107-1 -107-6** in example 106, using Compound **95** as starting materials.

Compound **95-1:** 120.5 mg, ESI[M+H]⁺ = 472.5.

¹H NMR (400 MHz, DMSO-*d6*) δ 8.63 (d, *J =* 4.3 Hz, 1H), 8.11 (d, *J =* 7.9 Hz, 1H), 8.05 (ddd, *J =* 9.4, 8.4, 1.9 Hz, 2H), 7.92 (d, *J =* 8.8 Hz, 1H), 7.82 (d, *J =* 2.2 Hz, 1H), 7.68 (s, 1H), 7.63 - 7.58 (m, 1H), 7.44 - 7.34 (m, 4H), 7.33 - 7.29 (m, 1H), 4.82 - 4.67 (m, 3H), 4.52 (dd, *J =* 10.0, 7.9 Hz, 1H), 4.36 - 4.30 (m, 1H), 2.43 (s, 3H).

Compound **95-2:** 97.6 mg, ESI[M+H]⁺ = 472.5.

¹H NMR (400 MHz, DMSO-*d6*) δ 8.62 (d, *J =* 4.8 Hz, 1H), 8.11 (d, *J =* 7.9 Hz, 1H), 8.08 - 8.00 (m, 2H), 7.92 (d, *J* = 8.8 Hz, 1H), 7.81 (d, *J =* 2.3 Hz, 1H), 7.67 (s, 1H), 7.60 (ddd, *J* = 7.4, 4.8, 1.1 Hz, 1H), 7.44 - 7.34 (m, 4H), 7.35 - 7.28 (m, 1H), 4.80 - 4.71 (m, 3H), 4.52 (dd, *J =* 10.0, 7.8 Hz, 1H), 4.37 - 4.31 (m, 1H), 2.43 (s, 3H).

### Example 109 Preparation of Compound 105-1 - 105-2

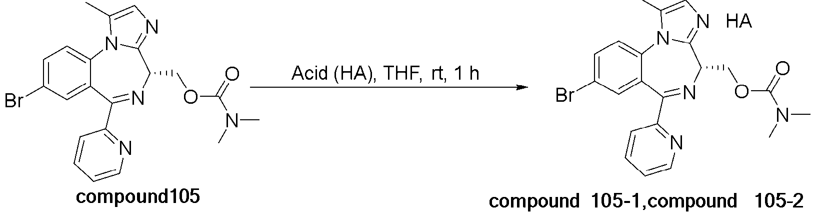

The preparation method of **Compound 105-1** - **105-2** are similar to Compound **107-1 -107-6** in example 106, using Compound **105** as starting materials.

Compound **105-1:** 92.9 mg, ESI[M+H]⁺ = 453.5.

¹H NMR (400 MHz, DMSO-*d6*) δ 8.63 (d, *J =* 4.2 Hz, 1H), 8.12 (d, *J =* 7.9 Hz, 1H), 8.09 - 8.00 (m, 2H), 7.92 (d, *J =* 8.8 Hz, 1H), 7.84 (d, *J =* 2.3 Hz, 1H), 7.66 - 7.55 (m, 2H), 4.94 - 4.81 (m, 2H), 4.77 (t, *J =* 6.7 Hz, 1H), 2.87 (s, 3H), 2.85 (s, 3H), 2.44 (s, 3H).

Compound **105-2:** 33.4 mg, ESI[M+H]⁺ = 453.5.

¹H NMR (400 MHz, DMSO-*d6*) δ 8.59 (s, 1H), 8.10 (d, *J =* 7.8 Hz, 1H), 8.02 - 7.99 (m, 2H), 7.89 - 7.73 (m, 2H), 7.57 (s, 1H), 7.39 - 7.24 (m, 1H), 4.88 - 4.84 (m, 1H), 4.63 - 4.55 (m, 1H), 4.31 - 4.29 (m, 1H), 2.86 (s, 6H), 2.39 (d, *J =* 4.7 Hz, 3H).

### Example 110 Preparation of Compound 212 and Compound 213

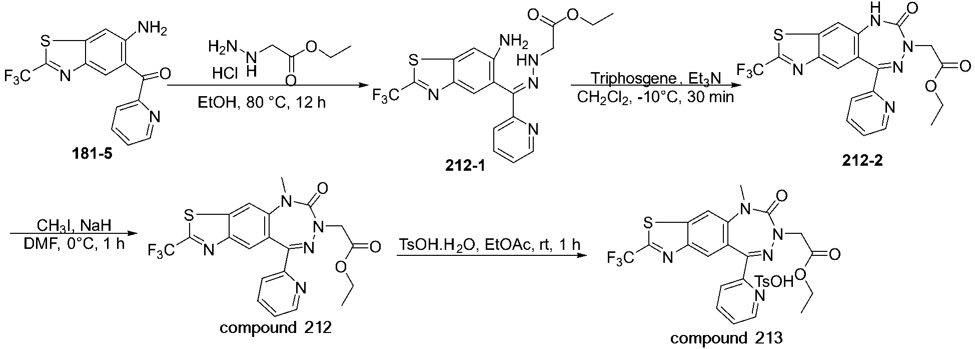

### Preparation of Compound 212-1

At room temperature, ethyl hydrazinoacetate hydrochloride (4.18 g, 27.0 mmol) was added to solution of Compound **181-5** (3.392 g, 10.5 mmol) in anhydrous ethanol (80 mL). The mixture was stirred at 80°C for 12 hours. After confirming reaction completion by TLC, the mixture was concentrated under reduced pressure. The crude product was purified by silica gel column chromatography (methanol/dichloromethane (v/v) = 1/100-1/10), TLC (ethyl acetate/petroleum ether, v/v =1/10) momnitoring and the fractions with Rf = 0.3-0.4 were collected to yield Compound **212-1** (3.523 g, yield 79.3%). ESI[M + H]⁺ = 424.1.

### Preparation of Compound 212-2

At -10°C, solution of triphosgene (1.359 g, 4.58 mmol) in dichloromethane (10 mL) was added dropwise to solution of Compound **212-1** (3.523 g, 8.32 mmol) in dichloromethane (80 mL). The mixture was stirred for 30 minutes. After confirming reaction completion by TLC, ice-water was added to the mixture. The pH was adjusted to 7 with saturated aqueous sodium bicarbonate solution, and the mixture was extracted with dichloromethane (3 × 30 mL). The combined organic phases were washed with brine, dried over anhydrous Na₂SO₄, filtered, and concentrated under reduced pressure to afford the crude product. The crude product was purified by silica gel column chromatography (methanol/dichloromethane (v/v) = 1/10-1/1), TLC (ethyl acetate/petroleum ether, v/v =1/1) momnitoring and the fractions with Rf = 0.2-0.4 were collected to yield Compound **212-2** (2.724 g, yield 72.8%). ESI[M + H]⁺ = 450.1.

### Preparation of Compound 212

At 0°C, Sodium hydride (165.0 mg, 60%, 4.125mmol) was added slowly to the solution of **212-2** in dry DMF, and the mixture was stirred for 30 minutes. Iodomethane (585.5 mg, 4.125 mmol) was then added to the mixture, and stirred at 0 °C for 1 hour.

After confirming reaction completion by TLC, ice-water (30 mL) was added to the mixture, and the mixture was extracted with dichloromethane (3 × 30 mL). The combined organic phases were washed with brine, dried over anhydrous Na₂SO₄, filtered, and concentrated under reduced pressure to afford the crude product. The crude product was purified by silica gel column chromatography (methanol/dichloromethane (v/v) = 1/10-1/1), TLC (ethyl acetate/petroleum ether, v/v =1/1) momnitoring and the fractions with Rf = 0.3-0.4 were collected to yield Compound **212** (1.296 g, yield 88.3%). ESI[M + H]⁺ = 464.2.

¹H NMR (400 MHz, DMSO-d6) δ 8.62 (d, *J =* 4.3 Hz, 1H), 8.45 (s, 1H), 8.10 - 8.05 (m, 2H), 8.02 (td, *J =* 7.7*,* 1.7 Hz, 1H), 7.59 - 7.52 (m, 1H), 4.42 (s, 2H), 4.13 (q, *J =* 7.1 Hz, 2H), 3.31 (s, 3H), 1.20 (t, *J =* 7.1 Hz, 3H).

### Preparation of Compound 213

At room temperature, solution of *p*-toluenesulfonic acid monohydrate (176.2 mg, 0.926 mmol) in ethyl acetate (2.5 mL) was added dropwise to the solution of Compound **212** (214.7 mg, 0.463 mmol) in ethyl acetate (5 mL). The mixture was stirred for 1 hour. The mixture was filtered, and the filter cake was dried under reduced pressure to yield the Compound **213** (130.0 mg, yield 44.1%). ESI[M + H]⁺ = 464.2.

¹H NMR (400 MHz, DMSO-d6) δ 8.63 (d, *J =* 4.7 Hz, 1H), 8.45 (s, 1H), 8.12 - 8.00 (m, 3H), 7.66 - 7.57 (m, 1H), 7.50 (d, *J =* 8.1 Hz, 2H), 7.13 (d, *J =* 7.9 Hz, 2H), 4.42 (s, 2H), 4.13 (q, *J* = 7.1 Hz, 2H), 3.31 (s, 3H), 2.31 (s, 3H), 1.20 (t, *J =* 7.1 Hz, 3H).

### Example 111 Preparation of Compound 214

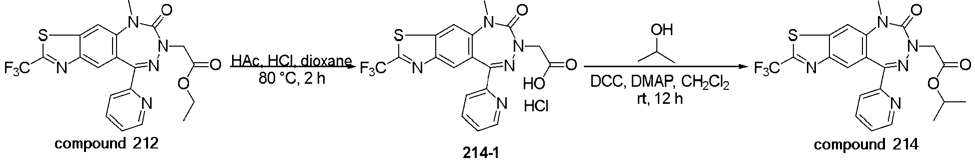

### Preparation of Compound 214-1

At room temperature, acetic acid (15.3 mL) and hydrochloric acid (6 N, 10.1 mL) were added to the solution of Compound **212** (1.296 g, 2.80 mmol) in dioxane (20.2 mL). The mixture was stirred at 80°C for 2 hours. After confirming reaction completion by TLC, the mixture was concentrated under reduced pressure to yield Compound **214-1** as white solid (1.319 g, yield 100%). ESI[M + H]⁺ = 436.1.

### Preparation of Compound 214

Compound **214-1** (500 mg, 1.06 mmol), DCC (436.9 mg, 2.12 mmol) and DMAP (517.5 mg, 4.24 mmol) were dissolved in dichloromethane (10 mL). The mixture was stirred at room temperature for 15 minutes. Isopropanol (95.4 mg, 1.59 mmol) was then added, and stirred at room temperature for 12 hours. After confirming reaction completion by TLC, methyl tert-butyl ether (10 mL) was added, and the mixture was stirred for 5 minutes. The mixture was filtered, and the filtrate was concentrated under reduced pressure to afford the crude product. The crude product was purified by silica gel column chromatography (methanol/dichloromethane (v/v) = 1/10-1/1), TLC (ethyl acetate/petroleum ether, v/v =1/1) momnitoring and the fractions with Rf = 0.3-0.4 were collected to yield Compound **214** as white solid (104.3 mg, yield 20.6%). ESI[M + H]⁺ = 478.2.

¹H NMR (400 MHz, DMSO-d6) δ 8.61 (d, *J =* 4.2 Hz, 1H), 8.45 (s, 1H), 8.10 - 8.05 (m, 2H), 8.02 (td, *J* = 7.7, 1.7 Hz, 1H), 7.59 - 7.54 (m, 1H), 5.01 - 4.91 (m, 1H), 4.38 (s, 2H), 3.31 (s, 3H), 1.19 (d, *J =* 6.2 Hz, 6H).

### Example 112 Preparation of Compound 215-220

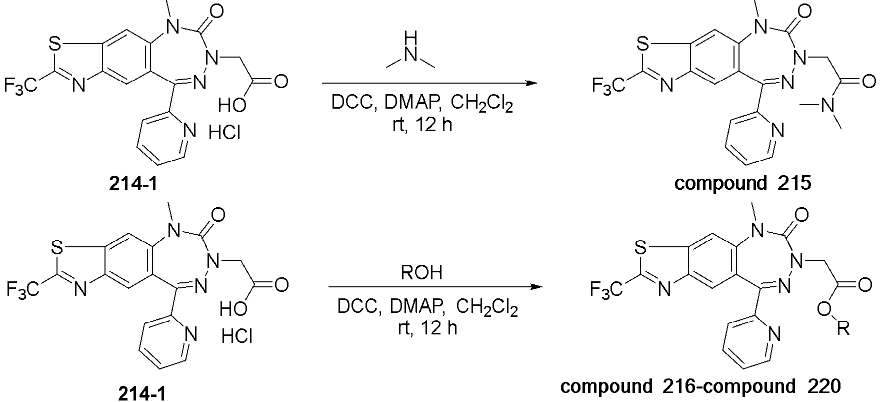

The preparation method of **Compound 215-220** are similar to Compound **214** in example 111, using Compound **214-1** and **the corresponding amine or alcohol** as starting materials.

Compound **215:** 117.8 mg, ESI[M+H]⁺ = 462.9.

¹H NMR (400 MHz, DMSO-*d6*) δ 8.63 - 8.59 (m, 1H), 8.42 (s, 1H), 8.08 - 8.03 (m, 2H), 8.02 - 7.97 (m, 1H), 7.55 (ddd, *J =* 7.3, 4.8, 1.3 Hz, 1H), 4.53 (s, 2H), 3.28 (s, 3H), 2.99 (s, 3H), 2.83 (s, 3H).

Compound 216: 14.1 mg, ESI[M+H]⁺ = 506.2.

¹H NMR (400 MHz, DMSO-*d6*) δ 8.62 (d, *J =* 4.8 Hz, 1H), 8.46 (s, 1H), 8.12 - 8.06 (m, 2H), 8.05 - 7.99 (m, 1H), 7.59 - 7.54 (m, 1H), 4.61 (d, *J =* 7.2 Hz, 2H), 4.44 - 4.42 (m, 4H), 3.32 (s, 3H), 1.64 (s, 3H).

Compound **217:** 10.9 mg, ESI[M+H]⁺ = 518.2.

¹H NMR (400 MHz, DMSO-d6) δ 8.62 (d, *J =* 4.1 Hz, 1H), 8.46 (s, 1H), 8.12 - 8.06 (m, 2H), 8.03 (td, *J* = 7.7, 1.7 Hz, 1H), 7.60 - 7.50 (m, 1H), 6.26 (dd, *J =* 17.4, 10.9 Hz, 1H), 5.36 (dd, *J* = 39.8, 14.2 Hz, 2H), 4.70 (d, *J =* 7.7 Hz, 2H), 4.62 (d, *J =* 7.8 Hz, 2H), 4.53 (s, 2H), 3.32 (s, 3H).

Compound **218:** 34.8 mg, ESI[M+H]⁺ = 491.8.

¹H NMR (400 MHz, DMSO-d6) δ 8.62 (d, *J =* 4.1 Hz, 1H), 8.45 (s, 1H), 8.10 - 8.05 (m, 2H), 8.02 (td, *J =* 7.7, 1.7 Hz, 1H), 7.56 (ddd, *J =* 7.4, 4.8, 1.2 Hz, 1H), 4.82 - 4.77 (m, 1H), 4.40 (s, 2H), 3.31 (s, 3H), 1.59 - 1.45 (m, 2H), 1.15 (d, *J =* 6.3 Hz, 3H), 0.86 - 0.74 (m, 3H).

Compound **219:** 17.9 mg, ESI[M+H]⁺ = 491.8.

¹H NMR (400 MHz, DMSO *d6)* δ 8.63 (d, *J =* 4.1 Hz, 1H), 8.47 (s, 1H), 8.12 - 8.06 (m, 2H), 8.03 (td, *J* = 7.7, 1.7 Hz, 1H), 7.57 (ddd, *J =* 7.4, 4.8, 1.2 Hz, 1H), 5.52 - 5.43 (m, 1H), 4.81 (t, *J =* 7.2 Hz, 2H), 4.51 - 4.47 (m, 4H), 3.31 (s, 3H).

Compound **220:** 10.8 mg, ESI[M+H]⁺ = 519.8.

¹H NMR (400 MHz, DMSO-d6) δ 8.61 (d, *J =* 4.1 Hz, 1H), 8.46 (s, 1H), 8.10 (d, *J =* 7.9 Hz, 1H), 8.06 (s, 1H), 8.05 - 7.99 (m, 1H), 7.59 - 7.53 (m, 1H), 4.57 (d, *J =* 7.6 Hz, 2H), 4.47 - 4.45 (m, 4H), 3.31 (s, 3H), 2.04 (q, *J* = 7.4 Hz, 2H), 0.87 (t, *J =* 7.4 Hz, 3H).

### Example 113 Preparation of Compound 221

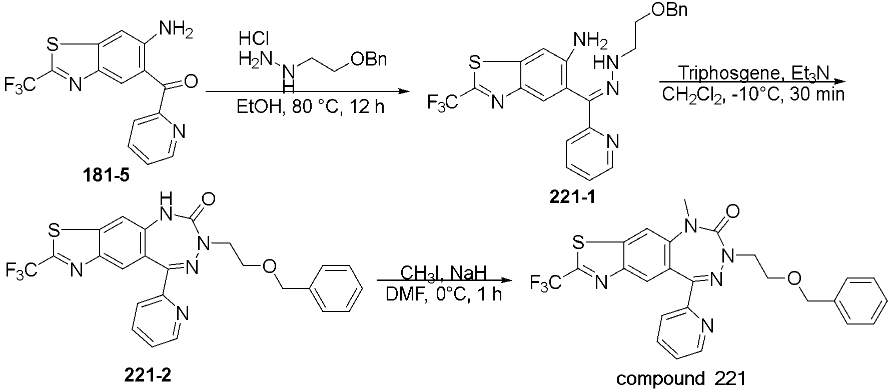

The preparation method of Compound 221 are similar to Compound 212 in example 110, using Compound 181-5 as starting materials.

Compound 221: 27.9 mg, ESI[M+H]⁺ = 511.5_{∘}

¹H NMR (400 MHz, DMSO *d6)* δ 8.61 (d, *J =* 4.1 Hz, 1H), 8.43 (s, 1H), 8.09 (d, *J =* 8.0 Hz, 1H), 8.05 (s, 1H), 7.98 (td, *J =* 7.7, 1.8 Hz, 1H), 7.55 (dd, *J =* 6.3, 4.8 Hz, 1H), 7.19 - 7.07 (m, 3H), 7.03 (d, *J* = 7.1 Hz, 2H), 4.37 (s, 2H), 4.20 - 3.52 (m, 4H), 3.30 (s, 3H).

### Example 114 Preparation of Compound 222 and Compound 235

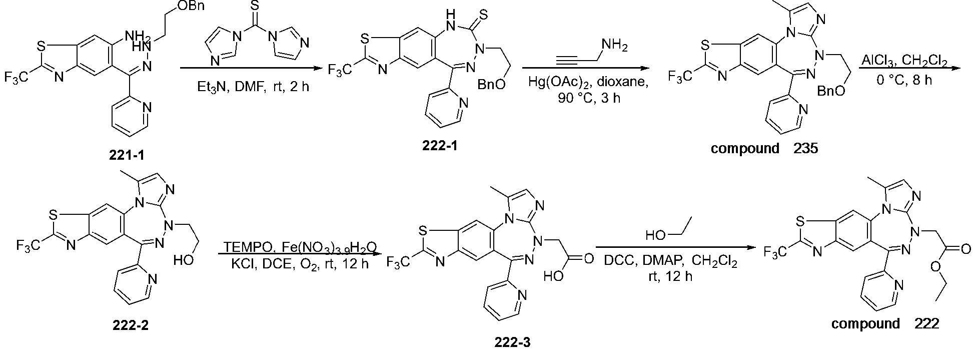

### Preparation of Compound 222-1

At room temperature, Compound **221-1** (4.3 g, 9.12 mmol) was dissolved in DMF (60 mL). N,N'-Thiocarbonyldiimidazole (4.88 g, 27.4 mmol) and triethylamine (0.922 g, 9.11 mmol) were added to the solution. The reaction mixture was stirred for 2 hours. After confirming reaction completion by TLC, water was added to the mixture and the mixture was extracted with dichloromethane (3 × 20 mL). The combined organic phases were washed with brine, dried over anhydrous Na₂SO₄, filtered, and concentrated under reduced pressure to afford the crude product. The crude product was purified by silica gel column chromatography (ethyl acetate/petroleum ether (v/v) = 1/50-1/2), TLC (ethyl acetate/petroleum ether, v/v =1/2) momnitoring and the fractions with Rf = 0.3-0.4 were collected to yield Compound **222-1** (3.26 g, yield 69.6%). ESI[M + H]⁺ = 514.1.

### Preparation of Compound 235

Compound **222-1** (3.26 g, 6.35 mmol) was dissolved in dioxane (40 mL). Propargylamine (3.49 g, 63.4 mmol) and mercury(II) acetate (4.05 g, 12.7 mmol) were added to the solution. The mixture was stirred at 90°C for 3 hours. After confirming reaction completion by TLC, the mixture was concentrated under reduced pressure. The residue was purified by silica gel column chromatography (ethyl acetate/petroleum ether (v/v) = 1/50-1/2). TLC (ethyl acetate/petroleum ether, v/v =1/2) momnitoring and the fractions with Rf = 0.3-0.4 were collected to yield Compound **235** as white solid (1.0 g, yield 29.4%). ESI[M + H]⁺ = 535.1.

¹H NMR (400 MHz, DMSO-d6) δ 8.61 (s, 1H), 8.57 (d, *J =* 4.7 Hz, 1H), 8.11 (s, 1H), 8.02 (d, *J* = 8.0 Hz, 1H), 7.96 (t, *J =* 6.9 Hz, 1H), 7.57 - 7.48 (m, 1H), 7.23 - 7.07 (m, 5H), 6.76 (s, 1H), 4.53 - 4.38 (m, 2H), 4.07 - 4.01 (m, 1H), 3.90 - 3.70 (m, 3H), 2.37 (s, 3H).

### Preparation of Compound 222-2

At 0°C, Compound **235** (972 mg, 1.82 mmol) was dissolved in dichloromethane (20 mL). Aluminum chloride (1.214 g, 9.10 mmol) was added to the solution, and the mixture was stirred for 8 hours. After confirming reaction completion by TLC, ice-water was poured into the mixture and extracted with dichloromethane (3 × 20 mL). The combined organic phases were washed with saturated aqueous sodium bicarbonate solution and brine, dried over anhydrous Na₂SO₄, filtered, and concentrated under reduced pressure to afford the crude product. The crude product was purified by silica gel column chromatography (ethyl acetate/petroleum ether (v/v) = 1/10-1/1). TLC (ethyl acetate/petroleum ether, v/v =1/1) momnitoring and the fractions with Rf = 0.3-0.4 were collected to yield Compound **222-2** (565.5 mg, yield 70.0%). ESI[M + H]⁺ = 445.1.

### Preparation of Compound 222-3

Compound **222-2** (455 mg, 1.02 mmol) was dissolved in 1,2-dichloroethane (45 mL). Iron(III) nitrate nonahydrate (82.8 mg, 0.20 mmol), 2,2,6,6-tetramethylpiperidine 1-oxyl (32.0 mg, 0.20 mmol), and potassium chloride (27.9 mg, 0.20 mmol) were added to the solution. The mixture was stirred under an oxygen atmosphere at room temperature for 12 hours. After confirming reaction completion by TLC, the mixture was concentrated under reduced pressure. The residue was purified by silica gel column chromatography (ethyl acetate/petroleum ether (v/v) = 1/10-1/1). TLC (ethyl acetate/petroleum ether, v/v =1/1) momnitoring and the fractions with Rf = 0.2-0.3 were collected to yield Compound **222-3** as white solid (415 mg, yield 88.4%). ESI[M + H]⁺ = 459.1.

### Preparation of Compound 222

Compound **222-3** (60 mg, 0.13 mmol), DCC (51.6 mg, 0.25 mmol) and DMAP (30.6 mg, 0.25 mmol) were dissolved in dichloromethane (2 mL). The mixture was stirred at room temperature for 15 minutes. Ethanol (12.0 mg, 0.26 mmol) was then added, and stirred at room temperature for 12 hours. After confirming reaction completion by TLC, methyl tert-butyl ether (2 mL) was added, and the mixture was stirred for 5 minutes. The mixture was filtered, and the filtrate was concentrated under reduced pressure to afford the crude product. The crude product was purified by silica gel column chromatography (ethyl acetate/petroleum ether (v/v) = 1/10-1/1). TLC (ethyl acetate/petroleum ether, v/v =1/1) momnitoring and the fractions with Rf = 0.3-0.4 were collected to yield Compound **222** as white solid (20.4 mg, yield 32.0%). ESI[M + H]⁺ = 487.1.

¹H NMR (400 MHz, DMSO-d6) δ 8.64 (s, 1H), 8.58 (d, *J =* 4.7 Hz, 1H), 8.13 (s, 1H), 8.06 - 7.95 (m, 2H), 7.55 (ddd, *J =* 6.8, 4.9, 1.9 Hz, 1H), 6.76 (d, *J =* 0.9 Hz, 1H), 4.72 (d, *J =* 16.4 Hz, 1H), 4.44 (d, *J* = 16.9 Hz, 1H), 4.13 (q, *J =* 7.1 Hz, 2H), 2.39 (s, 3H), 1.20 (t, *J =* 7.1 Hz, 3H).

### Example 115 Preparation of Compound 223-Compound 228

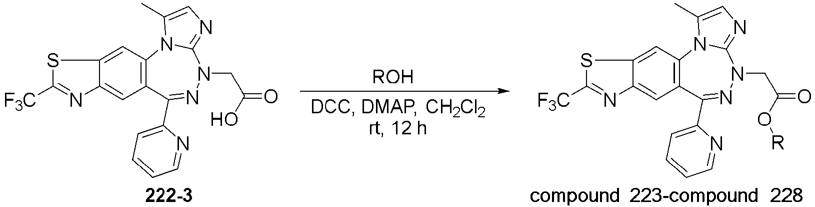

The preparation method of **Compound 223-228** are similar to Compound 222 in example 114, using Compound **222-3** and the corresponding alcohol as starting materials.

Compound **223:** 13.1 mg, ESI[M+H]⁺ = 501.1.

¹H NMR (400 MHz, DMSO-d6) δ 8.64 (s, 1H), 8.58 (d, *J =* 4.6 Hz, 1H), 8.13 (s, 1H), 8.01 - 8.00 (m, 2H), 7.56 - 7.53 (m, 1H), 6.76 (s, 1H), 4.97 - 4.91 (m, 1H), 4.67 (d, *J =* 17.9 Hz, 1H), 4.40 (d, *J* = 17.7 Hz, 1H), 2.39 (s, 3H), 1.19 (d, *J =* 6.2 Hz, 6H).

Compound **224:** 21.5 mg, ESI[M+H]⁺ = 513.1.

¹H NMR (400 MHz, DMSO-d6) δ 8.64 (s, 1H), 8.58 (d, *J* = 4.7 Hz, 1H), 8.12 (s, 1H), 8.01 - 7.98 (m, 2H), 7.60 - 7.48 (m, 1H), 6.77 (d, *J =* 1.0 Hz, 1H), 5.94 - 5.81 (m, 1H), 5.38 - 5.27 (m, 1H), 5.22 - 5.19 (m, 1H), 5.11 (d*, J* = 9.2 Hz, 1H), 4.74 (d, *J =* 13.5 Hz, 1H), 4.46 (d, *J =* 17.5 Hz, 1H), 2.39 (s, 3H), 1.26 (d, *J =* 8.0 Hz, 3H).

Compound **225:** 18.2 mg, ESI[M+H]⁺ = 529.2.

¹H NMR (400 MHz, DMSO-d6) δ 8.64 (s, 1H), 8.58 (d, *J =* 4.4 Hz, 1H), 8.11 (s, 1H), 8.04 - 7.98 (m, 2H), 7.54 (t, *J =* 5.2 Hz, 1H), 6.76 (s, 1H), 4.75 - 4.68 (m, 2H), 4.44 - 4.42 (m, 1H), 2.39 (s, 3H), 1.68 - 1.65 (m, 1H), 1.09 (d, *J =* 6.4 Hz, 3H), 0.76 - 0.75 (m, 6H).

Compound **226:** 18.4 mg, ESI[M+H]⁺ = 541.1.

¹H NMR (400 MHz, DMSO-d6) δ 8.65 (s, 1H), 8.58 (d, *J =* 4.6 Hz, 1H), 8.13 (s, 1H), 8.07 - 7.94 (m, 2H), 7.55 (ddd, *J =* 6.7, 4.8, 2.3 Hz, 1H), 6.79 (d, *J =* 1.1 Hz, 1H), 6.26 (dd, *J =* 17.4, 11.0 Hz, 1H), 5.40 (d, *J =* 17.4 Hz, 1H), 5.29 (d, *J =* 11.0 Hz, 1H), 4.85 - 4.68 (m, 3H), 4.62 - 4.54 (m, 3H), 2.40 (s, 3H).

Compound **227:** 11.2 mg, ESI[M+H]⁺ = 515.1.

¹H NMR (400 MHz, DMSO-d6) δ 8.65 (s, 1H), 8.58 (d, *J =* 4.7 Hz, 1H), 8.14 (s, 1H), 8.02 - 8.00 (m, 2H), 7.56 - 7.55 (m,, 1H), 6.78 (s, 1H), 5.44 - 5.42 (m, 1H), 4.87 - 4.78 (m, 3H), 4.55 - 4.50 (m, 3H), 2.39 (s, 3H).

Compound **228:** 17.2 mg, ESI[M+H]⁺ = 529.1.

¹H NMR (400 MHz, DMSO-*d6*) δ 8.64 (s, 1H), 8.58 (d, *J =* 4.6 Hz, 1H), 8.13 - 8.12 (m, 1H), 8.07 - 7.96 (m, 2H), 7.58 - 7.50 (m, 1H), 6.77 (d, *J =* 1.0 Hz, 1H), 4.74 (d, *J =* 16.4 Hz, 1H), 4.65 - 4.60 (m, 2H), 4.49 - 4.41 (m, 3H), 2.39 (s, 3H), 1.64 (s, 3H).

### Example 116 Preparation of Compound 236

### Method A:

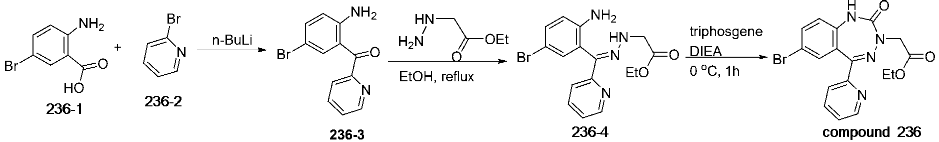

### Preparation of Compound 236-3

At -40°C, the solution of 2-bromopyridine (217 g, 1.37 mol, 4.4 eq) in dry tetrahydrofuran was added dropwise over 30 minutes to solution of n-butyllithium (2.5 M in hexanes, 500 mL, 4 eq) in dry tetrahydrofuran (300 mL). After stirring for 30 minutes, the solution of 2-amino-5-bromobenzoic acid (67.5 g, 0.31 mol, 1 eq) in dry tetrahydrofuran (45 mL) was added dropwise. The mixture was allowed to warm slowly to 0°C and stirred at this temperature for 3 hours. The temperature was subsequently lowered to -10°C, and saturated aqueous ammonium chloride solution (115 mL) was added dropwise. The reaction mixture was concentrated under reduced pressure, then residue was added the brine, the aqueous layer was extracted with ethyl acetate three times. The combined organic layers were dried over anhydrous sodium sulfate and concentrated. The crude product was purified by silica gel column chromatography (petroleum ether/ethyl acetate = 4/1) to yield Compound **236-3** as yellow solid (56.2 g, 65.1% yield).

### Preparation of Compound 236-4

A mixture of Compound **236-3** (13.85 g, 0.05 mol, 1 eq) and ethyl hydrazinoacetate hydrochloride (9.28 g, 0.06 mol, 1.2 eq) in ethanol (250 mL) was stirred at 85°C in sealed tube for 6 hours. Then the ethanol was removed under reduced pressure. The residue was partitioned between ethyl acetate and saturated aqueous sodium bicarbonate solution, and the aqueous layer was extracted with ethyl acetate three times. The combined organic layers were dried over anhydrous sodium sulfate and concentrated. The crude product was purified by silica gel column chromatography (petroleum ether/ethyl acetate = 2/1-1:1) to yield Compound **236-4** as yellow solid (14.6 g, 77.4% yield).

### Preparation of Compound 236

Under nitrogen atmosphere, solution of Compound **236-4** (14.6 g, 36.2 mmol, 1 eq) and DIPEA (18.7 g, 144.8 mmol, 4 eq) in anhydrous dichloromethane (150 mL) was cooled to 0°C. A solution of triphosgene (4.3 g, 14.5 mmol, 0.4 eq) in anhydrous dichloromethane (30 mL) was added dropwise slowly. After confirming reaction completion by TLC, saturated aqueous ammonium chloride solution was added, and the mixture was extracted with dichloromethane. The combined organic layers were dried over anhydrous sodium sulfate and concentrated to yield 17.8 g of the crude product. The crude product was triturated with the mixture of petroleum ether/ethyl acetate (2:1, 40 mL) to yield Compound **236** as off-white solid (15.4 g, yield 76.4%).

### Method B:

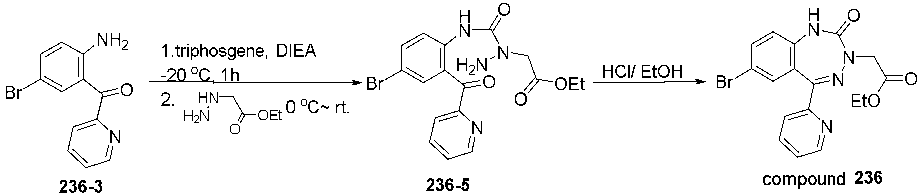

Under nitrogen atmosphere, the solution of Compound **236-3** (1.38 g, 5 mmol, 1 eq) and diisopropylethylamine (2.58 g, 20 mmol, 4 eq) in anhydrous dichloromethane (25 mL) was cooled to 0°C. A solution of triphosgene (0.60 g, 2 mmol, 0.4 eq) in anhydrous dichloromethane (2 mL) was added dropwise. The mixture was stirred at room temperature for 30 minutes, After confirming reaction completion by TLC, the solution of ethyl hydrazinoacetate (0.93 g, 6 mmol, 1.2 eq) in anhydrous dichloromethane (10 mL) was added dropwise to the mixture in ice-bath. The mixture was stirred at room temperature for 2 hours to yield Compound **236-5.**

The pH of the mixture was adjusted to 3-4 with ethanolic HCl solution and refluxed for 2 hours. Saturated aqueous sodium bicarbonate solution was added to adjust the pH to 8-9. The mixture was concentrated, and the residue was extracted with dichloromethane three times. The combined organic layers were dried over anhydrous sodium sulfate and concentrated. The crude product was purified by silica gel column chromatography (dichloromethane/ethyl acetate = 3/1-2/1) to yield Compound **236** as off-white solid (633 mg, three steps yield 31.5%).

### Example 117 Preparation of Compound 237-Compound258

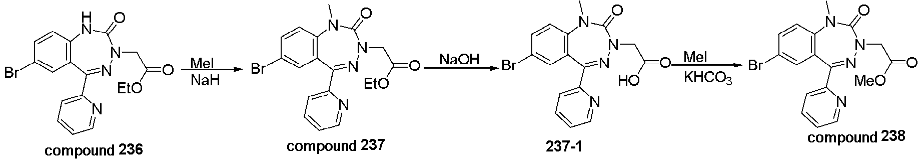

Under nitrogen atmosphere, Compound **236** (800 mg, 2 mmol) was dissolved in anhydrous DMF (10 mL), the solution was cooled to 0°C, then iodomethane (568 mg, 0.4 mmol) and sodium hydride (96 mg, 2.4 mmol) were added to the mixture. Then the mixture was stirred in ice-bath for 1 hour, the mixture was stirred at room temperature for 2 hours. The mixture was quenched with saturated ammonium chloride solution, and the mixture was partitioned between ethyl acetate and water. Then the mixture was extracted with ethyl acetate, the organic layers was combined and washed with brine three times, dried over anhydrous sodium sulfate, and concentrated. The crude product was purified by silica gel column chromatography (petroleum ether/ethyl acetate = 2/1) to yield Compound **237** as yellow solid (755 mg, yield 90.5%).

Compound **237** (83.4 mg) was redissolved in ethanol (20 mL), and aqueous sodium hydroxide solution (9.6 mg, 0.24 mmol) was added. The mixture was stirred at room temperature for 3 hours. The pH was adjusted to neutral with 1N aqueous hydrochloric acid, and the mixture was concentrated to yield the crude product Compound **237-1.** The Compound **237-1** was suspended in DMF, iodomethane (56.8 mg, 0.4 mmol) and potassium bicarbonate (30 mg, 0.3 mmol) were added. Then the mixture was stirred at room temperature for 2 hours, then the mixture was extracted with ethyl acetate, the organic layers was combined and washed with brine three times, dried over anhydrous sodium sulfate, and concentrated. The crude product was purified by silica gel column chromatography (petroleum ether/ethyl acetate = 3/1-2/1) to yield Compound 238 as off-white solid (68 mg, yield 84.4%).

The preparation method of **Compound 239, Compound 240 and Compound** 241 are similar to Compound 212 in example 116, using Compound 237-1(83.4 mg), 2-iodopropane (62.4 mg), iodo-isobutane (68 mg) and 2-iodobutane (68 mg) as starting materials.

### Preparation of Compound 242-Compound 255

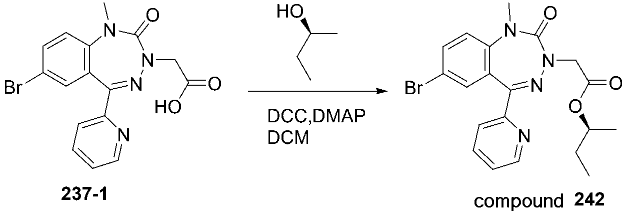

Under nitrogen atmosphere, the mixture of Compound 237-1 (117 mg, 0.3 mmol) and (S)-butan-2-ol (111 mg, 1.5 mmol) in anhydrous dichloromethane was cooled in ice-bath. DCC (82.5 mg, 0.4 mmol) and DMAP (10 mg, 0.08 mmol) were added, and the mixture was stirred at room temperature overnight. The mixture was concentrated to the crude product, and the crude product was suspended in ethyl acetate, filtered, and concentrated. Purification by silica gel column chromatography (petroleum ether/ethyl acetate = 3/1-2/1) to yield Compound **242** (92 mg, yield 67.1%).

The preparation method of **Compound243, Compound244, Compound245, Compound246, Compound247, Compound248, Compound249, Compound250, Compound251, Compound252** are similar to the above procedure, using **237-1** (117 mg), (R)-butan-2-ol (62.4 mg), cyclopropanol (68 mg), 3-oxetanol (111 mg), 1-methylazetidin-3-ol (185 mg), but-3-en-2-ol (108 mg), pentan-3-ol (132 mg), but-3-en-2-ol (108 mg), 3-methylbutan-2-ol (132 mg), 1-cyclopropylethanol (129 mg), (R)-but-3-yn-2-ol (105 mg), and (S)-but-3-yn-2-ol (105 mg) as starting materials.

### Preparation of Compound 253-Compound 255

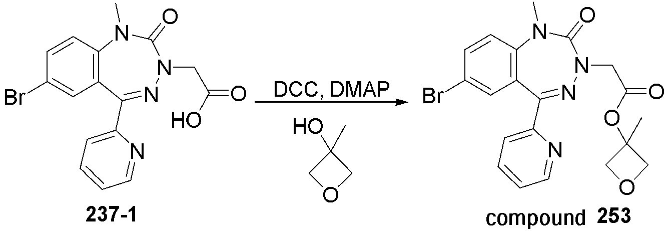

Under nitrogen atmosphere, the solution of **237-1** (117 mg, 0.3 mmol) in dry 3-oxetanol (528 mg, 6 mmol) was cooled in an ice-bath. DCC (82.5 mg, 0.4 mmol) and DMAP (10 mg, 0.08 mmol) were added. The mixture was stirred at room temperature for 48 hours. The mixture was concentrated under reduced pressure, and the crude product was extracted with ethyl acetate. The suspension was filtered, and the filtrate was concentrated. Purification by silica gel column chromatography (Petroleum ether/Ethyl acetate = 3/1-2/1) to yield Compound 253 (46.3 mg, 33.5%).

Following the same procedure, **238-1-6** (117 mg), 3-(ethynylloxy)oxetan-3-ol (600 mg) and tert-amyl alcohol (528 mg) as starting materials to yield Compound **254** and Compound **255.**

### Preparation of Compound 256-Compound257

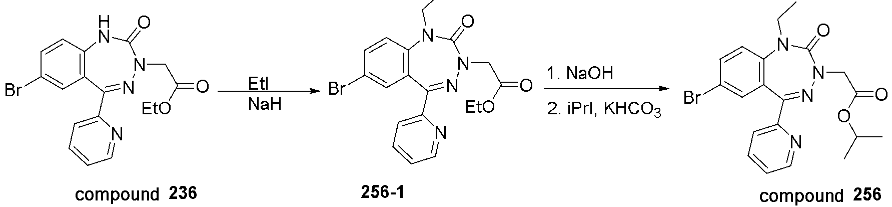

Under nitrogen atmosphere, Compound **236** (120 mg, 0.3 mmol) was dissolved in anhydrous DMF (3 mL). The solution was cooled to 0°C, iodoethane (94 mg, 0.6 mmol) and sodium hydride (13.2 mg, 0.33 mmol) were added. The mixture was stirred at room temperature for 2 hours. The mixture was quenched with saturated ammonium chloride solution, and the mixture was extracted with ethyl acetate three times. The combined organic layers were dried over anhydrous sodium sulfate and concentrated to afford crude Compound **256-1,** which was dissolved in ethanol (3 mL), aqueous sodium hydroxide solution (24 mg in 0.3 mL water) was added, and the mixture was stirred at room temperature for 3 hours. The pH was adjusted to neutral with 1N aqueous hydrochloric acid, and the solvent was removed under reduced pressure. The crude product was suspended in DMF, 2-iodopropane (102 mg, 0.6 mmol) and potassium bicarbonate (36 mg, 0.36 mmol) were added. Then the mixture was stirred at room temperature for 2 hours, the mixture was extracted with ethyl acetate three times. The combined organic layers were dried over anhydrous sodium sulfate and concentrated. Purification by silica gel column chromatography (petroleum ether/ethyl acetate = 3/1-2/1) to yield Compound **256** as off-white solid (88.3 mg, yield 66.1%).

Following the above procedure, Compound 256-1(117 mg) was reacted with 2-iodopropane (102 mg) to yeild Compound **257.**

### Preparation of Compound 258

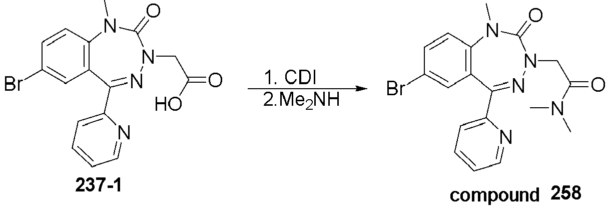

Under nitrogen atmosphere, Compound **237-1** (117 mg, 0.3 mmol) was dissolved in anhydrous dichloromethane (3 mL). The solution was stirred in ice-bath, then 1,1'-carbonyldiimidazole (CDI, 58 mg, 0.36 mmol) was added. The mixture was stirred in the ice-bath for 2 hours, the solution of dimethylamine in tetrahydrofuran (1 M, 3 mL) was added. The mixture was concentrated under reduced pressure, and the mixture was extracted with ethyl acetate three times. The combined organic layers were dried over anhydrous sodium sulfate and concentrated. Purification by silica gel column chromatography (petroleum ether/ethyl acetate = 1/1) to yield Compound **258** as white solid (90.3 mg, yield 72.5%).

| **Compound NO.** | **Compound structure** | **Characterization** |
|---|---|---|
| 1 | 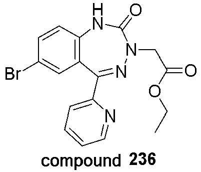 | white solid, ¹H NMR (400 MHz, DMSO-*d6*) δ 9.62 (s, 1H), 8.60 (d, *J =* 4.2 Hz, 1H), 7.96 (td, *J* = 7.7*,* 1.7 Hz, 1H), 7.89 (d, *J =* 7.8 Hz, 1H), 7.67 (dd, *J =* 8.7, 2.3 Hz, 1H), 7.55 - 7.48 (m, 1H), 7.18 (d, *J =* 2.3 Hz, 1H), 7.09 (d, *J =* 8.7 Hz, 1H), 4.35 (s, 2H), 4.12 (q, *J* = 7.1 Hz, 2H), 1.25 - 1.07 (m, 3H). LC-MS: [M+H] ⁺ Calcd 403.03, 405.03, Found 403.1,405.1. |
| 1.1 | 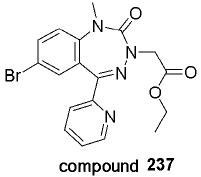 | white solid, ¹H NMR (400 MHz, DMSO-*d6*) δ 8.61 (d, *J* = 4.7 Hz, 1H), 8.02 - 7.90 (m, 2H), 7.77 (dd, *J =* 8.8, 2.4 Hz, 1H), 7.53 (dd, *J =* 8.9, 4.6 Hz, 1H), 7.37 (d, *J =* 8.9 Hz, 1H), 7.30 (d, *J =* 2.4 Hz, 1H), 4.34 (s, 2H), 4.11 (q, *J =* 7.1 Hz, 2H), 3.18 (s, 3H), 1.18 (t*, J =* 7.1 Hz, 3H). LC-MS: [M+H] ⁺ Calcd 417.05, 419.03, Found 417.1, 419.1. |
| 1.2 | 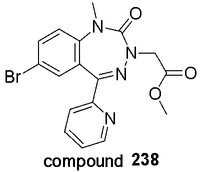 | white solid, ¹H NMR (400 MHz, DMSO-*d6*) δ 8.61 (d, *J* = 4.7 Hz, 1H), 7.97 (dd, *J =* 5.1, 1.5 Hz, 2H), 7.77 (dd, *J =* 8.8, 2.4 Hz, 1H), 7.56 - 7.48 (m, 1H), 7.37 (d, *J* = 8.9 Hz, 1H), 7.30 (d, *J =* 2.4 Hz, 1H), 5.01 - 4.80 (m, 1H), 4.30 (s, 2H), 3.18 (s, 3H), 1.17 (d, *J =* 6.2 Hz, 6H). LC-MS: [M+H] ⁺ Calcd403.03, 405.03, Found 403.1, 405.1. |
| 1.3 | 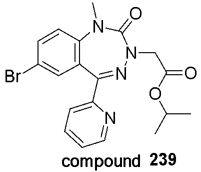 | white solid, ¹H NMR (400 MHz, DMSO--*d6*) δ 8.63 (d, *J* = 4.7 Hz, 1H), 8.00 (m, 2H), 7.79 (dd, *J* = 8.8, 2.4 Hz, 1H), 7.55 (m, 1H), 7.39 (d, *J =* 8.8 Hz, 1H), 7.32 (d, *J =* 2.3 Hz, 1H), 4.94 (hept, *J =* 6.2 Hz, 1H), 4.33 (s, 2H), 3.20 (s, 3H), 1.19 (d, *J =* 6.2 Hz, 6H). LC-MS: [M+H]⁺ Calcd 431.06, 433.06, Found 431.1, 433.1. |
| 1.4 | 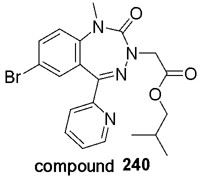 | white solid, ¹H NMR (400 MHz, DMSO-*d6*) δ 8.61 (d, *J* = 4.7 Hz, 1H), 7.96 (ddd, *J* = 9.6, 5.6, 1.4 Hz, 2H), 7.77 (dd, *J =* 8.8, 2.4 Hz, 1H), 7.52 (m, 1H), 7.37 (d, *J= 8.9* Hz, 1H), 7.29 (d, *J =* 2.3 Hz, 1H), 4.37 (s, 2H), 3.84 (d, *J =* 6.4 Hz, 2H), 3.18 (s, 3H), 1.81 (dp, *J =* 13.2, 6.6 Hz, 1H), 0.81 (d, *J =* 6.7 Hz, 6H). LC-MS: [M+H] ⁺ Calcd 445.08, 447.08, Found 445.0, 447.0. |
| 1.5 | 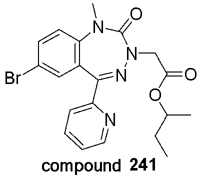 | white solid, ¹H NMR (400 MHz, DMSO-*d6*) δ 8.61 (dt, *J =* 4.8*,* 1.4 Hz, 1H), 7.97 (dd, *J =* 4.7*,* 1.3 Hz, 2H), 7.77 (dd, *J =* 8.8, 2.4 Hz, 1H), 7.52 (dd, *J =* 8.9, 4.6 Hz, 1H), 7.37 (d, *J =* 8.8 Hz, 1H), 7.30 (d, *J =* 2.4 Hz, 1H), 4.77 (dd, *J =* 12.3, 6.3 Hz, 1H), 4.32 (s, 2H), 3.30 (s, 1H), 3.18 (s, 3H), 1.48 (m, 2H), 1.13 (d, *J =* 6.3 Hz, 3H), 0.79 (t, *J=* 7.4 Hz, 3H). LC-MS: [M+H] ⁺ Calcd 445.08, 447.08, Found 445.1, 447.1. |
| 1.5(*S*) | 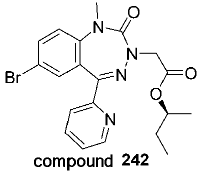 | white solid, ¹H NMR (400 MHz, DMSO-*d6*) δ 8.63 (d, *J* = 4.8 Hz, 1H), 7.99 (d, *J =* 3.9 Hz, 2H), 7.79 (dd, *J =* 8.8, 2.3 Hz, 1H), 7.54 (dd, *J =* 8.8, 4.4 Hz, 1H), 7.39 (d, *J =* 8.9 Hz, 1H), 7.32 (d, *J = 2.3* Hz, 1H), 4.79 (h, *J =* 6.2 Hz, 1H), 4.35 (s, 2H), 3.20 (s, 3H), 1.50 (m, 2H), 1.15 (d, *J =* 6.3 Hz, 3H), 0.81 (t, *J* = 7.4 Hz, 3H). LC-MS: [M+H] ⁺ Calcd 445.08, 447.08, Found 445.1, 447.1. |
| 1.5(*R*) | 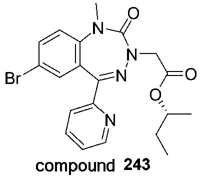 | white solid, ¹H NMR (400 MHz, DMSO*-d6*) δ 8.64 (d, *J* = 4.7 Hz, 1H), 7.99 (d, *J =* 3.8 Hz, 2H), 7.79 (dd, *J =* 8.8, 2.2 Hz, 1H), 7.55 (dd, *J =* 8.9, 4.4 Hz, 1H), 7.39 (d, *J =* 8.8 Hz, 1H), 7.33 (d, *J = 2.3* Hz, 1H), 4.79 (h, *J =* 6.0 Hz, 1H), 4.36 (brs, 2H), 3.20 (s, 3H), 1.50 (m, 2H), 1.16 (d, *J =* 6.3 Hz, 3H), 0.81 (t, *J = 7.4* Hz, 3H). LC-MS: [M+H] ⁺ Calcd 445.08, 447.08, Found 445.1, 447.1. |
| 1.6 | 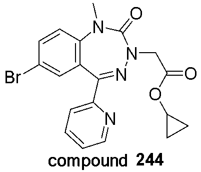 | white solid, ¹H NMR (400 MHz, DMSO-*d6*) δ 8.62 (d, *J* = 4.7 Hz, 1H), 7.99 - 7.92 (m, 2H), 7.78 (dd, *J* = 8.8, 2.4 Hz, 1H), 7.53 (dd, *J* = 8.4, 5.0 Hz, 1H), 7.37 (d, *J=* 8.9 Hz, 1H), 7.31 (d, *J= 2.3* Hz, 1H), 4.32 (s, 2H), 4.17 - 4.03 (m, 1H), 3.18 (s, 3H), 0.75 - 0.54 (m, 4H). LC-MS: [M+H] ⁺ Calcd 429.05, 431.05, Found 429.1, 431.1. |
| 1.7 | 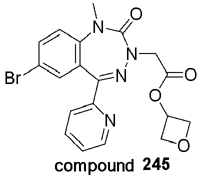 | white solid, ¹H NMR (400 MHz, DMSO-d6) δ 8.62 (d, *J= 5.0* Hz, 1H), 7.97 (d, *J=* 3.6 Hz, 2H), 7.78 (dd, J= 8.8, 2.4 Hz, 1H), 7.53 (dd, J= 8.3, 5.3 Hz, 1H), 7.38 (d, *J=* 8.9 Hz, 1H), 7.31 (d, *J= 2.4* Hz, 1H), 5.48 - 5.33 (m, 1H), 4.78 (s, 2H), 4.46 (dd, *J=* 7.7, 5.1 Hz, 4H), 3.19 (s, 3H). LC-MS: [M+H] ⁺ Calcd 444.04, 446.04, Found 444.1, 446.1. |
| 1.8 | 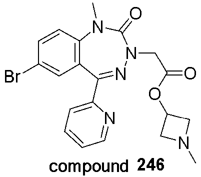 | white solid, ¹H NMR (400 MHz, DMSO-*d6*) δ 8.62 (d, *J* = 4.8 Hz, 1H), 7.97 (d, *J =* 3.6 Hz, 2H), 7.78 (dd, *J =* 8.8, 2.4 Hz, 1H), 7.60 - 7.49 (m, 1H), 7.37 (d, *J* = 8.7 Hz, 1H), 7.31 (d, *J =* 2.4 Hz, 1H), 4.99 - 4.84 (m, 1H), 4.37 (s, 2H), 3.59 - 3.47 (m, 2H), 3.18 (s, 3H), 2.95 - 2.83 (m, 2H), 2.21 (s, 3H). LC-MS: [M+H] ⁺ Calcd 458.07, 460.07, Found 458.1, 460.1. |
| 1.9 | 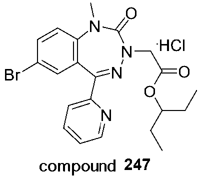 | yellow solid, ¹H NMR (400 MHz, DMSO-*d6*) δ 8.61 (m, 0H), 7.97 (dt, *J =* 7.7, 3.8 Hz, 0H), 7.77 (dd, *J =* 8.8, 2.4 Hz, 0H), 7.53 (h, *J =* 4.2 Hz, 0H), 7.37 (d, *J =* 8.9 Hz, 0H), 7.30 (d, *J =* 2.4 Hz, 0H), 4.68 (m, 1H), 4.36 (s, 2H), 3.18 (s, 3H), 1.49 (m, 4H), 0.78 (t, *J =* 7.4 Hz, 6H). LC-MS: [M+H] ⁺ Calcd 459.10, 461.10, Found 459.1, 461.1. |
| 1.10 | 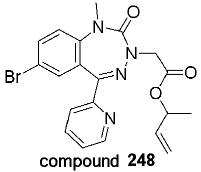 | white solid, ¹H NMR (400 MHz, DMSO-*d6*) δ 8.61 (dt, *J =* 4.8,1.4 Hz, 1H), 7.97 (m, 2H), 7.77 (dd, *J* = 8.8, 2.4 Hz, 1H), 7.53 (m, 1H), 7.37 (d, *J =* 8.9 Hz, 1H), 7.30 (d, *J =* 2.4 Hz, 1H), 5.84 (ddd, *J =* 17.3, 10.6, 5.3 Hz, 1H), 5.30 (m, 1H), 5.20 (dt, *J =* 17.3, 1.5 Hz, 1H), 5.10 (dt, *J =* 10.6, 1.4 Hz, 1H), 4.36 (s, 2H), 3.18 (s, 3H), 1.24 (d, *J =* 6.5 Hz, 3H). LC-MS: [M+H] ⁺ Calcd 443.06, 445.06, Found 443.0, 445.0. |
| 1.11 | 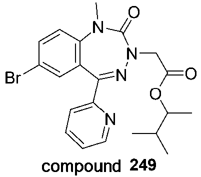 | white solid, ¹H NMR (400 MHz, DMSO-d6) δ 8.61 (dt, *J =* 4.8, 1.4 Hz, 1H), 7.98 (d, *J =* 1.3 Hz, 1H), 7.97 (t, *J =* 1.5 Hz, 1H), 7.77 (dd, *J* = 8.8, 2.4 Hz, 1H), 7.52 (m, 1H), 7.37 (d, *J =* 8.9 Hz, 1H), 7.30 (d, *J* = 2.4 Hz, 1H), 4.66 (m, 1H), 4.35 (s, 2H), 3.18 (s, 3H), 1.67 (qd, *J =* 12.1, 6.6 Hz, 1H), 1.08 (d, *J =* 6.4 Hz, 3H), 0.77 (dd, *J =* 8.0, 6.9 Hz, 6H). LC-MS: [M+H] ⁺ Calcd 459.10, 461.10, Found 459.0, 461.0. |
| 1.12 | 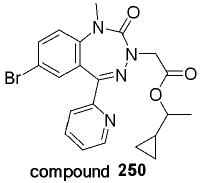 | white solid, ¹H NMR (400 MHz, DMSO-d6) δ 8.62 (dt, *J* = 4.8*,* 1.4 Hz, 1H), 8.62 (dt, *J* = 4.8*,* 1.4 Hz, 1H), 7.97 (m, 2H), 7.78 (dd, *J =* 8.8, 2.4 Hz, 1H), 7.53 (dd, *J =* 8.8, 4.8 Hz, 1H), 7.37 (d, *J =* 8.9 Hz, 1H), 7.31 (d, *J* = 2.4 Hz, 1H), 4.34 (td, *J =* 12.6, 6.2 Hz, 3H), 3.18 (s, 3H), 1.21 (d, *J =* 6.3 Hz, 5H), 0.95 (m, 1H), 0.43 (m, 2H), 0.25 (m, 2H). LC-MS: [M+H] ⁺ Calcd 457.08, 459.08, Found 457.0, 459.0. |
| 1.13 | 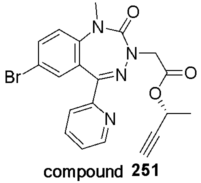 | white solid, ¹H NMR (400 MHz, DMSO-*d6*) δ 8.64 (d, *J = 4*.7 Hz, 1H), 8.00 (m, 2H), 7.80 (dd, *J*= 8.9, 2.4 Hz, 1H), 7.55 (dd, *J =* 8.2, 5.2 Hz, 1H), 7.40 (d, *J =* 8.9 Hz, 1H), 7.32 (d, *J =* 2.3 Hz, 1H), 5.43 (qd, *J =* 6.6, 2.1 Hz, 1H), 4.40 (s, 2H), 3.59 (d, *J=* 2.0 Hz, 1H), 3.21 (s, 3H), 1.43 (d, *J=* 6.7 Hz, 3H). LC-MS: [M+H] ⁺ Calcd 441.05, 443.05, Found 441.0, 443.0. |
| 1.14 | 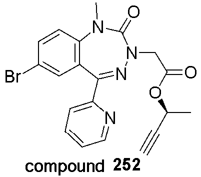 | white solid, ¹H NMR (400 MHz, DMSO-d6) δ 8.63 (d, *J= 4.7* Hz, 1H), 7.99 (m, 2H), 7.80 (dd, *J =* 8.8, 2.4 Hz, 1H), 7.55 (dd, *J =* 8.3, 5.3 Hz, 1H), 7.40 (d, *J =* 8.9 Hz, 1H), 7.32 (d, *J=* 2.3 Hz, 1H), 5.43 (qd, *J=* 6.4, 1.8 Hz, 1H), 4.39 (s, 2H), 3.59 (d, *J=* 2.0 Hz, 1H), 3.21 (s, 3H), 1.43 (d, *J=* 6.7 Hz, 3H). LC-MS: [M+H] ⁺ Calcd 441.05, 443.05, Found 441.0, 443.0. |
| 1.15 | 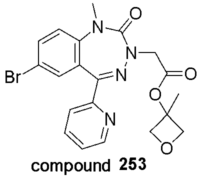 | white solid, ¹H NMR (400 MHz, DMSO) δ 8.61 (d, *J =* 4.7 Hz, 1H), 7.98 (d, *J =* 3.6 Hz, 2H), 7.78 (dd, *J =* 8.8, 2.3 Hz, 1H), 7.53 (d, *J =* 4.3 Hz, 1H), 7.38 (d, *J = 8.9* Hz, 1H), 7.31 (d, *J = 2.3* Hz, 1H), 4.59 (d, *J = 7.2* Hz, 2H), 4.41 (d, *J =* 7.6 Hz, 2H), 4.40 (s, 2H), 3.19 (s, 3H), 1.62 (s, 3H). LC-MS: [M+H] ⁺ Calcd 459.06, 461.06, Found 459.1, 461.1. |
| 1.16 | 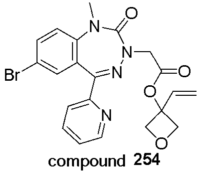 | white solid, ¹H NMR (400 MHz, DMSO-*d6*) δ 8.61 (d, *J =* 4.7 Hz, 1H), 7.98 (d, *J =* 3.4 Hz, 2H), 7.77 (dd, *J =* 8.8, 2.4 Hz, 1H), 7.53 (h, *J =* 4.2 Hz, 1H), 7.38 (d, *J = 8.9* Hz, 1H), 7.31 (d, *J =* 2.3 Hz, 1H), 6.24 (dd, *J =* 17.4, 11.0 Hz, 1H), 5.38 (d, *J =* 17.4 Hz, 1H), 5.29 (d, *J =* 11.0 Hz, 1H), 4.68 (d, *J =* 7.7 Hz, 2H), 4.60 (d, *J =* 7.7 Hz, 2H), 4.46 (s, 2H), 3.19 (s, 3H). LC-MS: [M+H] ⁺ Calcd 471.06, 473.06, Found 471.0, 473.0. |
| 1.17 | 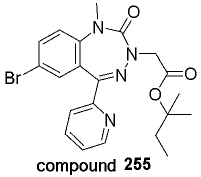 | white solid, ¹H NMR (400 MHz, DMSO-*d6*) δ 8.61 (dt, *J* = 4.8, 1.3 Hz, 1H), 7.97 (m, *J* = 4.9, 1.3 Hz, 2H), 7.77 (dd, *J =* 8.8, 2.4 Hz, 1H), 7.52 (m, 1H), 7.36 (d, *J =* 8.9 Hz, 1H), 7.30 (d, *J =* 2.4 Hz, 1H), 4.25 (s, 2H), 3.17 (s, 3H), 1.65 (q, *J* = 7.5 Hz, 2H), 1.34 (s, 6H), 0.75 (t, *J* = 7.5 Hz, 3H). LC-MS: [M+H] ⁺ Calcd 459.10, 461.10, Found 459.1, 461.1. |
| 1.18 | 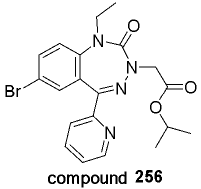 | Colorless syrup, ¹H NMR (400 MHz, DMSO-d6) δ 8.63 (dt, *J* = 4.8, 1.3 Hz, 1H), 7.97 (dd, *J* = 4.6, 1.2 Hz, 2H), 7.77 (dd, *J =* 8.8, 2.4 Hz, 1H), 7.53 (m, 1H), 7.39 (d, *J =* 8.9 Hz, 1H), 7.32 (d, *J =* 2.4 Hz, 1H), 4.91 (hept, *J =* 6.2 Hz, 1H), 4.30 (d, *J =* 39.4 Hz, 2H), 3.91 (s, 1H), 3.61 (s, 1H), 1.16 (d, *J =* 6.2 Hz, 6H), 1.05 (t, *J =* 7.1 Hz, 3H). LC-MS: [M+H] ⁺ Calcd 445.08, 447.08, Found 445.0, 447.0. |
| 1.19 | 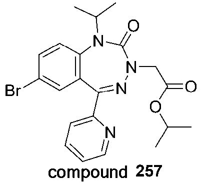 | Off-white solid, ¹H NMR (400 MHz, DMSO-*d6*) δ 8.63 (m, 1H), 7.96 (m, 2H), 7.74 (dd, *J =* 8.8, 2.4 Hz, 1H), 7.52 (ddd, *J =* 5.5*,* 5.0, 2.8 Hz, 1H), 7.39 (d, *J* = 8.8 Hz, 1H), 7.32 (d, *J =* 2.4 Hz, 1H), 4.90 (dt, *J =* 12.5, 6.2 Hz, 1H), 4.38 (d, *J =* 16.8 Hz, 1H), 4.12 (d, *J =* 16.8 Hz, 1H), 4.05 (dt, *J =* 13.6, 6.9 Hz, 1H), 1.37 (d, *J =* 6.7 Hz, 3H), 1.31 (d, *J =* 6.7 Hz, 3H), 1.15 (d, *J =* 3.4 Hz, 2H), 1.13 (d, *J =* 3.4 Hz, 3H). LC-MS: [M+H] ⁺ Calcd 459.10, 461.10, Found 459.2, 461.2. |
| 1.20 | 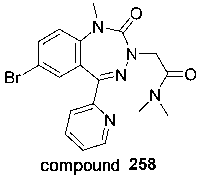 | white solid, ¹H NMR (400 MHz, DMSO-d6) δ 8.60 (m,1H), 7.95 (m, 2H), 7.75 (dd, *J =* 8.8, 2.4 Hz, 1H), 7.51 (ddd, *J =* 6.3, 5.0, 2.5 Hz, 1H), 7.34 (d, *J* = 8.9 Hz, 1H), 7.27 (d, *J =* 2.3 Hz, 1H), 5.32 (t, *J*= 4.6 Hz, 1H), 4.45 (s, 2H), 3.15 (s, 3H), 2.97 (s, 3H), 2.80 (s, 3H). LC-MS: [M+H] ⁺ Calcd 416.06, 418.06, Found 416.0, 418.0. |

### Example 118 Preparation of Compound 259

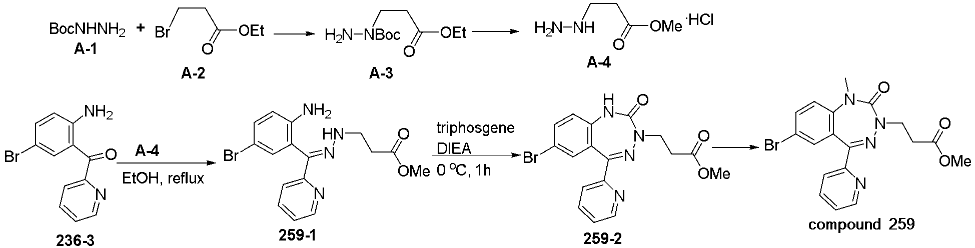

At room temperature, methyl 3-bromopropionate (24.9 g, 150.0 mmol) and N,N-diisopropylethylamine (25.8 g, 200.0 mmol) were added to the solution of tert-butyl hydrazinecarboxylate (13.2 g, 100.0 mmol) in toluene (100 mL). The mixture was stirred at 80°C for 96 hours. After confirming reaction completion by TLC, ice-water (100 mL) was added, and the mixture was extracted with ethyl acetate (3 × 100 mL). The combined organic layers were washed with brine, dried over anhydrous Na₂SO₄, filtered, and concentrated under reduced pressure. The crude product was purified by silica gel column chromatography (methanol/dichloromethane (v/v) = 1:100 to 1:10). TLC (methanol/dichloromethane (v/v) = 1:10) momnitoring was performed, and the fractions with Rf = 0.4-0.5 were collected to yield Compound **A-3** as solid (16.2 g, yield 74.1%). ESI [M + H]⁺ = 218.1.

At room temperature, trifluoroacetic acid (84.7 g, 743 mmol) was added to solution of Compound **A-3** (16.2 g, 74.3 mmol) in dichloromethane (60 mL). The mixture was stirred at room temperature overnight. After confirming reaction completion by TLC, aqueous sodium carbonate solution (50 mL) was added, and the mixture was concentrated under reduced pressure. The crude product was purified by silica gel column chromatography (methanol/dichloromethane (v/v) = 1:20 to 1:2). Fractions with Rf = 0.4-0.5, as monitored by TLC (methanol/dichloromethane (v/v) = 1:2), were collected to yield Compound **A-4** as solid (7.12 g, yield 82.1%). ESI [M + H]⁺ = 118.1.

At room temperature, Compound **A-4** (7.12 g, 60.9 mmol) was added to solution of Compound **236-3** in ethanol (100 mL). The mixture was refluxed overnight. After confirming reaction completion by TLC, the mixture was concentrated under reduced pressure. The crude product was purified by silica gel column chromatography (methanol/dichloromethane (v/v) = 1/20-1/1). TLC (methanol/dichloromethane, v/v =1/1) momnitoring was performed and the fractions with Rf = 0.2-0.3 were collected to yield Compound **259-1** as solid (16.5 g, yield 72.1%). ESI [M + H]⁺ = 376.1.

At 0°C, triphosgene (4.7 g, 15.9 mmol) was dissolved in dichloromethane, and added dropwise to the solution of Compound **259-1** (6.0 g, 15.9 mmol) and triethylamine (4.8 g, 47.7 mmol) in dichloromethane (50 mL). The mixture was stirred for overnight. After confirming reaction completion by TLC, ice-water (100 mL) was added to the mixture, then the mixture was extracted with dichloromethane (3 × 100 mL). The combined organic layers were washed with brine, dried over anhydrous Na₂SO₄, filtered, and concentrated under reduced pressure. The crude product was purified by silica gel column chromatography (ethyl acetate/petroleum ether (v/v) = 1/20-1:2). TLC (methanol/dichloromethane, v/v =1/1) momnitoring was performed and the fractions with Rf = 0.4-0.5 were collected to yield Compound **259-2** as solid (5.58 g, yield 87.4%). ESI [M + H]⁺ = 402.1.

At 0°C, sodium hydride (667 mg, 27.76 mmol) was added to the solution of Compound **259-2** (5.58 g, 13.88 mmol) in N,N-dimethylformamide (40 mL). The mixture was stirred at 0°C for 0.5 hours, iodomethane (2.37 g, 16.66 mmol) was added to the mixture. The mixture was allowed to warm to room temperature and stirred for 0.5 hours. After confirming reaction completion by TLC, ice-water (100 mL) was added, and the mixture was extracted with ethyl acetate (3 × 50 mL). The combined organic layers were washed with brine, dried over anhydrous Na₂SO₄, filtered, and concentrated under reduced pressure. The crude product was purified by silica gel column chromatography (ethyl acetate/petroleum ether (v/v) = 1/20-1/2).TLC (methanol/dichloromethane, v/v =1/1) momnitoring was performed and the fractions with Rf = 0.4-0.5 were collected to yield Compound **259** as solid (5.3 g, yield 91.8%). ESI [M + H]⁺ = 416.1.

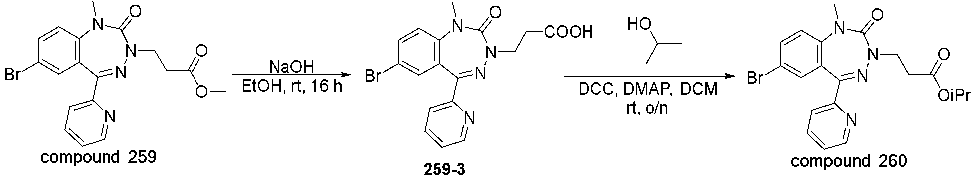

**The preparation method of Compound260, Compound261 and Compound262 are similar** in **example 117.**

| **Compound NO.** | **Compound structure** | **Characterization** |
|---|---|---|
| 2 | 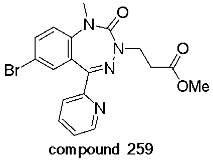 | white solid, ¹H NMR (400 MHz, DMSO-d6) δ 8.61 (d, *J* = 4.7 Hz, 1H), 7.99 (ddd, *J =* 11.7, 9.7, 4.8 Hz, 2H), 7.76 (dd, *J =* 8.8, 2.4 Hz, 1H), 7.53 (ddd, *J =* 7.2, 4.8, 1.4 Hz, 1H), 7.35 (d, *J =* 8.9 Hz, 1H), 7.32 (d, *J =* 2.4 Hz, 1H), 3.77 (s, 2H), 3.50 (s, 3H), 3.17 (s, 3H), 2.68 (t, *J* = 6.8 Hz, 2H), 2.51 (t, *J =* 6.8 Hz, 2H). LC-MS: [M+H]⁺ Calcd 417.05, 419.05, Found 417.1, 419.1. |
| 2.1 | 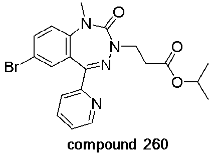 | white solid, ¹H NMR (400 MHz, DMSO-d6) δ 8.61 (d, *J=* 4.4 Hz, 1H), 8.05 (d, *J =* 7.9 Hz, 1H), 7.98 (dd, *J =* 7.6, 1.7 Hz, 1H), 7.74 (dd, *J =* 8.8, 2.4 Hz, 1H), 7.52 (dd, *J =* 6.3, 4.9 Hz, 1H), 7.34 (d, *J =* 8.9 Hz, 1H), 7.29 (d, *J =* 2.4 Hz, 1H), 4.84 - 4.67 (m, 1H), 3.17 (s, 3H), 2.61 (t, *J =* 6.6 Hz, 2H), 2.50 (t, *J =* 6.6 Hz, 2H), 1.00 (d, *J =* 5.6 Hz, 6H). |
| 2.2 | 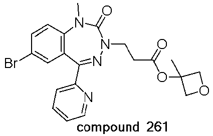 | white solid ¹H NMR (400 MHz, DMSO-*d6*) δ 8.61 (s, 1H), 8.32 (s, 0H), 8.08 (d, *J =* 7.8 Hz, 1H), 7.98 (d, *J =* 5.7 Hz, 1H), 7.81 - 7.70 (m, 1H), 7.54 (d, *J=* 5.4 Hz, 1H), 7.35 (d, *J =* 8.9 Hz, 1H), 7.30 (d, *J =* 2.3 Hz, 1H), 4.36 (s, 2H), 4.28 (s, 2H), 3.18 (s, 3H), 2.68 (t, *J=* 6.5 Hz, 2H), 2.45 (t, *J =* 6.5 Hz, 2H), 1.36 (s, 3H). LC-MS: [M+H]⁺ Calcd 473.07, 475.07, Found 473.1, 475.1. |
| 2.3 | 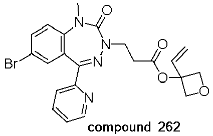 | white solid, ¹H NMR (400 MHz, DMSO-*d6*) δ 8.62 (d, *J =* 4.4 Hz, 1H), 8.07 (s, 1H), 7.98 (dd, *J =* 8.5, 6.8 Hz, 1H), 7.76 (dd, *J* = 8.8, 2.4 Hz, 1H), 7.58 - 7.50 (m, 1H), 7.36 (d, *J =* 8.8 Hz, 1H), 7.30 (d, *J* = 2.4 Hz, 1H), 5.96 (dd, *J =* 17.4, 11.0 Hz, 1H), 5.10 (dd, *J =* 34.6, 14.1 Hz, 2H), 4.47 (s, 4H), 3.19 (s, 3H), 2.76 (t, *J =* 6.5 Hz, 2H), 2.49 (t, *J =* 6.5 Hz, 2H). LC-MS: [M+H]⁺ Calcd 485.07,487.07, Found 485.1, 487.1. |

### Example 119 Preparation of Compound 263-Compound269

### Preparation of Compound 263

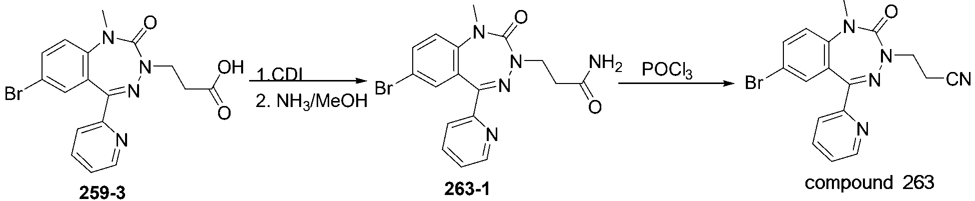

Under nitrogen atmosphere, **259-3** (405 mg, 1 mmol) was dissolved in dry DCM (3 mL). CDI (194 mg, 1.2 mmol) was added in ice-bath, and the mixture was stirred for 2 hours in the ice-bath. Then, 7 M ammonia in methanol (1 mL) was added, and the mixture was stirred at room temperature for 30 minutes. The mixture was concentrated, and the mixture was extracted three times with ethyl acetate and was washed with saturated brine. The combined organic layers were dried over anhydrous sodium sulfate and concentrated. Purification by silica gel column chromatography (petroleum ether/ethyl acetate = 1/1) to yield Compound **263-1.**

Under nitrogen atmosphere, Compound **263-1** was dissolved in dry acetonitrile (3 mL) . Phosphorus oxychloride (306 mg, 2 mmol) was added, and the mixture was refluxed for 2 hours. Then the mixture was stirred at room temperature, ice-water was poured into the mixture to quench the reaction. The pH was adjusted to neutral with aqueous sodium bicarbonate solution under ice-bath. The mixture was extracted with ethyl acetate three times and was washed with saturated brine. The combined organic layers were dried over anhydrous sodium sulfate and concentrated. Purification by silica gel column chromatography (petroleum ether/ethyl acetate = 3/1-2/1) to yield Compound **263** as white solid (284 mg, 74.3%).

### Preparation of Compound 264-Compound267

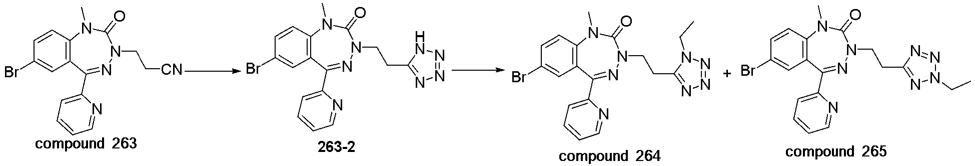

Under nitrogen atmosphere, Compound **263** (148 mg, 0.4 mmol) was dissolved in dry toluene (2 mL). Trimethylsilyl azide (101 mg, 0.88 mmol) and dibutyltin oxide (10 mg, 0.04 mmol) were added, the mixture was stirred at 50°C for one hour, then the mixture was stirred at 95°C for 16 hours. The mixture was concentrated under reduced pressure, the residue was dissolved in dry DMF. Potassium bicarbonate (80 mg, 0.8 mmol) and the corresponding iodoethane (125 mg, 0.8 mmol) were added, and the mixture was stirred at room temperature for 4 hours. After confirming reaction completion by TLC, water was added to the mixture and extracted with ethyl acetate, the combined organic phases were washed with saturated aqueous sodium bicarbonate solution and brine, dried over anhydrous Na₂SO₄, filtered, and concentrated under reduced pressure to afford the crude product. The crude product was purified by silica gel column chromatography (ethyl acetate/petroleum ether (v/v) = 1/4-1/2) to yield Compound **264** as white solid (91 mg, yield 50.0%) and **Compound 265** as white solid (48 mg, yield26.4%).

Following the procedure described above, starting with Compound **263**(148 mg) and using the corresponding 2-iodopropane for the alkylation of the tetrazole, to yield Compound **266** as white solid(53 mg, yield 28.3% ) and Compound **267** as white solid (41 mg, yield 22.5%).

### Preparation of Compound 268 -Compound 269

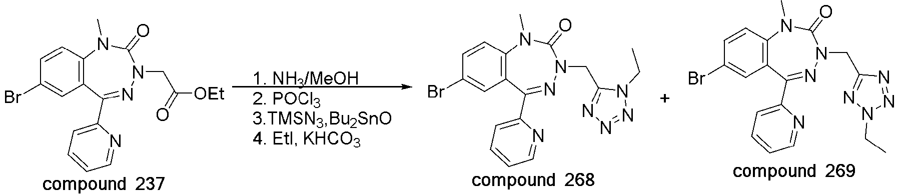

Compound **237** (208 mg, 0.5 mmol) was dissolved in 7 M ammonia in methanol (5 mL). The solution was stirred in sealed tube at 90°C for 48 hours. The mixture was concentrated under reduced pressure to afford syrupy residue. The residue was dissolved in dry acetonitrile (3 mL). Under nitrogen atmosphere, phosphorus oxychloride (306 mg, 2 mmol) was added, and the mixture was refluxed for 2 hours. The mixture was stirred at room temperature, ice-water was added to the mixture. The pH was adjusted to neutral with aqueous sodium bicarbonate solution under ice-cooling. The mixture was extracted with ethyl acetate three times and was washed with saturated brine. The combined organic layers were dried over anhydrous sodium sulfate and concentrated to yield syrupy residue. The resulting syrup was dissolved in dry toluene (2 mL). Trimethylsilyl azide (101 mg, 0.88 mmol) and dibutyltin oxide (10 mg, 0.04 mmol) were added. Under nitrogen atmosphere, the mixture was stirred at 50°C for 1 hour, then the mixture was stirred at 95°C for 16 hours. The mixture was distilled under reduced pressure, and the residue was dissolved in dry DMF. Potassium bicarbonate (80 mg, 0.8 mmol) and the corresponding iodoethane (125 mg, 0.8 mmol) were added, and the mixture was stirred at room temperature for 4 hours, water was added to the mixture and extracted with ethyl acetate, the combined organic layers were dried over anhydrous sodium sulfate and concentrated. Purification by silica gel column chromatography (ethyl acetate/petroleum ether (v/v) = 1/4-1/2) to yield Compound **268** as white solid(68 mg, yield 30.8%) and Compound **269** as white solid (49 mg, yield 22.2%).

| **Compound NO.** | **Compound structure** | **Characterization** |
|---|---|---|
| 3 | 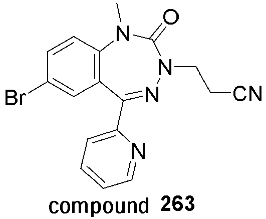 | white solid, ¹H NMR (400 MHz, DMSO-*d6*) δ 8.61 (m, 1H), 8.19 (d, *J* = 7.9 Hz, 1H), 7.97 (td, *J* = 7.8, 1.8 Hz, 1H), 7.77 (dd, *J =* 8.8, 2.4 Hz, 1H), 7.53 (ddd, *J =* 7.6, 4.8, 1.1 Hz, 1H), 7.38 (d, *J =* 8.8 Hz, 1H), 7.33 (d, *J =* 2.4 Hz, 1H), 3.78 (d, *J =* 34.2 Hz, 2H), 3.20 (s, 3H), 2.88 (s, 2H). LC-MS: [M+H]⁺ Calcd 384.04, 386.04, Found 384.0, 386.0. |
| 3.1 | 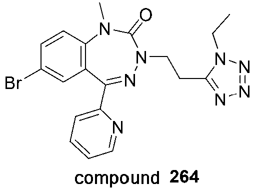 | white solid, ¹H NMR (400 MHz, DMSO-*d6*) δ 8.60 (m, 1H), 7.98 (m, 2H), 7.72 (dd, *J =* 8.8, 2.4 Hz, 1H), 7.52 (ddd, *J* = 6.8, 4.8, 1.8 Hz, 1H), 7.30 (d, *J =* 8.9 Hz, 1H), 7.24 (d, *J =* 2.4 Hz, 1H), 4.50 (q, *J =* 7.3 Hz, 2H), 3.92 (d, *J =* 53.5 Hz, 2H), 3.21 (t, *J =* 6.0 Hz, 2H), 3.15 (s, 3H), 1.36 (t, *J =* 7.3 Hz, 3H). LC-MS: [M+H]⁺ Calcd 455.09, 457.09, Found 455.1, 457.1. |
| 3.2 | 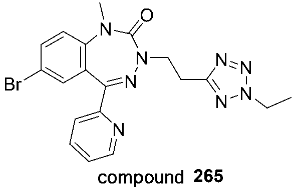 | white solid, ¹H NMR (400 MHz, DMSO-d6) δ 8.61 (dt, *J* = 4.7, 1.3 Hz, 1H), 7.96 (m, 2H), 7.75 (dd, *J =* 8.8, 2.4 Hz, 1H), 7.52 (ddd, *J =* 6.0, 4.9, 2.9 Hz, 1H), 7.31 (dd, *J =* 20.3, 5.6 Hz, 2H), 4.27 (q, *J =* 7.3 Hz, 2H), 3.97 (s, 2H), 3.29 (t, *J =* 7.2 Hz, 2H), 3.18 (s, 3H), 1.33 (t, *J =* 7.3 Hz, 3H). LC-MS: [M+H]⁺ Calcd 455.09, 457.09, Found 455.1, 457.1. |
| 3.3 | 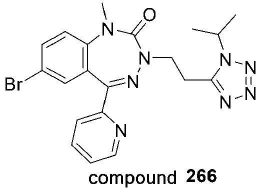 | white solid, ¹H NMR (400 MHz, DMSO-d6) δ 8.60 (ddd, *J =* 4.8, 1.7, 0.9 Hz, 1H), 8.01 (dt, *J =* 8.0, 1.0 Hz, 1H), 7.97 (td, *J = 7.7,* 1.8 Hz, 1H), 7.71 (dd, *J =* 8.8, 2.4 Hz, 1H), 7.52 (m, 1H), 7.30 (d, *J =* 8.8 Hz, 1H), 7.22 (d, *J =* 2.4 Hz, 1H), 4.91 (hept, *J =* 6.6 Hz, 1H), 4.00 (s, 1H), 3.83 (s, 1H), 3.21 (s, 2H), 1.39 (d, *J =* 6.7 Hz, 6H). LC-MS: [M+H]+ Calcd 469.10, 471.10, Found 469.1, 471.1. |
| 3.4 | 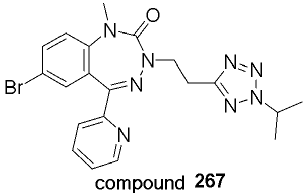 | white solid, ¹H NMR (400 MHz, DMSO-*d6*) δ 8.61 (d, *J = 4.7* Hz, 1H), 7.96 (m, 2H), 7.75 (dd, *J* = 8.8, 2.4 Hz, 1H), 7.53 (ddd, *J* = 5.9, 4.9, 2.9 Hz, 1H), 7.34 (d, *J = 8.8* Hz, 1H), 7.29 (d, *J* = 2.3 Hz, 1H), 4.27 (m, 1H), 3.98 (brs, 2H), 3.29 (t, *J* = 7.8 Hz, 2H), 3.18 (s, 3H), 1.33 (d, *J =* 6.1 Hz, 6H). LC-MS: [M+H] ⁺ Calcd 469.10, 471.10, Found 469.1, 471.1. |
| 3.5 | 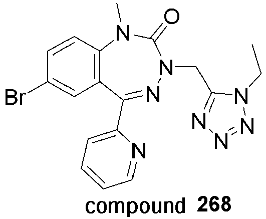 | white solid, ¹H NMR (400 MHz, DMSO-*d6*) δ 8.59 (ddd, *J* = 4.8, 1.7, 0.9 Hz, 1H), 7.94 (td, *J* = 7.7, 1.8 Hz, 1H), 7.82 (dt, *J* = 8.0, 1.0 Hz, 1H), 7.78 (dd, *J* = 8.8, 2.4 Hz, 1H), 7.51 (ddd, *J =* 7.5, 4.8, 1.2 Hz, 1H), 7.39 (d, *J =* 8.9 Hz, 1H), 7.27 (d, *J* = 2.4 Hz, 1H), 5.13 (s, 1H), 4.90 (s, 1H), 4.65 (q, *J =* 7.3 Hz, 2H), 3.22 (s, 3H), 1.47 (t, *J* = 7.3 Hz, 3H). LC-MS: [M+H] ⁺ Calcd 441.07, 443.07, Found 441.0, 443.0. |
| 3.6 | 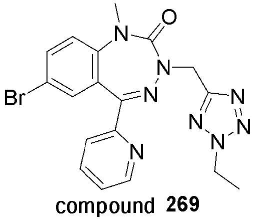 | white solid, ¹H NMR (400 MHz, DMSO-*d6*) δ 8.60 (ddd, *J* = 4.8, 1.7, 0.9 Hz, 1H), 7.95 (td, *J* = 7.8, 1.8 Hz, 1H), 7.80 (dd, *J =* 8.8, 2.4 Hz, 1H), 7.75 (dt, *J* = 8.0, 1.0 Hz, 1H), 7.52 (ddd, *J* = 7.6, 4.8, 1.2 Hz, 1H), 7.41 (d, *J =* 8.9 Hz, 1H), 7.33 (d, *J* = 2.4 Hz, 1H), 5.24 (s, 1H), 5.00 (s, 1H), 4.32 (q, *J* = 7.3 Hz, 2H), 3.24 (s, 3H), 1.21 (t, *J* = 7.3 Hz, 4H). LC-MS: [M+H] ⁺ Calcd 441.07, 443.07, Found 441.0, 443.0. |

### Example 120 Preparation of Compound 270-3

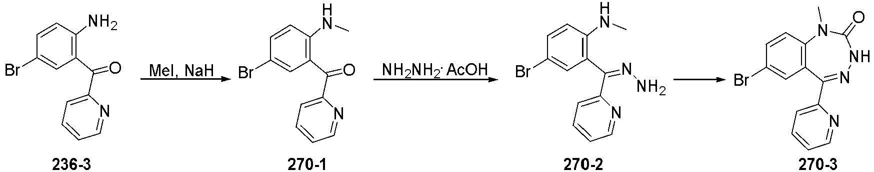

### Preparation of Compound 270-1

Under nitrogen atmosphere, Compound **236-3** (2.76 g, 10 mmol, 1 eq) and MeI (2.84 g, 20 mmol, 2 eq) were dissolved in dry N,N-dimethylformamide (25 mL), then the mixture was stirreded at 0°C. Sodium hydride (440 mg, 11 mmol, 1.1 eq, 40% dispersion in mineral oil) was added. The mixture was stirred at room temperature for 6 hours. The mixture was quenched by saturated ammonium chloride solution (2 mL) and brine (50 mL). The mixture was extracted with ethyl acetate three times, the combined organic layers were dried over anhydrous sodium sulfate and concentrated. Purification by silica gel column chromatography (ethyl acetate/petroleum ether (v/v) =1/3) to yield Compound **270-1** as yellow solid (2.34 g, yield 80.7%).

### Preparation of Compound 270-2

A mixture of **270-1** (2.34 g, 8.07 mmol, 1 eq) and hydrazine acetate (606.9 mg, 8.86 mmol, 1.1 eq) in ethanol (50 mL) , the mixture was stirred at 85°C for 16 hours in sealed tube . The mixture was then quenched by the triethylamine (1 mL). The mixture was concentrated, and purification by silica gel column chromatography (ethyl acetate/petroleum ether (v/v) = 1/2) to yield Compound **270-2 as** yellow solid (2.04 g, yield 83.2%).

### Preparation of Compound 270-3

Under nitrogen atmosphere, solution of Compound **270-2** (2.04 g, 7.67 mmol, 1 eq) and DIPEA (3.96 g, 30.67 mmol, 4 eq) in dry dichloromethane (100 mL), then the mixture was sstirred at 0°C. A solution of triphosgene (911 mg, 3.07 mmol, 0.4 eq) in dry dichloromethane (10 mL) was added to the mixture. After confirming reaction completion by TLC. The mixture was quenched with saturated aqueous ammonium chloride solution and the mixture was stirred at room temperature. The layers were separated, and the aqueous layer was extracted with dichloromethane. The combined organic layers were dried over anhydrous sodium sulfate and concentrated. Purification by silica gel column chromatography (ethyl acetate/petroleum ether (v/v) = 1:1) to yield Compound **270-3** as yellow solid (1.87 g, yield 68.1%).

### Example 121 Preparation of Compound 270-Compound272

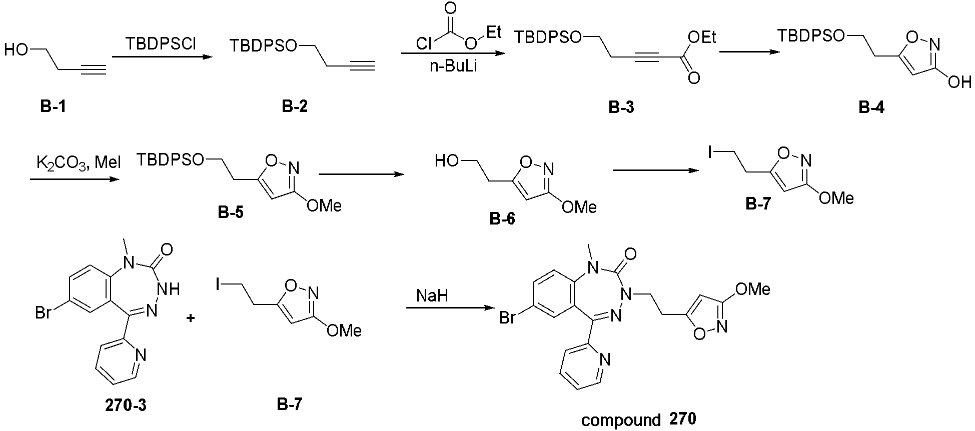

Under nitrogen atmosphere, but-2-yn-1-ol B-1 (700 mg, 10 mmol) and imidazole (816 mg, 12 mmol) were dissolved in dry dichloromethane, then the mixture was cooled to 0°C, and tert-butyl(chloro)diphenylsilane was added dropwise. The mixture was stirred at room temperature for 2 hours, the mixture was quenched with water. The mixture was extracted with ethyl acetate three times, and the combined organic layers were dried over anhydrous sodium sulfate and concentrated. Purification by silica gel column chromatography (petroleum ether/ethyl acetate = 8/1-4/1) to yield Compound **B-2** as colorless oil (3.08 g, yield 100%).

Under nitrogen atmosphere, Compound **B-2** was dissolved in dry diethyl ether and the mixture was stirred at -78°C, n-Butyllithium (2.5 M, 4.4 mL, 12 mmol) was added dropwise to the mixture, followed by the addition of ethyl chloroformate (1.3 g, 12 mmol) after 30 minutes. The mixture was stirred for 2 hours, and then the mixture was quenched with saturated ammonium chloride solution, water was added to the mixture and extracted with ethyl acetate, and the organic layer was separated. The combined organic layers were dried over anhydrous sodium sulfate and concentrated. Purification by silica gel column chromatography (petroleum ether/ethyl acetate = 8/1-4/1) to yield Compound **B-3** as colorless oil (3.4 g, yield 89.5%).

Sodium hydroxide (1.08 g, 26.9 mmol) and hydroxylamine hydrochloride (746 mg, 10.74 mmol) were dissolved in water (9 mL). solution of **B-3** (3.4 g, 8.95 mmol) in ethanol (7.2 mL) was added dropwise to the mixture. The mixture was stirred at room temperature for 16 hours. After confirming reaction completion by TLC, the mixture was extracted with ethyl acetate three times. The combined organic layers were concentrated to yield Compound **B-4.**

To solution of Compound **B-4** (1 g, 2.72 mmol) and iodomethane (774 mg, 5.45 mmol) in DMF were added potassium carbonate (1.13 g, 8.16 mmol). The mixture was stirred at room temperature for 16 hours. After confirming reaction completion by TLC, the mixture was extracted with ethyl acetate (3 × 50 mL). The combined organic layers was purified by silica gel column chromatography (petroleum ether/ethyl acetate, v/v = 5/1-4/1) to yield Compound **B-5** (334 mg, yield 32.4%). Compound **B-5** was then dissolved in tetrahydrofuran, and tetrabutylammonium fluoride (1 M in THF, 1 mL) was added, the mixture was stirred at room temperature for 1 hour, the mixture was partitioned between ethyl acetate and water, the mixture was extracted with ethyl acetate. The combined organic layers were concentrated and purified by silica gel column chromatography (petroleum ether/ethyl acetate = 2/1) to yield Compound **B-6.**

Under nitrogen atmosphere, Compound **B-6** was dissolved in dry tetrahydrofuran, and imidazole (368 mg, 5.4 mmol) and triphenylphosphine (944 mg, 3.6 mmol) were added to the mixture, the mixture was stirred at 0°C. Then iodine (914 mg, 3.6 mmol) was added portionwise to the mixture. The mixture was stirred at room temperature for 16 hours, the partitioned between ethyl acetate and water, the mixture was extracted with ethyl acetate. The combined organic layers were concentrated and purified by silica gel column chromatography (petroleum ether/ethyl acetate = 4:1 to 3:1) to yield Compound **B-7** as off-yellow oil (106 mg, , two steps yield 47.9%).

Under nitrogen atmosphere, Compound **270-3** (82.6 mg, 0.25 mmol) was dissolved in dry N,N-dimethylformamide (2 mL), then the mixture was cooled to 0°C, Sodium hydride (20 mg, 0.5 mmol, 40% dispersion in mineral oil) was added portionwise to the mixture. The mixture was stirred for 30 minutes, Compound **B-7** (134 mg, 0.5 mmol) was added. The mixture was stirred at 50°C for 16 hours. The mixture was quenched with saturated ammonium chloride solution (2 mL). The mixture was partitioned between ethyl acetate and water, the mixture was extracted with ethyl acetate, and the combined organic layers were dried over anhydrous sodium sulfate, concentrated, and purified by silica gel column chromatography (petroleum ether/ethyl acetate = 3/1-2/1) to yield Compound **270** as off-white solid (38 mg, yield 41.7%).

Following the procedure described above, using Compound **B-4** (1 g, 2.72 mmol), iodoethane (850 mg, 5.45 mmol) and 2-iodopropane (927 mg, 5.45 mmol) the starting material to yield Compound **271** and Compound **272.**

### Example 122 Preparation of Compound 273-Compound275

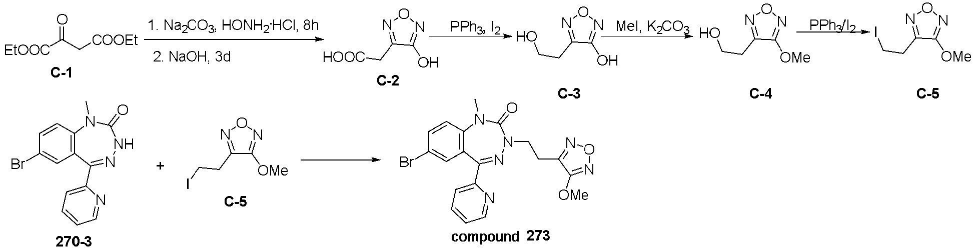

Hydroxylamine hydrochloride (3.7 g, 53 mmol) and sodium carbonate (2.55 g, 23.9 mmol) were dissolved in water (16 mL). Diethyl oxaloacetate (5 g, 26.55 mmol) was added, and the mixture was stirred at room temperature for 8 hours. Subsequently, aqueous solution of sodium hydroxide (2.45 g, 61 mmol in 3 mL water) was added, and the mixture was stirred at 90°C for 3 days. The mixture was stirred at ice-bath, the pH was adjusted to approximately 1 using 5 M sulfuric acid. After confirming reaction completion by TLC, the mixture was extracted with ethyl acetate three times, and the combined organic layers were concentrated to yield Compound **C-2** as off-white solid (1.73 g, yield 45.2%).

Under nitrogen atmosphere, Compound **C-2** (1.4 g, 10 mmol) was dissolved in dry tetrahydrofuran at 0°C, borane-THF solution (1 M, 15 mL) was added dropwise, and the mixture was allowed to warm slowly to room temperature. Then the mixture was stirred at room temperature for 2 hours, the mixture was quenched with methanol (2 mL). then aqueous hydrochloric acid solution (1 N) was added, and the mixture was stirred for 30 minutes. The pH was then adjusted to neutral with saturated sodium bicarbonate solution. The mixture was extracted with ethyl acetate, and the combined organic phases were concentrated to yield Compound **C-3** (1.15 g, yield 88.5%).

Compound **C-3** (390 mg, 3 mmol) was dissolved in dry DMF, Iodomethane (852 mg, 6 mmol) and potassium carbonate (828 mg, 6 mmol) were added, and the mixture was stirred at room temperature overnight. The mixture was partitioned between ethyl acetate and water, the mixture was extracted with ethyl acetate. The combined organic phases were concentrated to yield Compound **C-4** (270 mg, yield 62.5%).

Compound **C-4** (201 mg, 1.5 mmol) was dissolved in dry tetrahydrofuran. Imidazole (306 mg, 4.5 mmol) and triphenylphosphine (761.4 mg, 3 mmol) were added. Under nitrogen atmosphere, the mixture was stirred at 0°C, and iodine (787 mg, 3 mmol) was added portionwise. Then the mixture was stirred at room temperature for 16 hours, the mixture was partitioned between ethyl acetate and water, the mixture was extracted with ethyl acetate. The combined organic layers were concentrated and purified by silica gel column chromatography (petroleum ether/ethyl acetate = 4/1-3/1) to yield Compound **C-5** as off-yellow oil (228 mg, yield 59.8%).

Under nitrogen atmosphere, Compound **270-3** (82.6 mg, 0.25 mmol) was dissolved in dry N,N-dimethylformamide (2 mL) and cooled to 0°C. Sodium hydride (20 mg, 0.5 mmol, 40% dispersion in mineral oil) was added portionwise. The mixture was stirred for 30 minutes, Compound **C-5** (127 mg, 0.5 mmol) was added. The mixture was stirred stirred at 50°C for 16 hours. The mixture was quenched with saturated ammonium chloride solution (2 mL). The mixture was partitioned between ethyl acetate and water, the mixture was extracted with ethyl acetate, and the combined organic layers were dried over anhydrous sodium sulfate, concentrated, and purified by silica gel column chromatography (petroleum ether/ethyl acetate = 3/1-2/1) to yield Compound **273** as yellow solid (19.2 mg, yield 16.8%).

Following the procedure described above, using Compound **C-3** (390 mg, 3 mmol), iodoethane (936 mg, 6 mmol) and 2-iodopropane (1.02 g, 6 mmol) the starting material to yield Compound **274** and Compound **275.**

| **Compound NO.** | **Compound structure** | **Characterization** |
|---|---|---|
| 4.1 | 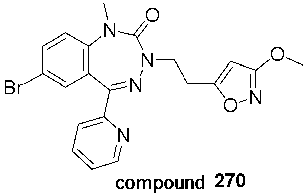 | Off-white solid, ¹H NMR (400 MHz, DMSO-*d6*) δ 8.62 (d, *J* = 4.1 Hz, 1H), 8.05 (d, *J =* 7.9 Hz, 1H), 7.98 (td, *J* = 7.7, 1.7 Hz, 1H), 7.76 (dd, *J* = 8.8, 2.4 Hz, 1H), 7.54 (m, 1H), 7.33 (dd, *J* = 15.6, 5.6 Hz, 2H), 5.89 (s, 1H), 3.90 (brs, 1H), 3.79 (brs, 4H), 3.19 (s, 3H), 3.04 (brs, 2H). LC-MS: [M+H] ⁺ Calcd 456.06, 458.06, Found 456.3, 458.3 |
| 4.2 | 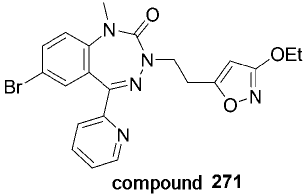 | Off-white solid, ¹H NMR (400 MHz, DMSO-*d6*) δ 8.62 (d, *J* = 4.2 Hz, 1H), 8.05 (d, *J = 7.9* Hz, 1H), 7.97 (td, *J* = 7.8*,* 1.6 Hz, 1H), 7.76 (dd, *J* = 8.8, 2.3 Hz, 1H), 7.54 (dd, *J* = 6.3, 5.0 Hz, 1H), 7.32 (dd, *J* = 15.6, 5.6 Hz, 2H), 5.86 (s, 1H), 4.09 (q, *J =* 7.0 Hz, 2H), 3.91 (brs, 1H), 3.76 (brs, 1H), 3.18 (s, 3H), 3.04 (s, 2H), 1.28 (t, *J* = 7.0 Hz, 3H). LC-MS: [M+H] ⁺ Calcd 470.07, 472.07, Found 470.4, 472.4. |
| 4.3 | 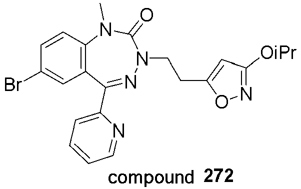 | light brown solid, 1H NMR (400 MHz, DMSO-d6) δ 8.62 (d, J = 4.5 Hz, 1H), 8.06 (d, J = 8.0 Hz, 1H), 7.97 (td, J = 7.7, 1.7 Hz, 1H), 7.75 (dd, J = 8.8, 2.4 Hz, 1H), 7.54 (ddd, J = 7.3, 4.8, 1.0 Hz, 1H), 7.35 (d, J = 8.9 Hz, 1H), 7.30 (d, J = 2.3 Hz, 1H), 5.84 (s, 1H), 4.61 (dt, J = 12.2, 6.1 Hz, 1H), 3.84 (d, J = 64.7 Hz, 2H), 3.18 (s, 3H), 3.03 (s, 2H), 1.25 (d, J = 6.1 Hz, 6H). LC-MS: [M+H] + Calcd 484.09, 486.09, Found 484.2, 486.2. |
| 4.4 | 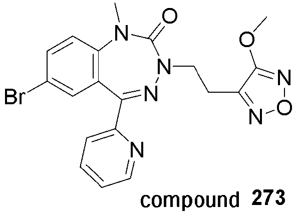 | Orange Solid, ¹H NMR (400 MHz, DMSO-*d6*) δ 8.63 (d, *J* = 4.5 Hz, 1H), 7.99 (m, 2H), 7.76 (dd, *J* = 8.8, 2.4 Hz, 1H), 7.55 (ddd, *J* = 6.7, 4.8, 1.8 Hz, 1H), 7.35 (d, *J* = 8.8 Hz, 1H), 7.29 (d, *J* = 2.4 Hz, 1H), 3.94 (s, 3H), 3.88 (brs, 2H), 3.18 (s, 3H), 3.07 (t, *J = 6.8* Hz, 2H). LC-MS: [M+H]+ Calcd 457.05, 459.05, Found 457.1,459.1. |
| 4.5 | 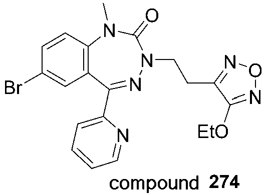 | Orange Solid, ¹H NMR (400 MHz, DMSO-*d6*) δ 8.63 (d, *J =* 4.7 Hz, 1H), 7.98 (td, *J = 7.6,* 1.2 Hz, 1H), 7.96 (t, *J =* 7.6 Hz, 1H), 7.76 (dd, *J* = 8.8, 2.4 Hz, 1H), 7.55 (ddd, *J =* 6.6, 4.8, 1.4 Hz, 1H), 7.34 (d, *J =* 8.8 Hz, 1H), 7.26 (d, *J* = 2.3 Hz, 1H), 4.24 (q, *J* = 7.0 Hz, 2H), 3.91 (s, 2H), 3.18 (s, 3H), 3.08 (t, *J =* 6.1 Hz, 2H), 1.30 (t, *J =* 7.0 Hz, 3H). LC-MS: [M+H] ⁺ Calcd 471.07, 473.07, Found 471.1, 473.1. |
| 4.6 | 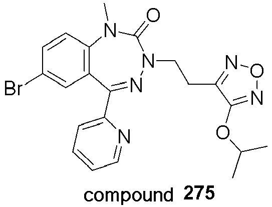 | Orange Solid, ¹H NMR (400 MHz, DMSO-*d6*) δ 8.62 (d, *J* = 4.3 Hz, 1H), 7.98 (td, *J = 7.6,* 1.2 Hz, 1H), 7.94 (t, *J = 7.6* Hz, 1H), 7.75 (dd, *J* = 8.8, 2.4 Hz, 1H), 7.54 (ddd, *J* = 7.0, 4.8, 1.4 Hz, 1H), 7.33 (d, *J =* 8.9 Hz, 1H), 7.25 (d, *J* = 2.4 Hz, 1H), 4.70 (hept, *J =* 6.1 Hz, 1H), 3.90 (s, 2H), 3.17 (s, 3H), 3.07 (s, 2H), 1.29 (d, *J =* 6.1 Hz, 6H). LC-MS: [M+H] ⁺ Calcd 485.09, 487.09, Found485.1, 487.1. |

### Example 123 Preparation of Compound 276-Compound282

### Preparation of Compound 276-Compound278

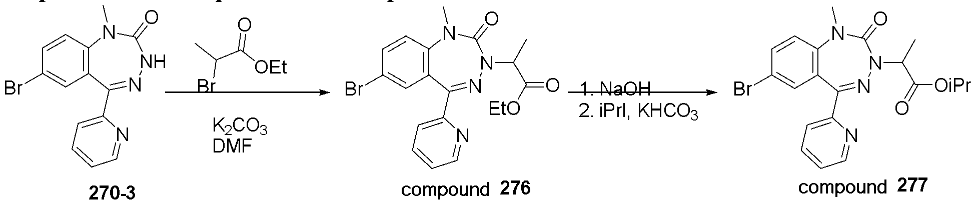

Compound **270-3** (165 mg, 0.5 mmol) was dissolved in dry DMF. Ethyl α-bromobutyrate (181 mg, 1 mmol) and potassium carbonate (138 mg, 1 mmol) were added, and the mixture was stirred at room temperature for 16 hours. The mixture was partitioned between ethyl acetate and water, the mixture was extracted with ethyl acetate. The combined organic phase was washed with brine, concentrated, and purified by silica gel column chromatography (petroleum ether/ethyl acetate = 3/1) to yield Compound **276** (138 mg, yield 64.0%).

Compound **276** (64 mg, 0.15 mmol) was dissolved in DMF. 2-Iodopropane (51 mg, 0.3 mmol) and potassium bicarbonate (30 mg, 0.3 mmol) were added, and the mixture was stirred at room temperature for 2 hours. The mixture was then partitioned between ethyl acetate and saturated brine, the mixture was extracted with ethyl acetate. The combined organic layers were dried over anhydrous sodium sulfate, concentrated, and purified by silica gel column chromatography (petroleum ether/ethyl acetate = 3/1) to yield Compound **277** as off-white solid (55 mg, yield 82.6%).

Following the procedure described above, using Compound **270-4** (165 mg, 0.5 mmol), and ethyl 2-bromoisovalerate (209 mg, 1 mmol) the starting material to yield Compound **278.**

### Preparation of Compound 279-Compound282

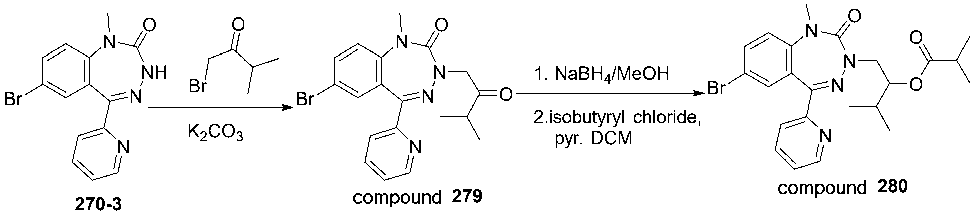

Compound **270-3** (165 mg, 0.5 mmol) was dissolved in dry DMF. 1-Bromo-3-methyl-2-butanone (165 mg, 1 mmol) and potassium carbonate (138 mg, 1 mmol) were added, and the mixture was stirred at room temperature for 16 hours. The mixture was then partitioned between ethyl acetate and water, the mixture was extracted with ethyl acetate, the organic phase was washed with brine, concentrated, and purified by silica gel column chromatography (petroleum ether/ethyl acetate = 3/1) to yield Compound **279** (162 mg, yield 78.0%).

Under nitrogen atmosphere, Compound **279** (83 mg, 0.2 mmol) was dissolved in the mixture of dry methanol and tetrahydrofuran (1 mL/1 mL). The solution was stirred in ice-bath, and sodium borohydride (38 mg, 1 mmol) was added portionwise. The mixture was stirred at room temperature for 16 hours. The mixture was quenched with saturated ammonium chloride solution, then partitioned between ethyl acetate and water, the mixture was extracted with ethyl acetate, the organic phase was washed with brine, filtered and concentrated under reduced pressure to obtain crude product. The crude product was dissolved in dry dichloromethane (2 mL) and stirred at -30°C. Pyridine (79 mg, 1 mmol) and isobutyryl chloride (43 mg, 0.4 mmol) were added dropwise sequentially. The mixture was quenched with water and partitioned between ethyl acetate and water, the mixture was extracted with ethyl acetate, The combined organic layers were washed with brine, concentrated, and purified by silica gel column chromatography (petroleum ether/ethyl acetate = 3/1) to yield Compound **280** (162 mg, yield 78.0%).

Following the procedure described above, using Compound **270-3** (83 mg, 0.2 mmol), and α-bromobutanone (151 mg, 1 mmol) as the starting material to yield **Compound 281** and **Compound 282.**

| **Compound NO.** | **Compound structure** | **Characterization** |
|---|---|---|
| 4.7 | 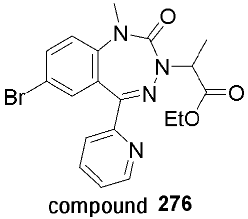 | white solid, ¹H NMR (400 MHz, DMSO-*d6*) δ 8.62 (d, *J= 4.7* Hz, 1H), 7.99 (m, 2H), 7.78 (dd, *J =* 8.8, 2.3 Hz, 1H), 7.54 (m, 1H), 7.37 (d, *J* = 8.8 Hz, 1H), 7.31 (d, *J* = 2.4 Hz, 1H), 4.71 (q, *J* = 7.0 Hz, 1H), 4.11 (m, 2H), 3.18 (s, 3H), 1.50 (brs, 3H), 1.12 (brs, 3H). LC-MS: [M+H]⁺ Calcd 431.06, 433.06. Found 431.1, 433.1. |
| 4.8 | 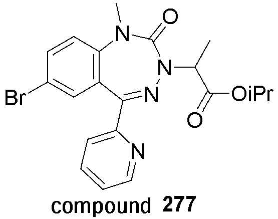 | white solid, ¹H NMR (400 MHz, DMSO-*d6*) δ 8.63 (d, *J = 4.7* Hz, 1H), 7.99 (m, 2H), 7.78 (dd, *J =* 8.8, 2.0 Hz, 1H), 7.54 (m, 1H), 7.38 (d, *J* = 8.8 Hz, 1H), 7.32 (d, *J* = 2.3 Hz, 1H), 4.90 (s, 1H), 4.68 (q, *J =* 6.7 Hz, 1H), 3.18 (s, 3H), 1.51 (s, 3H), 1.12 (s, 6H). LC-MS: [M+H]⁺ Calcd 445.08, 447.08 Found 445.1.1,447.1. |
| 4.9 | 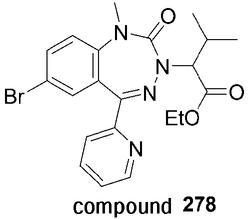 | white solid, ¹H NMR (400 MHz, DMSO-*d6*) δ 8.63 (d, *J* = 4.8 Hz, 1H), 8.00 (m, 2H), 7.78 (dd, *J =* 8.7, 2.0 Hz, 1H), 7.55 (dd, *J =* 8.7, 4.8 Hz, 1H), 7.38 (d, *J* = 9.1 Hz, 1H), 7.36 (d, *J = 2.3* Hz, 1H), 4.35 (s, 1H), 4.14 (brs, 2H), 3.18 (s, 3H), 1.17 (s, 3H), 0.91 (brs, 3H), 0.80 (brs, 3H). LC-MS: [M+H]⁺ Calcd 459.10, 461.09 Found 459.1, 461.1. |
| 4.10 | 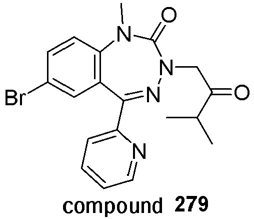 | white solid, ¹H NMR (400 MHz, DMSO-*d6*) δ 8.58 (d, *J* = 4.3 Hz, 1H), 7.93 (ddd, *J* = 12.6, 9.4, 4.6 Hz, 2H), 7.74 (dd, *J* = 8.8, 2.4 Hz, 1H), 7.49 (ddd, *J* = 6.7, 4.8, 1.6 Hz, 1H), 7.30 (dd, *J* = 24.9, 5.6 Hz, 2H), 4.51 (s, 2H), 3.14 (s, 3H), 2.68 (dt, *J* = 13.8, 6.9 Hz, 1H), 1.00 (d, *J* = 6.9 Hz, 6H). LC-MS: [M+H]⁺ Calcd 415.07, 417.07, Found 415.1, 417.1. |
| 4.11 | 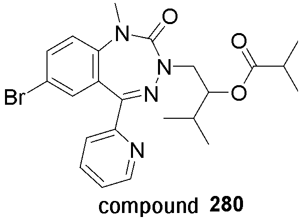 | Off-white solid, ¹H NMR (400 MHz, DMSO-*d6*) δ 8.63 (d, *J =* 4.3 Hz, 1H), 8.06 (m, 2H), 7.75 (dd, *J* = 8.7, 1.9 Hz, 1H), 7.56 (m, 1H), 7.34 (d, *J* = 8.8 Hz, 1H), 7.14 (s, 1H), 5.00 (s, 1H), 3.91 (m, 1H), 3.55 (d, *J =* 12.0 Hz, 1H), 3.18 (s, 3H), 2.12 (s, 1H), 1.88 (s, 1H), 0.83 (m, 12H). LC-MS: [M+H]⁺ Calcd 487.13, 489.13, Found 487.2, 489.2. |
| 4.12 | 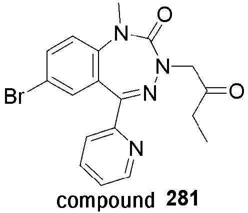 | white solid, ¹H NMR (400 MHz, DMSO-*d6*) δ 8.61 (d, *J* = 4.6 Hz, 1H), 7.95 (m, 2H), 7.77 (dd, *J =* 8.8, 2.4 Hz, 1H), 7.52 (ddd, *J* = 6.7, 4.8, 2.1 Hz, 1H), 7.36 (d, *J =* 8.9 Hz, 1H), 7.31 (d, *J* = 2.3 Hz, 1H), 4.44 (s, 2H), 3.17 (s, 3H), 2.44 (q, *J* = 7.3 Hz, 2H), 0.92 (t, *J* = 7.3 Hz, 3H). LC-MS: [M+H]⁺ Calcd 401.05, 403.05, Found 401.1, 403.1. |
| 4.13 | 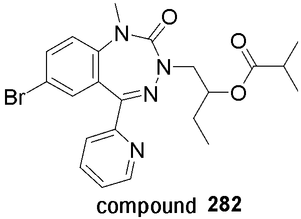 | Off-white solid, ¹H NMR (400 MHz, DMSO-*d6*) δ 8.63 (d, *J = 4.8* Hz, 1H), 8.10 (d, *J =* 7.3 Hz, 1H), 8.02 (t, *J = 7.6* Hz, 1H), 7.75 (dd, *J =* 8.8, 1.6 Hz, 1H), 7.55 (m, 1H), 7.34 (d, *J =* 8.8 Hz, 1H), 7.15 (s, 1H), 5.03 (m, *J* = 49.7 Hz, 1H), 4.08 (m, *J =* 157.3 Hz, 1H), 3.52 (m, *J* = 10.1 Hz, 1H), 3.18 (s, 3H), 2.14 (m, 1H), 1.55 (m, 2H), 0.75 (m, 9H). LC-MS: [M+H]⁺ Calcd 473.11, 475.11, Found 473.1, 475.1. |

### Example 124 Preparation of Compound 276-Compound 282

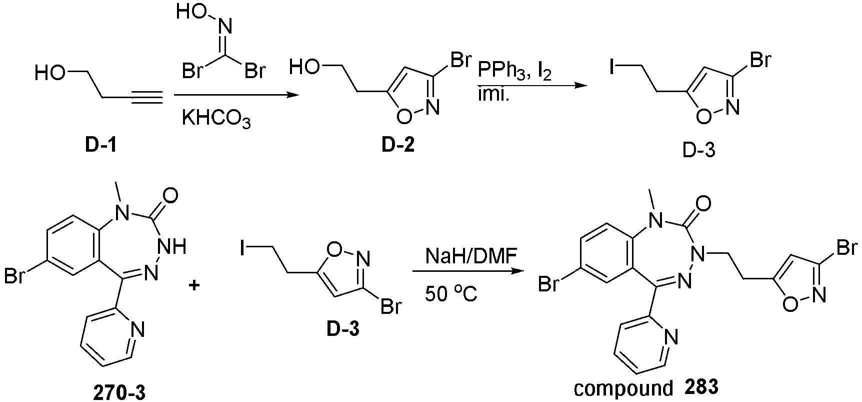

But-2-yn-1-ol (7 g, 100 mmol) and potassium bicarbonate (7.5 g, 75 mmol) were dissolved in the mixture of ethyl acetate (50 mL) and water (5 mL). the mixture was stirred at room temperature, 1,1-dibromoformaldehyde oxime (5 g, 24.4 mmol) was added dropwise. The reaction mixture was stirred at room temperature for 16 hours. The layers were separated, the combined organic extract were dried over anhydrous sodium sulfate, concentrated, and purified by silica gel column chromatography (petroleum ether/ethyl acetate = 3/1) to yield Compound **D-2** as yellow oil (3.64 g, yield 77.7%).

Under nitrogen atmosphere, Compound **D-2** (201 mg, 1.5 mmol) was dissolved in dry tetrahydrofuran. Imidazole (306 mg, 4.5 mmol) and triphenylphosphine (761.4 mg, 3 mmol) were added, the mixture was stirred at 0°C, and iodine (787 mg, 3 mmol) was added portionwise. The mixture was stirred at room temperature for 16 hours, the mixture was partitioned between ethyl acetate and water, the mixture was extracted with ethyl acetate. The combined organic layers were concentrated and purified by silica gel column chromatography (petroleum ether/ethyl acetate = 4/1-3/1) to yield Compound **D-3** as off-yellow oil (228 mg, yield 59.8%).

The preparation method of compound 2**83** is similar to example 42, using compound **D-3** as starting materials.

### Preparation of Compound 284-Compound 286

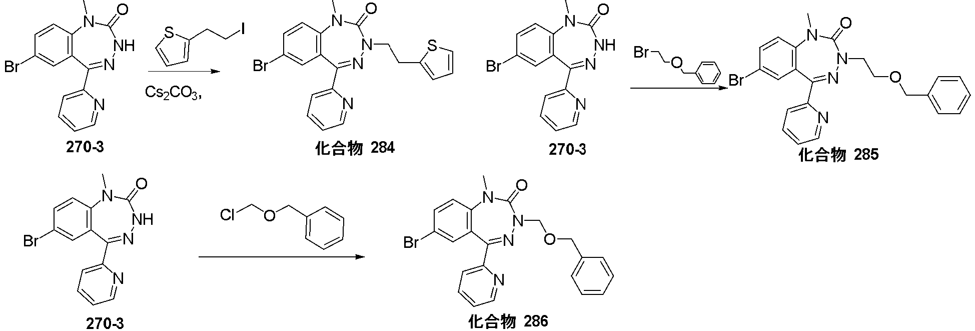

Compound **270-3** (61 mg, 0.2 mmol) was dissolved in dry DMF, and cesium carbonate (195.3 mg, 0.4 mmol) was added. The resulting mixture were separately added 2-(2-iodoethyl)thiophene (95 mg, 0.4 mmol), benzyl 2-bromoethyl ether (86 mg, 0.4 mmol), and benzyl chloromethyl ether (63 mg, 0.4 mmol) respectively. The mixtures were stirred at room temperature for 16 hours. ethyl acetate and water were added, the mixture was extracted with ethyl acetate, and the organic phases were combined, washed with brine, and concentrated. The residue was purified by silica gel column chromatography (petroleum ether/ethyl acetate = 3/1) to yield thet compounds: Compound **284** (35 mg, yield 39.6%), Compound **285** (67 mg, yield 72.0%), and Compound **286** (18 mg, yield 20.0%).

| **Compound NO.** | **Compound structure** | **Characterization** |
|---|---|---|
| 4.14 | 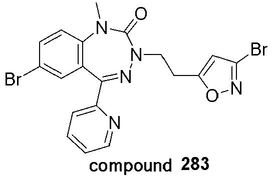 | yellow solid, ¹H NMR (400 MHz, DMSO-*d6*) δ 8.63 (d, *J =* 4.5 Hz, 1H), 7.98 (m, 2H), 7.76 (dd, *J* = 8.8, 2.4 Hz, 1H), 7.55 (m, 1H), 7.35 (d, *J* = 8.8 Hz, 1H), 7.31 (d, *J* = 2.3 Hz, 1H), 6.47 (s, 1H), 3.89 (d, *J* = 47.6 Hz, 2H), 3.31 (brs, 2H), 3.19 (brs, 5H). LC-MS: [M+H] ⁺ Calcd 503.96, 505.96, 507.96, Found 504.0, 506.0, 508.0. |
| 4.15 | 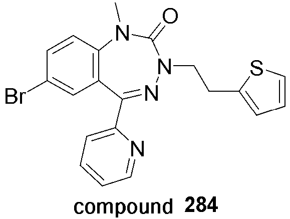 | Off-yellow solid, ¹H NMR (400 MHz, DMSO-*d6*) δ 8.63 (d, *J* = 4.3 Hz, 1H), 8.09 (d, *J =* 7.9 Hz, 1H), 7.99 (td, *J* = 7.8, 1.7 Hz, 1H), 7.76 (dd, *J* = 8.8, 2.4 Hz, 1H), 7.54 (ddd, *J* = 7.4, 4.8, 1.0 Hz, 1H), 7.35 (d, *J = 8.8* Hz, 1H), 7.32 (d, *J = 2.3* Hz, 1H), 7.25 (dd, *J =* 5.1, 1.1 Hz, 1H), 6.86 (dd, *J =* 5.1, 3.4 Hz, 1H), 6.81 (d, *J* = 3.1 Hz, 1H), 3.89 (s, 1H), 3.74 (s, 1H), 3.19 (s, 3H), 3.15 (t, *J* = 7.0 Hz, 2H). LC-MS: [M+H] ⁺ Calcd 441.03.10, 443.03, Found 441.0, 443.0. |
| 4.16 | 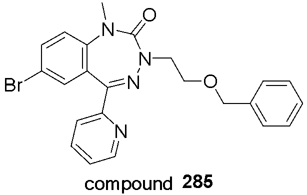 | Off-yellow solid, ¹H NMR (400 MHz, DMSO-*d6*) δ 8.63 (d, *J =* 4.7 Hz, 1H), 7.95 (m, 2H), 7.79 (dd, *J* = 8.8, 2.3 Hz, 1H), 7.53 (m, 1H), 7.38 (d, *J* = 8.8 Hz, 1H), 7.32 (d, *J* = 2.3 Hz, 1H), 7.25 (m, 3H), 7.11 (m, 2H), 4.41 (s, 2H), 4.03 (s, 1H), 3.66 (m, 3H), 3.19 (s, 3H). LC-MS: [M+H]⁺ Calcd 465.08, 467.08, Found 465.1, 467.1. |
| 4.17 | 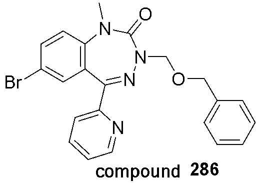 | white solid, ¹H NMR (400 MHz, DMSO-*d6*) δ 8.64 (d, *J =* 4.6 Hz, 1H), 8.06 (d, *J* = 7.9 Hz, 1H), 8.00 (td, *J* = 7.7, 1.6 Hz, 1H), 7.79 (dd, *J* = 8.8, 2.4 Hz, 1H), 7.55 (ddd, *J =* 7.1, 4.9, 1.0 Hz, 1H), 7.39 (d, *J* = 8.8 Hz, 1H), 7.32 (d, *J = 2.3* Hz, 1H), 7.26 (s, 2H), 7.20 (m, 2H), 5.34 (s, 1H), 4.81 (s, 1H), 4.51 (s, 2H), 3.21 (s, 3H). LC-MS: [M+H]⁺ Calcd 451.07, 453.07 Found 451.1, 453.1. |

### Example 125 Preparation of Compound 287-Compound 296

### Preparation of Compound 287-Compound 290

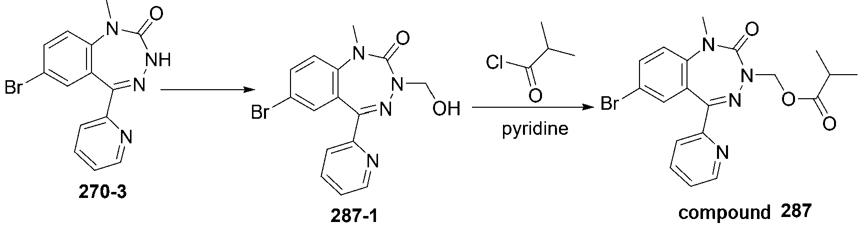

Compound **270-3** (329 mg, 1 mmol) was dissolved in ethanol (3 mL). Formaldehyde aqueous solution (30%, 1 mL) was added, and the mixture was sstirred at 90°C in sealed tube for 18 hours. The mixture was concentrated, and the residue was partitioned between dichloromethane and saturated ammonium chloride solution. The organic layer was separated, and the combined organic phase were dried over anhydrous sodium sulfate, concentrated, and purified by silica gel column chromatography (petroleum ether/ethyl acetate = 1/1) to yield Compound **287-1** as off-yellow (305 mg, yield 84.7%).

Unde nitrogen atmosphere, Compound **287-1** (50 mg, 0.15 mmol) and pyridine (158 mg, 2 mmol) were dissolved in dry dichloromethane (2 mL). The mixture was cooled in ice-water bath, and isobutyryl chloride (22 mg, 0.2 mmol) was added dropwise. The mixture was stirred at room temperature for 2 hours, the mixture was concentrated under reduced pressure. The residue was partitioned between ethyl acetate and saturated brine. The aqueous layer was extracted with ethyl acetate three times, and the combined organic layers were dried over anhydrous sodium sulfate, concentrated, and purified by silica gel column chromatography (petroleum ether/ethyl acetate = 3/1-2/1) to yield Compound **287.**

Following the procedure described above, Compound **287-5** (50 mg, 0.15 mmol) was separately reacted with the corresponding acid chlorides-chloroacetyl chloride (23.0 mg, 0.2 mmol), isopropoxycarbonyl chloride (91.5 mg, 0.75 mmol) and 3-chloropropionyl chloride (25.4 mg, 0.2 mmol) to yield Compound **288-** Compound **290.**

### Preparation of Compound 291-Compound 296

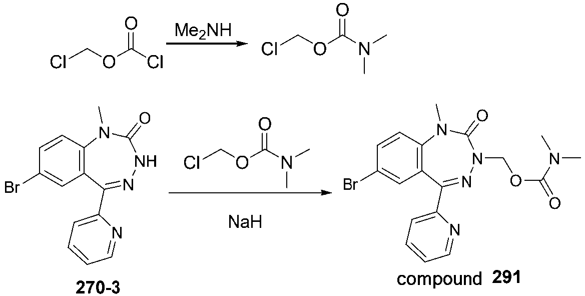

Under nitrogen atmosphere, solution of chloromethyl chloroformate (129 mg, 1 mmol) in dry tetrahydrofuran (5 mL), the mixture was stirred at -20°C, solution of dimethylamine in tetrahydrofuran (1 M, 2 mL, 2 mmol) was added dropwise. The mixture was stirred at room temperature, then the mixture was concentrated under reduced pressure the residue. The residue was partitioned between methyl tert-butyl ether and water. The combined organic layers were dried over anhydrous sodium sulfate and concentrated to yield (chloromethoxy)carbonyl dimethylamine (74 mg, yield 53.8%).

Under nitrogen atmosphere, solution of Compound **270-3** (66 mg, 0.2 mmol) in dry DMF was cooled to 0°C. Sodium hydride (16 mg, 0.4 mmol) was added, and the mixture was stirred for 30 minutes. The mixture was stirried for 1hour, (chloromethoxy)carbonyl dimethylamine (30 mg, 0.22 mmol) was added. The mixture was stirred for 2 hours and then quenched with saturated ammonium chloride solution, and then extracted with ethyl acetate. The combined organic layers were dried over anhydrous sodium sulfate, concentrated, and purified by silica gel column chromatography (petroleum ether/ethyl acetate = 2/1-1/1) to yield Compound **291** (58.6 mg, yield 67.8%).

Following the procedure described above, tetrahydrofuran solution of chloromethyl chloroformate (129 mg, 1 mmol) was added with methyl ethylamine (118 mg, 2 mmol), methyl isopropylamine (146 mg, 2 mmol), dimethylhydroxylamine (195 mg, 2 mmol), pyrrole (142 mg, 2 mmol), and ethyl isopropylamine (174 mg, 2 mmol) respectively to synthesize the corresponding chloromethoxyformamides. 66 mg of Compound **270-4** was alkylated with each respective chloromethoxyformamide, to yield Compound **292** (67.8 mg, yield 76.0%), Compound **293** (66.1 mg, yield 71.8%), Compound **294** (22.4 mg, yield 25.0%), Compound **295** (67.6 mg, 73.8% yield), and Compound **296** (16.0 mg, yield 16.9%).

| **Compound NO.** | **Compound structure** | **Characterization** |
|---|---|---|
| 5.1 | 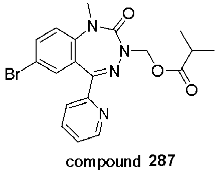 | white solid, ¹H NMR (400 MHz, DMSO-*d6*) δ 8.62 (d, *J = 4.7* Hz, 1H), 8.00 (d, *J* = 3.5 Hz, 2H), 7.78 (dd, *J =* 8.8, 2.4 Hz, 1H), 7.54 (dd, *J* = 9.0, 4.6 Hz, 1H), 7.37 (dd, *J =* 19.0, 5.6 Hz, 2H), 5.58 (s, 2H), 3.21 (s, 3H), 2.55 (m, 1H), 1.03 (d, *J* = 6.9 Hz, 6H). LC-MS: [M+Na]⁺ Calcd 453.05, 455.05, Found 453.0, 455.0. |
| 5.2 | 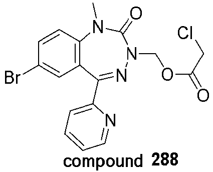 | white solid, ¹H NMR (400 MHz, DMSO-*d6*) δ 8.64 (d, *J* = 4.7 Hz, 1H), 8.02 (m, 2H), 7.81 (dd, *J* = 8.8, 2.4 Hz, 1H), 7.57 (ddd, *J* = 6.8, 4.8, 2.0 Hz, 1H), 7.42 (d, *J =* 8.9 Hz, 1H), 7.37 (d, *J* = 2.4 Hz, 1H), 5.69 (s, 2H), 4.46 (s, 2H), 3.24 (s, 3H). LC-MS: [M+Na]⁺ Calcd 458.98, 460.98, Found 459.0, 461.0. |
| 5.3 | 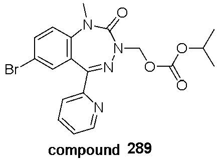 | white solid, ¹H NMR (400 MHz, DMSO-*d6*) δ 8.64 (ddd, *J* = 4.8, 1.5, 1.0 Hz, 1H), 8.04 (d, *J* = 7.5 Hz, 1H), 8.00 (m, 1H), 7.81 (dd, *J* = 8.8, 2.4 Hz, 1H), 7.56 (ddd, *J* = 7.1, 4.8, 1.6 Hz, 1H), 7.42 (d, *J* = 8.9 Hz, 1H), 7.39 (d, *J =* 2.4 Hz, 1H), 5.60 (s, 2H), 4.79 (m, 1H), 3.24 (s, 3H), 1.21 (d, *J* = 6.2 Hz, 6H). LC-MS: [M+ H]⁺ Calcd 469.05, 471.05, Found 469.0, 471.0. |
| 5.4 | 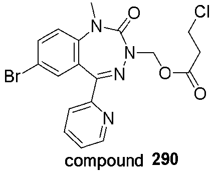 | white solid, ¹H NMR (400 MHz, DMSO-*d6*) δ 8.64 (d, *J = 4.8* Hz, 1H), 8.06 (d, *J = 7.9* Hz, 1H), 8.00 (td, *J* = 7.7, 1.7 Hz, 1H), 7.81 (dd, *J* = 8.8, 2.4 Hz, 1H), 7.57 (m, 1H), 7.42 (d, *J* = 8.9 Hz, 1H), 7.37 (d, *J* = 2.3 Hz, 1H), 5.69 (s, 2H), 3.80 (t, *J =* 6.0 Hz, 2H), 3.24 (s, 3H), 2.87 (t, *J =* 6.1 Hz, 2H). LC-MS: [M+ Na⁺ Calcd 473.00, 475.00, Found 473.0, 475.0. |
| 5.5 | 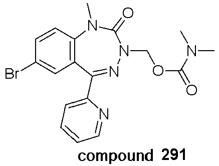 | white solid, ¹H NMR (400 MHz, DMSO-*d6*) δ 8.64 (d, *J* = 4.7 Hz, 1H), 8.02 (m, 2H), 7.81 (dd, *J* = 8.8, 2.4 Hz, 1H), 7.56 (ddd, *J* = 6.8, 4.8, 2.0 Hz, 1H), 7.42 (d, *J* = 8.9 Hz, 1H), 7.38 (d, *J* = 2.3 Hz, 1H), 5.65 (s, 1H), 5.50 (s, 1H), 3.24 (s, 3H), 2.83 (s, 6H). LC-MS: [M+H]⁺ Calcd 432.06, 434.06, Found 432.1, 434.1. |
| 5.6 | 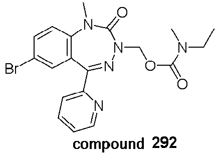 | white solid, ¹H NMR (400 MHz, DMSO-*d6*) δ 8.64 (d, *J* = 4.7 Hz, 1H), 8.02 (m, 2H), 7.80 (dd, *J* = 8.8, 2.4 Hz, 1H), 7.56 (ddd, *J* = 6.8, 4.8, 1.6 Hz, 1H), 7.42 (d, *J* = 8.8 Hz, 1H), 7.37 (s, 1H), 5.69 (brs, 1H), 5.46 (brs, 1H), 3.23 (brs, 5H), 2.80 (s, 3H), 0.97 (m, 3H). LC-MS: [M+H]⁺ Calcd 446.07, 448.07, Found 446.1, 448.1. |
| 5.7 | 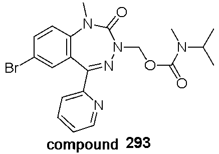 | white solid, ¹H NMR (400 MHz, DMSO-*d6*) δ 8.64 (d, *J = 4.7* Hz, 1H), 8.02 (m, 2H), 7.80 (dd, *J* = 8.8, 2.4 Hz, 1H), 7.56 (m, 1H), 7.42 (d, *J* = 8.9 Hz, 1H), 7.37 (s, 1H), 5.69 (brs, 1H), 5.47 (brs, 1H), 4.20 (d, *J =* 47.2 Hz, 1H), 3.23 (brs, 3H), 2.66 (brs, 3H), 1.02 (brs, 6H). LC-MS: [M+H]⁺ Calcd 460.09, 462.09, Found 460.2, 462.2. |
| 5.8 | 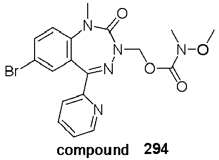 | white solid, ¹H NMR (400 MHz, DMSO-*d6*) δ 8.64 (d, *J* = 4.5 Hz, 1H), 8.05 (d, *J =* 7.7 Hz, 1H), 8.01 (td, *J =* 8.0, 1.2 Hz, 1H), 7.81 (dd, *J* = 8.8, 2.4 Hz, 1H), 7.56 (m, 1H), 7.42 (d, *J =* 8.9 Hz, 1H), 7.38 (d, *J* = 2.4 Hz, 1H), 5.61 (s, 2H), 3.56 (s, 3H), 3.24 (s, 3H). LC-MS: [M+Na]⁺ Calcd 470.04, 472.04, Found 470.1, 472.1. |
| 5.9 | 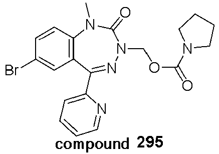 | white solid, ¹H NMR (400 MHz, DMSO-*d6*) δ 8.64 (d, *J =* 4.6 Hz, 1H), 8.01 (m, 2H), 7.80 (dd, *J* = 8.8, 2.4 Hz, 1H), 7.56 (ddd, *J* = 6.8, 4.9, 2.0 Hz, 1H), 7.41 (d, *J =* 8.9 Hz, 1H), 7.38 (d, *J* = 2.4 Hz, 1H), 5.69 (s, 1H), 5.47 (s, 1H), 3.25 (m, 7H), 1.77 (brs, 4H). LC-MS: [M+H]⁺ Calcd 458.07, 460.07, Found 458.2, 460.2. |
| 5.10 | 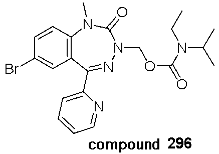 | white solid, ¹H NMR (400 MHz, DMSO-*d6*) δ 8.64 (d, *J = 4.8* Hz, 1H), 8.06 (d, *J = 7.9* Hz, 1H), 8.00 (td, *J* = 7.7, 1.6 Hz, 1H), 7.80 (dd, *J* = 8.8, 2.4 Hz, 1H), 7.56 (ddd, *J* = 7.3, 4.8, 1.2 Hz, 1H), 7.42 (d, *J =* 8.9 Hz, 1H), 7.34 (d, *J =* 2.3 Hz, 1H), 5.73 (brs, 1H), 5.44 (brs, 1H), 4.07 (brs, *J* = 58.6 Hz, 1H), 3.22 (s, 3H), 3.08 (brs, 2H), 1.04 (d, *J* = 5.9 Hz, 6H), 0.97 (brs, 3H). LC-MS: [M+H]⁺ Calcd 474.11, 476.11, Found 474.2, 476.2. |
| 5.11 | 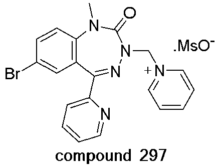 | yellow solid, ¹H NMR (400 MHz, DMSO-*d6*) δ 9.34 (d, *J =* 5.6 Hz, 2H), 8.97 (d, *J =* 5.1 Hz, 0H), 8.71 (t, *J =* 7.8 Hz, 1H), 8.65 (d, *J* = 4.1 Hz, 1H), 8.23 (m, 2H), 8.16 (d, *J* = 7.9 Hz, 1H), 8.04 (td, *J* = 7.8, 1.7 Hz, 1H), 7.86 (dd, *J* = 8.8, 2.4 Hz, 1H), 7.60 (m, 1H), 7.48 (d, *J* = 8.9 Hz, 1H), 7.35 (d, *J* = 2.3 Hz, 1H), 6.33 (s, 2H), 3.26 (s, 3H), 2.37 (s, 3H). LC-MS: [M]⁺ Calcd 422.06, 424.06 Found 422.0, 424.0. |

### Example 126 Preparation of Compound 298-Compound 305

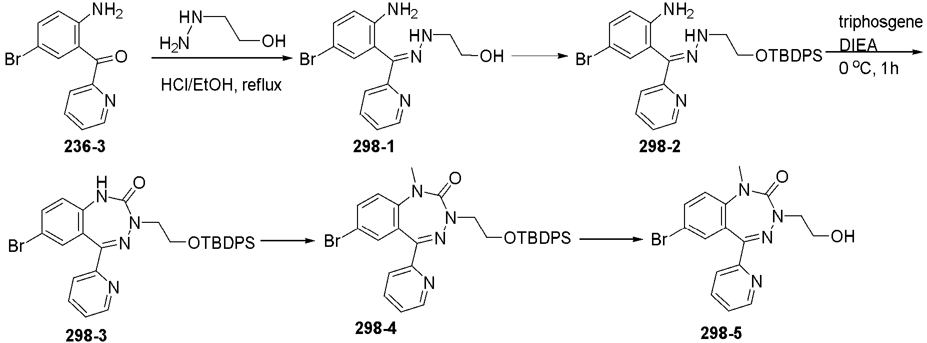

### Preparation of Compound 298-1

A solution of Compound **1-3** (13.85 g, 0.05 mol, 1.0 equiv) and 2-hydrazinylethanol (4.56 g, 0.06 mol, 1.2 equiv) in ethanol (250 mL) was adjusted to pH 4-5 using hydrogen chloride solution in ethanol. The mixture was stirred at 85°C in sealed tube for 6 hours. The mixture was concentrated to give the residue, the residue was partitioned between ethyl acetate and saturated sodium bicarbonate solution. The mixture was extracted with ethyl acetate for three times. The combined organic layers were dried over anhydrous sodium sulfate, concentrated, and purified by silica gel column chromatography (petroleum ether/ethyl acetate = 1/1-1/4) to yield Compound **298-1** as yellow syrup (12.1 g, yield 72.4%).

### Preparation of Compound 298-2

Under nitrogen atmosphere, Compound **298-1** (12.1 g, 36.2 mmol, 1.0 equiv) and imidazole (4.7 g, 72.4 mmol, 2.0 equiv) were dissolved in dry dichloromethane (150 mL), then the mixture was stirred at 0°C. Diisopropylphenylchlorosilane (11.9 g, 43.5 mmol, 1.2 equiv) was then added dropwise. The mixture was stirred at room temperature for 2 hours. The mixture was concentrated to give the residue, and the residue was partitioned between ethyl acetate and saturated brine. The organic layer was extracted with ethyl acetate for three times. The combined organic layers were dried over anhydrous sodium sulfate, concentrated, and purified by silica gel column chromatography (petroleum ether/ethyl acetate = 3/1-2/1) to yield Compound **298-2** as yellow syrup (17.1 g, yield 82.3%).

### Preparation of Compound 298-3

Unde nitrogen atmosphere, solution of Compound **298-2** (17.1 g, 29.8 mmol, 1.0 equiv) and DIPEA (15.4 g, 119.2 mmol, 4.0 equiv) in dry dichloromethane (150 mL),then the mixture was cooled to 0°C. The solution of triphosgene (3.5 g, 11.9 mmol, 0.4 equiv) in dry dichloromethane (30 mL) was added dropwise slowly. After confirming reaction completion by TLC. The reaction was quenched with saturated ammonium chloride solution and stirred at room temperature. The mixture was extracted with dichloromethane. The combined organic layers were dried over anhydrous sodium sulfate, concentrated, and purified by silica gel column chromatography (petroleum ether/ethyl acetate = 4/1-3/1) to yield Compound **298-3** as off-white solid (16.2 g, yield 90.7%).

### Preparation of Compound 298-4

Under nitrogen atmosphere, Compound **298-3** (16.2 g, 27.0 mmol) was dissolved in dry DMF (10 mL) and cooled to 0°C. Iodomethane (4.6 g, 32 mmol) and sodium hydride (1.28 g, 32 mmol) were added sequentially. The mixture was stirred in ice-bath for 1 hour, then stirred at room temperature for 2 hours. The mixture was quenched with saturated ammonium chloride solution and partitioned between ethyl acetate and water. The combined organic layers were washed with brine fot three times, dried over anhydrous sodium sulfate, concentrated, and purified by silica gel column chromatography (petroleum ether/ethyl acetate = 2/1) to yield Compound **1.1** as yellow solid (14.7 g, yield 90.5%).

### Preparation of Compound 298-5

The solution of tetrabutylammonium fluoride in THF (1 M, 29.7 mL, 29.7 mmol, 1.2 equiv) was added dropwise to solution of Compound **298-4** (14.7 g, 24.6 mmol, 1.0 equiv) in THF (100 mL). The mixture was stirred at room temperature for 2 hours, After confirming reaction completion by TLC. The mixture was concentrated and purified by silica gel column chromatography (petroleum ether/ethyl acetate = 1/1-1/2) to yield Compound **298-5** as off-white solid (8.55 g, yield 87.7%).

### Preparation of Compound 298- Compound 302

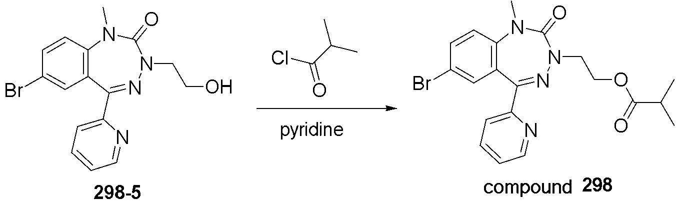

Under nitrogen atmosphere, Compound **298-5** (75 mg, 0.2 mmol) and pyridine (47 mg, 0.6 mmol) were dissolved in dry dichloromethane (2 mL). The mixture was stirred in ice-bath, and isobutyryl chloride (43 mg, 0.4 mmol) was added dropwise. The mixture was stirred at room temperature for 2 hours, the mixture was concentrated under reduced pressure. The residue was partitioned between ethyl acetate and saturated brine, and the aqueous layer was extracted with ethyl acetate for three times. The combined organic layers were dried over anhydrous sodium sulfate, concentrated, and purified by silica gel column chromatography (petroleum ether/ethyl acetate = 3/1-2/1) to yield Compound **298** (38 mg, yield 42.8%).

Following the procedure described above, Compound **298-6** (75 mg, 0.2 mmol) was separately mixed with the corresponding β-chloropropionyl chloride (50.8 mg, 0.4 mmol), α-chloroacetyl chloride (46.0 mg, 0.4 mmol), allyl chloride (18.0 mg, 0.4 mmol), and isopropoxymethyl chloride (61.0 mg, 0.5 mmol). The mixture was stirred at room temperature for 2 hours, the mixture was concentrated under reduced pressure. Ethyl acetate and saturated brine were added for liquid-liquid separation. The aqueous layer was extracted with ethyl acetate for three times, and the combined organic layers were dried over anhydrous sodium sulfate, concentrated, and purified by silica gel column chromatography (petroleum ether/ethyl acetate = 3/1-2/1) to yield the corresponding Compound **299** (45.0 mg, yield 48.5%), Compound **300** (41.3 mg, yield 45.9%), Compound **301** (15.3 mg, yield 17.8%) and Compound **302** (26.4 mg, yield 28.7%.

### Preparation of Compound 303

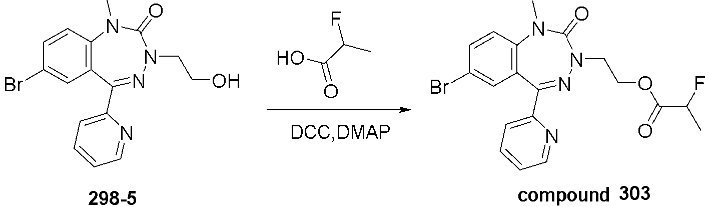

Under nitrogen atmosphere, Compound **298-6** (75 mg, 0.2 mmol) and α-fluoropropanoic acid (22.1 mg, 0.24 mmol) were dissolved in dry dichloromethane. DCC (59.1 mg, 0.288 mmol) and DMAP (3 mg, 0.04 mmol) were added in ice-bath. The mixture was stirred at room temperature for 16 hours. The mixture was concentrated under reduced pressure to give the crude product, the crude product was suspended in ethyl acetate, filtered. The filtrate was concentrated and purified by silica gel column chromatography (petroleum ether/ethyl acetate = 3/1-2/1) to yield Compound **303** (48.0 mg, 53.4%).

### Preparation of Compound 304

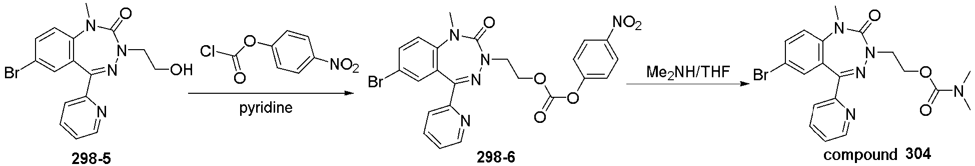

Under nitrogen atmosphere, Compound **298-5** (75 mg, 0.2 mmol) and pyridine (75 mg, 0.2 mmol) were dissolved in dry dichloromethane. Phenyl chloroformate (1.2 equivalents) was added in ice-bath, and the mixture was stirred at room temperature for 2 hours, then the mixture was concentrated to give the crude product, ethyl acetate was added to the crude product, and washed with dilute hydrochloric acid, the organic layers were combined, concentrated, and purified by silica gel column chromatography (petroleum ether/ethyl acetate = 4/1-3/1) to yield Compound **298-6.** Compound **298-6-5** was dissolved in dry tetrahydrofuran, and tetrahydrofuran solution of dimethylamine (2 equivalents) was added. The mixture was stirred at room temperature for 3 hours, the mixture was concentrated and purified by silica gel column chromatography (petroleum ether/ethyl acetate = 2/1-1/1) to yield Compound **304** (10.2 mg, yield 11.4%).

| **Compound NO.** | **Compound structure** | **Characterization** |
|---|---|---|
| 6.1 | 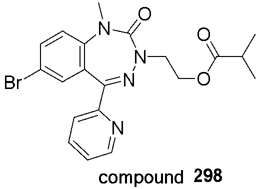 | Colorless Syrup , ¹H NMR (400 MHz, DMSO-*d6*) δ 8.61 (ddd, *J =* 4.8, 1.7, 0.9 Hz, 1H), 8.08 (dt, *J* = 7.9, 1.0 Hz, 1H), 7.98 (td, *J* = 7.7, 1.8 Hz, 1H), 7.74 (dd, *J* = 8.8, 2.4 Hz, 1H), 7.53 (ddd, *J* = 7.5, 4.8, 1.2 Hz, 1H), 7.34 (d, *J =* 8.8 Hz, 1H), 7.24 (d, *J* = 2.4 Hz, 1H), 4.44 (brs, 1H), 4.12 (brs, 1H), 4.02 (brs, 1H), 3.60 (brs, 1H), 3.17 (s, 3H), 2.28 (dt, *J =* 13.9, 7.0 Hz, 1H), 0.77 (brs, 6H). LC-MS: [M+H] ⁺ Calcd 445.08.10, 447.08, Found 445.0, 447.0. |
| 6.2 | 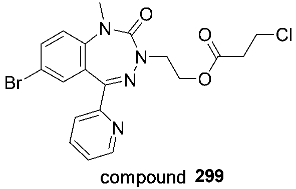 | Colorless Syrup , ¹H NMR (400 MHz, DMSO-*d6*) δ 8.63 (d, *J* = 4.1 Hz, 1H), 8.09 (d, *J* = 7.9 Hz, 1H), 8.00 (td, *J* = 7.8, 1.7 Hz, 1H), 7.77 (dd, *J* = 8.8, 2.4 Hz, 1H), 7.55 (ddd, *J* = 7.4, 4.8, 1.0 Hz, 1H), 7.36 (d, *J* = 8.9 Hz, 1H), 7.30 (d, *J* = 2.3 Hz, 1H), 4.42 (s, 1H), 4.30 (s, 1H), 4.01 (s, 1H), 3.67 (s, 1H), 3.59 (t, *J* = 6.3 Hz, 2H), 3.20 (s, 3H), 2.61 (s, 2H). LC-MS: [M+H] ⁺ Calcd 465.03, 467.03, Found 465.0, 467.1. |
| 6.3 | 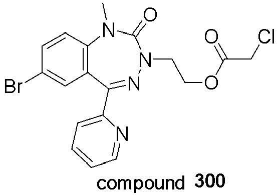 | Colorless Syrup , ¹H NMR (400 MHz, DMSO-*d6*) δ 8.63 (d, *J* = 4.2 Hz, 1H), 8.10 (d, *J* = 7.9 Hz, 1H), 8.00 (td, *J* = 7.8, 1.5 Hz, 1H), 7.77 (dd, *J* = 8.8, 2.3 Hz, 1H), 7.55 (dd, *J =* 6.5, 4.9 Hz, 1H), 7.36 (d, *J* = 8.8 Hz, 1H), 7.31 (d, *J* = 2.3 Hz, 1H), 4.45 (s, 1H), 4.40 (s, 1H), 4.20 (s, 2H), 3.99 (s, 1H), 3.73 (s, 1H), 3.20 (s, 3H). LC-MS: [M+H]⁺ Calcd 451.01.10, 453.01, Found 451.0, 453.0. |
| 6.4 | 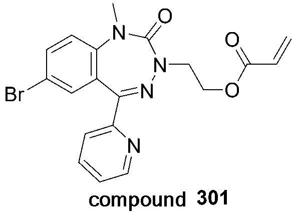 | Yellow Syrup, ¹H NMR (400 MHz, DMSO-*d6*) δ 8.63 (d, *J =* 4.7 Hz, 1H), 8.10 (d, *J =* 7.9 Hz, 1H), 8.00 (td, *J* = 7.8, 1.7 Hz, 1H), 7.75 (dd, *J* = 8.8, 2.4 Hz, 1H), 7.55 (dd, *J* = 6.9, 5.4 Hz, 1H), 7.35 (d, *J* = 8.9 Hz, 1H), 7.28 (d, *J* = 2.3 Hz, 1H), 6.12 (dd, *J* = 17.2, 1.8 Hz, 1H), 6.01 (dd, *J* = 17.2, 10.1 Hz, 1H), 5.81 (dd, *J* = 10.1, 1.8 Hz, 1H), 4.47 (s, 1H), 4.33 (s, 1H), 4.04 (s, 1H), 3.71 (s, 1H), 3.19 (s, 3H). LC-MS: [M+H]⁺ Calcd 429.05.10, 431.05, Found 429.1, 431.1. |
| 6.5 | 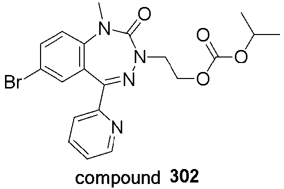 | Colorless Syrup , ¹H NMR (400 MHz, DMSO-*d6*) δ 8.61 (ddd, *J =* 4.8, 1.7, 0.9 Hz, 1H), 8.08 (dt, *J* = 8.0, 1.0 Hz, 1H), 7.98 (td, *J* = 7.7, 1.8 Hz, 1H), 7.75 (dd, *J =* 8.8, 2.4 Hz, 1H), 7.53 (ddd, *J* = 7.5, 4.8, 1.2 Hz, 1H), 7.35 (d, *J =* 8.9 Hz, 1H), 7.24 (d, *J* = 2.4 Hz, 1H), 4.48 (dt, *J* = 12.5, 6.3 Hz, 1H), 4.45 (brs, 1H), 4.26 (brs, 1H), 3.99 (brs, 1H), 3.62 (brs, 0H), 3.18 (s, 3H), 1.04 (brs, 6H). LC-MS: [M+H]⁺ Calcd 461.07, 463.07, Found 461.1, 463.1. |
| 6.6 | 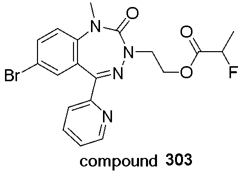 | white solid, ¹H NMR (400 MHz, DMSO-*d6*) δ 8.63 (d, *J* = 4.0 Hz, 1H), 8.11 (d, *J* = 8.0 Hz, 1H), 8.00 (td, *J* = 7.8, 1.7 Hz, 1H), 7.77 (dd, *J* = 8.8, 2.4 Hz, 1H), 7.55 (ddd, *J* = 7.5, 4.9, 1.1 Hz, 1H), 7.37 (d, *J* = 8.9 Hz, 1H), 7.29 (d, *J* = 2.3 Hz, 1H), 5.00 (dq, *J* = 47.9, 6.7 Hz, 1H), 4.48 (brs, 2H), 4.04 (brs, 1H), 3.68 (brs, 1H), 3.20 (s, 3H), 1.12 (d, *J* = 23.0 Hz, 3H). [M+H]⁺ Calcd 449.05, 451.05, Found 449.1, 451.1. |
| 6.7 | 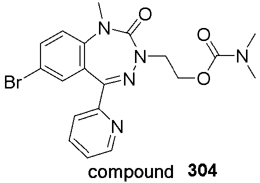 | Off-yellow solid , ¹H NMR (400 MHz, DMSO-*d6*) δ 8.61 (ddd, *J =* 4.8, 1.7, 0.9 Hz, 3H), 8.61 (ddd, *J* = 4.8, 1.7, 0.9 Hz, 3H), 8.06 (dt, *J* = 7.9, 1.0 Hz, 3H), 8.06 (dt, *J* = 7.9, 1.0 Hz, 3H), 7.98 (td, *J* = 7.7, 1.8 Hz, 3H), 7.98 (td, *J* = 7.7, 1.8 Hz, 3H), 7.75 (dd, *J* = 8.8, 2.4 Hz, 3H), 7.53 (ddd, *J* = 7.5, 4.8, 1.2 Hz, 3H), 7.31 (dd, *J =* 24.5, 5.6 Hz, 6H), 4.21 (s, 3H), 4.00 (brs, 1H), 3.64 (brs, 1H), 3.17 (s, 10H), 2.64 (s, 5H), 2.55 (s, 9H). LC-MS: [M+H]⁺ Calcd 446.07, 448.07, Found 446.0, 448.0. |

### Example 127 Preparation of Compound 305

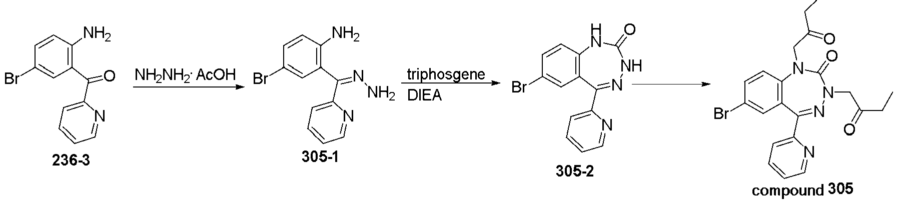

### Preparation of Compound 305-1

Compound **236-3** (2.21 g, 8.07 mmol, 1 eq) and hydrazine acetate (606.9 mg, 8.86 mmol, 1.1 eq) were dissolved in 50 mL of ethanol, the mixture was stirred at 85 °C for 16 hours in sealed tube. The mixture was quenched with 1 mL of triethylamine, concentrated under reduced pressure, and purified by silica gel column chromatography (petroleum ether/ethyl acetate = 2/1-1:1) to yield Compound **305-1** as yellow solid(1.77 g, yield 75.4%).

### Preparation of Compound 305-2

Under nitrogen atmosphere, solution of Compound **5-2** (1.77 g, 5.78 mmol) and DIPEA (3.96 g, 30.67 mmol) in 100 mL of dry dichloromethane, the mixture was cooled to 0°C. A solution of triphosgene (687 mg, 2.31 mmol, 0.4 eq) in dry dichloromethane (10 mL) was added dropwise slowly. After confirming reaction completion by TLC, the mixture was quenched with saturated aqueous ammonium chloride at room temperature. The mixture was extracted with dichloromethane. The combined organic layers were dried over anhydrous sodium sulfate, concentrated, and purified by silica gel column chromatography (petroleum ether/ethyl acetate = 1/1) to yield Compound **305-2 as** yellow solid (1.43 g, yield 74.7%).

Under nitrogen atmosphere, solution of Compound **5-2** (1.77 g, 5.78 mmol) and DIPEA (3.96 g, 30.67 mmol) in dry dichloromethane (100 mL), the mixture was cooled to 0°C. A solution of triphosgene (687 mg, 2.31 mmol, 0.4 eq) in dry dichloromethane (10 mL) was added dropwise slowly. After confirming reaction completion by TLC, saturated aqueous ammonium chloride was added, and the mixture was stirred at room temperature. The mixture was extracted with dichloromethane. The combined organic layers were dried over anhydrous sodium sulfate, concentrated under reduced pressure, and purified by silica gel column chromatography (petroleum ether/ethyl acetate = 1/1) to yield Compound **305-2** as yellow solid(1.43 g, yield 74.7%).

Compound **305-2** (32 mg, 0.1 mmol) was dissolved in dry DMF. 1-Bromo-2-butanone (46 mg, 0.3 mmol) and potassium carbonate (138 mg, 1 mmol) were added. The mixture was stirred at room temperature for 16 hours. After confirming reaction completion by TLC, ethyl acetate and water were added. The mixture was extracted with ethyl acetate. The combined organic layers were washed with brine, concentrated under reduced pressure, and purified by silica gel column chromatography (petroleum ether/ethyl acetate = 3/1) to yield Compound **305** (21 mg, yield 23.6%).

| **Compound NO.** | **Compound structure** | **Characterization** |
|---|---|---|
| 7 | 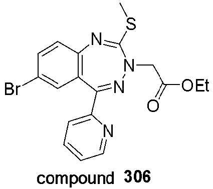 | white solid, ¹H NMR (400 MHz, DMSO-*d6*) δ 8.62 (d, *J* = 4.2 Hz, 1H), 7.95 (m, 2H), 7.74 (dd, *J* = 8.9, 2.4 Hz, 1H), 7.53 (m, 1H), 7.33 (d, *J* = 2.3 Hz, 1H), 7.19 (d, *J* = 8.9 Hz, 1H), 4.69 (s, 2H), 3.31 (s, 2H), 2.70 (q, *J* = 7.6 Hz, 2H), 2.50 (q, *J* = 7.6 Hz, 2H),1.02 (d, *J* = 7.8 Hz, 6H). LC-MS: [M+H]⁺ Calcd 457.08, 459.08, Found 457.1, 459.1. |

### Example 128 Preparation of Compound 306-Compound 317

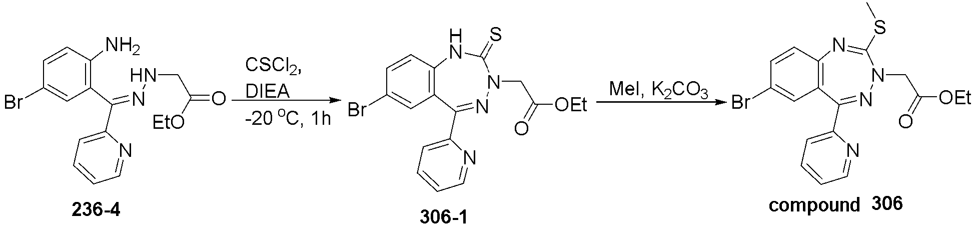

Under nitrogen atmosphere, solution of Compound **236-4** (5.74 g, 10 mmol, 1 eq) and DIPEA (3.9 g, 30 mmol, 3 eq) in dry dichloromethane (100 mL), the mixture was stirred at 0°C. A solution of thiophosgene (1.38 g, 12 mmol, 1.2 eq) in dry dichloromethane (50 mL) was added dropwise slowly. After confirming reaction completion by TLC, saturated aqueous ammonium chloride was added, and the mixture was stirred at room temperature. The mixture was extracted with dichloromethane. The combined organic layers were dried over anhydrous sodium sulfate, concentrated under reduced pressure, and purified by silica gel column chromatography (petroleum ether/ethyl acetate = 5/1-3/1) to yield Compound **306-1** as yellow solid (3.54 g, yield 57.5%).

Compound **306-1** (3.54 g, 5.8 mmol, 1 eq) and MeI (1.6 g, 11.5 mmol, 2 eq) were dissolved in dry N,N-dimethylformamide (25 mL). Potassium carbonate (952.2 mg, 6.9 mmol, 1.2 eq) was added, and the mixture was stirred at room temperature for 6 hours. After confirming reaction completion by TLC, water (80 mL) and ethyl acetate (50 mL) were added, and the mixture was extracted with ethyl acetat. The combined organic layers were washed with brine, dried over anhydrous sodium sulfate, concentrated under reduced pressure, and purified by silica gel column chromatography (petroleum ether/ethyl acetate = 4/1) to yield Compound **306** as yellow solid (3.01 g, yield 82.4%).

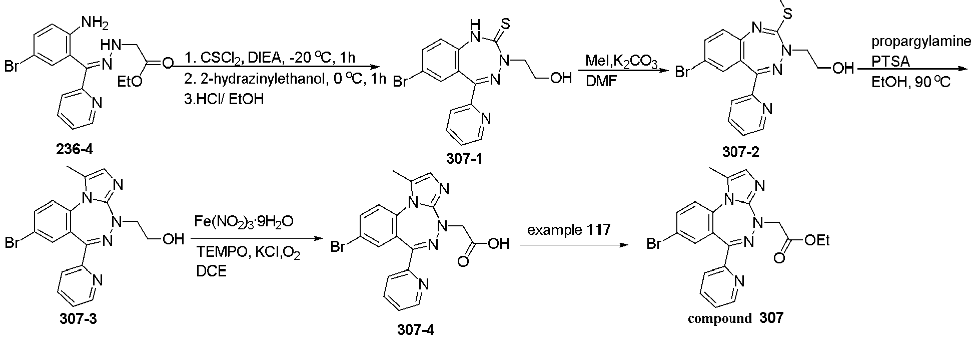

Under nitrogen atmosphere, solution of Compound **236-4** (13.85 g, 0.05 mol, 1 eq) and disopropylethylamine (16.15 g, 125 mmol, 2.5 eq) in dry dichloromethane (250 mL), the mixture was stirred at 0°C. A solution of thiophosgene (6.90 g, 0.06 mol, 1.2 eq) in dry dichloromethane (20 mL) was added dropwise. The mixture was stirred at room temperature for 30 minutes, After confirming reaction completion by TLC, the solution of 2-hydrazinoethanol (4.60 g, 0.06 mol, 1.2 eq) in ethanol (100 mL) was added dropwise to the mixture under ice-bath. The mixture was stirred at room temperature for 2 hours. The pH was adjusted to 3-4 using ethanolic HCl solution, and the mixture was refluxed for 2 hours. Then the pH was adjusted to 8-9 with saturated sodium bicarbonate solution. The mixture was concentrated under reduced pressure, and the residue was extracted with dichloromethane. The combined organic layers were dried over anhydrous sodium sulfate, concentrated, and purified by silica gel column chromatography (petroleum ether/ethyl acetate = 2/1-1/1) to yield Compound **307-1** as yellow solid (11.8 g, yield 62.6% ).

Compound **307-1** (11.80 g, 31.3 mmol, 1 eq) and MeI (5.33 g, 37.6 mmol, 1.2 eq) were dissolved in dry N,N-dimethylformamide (100 mL). Potassium carbonate (5.19 g, 37.6 mmol, 1.2 eq) was added, and the mixture was stirred at room temperature for 6 hours. After confirming reaction completion by TLC, 200 mL of water and 200 mL of ethyl acetate were added, and the mixture was extracted with ethyl acetate (twice). The combined organic layers were washed with brine, dried over anhydrous sodium sulfate, concentrated under reduced pressure, and purified by silica gel column chromatography (petroleum ether/ethyl acetate = 1/1) to yield Compound **307-2** as yellow solid (11.63 g, yield 94.9%).

Compound **307-2** (11.63 g, 29.7 mmol, 1 eq), propargylamine (3.27 g, 59.4 mmol, 5 eq) and p-toluenesulfonic acid (1.13 g, 5.94 mmol, 0.2 eq) were dissolved in ethanol (100 mL) , the mixture was stirred in sealed tube at 90°C for 16 hours. The mixture was concentrated under reduced pressure, the residue was purified by silica gel column chromatography (petroleum ether/ethyl acetate = 1/1 -100% ethyl acetate) to yield Compound **307-3** as yellow solid (6.8 g, yield 57.2%). Iron(III) nitrate nonahydrate (685 mg, 1.7 mmol), potassium chloride (127 mg, 1.7 mmol) and 2,2,6,6-tetramethylpiperidine 1-oxyl (265 mg, 1.7 mmol) were suspended in 1,2-dichloroethane. Under oxygen atmosphere, Compound **9-3** (6.8 g, 17.1 mmol) was added, and the mixture was stirred at room temperature for 24 hours. The mixture was filtered, and the filtrate was concentrated. The residue was recrystallized from petroleum ether/ethyl acetate(1/1) to yield Compound **307-4** as yellow solid (4.12 g, yield 60.4%).

The preparation method of compound **307-** compound **314** are similar in example **117,** using **307-4** (88 mg, 0.2 mmol) as starting materials.

Compound **308** (180 mg, 0.4 mmol) was dissolved in ethyl acetate (2 mL). A solution of hydrogen chloride in ethyl acetate (1 M, 0.5 mL) was added dropwise under ice-bath. The mixture was stirred for 2 hours under ice-bath to give the product. The product was filtered to yield Compound **308-1** as yellow solid (93.1 mg, yield 47.5%).

### Preparation of Compound 315-Compound 317

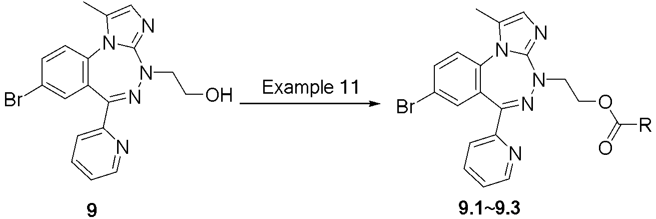

The preparation method of **Compound315-Compound317** is similar in example **126.**

Compound **315** (187 mg, 0.4 mmol) was dissolved in dry acetonitrile (4 mL). A solution of p-toluenesulfonic acid in ethanol (0.2 mL, 172 mg, 1.0 mmol) was added dropwise under ice-bath. The mixture was refluxed fo two hours, product was precipitated, filtered to yield Compound **315-1** as **yellow solid** (204 mg, yield 62.9%).

Compound **317** (186 mg, 0.4 mmol) was dissolved in dry acetonitrile (4 mL). The solution of p-toluenesulfonic acid in ethanol (0.2 mL, 172 mg, 1.0 mmol) was added dropwise under ice-bath. The mixture was refluxed for 2 hours, product was precipitated, filtered to yield Compound **317-1** as yellow solid (243 mg, yield 74.9%).

### Example 129 Preparation of Compound 318-Compound 320

### Preparation of Compound 318-4

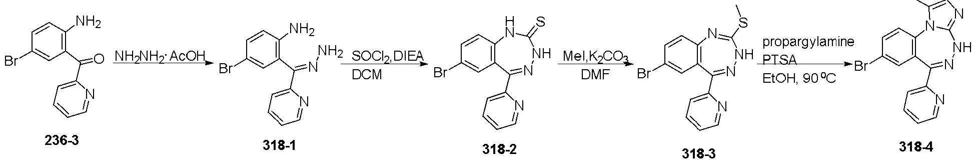

A mixture of Compound **236-3** (2.76 g, 10 mmol) and hydrazine hydrochloride (753.5 mg, 11 mmol) in ethanol (50 mL), the mixture was stirred at 85°C in sealed tube for 16 hours. The mixture was quenched with triethylamine (1 mL), and concentrated under reduced pressure. Purification by silica gel column chromatography (Petroleum ether/Ethyl acetate = 2/1-1/1) to yield Compound **318-1** as yellow solid (2.34 g, yield 79.9%).

Under nitrogen atmosphere, solution of Compound **10-1** (2.34 g, 8 mmol) and N,N-diisopropylethylamine (2.58 g, 20 mmol) in dry dichloromethane (250 mL), the mixture was cooled to 0°C. A solution of thiophosgene (1.1 g, 9.6 mmol) in dry dichloromethane (20 mL) was added dropwise. The mixture was stirred at room temperature for 30 minutes, after confirming reaction completion by TLC. The mixture was quenched with saturated aqueous ammonium chloride solution. The mixture was extracted with dichloromethane. The combined organic phase was dried over anhydrous sodium sulfate, concentrated under reduced pressure, and purified by silica gel column chromatography (Petroleum ether/Ethyl acetate = 1/1-1/5) to yield Compound **318-2** as yellow solid (1.71 g, yield 65.1%).

### Preparation of Compound 318-3

A mixture of Compound **318-2** (1.71 g, 5.2 mmol) and iodomethane (887 mg, 6.3 mmol) in dry N,N-dimethylformamide (25 mL) was treated with potassium carbonate (870 mg, 6.3 mmol). The mixture was stirred at room temperature for 6 hours. After confirming reaction completion by TLC, water (200 mL) and ethyl acetate (200 mL) were added. The mixture was extracted with ethyl acetate (2 × 200 mL). The combined organic phase was washed with brine, dried over anhydrous sodium sulfate, and concentrated under reduced pressure. Purification by silica gel column chromatography (Petroleum ether/Ethyl acetate = 1/1) to yield Compound **318-3** as yellow solid (1.67 g, yield 92.5%).

### Preparation of Compound 318-4

A mixture of Compound **318-3** (1.67 g, 4.8 mmol), prop-2-yn-1-amine (1.32 mg, 24 mmol) and p-toluenesulfonic acid (165 mg, 0.96 mmol) in ethanol (40 mL), the mixture was stirred at 90°C in sealed tube for 16 hours. Then the mixture was concentrated under reduced pressure, the residue was purified by silica gel column chromatography (Petroleum ether/Ethyl acetate = 1/1-100% Ethyl acetate) to yield Compound **318-4** as yellow solid (851 mg, yield 50.1%).

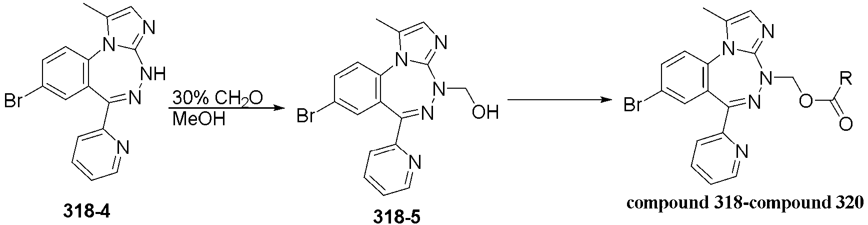

The preparation method of compound **318-320** are similar to example 125.

| **Compound NO.** | **Compound structure** | **Characterization** |
|---|---|---|
| 8 | 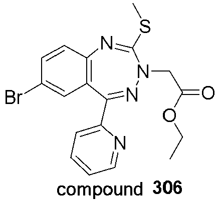 | yellow solid , ¹H NMR (400 MHz, DMSO-*d6*) δ 8.60 (ddd, *J* = 5.0, 1.8, 1.0 Hz, 1H), 7.94 (td, *J =* 7.7, 1.8 Hz, 1H), 7.75 (dt, *J* = 7.9, 1.0 Hz, 1H), 7.64 (m, 1H), 7.52 (m, 1H), 7.13 (d, *J =* 2.3 Hz, 1H), 7.05 (d, *J* = 8.6 Hz, 1H), 4.41 (s, 2H), 4.13 (q, *J* = 7.2 Hz, 2H), 3.30 (s, 3H), 1.18 (t, *J* = 7.1 Hz, 3H). LC-MS: [M+H]⁺ Calcd 433.03, 435.03, Found 433.0, 435.0. |
| 9.1 | 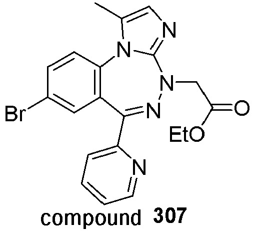 | yellow solid , ¹H NMR (400 MHz, DMSO-*d6*) δ 8.59 (d, *J* = 4.8 Hz, 1H), 7.95 (m, 2H), 7.88 (dd, *J* = 8.6, 2.0 Hz, 1H), 7.58 (d, *J* = 8.7 Hz, 1H), 7.53 (t, *J* = 5.7 Hz, 1H), 7.40 (d, *J* = 2.0 Hz, 1H), 6.71 (s, 1H), 4.68 (d, *J* = 16.2 Hz, 1H), 4.40 (d, *J* = 16.2 Hz, 1H), 4.13 (q, *J* = 7.1 Hz, 2H), 2.30 (s, 3H), 1.20 (t, *J* = 7.1 Hz, 3H). LC-MS: [M+H]⁺ Calcd 440.06, 442.06, Found 440.0, 442.0. |
| 9.2 | 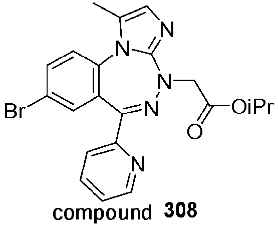 | yellow solid , ¹H NMR (400 MHz, DMSO-*d6*) δ 8.59 (d, *J* = 4.4 Hz, 1H), 7.96 (m, 2H), 7.88 (dd, *J* = 8.7, 2.3 Hz, 1H), 7.57 (d, *J* = 8.7 Hz, 1H), 7.53 (m, 1H), 7.40 (d, *J* = 2.3 Hz, 1H), 6.70 (d, *J* = 1.0 Hz, 1H), 4.94 (m, 1H), 4.63 (d, *J* = 16.1 Hz, 1H), 4.35 (d, *J* = 16.7 Hz, 1H), 2.30 (s, 3H), 1.20 (d, *J* = 6.2 Hz, 6H). LC-MS: [M+H]⁺ Calcd 454.08, 456.08, Found454.1, 456.1. |
| 9.2-1 | 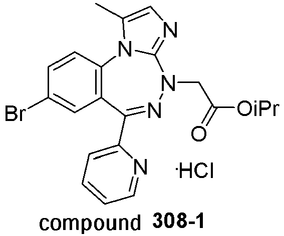 | yellow solid. ¹H NMR (400 MHz, DMSO-d6) δ 8.61 (d, J = 4.7 Hz, 1H), 8.00 (td, J = 7.7, 1.6 Hz, 1H), 7.91 (m, 2H), 7.61 (d, J = 8.7 Hz, 1H), 7.55 (ddd, J = 7.1, 4.9, 1.0 Hz, 1H), 7.44 (d, J = 2.2 Hz, 1H), 6.80 (s, 1H), 4.95 (m, 1H), 4.64 (d, J = 16.4 Hz, 1H), 4.39 (d, J = 16.4 Hz, 1H), 2.31 (s, 3H), 1.20 (d, J = 6.2 Hz, 6H). |
| 9.3 | 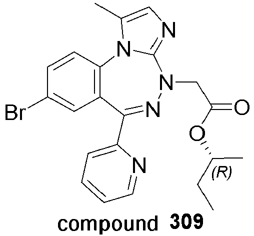 | yellow solid , ¹H NMR (400 MHz, DMSO-*d6*) δ 8.60 (d, *J =* 4.7 Hz, 1H), 7.96 (td, *J* = 7.6, 1.2 Hz, 1H), 7.94 (t, *J* = 7.6 Hz, 1H),7.88 (dd, *J =* 8.7, 2.1 Hz, 1H), 7.55 (m, 2H), 7.39 (s, 1H), 6.71 (s, 1H), 4.79 (m, 1H), 4.66 (d, *J =* 19.3 Hz, 1H), 4.38 (dd, *J* = 16.1, 9.6 Hz, 1H), 2.30 (s, 3H), 1.50 (dd, *J =* 14.1, 7.3 Hz, 2H), 1.16 (d, *J* = 6.3 Hz, 3H), 0.80 (t, *J* = 7.4 Hz, 3H). LC-MS: [M+H]⁺ Calcd 468.10, 470.09, Found 468.2, 470.2. |
| 9.4 | 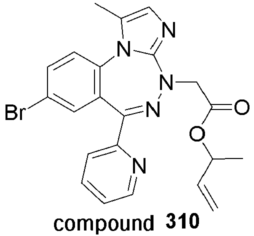 | yellow solid , ¹H NMR (400 MHz, DMSO-*d6*) δ 8.60 (d, *J* = 4.4 Hz, 1H), 7.97 (td, *J* = 7.6, 1.2 Hz, 1H), 7.92 (d, *J* = 7.8 Hz, 1H), 7.88 (dd, *J* = 8.7, 2.3 Hz, 1H), 7.58 (d, *J* = 8.7 Hz, 1H), 7.53 (ddd, *J* = 7.1, 4.9, 1.3 Hz, 1H), 7.40 (d, *J* = 2.2 Hz, 1H), 6.71 (d, *J* = 1.1 Hz, 1H), 5.87 (ddd, *J* = 17.3, 11.6, 5.8 Hz, 1H), 5.32 (m, 1H), 5.21 (dd, *J* = 17.8, 4.6 Hz, 1H), 5.12 (t, *J* = 8.7 Hz, 1H), 4.71 (dd, *J =* 16.0, 4.1 Hz, 1H), 4.42 (dd, *J* = 17.0, 4.8 Hz, 1H), 2.30 (s, 3H), 1.27 (d, *J* = 6.7 Hz, 3H). LC-MS: [M+H]⁺ Calcd 466.08, 468.08, Found 466.3, 468.3. |
| 9.5 | 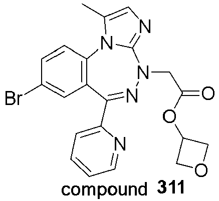 | yellow solid , ¹H NMR (400 MHz, DMSO-*d6*) δ 8.59 (m, 1H), 7.97 (dt, *J* = 16.1, 5.3 Hz, 2H), 7.87 (dd, *J =* 8.7, 2.3 Hz, 1H), 7.58 (d, *J* = 8.7 Hz, 1H), 7.53 (ddd, *J* = 6.7, 4.8, 1.7 Hz, 1H), 7.41 (d, *J =* 2.2 Hz, 1H), 6.72 (d, *J* = 1.0 Hz, 1H), 5.43 (m, 1H), 4.78 (dd, *J* = 18.9, 12.0 Hz, 3H), 4.49 (m, 3H), 2.30 (s, 3H). LC-MS: [M+H]⁺ Calcd 468.06, 470.06, Found 468.3, 470.3. |
| 9.6 | 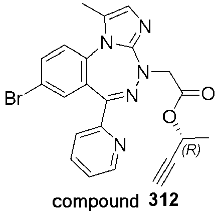 | yellow solid , ¹H NMR (400 MHz, DMSO-*d6*) δ 8.59 (d, *J =* 4.7 Hz, 1H), 7.96 (dt, *J =* 16.3, 5.1 Hz, 2H), 7.88 (dd, *J* = 8.7, 2.3 Hz, 1H), 7.58 (d, *J =* 8.7 Hz, 1H), 7.52 (m, 1H), 7.38 (s, 1H), 6.71 (s, 1H), 5.42 (qd, *J* = 6.6, 1.9 Hz, 1H), 4.69 (d, *J =* 15.2 Hz, 1H), 4.42 (d, *J* = 16.4 Hz, 1H), 3.62 (s, 1H), 2.29 (s, 3H), 1.43 (d, *J* = 6.2 Hz, 3H). LC-MS: [M+H]⁺ Calcd 464.06, 466.06, Found 464.3, 466.3. |
| 9.7 | 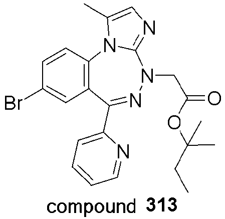 | yellow solid , ¹H NMR (400 MHz, DMSO-*d6*) δ 8.60 (d, *J* = 4.5 Hz, 1H), 7.98 (m, 1H), 7.94 (d, *J =* 7.7 Hz, 1H), 7.87 (dd, *J* = 8.7, 2.2 Hz, 1H), 7.57 (d, *J* = 8.7 Hz, 1H), 7.53 (m, 1H), 7.39 (d, *J* = 2.2 Hz, 1H), 6.71 (s, 1H), 4.59 (d, *J* = 16.6 Hz, 1H), 4.29 (d, *J* = 16.7 Hz, 1H), 2.30 (s, 3H), 1.67 (q, *J =* 7.5 Hz, 2H), 1.37 (s, 6H), 0.76 (t, *J* = 7.4 Hz, 3H). LC-MS: [M+H]⁺ Calcd 482.11, 484.11, Found 482.3, 484.3. |
| 9.8 | 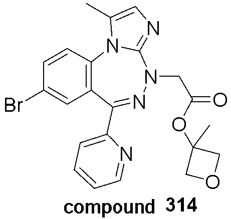 | yellow solid , ¹H NMR (400 MHz, DMSO-*d6*) δ 8.60 (d, *J =* 4.6 Hz, 1H), 7.96 (m, 2H), 7.88 (dd, *J* = 8.7, 2.3 Hz, 1H), 7.58 (d, *J* = 8.7 Hz, 1H), 7.54 (m, 1H), 7.41 (d, *J =* 2.2 Hz, 1H), 6.72 (s, 1H), 4.59 (m, 5H), 3.98 (m, 1H), 2.30 (s, 3H), 1.65 (s, 3H). LC-MS: [M+H]⁺ Calcd 482.07, 484.07, Found 482.3, 484.3. |
| 9.9 | 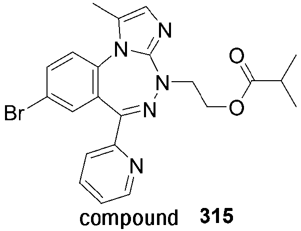 | yellow solid , ¹H NMR (400 MHz, DMSO-*d6*) δ 8.57 (d, *J* = 4.1 Hz, 1H), 8.04 (d, *J* = 7.9 Hz, 1H), 7.96 (td, *J* = 7.7, 1.7 Hz, 1H), 7.82 (dd, *J* = 8.7, 2.3 Hz, 1H), 7.51 (m, 2H), 7.30 (d, *J = 2.3* Hz, 1H), 6.70 (d, *J =* 1.1 Hz, 1H), 4.54 (ddd, *J* = 12.6, 8.7, 4.1 Hz, 1H), 4.22 (dt, *J =* 9.3, 4.4 Hz, 1H), 3.96 (ddd, *J =* 13.3, 8.6, 4.6 Hz, 1H), 3.77 (dt, *J* = 13.3, 4.2 Hz, 1H), 2.33 (dt, *J* = 13.9, 7.0 Hz, 1H), 2.26 (d, *J* = 0.8 Hz, 3H), 0.84 (d, *J* = 6.9 Hz, 3H), 0.76 (d, *J* = 7.0 Hz, 3H). LC-MS: [M+H]⁺ Calcd 468.10, 470.10, Found 468.1, 470.1. |
| 9.9-1 | 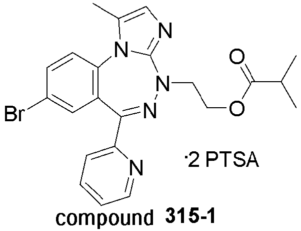 | yellow solid. ¹H NMR (400 MHz, DMSO-d6) δ 8.59 (d, J = 4.6 Hz, 1H), 8.06 (d, J = 7.9 Hz, 1H), 7.99 (td, J = 7.7, 1.5 Hz, 1H), 7.85 (dd, J = 8.7, 2.2 Hz, 1H), 7.54 (m, 2H), 7.47 (d, J = 8.0 Hz, 4H), 7.34 (d, J = 2.2 Hz, 1H), 7.11 (d, J = 7.9 Hz, 4H), 6.81 (s, 1H), 4.55 (m, 1H), 4.23 (m, 1H), 3.99 (m, 2H), 2.34 (m, 1H), 2.29 (s, 9H), 0.84 (d, J = 6.9 Hz, 3H), 0.76 (d, J = 7.0 Hz, 3H). |
| 9.10 | 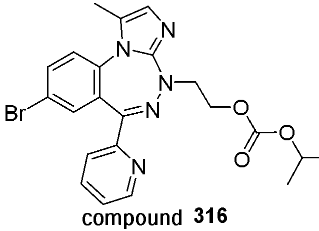 | yellow solid , ¹H NMR (400 MHz, DMSO-*d6*) δ 8.59 (d, *J* = 4.3 Hz, 1H), 8.06 (d, *J* = 7.9 Hz, 1H), 7.97 (td, *J* = 7.8, 1.5 Hz, 1H), 7.84 (dd, *J* = 8.8, 2.3 Hz, 1H), 7.53 (dd, *J* = 11.6, 7.1 Hz, 2H), 7.32 (d, *J* = 2.2 Hz, 1H), 6.72 (s, 1H), 4.53 (dt, *J* = 12.4, 6.3 Hz, 2H), 4.35 (m, 1H), 4.00 (m, 1H), 3.80 (dt, *J* = 9.3, 4.0 Hz, 1H), 2.29 (s, 3H), 1.10 (d, *J =* 6.2 Hz, 3H), 1.04 (d, *J* = 6.2 Hz, 3H). LC-MS: [M+H]⁺ Calcd 484.09, 486.09, Found484.1, 486.1. |
| 9.11 | 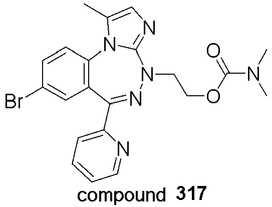 | yellow solid , ¹H NMR (400 MHz, DMSO-*d6*) δ 8.59 (d, *J* = 4.8 Hz, 1H), 8.05 (d, *J* = 7.9 Hz, 1H), 7.99 (ddd, *J* = 16.2, 9.5, 4.8 Hz, 2H), 7.97 (td, *J* = 7.8, 1.7 Hz, 1H), 7.85 (dd, *J* = 8.7, 2.3 Hz, 1H), 7.53 (ddd, *J* = 7.3, 6.2, 5.2 Hz, 2H), 7.38 (d, *J* = 2.3 Hz, 1H), 6.72 (s, 1H), 4.36 (m, 1H), 4.32 (m, 2H), 4.27 (dd, *J* = 10.9, 5.6 Hz, 1H), 4.02 (m, 1H), 3.80 (m, 1H), 2.68 (d, *J* = 13.2 Hz, 6H), 2.29 (s, 3H). LC-MS: [M+H]⁺ Calcd 469.09, 471.09, Found 469.1, 471.1. |
| 9.11-1 | 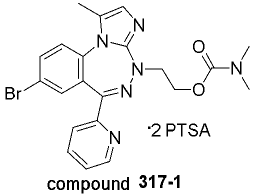 | yellow solid. ¹H NMR (400 MHz, DMSO-d6) δ 8.62 (d, J = 4.6 Hz, 1H), 8.03 (ddd, J = 11.6, 9.4, 4.7 Hz, 2H), 7.90 (dd, J = 8.7, 2.3 Hz, 1H), 7.58 (m, 2H), 7.47 (dd, J = 15.2, 5.1 Hz, 5H), 7.13 (d, J = 7.9 Hz, 5H), 6.96 (s, 1H), 6.50 (brs, 2H), 4.38 (m, 1H), 4.28 (m, 1H), 4.02 (m, 1H), 3.84 (dt, J = 9.9, 4.7 Hz, 1H), 2.69 (s, 3H), 2.64 (s, 3H), 2.32 (s, 3H), 2.31 (s, 6H). |
| 10.1 | 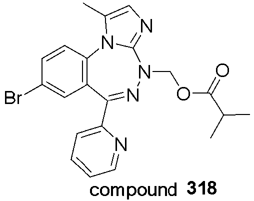 | yellow solid , ¹H NMR (400 MHz, DMSO-*d6*) δ 8.61 (d, *J* = 4.7 Hz, 1H), 8.00 (m, 2H), 7.88 (dd, *J* = 8.7, 2.3 Hz, 1H), 7.55 (m, 2H), 7.44 (d, *J = 2.3* Hz, 1H), 6.75 (d, *J =* 1.1 Hz, 1H), 5.89 (d, *J* = 9.8 Hz, 1H), 5.60 (d, *J* = 9.7 Hz, 1H), 2.58 (m, 1H), 2.30 (d, *J =* 0.8 Hz, 3H), 1.08 (dd, *J* = 14.9, 6.9 Hz, 6H). LC-MS: [M+H]⁺ Calcd 476.07, 478.07, Found 476.2, 478.2. |
| 10.2 | 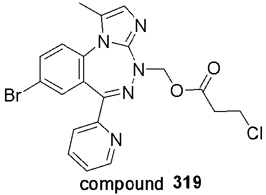 | yellow solid , ¹H NMR (400 MHz, DMSO-*d6*) δ 8.60 (d, *J* = 4.7 Hz, 1H), 8.00 (m, 1H), 7.88 (dd, *J* = 8.7, 2.3 Hz, 1H), 7.55 (m, 2H), 7.42 (d, *J = 2.3* Hz, 1H), 6.76 (d, *J =* 1.0 Hz, 1H), 5.97 (d, *J* = 10.3 Hz, 1H), 5.65 (d, *J* = 9.9 Hz, 1H), 3.81 (t, *J* = 6.2 Hz, 2H), 2.89 (t, *J* = 6.1 Hz, 2H), 2.30 (s, 2H). LC-MS: [M+H]⁺ Calcd 496.02, 498.02, Found 496.1,498.1. |
| 10.3 | 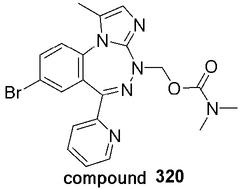 | yellow solid , ¹H NMR (400 MHz, DMSO-*d6*) δ 8.66 (d, *J =* 4.4 Hz, 1H), 8.60 (m, 0H), 8.17 (d, *J* = 8.0 Hz, 1H), 8.04 (td, *J* = 7.8, 1.6 Hz, 1H), 7.83 (dd, *J =* 8.8, 2.3 Hz, 1H), 7.58 (dd, *J* = 6.7, 4.9 Hz, 1H), 7.49 (d, *J* = 8.8 Hz, 1H), 7.20 (d, *J* = 2.3 Hz, 1H), 6.60 (s, 1H), 5.67 (s, 2H), 2.78 (s, 6H), 2.23 (s, 3H). LC-MS: [M+H]⁺ Calcd 455.08, 457.08, Found 455.2, 457.2. |

### Example 130 Preparation of Compound 321-Compound 325

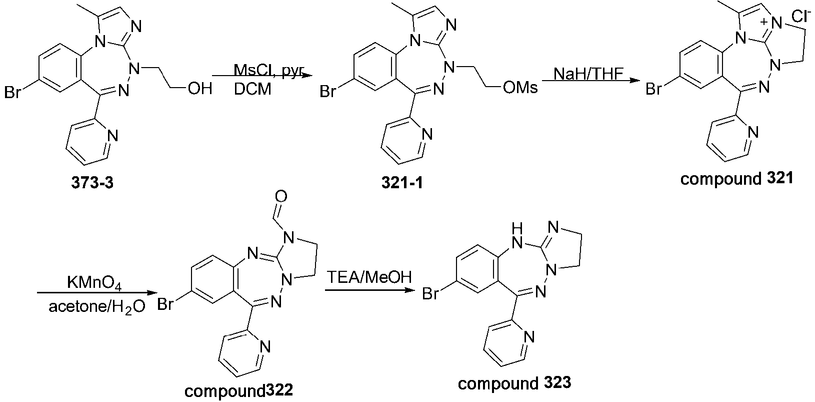

Under nitrogen atmosphere, the solution of Compound **373-3** (800 mg, 2 mmol) in dichloromethane, the mixture was cooled to -30°C. Methanesulfonyl chloride (234 mg, 2.4 mmol) and pyridine (396 mg, 3.0 mmol) were added dropwise. The mixture was stirred at room temperature, after confirming reaction completion by TLC, the mixture was quenched with water and extracted with ethyl acetate. The combined organic layers were washed with brine three times, dried over anhydrous sodium sulfate, and concentrated under reduced pressure to yield the crude product Compound **321-1,** which was used directly in the next step. The crude Compound **321-1** was dissolved in dry tetrahydrofuran. Then the mixture was cooled, and sodium hydride (96 mg, 2.4 mmol) was added in ice-bath, the mixture was stirred for 30 minutes in ice-bath, the mixture was stirred at room temperature for overnight. The mixture was quenched with saturated ammonium chloride solution. The organic phase was concentrated under reduced pressure, and the pH of the residual aqueous solution was adjusted to 3-4 with dilute hydrochloric acid. Purification by C18 reverse-phase silica gel column chromatography (Acetonitrile/Water = 1/1-3/2) to yield Compound **321** (620 mg, two steps yield 81.5%).

A solution of Compound **321** (536 mg, 1.4 mmol) in acetone (7 mL) and water (7 mL), the mixture was stirred in ice-bath, Potassium permanganate (774 mg, 4.9 mmol) was added portionwise. Then the mixture was stirred in ice-bath for 30 minutes, after confirming reaction completion by TLC, the mixture was quenched with sodium sulfite and extracted with ethyl acetate, the combined organic phase was concentrated under reduced pressure. Purification by silica gel column chromatography (Petroleum ether/Ethyl acetate = 2/1-1/1) to yield Compound **322** (282 mg, yield 54.3%).
Compound **322** (223 mg, 0.6 mmol) was dissolved in methanol (6 mL). Triethylamine (1 mL) was added, and the mixture was stirred at room temperature for 1 hour. The mixture was concentrated under reduced pressure to yield Compound **323** (205 mg, yield 100%).

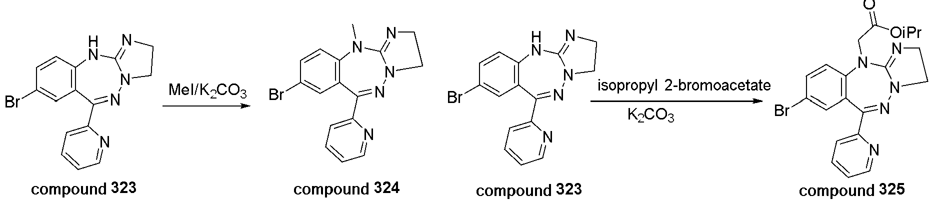

A mixture of Compound **323** (69 mg, 0.2 mmol) and iodomethane (44 mg, 0.3 mmol) in DMF was treated with potassium carbonate (28 mg, 0.2 mmol). The mixture was stirred at room temperature overnight. After confirming reaction completion by TLC, water and ethyl acetate were added and extracted with ethyl acetate, the combined organic phase was concentrated under reduced pressure. Purification by silica gel column chromatography (Petroleum ether/Ethyl acetate = 2/1-1/1) to yield Compound **324** (41 mg, yield 57.6%).

A mixture of Compound **323** (69 mg, 0.2 mmol) and iodomethane (44 mg, 0.3 mmol) in DMF was treated with potassium carbonate (28 mg, 0.2 mmol). The mixture was stirred at room temperature overnight. After confirming reaction completion by TLC, water and ethyl acetate were added and extracted with ethyl acetate, the combined organic phase was concentrated under reduced pressure. Purification by silica gel column chromatography (Petroleum ether/Ethyl acetate = 2/1-1/1) to yield Compound **324** (41 mg, yield 57.6%).

| **Compound NO.** | **Compound structure** | **Characterization** |
|---|---|---|
| 11 | 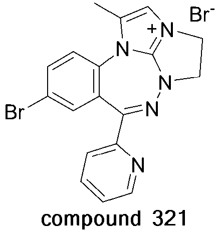 | yellow solid, ¹H NMR (400 MHz, DMSO-*d6*) δ 8.60 (d, *J* = 4.4 Hz, 1H), 7.98 (m, 3H), 7.72 (d, *J* = 8.8 Hz, 1H), 7.56 (m, 2H), 7.41 (d, *J* = 2.2 Hz, 1H), 4.66 (m, 2H), 4.43 (m, 2H), 2.54 (s, 3H). LC-MS: [M]⁺ Calcd 380.005, 382.05, Found 380.2, 382.2. |
| 11.1 | 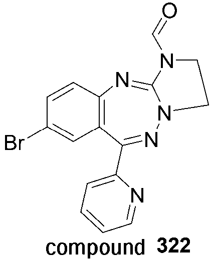 | yellow solid, ¹H NMR (400 MHz, DMSO) δ 9.16 (s, 1H), 8.60 (d, *J* = 4.6 Hz, 1H), 7.95 (dt, *J =* 7.7, 3.9 Hz, 1H), 7.84 (d, *J =* 7.9 Hz, 1H), 7.60 (dd, *J* = 8.6, 2.3 Hz, 1H), 7.52 (ddd, *J* = 7.4, 4.9, 0.9 Hz, 1H), 7.02 (d, *J =* 2.3 Hz, 1H), 6.96 (d, *J =* 8.6 Hz, 1H), 3.91 (m, 2H), 3.81 (m, 2H). LC-MS: [M+H]⁺ Calcd 370.02, 372.02, Found 370.2, 372.2. |
| 11.2 | 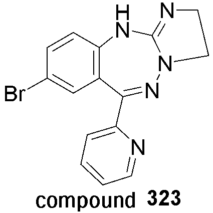 | Off-white solid, ¹H NMR (400 MHz, DMSO-*d6*) δ 8.58 (d, *J* = 4.1 Hz, 1H), 7.92 (td, *J* = 7.7, 1.8 Hz, 1H), 7.83 (d, *J* = 7.8 Hz, 1H), 7.47 (ddd, *J* = 6.1, 4.9, 1.7 Hz, 2H), 6.94 (d, *J* = 2.2 Hz, 1H), 6.85 (d, *J* = 8.6 Hz, 1H), 3.86 (t, *J* = 7.5 Hz, 2H), 3.49 (t, *J* = 7.4 Hz, 2H). LC-MS: [M]⁺ Calcd 342.03, 344.03, Found 342.2, 344.2. |
| 11.3 | 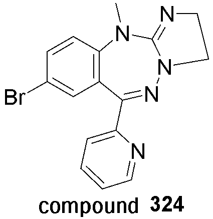 | yellow solid, ¹H NMR (400 MHz, DMSO-*d6*) δ 8.58 (d, *J* = 4.1 Hz, 1H), 7.92 (td, *J* = 7.7, 1.8 Hz, 1H), 7.81 (d, *J* = 7.9 Hz, 1H), 7.47 (m, 2H), 6.88 (dd, *J* = 26.6, 5.4 Hz, 2H), 3.80 (t, *J* = 7.3 Hz, 2H), 3.54 (t, *J* = 7.4 Hz, 2H), 2.95 (s, 3H). LC-MS: [M+H]⁺ Calcd 356.04, 358.04, Found 356.2, 358.2. |
| 11.4 | 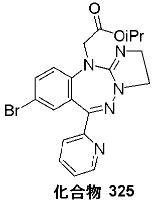 | yellow solid, ¹H NMR (400 MHz, DMSO-*d6*) δ 8.58 (d, *J* = 4.1 Hz, 1H), 7.93 (td, *J* = 7.7, 1.8 Hz, 1H), 7.82 (d, *J* = 7.9 Hz, 1H), 7.49 (ddd, *J* = 9.4, 5.8, 1.7 Hz, 1H), 7.47 (d, *J* = 2.4 Hz, 1H), 6.92 (d, *J* = 2.4 Hz, 1H), 6.76 (d, *J* = 8.6 Hz, 1H), 4.95 (hept, *J* = 6.5 Hz, 1H), 3.84 (t, *J* = 7.3 Hz, 2H), 3.62 (t, *J =* 7.3 Hz, 2H), 1.19 (d, *J* = 6.2 Hz, 6H). LC-MS: [M+H]⁺ Calcd 442.08, 444.08, Found 442.2, 444.2. |

### Example 131 Preparation of Compound 346-Compound 348

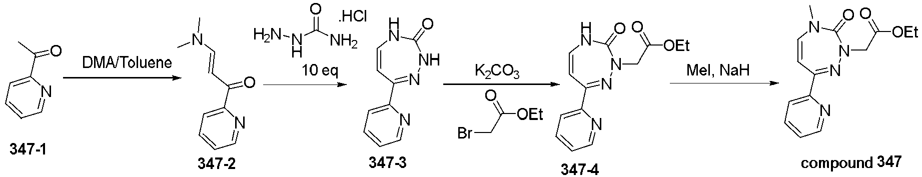

A mixture of 2-acetylpyridine (12.1 g, 0.1 mol) and N,N-dimethylformamide dimethyl acetal (36.0 g, 0.3 mol) in toluene, the mixture was stirred at 120°C for 18 hours. The mixture was concentrated under reduced pressure, and the crude product was triturated with ethyl acetate to yield Compound **347-2** as orange solid (13.8 g, yield 78.4%).

A mixture of carbohydrazide hydrochloride (43.7 g, 0.4 mol) and Compound **347-2** (13.8 g, 78.4 mmol) in ethanol, the mixture was stirred at 90°C for 2 hours. The mixture was concentrated under reduced pressure, the resulting mixture was partitioned between CH₂Cl₂ and water, and then extracted with CH₂Cl₂, the organic phase was concentrated and purified by silica gel chromatography (Petroleum ether/Ethyl acetate = 1/1) to yield Compound **347-3** (1.68 g, yield 11.4%).

A mixture of Compound **347-3** (1.68 g, 8.93 mmol), K₂CO₃ (2.48 g, 17.96 mmol) and ethyl bromoacetate (1.60 g, 10.72 mmol) in dry DMF, the mixture was stirred at room temperature for 3 hours. the combined organic phase was concentrated under reduced pressure. Purification by silica gel column chromatography (Petroleum ether/Ethyl acetate = 2/1-1/1) to yield Compound **347-4** (1.31 g, yield 53.5 %).

A mixture of Compound **347-4** (1.31 g, 4.5 mmol) and iodomethane (852 mg, 6.0 mmol) in dry DMF, the mixture was cooled to -10 °C. Sodium hydride (240 mg, 6.0 mmol) was added portionwise. Th mixture was stirred unde ice-bath for 1 hour and then quenched with saturated aqueous ammonium chloride solution. The mixture was partitioned between ethyl acetate and water. The combined organic layers were concentrated under reduced pressure and purified by silica gel column chromatography (Petroleum ether/Ethyl acetate = 3/1-2/1) to yield Compound **347** (641 mg, 49.4%).

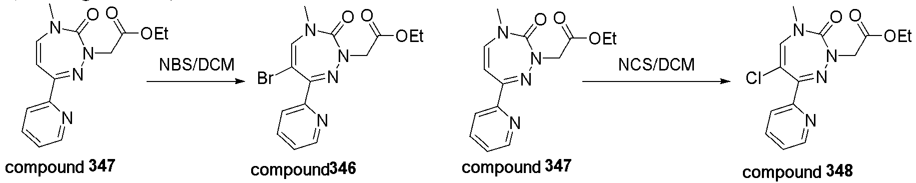

A solution of Compound **347** (144 mg, 0.5 mmol) and N-bromosuccinimide (NBS, 106.8 mg, 0.6 mmol) in dichloromethane, the mixture was stirred at room temperature for 18 hours. The mixture was quenched with aqueous sodium thiosulfate solution. The mixture was partitioned between ethyl acetate and water. The combined organic layers were concentrated under reduced pressure and purified by silica gel column chromatography (Petroleum ether/Ethyl acetate = 3/1-2/1) to yield Compound **346** (17.2 mg, yield 9.4%).

A solution of Compound **347** (144 mg, 0.5 mmol) and N-chlorosuccinimide (NCS, 106.8 mg, 0.6 mmol) in dichloromethane, then the mixture was stirred at room temperature for 18 hours. The mixture was quenched with aqueous sodium thiosulfate solution. The mixture was partitioned between ethyl acetate and water. The combined organic layers were concentrated under reduced pressure and purified by silica gel column chromatography (Petroleum ether/Ethyl acetate = 3/1 -2/1) to yield Compound **348** (20 mg, 12.4%).

| **Compound NO.** | **Compound structure** | **Characterization** |
|---|---|---|
| NS-131 | 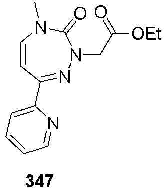 | white solid, ¹H NMR (400 MHz, DMSO-*d6*) δ 8.64 (d, *J* = 4.8 Hz, 1H), 8.35 (d, *J* = 2.7 Hz, 1H), 7.91 (dd, *J =* 7.5, 6.0 Hz, 2H), 7.41 (dd, *J* = 8.7, 4.9 Hz, 1H), 7.01 (s, 1H), 4.60 (brs, 2H), 4.17 (q, *J* = 7.1 Hz, 2H), 3.17 (brs, 3H), 1.20 (brs, 3H). LC-MS: [M+H]⁺ Calcd 289.12, Found 289.1. |
| NS-132 | 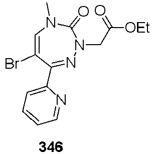 | white solid, ¹H NMR (400 MHz, DMSO-*d6*) δ 8.70 (d, *J* = 4.8 Hz, 1H), 8.60 (s, 1H), 7.94 (td, *J =* 7.7, 1.7 Hz, 1H), 7.79 (brs, 1H), 7.47 (dd, *J* = 8.5, 4.9 Hz, 1H), 4.55 (brs, 2H), 4.14 (brs, 2H),3.15 (s, 3H), 1.23 (brs, 3H). LC-MS: [M+H]⁺ Calcd 367.03, 369.03, Found 367.0, 369.0. |
| NS-133 | 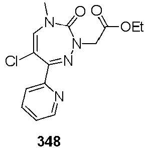 | white solid, ¹H NMR (400 MHz, DMSO-*d6*) δ 8.70 (d, *J* = 4.7 Hz, 1H), 8.62 (s, 1H), 7.94 (dd, *J =* 7.7, 6.0 Hz, 1H), 7.79 (s, 1H),, 7.47 (m, 1H), 4.55 (brs, 2H), 4.15 (brs, 2H), 3.15 (s, 3H), 1.23 (s, 3H). LC-MS: [M+H]⁺ Calcd 323.08, Found 323.0. |

### Example Determination of Pharmacological Data and Safety Data for Compounds of the Present Invention

### 1. The method of experimental

### Determination of the anesthetic effect (determination of the minimum effective anesthetic dose) of the representative compound of the present invention in Rats via tail vein injection:

SD rats were used, with a single intravenous injection via the tail vein ( administration rate of 0.02 ml/s and administration volume of 0.6 ml per rat) in the experiment. The initial dose of the test compound started at 1mg/kg, and the actual administration dose was calculated based on the body weight measured before the experiment for each animal. The increase or decrease in subsequent doses was determined based on the results of the experimental animals (whether the LORR(Loss of righting reflex) occureed). The dose at which the LORR first occur was determined as the minimum effective anesthetic dose.

Determination of the anesthetic effect (determination of median effective dose, ED₅₀) and the pharmacological characteristics of equivalent doses of the representative compound of the present invention in rats via tail vein injection.:
SD rats were used, with a single intravenous injection via the tail vein (administration rate of 0.02 ml/s and administration volume of 0.6 ml per rat) in the experiment. The Up and down method was used to determine the ED₅₀ of the compound causing the LORR. The initial dose of the test compound started at 5 mg/kg, and the actual administration dose was calculated based on the body weight measured before the test for each animal. The increase or decrease in subsequent doses was determined based on the results of the experimental animals (whether the LORR occurred). When five consecutive crosses in which the LORR dose turns to the no-LORR dose were observed, the ED₅₀ of the compound causing the LORR was calculated using a formula in the Up and down method. After determining the ED₅₀, the onset time, duration of anesthesia, and emergence time of anesthesia were determined at an injection dose of 2ED₅₀.

Determination of the effects on blood pressure, heart rate, and respiratory rate in rats of the representative compounds of the present invention via tail vein injection at a injection dose of 2ED₅₀:
According to the ED₅₀ of the compounds, the SD rats were still selected, to be catheterized in their tail artery, and a single injection of 2ED₅₀ dose was given to the tail vein (with an administration rate of 0.02 ml/s and an administration volume of 0.6 ml per rat). Arterial blood pressure, heart rate, and respiratory rate of the rats were continuously monitored during the whole course of the expriment. The effects of the compounds on blood pressure, heart rate and respiratory rate of rats at 2ED₅₀ dose were observed.
Determination of the minimum lethal dose of the compound of the present invention in rats via tail vein injection:
SD rats were used, with a single intravenous injection via the tail vein ( administration rate of 0.02 ml/s and administration volume of of 0.5-1.0 ml per 100 g body weight) in the experiment. The initial dose of the test compound started at 4ED₅₀, and the actual administration dose was calculated based on the body weight measured before the test for each animal. The increase or decrease in subsequent doses was determined based on the results of the experimental animals (whether death occurred). The dose at which death first occurred was determined as the minimum lethal dose. In the present invention, the ratio of the minimum lethal dose to the minimum effective anesthetic dose is defined as the approximate therapeutic index.

Determination of the drug withdrawal recovery time of the compound of the present invention after stopping administration following continuous infusion for different infusion duration in rats:
The experiment used SD rats, with a single intravenous injection of 1.5ED₅₀ via the tail vein, followed by another 1ED₅₀ dose after 1 minute. After administration, the tail vein catheter was connected to a syringe on the infusion pump via an extension tube. The initial infusion rate for each rat was determined based on the specific compound, and the infusion rate was halved after 5 minutes, with adjustments made every 5 minutes thereafter based on the rat's response. When the predetermined infusion time was reached, the infusion was stopped, and the time to recovery of LORR, as well as the time and type of adverse reactions observed during the test were recorded.

In the above experiments, in addition to recording the dose at which the LORR occurred, the onset time and emergence time of anesthesia, the duration of the LORR, and the duration of sedative effects could also be recorded. Meanwhile, circulatory effects experiments can be added to observe the effects of the compound on circulation and respiration in rats.

### 2.The Results of Experimental

**Table 1. The pharmacological data of a single intravenous injection of the salt form of the compound of this invention**

| **Compound NO.** | **Minimal effective anesthetic dose (mg/kg)** | **Onset time (min)** | **Righting feflex duration (min)** | **Sedation Duration (min)** | **TI** | **MLD (mg/kg )** |
|---|---|---|---|---|---|---|
| Compound 1 | 4.5 | 0.2-0.4 | 0.9-3.4 | 5.5-8.0 | >1.5 | >6.75 |
| Compound 1D | 5.0 | 0.2-0.4 | 2.5-11.4 | 6.3-31.3 | 3.0 | 15 |
| Compound 2D | 2.5 | 0.1-0.2 | 1.3-7.7 | 9.3-23.4 | 8.0 | 20 |
| Compound 4 | 15.0 | 0.2-0.4 | 1.1-3.5 | 4.2-10.8 | >1.5 | >22.5 |
| Compound 5 | 3.0 | 0.4-0.7 | 2.4-5.0 | 7.7-9.4 | >2.1 | >6.2 |
| Compound 6 | 1.3 | 0.3-0.8 | 0.6-3.4 | 4.0-10.7 | >3.8 | >5 |
| Compound 6D | 5.0 | 0.3-0.5 | 1.3-11.8 | 9.2-29.2 | 4.0 | 20 |
| Compound 7D | 1.8 | 0.2-0.6 | 2.3-8.2 | 16.5-28.1 | 8.3 | 15 |
| Compound 12 | 20.0 | 0.7 | 1.0 | 12.1 | >1.0 | >20 |
| Compound 13 | 1.0 | 0.2-0.8 | 0.7-2.2 | 5.4-8.6 | 40.0 | 40 |
| Compound 14 | 0.5 | 0.2-0.7 | 0.6-3.2 | 3.5-7.0 | >2 | >1 |
| Compound 15 | 1.3 | 0.2-0.8 | 0.8-5.3 | 4.0-11.7 | >3.8 | >5 |
| Compound 16 | 5.0 | 0.2-0.5 | 0.6-1.7 | 3.2-5.3 | >4.5 | >22.5 |
| Compound 17 | 1.3 | 0.2-0.5 | 1.6-3.3 | 6.6-9.9 | >3.8 | >5 |
| Compound 17E | 1.3 | 0.2-1.7 | 0.9-6.7 | 6.9-11.3 | >3.8 | >5 |
| Compound 18 | 0.5 | 0.2-0.9 | 0.8-4.2 | 4.5-10.7 | >2.4 | >1.2 |
| Compound 19 | 1.0 | 0.4-1.0 | 1.2-5.5 | 9.7-14.8 | >1.9 | >1.9 |
| Compound 19E | 10.0 | 0.2-1.1 | 0.7-7.0 | 8.8-13.8 | >2.3 | >22.5 |
| Compound 20 | 15.0 | 0.3-0.5 | 4.1-6.4 | 17.0-20.3 | >1.5 | >22.5 |
| Compound 27 | 10.0 | 0.2-0.5 | 1.3-3.9 | 4.4-10.7 | >1.8 | >18 |
| Compound 28 | 5.0 | 0.2-0.5 | 0.5-1.6 | 4.1-7.3 | >2.3 | >11.3 |
| Compound 32 | 5.0 | 0.2-0.4 | 1.8-7.7 | 21.1-45.7 | 6.0 | 30 |
| Compound 35 | 30.0 | 0.5 | 1.2 | 25.3 | >1.0 | >30 |
| Compound 36 | 10.0 | 0.4-0.9 | 0.7-4.1 | 8.6-14.7 | 1.9 | 18.7 |
| Compound 37 | 5.0 | 0.3-0.6 | 0.7-5.8 | 15.3-21.3 | >2.0 | >10 |
| Compound 40 | 5.0 | 0.2-0.5 | 1.3-17.2 | 40.2-364.0 | 8.0 | 40 |
| Compound 41 | 2.5 | 0.2-0.5 | 3.3-36.7 | 63.6-162.8 | 16.0 | 40 |
| Compound 42 | 12.5 | 0.3-0.7 | 5.9-15.8 | 61.1-66.8 | >1.6 | >20 |
| Compound 43 | 7.5 | 0.2-0.4 | 0.7-58.6 | 29.3-393.9 | 8.0 | 60 |
| Compound 44 | 5.0 | 0.4-0.7 | 3.1-7.5 | 38.1-49.8 | >2.0 | >10 |
| Compound 45 | 5.0 | 0.2-0.9 | 1.7-4.5 | 39.6-94.6 | >4.0 | >20 |
| Compound 46 | 17.5 | 0.5-0.6 | 6.9-17.2 | 85.5-130.0 | 1.9 | 32.6 |
| Compound 47 | 20.0 | 0.2-0.6 | 2.5-11.5 | 121.4-208.7 | 2.2 | 44 |
| Compound 48 | 30.0 | 0.3-0.40 | 8.8-24.4 | 124.6-13.0 | 2.0 | 60 |
| Compound 49 | 7.5 | 0.2-0.6 | 0. 6-36.0 | 70.2-111.3 | >4.0 | >30 |
| Compound 51 | 15.0 | 0.2-0.3 | 0.7-7.9 | 23.12-78.67 | >1.8 | >27.4 |
| Compound 54 | 5.0 | 0.2-0.3 | 3.9-13.3 | 28.72-158.20 | 7.0 | 35 |
| Compound 55 | 1.0 | 0.2-0.7 | 2.4-25.4 | 11.3-141.0 | >20. 0 | >20 |
| Compound 56 | 2.5 | 0.2 | 1.7 | 83.6 | 4.0 | 10 |
| Compound 57 | 15.0 | 0.4-0.9 | 1.4-3.5 | 12.6-25.7 | 2.0 | 30 |
| Compound 58 | 5.0 | 0.4-0.7 | 0.9-19.0 | 86.4-174.7 | 4.0 | 20 |
| Compound 61 | 10.0 | 0.9 | 0.5 | 33.3 | >1.0 | >10 |
| Compound 62 | 5.0 | 0.2-0.4 | 4.1-12.6 | 123.7-165.1 | >4.0 | >20 |
| Compound 63 | 1.8 | 0.3-0.6 | 5.6-9.5 | 29.07-164.55 | 11.1 | 20 |
| Compound 66 | 3.8 | 0.2-0.5 | 8.3-35.3 | 51.5-164.7 | >5.3 | >20 |
| Compound 67 | 20.0 | 0.3-1.5 | 5.1-20.2 | 41.8-42.9 | >2.0 | >40 |
| Compound 68 | 10.0 | 0.2-0.5 | 3.2-48.8 | 28.8-140.4 | >8.0 | >80 |
| Compound 69 | 15.0 | 0.2-1.8 | 2.0-37.7 | 48.7-189.7 | 7.4 | 110.4 |
| Compound 72 | 10.0 | 0.6 | 2.6 | 11.2 | 3.0 | 30 |
| Compound 76 | 20.0 | 0.7-0.8 | 3.4-14.4 | 11.5-116.4 | 3.0 | 60 |
| Compound 77 | 20.0 | 0.4 | 2.0 | 12.4 | 1.5 | 30 |
| Compound 79 | 20.0 | 0.8 | 2.5 | 102.0 | 2.0 | 40 |
| Compound 81 | | | | | | 40 |
| Compound 82 | 30.0 | 0.8 | 3.0 | 50.4 | 1.3 | 40 |
| Compound 84 | 15.0 | 0.2-0.6 | 9.1-20.6 | 43.6-189.5 | >1.7 | >25 |
| Compound 85 | 13.2 | 0.2-0.3 | 4.7-18.7 | 28.0-84.0 | >1.5 | >20 |
| Compound 86 | 20.0 | 0.6 | 3.3 | 28.3 | >1.0 | >20 |
| Compound 87 | 20.0. | 0.3-0.5 | 13.7-60.7 | 64.9-263.3 | 3.0 | 60 |
| Compound 91 | 20.0 | 0.3 | 1.5 | 39.6 | >1.0 | >20 |
| Compound 95 | 1.0 | 0.2-0.7 | 0.6-56.9 | 23.9-178.8 | 30.0 | 30 |
| Compound 96 | 20.0 | 1.8 | 55.3 | 293.4 | >1.0 | >20 |
| Compound 97 | 2.0 | 0.4-0.7 | 0.7-129.9 | 16.9-187.5 | 15.0 | 30 |
| Compound 97B | 10.0 | 0.2-0.4 | 2.0-10.0 | 73.4-199.7 | >2.3 | >22.5 |
| Compound 97D | 5.0 | 0.2-0.5 | 2.6-13.4 | 56.9-152.0 | >2.9 | >14.5 |
| Compound 98 | 5.0 | 0.5-0.6 | 11.6-17.4 | 72.5-208.7 | 6.0 | 30 |
| Compound 98D | 5.0 | 0.2-0.4 | 0.7-45.0 | 42.8-375.8 | >3.6 | >18 |
| Compound 99 | 5.0 | 0.2-0.8 | 1.8-30.1 | 123.9-328.9 | 8.0 | 40 |
| Compound 100 | 0.75 | 0.3-0.8 | 9.0-69.7 | 36.6-183.3 | 10.0 | 7.5 |
| Compound 101 | 1.8 | 0.2-1.0 | 0.6-15.8 | 27.5-180.9 | >11. 1 | >20 |
| Compound 101D | 2.3 | 0.3-0.7 | 0.6-1.1 | 15.7-103.4 | >2.2 | >5 |
| Compound 102 | 7.5 | 0.2-0.5 | 2.7-39.5 | 72.0-341.3 | 5.3 | 40 |
| Compound 103 | 5.0 | 0.2-0.7 | 9.1-13.8 | 67.3-271.0 | >4.0 | >20 |
| Compound 103D | 2.4 | 0.2-0.6 | 0.5-8.1 | 25.7-108.5 | >2.1 | >5 |
| Compound 104 | 1.8 | 0.2-0.9 | 2.1-53.3 | 36.1-326.7 | 22.2 | 40 |
| Compound 105 | 12.5 | 0.3-0.8 | 6.4-9.8 | 25.1-87.8 | 2.4 | 30 |
| Compound 105-1 | 30 | 0.32 | 7.22 | 63.95 | 1.3 | 40 |
| Compound 105-2 | | | | | | 20 |
| Compound 107 | 10.0 | 0.3-0.7 | 2.0-15.1 | 13.7-59.8 | >2.6 | >25.5 |
| Compound 107-1 | 15 | 0.40-0.67 | 5.30-16.58 | 70.88-95.90 | 2 | 30 |
| Compound 107-2 | 12 | 0.35-0.52 | 6.15-14.97 | 17.03-214.37 | >2 | >24.3 |
| Compound 107-3 | 15 | 0.52-1.28 | 3.03-4.97 | 20.62-30.75 | 2 | 30 |
| Compound 107-5 | 17.5 | 0.18-0.38 | 3.22-16.55 | 12.07-58.72 | >1.9 | >33 |
| Compound 108 | 10.0 | 0.2-0.4 | 4.1-15.6 | 57.1-99.6 | 4 | 40 |
| Compound 111 | | | | | | 40 |
| Compound 113 | 15.0 | 0.2-0.3 | 0.7-29.5 | 20.6-77.7 | 2.7 | 40 |
| Compound 114 | 0.5 | 0.4-1.1 | 0.8-27.6 | 16.5-140.3 | 40.0 | 20 |
| Compound 115 | 2.5 | 0.2-0.4 | 4.9-14.1 | 12.4-56.0 | 16 | 40 |
| Compound 117 | 25.0 | 0.4-0.5 | 2.7-3.8 | 14.6-32.0 | 1.6 | 40 |
| Compound 118 | 20.0 | 0.6-0.8 | 0.9-2.5 | 13.3-13.7 | >1.5 | >30 |
| Compound 120 | 5.0 | 0.2-0.3 | 0.9-6.9 | 5.4-29.8 | 12.0 | 60 |
| Compound 121 | 1.8 | 0.2-0.6 | 4.6-15.6 | 21.0-104.9 | 16.7 | 30 |
| Compound 122 | 5.0 | 0.1-0.3 | 0.9-4.8 | 9.2-34.0 | >4.0 | >20 |
| Compound 123 | 10.0 | 0.2 | 2.7 | 22.9 | 3.1 | 31 |
| Compound 124 | 1.8 | 0.2-0.5 | 2.3-13.1 | 15.1-40.9 | 22.2 | 40 |
| Compound 125 | 1.8 | 0.2-0.7 | 0.9-16.5 | 11.3-45.9 | 16.7 | 30 |
| Compound 126 | 15.0 | 0.1-0.2 | 1.4-5.0 | 16.0-18.7 | >1.3 | >20 |
| Compound 127 | 7.0 | 0.2-0.6 | 5.0-33.6 | 43.0-62.3 | >5.7 | >40 |
| Compound 128 | 5.0 | 0.3-0.9 | 12.3-27.7 | 33.9-78.3 | 4.0 | 20 |
| Compound 129 | 20.0 | 0.5 | 2.0 | 15.2 | 2.0 | 40 |
| Compound 130 | 1.0 | 0.2-1.9 | 6.4-138.1 | 51.0-209.7 | >33. 0 | >33 |
| Compound 131 | 2.5 | 0.2-0.9 | 0.6-7.9 | 12.7-16.7 | 4.0 | 10 |
| Compound 132 | 2.5 | 0.8 | 1.1 | 13.1 | 2.0 | 5 |
| Compound 133 | 7.5 | 0.5 | 1.4 | 24.0 | 1.3 | 10 |
| Compound 134 | | | | | | 20 |
| Compound 135 | 0.5 | 0.2-0.6 | 1.4-16.5 | 13.4-164.2 | 60.0 | 30 |
| Compound 136 | 10.0 | 0.2-0.3 | 3.4-10.9 | 27.8-30.8 | 1.4 | 14.2 |
| Compound 137 | 2.5 | 0.3-0.4 | 1.4-8.0 | 19.8-62.2 | 4.0 | 10 |
| Compound 140 | | | | | | 20 |
| Compound 144 | | | | | | 3.8 |
| Compound 145 | 5.0 | 0.2-0.4 | 0.5-14.3 | 33.1-88.2 | 6.0 | 30 |
| Compound 145D | 7.5 | 0.2-0.5 | 1.7-19.9 | 23.0-63.0 | >2.3 | >16.9 |
| Compound 146 | 1.0 | 0.3-0.6 | 3.7-9.7 | 17.3-27.1 | 5.0 | 5 |
| Compound 147 | 2.5 | 0.2-0.5 | 7.2-11. 8 | 27.0-48.7 | 6.0 | 15 |
| Compound 148 | 1.8 | 0.2-1.1 | 1.0-60.8 | 20.1-82.4 | 33.3 | 60 |
| Compound 149 | 6.8 | 0.3 | 16.0 | 34.7 | 1.1 | 7.5 |
| Compound 150 | 2.5 | 0.2-0.4 | 0.8-13.5 | 16.9-33.9 | 5.5 | 13.7 |
| Compound 151 | 1.8 | 0.2-3.4 | 3.7-38.6 | 19.0-68.4 | 16.3 | 29.4 |
| Compound 153 | 7.5 | 0.3-0.4 | 2.9-5.8 | 20.0-21.9 | 2.7 | 20 |
| Compound 154 | 3.8 | 0.5-0.9 | 1.3-6.2 | 14.1-17.0 | 2.0 | 7.5 |
| Compound 155 | | | | | | 1.8 |
| Compound 156 | 1.8 | 0.2-0.4 | 1.7-31.9 | 9.3-91.5 | 11.1 | 20 |
| Compound 156D | 5.0 | 0.2-0.6 | 0.6-10.9 | 12.8-36.9 | >1.5 | >7.5 |
| Compound 157 | 1.8 | 0.2-0.6 | 1.5-11.1 | 11.4-53.8 | 5.6 | 10 |
| Compound 158 | 2.5 | 0.5 | 6.3 | 9.5 | 1.5 | 3.8 |
| Compound 159 | 1.8 | 0.2-0.6 | 0.8-10.7 | 14.5-22.4 | 8.3 | 15 |
| Compound 159D | 3.0 | 0.3-0.8 | 0.60-7.1 | 14.9-30.4 | 2.3 | 6.8 |
| Compound 162 | 27.4 | 0.2 | 0.8 | 124.6 | >1.0 | >27.4 |
| Compound 164 | 5.0 | 0.2-1.0 | 1.2-50.4 | 116.7-284.0 | 8.0 | 40 |
| Compound 166 | 20.0 | 0.4 | 20.7 | 64.0 | 2.0 | 38.9 |
| Compound 167 | 40.0 | 0.28-0.3 | 6.1-18.9 | 176.5-187.0 | 1.3 | >50.3 |
| Compound 169 | 2.5 | 0.47-0.53 | 3.1-6.4 | 13.1-21. 8 | 4.0 | 10 |
| Compound 170 | | | | | | 10 |
| Compound 171 | | | | | | 10 |
| Compound 172 | | | | | | 5 |
| Compound 173 | 5.0 | 0.2-0.4 | 0.6-32.4 | 44.7-176.1 | 6.0 | 30 |
| Compound 174 | 15.0 | 0.4-0.5 | 0.9-11.3 | 23.0-155.8 | 2. 7 | 40 |
| Compound 176 | 15.0 | 0.2-0.4 | 6.5-15.3 | 52.7-68.9 | 4.0 | 60 |
| Compound 177 | | | | | | 40 |
| Compound 180 | | | | | | 40 |
| Compound 181 | 40.0 | 0.8 | 7.8 | 53.0 | >1.0 | >40 |
| Compound 188 | 2.5 | 0.2-3.2 | 0.6-46.2 | 39.1-291.8 | 16 | 40 |
| Compound 189 | 1.8 | 0.02-0.9 | 8.0-31.6 | 82.4-287.1 | 14.3 | 25 |
| Compound 190 | 5.0 | 0.2-1.3 | 16.2-46.6 | 150.8-166.4 | 2.5 | 12.5 |
| Compound 191 | 5.0 | 0.45-0.53 | 0.7-12.8 | 53.5-155.3 | 6.8 | 34.0 |
| Compound 192 | 5.0 | 0.2-0.4 | 5.9-14.9 | 156.7-200.0 | 2.0 | 10.0 |
| Compound 193 | 0.5 | 0.4-0.9 | 4.7-38.2 | 27.1-142.8 | 10.0 | 5.0 |
| Compound 194 | 2.5 | 0.2-0.6 | 18.1-33.4 | 117.4-236.1 | >10 | >25 |
| Compound 195 | 7.5 | 0.2-0.5 | 5.3-76.6 | 78.5-195.4 | >2.9 | >21.8 |
| Compound 196 | 15 | 0.2-1.4 | 9.5-23.6 | 214.5-403.5 | 3.3 | 50 |
| Compound 197 | | | | | | 15.0 |
| Compound 198 | 10.0 | 0.3-0.4 | 1.4-2.8 | 28.2-31.3 | 2.0 | 20.0 |
| Compound 199 | 10.0 | 0.2-0.6 | 1.0-14.5 | 20.9-150.5 | 6.0 | 60.0 |
| Compound 199-1 | 15 | 1.17 | 1.20 | 13.68 | 1.3 | 20 |
| Compound 199-3 | 20 | 0.68 | 9.65 | 35.72 | 1.5 | 30 |
| Compound 199-4 | | | | | | >40 |
| Compound 200 | | | | | | 20.0 |
| Compound 201 | 5 | 0.3-0.7 | 0.7-8.4 | 90.6-140.0 | >2 | >10 |
| Compound 202 | 2.5 | 0.3-1.1 | 6.7-67.5 | 37.4-67.5 | 8 | 20 |
| Compound 203 | 1 | 0.3-0.8 | 3.7-35.4 | 47.9-200.1 | 20 | 20 |
| Compound 204 | 1 | 0.2-1.1 | 7.9-58.2 | 25.6-264.8 | 26.4 | 26.4 |
| Compound 205 | 7.5 | 1.10-5.52 | 6.28-40.97 | 72.25-263.53 | 4 | 30 |
| Compound 206 | 17.5 | 0.25-0.50 | 11.1-17.0 | 96.0-102.67 | 1.7 | 30 |
| Compound 207 | 6.5 | 0.55-0.95 | 5.8-42.3 | 75.0-318.1 | 3.7 | 23.7 |
| Compound 208 | | | | | | 3.75 |
| Compound 209 | 7.5 | 1.42 | 67.42 | 178.95 | 1.3 | 10 |
| Compound 210 | 7.5 | 2.63 | 9.47 | 184.50 | 1.3 | 10 |
| Compound 212 | 15 | 0.22-0.67 | 4.28-28.33 | 17.02-103.55 | 4.2 | 63.6 |
| Compound 213 | 40 | 0.42 | 5.77 | 128.9 | > 1 | >40 |
| Compound 214 | 15 | 0.63-1.02 | 0.98-25.83 | 23.65-223.32 | 2.7 | 40 |
| Compound 215 | 30 | 0.62 | 3.20 | 128.53 | 1.3 | 40 |
| Compound 216 | 20 | 0.38 | 5.87 | 83.67 | > 1 | >20 |
| Compound 217 | | | | | | > 16.5 |
| Compound 218 | 15 | 0.87-2.47 | 2.48-5.35 | 65.30-74.63 | 2 | 30 |
| Compound 219 | 20 | 0.17 | 11.33 | 142.67 | >1 | >20 |
| Compound 220 | | | | | | 16.2 |
| Compound 221 | | | | | | 20 |
| Compound 222 | 7.5 | 0.47-1.02 | 2.32-5.37 | 21.48-140.83 | 2 | 15 |
| Compound 223 | | | | | | 20 |
| Compound 224 | | | | | | 15 |
| Compound 225 | 20 | 1.30 | 1.10 | 69.45 | >1 | >20 |
| Compound 226 | 20 | 1.63 | 4.42 | 434.78 | > 1 | >20 |
| Compound 228 | 13.7 | 1.12-1.45 | 2.72-7.48 | 90.12-103.32 | > 1.5 | >20 |
| Compound 235 | 20 | 0.83 | 3.67 | 176.20 | 1.5 | 30 |
| Compound 239 | 5 | 0.17-0.75 | 1.30-11.58 | 17.72-23.57 | 8 | 40 |
| Compound 240 | 10 | 0.30-0.48 | 0.62-15.83 | 15.27-36.70 | 3 | 30 |
| Compound 241 | 7.5 | 0.43-0.90 | 3.47-30.13 | 18.10-73.95 | 4 | 30 |
| Compound 242 | 7.5 | 0.28-0.55 | 5.05-18.42 | 19.98-46.92 | 4 | 30 |
| Compound 243 | 5 | 0.22-0.67 | 0.65-19.85 | 21.98-55.45 | 6 | 30 |
| Compound 244 | 15 | 0.15-0.18 | 0.78-3.85 | 11.42-18.83 | 2.7 | 40 |
| Compound 245 | 10 | 0.15-0.67 | 1.27-1.40 | 11.02-18.83 | 4 | 40 |
| Compound 247 | 20 | 0.28 | 30.15 | 88.25 | 1.5 | 30 |
| Compound 248 | 10 | 0.15-0.52 | 2.97-38.32 | 138.4-240 | 4 | 40 |
| Compound 249 | 10 | 0.50-0.62 | 6.55-13.37 | 23.30-42.37 | 2 | 20 |
| Compound 250 | 20 | 0.53 | 13.62 | 21.55 | >1 | / |
| Compound 251 | 7.5 | 0.12-0.62 | 0.63-17.38 | 12.5-102.08 | 5.3 | 40 |
| Compound 252 | 20 | 0.43 | 2.92-5.05 | 9.95-10.58 | 2 | 40 |
| Compound 253 | 10 | 0.87-0.88 | 1.38-11.07 | 10.13-31.28 | 3 | 30 |
| Compound 254 | 10 | 0.60-0.65 | 0.78-3.10 | 29.25-40.22 | 2 | 20 |
| Compound 255 | 15 | 0.53-0.97 | 12.27-13.62 | 43.43-66.14 | 2 | 30 |
| Compound 256 | 5 | 0.17-0.50 | 0.32-26.03 | 13.38-59.65 | 6 | 30 |
| Compound 257 | 10 | 0.77-1.48 | 5.85-20.40 | 33.47-64.03 | 4 | 40 |
| Compound 258 | | | | | | 40 |
| Compound 259 | | | | | | >20 |
| Compound 260 | 40 | 0.12 | 7.52 | 55.52 | 1.4 | 55 |
| Compound 262 | | | | | | >20 |
| Compound 263 | | | | | | 40 |
| Compound 264 | 5 | 0.43 | 1.77 | 8.93 | 2 | 10 |
| Compound 265 | | | | | | 40 |
| Compound 266 | 5 | 0.40-0.52 | 0.58-4.00 | 12.07-16.32 | 3 | 15 |
| Compound 267 | 20 | 0.72 | 0.28 | 12.33 | 2 | 40 |
| Compound 268 | | | | | | 40 |
| Compound 269 | | | | | | >36 |
| Compound 270 | | | | | | 29.64 |
| Compound 271 | | | | | | 30 |
| Compound 272 | | | | | | 30 |
| Compound 273 | 10 | 0.25-0.27 | 2.38-9.68 | 43.97-44.82 | >2 | >20 |
| Compound 274 | 27.4 | 0.18 | 4.57 | 25.87 | >1 | >27.4 |
| Compound 275 | 30 | 0.93-4.90 | 6.37-17.25 | 127.83-197.3 2 | >2 | >60 |
| Compound 276 | | | | | | 20 |
| Compound 277 | | | | | | 20 |
| Compound 278 | | | | | | 40 |
| Compound 279 | 15 | 0.35 | 3.77 | 62.22 | 1.3 | 20 |
| Compound 280 | | | | | | 10 |
| Compound 281 | | | | | | 40 |
| Compound 282 | | | | | | 15 |
| Compound 283 | | | | | | 40 |
| Compound 284 | 40 | 0.2 | 20 | 70.72 | >1 | >40 |
| Compound 285 | | | | | | 15 |
| Compound 286 | 2.5 | 1.32-3.20 | 3.38-9.38 | 42.15-64.37 | 3 | 7.5 |
| Compound 287 | 10 | 0.32-0.43 | 0.63-7.53 | 44.20-46.25 | 3 | 30 |
| Compound 288 | 15 | 0.80 | 2.45 | 33.18 | 1.3 | 20 |
| Compound 289 | 5 | 0.15-0.68 | 0.53-32.23 | 20.27-311.87 | 8.3 | 41.7 |
| Compound 290 | 15 | 0.45 | 6.63 | 18.08 | 1.3 | 20 |
| Compound 291 | 15 | 0.65-0.92 | 2.03-5.98 | 142.15-175.6 0 | 2 | 30 |
| Compound 292 | 20 | 0.40 | 2.75 | 46.82 | 1.5 | 30 |
| Compound 293 | 15 | 0.95 | 4.20 | 82.85 | 1.3 | 20 |
| Compound 294 | 15 | 0.52-0.55 | 3.35-6.47 | 28.68-40.58 | >1.3 | >20 |
| Compound 295 | 20 | 0.20-0.52 | 3.70-13.25 | 34.23-172.50 | 2 | 40 |
| Compound 296 | | | | | | 15 |
| Compound 297 | | | | | | 5 |
| Compound 298 | 10 | 0.22-0.38 | 6.73-8.97 | 17.03-30.07 | 2 | 20 |
| Compound 299 | | | | | | 15 |
| Compound 300 | 40 | 0.25 | 4.67 | 76.20 | >1 | >40 |
| Compound 301 | | | | | | 27.4 |
| Compound 302 | | | | | | 20 |
| Compound 303 | 20 | 0.43 | 14.30 | 74.88 | >1 | >20 |
| Compound 304 | 5 | 0.18-0.57 | 1.92-5.35 | 10.57-18.57 | 4 | 20 |
| Compound 305 | | | | | | 34.7 |
| Compound 306 | | | | | | 40 |
| Compound 307 | 8.75 | 0.27-0.73 | 0.90-4.27 | 20.87-32.75 | >1.7 | >15.15 |
| Compound 308 | 5 | 0.20-0.50 | 4.13-11.73 | 22.47-36.30 | 4 | 20 |
| Compound 308-1 | 5 | 0.28-0.32 | 3.38-23.18 | 20.07-43.85 | 5.1 | 25.3 |
| Compound 309 | 2.5 | 0.35-0.55 | 2.38-18.82 | 27.68-46.83 | 6 | 15 |
| Compound 310 | 10 | 0.15-0.52 | 2.97-38.32 | 138.40-240 | 4 | 40 |
| Compound 311 | 5 | 0.17-0.48 | 2.28-12.30 | 25.88-54.23 | 6 | 30 |
| Compound 312 | 10 | 0.15-0.52 | 2.97-38.32 | 138.40-240 | 4 | 40 |
| Compound 313 | 15 | 0.53-0.97 | 12.27-13.62 | 43.43-66.15 | 2 | 30 |
| Compound 314 | 10 | 0.15-0.67 | 1.27-1.40 | 11.02-18.83 | 4 | 40 |
| Compound 315 | 3.75 | 0.38-0.75 | 0.58-2.35 | 14.12-24.55 | 2.7 | 10 |
| Compound 315-1 | 7.5 | 0.13-0.50 | 0.78-17.35 | 16.32-35.42 | 6 | 45 |
| Compound 316 | | | | | | 10 |
| Compound 317 | 5 | 0.22-0.38 | 4.08-25.97 | 18.73-70.28 | 4 | 20 |
| Compound 317-1 | 7.5 | 0.18-0.75 | 2.25-35.03 | 49.02-125.10 | 10.5 | 79 |
| Compound 318 | 5 | 0.52-0.63 | 0.58-10.53 | 24.95-35.65 | >2 | >10 |
| Compound 319 | 7.5 | 0.38-0.48 | 7.58-8.00 | 21.82-52.53 | >1.3 | >10 |
| Compound 320 | 15 | 0.65-0.92 | 2.03-5.98 | 142.15-175.6 0 | 2 | 30 |
| Compound 321 | | | | | | 10 |
| Compound 322 | | | | | | 34 |
| Compound 323 | 30 | 0.95-1.07 | 13.12-21.08 | 147.07-196.9 2 | >1.3 | >40 |
| Compound 324 | | | | | | >15.7 |
| Compound 325 | 40 | 0.65 | 3.65 | 59.22 | >1 | >40 |
| Compound 346 | 18.4 | 0.57 | 2.63 | 45.10 | >1 | >18.4 |
| Compound 347 | 20 | 0.13-0.28 | 2.25-4.47 | 15.35-54.20 | 2 | 40 |
| Compound 348 | 20 | 0.75 | 2.03 | 116.72 | >1 | >20 |

**Table 2 Minimum Effective Analgesic Dose of Single Intravenous Injection for Compounds of the Present Invention**

| **Compound NO**. | **Minimal effective analgesic dose (mg/kg)** | **TC-ED_{5 0} (mg/kg)** | **Compound NO.** | **Minimal effective analgesic dose (mg/kg)** | **TC-ED_{5 0} (mg/kg)** |
|---|---|---|---|---|---|
| Compound 41 | 30 | | Compound218 | 20 | |
| Compound 47 | 40 | | Compound219 | 20 | |
| Compound 49 | 20 | 21.4 | Compound226 | 20 | 6.71 |
| Compound 54 | 20 | | Compound 239 | 10 | |
| Compound 55 | 20 | 15.4 | Compound 240 | 30 | |
| Compound 69 | 60 | | Compound 245 | 20 | |
| Compound 79 | 20 | | Compound 247 | 20 | |
| Compound 84 | 20 | 19.7 | Compound 248 | 20 | |
| Compound 87 | 40 | | Compound 249 | 15 | |
| Compound 96 | 15 | | Compound 251 | 20 | |
| Compound 97 | 10 | 9.9 | Compound 252 | 30 | |
| Compound 97B | 22.5 | | Compound253 | 20 | |
| Compound 97D | 10 | 12.2 | Compound 255 | 20 | |
| Compound 98 | 20 | 22.0 | Compound 256 | 10 | |
| Compound 98D | 18 | | Compound 257 | 10 | |
| Compound 99 | 20 | | Compound 260 | 40 | |
| Compound 102 | 20 | 23 | Compound 289 | 20 | |
| Compound 105 | 15 | 17 | Compound 292 | 20 | |
| Compound 107 | 15 | | Compound 293 | 15 | |
| Compound 108 | 15 | | Compound 290 | 15 | |
| Compound 128 | 15 | 12.6 | Compound 291 | 15 | |
| Compound 145 | 20 | | Compound 308 | 10 | |
| Compound 166 | 20 | | Compound 309 | 12.5 | |
| Compound 181 | 40 | | Compound 310 | 20 | |
| Compound189 | 15 | | Compound 311 | 10 | |
| Compound 191 | 20 | | Compound 312 | 10 | |
| Compound 193 | 3.8 | | Compound 313 | 20 | |
| Compound 194 | 15 | | Compound 315 | 30 | |
| Compound195 | 21.8 | | Compound 317 | 10 | |
| Compound 199 | 15 | | Compound 320 | 15 | |
| Compound 202 | 10 | | Compound 323 | 30 | |
| Compound203 | 10 | | Compound 325 | 40 | |
| Compound204 | 20 | | Compound 346 | 18.4 | |
| Compound212 | 15 | 13.72 | Compound 347 | 20 | |
| Compound214 | 20 | | | | |

Finally, it should be noted that all the above embodiments are only used to illustrate the technical solutions of the present invention, not to limit them; although the present invention has been described in detail with reference to the foregoing embodiments, those of ordinary skill in the art should understand that they can still modify the technical solutions recorded in the foregoing embodiments, or equivalently replace some or all of the technical features therein; and these modifications or replacements do not make the essence of the corresponding technical solutions deviate from the scope of the technical solutions of the embodiments of the present invention.

## Claims

1. A compound of formula I, or a pharmaceutically acceptable salt thereof, a stereoisomer thereof, a prodrug thereof, a solvate thereof, or a deuterated derivative thereof: 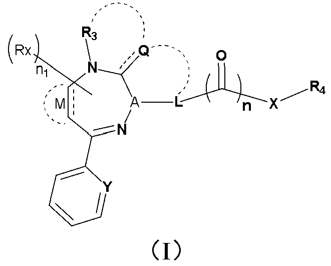
wherein, Y is selected from N or -CF;
A is selected from N or CH;
M is selected from none, or M together with the adjacent carbon atom which they are attached to form fused ring, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl; when M together with the adjacent carbon atom which they are attached to form fused ring, then Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl),-OC₀₋₁₀ alkyl,C₃₋₁₀ cycloalkyl, N-heterocyclyl, O-heterocyclyl, S-heterocyclyl, -NHCO(C₀₋₁₀ alkyl);
Q is selected from O, S-Rxx, N or 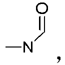 R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O, or S atom as ring atom;
Rxx is selected from H, straight-chain or branched-chain C₁₋₅ alkyl, said the H of alkyl which are optionally substituents selected from the group consisting of: halogen, nitro, cyano, -OC₀₋₁₀ alkyl;
when Q is N, then R₃ and Q together with the adjacent N and C atoms which they are attached to form five-membered heteroaryl or six-membered heteroaryl, which containing at least two heteroatoms;
L is none, or L is selected from C₂₋₈ alkenyl or C₁₋₈ alkylene, wherein, the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
when Q is selected from N or 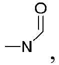 and A is N, then Q, A, L together with the adjacent atoms which they are attached to form five-membered N-heterocycle or six-membered N-heterocycle;
n is 0 or 1, when n is 0, it means the -CO- is none;
X is none, or when n is 1, X is O, when n is 0, X is selected from O, substituted or unsubstituted N-alkyl, substituted or unsubstituted N-heterocycalkyl;
R₄ is selected from -H, halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, heterocyclic, aryl, N-heteroaryl, O-heteroaryl, S-heteroaryl, -SO₂(C₀₋₁₀ alkyl), -SO(C₀₋₁₀ alkyl), -SO₂O(C₀₋₁₀ alkyl), -SO₂N(C₀₋₁₀ alkyl)(C₀₋₁₀ alkyl), -SO₂(C₃₋₁₀ cycloalkyl), -SO₂-aryl, -CON(C₀₋₁₀ alkyl)(C₀₋₁₀ alkyl), -CO(C₀₋₁₀ alkyl), -CO(C₃₋₁₀ cycloalkyl), -CO(3-6-memenber heterocloalkyl), -(C₀₋₁₀ alkyl)COO(C₀₋₁₀ alkyl), -COO(C₃₋₁₀ cycloalkyl), -COO(3-6-memenber heterocloalkyl), alkenyl, alkynyl, said heterocloalkyl contains at least one N, O or S ring atom, wherein, the H on the aforementioned group is optionally substitured with one or more substituents selected from the group consisting of: halogen, -CN, -NO₂, -CF₃, straight-chain C₁₋₃ alkyl, -OC₀₋₁₀ alkyl, C₃₋₆ cycloalkyl, C₃₋₆ heterocycloalkyl, -N(C₀₋₁₀ alkyl)(C₀₋₁₀ alkyl), -COO(C₀₋₁₀ alkyl), -COO(C₃₋₁₀ cycloalkyl), -COO(heterocycloalkyl), -CO(C₀₋₁₀ alkyl), -OCO(C₀₋₁₀ alkyl), -CON(C₀₋₁₀ alkyl)(C₀₋₁₀ alkyl), -OCOO(C₀₋₁₀ alkyl), phenyl, N-heteroaryl, O-heteroaryl, S-heteroaryl, alkenyl, alkynyl.

2. The compound according to claim 1, or a pharmaceutically acceptable salt thereof, a stereoisomer thereof, a prodrug thereof, a solvate thereof, or a deuterated derivative thereof, **characterized in that** said compound has a structure of formula I-a: 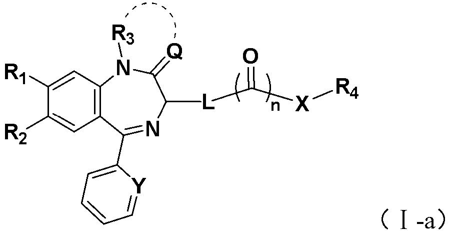
wherein, Y is selected from N or -CF;
R₁ and R₂ are each independently selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalky, N-heteroaryl, O-heteroaryl, S-heteroaryl, or R₁ and R₂ together with the carbon atom which they are attached to form five-membered aromatic heterocycle, six-membered aromatic heterocycle, aromatic ring; said five-membered aromatic heterocycle is selected from the group consisting of: furan, thiophene, pyrrole, pyrazole, imidazole, oxazole, thiazole; said six-membered aromatic heteroaryl is selected from the group consisting of: pyridine, pyridazine, pyrimidine, pyrazine; optionally, the hydrogen atoms on the said five-membered aromatic heterocycle, six-membered aromatic heterocycle or aromatic ring may be substituted with the following groups: halogen, -CN, -CF₃, straight-chain or branched C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
Q is selected from O or N, R₃ is selected from -H, straight-chain or branched-chain C₁₋₅ alkyl, said
the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, said the heterocyclyl contains at least one N, O, or S atom as ring atom;
when Q is N, R₃ and Q together with the N and C atoms which they are attached to form five-membered aromatic heteroaryl or six-membered aromatic heteroaryl, which containing at least two heteroatoms;
L is none, or L is selected from C₂₋₈ alkenyl, C₁₋₈ alkylene, wherein, H of the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
n is 0 or 1, when n is 0, it means the -CO- is none;
X is none, or when n is 1, X is O, when n is 0, X is selected from O, substituted or unsubstituted N-alkyl, substituted or unsubstituted N-heterocycalkyl;
R₄ is selected from -H, halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, heterocyclic, aryl, N-heteroaryl, O-heteroaryl, S-heteroaryl, -SO₂(C₀₋₁₀ alkyl), -SO(C₀₋₁₀ alkyl), -SO₂O(C₀₋₁₀ alkyl), -SO₂N(C₀₋₁₀ alkyl)(C₀₋₁₀ alkyl), -SO₂(C₃₋₁₀ cycloalkyl), -SO₂-aryl, -CON(C₀₋₁₀ alkyl)(C₀₋₁₀ alkyl), -CO(C₀₋₁₀ alkyl), -CO(C₃₋₁₀ cycloalkyl), -CO(3-6-memenber heterocloalkyl), -(C₀₋₁₀ alkyl)COO(C₀₋₁₀ alkyl), -COO(C₃₋₁₀ cycloalkyl), -COO(3-6-memenber heterocloalkyl), alkenyl, alkynyl, said heterocloalkyl contains at least one N, O or S ring atom, wherein, said group is optionally substitured with one or more substituents selected from the group consisting of: halogen, -CN, -NO₂, -CF₃, straight-chain C₁₋₃ alkyl, -OC₀₋₁₀ alkyl, C₃₋₆ cycloalkyl, C₃₋₆ heterocycloalkyl, -N(C₀₋₁₀ alkyl)(C₀₋₁₀ alkyl), -COO(C₀₋₁₀ alkyl), -COO(C₃₋₁₀ cycloalkyl), -COO(heterocycloalkyl), -CO(C₀₋₁₀ alkyl), -OCO(C₀₋₁₀ alkyl), -CON(C₀₋₁₀ alkyl)(C₀₋₁₀ alkyl), -OCOO(C₀₋₁₀ alkyl), phenyl, N-heteroaryl, O-heteroaryl, S-heteroaryl, alkenyl, alkynyl.

3. The compound according to claim 2, or a pharmaceutically acceptable salt thereof, a stereoisomer thereof, a prodrug thereof, a solvate thereof, or a deuterated derivative thereof, **characterized in that** said compound has a structure of formul: 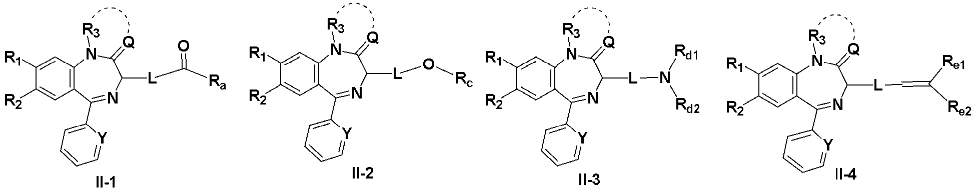
wherein, Y is selected N or -CF;
R₁ and R₂ are each independently selected from -H, -F, -Cl, -Br, -NO₂, -CN, -CF₃, C₂₋₄ alkenyl, C₂₋₄ alkynyl, straight-chain or branched-chai C₁₋₁₀ alkyl, -N(C₀₋₁₀ alkyl)(C₀₋₁₀ alkyl), -OC₀₋₁₀ alkyl, C₃₋₁₀ cycloalky, N-heteroaryl, O-heteroaryl, S-heteroaryl, or R₁ and R₂ together with the carbon atom which they are attached to form five-membered aromatic heterocycle, six-membered aromatic heterocycle or benzene ring; said five-membered aromatic heterocycle is selected from the group consisting of: furan, thiophene, pyrrole, pyrazole, imidazole, oxazole, thiazole; said six-membered aromatic heteroaryl is selected from the group consisting of: pyridine, pyridazine, pyrimidine, pyrazine; optionally, any hydrogen atom on said five-membered aromatic heterocycle, six-membered aromatic heterocycle, or benzene ring may be substituted by substituent selected from the group consisting of: halogen, -CN, -CF₃, straight-chain or branched C₁-₁₀ alkyl, -N(C₀-₁₀ alkyl)(C₀-₁₀ alkyl), -OC₀-₁₀ alkyl, C₃-₁₀ cycloalkyl, N-heterocycloalkyl, O-heterocycloalkyl, S-heterocycloalkyl;
Q is selected from O or N, R₃ is selected from -H, straight-chain or branched-chain C₁₋₅ alkyl, said
the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, said the heterocyclyl contains at least one N, O, or S atom as ring atom;
when Q is N, then R₃ and Q together with N and C atoms which they are attached to form five-membered aromatic heterocycle or six-membered aromatic heterocycle, which containing at least two heteroatoms;
L is none, or L is selected from C₂₋₄ alkenyl or C₁₋₈ alkynyl, wherein, H of the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, 3-10-membered cycloalkyl, -CO(C₀₋₁₀ alkyl), -COO(C₀₋₁₀ alkyl);
Rₐ is selected from-H, straight-chain or branched-chain C₁₋₁₀ alkyl, -OR_{b}, R_{b} is selected from straight-chain or branched-chain C₁₋₁₀ alkyl, C₃₋₁₀ cycloalkyl, 3-8 membered heterocycloalkyl, aryl, heteroaryl, wherein, the aforementioned group is optionally substituents selected from the group consisting of: halogen, -CN, -CH₃, -C₂H₅, -OC₁₋₅, C₃₋₆ cycloalkyl, 3-6 membered heterocycloalkyl, -CO(C₀₋₁₀ alkyl), -OCO(C₀₋₁₀ alkyl), ethenyl, propadienyl, ethynyl; further, the aforementioned alkyl is optionally substituents selected from the group consisting of: -CN, -OCH₂F, -OCHF₂, -OCF₃, -OC₀₋₁₀ alkyl, C₃₋₄ alkyl;
R_{c} is selected from -H, straight-chain or branched-chain C₁₋₁₀ alkyl, C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocycloalkyl), -CH₂CON(C₀₋₁₀ alkyl)(C₀₋₁₀ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocycloalkyl, -OC₁₋₅ alkyl, -N(C₁₋₃ alkyl)(C₁₋₃ alkyl), C₂₋₈ alkenyl, imidazolyl, oxazolyl, thiazolyl, pyridinyl, pyrimidinyl; or R_{c} is selected from 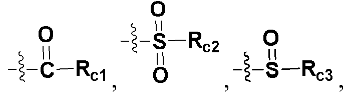 _{c1}, R_{c2}, R_{c3} are independently selected from straight-chain or branched-chain C₁₋₁₀ alkyl, -OC₀₋₅ alkyl, -O(C₃₋₆ cycloalkyl), -O(C₃₋₆ heterocycloalkyl), C₃₋₁₀ cycloalkyl, 3-6 membered heterocycloalkyl, -N(C₀₋₁₀ alkyl)(C₀₋₁₀ alkyl), alkenyl, alkynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocycloalkyl, ethenyl; the aforementioned alkyl moieties are replaceable with substituent selected from the group consisting of: -CN, -OCF₃, -OC₀₋₁₀ alkyl, C₃₋₄ cycloalkyl;
R_{d1} and R_{d2} are independently selected from H, straight-chain or branched-chain C₁₋₁₀ alkyl, -COO(C₀₋₁₀ alkyl), -CO(C₀₋₁₀ alkyl), 3-6 membered cycloalkyl, 3-6 membered heterocycloalkyl, aryl, or R_{d1} and R_{d2} together with the nitrogen atom which they are attached to form 3-6-membered heterocycloalkyl, the hydrogen atom on these groups can be substituted with the following substituents: halogen, -CN, -NO₂, straight-chain or branched-chain C₁₋₃ alkyl, -OC₁₋₃ alkyl, C₃₋₆ cycloalkyl, -(C₀₋₁₀ alkyl)COO(C₀₋₁₀ alkyl), -CO(C₀₋₁₀ alkyl), aryl, -N(C₁₋₃ alkyl)(C₁₋₃ alkyl); the aforementioned alkyl groups are optionally substituted with substituent selected from the group consisting of: -CN, -OCF₃, -OC₀₋₁₀ alkyl, -CO(C₀₋₁₀ alkyl), C₃₋₄ cycloalkyl, aryl;
Rₑ₁ and Rₑ₂ are independently selected from H, straight-chain or branched-chain C₁₋₃ alkyl.

4. The compound according to claim 3, or a pharmaceutically acceptable salt thereof, a stereoisomer thereof, a prodrug thereof, a solvate thereof, or a deuterated derivative thereof, **characterized in that** said compound has a structure of the following formula: 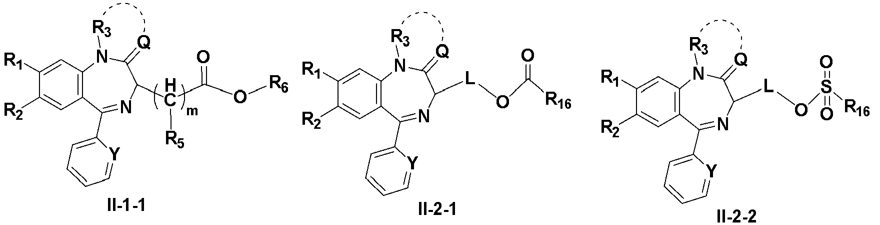 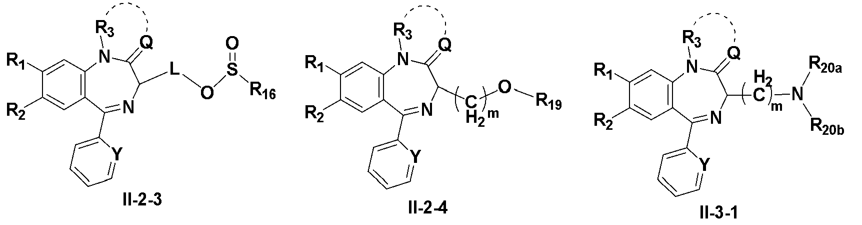
m is an integer from 0 to 4; R₅ is selected from -H, halogen, -CN, -CF₃, -CO(C₀₋₁₀ alkyl), -COO(C₀₋₁₀ alkyl);
R₆ is selected from -H, straight-chain or branched-chain C₁₋₁₀ alkyl, C₃₋₁₀ cycloalkyl, 3-6-membered heterocycloalkyl, aryl, wherein, the hydrogen atom on these groups can be substituted with the following substituents: halogen, -CN, -CH₃, -C₂H₅, -OCH₃, C₃₋₆ cycloalkyl, 3-6-membered heterocycloalkyl, -CO(C₀₋₁₀ alkyl), -OCO(C₀₋₁₀ alkyl), ethenyl, propadienyl, ethynyl, further, the aforementioned alkyl is optionally substituents selected from the group consisting of: -CN, -OCH₂F, -OCHF₂, -OCF₃, -OC₀₋₁₀ alkyl, C₃₋₄ cycloalkyl;
R₁₆ is selected from straight-chain or branched-chain C₁₋₁₀ alkyl, -OC₀₋₅ alkyl, -O(C₃₋₄ cycloalkyl), -O(C₃₋₄ heterocycloalkyl), C₃₋₁₀ cycloalkyl, 3-6 membered heterocycloalkyl, -N(C₀₋₁₀ alkyl)(C₀₋₁₀ alkyl), ethenyl, ethynyl, aryl, pyridinyl, imidazolyl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocycloalkyl, ethenyl, the aforementioned alkyl moieties are replaceable with substituent selected from the group consisting of: -CN, -OCF₃, -OC₀₋₁₀ alkyl, C₃₋₄ cycloalkyl;
R₁₉ is selected from -H, straight-chain or branched-chain C₁₋₁₀ alkyl, -C₃₋₁₀ cycloalkyl, -CH₂CO(C₁₋₅ alkyl), -CH₂COO(C₁₋₅ alkyl), -CH₂COO(C₃₋₆ cycloalkyl), -CH₂COO(C₃₋₆ heterocycloalkyl), -CH₂CON(C₁₋₃ alkyl)(C₁₋₃ alkyl), benzyl, aryl, the hydrogen atom on these groups can be substituted with the following substituents: -F, -Cl, -CN, -NO₂, -CH₃, -C₂H₅, -C₃H₇, 3-6 membered heterocycloalkyl, -OC₁₋₃ alkyl, -N(C₁₋₃ alkyl)(C₁₋₃ alkyl), ethenyl, imidazolyl, oxazolyl, thiazolyl, pyridinyl;
R₂₀ₐ and R_{20b} are independently selected from -H, straight-chain or branched-chain C₁₋₁₀ alkyl, -COO(C₀₋₁₀ alkyl), -CO(C₀₋₁₀ alkyl), C₃₋₅ cycloalkyl, 3-6 membered heterocycloalkyl, aryl, or R_{d1} and R_{d2} together with the nitrogen atom which they are attached to form 3-6 membered N-heterocycloalkyl, the hydrogen atom on these groups can be substituted with the following substituents: -F, -Cl, -CN, -NO₂, -CH₃, -C₂H₅, -OC₁₋₃ alkyl, C₃₋₆ cycloalkyl, -(C₀₋₃ alkyl)COO(C₀₋₅ alkyl), -CO(C₀₋₅ alkyl), -N(C₁₋₃ alkyl)(C₁₋₃ alkyl), aryl, the alkyl on these groups can be substituted with the following substituents: -CN, -OCF₃, -OC₀₋₃ alkyl, -CO(C₀₋₅ alkyl), C₃₋₄ cycloalkyl, aryl.

5. The compound according to claim 4, or a pharmaceutically acceptable salt thereof, a stereoisomer thereof, a prodrug thereof, a solvate thereof, or a deuterated derivative thereof, **characterized in that** said compound has a structure of the following formula: 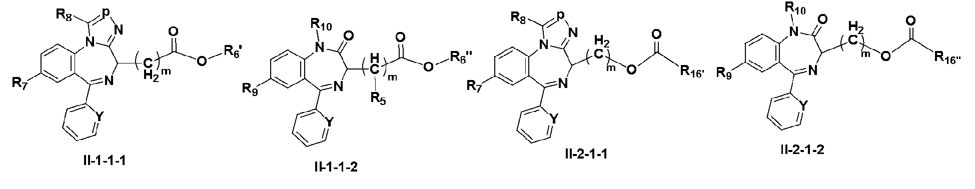 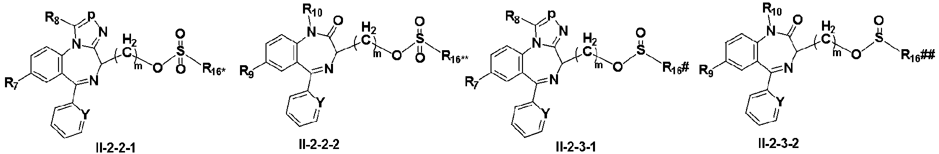 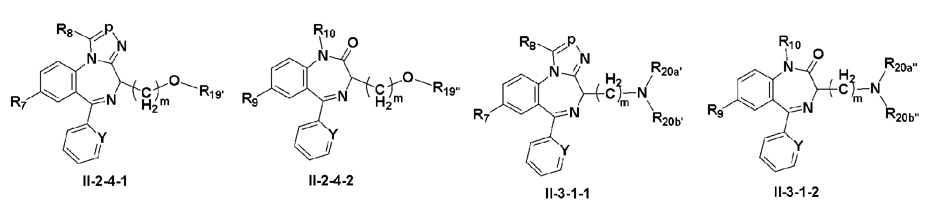
wherein, Y is selected from N or -CF; P is N or CH;
m is an integer from 0 to 4;
R₇ is selected from H, -F, -Cl, -Br, -I, -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₈ is selected from -H, -CN, -CF₃, straight-chain or branched-chain C₁₋₄ alkyl, C₃₋₁₀ cycloalkyl;
R₉ is selected from -H, -F, -Cl, -Br, -I, -NO₂, -CN, alkenyl, alkynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₀ is selected from -H, straight-chain or branched-chain C₁₋₃ alkyl, hydrogen atoms on the alkyl are replaceable with substituent selected from the group consisting of: -N(C₁-C₃ alkyl)₂, 5-membered N-heterocycloalkyl, 6-membered N-heterocycloalkyl;
R_{6'} and R_{6"} are independently selected from -H, straight-chain or branched-chain C₁₋₁₀ alkyl, C₃₋₁₀ cycloalkyl, 3-6 membered heterocycloalkyl, aryl, wherein, the hydrogen atom on these groups can be substituted with the following substituents: halogen, -CN, -CH₃, -C₂H₅, -OCH₃, C₃₋₆ cycloalkyl, 3-6 membered heterocycloalkyl, -CO(C₀₋₁₀ alkyl), -OCO(C₀₋₁₀ alkyl), ethenyl, propadienyl, ethynyl, further, the aforementioned alkyl moieties are replaceable with substituent selected from the group consisting of: -CN, -OCH₂F, -OCHF₂, -OCF₃, -OC₀₋₁₀ alkyl, C₃₋₄ cycloalkyl;
R_{16'}, R_{16"}, R_{16*}, R_{16**}, R_{16#} and R_{16##} are independently selected from straight-chain or branched-chain C₁₋₁₀ alkyl, -OC₀₋₅ alkyl, -O(C₃₋₄ cycloalkyl), -O(C₃₋₄ heterocycloalkyl), C₃₋₁₀ cycloalkyl, 3-6 membered heterocycloalkyl, -N(C₀₋₁₀ alkyl)(C₀₋₁₀ alkyl), ethenyl, ethynyl, aryl, pyridyl, imidazolyl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocycloalkyl, ethenyl, the aforementioned alkyl moieties are replaceable with a substituent selected from the group consisting of: -CN, -OCF₃, -OC₀₋₁₀ alkyl, C₃₋₄ cycloalkyl;
R_{19'} and R_{19"} are independently selected from -H, straight-chain or branched-chain -C₁₋₁₀ alkyl, -C₃₋₁₀ cycloalkyl, -CH₂CO(C₁₋₅ alkyl), -CH₂COO(C₃₋₆ alkyl), -CH₂COO(C₃₋₆ cycloalkyl), -CH₂COO(C₃₋₆ heterocycloalkyl), -CH₂CON(C₁₋₃ alkyl)(C₁₋₃ alkyl), benzyl, aryl, the hydrogen atom on these groups can be substituted with the following substituents: -F, -Cl, -CN, -NO₂, -CH₃, -C₂H₅, -C₃H₇, 3-6 membered heterocycloalkyl, -OC₁₋₃ alkyl, -N(C₁₋₃ alkyl)(C₁₋₃ alkyl), ethenyl, imizazole, oxazolyl, thiazolyl, pyridinyl;
R_{20a'}, R_{20a"}, R_{20b'} and R_{20b"}, are independently selected from -H, straight-chain or branched-chain C₁₋₁₀ alkyl, -COO(C₀₋₁₀ alkyl), -CO(C₀₋₁₀ alkyl), C₃₋₅ cycloalkyl, 3-6 membered heterocycloalkyl, aryl, or R_{d1} and R_{d2} are taken together with the N atom to which they are attached to form 3-6 membered N-heterocycloalkyl, the hydrogen atom on these groups can be substituted with the following substituents: -F, -Cl, -CN, -NO₂, -CH₃, -C₂H₅, -OC₁₋₃ alkyl, C₃₋₆ cycloalkyl, -(C₀₋₃ alkyl)COO(C₀₋₅ alkyl), -CO(C₀₋₅ alkyl), -N(C₁₋₃ alkyl)(C₁₋₃ alkyl), aryl, the aforementioned alkyl moieties are replaceable with substituent selected from the group consisting of: -CN, -OCF₃, -OC₀₋₃ alkyl, -CO(C₀₋₅ alkyl), C₃₋₄ cycloalkyl, aryl.

6. The compound according to claim 5, or a pharmaceutically acceptable salt thereof, a stereoisomer thereof, a prodrug thereof, a solvate thereof, or a deuterated derivative thereof, **characterized in that** said compound has a structure of the following formula: 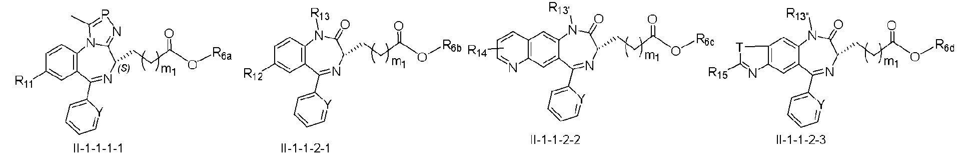
wherein, Y is selected from N or -CF; P is N or CH;
m₁ is an integer from 0 to 2;
R₁₁ is selected from -F, -Cl, -Br, -I, -NO₂, ethyne, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S- heterocycloalkyl;
R₆ₐ is selected from 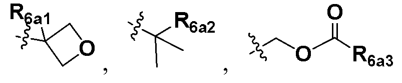 or C₁₋₄ alkyl, the alkyl is optionally substituted by one or more substituents selected from -F, -OCH₃, C₃₋₆ cycloalkyl, R₆ₐ₁ is selected from -H, -CN, -CH₃, -C₂H₅, ethenyl, propadienyl, ethynyl; R₆ₐ₂ is selected from ethenyl, ethynyl, -COCH₃, -COC₂H₅, 3-4 membered epoxyalkyl; R₆ₐ₃ is selected from -CH₃, -C₂H₅, -OCH₃, -OC₂H₅;
R₁₂ is selected from -F, -Cl, -Br, -I, -NO₂, ethynyl, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₃, R_{13'}, R_{13"} are independently selected from -H, straight-chain or branched-chain C₁₋₃ alkyl, the alkyl is optionally substituted by one or more substituents selected from -N(C₁₋₃ alkyl)(C₁₋₃ alkyl), 5-membered N-heterocycloalkyl, 6-membered N-heterocycloalkyl;
R_{6b} is selected from -H, -CH₃, -C₂H₅, propyl, isopropyl, butyl, tert-Butyl, 3-4 membered saturated oxacycloalkyl, the hydrogen atom on these groups can be substituted with the following substituents: -F, -Cl, -CN, -CH₃, -C₂H₅, -OCH₃, C₃₋₄ cycloalkyl, 3-6 membered heterocycloalkyl, -CO(C₁₋₃ alkyl), -OCO(C₁₋₃ alkyl), ethylene, ethynyl, further, the alkyl group is optionally substituted by -OCH₃, -OC₂H₅;
R₁₄ is selected from -H, halogen, -NO₂, -CN, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl;
R_{6c} is selected from -H, traight-chain or branched-chain C₁₋₅ alkyl, -OC₀₋₅ alkyl, C₃₋₆ cycloalkyl, O-heterocycloalkyl, the hydrogen atom on these groups can be substituted with the following substituents: -F, -Cl, -CN, -CH₃, -C₂H₅, -OCH₃, C₃₋₄ cycloalkyl, ethylene, ethynyl;
T is selected from C, N, O or S;
R₁₅ is selected from -H, halogen, -NO₂, -CN, -CF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl;
R_{6d} is selected from -H, traight-chain or branched-chain C₁₋₅ alkyl, -OC₀₋₅ alkyl, C₃₋₆ cycloalkyl, O-heterocycloalkyl, the hydrogen atom on these groups can be substituted with the following substituents: -F, -Cl, -CN, -CH₃, -C₂H₅, -OCH₃, C₃₋₄ cycloalkyl, ethylene, ethynyl.

7. The compound according to claim 5, or a pharmaceutically acceptable salt thereof, a stereoisomer thereof, a prodrug thereof, a solvate thereof, or a deuterated derivative thereof, **characterized in that** said compound has a structure of the following formula: 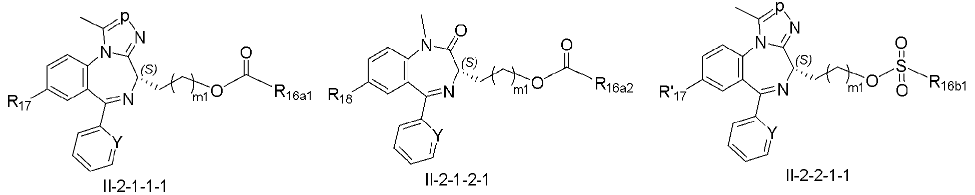 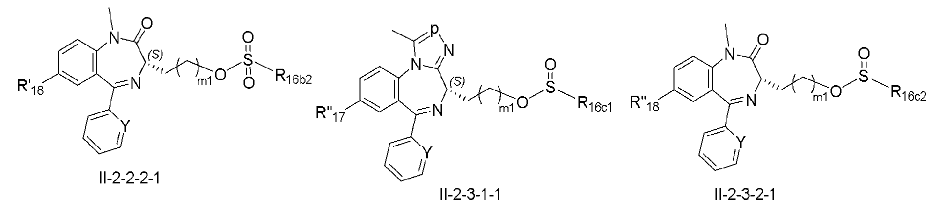
wherein, Y is selected from N or -CF;
m₁ is an integer from 0 to 2;
R₁₇, R'₁₇, R"₁₇, R₁₈, R'₁₈, R"₁₈ are independently selected from -F, -Cl, -Br, -I, -NO₂, ethynyl, -CF₃, straight-chain or branched-chain C₁₋₁₀ alkyl, -N(C₀₋₁₀ alky)(C₀₋₁₀ alky), -OC₀₋₁₀oalky, C₃₋₁₀ cycloalkyl, N-heterocycloalkyl, O-heterocycloalkyl, S-heterocycloalkyl;
R₁₆ₐ₁ and R₁₆ₐ₂ are independently selected from sraight-chain or branched-chain C₁₋₅ alkyl, -OC₁₋₃ alkyl, -O(C₃₋₄ cycloalkyl), -O(C₃₋₄ heterocycloalkyl), C₃₋₆ cycloalkyl, 3-6 membered N-heterocycloalkyl, 3-6 membered O-heterocycloalkyl, -N(C₀₋₁₀ alkyl)(C₀₋₁₀ alkyl), ethylene, ethynyl, aryl, pyridinyl, imidazolyl, the hydrogen atom on these groups can be substituted with the following substituents: halogen, -CN, -CH₃, -C₂H₅, -OCH₃, -N(C₁₋₃ alkyl)(C₁₋₃ alkyl), ethylene;
R_{16b1} and R_{16b} are independently selected from sraight-chain or branched-chain C₁₋₅ alkyl, -OC₁₋₃ alkyl, C₃₋₆ cycloalkyl, aryl, -N(C₁₋₃ alkyl)(C₁₋₃ alkyl), the hydrogen atom on these groups can be substituted with the following substituents: halogen, -CN, -CH₃, -C₂H₅, -OCH₃;
R_{16c1} and R_{16c2} are independently selected from sraight-chain or branched-chain C₁₋₅ alkyl, -OC₁₋₃ alkyl, C₃₋₆ cycloalkyl, aryl.

8. The compound according to claim 5, or a pharmaceutically acceptable salt thereof, a stereoisomer thereof, a prodrug thereof, a solvate thereof, or a deuterated derivative thereof, **characterized in that** said compound has a structure of the following formula: 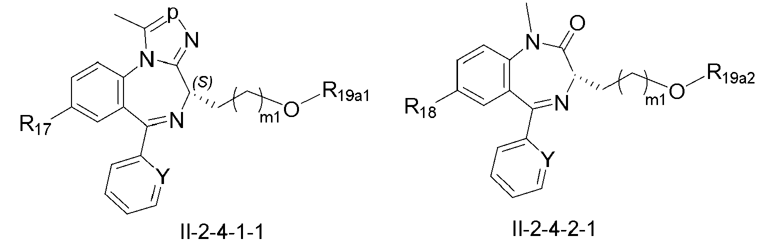
wherein, Y is selected from N or -CF;
m₁ is an integer from 0 to 2;
R₁₇ and R₁₈ are independently selected from -F, -Cl, -Br, -I, -NO₂, ethynyl, -CF₃, sraight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₁₉ₐ₁ and R₁₉ₐ₂ are independently selected from -H, sraight-chain or branched-chain C₁₋₃ alkyl, C₃₋₆ cycloalkyl, -CH₂CO(C₁₋₃ alkyl), -CH₂COO(C₁₋₃ alkyl), -CH₂COO(C₃₋₆ heterocycloalkyl), -CH₂CON(C₁₋₃ alkyl)(C₁₋₃ alkyl), benzyl, aryl, the hydrogen atom on these groups can be substituted with the following substituents: -F, -Cl, -CN, -NO₂, -CH₃, -C₂H₅, -C₃H₇, -OC₁₋₃ alkyl, -N(C₁₋₃ alkyl)(C₁₋₃ alkyl), ethylene, imidazolyl, thiophene, pyridinyl.

9. The compound according to claim 5, or a pharmaceutically acceptable salt thereof, a stereoisomer thereof, a prodrug thereof, a solvate thereof, or a deuterated derivative thereof, **characterized in that** said compound has a structure of the following formula: 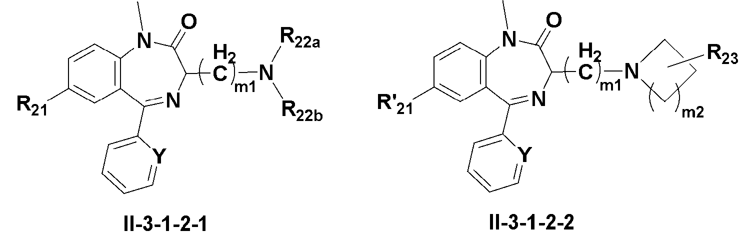
wherein, Y is selected from N or -CF;
m₁ is an integer from 0 to 2;
R₂₁ and R'₂₁ are independently selected from -F, -Cl, -Br, -I, -NO₂, ethynyl, -CF₃, sraight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocycloalkyl, O-heterocycloalkyl, S-heterocycloalkyl;
R₂₂ₐ and R_{22b} are independently selected from -H, sraight-chain or branched-chain C₁₋₃ alkyl, -COO(C₁₋₃ alkyl), -CO(C₁₋₃ alkyl), C₃₋₅ cycloalkyl, 3-6 membered heterocycloalkyl, aryl, the hydrogen atom on these groups can be substituted with the following substituents: -F, -Cl, -CN, -NO₂, -CH₃, -C₂H₅, -OCH₃, -(C₀₋₃ alkyl)COO(C₁₋₃ alkyl);
m₂ is an integer from 1 to 3;
R₂₃ is independently selected from -H, sraight-chain or branched-chain C₁₋₃ alkyl, -COO(C₁₋₃ alkyl), -CO(C₁₋₃ alkyl), C₃₋₅ cycloalkyl, 3-6 membered heterocycloalkyl, -N(C₀₋₃ alkyl)(C₀₋₃ alkyl), aryl, the hydrogen atom on these groups can be substituted with the following substituents: -F, -Cl, -CN, -NO₂, -CH₃, -C₂H₅, -OC₁₋₃ alkyl, C₃₋₆ cycloalkyl, -(C₀₋₃ alkyl)COO(C₀₋₅ alkyl), -CO(C₀₋₅ alkyl), aryl.

10. The compound according to claim 1, or a pharmaceutically acceptable salt thereof, a stereoisomer thereof, a prodrug thereof, a solvate thereof, or a deuterated derivative thereof, **characterized in that** said compound has a structure of the following formula: 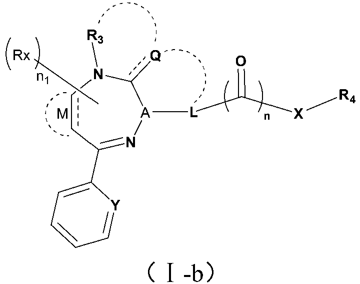
wherein, Y is selected from N or -CF;
M is selected from none, or M together with the adjacent carbon atom which they are attached to form fused ring, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl; when M together with the adjacent carbon atom which they are attached to form fused ring, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
Q is selected from O, S-Rxx, N or 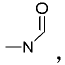 R₃ is selected from -H, straight-chain or branched-chain
C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O or S atom as ring atom;
Rxx is selected from H, straight-chain or branched-chain C₁₋₅ alkyl, said the H of alkyl which are optionally substituents selected from the group consisting of: halogen, nitro, cyano, -OC₀₋₁₀ alkyl;
when Q is N, R₃ and Q are together with the N and C atoms which they are attached to form five-membered aromatic heterocycle or six-membered aromatic heterocycle containing at least two heteroatoms;
L is none, or L is selected from C₂₋₈ alkenyl, C₁₋₈ alkylene, wherein, the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, -CO(C₀₋₁₀ alkyl), -COO(C₀₋₁₀ alkyl);
when Q is selected from N or 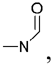 and A is N, then Q, A, L together with the adjacen atom which they are attached to form five-membered N-heterocycle or six-membered N-heterocycle;
n is 0 or 1, when n is 0, it means the -CO- is none;
X is none, or when n is 1, X is O, when n is 0, X is selected from O, substituted or unsubstituted N-alkyl, substituted or unsubstituted N-heterocycalkyl;
R₄ is selected from-H, halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, heterocyclic, aryl, N-heteroaryl, O-heteroaryl, S-heteroaryl, -SO₂(C₀₋₁₀ alkyl), -SO(C₀₋₁₀ alkyl), -SO₂O(C₀₋₁₀ alkyl), -SO₂N(C₀₋₁₀ alkyl)(C₀₋₁₀ alkyl), -SO₂(C₃₋₁₀ cycloalkyl), -SO₂-aryl, -CON(C₀₋₁₀ alkyl)(C₀₋₁₀ alkyl), -CO(C₀₋₁₀ alkyl), -CO(C₃₋₁₀ cycloalkyl), -CO(3-6-memenber heterocloalkyl), -(C₀₋₁₀ alkyl)COO(C₀₋₁₀ alkyl), -COO(C₃₋₁₀ cycloalkyl), -COO(3-6-memenber heterocloalkyl), alkenyl, alkynyl, said heterocloalkyl contains at least one N, O or S ring atom, wherein, said group is optionally substitured with one or more substituents selected from the group consisting of: halogen, -CN, -NO₂, -CF₃, straight-chain C₁₋₃ alkyl, -OC₀₋₁₀ alkyl, C₃₋₆ cycloalkyl, C₃₋₆ heterocycloalkyl, -N(C₀₋₁₀ alkyl)(C₀₋₁₀ alkyl), -COO(C₀₋₁₀ alkyl), -COO(C₃₋₁₀ cycloalkyl), -COO(heterocycloalkyl), -CO(C₀₋₁₀ alkyl), -OCO(C₀₋₁₀ alkyl), -CON(C₀₋₁₀ alkyl)(C₀₋₁₀ alkyl), -OCOO(C₀₋₁₀ alkyl), phenyl, N-heteroaryl, O-heteroaryl, S-heteroaryl, alkenyl, alkynyl.

11. The compound according to claim 1 or claim 10, or a pharmaceutically acceptable salt thereof, a stereoisomer thereof, a prodrug thereof, a solvate thereof, or a deuterated derivative thereof, **characterized in that** said M together with the adjacent carbon atom form the group selected from: saturated or unsaturated 3-8 membered cycloalkyl, saturated or unsaturated 3-8-membered heterocyclic, phenyl, 5-membered heteroaryl or 6-membered heteroaryl, heteroaryl ring formed by benzene ring fused with one or two five-membered or six-membered monocyclic heteroaryl groups, or heteroaryl ring formed by two or three five-membered and/or six-membered monocyclic heteroaryl groups fused together;
wherein, the hydrogen atom on these groups can be substituted with the following substituents: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heteroaryl, O-heteroaryl, S-heteroaryl, -NHCO(C₀₋₁₀ alkyl).

12. The compound according to claim 11, or a pharmaceutically acceptable salt thereof, a stereoisomer thereof, a prodrug thereof, a solvate thereof, or a deuterated derivative thereof, **characterized in that** said M together with the adjacent carbon atom which they are attached to form the group selected from: saturated or unsaturated 3-6 membered cycloalkyl, saturated or unsaturated 3-6 membered heterocyclic, phenyl, pyrrole, thiophene, furan, pyridine, pyrimidine, pyrazine, triazine, imidazole, oxazole, thiazole, pyrazole, benzofuran, benzoxazole, benzimidazole, benzothiophene, benzothiazole, indole, or imidazopyridine;
wherein said 3- 6 membered saturated or unsaturated heterocycle optionally contains one or more heteroatoms selected from N, O, and S;
wherein, the hydrogen atom on these groups can be substituted with the following substituents: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heteroaryl, O-heteroaryl, S-heteroaryl, -NHCO(C₀₋₁₀ alkyl).

13. The compound according to claim 10, or a pharmaceutically acceptable salt thereof, a stereoisomer thereof, a prodrug thereof, a solvate thereof, or a deuterated derivative thereof, **characterized in that** said compound has a structure of the following formula: 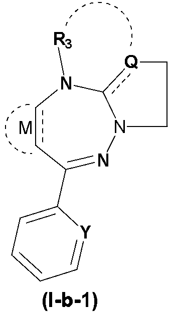
wherein, Y is selected from N or -CF;
M is selected from none, or M together with the adjacent carbon atom which they are attached to form fused ring, together forming saturated or unsaturated cycloalkyl, saturated or unsaturated heterocyclyl, aryl or heteroaryl;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
Q is selected from N or 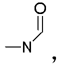 R₃ is selected from -H, straight-chain or branched-chain
C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl,
-COO(C₀₋₁₀ alkyl), preferably, said the H of alkyl which are optionally substituents selected from the group consisting of: -N(C₀₋₁₀ alkyl)(C₀₋₁₀ alkyl), -OC₁₋₆ alkyl, C₃₋₆ cycloalkyl, 3-6-membered heterocyclyl, -COO(C₁₋₆ alkyl); the heterocyclyl contains at least one N, O or S atom as ring atom;
when Q is N, R₃ and Q together with the N and C atom which they are attached to form five-membered heteroaryl or six-membered heteroaryl, which containing at least two heteroatoms.

14. The compound according to claim 13, or a pharmaceutically acceptable salt thereof, a stereoisomer thereof, a prodrug thereof, a solvate thereof, or a deuterated derivative thereof, **characterized in that** said compound has a structure of the following formula: 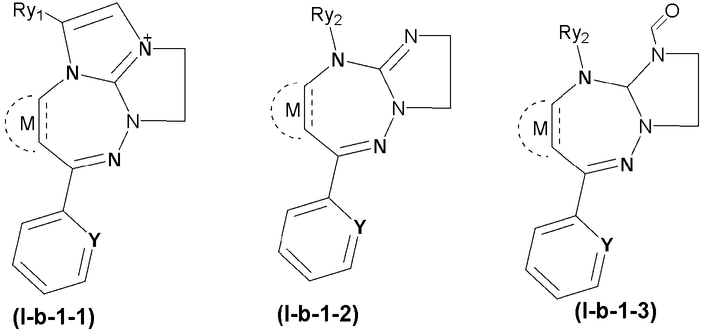
wherein, Y is selected from N or -CF;
M is selected from none, or M together with the adjacent carbon atom which they are attached to form fused ring, together forming saturated or unsaturated cycloalkyl, saturated or unsaturated heterocyclyl, aryl or heteroaryl;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
R_{y1} is selected from -H, -CN, -CF₃, straight-chain or branched-chain C₁₋₄ alkyl, C₃₋₁₀ cycloalkyl;
R_{y2} is selected from -H, straight-chain or branched-chain C₁₋₃ alkyl, the hydrogen atom on said alkyl may be replaced by the substituent selected from the group consisting of: -N(C₁₋₃ alkyl)(C₁₋₃ alkyl), -COO(C₀₋₅ alkyl), 5-membered N-heterocycloalkyl, 6-membered N-heterocycloalkyl.

15. The compound according to claim 10, or a pharmaceutically acceptable salt thereof, a stereoisomer thereof, a prodrug thereof, a solvate thereof, or a deuterated derivative thereof, **characterized in that** said compound has a structure of the following formula: 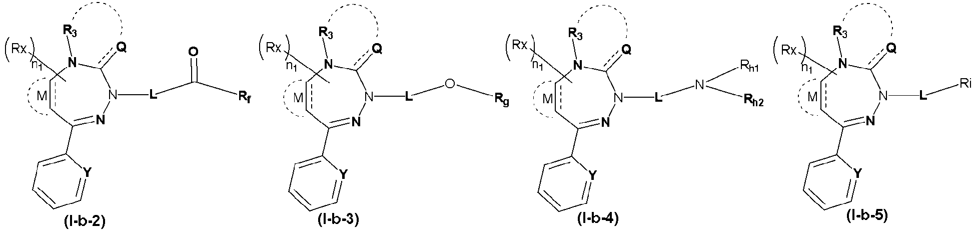
wherein, Y is selected from N or -CF;
M is selected from none, or M together with the adjacent carbon atom which they are attached to form fused ring, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl,
C₃₋₁₀ cycloalkyl, N-heterocyclyl, O-heterocyclyl, S-heterocyclyl; when M together with the adjacent carbon atom which they are attached to form fused ring, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
Q is selected from O, S-Rxx, N, R₃ is selected from -H, straight-chain or branched-chain C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8-membered heterocyclyl, -COO(C₀₋₁₀ alkyl), said the heterocyclyl contains at least one N, O, or S atom as ring atom;
when Q is N, R₃ and Q together with the N and C atom which they are attached to form five-membered heteroaryl or six-membered heteroaryl, which containing at least two heteroatoms;
Rxx is selected from H, straight-chain or branched-chain C₁₋₅ alkyl, said the H of alkyl which are optionally substituents selected from the group consisting of: halogen, nitro, cyano, -OC₀₋₁₀ alkyl;
L is none, or L is selected from C₂₋₈ alkenyl, C₁₋₈ alkylene, wherein, the aforementioned group is optionally substituted with one or more substituents selected from the group consisting of: halogen, -CN, -CF₃, -OCH₂F, -OCHF₂, -OCF₃, straight-chain or branched-chain C₁₋₁₀ alkyl, -N(C₀₋₁₀ alkyl)(C₀₋₁₀ alkyl), -OC₀₋₁₀ alkyl, 3-10-membered cycloalkyl, -CO(C₀₋₁₀ alkyl), -COO(C₀₋₁₀ alkyl);
R_{f} is selected from H, straight-chain or branched-chain C₁₋₁₀ alkyl, ORⱼ, Rⱼ is selected from straight-chain or branched-chain C₃₋₁₀ alkyl, C₃₋₁₀ cycloalkyl, 3-8 membered heterocyclyl, aryl, heteroaryl, wherein, the hydrogen atom on these groups can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, ethynyl, further, said alkyl may be substituted by substituent selected from the group consisting of: -CN, -OCH₂F, -OCHF₂, -OCF₃, -OC₀₋₁₀ alkyl, C₃₋₄ cycloalkyl;
R_{g} is selected from H, straight-chain or branched-chain C₁₋₁₀ alkyl, -C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocycloalkyl), -CH₂CON(C₀₋₁₀ alkyl)(C₀₋₁₀ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OC₁₋₅ alkyl, -N(C₁₋₃ alkyl)(C₁₋₃ alkyl), C₂₋₈ alkenyl, imidazolyl, oxazolyl, thiazolyl, pyridinyl, pyrimidinyl, pheny; or R_{g} is selected from 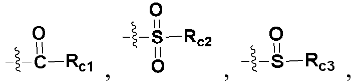 R_{c1}, R_{c2}, R_{c3} are independently selected from straight-chain or branched-chain C₁₋₁₀ alkyl, -OC₀₋₅ alkyl, -O(C₃₋₆ cycloalkyl), -O(C₃₋₆ heterocycloalkyl), C₃₋₁₀ cycloalkyl, 3-6 membered heterocyclyl, -N(C₀₋₁₀ alkyl)(C₀₋₁₀ alkyl), alkenyl, alkynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl, ethenyl, further, said alkyl is substituted with substituent selected from the group consisting of: -CN, -OCF₃, -OC₀₋₁₀ alkyl, C₃₋₄ cycloalkyl;
Rₕ₁ and Rₕ₂ are independently selected from H, straight-chain or branched-chain C₁₋₁₀ alkyl, -COO(C₀₋₁₀ alkyl), -CO(C₀₋₁₀ alkyl), 3-6 membered cycloalkyl, 3-6 membered heterocyclyl, aryl, or Rh¹ and Rh² together with the nitrogen atom which they are attached to form 3-6-membered heterocycloalkyl, the hydrogen atom on these groups can be substituted with the following substituents: halogen, -CN, -NO₂, straight-chain or branched-chain C₁₋₃ alkyl, -OC₁₋₃ alkyl, C₃₋₆ cycloalkyl, -(C₀₋₁₀ alkyl)COO(C₀₋₁₀ alkyl), -CO(C₀₋₁₀ alkyl), aryl, -N(C₁₋₃ alkyl)(C₁₋₃ alkyl), further, said alkyl is substituted with substituent selected from the group consisting of: -CN, -OCF₃, -OC₀₋₁₀, -CO(C₀₋₁₀ alkyl), C₃₋₄ cycloalkyl, aryl;
Rⱼ is selected from halogen, cyano, aryl, heteroaryl, 3-6 membered cycloalkyl or 3-6 membered heterocyclyl; the hydrogen atom on these groups can be substituted with the following substituents: halogen, -CN, -NO₂, straight-chain or branched-chain C₁₋₁₀ alkyl, -OC₁₋₁₀ alkyl.

16. The compound according to claim 15, or a pharmaceutically acceptable salt thereof, a stereoisomer thereof, a prodrug thereof, a solvate thereof, or a deuterated derivative thereof, **characterized in that** said compound has a structure of the following formula: 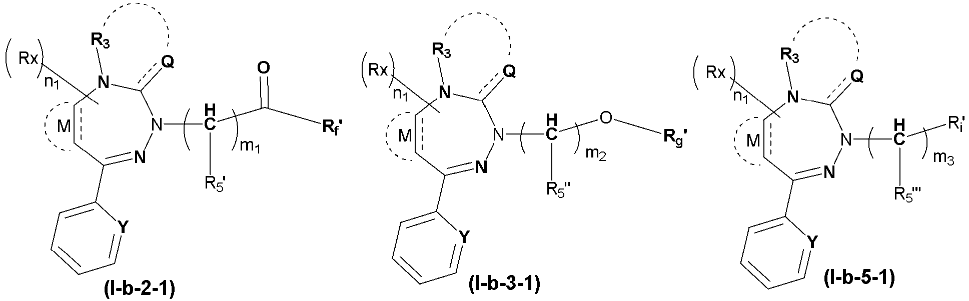
wherein, Y is selected from N or -CF;
M is selected from none, or M together with the adjacent carbon atom which they are attached to form fused ring, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl,
C₃₋₁₀ cycloalkyl, N-heterocyclyl, O-heterocyclyl, S-heterocyclyl; when M togerher with the adjacent carbon which they are attached to form fused ring, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
Q is selected from O, S-Rxx or N, R₃ is selected from -H, straight-chain or branched-chain
C₁₋₅ alkyl, said the H of alkyl which are optionally substituents selected from the group consisting of: -N(C₀₋₁₀ alkyl)(C₀₋₁₀ alkyl), -OC₀₋₁₀ alkyl, C₃₋₁₀ cycloalkyl, 3-8 membered heterocyclyl,
-COO(C₀₋₁₀ alkyl), said the heterocyclyl contains at least one N, O or S atom as ring atom;
When Q is N, R₃ and Q together with the N and C atom which they are attached to form five-membered heteroaryl or six-membered heteroaryl, which containing at least two heteroatoms;
Rxx is selected from H, straight-chain or branched-chain C₁₋₅ alkyl, said the H of alkyl which are optionally substituents selected from the group consisting of: halogen, nitro, cyano, -OC₀₋₁₀ alkyl;
m₁, m₂, m₃ are independently selected from integer from 0 to 5;
R₅', R₅", R₅‴ are independently selected from H, halogen, -CN, -CF₃, -C₁₋₁₀ alkyl, -CO(C₀₋₁₀ alkyl), -COO(C₀₋₁₀ alkyl);
R_{f}' is selected from -H, straight-chain or branched-chain C₁₋₁₀ alkyl, -N(C₀₋₁₀ alkyl)(C₀₋₁₀ alkyl), ORⱼ', Rⱼ' is selected from straight-chain or branched-chain C₁₋₁₀ alkyl, C₃₋₁₀ cycloalkyl, 3-8 membered heterocyclyl, aryl, heteroaryl, wherein, the hydrogen atom on these groups can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, and ethynyl, futermore, said alkyl may be substituted by substituent selected from the group consisting of: -CN, -OCH₂F, -OCHF₂, -OCF₃, -OC₀₋₁₀ alkyl, C₃₋₄ cycloalkyl;
R_{g}' is selected from -H, straight-chain or branched-chain C₁₋₁₀ alkyl, -C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocyclyl), -CH₂CON(C₀₋₁₀ alkyl)(C₀₋₁₀ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OC₁₋₅ alkyl, -N(C₁₋₃ alkyl)(C₁₋₃ alkyl), C₂₋₈ alkenyl, imidazolyl, oxazolyl, thiophene, pyridinyl, pyrimidinyl, phenyl; or R_{g}' is selected from 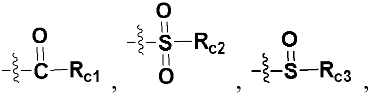 R_{c1}, R_{c2}, R_{c3} are independently selected from straight-chain or branched-chain C₁₋₁₀ alkyl, -OC₀₋₅ alkyl, -O(C₃₋₆ cycloalkyl), -O(C₃₋₆ heterocyclyl), C₃₋₁₀ cycloalkyl, 3-6 membered heterocyclyl, -N(C₀₋₁₀ alkyl)(C₀₋₁₀ alkyl), alkenyl, alkynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl, ethenyl, said alkyl may be substituted by substituent selected from the group consisting of: -CN, -OCF₃, -OC₀₋₁₀ alkyl, C₃₋₄ cycloalkyl;
Rᵢ' is selected from halogen, cyano, phenyl, 5-6 membered heteroaryl containing one or more heteroatoms selected from N, O, and S, 3-6 membered cycloalkyl or 3-6 membered heterocyclyl; the hydrogen atom on these groups can be substituted with the following substituents: halogen, -CN, -NO₂, straight-chain or branched-chain C₁₋₆ alkyl, -OC₁₋₆ alkyl.

17. The compound according to claim 16, or a pharmaceutically acceptable salt thereof, a stereoisomer thereof, a prodrug thereof, a solvate thereof, or a deuterated derivative thereof, **characterized in that** said compound has a structure of the following formula: 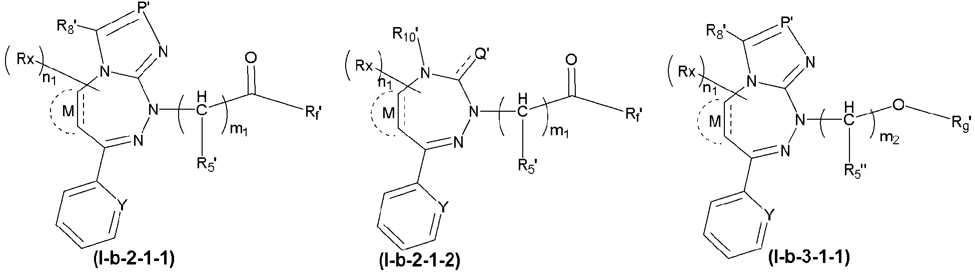 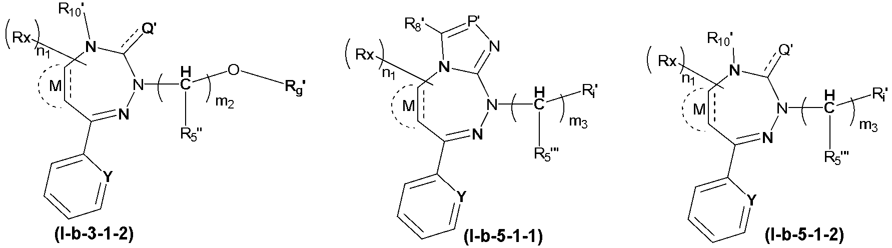
wherein, Y is selected from N or -CF; P' is selected from N or CH;
M is selected from none, or M together with the adjacent carbon atom which they are attached to form fused ring, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl,
C₃₋₁₀ cycloalkyl, N-heterocyclyl, O-heterocyclyl, S-heterocyclyl; when M forms fused ring with the adjacent carbon, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
R₈' is selected from -H, halogen, -CN, -NO₂, -CF₃, straight-chain or branched-chain C₁₋₄ alkyl, C₃₋₁₀ cycloalkyl;
R₁₀' is selected from -H, straight-chain or branched-chain C₁₋₃ alkyl, the hydrogen atom on these groups can be substituted with the following substituents: -N(C₁₋₃ alkyl)(C₁₋₃ alkyl), -CO(C₀₋₁₀ alkyl), -COO(C₀₋₁₀ alkyl), 5-membered N-heterocyclyl, 6-membered N-heterocyclyl;
Q' is selected from =O or S-Rxx; Rxx is selected from H, straight-chain or branched-chain C₁₋₃ alkyl, said the H of alkyl which are optionally substituents selected from the group consisting of: halogen, nitro, cyano, -OC₀₋₁₀ alkyl;
m₁, m₂, m₃ are independently selected from integer from 0 to 4;
R₅', R₅", R₅‴ are independently selected from H, halogen, -CN, -CF₃, -C₁₋₁₀ alkyl, -CO(C₀₋₁₀ alkyl), -COO(C₀₋₁₀ alkyl);
R_{f}' is selected from -H, straight-chain or branched-chain C₁₋₁₀ alkyl, -N(C₀₋₁₀ alkyl)(C₀₋₁₀ alkyl), ORⱼ', Rⱼ' is selected from straight-chain or branched-chain C₁₋₁₀ alkyl, C₃₋₁₀ cycloalkyl, 3-8 membered heterocyclyl, aryl, heteroaryl, wherein, the hydrogen atom on these groups can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, ethynyl, further, the aforementioned alkyl moieties are replaceable with a substituent selected from the group consisting of: -CN, -OCH₂F, -OCHF₂, -OCF₃, -OC₀₋₁₀ alky, C₃₋₄ cycloalkyl;
R_{g}' is selected from -H, straight-chain or branched-chain C₁₋₁₀ alkyl, -C₃₋₁₀ cycloalkyl, -CH₂CO(C₀₋₁₀ alkyl), -CH₂COO(C₀₋₆ alkyl), -CH₂COO(C₃₋₆ cycloalkyl), -CH₂COO(C₃₋₆ heterocyclyl), -CH₂CON(C₀₋₁₀ alkyl)(C₀₋₁₀ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OC₁₋₅ alkyl, -N(C₁₋₃ alkyl)(C₁₋₃ alkyl), C₂₋₈ alkeny, imidazolyl, oxazolyl, thiazolyl, pyridinyl, pyrimidinyl, phenyl; or, R_{g}' is selected from 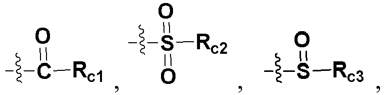 R_{c1}, R_{c2}, R_{c3} are independently selected from straight-chain or branched-chain C₁₋₁₀ alkyl, -OC₀₋₅ alkyl, -O(C₃₋₆ cycloalkyl), -O(C₃₋₆ heterocyclyl), C₃₋₁₀ cycloalkyl, 3-6 membered heterocyclyl, -N(C₀₋₁₀ alkyl)(C₀₋₁₀ alkyl), alkenyl, alkynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl, ethenyl, the aforementioned alkyl moieties are replaceable with a substituent selected from the group consisting of: -CN, -OCF₃, -OC₀₋₁₀ alkyl, C₃₋₄ cycloalkyl;
Rᵢ' is selected from halogen, cyano, phenyl, 5-6 membered heteroaryl containing N, O and/or S, 3-6 membered cycloalkyl or 3-6 membered heterocyclyl; the hydrogen atom on these groups can be substituted with the following substituents: halogen, -CN, -NO₂, straight-chain or branched-chain C₁₋₆ alkyl, -OC₁₋₆ alkyl.

18. The compound according to claim 17, or a pharmaceutically acceptable salt thereof, a stereoisomer thereof, a prodrug thereof, a solvate thereof, or a deuterated derivative thereof, **characterized in that** said Y is selected from N or -CF; P' is N or CH;
M is selected from none, or M together with the adjacent carbon atom which they are attached to form fused ring, together forming saturated or unsaturated cycloalkyl, saturated or unsaturated heterocyclyl, aryl or heteroaryl;
when M is selected from none, Rx is selected from -H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl,
C₃₋₁₀ cycloalkyl, N-heterocyclyl, O-heterocyclyl, S-heterocyclyl; when M together with the adjacent carbon atom which they are attached to form fused ring, Rx is absent;
n₁ is 0, 1, 2;
the H of above in saturated or unsaturated cycloalkyl, saturated or unsaturated heterocyclyl, aryl or heteroaryl, which are optionally substituents selected from the group consisting of: halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
R₈' is selected from -H or methyl;
R₁₀' is selected from -H, straight-chain or branched-chain C₁₋₃ alkyl, the hydrogen atom on alkyl can be substituted with the following substituents: -N(C₁₋₃ alkyl)(C₁₋₃ alkyl), -CO(C₁₋₃ alkyl), -COO(C₁₋₃ alkyl), piperazinyl, N-methylpiperazinyl or N-ethylpiperazinyl;
Q' is selected from =O or -S-Rxx; Rxx is selected from H or methyl;
m₁, m₂, m₃ are independently selected from integer from 0 to 4;
R₅', R₅", R₅‴ are independently selected from -H, halogen, -CN, -CF₃, -C₁₋₆ alkyl, -CO(C₀₋₁₀ alkyl), -COO(C₀₋₁₀ alkyl);
R_{f}' is selected from -H, straight-chain or branched-chain C₁₋₆ alkyl, -N(C₀₋₆ alkyl)(C₀₋₆ alkyl), ORⱼ', Rⱼ' is selected from straight-chain or branched-chain C₁₋₆ alkyl, cyclopropyl, cyclobutyl, cyclopentyl, cyclohexyl, phenyl, thiophenyl, furanyl, pyrrolyl, pyridinyl or the following group: 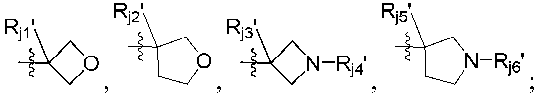
Rⱼ₁', Rⱼ₂', Rⱼ₃', Rⱼ₄', Rⱼ₅', Rⱼ₆' are independently selected from H, halogen, -CN, straight-chain or branched-chain C₁₋₆ alkyl, straight-chain or branched-chain C₂₋₆ alkenyl, straight-chain or branched-chain -OC₁₋₆ alkyl, C₃₋₆ cycloalkyl, 3-6 membered heterocyclyl, -CO(C₀₋₁₀ alkyl), -OCO(C₀₋₁₀ alkyl), ethynyl;
wherein, the hydrogen atom on R_{f}' can be substituted with the following substituents: halogen, -CN, -CH₃, -C₂H₅, -OC₁₋₅, C₃₋₆ cycloalkyl, 3-6 membered heterocyclyl, -CO(C₀₋₁₀ alkyl), -OCO(C₀₋₁₀ alkyl), ethenyl, propadienyl, ethynyl, further, the aforementioned alkyl moieties are replaceable with substituent selected from the group consisting of: -CN, -OCH₂F, -OCHF₂, -OCF₃, -OC₀₋₁₀ alky, C₃₋₄ cycloalkyl;
R_{g}' is selected from -H, straight-chain or branched-chain C₁₋₆ alkyl, -C₃₋₆ cycloalkyl, -CH₂CO(C₁₋₆ alkyl), -CH₂COO(C₀₋₆ alkyl), -CH₂COO(C₃₋₆ cycloalkyl), -CH₂COO(C₃₋₆ heterocyclyl), -CH₂CON(C₀₋₆ alkyl)(C₀₋₆ alkyl), benzyl, aryl, the hydrogen atom on these groups can be substituted with the following substituents: -F, -Cl, -CN, -NO₂, straight-chain or branched-chain C₁₋₁₀ alkyl, 3-6 membered heterocyclyl, -OH, -OC₁₋₅ alkyl, -N(C₁₋₃ alkyl)(C₁₋₃ alkyl), C₂₋₈ alkenyl, imidazolyl, oxazolyl, thiazolyl, pyridinyl, pyrimidinyl, phenyl; or R_{g}' is selected from 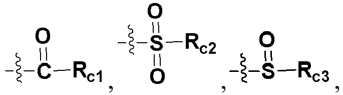 R_{c1}, R_{c2}, R_{c3} are independently selected from are independently selected from C₁₋₁₀ alkyl, -OC₀₋₅ alkyl, -O(C₃₋₆ cycloalkyl), -O(C₃₋₆ heterocyclyl), C₃₋₁₀ cycloalkyl, 3-6 membered heterocyclyl, -N(C₀₋₁₀ alkyl)(C₀₋₁₀ alkyl), alkenyl, alkynyl, aryl, heteroaryl, the hydrogen atom on these groups can be substituted with the following substituents: halogen, -CN, -CH₃, -C₂H₅, -OCH₃, -N(C₁₋₃ alkyl)(C₁₋₃ alkyl), -C₃₋₁₀ cycloalkyl, 3-6 membered heterocyclyl, ethenyl, the aforementioned alkyl moieties are replaceable with substituent selected from the group consisting of: -CN, -OCF₃, -OC₀₋₁₀ alkyl, C₃₋₄ cycloalkyl;
Rᵢ' is selected from halogen, cyano, phenyl, pyrrolyl, thiophenyl, furanyl, imidazolyl, oxazolyl, triazolyl, isoxazolyl, oxazolyl, pyridinyl, pyrimidinyl, pyrazinyl, triazinyl, cyclopropyl, cyclobutyl, cyclopentyl, cyclohexyl or the following group: 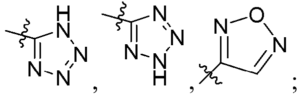 the aforementioned Rᵢ' are replaceable with a substituent selected from the group consisting of: halogen,
-CN, -NO₂, straight-chain or branched-chain C₁₋₆ alkyl, -OC₁₋₆ alkyl;
Wherein, 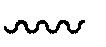 means substitution position.

19. The compound according to any of claims 10-18, or a pharmaceutically acceptable salt thereof, a stereoisomer thereof, a prodrug thereof, a solvate thereof, or a deuterated derivative thereof, **characterized in that** said M and the adjacent carbon atom together form a structure selected from: 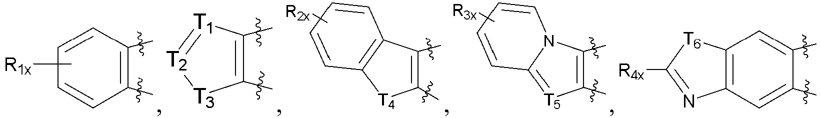
R₁ₓ, R₂ₓ, R₃ₓ, R₄ₓ are independently selected from: H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
T₁, T₂, T₃, T₄, T₅ are independently selected from: C-R₂₄, N, O or S;
T₆ is selected from C, N, O or S;
R₂₄ is selected from H, halogen, -CN, -NO₂, -NHCO(C₀₋₁₀ alkyl), CF₃, straight-chain C₁₋₃ alkyl, -OC₀₋₁₀ alkyl;
wherein, 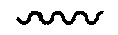 means substitution position.

20. The compound according to claims 19, or a pharmaceutically acceptable salt thereof, a stereoisomer thereof, a prodrug thereof, a solvate thereof, or a deuterated derivative thereof, **characterized in that** said M and the adjacent carbon atom together form a structure selected from: 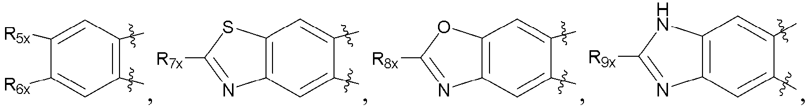 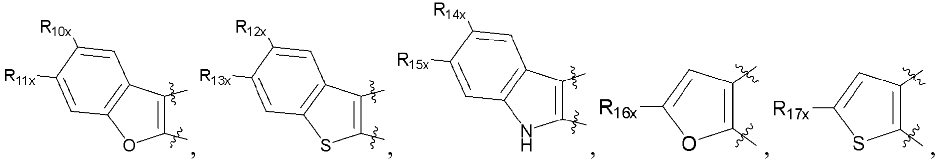 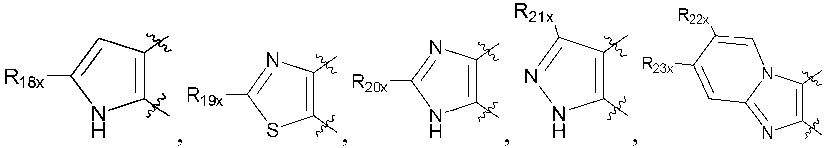
R₅ₓ-R₂₃ₓ are independently selected from:H, halogen, -NO₂, -CN, -CF₃, C₂₋₈ alkenyl, C₂₋₈ alkynyl, straight-chain or branched-chain C₁₋₁₀ alkyl, -N(C₀₋₁₀ alkyl)(C₀₋₁₀ alkyl), -OC₀₋₁₀ alkyl, C₃₋₁₀ cycloalkyl, N-heterocyclyl, O-heterocyclyl, S-heterocyclyl, -NHCO(C₀₋₁₀ alkyl);
wherein, 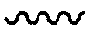 means substitution position.

21. The compound according to claim 1, or a pharmaceutically acceptable salt thereof, a stereoisomer thereof, a prodrug thereof, a solvate thereof, or a deuterated derivative thereof, **characterized in that** said compound has a structure of the following formula:

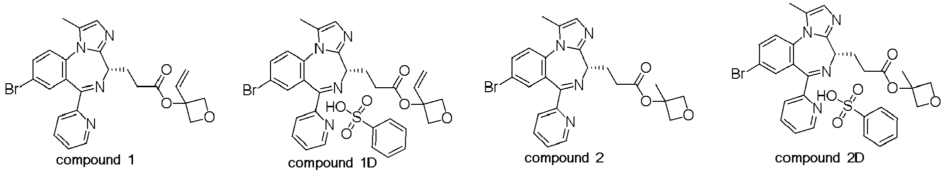

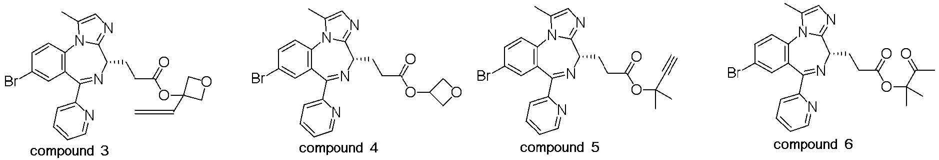

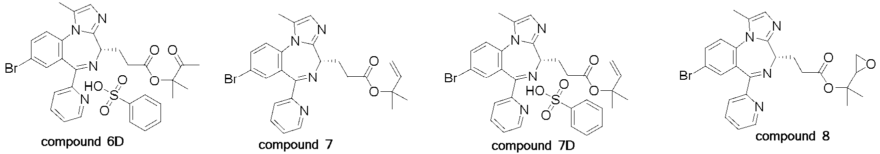

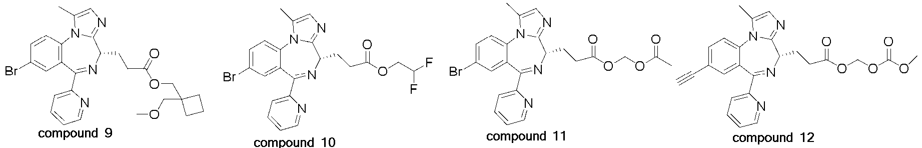

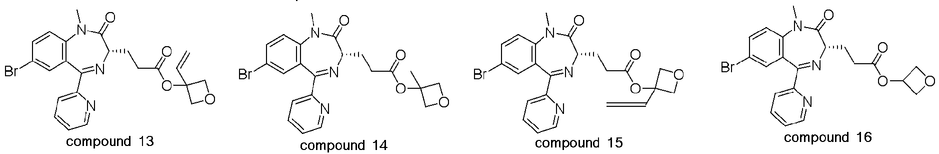

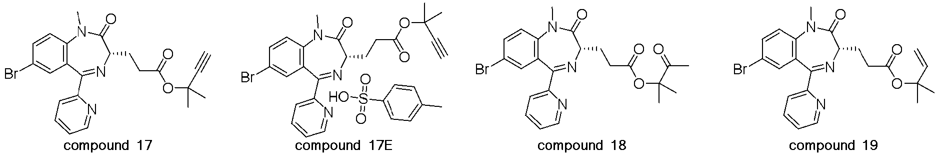

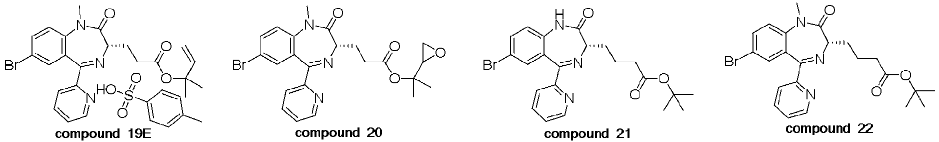

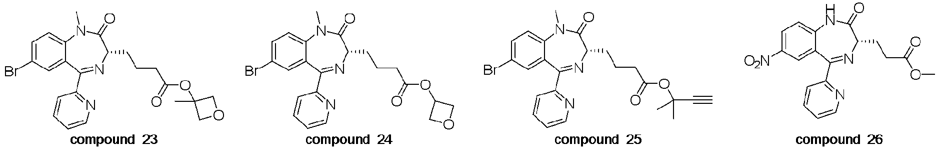

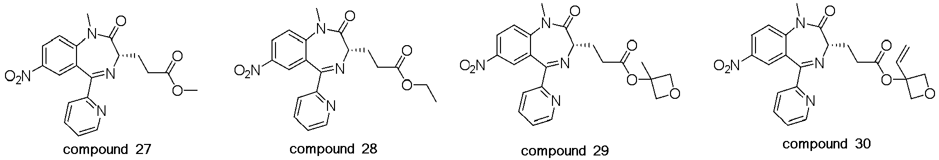

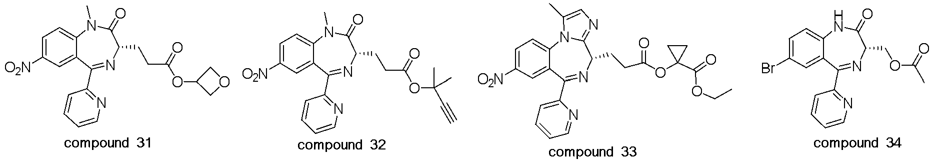

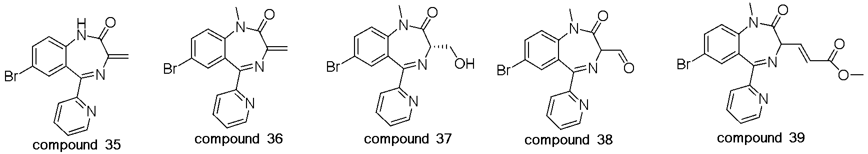

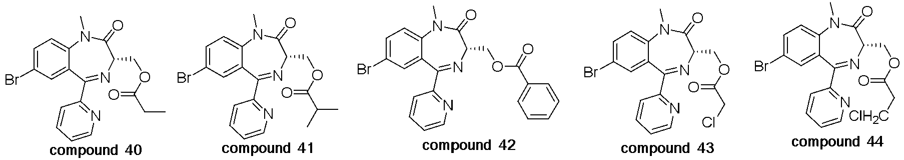

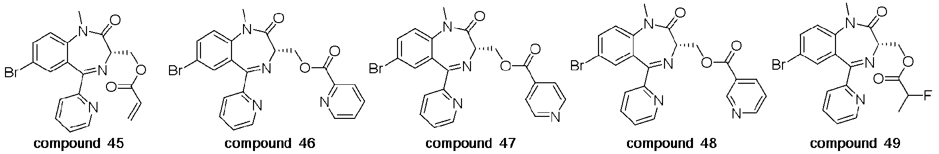

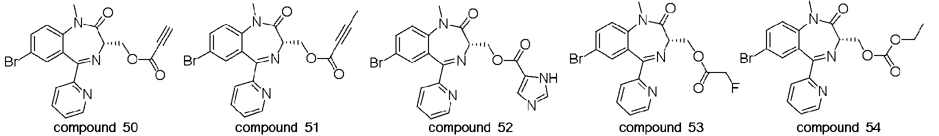

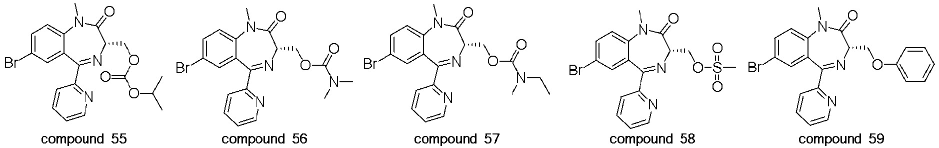

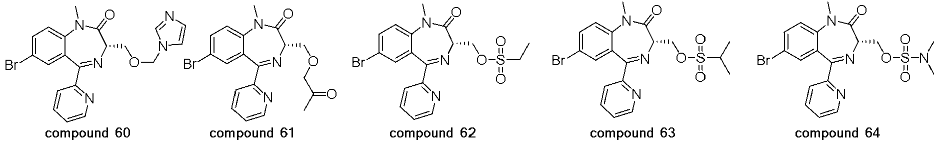

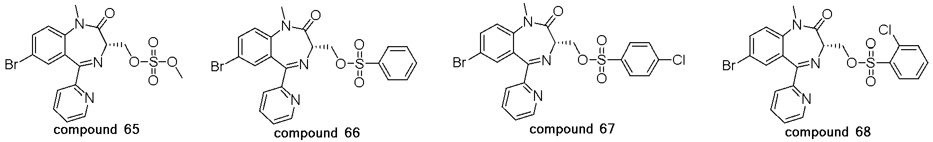

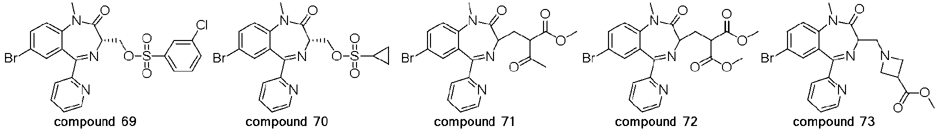

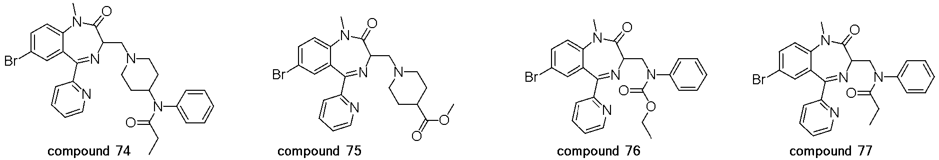

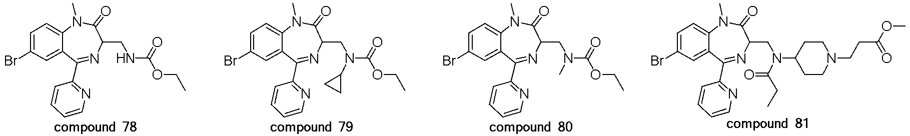

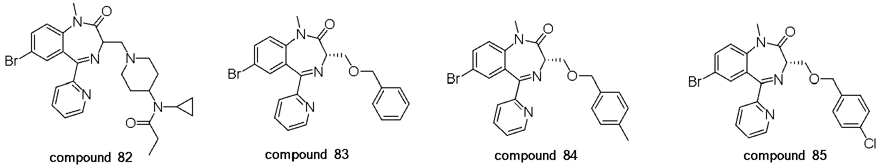

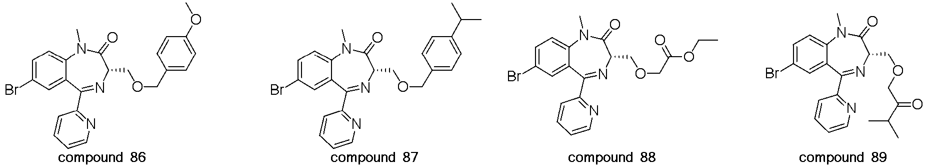

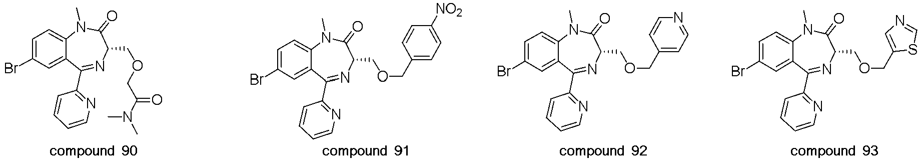

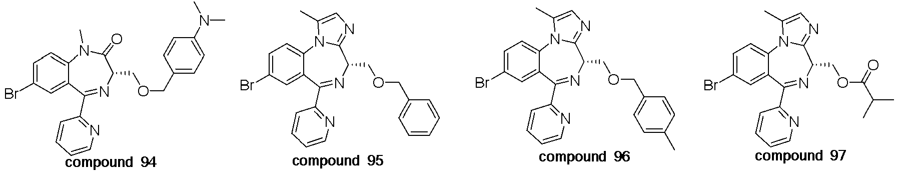

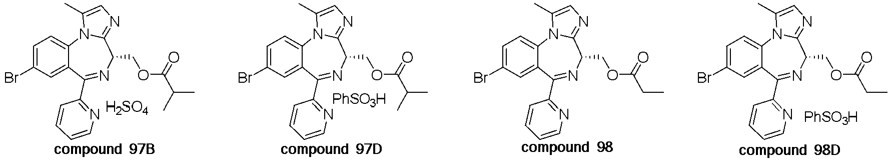

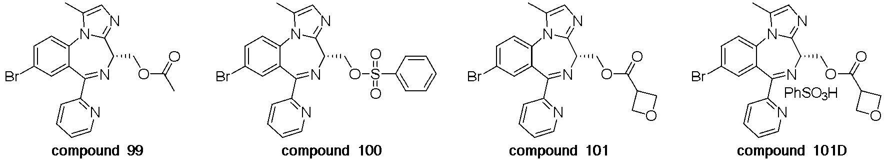

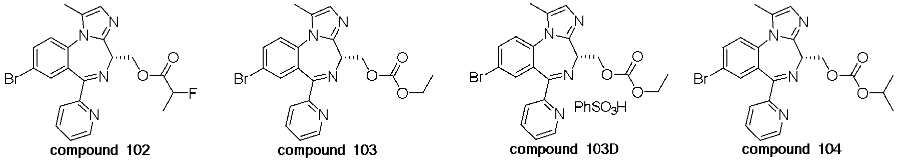

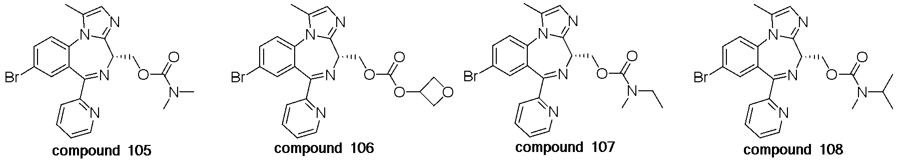

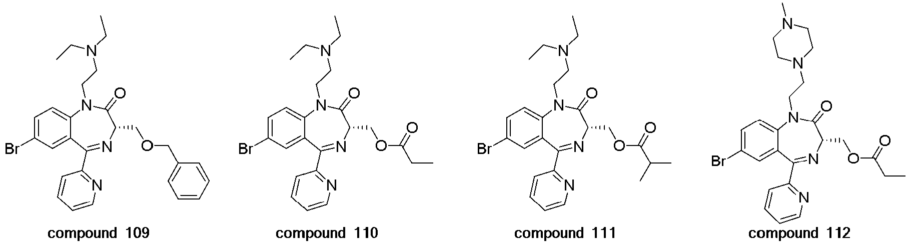

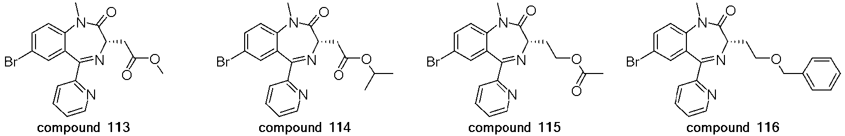

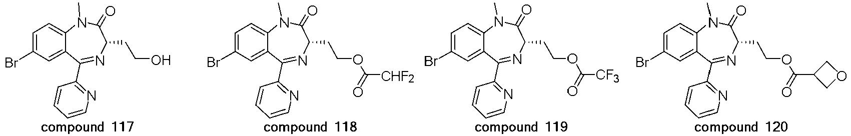

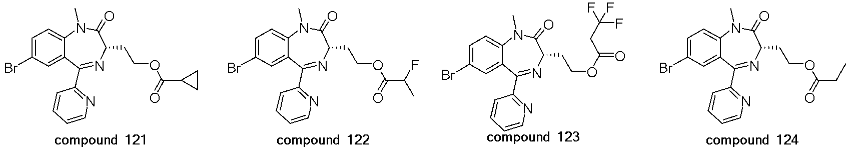

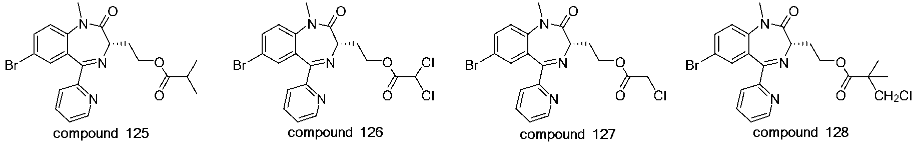

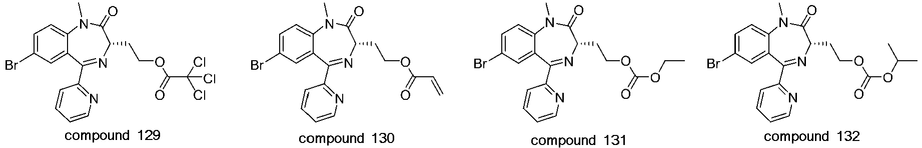

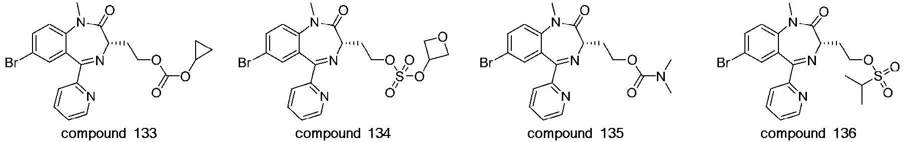

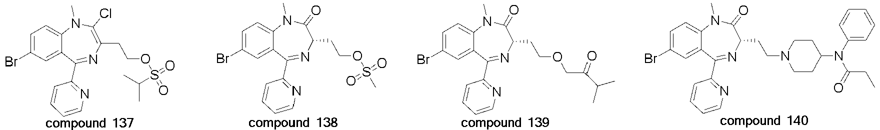

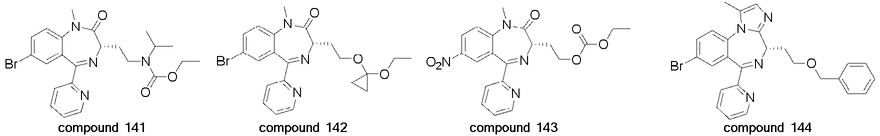

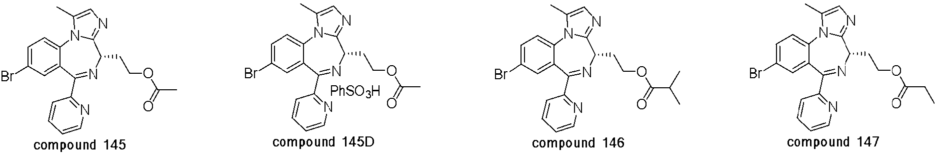

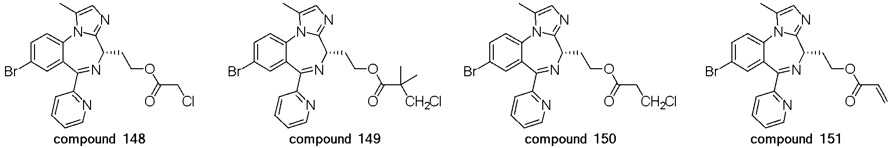

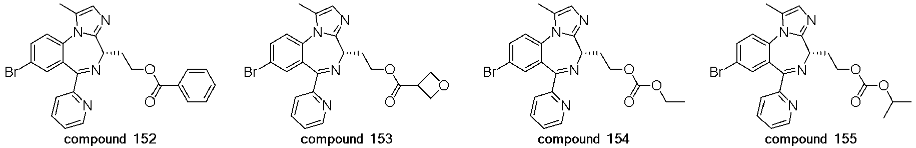

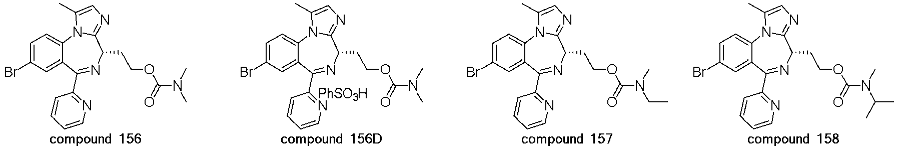

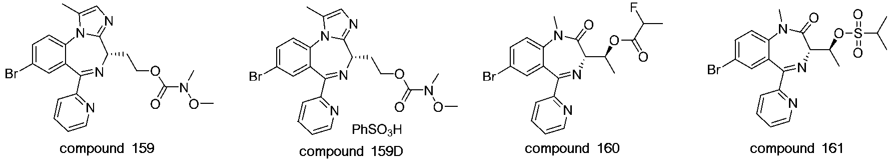

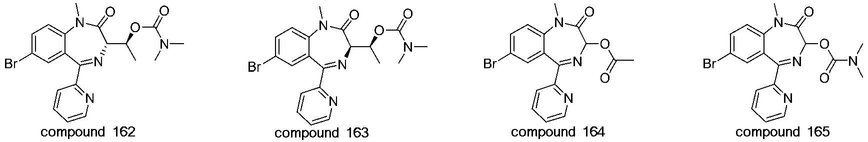

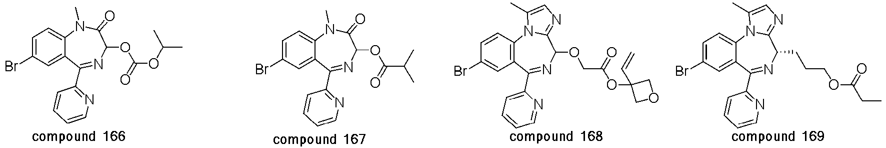

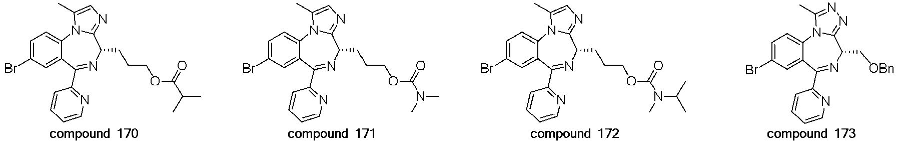

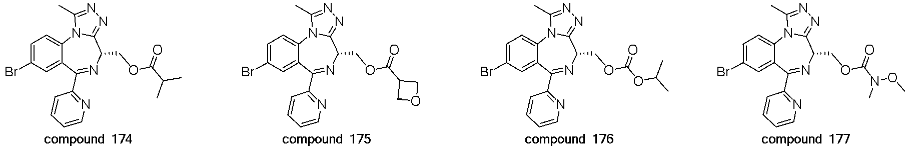

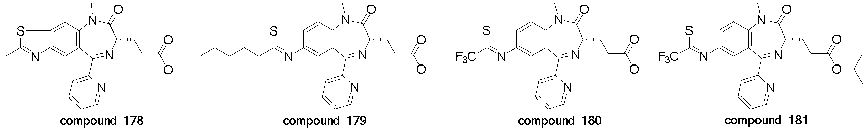

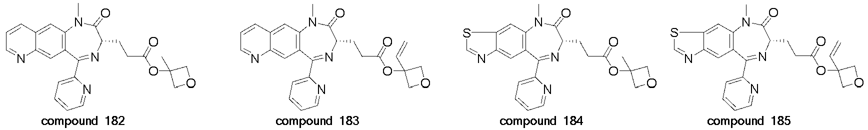

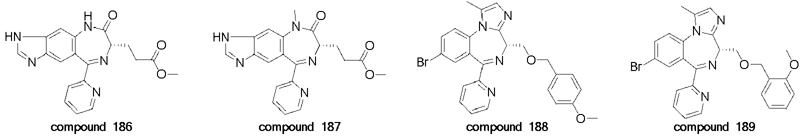

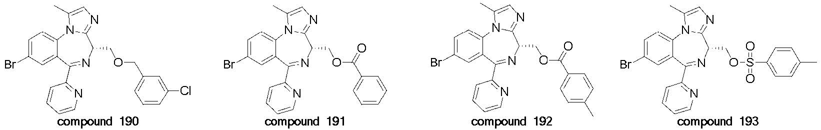

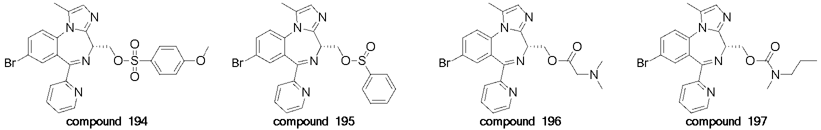

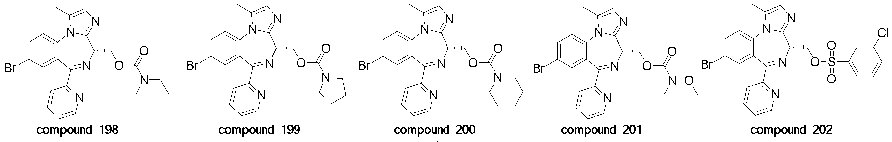

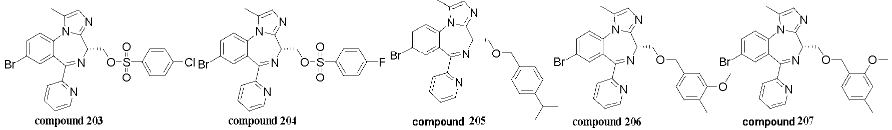

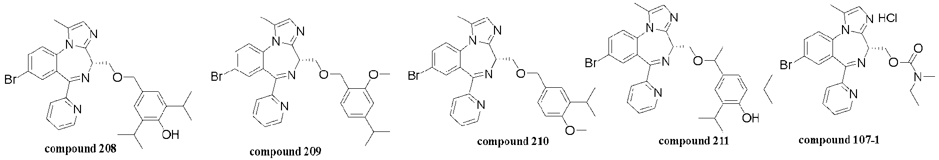

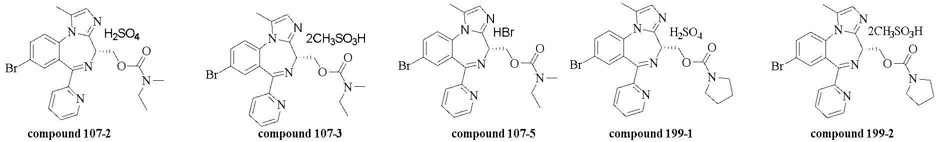

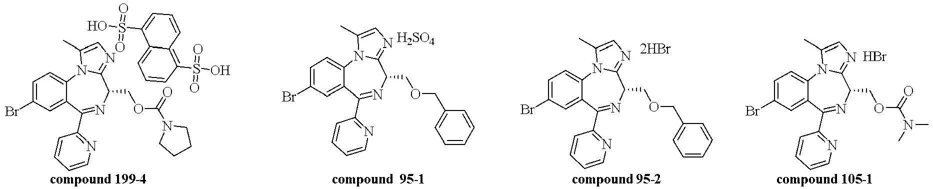

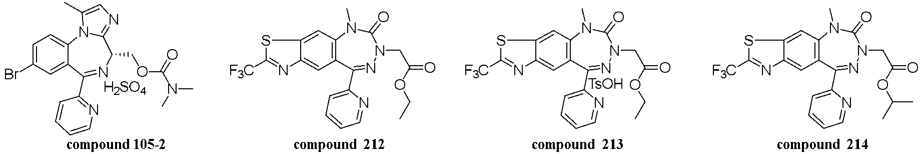

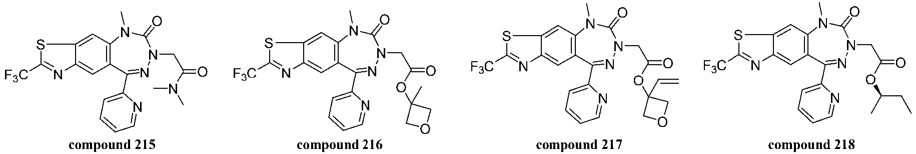

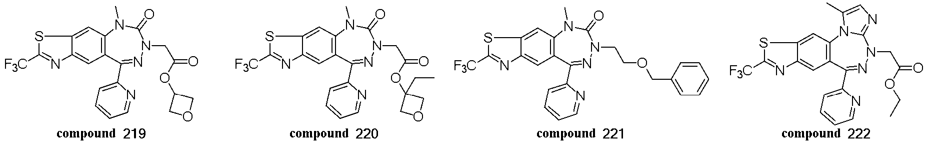

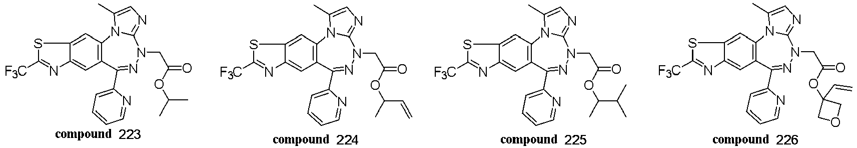

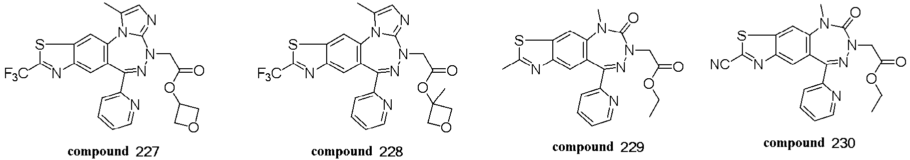

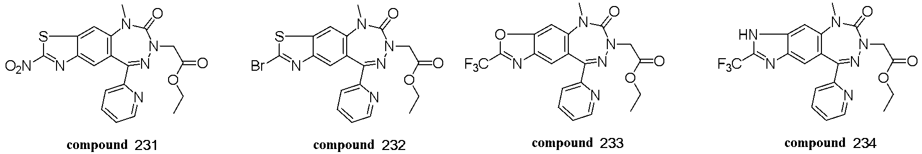

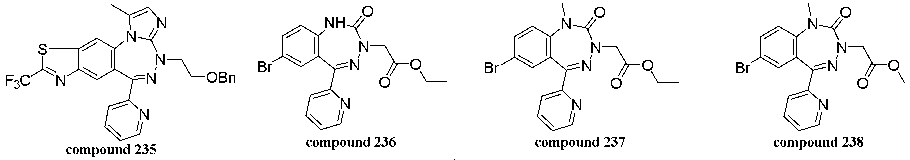

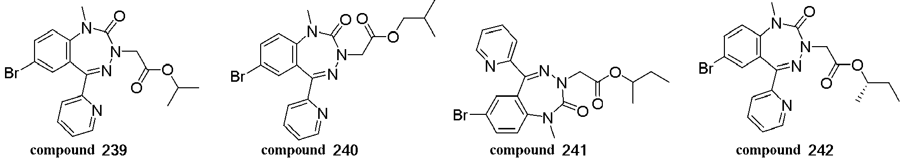

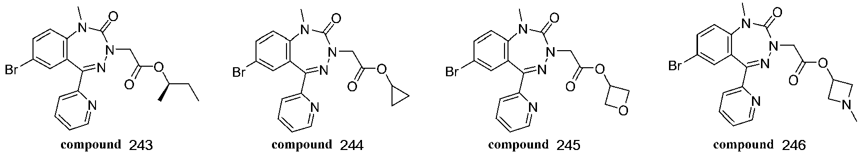

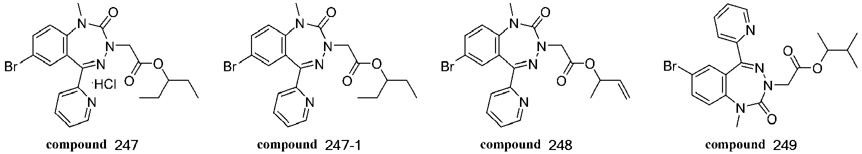

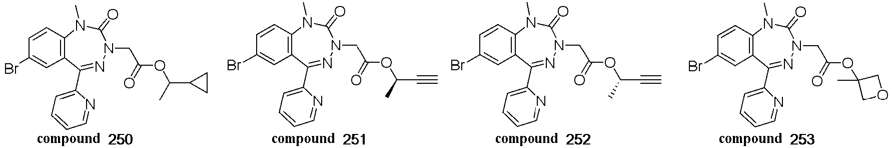

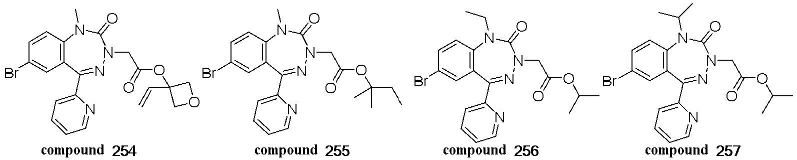

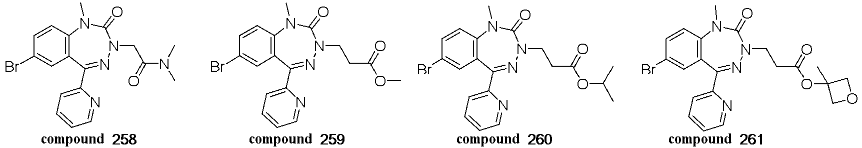

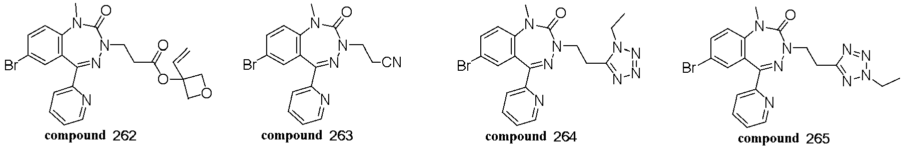

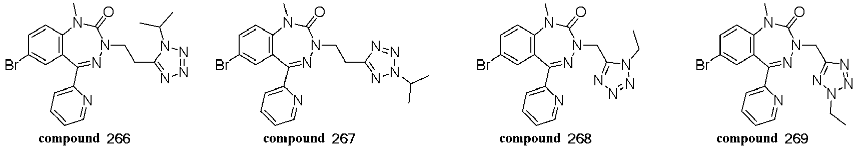

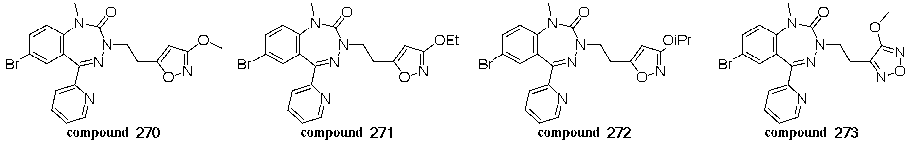

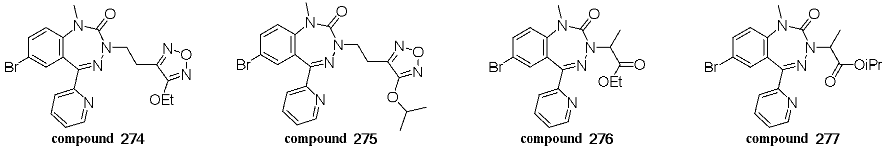

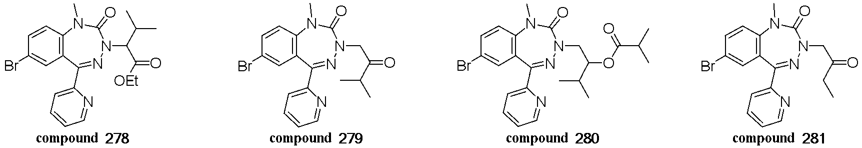

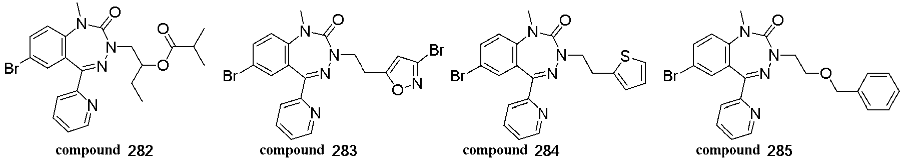

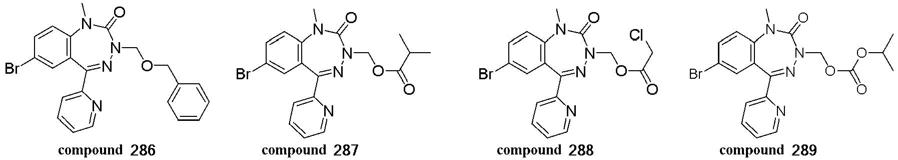

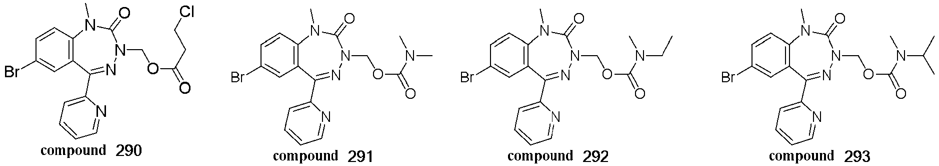

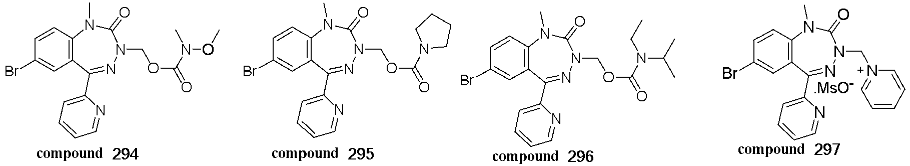

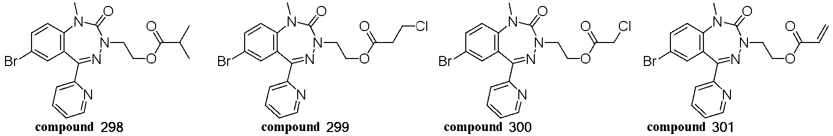

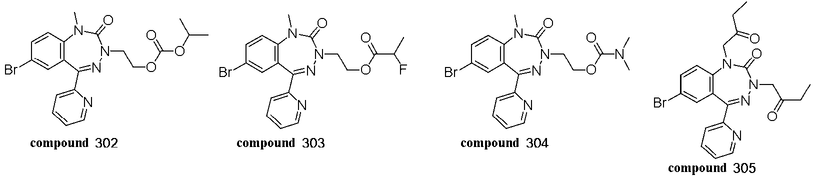

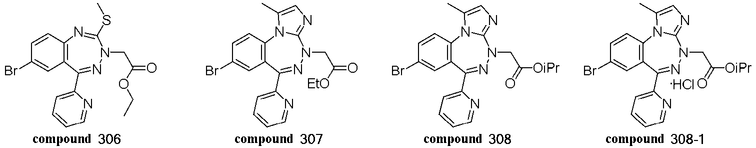

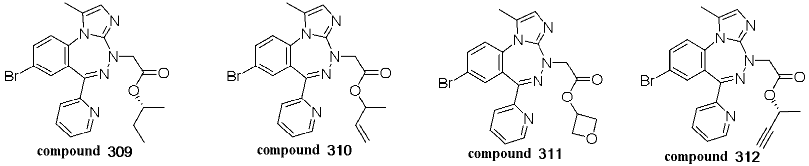

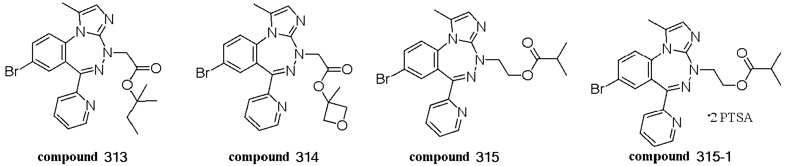

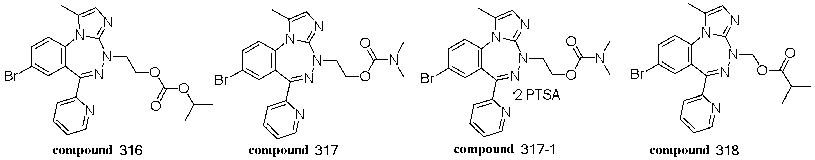

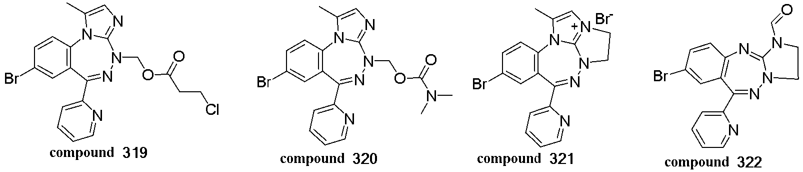

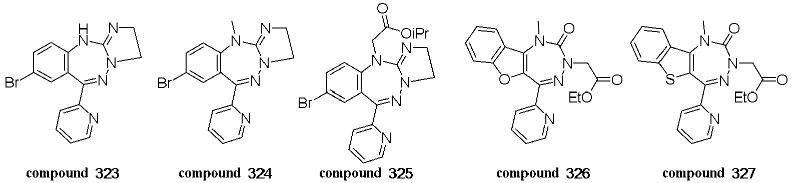

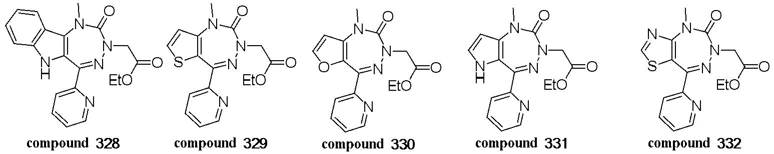

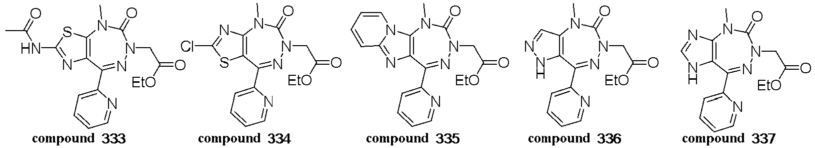

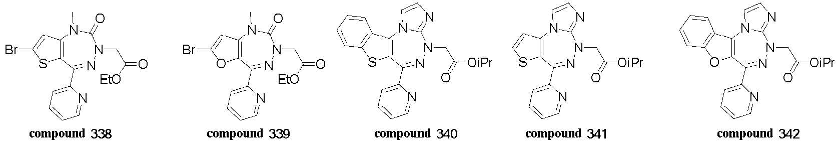

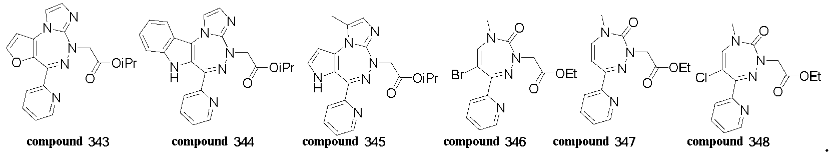

22. A drug, **characterized in that** said drug is prepared by using the compound according to anyone of claims 1-21, or a stereoisomer thereof, or a pharmaceutically acceptable salt thereof, or a solvate thereof, or a prodrug thereof, or a metabolite thereof, or a deuterated derivative thereof as active ingredients, with addition of pharmaceutically acceptable excipients.

23. Use of compound according to anyone of claims 1-21, or a stereoisomer thereof, or a pharmaceutically acceptable salt thereof, or a solvate thereof, or a prodrug thereof, or a metabolite thereof, or a deuterated derivative thereof, in preparation of a drug for exerting analgesic effect, and/or inducing anesthesia, sedation, hypnosis, and/or for controlling status epilepticus.
